# EUROPEAN PATENT SPECIFICATION

(11) **EP 3 972 414 B1**
(45) Date of publication and mention of the grant of the patent: **02.12.2026**
(21) Application number: 20809437.5
(22) Date of filing: 22.05.2020
(51) Int. Cl.: C07C 257/10, A01N 37/52, A01P 3/00, C07C 257/12, A01N 43/40, A01N 43/58, A01N 43/60, A01N 55/00, C07C 257/14, C07C 275/42, C07D 333/16

(54) **FUNGICIDAL ARYL AMIDINES**
FUNGIZIDE ARYLAMIDINE
ARYLAMIDINES FONGICIDES

(30) Priority: 23.05.2019 US 201962852074 P
(43) Date of publication of application: 30.03.2022
(73) Proprietor: Corteva Agriscience LLC, Indianapolis, IN 46268 (US)
(72) Inventor: BUYSSE, Ann M., Indianapolis, Indiana 46268 (US); NUGENT, Benjamin M., Indianapolis, Indiana 46268 (US); GUSTAFSON, Gary D., Indianapolis, Indiana 46268 (US); MEYER, Stacy T., Indianapolis, Indiana 46268 (US); LOY, Brian A., Indianapolis, Indiana 46268 (US); KISTER, Jeremy, Indianapolis, Indiana 46268 (US); GRUBER, Joseph M., Indianapolis, Indiana 46268 (US); JONES, David M., Indianapolis, Indiana 46268 (US); AVILA-ADAME, Cruz, Indianapolis, Indiana 46268 (US); WANG, Weiwei, Indianapolis, Indiana 46268 (US); BABIJ, Nicholas, Indianapolis, Indiana 46268 (US); PETKUS, Jeff, Indianapolis, Indiana 46268 (US)
(74) Representative: f & e patent
(86) International application number: PCT/US2020/034174
(87) International publication number: WO 2020/237131

(56) References cited:
- WO-A1-03/093224
- WO-A1-2017/005710
- WO-A2-03/032916
- US-A- 6 107 485
- US-A1- 2018 199 571
- DOUB ET AL.: "Some Phenylthiourea Derivatives and their Antituberculous Activity", JOURNAL OF THE AMERICAN CHEMICAL SOCIETY, vol. 80, no. 9, 1 January 1958 (1958-01-01), pages 2205 - 2217
- DATABASE PUBCHEM Substance [online] 17 March 2015 (2015-03-17), XP055761690, retrieved from ncbi Database accession no. SID 246582197

## Description

### CROSS-REFERNCE TO RELATED APPLICATIONS

This disclosure claims the benefit of U.S. Provisional Patent Application Serial Number 62/852,074.

### BACKGROUND & SUMMARY

Fungicides are compounds, of natural or synthetic origin, which act to protect and/or cure plants against damage caused by agriculturally relevant fungi. Generally, no single fungicide is useful in all situations. Consequently, research is ongoing to produce fungicides that may have better performance, are easier to use, and cost less.

WO 2017/005710 A1 discloses fungicide compositions for use in controlling fungal attacks on plants.

The present disclosure relates to aryl amidines and their use as fungicides. The compounds of the present disclosure may offer protection against ascomycetes, basidiomycetes, deuteromycetes and oomycetes.

One embodiment of the present disclosure may include compounds of Formula I: 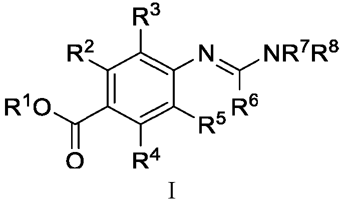 wherein
R' is selected from the group consisting of C₁-C₈ alkyl, C₁-C₈ substituted alkyl, C₂-C₈ alkenyl, C₂-C₈ substituted alkenyl, C₂-C₈ alkynyl, C₂-C₈ substituted alkynyl, C₃-C₈ cycloalkyl, C₃-C₈ substituted cycloalkyl, C₃-C₈ heterocycloalkyl, C₃-C₈ substituted heterocycloalkyl, C₅-C₇ heteroaryl, C₅-C₇ substituted heteroaryl, phenyl, substituted phenyl, benzyl, and substituted benzyl;
each R², R⁴, and R⁵ independently is selected from the group consisting of hydrogen, halogen, cyano, nitro, C₁-C₈ alkyl, C₁-C₈ substituted alkyl, C₂-C₈ alkenyl, C₂-C₈ substituted alkenyl, C₂-C₈ alkynyl, C₂-C₈ substituted alkynyl, C₁-C₈ alkoxy, and C₁-C₈ substituted alkoxy;
R³ is selected from the group consisting of consisting of halogen, cyano, C₁-C₈ alkyl, C₁-C₈ substituted alkyl, C₂-C₈ alkenyl, C₂-C₈ substituted alkenyl, C₂-C₈ alkynyl, C₂-C₈ substituted alkynyl, C₁-C₈ alkoxy, and C₁-C₈ substituted alkoxy;
R⁶ is selected from the group consisting of hydrogen, C₁-C₈ alkyl, C₁-C₈ substituted alkyl, C₂-C₈ alkenyl, C₂₋-C₈ substituted alkenyl, C₂-C₈ alkynyl, C₂-C₈ substituted alkynyl, C₁-C₈ alkoxy, C₁-C₈ substituted alkoxy, thiol, alkylthio, and substituted alkylthio;
or R⁶ and R⁷ may be covalently bonded together to form a saturated or unsaturated C₃-C₈ heterocycloalkyl or C₃-C₈ substituted heterocycloalkyl group;
each R⁷ and R⁸ independently is selected from the group consisting of C₁-C₈ alkyl, C₁-C₈ substituted alkyl, C₂-C₈ alkenyl, C₂-C₈ substituted alkenyl, C₂-C₈ alkynyl, C₂-C₈ substituted alkynyl, C₃-C₈ cycloalkyl, C₃-C₈ substituted cycloalkyl, benzyl, and substituted benzyl;
or R⁷ and R⁸ may be covalently bonded together to form a saturated or unsaturated C₃-C₈ heterocycloalkyl or C₃-C₈ substituted heterocycloalkyl group;
wherein any and all heterocyclic rings may contain up to three heteroatoms selected from the group consisting of O, N, and S;
or a tautomer or salt thereof,
with the proviso that the compound is not ethyl 4-(((dimethylamino)methylene)amino)-3,5-dimethylbenzoate hydrochloride, or methyl 3-bromo-4-[(E)-dimethylaminomethylideneamino]benzoate.

Another embodiment of the present disclosure may include a fungicidal composition for the control or prevention of fungal attack comprising the compounds described above and a phytologically acceptable carrier material.

Yet another embodiment of the present disclosure may include a method for the control or prevention of fungal attack on a plant, the method including the steps of applying a fungicidally effective amount of one or more of the compounds described above to at least one of the fungus, a seed, the plant, and an area adjacent to the plant.

It will be understood by those skilled in the art that the following terms may include generic "R"-groups within their definitions, e.g., "the term alkoxy refers to an -OR substituent". It is also understood that within the definitions for the following terms, these "R" groups are included for illustration purposes and should not be construed as limiting or being limited by substitutions about Formula I.

The term "alkyl" refers to a branched, unbranched, or saturated acyclic substituent consisting of carbon and hydrogen atoms including, but not limited to, methyl, ethyl, propyl, butyl, isopropyl, isobutyl, tertiary butyl, pentyl, hexyl, and the like.

The term "alkenyl" refers to an acyclic, unsaturated (at least one carbon-carbon double bond), branched or unbranched, substituent consisting of carbon and hydrogen, including, but not limited to, ethenyl, propenyl, butenyl, isopropenyl, isobutenyl, and the like.

The term "alkynyl" refers to an acyclic, unsaturated (at least one carbon-carbon triple bond), branched or unbranched, substituent consisting of carbon and hydrogen, for example, ethynyl, propargyl, butynyl, and pentynyl.

The term "cycloalkenyl" refers to a monocyclic or polycyclic, unsaturated (at least one carbon-carbon double bond) substituent consisting of carbon and hydrogen, for example, cyclobutenyl, cyclopentenyl, cyclohexenyl, norbomenyl, bicyclo[2.2.2]octenyl, tetrahydronaphthyl, hexahydronaphthyl, and octahydronaphthyl.

The term "cycloalkyl" refers to a monocyclic or polycyclic, saturated substituent consisting of carbon and hydrogen, for example, cyclopropyl, cyclobutyl, cyclopentyl, norbomyl, bicyclo[2.2.2]octyl, and decahydronaphthyl.

The term "cycloalkoxy" refers to a cycloalkyl further consisting of a carbon-oxygen single bond, for example, cyclopropyloxy, cyclobutyloxy, cyclopentyloxy, norbomyloxy, and bicyclo[2.2.2]octyloxy.

The terms "aryl" and "Ar" refer to any aromatic ring, mono- or bi-cyclic, containing 0 heteroatoms, for example phenyl and naphthyl.

The terms "heteroaryl" refers to any aromatic ring, mono- or bi-cyclic, containing 1 or more heteroatoms, for example pyridinyl, piperazinyl, and thiophenyl.

The term "heterocycloalkyl" refers to any non-aromatic, mono- or bi-cyclic ring, containing carbon and hydrogen atoms and one or more heteroatoms.

The term "alkoxy" refers to an -OR substituent.

The term "cyano" refers to a -C≡N substituent.

The term "amino" refers to an -N(R)₂ substituent.

The term "halogen" or "halo" refers to one or more halogen atoms, defined as F, Cl, Br, and I.

The term "nitro" refers to a -NO₂ substituent.

The term "thiol" refers to a -SH substituent.

The term "alkylthio" refers to a -SR substituent.

The term "benzyl" refers to a -CH₂-phenyl substituent.

Throughout the disclosure, reference to the compounds of Formula I is read as also including all stereoisomers, for example diastereomers, enantiomers, and mixtures thereof. In another embodiment, Formula (I) is read as also including salts or hydrates thereof. Exemplary salts include, but are not limited to: hydrochloride, hydrobromide, hydroiodide, trifluoroacetate, and trifluoromethane sulfonate.

It is also understood by those skilled in the art that additional substitution is allowable, unless otherwise noted, as long as the rules of chemical bonding and strain energy are satisfied and the product still exhibits fungicidal activity.

Another embodiment of the present disclosure is a use of a compound of Formula I, for protection of a plant against attack by a phytopathogenic organism or the treatment of a plant infested by a phytopathogenic organism, comprising the application of a compound of Formula I, or a composition comprising the compound to soil, a plant, a part of a plant, foliage, and/or roots.

Additionally, another embodiment of the present disclosure is a composition useful for protecting a plant against attack by a phytopathogenic organism and/or treatment of a plant infested by a phytopathogenic organism comprising a compound of Formula I and a phytologically acceptable carrier material.

### DETAILED DESCRIPTION

The compounds of the present disclosure may be applied by any of a variety of known techniques, either as the compounds or as formulations comprising the compounds. For example, the compounds may be applied to the roots or foliage of plants for the control of various fungi, without damaging the commercial value of the plants. The materials may be applied in the form of any of the generally used formulation types, for example, as solutions, dusts, wettable powders, flowable concentrate, or emulsifiable concentrates.

Preferably, the compounds of the present disclosure are applied in the form of a formulation, comprising one or more of the compounds of Formula I with a phytologically acceptable carrier. Concentrated formulations may be dispersed in water, or other liquids, for application, or formulations may be dust-like or granular, which may then be applied without further treatment. The formulations can be prepared according to procedures that are conventional in the agricultural chemical art.

The present disclosure contemplates all vehicles by which one or more of the compounds may be formulated for delivery and used as a fungicide. Typically, formulations are applied as aqueous suspensions or emulsions. Such suspensions or emulsions may be produced from water-soluble, water-suspendible, or emulsifiable formulations which are solids, usually known as wettable powders; or liquids, usually known as emulsifiable concentrates, aqueous suspensions, or suspension concentrates. As will be readily appreciated, any material to which these compounds may be added may be used, provided it yields the desired utility without significant interference with the activity of these compounds as antifungal agents.

Wettable powders, which may be compacted to form water-dispersible granules, comprise an intimate mixture of one or more of the compounds of Formula I, an inert carrier and surfactants. The concentration of the compound in the wettable powder may be from about 10 percent to about 90 percent by weight based on the total weight of the wettable powder, more preferably about 25 weight percent to about 75 weight percent. In the preparation of wettable powder formulations, the compounds may be compounded with any finely divided solid, such as prophyllite, talc, chalk, gypsum, Fuller's earth, bentonite, attapulgite, starch, casein, gluten, montmorillonite clays, diatomaceous earths, purified silicates or the like. In such operations, the finely divided carrier and surfactants are typically blended with the compound(s) and milled.

Emulsifiable concentrates of the compounds of Formula I may comprise a convenient concentration, such as from about 1 weight percent to about 50 weight percent of the compound, in a suitable liquid, based on the total weight of the concentrate. The compounds may be dissolved in an inert carrier, which is either a water-miscible solvent or a mixture of water-immiscible organic solvents, and emulsifiers. The concentrates may be diluted with water and oil to form spray mixtures in the form of oil-in-water emulsions. Useful organic solvents include aromatics, especially the high-boiling naphthalenic and olefinic portions of petroleum such as heavy aromatic naphtha. Other organic solvents may also be used, for example, terpenic solvents, including rosin derivatives, aliphatic ketones, such as cyclohexanone, and complex alcohols, such as 2-ethoxyethanol.

Emulsifiers which may be advantageously employed herein may be readily determined by those skilled in the art and include various nonionic, anionic, cationic and amphoteric emulsifiers, or a blend of two or more emulsifiers. Examples of nonionic emulsifiers useful in preparing the emulsifiable concentrates include the polyalkylene glycol ethers and condensation products of alkyl and aryl phenols, aliphatic alcohols, aliphatic amines or fatty acids with ethylene oxide, propylene oxides such as the ethoxylated alkyl phenols and carboxylic esters solubilized with the polyol or polyoxyalkylene. Cationic emulsifiers include quaternary ammonium compounds and fatty amine salts. Anionic emulsifiers include the oilsoluble salts (e.g., calcium) of alkylaryl sulphonic acids, oil-soluble salts or sulfated polyglycol ethers and appropriate salts of phosphated- polyglycol ether.

Representative organic liquids which may be employed in preparing the emulsifiable concentrates of the compounds of the present disclosure are the aromatic liquids such as xylene, propyl benzene fractions; or mixed naphthalene fractions, mineral oils, substituted aromatic organic liquids such as dioctyl phthalate; kerosene; dialkyl amides of various fatty acids, particularly the dimethyl amides of fatty glycols and glycol derivatives such as the n-butyl ether, ethyl ether or methyl ether of diethylene glycol, the methyl ether of triethylene glycol, petroleum fractions or hydrocarbons such as mineral oil, aromatic solvents, paraffinic oils, and the like; vegetable oils such as soy bean oil, rape seed oil, olive oil, castor oil, sunflower seed oil, coconut oil, corn oil, cotton seed oil, linseed oil, palm oil, peanut oil, safflower oil, sesame oil, tung oil and the like; esters of the above vegetable oils; and the like. Mixtures of two or more organic liquids may also be employed in the preparation of the emulsifiable concentrate. Organic liquids include xylene, and propyl benzene fractions, with xylene being most preferred in some cases. Surface-active dispersing agents are typically employed in liquid formulations and in an amount of from 0.1 to 20 percent by weight based on the combined weight of the dispersing agent with one or more of the compounds. The formulations can also contain other compatible additives, for example, plant growth regulators and other biologically active compounds used in agriculture.

Aqueous suspensions comprise suspensions of one or more water-insoluble compounds of Formula I, dispersed in an aqueous vehicle at a concentration in the range from about 1 to about 50 weight percent, based on the total weight of the aqueous suspension. Suspensions are prepared by finely grinding one or more of the compounds, and vigorously mixing the ground material into a vehicle comprised of water and surfactants chosen from the same types discussed above. Other components, such as inorganic salts and synthetic or natural gums, may also be added to increase the density and viscosity of the aqueous vehicle.

The compounds of Formula I can also be applied as granular formulations, which are particularly useful for applications to the soil. Granular formulations generally contain from about 0.5 to about 10 weight percent, based on the total weight of the granular formulation of the compound(s), dispersed in an inert carrier which consists entirely or in large part of coarsely divided inert material such as attapulgite, bentonite, diatomite, clay or a similar inexpensive substance. Such formulations are usually prepared by dissolving the compounds in a suitable solvent and applying it to a granular carrier which has been preformed to the appropriate particle size, in the range of from about 0.5 to about 3 mm. A suitable solvent is a solvent in which the compound is substantially or completely soluble. Such formulations may also be prepared by making a dough or paste of the carrier and the compound and solvent, and crushing and drying to obtain the desired granular particle.

Dusts containing the compounds of Formula I may be prepared by intimately mixing one or more of the compounds in powdered form with a suitable dusty agricultural carrier, such as, for example, kaolin clay, ground volcanic rock, and the like. Dusts can suitably contain from about 1 to about 10 weight percent of the compounds, based on the total weight of the dust.

The formulations may additionally contain adjuvant surfactants to enhance deposition, wetting, and penetration of the compounds onto the target crop and organism. These adjuvant surfactants may optionally be employed as a component of the formulation or as a tank mix. The amount of adjuvant surfactant will typically vary from 0.01 to 1.0 percent by volume, based on a spray-volume of water, preferably 0.05 to 0.5 volume percent. Suitable adjuvant surfactants include, but are not limited to ethoxylated nonyl phenols, ethoxylated synthetic or natural alcohols, salts of the esters or sulphosuccinic acids, ethoxylated organosilicones, ethoxylated fatty amines, blends of surfactants with mineral or vegetable oils, crop oil concentrate (mineral oil (85%) + emulsifiers (15%)); nonylphenol ethoxylate; benzylcocoalkyldimethyl quaternary ammonium salt; blend of petroleum hydrocarbon, alkyl esters, organic acid, and anionic surfactant; C₉ - C₁₁ alkylpolyglycoside; phosphated alcohol ethoxylate; natural primary alcohol (C₁₂ - C₁₆) ethoxylate; di-sec-butylphenol EO-PO block copolymer; polysiloxane-methyl cap; nonylphenol ethoxylate + urea ammonium nitrrate; emulsified methylated seed oil; tridecyl alcohol (synthetic) ethoxylate (8EO); tallow amine ethoxylate (15 EO); PEG(400) dioleate-99. The formulations may also include oil-in-water emulsions such as those disclosed in U.S. Patent Application Serial No. 11/495,228.

The formulations may optionally include combinations that contain other pesticidal compounds. Such additional pesticidal compounds may be fungicides, insecticides, herbicides, nematocides, miticides, arthropodicides, bactericides or combinations thereof that are compatible with the compounds of the present disclosure in the medium selected for application, and not antagonistic to the activity of the present compounds. Accordingly, in such embodiments, the other pesticidal compound is employed as a supplemental toxicant for the same or for a different pesticidal use. The compounds of Formula I and the pesticidal compound in the combination can generally be present in a weight ratio of from 1:100 to 100:1.

The compounds of the present disclosure may also be combined with other fungicides to form fungicidal mixtures and synergistic mixtures thereof. The fungicidal compounds of the present disclosure are often applied in conjunction with one or more other fungicides to control a wider variety of undesirable diseases. When used in conjunction with other fungicide(s), the presently claimed compounds may be formulated with the other fungicide(s), tank-mixed with the other fungicide(s) or applied sequentially with the other fungicide(s). Such other fungicides may include 2-(thiocyanatomethylthio)-benzothiazole, 2-phenylphenol, 8-hydroxyquinoline sulfate, ametoctradin, aminopyrifen, amisulbrom, antimycin, *Ampelomyces quisqualis,* azaconazole, *Bacillus subtilis, Bacillus subtilis* strain QST713, benalaxyl, benomyl, benthiavalicarb-isopropyl, benzovindiflupyr, benzylaminobenzene-sulfonate (BABS) salt, bicarbonates, biphenyl, bismerthiazol, bitertanol, bixafen, blasticidin-S, borax, Bordeaux mixture, boscalid, bromuconazole, bupirimate, calcium polysulfide, captafol, captan, carbendazim, carboxin, carpropamid, carvone, chlazafenone, chloroneb, chlorothalonil, chlozolinate, *Coniothyrium minitans,* copper hydroxide, copper octanoate, copper oxychloride, copper sulfate, copper sulfate (tribasic), cuprous oxide, cyazofamid, cyflufenamid, cymoxanil, cyproconazole, cyprodinil, dazomet, debacarb, diammonium ethylenebis-(dithiocarbamate), dichlofluanid, dichlorophen, diclocymet, diclomezine, dichloran, diethofencarb, difenoconazole, difenzoquat ion, diflumetorim, dimethomorph, dimoxystrobin, diniconazole, diniconazole-M, dinobuton, dinocap, diphenylamine, dithianon, dodemorph, dodemorph acetate, dodine, dodine free base, edifenphos, enestrobin, enestroburin, epoxiconazole, ethaboxam, ethoxyquin, etridiazole, famoxadone, fenamidone, fenarimol, fenbuconazole, fenfuram, fenhexamid, fenoxanil, fenpiclonil, fenpropidin, fenpropimorph, fenpyrazamine, fentin, fentin acetate, fentin hydroxide, ferbam, ferimzone, fluazinam, fludioxonil, fluindapyr, flumorph, fluopicolide, fluopyram, fluoroimide, fluoxapiprolin, fluoxastrobin, fluquinconazole, flusilazole, flusulfamide, flutianil, flutolanil, flutriafol, fluxapyroxad, folpet, formaldehyde, fosetyl, fosetyl-aluminium, fuberidazole, furalaxyl, furametpyr, guazatine, guazatine acetates, GY-81, hexachlorobenzene, hexaconazole, hymexazol, imazalil, imazalil sulfate, imibenconazole, iminoctadine, iminoctadine triacetate, iminoctadine tris(albesilate), inpyrfluxam, iodocarb, ipconazole, ipfenpyrazolone, iprobenfos, iprodione, iprovalicarb, isofetamide, isoflucypram, isoprothiolane, isopyrazam, isotianil, kasugamycin, kasugamycin hydrochloride hydrate, kresoxium-methyl, laminarin, mancopper, mancozeb, mandipropamid, maneb, mefenoxam, mepanipyrim, mepronil, meptyl-dinocap, mercuric chloride, mercuric oxide, mercurous chloride, metalaxyl, metalaxyl-M, metam, metam-ammonium, metam-potassium, metam-sodium, metconazole, methasulfocarb, methyl iodide, methyl isothiocyanate, metiram, metominostrobin, metrafenone, mildiomycin, myclobutanil, nabam, nitrothal-isopropyl, nuarimol, octhilinone, ofurace, oleic acid (fatty acids), orysastrobin, oxadixyl, oxathiapiprolin, oxine-copper, oxpoconazole fumarate, oxycarboxin, pefurazoate, penconazole, pencycuron, penflufen, pentachlorophenol, pentachlorophenyl laurate, penthiopyrad, phenylmercury acetate, phosphonic acid, phthalide, picoxystrobin, polyoxin B, polyoxins, polyoxorim, potassium bicarbonate, potassium hydroxyquinoline sulfate, probenazole, prochloraz, procymidone, propamocarb, propamocarb hydrochloride, propiconazole, propineb, proquinazid, prothioconazole, pydiflumetofen, pyrametostrobin, pyraoxystrobin, pyraclostrobin, pyraziflumid, pyrazophos, pyribencarb, pyributicarb, pyrifenox, pyrimethanil, pyriofenone, pyroquilon, quinoclamine, quinoxyfen, quintozene, *Reynoutria sachalinensis* extract, sedaxane, silthiofam, simeconazole, sodium 2-phenylphenoxide, sodium bicarbonate, sodium pentachlorophenoxide, spiroxamine, sulfur, SYP-Z048, tar oils, tebuconazole, tebufloquin, tecnazene, tetraconazole, thiabendazole, thifluzamide, thiophanate-methyl, thiram, tiadinil, tolclofos-methyl, tolylfluanid, triadimefon, triadimenol, triazoxide, tricyclazole, tridemorph, trifloxystrobin, triflumizole, triforine, triticonazole, validamycin, valifenalate, valiphenal, vinclozolin, zineb, ziram, zoxamide, *Candida oleophila, Fusarium oxysporum, Gliocladium* spp., *Phlebiopsis gigantea, Streptomyces griseoviridis, Trichoderma* spp., (RS)-N-(3,5-dichlorophenyl)-2-(methoxymethyl)-succinimide, 1,2-dichloropropane, 1,3-dichloro-1,1,3,3-tetrafluoroacetone hydrate, 1-chloro-2,4-dinitronaphthalene, 1-chloro-2-nitropropane, 2-(2-heptadecyl-2-imidazolin-1-yl)ethanol, 2,3-dihydro-5-phenyl-1,4-dithi-ine 1,1,4,4-tetraoxide, 2-methoxyethylmercury acetate, 2-methoxyethylmercury chloride, 2-methoxyethylmercury silicate, 3-(4-chlorophenyl)-5-methylrhodanine, 4-(2-nitroprop-1-enyl)phenyl thiocyanateme, ampropylfos, anilazine, azithiram, barium polysulfide, Bayer 32394, benodanil, benquinox, bentaluron, benzamacril; benzamacril-isobutyl, benzamorf, binapacryl, bis(methylmercury) sulfate, bis(tributyltin) oxide, buthiobate, cadmium calcium copper zinc chromate sulfate, carbamorph, CECA, chlobenthiazone, chloraniformethan, chlorfenazole, chlorquinox, climbazole, copper bis(3-phenylsalicylate), copper zinc chromate, coumoxystrobin, cufraneb, cupric hydrazinium sulfate, cuprobam, cyclafuramid, cypendazole, cyprofuram, decafentin, dichlobentiazox, dichlone, dichlozoline, diclobutrazol, dimethirimol, dinocton, dinosulfon, dinoterbon, dipymetitrone, dipyrithione, ditalimfos, dodicin, drazoxolon, EBP, enoxastrobin, ESBP, etaconazole, etem, ethirim, fenaminstrobin, fenaminosulf, fenapanil, fenitropan, fenpicoxamid, florylpicoxamid, flufenoxystrobin, fluopimomide, fluotrimazole, furcarbanil, furconazole, furconazole-cis, furmecyclox, furophanate, glyodine, griseofulvin, halacrinate, Hercules 3944, hexylthiofos, ICIA0858, ipfentrifluconazole, ipflufenoquin, isopamphos, isovaledione, mandestrobin, mebenil, mecarbinzid, mefentrifluconazole, metazoxolon, methfuroxam, methylmercury dicyandiamide, metsulfovax, metyltetraprole, milneb, mucochloric anhydride, myclozolin, N-3,5-dichlorophenyl-succinimide, N-3-nitrophenylitaconimide, natamycin, *N*-ethylmercurio-4-toluenesulfonanilide, nickel bis(dimethyldithiocarbamate), OCH, phenylmercury dimethyldithiocarbamate, phenylmercury nitrate, phosdiphen, prothiocarb; prothiocarb hydrochloride, pyracarbolid, pyrapropoyne, pyridachlometyl, pyridinitril, pyrisoxazole, pyroxychlor, pyroxyfur, quinacetol; quinacetol sulfate, quinazamid, quinconazole, quinofumelin, rabenzazole, salicylanilide, SSF-109, sultropen, tecoram, thiadifluor, thicyofen, thiochlorfenphim, thiophanate, thioquinox, tioxymid, triamiphos, triarimol, triazbutil, trichlamide, triclopyricarb, triflumezopyrim, urbacid, zarilamid, and any combinations thereof.

Additionally, the compounds described herein may be combined with other pesticides, including insecticides, nematocides, miticides, arthropodicides, bactericides or combinations thereof that are compatible with the compounds of the present disclosure in the medium selected for application, and not antagonistic to the activity of the present compounds to form pesticidal mixtures and synergistic mixtures thereof. The fungicidal compounds of the present disclosure may be applied in conjunction with one or more other pesticides to control a wider variety of undesirable pests. When used in conjunction with other pesticides, the presently claimed compounds may be formulated with the other pesticide(s), tank-mixed with the other pesticide(s) or applied sequentially with the other pesticide(s). Typical insecticides include, but are not limited to: 1,2-dichloropropane, abamectin, acephate, acetamiprid, acethion, acetoprole, acrinathrin, acrylonitrile, acynonapyr, afidopyropen, alanycarb, aldicarb, aldoxycarb, aldrin, allethrin, allosamidin, allyxycarb, alpha-cypermethrin, alpha-ecdysone, alpha-endosulfan, amidithion, aminocarb, amiton, amiton oxalate, amitraz, anabasine, athidathion, azadirachtin, azamethiphos, azinphos-ethyl, azinphos-methyl, azothoate, barium hexafluorosilicate, barthrin, bendiocarb, benfuracarb, bensultap, benzpyrimoxan, beta-cyfluthrin, beta-cypermethrin, bifenthrin, bioallethrin, bioethanomethrin, biopermethrin, bistrifluron, borax, boric acid, broflanilide, bromfenvinfos, bromocyclen, bromo-DDT, bromophos, bromophos-ethyl, bufencarb, buprofezin, butacarb, butathiofos, butocarboxim, butonate, butoxycarboxim, cadusafos, calcium arsenate, calcium polysulfide, camphechlor, carbanolate, carbaryl, carbofuran, carbon disulfide, carbon tetrachloride, carbophenothion, carbosulfan, cartap, cartap hydrochloride, chlorantraniliprole, chlorbicyclen, chlordane, chlordecone, chlordimeform, chlordimeform hydrochloride, chlorethoxyfos, chlorfenapyr, chlorfenvinphos, chlorfluazuron, chlormephos, chloroform, chloropicrin, chloroprallethrin, chlorphoxim, chlorprazophos, chlorpyrifos, chlorpyrifos-methyl, chlorthiophos, chromafenozide, cinerin I, cinerin II, cinerins, cismethrin, cloethocarb, closantel, clothianidin, copper acetoarsenite, copper arsenate, copper naphthenate, copper oleate, coumaphos, coumithoate, crotamiton, crotoxyphos, crufomate, cryolite, cyanofenphos, cyanophos, cyanthoate, cyantraniliprole, cyclaniliprole, cyclethrin, cycloprothrin, cyfluthrin, cyhalodiamide, cyhalothrin, cypermethrin, cyphenothrin, cyromazine, cythioate, DDT, decarbofuran, deltamethrin, demephion, demephion-O, demephion-S, demeton, demeton-methyl, demeton-O, demeton-O-methyl, demeton-S, demeton-S-methyl, demeton-S-methylsulphon, diafenthiuron, dialifos, diatomaceous earth, diazinon, dicapthon, dichlofenthion, dichlorvos, dicloromezotiaz, dicresyl, dicrotophos, dicyclanil, dieldrin, diflubenzuron, dilor, dimefluthrin, dimefox, dimetan, dimethoate, dimethrin, dimethylvinphos, dimetilan, dinex, dinex-diclexine, dinoprop, dinosam, dinotefuran, diofenolan, dioxabenzofos, dioxacarb, dioxathion, disulfoton, dithicrofos, d-limonene, DNOC, DNOC-ammonium, DNOC-potassium, DNOC-sodium, doramectin, ecdysterone, emamectin, emamectin benzoate, EMPC, empenthrin, endosulfan, endothion, endrin, EPN, epofenonane, eprinomectin, epsilon-metofluthrin, epsilon-momfluorothrin, esdepalléthrine, esfenvalerate, etaphos, ethiofencarb, ethion, ethiprole, ethoate-methyl, ethoprophos, ethyl formate, ethyl-DDD, ethylene dibromide, ethylene dichloride, ethylene oxide, etofenprox, etrimfos, EXD, famphur, fenamiphos, fenazaflor, fenchlorphos, fenethacarb, fenfluthrin, fenitrothion, fenobucarb, fenoxacrim, fenoxycarb, fenpirithrin, fenpropathrin, fensulfothion, fenthion, fenthion-ethyl, fenvalerate, fipronil, flometoquin, flonicamid, fluazaindolizine, flubendiamide, flucofuron, flucycloxuron, flucythrinate, fluensulfone, flufenerim, flufenoxuron, flufenprox, flufiprole, fluhexafon, flupyradifurone, flupyrimin, fluvalinate, fluxametamide, fonofos, formetanate, formetanate hydrochloride, formothion, formparanate, formparanate hydrochloride, fosmethilan, fospirate, fosthietan, furathiocarb, furethrin, gamma-cyhalothrin, gamma-HCH, halfenprox, halofenozide, HCH, HEOD, heptachlor, heptafluthrin, heptenophos, heterophos, hexaflumuron, HHDN, hydramethylnon, hydrogen cyanide, hydroprene, hyquincarb, imidacloprid, imiprothrin, indoxacarb, iodomethane, IPSP, isazofos, isobenzan, isocarbophos, isocycloseram, isodrin, isofenphos, isofenphos-methyl, isoprocarb, isoprothiolane, isothioate, isoxathion, ivermectin, jasmolin I, jasmolin II, jodfenphos, juvenile hormone I, juvenile hormone II, juvenile hormone III, kappa-bifenthrin, kappa-tefluthrin, kelevan, kinoprene, lambda-cyhalothrin, lead arsenate, lepimectin, leptophos, lindane, lirimfos, lufenuron, lythidathion, malathion, malonoben, mazidox, mecarbam, mecarphon, menazon, meperfluthrin, mephosfolan, mercurous chloride, mesulfenfos, metaflumizone, methacrifos, methamidophos, methidathion, methiocarb, methocrotophos, methomyl, methoprene, methoxychlor, methoxyfenozide, methyl bromide, methyl isothiocyanate, methylchloroform, methylene chloride, metofluthrin, metolcarb, metoxadiazone, mevinphos, mexacarbate, milbemectin, milbemycin oxime, mipafox, mirex, molosultap, momfluorothrin, monocrotophos, monomehypo, monosultap, morphothion, moxidectin, naftalofos, naled, naphthalene, nicotine, nifluridide, nitenpyram, nithiazine, nitrilacarb, novaluron, noviflumuron, omethoate, oxamyl, oxazosulfyl, oxydemeton-methyl, oxydeprofos, oxydisulfoton, para-dichlorobenzene, parathion, parathion-methyl, penfluron, pentachlorophenol, permethrin, phenkapton, phenothrin, phenthoate, phorate, phosalone, phosfolan, phosmet, phosnichlor, phosphamidon, phosphine, phoxim, phoxim-methyl, pirimetaphos, pirimicarb, pirimiphos-ethyl, pirimiphos-methyl, potassium arsenite, potassium thiocyanate, pp'-DDT, prallethrin, precocene I, precocssene II, precocene III, primidophos, profenofos, profluralin, promacyl, promecarb, propaphos, propetamphos, propoxur, prothidathion, prothiofos, prothoate, protrifenbute, pyflubumide, pyraclofos, pyrafluprole, pyrazophos, pyresmethrin, pyrethrin I, pyrethrin II, pyrethrins, pyridaben, pyridalyl, pyridaphenthion, pyrifluquinazon, pyrimidifen, pyriminostrobin, pyrimitate, pyriprole, pyriproxyfen, quassia, quinalphos, quinalphos-methyl, quinothion, rafoxanide, resmethrin, rotenone, ryania, sabadilla, schradan, selamectin, silafluofen, silica gel, sodium arsenite, sodium fluoride, sodium hexafluorosilicate, sodium thiocyanate, sophamide, spinetoram, spinosad, spiromesifen, spiropidion, spirotetramat, sulcofuron, sulcofuron-sodium, sulfluramid, sulfotep, sulfoxaflor, sulfuryl fluoride, sulprofos, tau-fluvalinate, tazimcarb, TDE, tebufenozide, tebufenpyrad, tebupirimfos, teflubenzuron, tefluthrin, temephos, TEPP, terallethrin, terbufos, tetrachlorantraniliprole, tetrachloroethane, tetrachlorvinphos, tetramethrin, tetramethylfluthrin, tetraniliprole, theta-cypermethrin, thiacloprid, thiamethoxam, thicrofos, thiocarboxime, thiocyclam, thiocyclam oxalate, thiodicarb, thiofanox, thiometon, thiosultap, thiosultap-disodium, thiosultap-monosodium, thuringiensin, tioxazafen, tolfenpyrad, tralomethrin, transfluthrin, transpermethrin, triarathene, triazamate, triazophos, trichlorfon, trichlormetaphos-3, trichloronat, trifenofos, triflumezopyrim, triflumuron, trimethacarb, triprene, tyclopyrazoflor, vamidothion, vaniliprole, XMC, xylylcarb, zeta-cypermethrin, zolaprofos, and any combinations thereof.

Additionally, the compounds described herein may be combined with herbicides that are compatible with the compounds of the present disclosure in the medium selected for application, and not antagonistic to the activity of the present compounds to form pesticidal mixtures and synergistic mixtures thereof. The fungicidal compounds of the present disclosure may be applied in conjunction with one or more herbicides to control a wide variety of undesirable plants. When used in conjunction with herbicides, the presently claimed compounds may be formulated with the herbicide(s), tank-mixed with the herbicide(s) or applied sequentially with the herbicide(s). Typical herbicides include, but are not limited to: 4-CPA; 4-CPB; 4-CPP; 2,4-D; 3,4-DA; 2,4-DB; 3,4-DB; 2,4-DEB; 2,4-DEP; 3,4-DP; 2,3,6-TBA; 2,4,5-T; 2,4,5-TB; acetochlor, acifluorfen, aclonifen, acrolein, alachlor, allidochlor, alloxydim, allyl alcohol, alorac, ametridione, ametryn, amibuzin, amicarbazone, amidosulfuron, aminocyclopyrachlor, aminopyralid, amiprofos-methyl, amitrole, ammonium sulfamate, anilofos, anisuron, asulam, atraton, atrazine, azafenidin, azimsulfuron, aziprotryne, barban, BCPC, beflubutamid, beflubutamid-M, benazolin, bencarbazone, benfluralin, benfuresate, bensulfuron, bensulide, bentazone, benzadox, benzfendizone, benzipram, benzobicyclon, benzofenap, benzofluor, benzoylprop, benzthiazuron, bicyclopyrone, bifenox, bilanafos, bispyribac, bixlozone, borax, bromacil, bromobonil, bromobutide, bromofenoxim, bromoxynil, brompyrazon, butachlor, butafenacil, butamifos, butenachlor, buthidazole, buthiuron, butralin, butroxydim, buturon, butylate, cacodylic acid, cafenstrole, calcium chlorate, calcium cyanamide, cambendichlor, carbasulam, carbetamide, carboxazole chlorprocarb, carfentrazone, CDEA, CEPC, chlomethoxyfen, chloramben, chloranocryl, chlorazifop, chlorazine, chlorbromuron, chlorbufam, chloreturon, chlorfenac, chlorfenprop, chlorflurazole, chlorflurenol, chloridazon, chlorimuron, chlornitrofen, chloropon, chlorotoluron, chloroxuron, chloroxynil, chlorpropham, chlorsulfuron, chlorthal, chlorthiamid, cinidon-ethyl, cinmethylin, cinosulfuron, cisanilide, clacyfos, clethodim, cliodinate, clodinafop, clofop, clomazone, clomeprop, cloprop, cloproxydim, clopyralid, cloransulam, CMA, copper sulfate, CPMF, CPPC, credazine, cresol, cumyluron, cyanatryn, cyanazine, cycloate, cyclopyranil, cyclopyrimorate, cyclosulfamuron, cycloxydim, cycluron, cyhalofop, cyperquat, cyprazine, cyprazole, cypromid, daimuron, dalapon, dazomet, delachlor, desmedipham, desmetryn, di-allate, dicamba, dichlobenil, dichloralurea, dichlormate, dichlorprop, dichlorprop-P, diclofop, diclosulam, diethamquat, diethatyl, difenopenten, difenoxuron, difenzoquat, diflufenican, diflufenzopyr, dimefuron, dimepiperate, dimethachlor, dimethametryn, dimethenamid, dimethenamid-P, dimexano, dimidazon, dinitramine, dinofenate, dinoprop, dinosam, dinoseb, dinoterb, diphenamid, dipropetryn, diquat, disul, dithiopyr, diuron, DMPA, DNOC, DSMA, EBEP, eglinazine, endothal, epronaz, EPTC, erbon, esprocarb, ethalfluralin, ethametsulfuron, ethidimuron, ethiolate, ethofumesate, ethoxyfen, ethoxysulfuron, etinofen, etnipromid, etobenzanid, EXD, fenasulam, fenoprop, fenoxaprop, fenoxaprop-P, fenoxasulfone, fenquinotrione, fenteracol, fenthiaprop, fentrazamide, fenuron, ferrous sulfate, flamprop, flamprop-M, flazasulfuron, florasulam, florpyrauxifen, fluazifop, fluazifop-P, fluazolate, flucarbazone, flucetosulfuron, fluchloralin, flufenacet, flufenican, flufenpyr, flumetsulam, flumezin, flumiclorac, flumioxazin, flumipropyn, fluometuron, fluorodifen, fluoroglycofen, fluoromidine, fluoronitrofen, fluothiuron, flupoxam, flupropacil, flupropanate, flupyrsulfuron, fluridone, flurochloridone, fluroxypyr, flurtamone, fluthiacet, fomesafen, foramsulfuron, fosamine, furyloxyfen, glufosinate, glufosinate-P, glyphosate, halauxifen, halosafen, halosulfuron, haloxydine, haloxyfop, haloxyfop-P, hexachloroacetone, hexaflurate, hexazinone, imazamethabenz, imazamox, imazapic, imazapyr, imazaquin, imazethapyr, imazosulfuron, indanofan, indaziflam, iodobonil, iodomethane, iodosulfuron, iofensulfuron, ioxynil, ipazine, ipfencarbazone, iprymidam, isocarbamid, isocil, isomethiozin, isonoruron, isopolinate, isopropalin, isoproturon, isouron, isoxaben, isoxachlortole, isoxaflutole, isoxapyrifop, karbutilate, ketospiradox, lancotrione, lactofen, lenacil, linuron, MAA, MAMA, MCPA, MCPA-thioethyl, MCPB, mecoprop, mecoprop-P, medinoterb, mefenacet, mefluidide, mesoprazine, mesosulfuron, mesotrione, metam, metamifop, metamitron, metazachlor, metazosulfuron, metflurazon, methabenzthiazuron, methalpropalin, methazole, methiobencarb, methiozolin, methiuron, methometon, methoprotryne, methyl bromide, methyl isothiocyanate, methyldymron, metobenzuron, metobromuron, metolachlor, metosulam, metoxuron, metribuzin, metsulfuron, molinate, monalide, monisouron, monochloroacetic acid, monolinuron, monuron, morfamquat, MSMA, naproanilide, napropamide, napropamide-M, naptalam, neburon, nicosulfuron, nipyraclofen, nitralin, nitrofen, nitrofluorfen, norflurazon, noruron, OCH, orbencarb, ortho-dichlorobenzene, orthosulfamuron, oryzalin, oxadiargyl, oxadiazon, oxapyrazon, oxasulfuron, oxaziclomefone, oxyfluorfen, parafluron, paraquat, pebulate, pelargonic acid, pendimethalin, penoxsulam, pentachlorophenol, pentanochlor, pentoxazone, perfluidone, pethoxamid, phenisopham, phenmedipham, phenmedipham-ethyl, phenobenzuron, phenylmercury acetate, picloram, picolinafen, pinoxaden, piperophos, potassium arsenite, potassium azide, potassium cyanate, pretilachlor, primisulfuron, procyazine, prodiamine, profluazol, profluralin, profoxydim, proglinazine, prometon, prometryn, propachlor, propanil, propaquizafop, propazine, propham, propisochlor, propoxycarbazone, propyrisulfuron, propyzamide, prosulfalin, prosulfocarb, prosulfuron, proxan, prynachlor, pydanon, pyraclonil, pyraflufen, pyrasulfotole, pyrazolynate, pyrazosulfuron, pyrazoxyfen, pyribenzoxim, pyributicarb, pyriclor, pyridafol, pyridate, pyriftalid, pyriminobac, pyrimisulfan, pyrithiobac, pyroxasulfone, pyroxsulam, quinclorac, quinmerac, quinoclamine, quinonamid, quizalofop, quizalofop-P, rhodethanil, rimsulfuron, saflufenacil, S-metolachlor, sebuthylazine, secbumeton, sethoxydim, siduron, simazine, simeton, simetryn, SMA, sodium arsenite, sodium azide, sodium chlorate, sulcotrione, sulfallate, sulfentrazone, sulfometuron, sulfosulfuron, sulfuric acid, sulglycapin, swep, TCA, tebutam, tebuthiuron, tefuryltrione, tembotrione, tepraloxydim, terbacil, terbucarb, terbuchlor, terbumeton, terbuthylazine, terbutryn, tetrafluron, thenylchlor, thiazafluron, thiazopyr, thidiazimin, thidiazuron, thiencarbazone-methyl, thifensulfuron, thiobencarb, tiafenacil, tiocarbazil, tioclorim, tolpyralate, topramezone, tralkoxydim, triafamone, tri-allate, triasulfuron, triaziflam, tribenuron, tricamba, triclopyr, tridiphane, trietazine, trifloxysulfuron, trifludimoxazin, trifluralin, triflusulfuron, trifop, trifopsime, trihydroxytriazine, trimeturon, tripropindan, tritac, tritosulfuron, vernolate, and xylachlor.

In another embodiment of this invention, Formula 1 may be used in combination (such as, in a compositional mixture, or a simultaneous or sequential application) with one or more active ingredients such as those described above.

In another embodiment of this invention, Formula 1 may be used in combination (such as, in a compositional mixture, or a simultaneous or sequential application) with one or more active ingredients each having a mode of action (MoA) that is the same as, similar to, or, preferably, different from, the MoA of Formula 1.

In another embodiment, Formula 1 may be used in combination (such as, in a compositional mixture, or a simultaneous or sequential application) with one or more molecules having acaricidal, algicidal, avicidal, bactericidal, fungicidal, herbicidal, insecticidal, molluscicidal, nematicidal, rodenticidal, and/or virucidal properties.

In another embodiment, Formula 1 may be used in combination (such as, in a compositional mixture, or a simultaneous or sequential application) with one or more molecules that are antifeedants, bird repellents, chemosterilants, herbicide safeners, insect attractants, insect repellents, mammal repellents, mating disrupters, plant activators, plant growth regulators, plant health stimulators or promoters, nitrification inhibitors, and/or synergists.

In another embodiment, Formula 1 may also be used in combination (such as in a compositional mixture, or a simultaneous or sequential application) with one or more biopesticides.

In another embodiment, in a pesticidal composition combinations of Formula 1 and an active ingredient may be used in a wide variety of weight ratios. For example, in a two-component mixture, the weight ratio of Formula 1 to an active ingredient, the weight ratios in Table 1 may be used. However, in general, weight ratios less than about 10:1 to about 1:10 are preferred.

| **TABLE 1** |
|---|
| **Weight Ratios Formula One : active ingredient** |
| 100:1 to 1:100 |
| 50:1 to 1:50 |
| 20:1 to 1:20 |
| 10:1 to 1:10 |
| 5:1 to 1:5 |
| 3:1 to 1:3 |
| 2:1 to 1:2 |
| 1:1 |

Weight ratios of a molecule of Formula 1 to an active ingredient may also be depicted as X:Y; wherein X is the parts by weight of Formula 1 and Y is the parts by weight of the active ingredient. The numerical range of the parts by weight for X is 0 < X ≤ 100 and the parts by weight for Y is 0 < Y ≤ 100 and is shown graphically in Table 2. By way of non-limiting example, the weight ratio of Formula 1 to an active ingredient may be 20:1.

| **TABLE 2** | | | | | | | | | | |
|---|---|---|---|---|---|---|---|---|---|---|
| | **100** | ***X,Y*** | | ***X,Y*** | | | ***X,Y*** | | | |
| | **50** | ***X,Y*** | ***X,Y*** | ***X,Y*** | | | ***X,Y*** | ***X,Y*** | | |
| | **20** | ***X,Y*** | | ***X,Y*** | ***X,Y*** | | ***X,Y*** | | ***X,Y*** | |
| | **15** | ***X,Y*** | ***X,Y*** | | | | | ***X,Y*** | ***X,Y*** | ***X,Y*** |
| active ingredient | **10** | ***X,Y*** | | ***X,Y*** | | | | | | |
| | **5** | ***X,Y*** | ***X,Y*** | ***X,Y*** | | | | ***X,Y*** | | |
| *(Y) Parts by weight* | | | | | | | | | | |
| | **3** | ***X,Y*** | ***X,Y*** | | ***X,Y*** | ***X,Y*** | | ***X,Y*** | ***X,Y*** | ***X,Y*** |
| | **2** | ***X,Y*** | | ***X,Y*** | ***X,Y*** | | ***X,Y*** | | ***X,Y*** | |
| | **1** | ***X,Y*** | ***X,Y*** | ***X,Y*** | ***X,Y*** | ***X,Y*** | ***X,Y*** | ***X,Y*** | ***X,Y*** | ***X,Y*** |
| | | **1** | **2** | **3** | **5** | **10** | **15** | **20** | **50** | **100** |
| | Formula One, also known as **F1,** *(X) Parts by weight* | | | | | | | | | |

Ranges of weight ratios of Formula 1 to an active ingredient may be depicted as*X₁*:*Y₁* to *X₂*:*Y₂,* wherein *X* and *Y* are defined as above.

In one embodiment, the range of weight ratios may be *X₁*:*Y₁* to *X₂*:*Y₂,* wherein *X₁ > Y₁* and *X₂ < Y₂.* By way of non-limiting example, the range of a weight ratio of Formula 1 to an active ingredient may be between 3:1 and 1:3, inclusive of the endpoints.

In another embodiment, the range of weight ratios may be *X₁*:*Y₁* to *X₂*:*Y₂,* wherein *X₁ > Y₁* and *X₂ > Y₂.* By way of non-limiting example, the range of weight ratio of Formula 1 to an active ingredient may be between 15:1 and 3:1, inclusive of the endpoints.

In another embodiment, the range of weight ratios may be *X₁*:*Y₁* to *X₂*:*Y₂,* wherein *X₁ < Y₁* and *X₂ < Y₂.* By way of non-limiting example, the range of weight ratios of Formula 1 to an active ingredient may be between about 1:3 and about 1:20, inclusive of the endpoints.

Another embodiment of the present disclosure is a method for the control or prevention of fungal attack. This method comprises applying to the soil, plant, roots, foliage, or locus of the fungus, or to a locus in which the infestation is to be prevented (for example applying to cereal or grape plants), a fungicidally effective amount of one or more of the compounds of Formula I. The compounds are suitable for treatment of various plants at fungicidal levels, while exhibiting low phytotoxicity. The compounds may be useful both in a protectant and/or an eradicant fashion.

The compounds have been found to have significant fungicidal effect particularly for agricultural use. Many of the compounds are particularly effective for use with agricultural crops and horticultural plants.

It will be understood by those skilled in the art that the efficacy of the compound for the foregoing fungi establishes the general utility of the compounds as fungicides.

The compounds have broad ranges of activity against fungal pathogens. Exemplary pathogens may include, but are not limited to, causing agent of wheat leaf blotch (*Zymoseptoria tritici),* wheat brown rust *(Puccinia triticina*), wheat stripe rust *(Puccinia striiformis*), scab of apple (*Venturia inaequalis),* powdery mildew of grapevine (*Uncinula necator),* barley scald *(Rhynchosporium commune),* blast of rice *(Magnaporthe grisea),* rust of soybean (*Phakopsora pachyrhizi),* glume blotch of wheat (*Parastagonospora nodorum),* powdery mildew of wheat (*Blumeria graminis f. sp.tritici),* powdery mildew of barley (*Blumeria graminis f. sp. hordei),* powdery mildew of cucurbits *(Erysiphe cichoracearum),* anthracnose of cucurbits (*Glomerella lagenarium*), leaf spot of beet *(Cercospora beticola*), early blight of tomato *(Alternaria solani),* spot blotch of barley *(Cochliobolus sativus),* and net blotch of barley (*Pyrenophora teres).* The exact amount of the active material to be applied is dependent not only on the specific active material being applied, but also on the particular action desired, the fungal species to be controlled, and the stage of growth thereof, as well as the part of the plant or other product to be contacted with the compound. Thus, all the compounds, and formulations containing the same, may not be equally effective at similar concentrations or against the same fungal species.

The compounds are effective in use with plants in a disease-inhibiting and phytologically acceptable amount. The term "disease-inhibiting and phytologically acceptable amount" refers to an amount of a compound that kills or inhibits the plant disease for which control is desired, but is not significantly toxic to the plant. This amount will generally be from about 0.1 to about 1000 ppm (parts per million), with 1 to 500 ppm being preferred. The exact concentration of compound required varies with the fungal disease to be controlled, the type of formulation employed, the method of application, the particular plant species, climate conditions, and the like. A suitable application rate is typically in the range from about 0.10 to about 4 pounds/acre (about 0.01 to 0.45 grams per square meter, g/m²).

Any range or desired value given herein may be extended or altered without losing the effects sought, as is apparent to the skilled person for an understanding of the teachings herein.

The compounds of Formula I may be made using well-known chemical procedures. Intermediates not specifically mentioned in this disclosure are either commercially available, may be made by routes disclosed in the chemical literature, or may be readily synthesized from commercial starting materials utilizing standard procedures.

### GENERAL SCHEMES

The following schemes illustrate approaches to generating aryl amidine compounds of Formula (I). The following descriptions and examples are provided for illustrative purposes and should not be construed as limiting in terms of substituents or substitution patterns.

Compounds of Formula 1.4, wherein R², R³, R⁴ and R⁵ are as originally defined, can be prepared by the method shown in **Scheme 1, steps *a-c.*** Compounds of Formula 1.2, wherein R², R⁴ and R⁵ are as originally defined, can be prepared by the method shown in **Scheme 1, step *a*.** The compound of Formula 1.1, wherein R², R⁴ and R⁵ are as originally defined, can be treated with sodium periodate, in the presence of iodine (I₂), in a solvent, such as N,N-dimethylformamide (DMF), at a temperature of about 23 °C to 50 °C to afford compounds of Formula 1.2, wherein R², R⁴ and R⁵ are as originally defined, as shown in ***a*.** Compounds of Formula 1.3, wherein R², R³, R⁴ and R⁵ are as originally defined, can be prepared by the method shown in **Scheme 1, step *b*.** The compound of Formula 1.2, wherein R², R⁴ and R⁵ are as originally defined, can be treated with a catalyst, such as [1,1'-bis(diphenylphosphino)ferrocene]dichloropalladium(II) complex with dichloromethane (PdCl₂(dppf)DCM), and a boronic anhydride, such as B₃O₃R³₃, wherein R³ is as originally defined, in the presence of a base, such as cesium carbonate (Cs₂CO₃), in a solvent, such as 1,4-dioxane, at a temperature of about 23 °C to 120 °C under microwave radiation to afford compounds of Formula 1.3, wherein R², R³, R⁴ and R⁵ are as originally defined, as shown in b. Compounds of Formula 1.4, wherein R², R³, R⁴ and R⁵ are as originally defined, can be prepared by the method shown in **Scheme 1, step *c*.** The compound of Formula 1.3, wherein R², R³, R⁴ and R⁵ are as originally defined, can be treated with a base, such as lithium hydroxide (LiOH) in a solvent mixture, such as 3:2:1 tetrahydrofurane (THF):methanol (MeOH):water (H₂O), at a temperature of about 23 °C to 70 °C reflux to afford compounds of Formula 1.4, wherein R², R³, R⁴ and R⁵ are as originally defined, as shown in c.

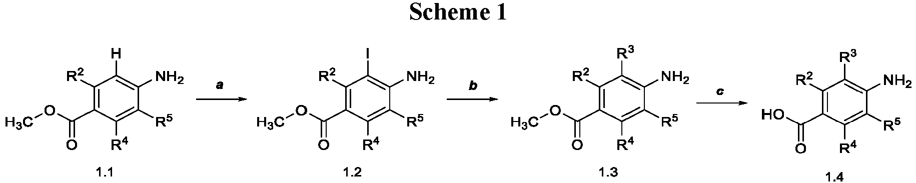

Alternatively, compounds of Formula 1.4, wherein R², R³, R⁴ and R⁵ are as originally defined, can be prepared by the method shown in **Scheme 2, steps *d-f.*** Compounds of Formula 2.2, wherein R²_{,} R⁴ and R⁵ are as originally defined, can be prepared by the method shown in **Scheme 2, step** d. The compound of Formula 2.1, wherein R²_{,} R⁴ and R⁵ are as originally defined, can be treated with a halogenating reagent, such as N-bromosuccinimide (NBS), in a solvent, such as *N,N-*dimethylformamide (DMF), at a temperature of about 0 °C to 23 °C to afford compounds of Formula 2.2, wherein R²_{,} R⁴ and R⁵ are as originally defined, as shown in *d*. Compounds of Formula 2.3, wherein R², R³, R⁴ and R⁵ are as originally defined, can be prepared by the method shown in **Scheme 2, step** *e.* The compound of Formula 2.2, wherein R²_{,} R⁴ and R⁵ are as originally defined, can be treated with a catalyst, such as (2-Dicyclohexylphosphino-2',4',6'-triisopropyl-1,1'-biphenyl)[2-(2'-amino-1,1'-biphenyl)]palladium(II) methanesulfonate (XPhos-Pd-G3), and a boronic anhydride, such as B₃O₃R³₃, wherein R³ is as originally defined, in the presence of a base, such as potassium phosphate tribasic (K₃PO₄), in a solvent mixture, such as 10:1 1,4-dioxane:water, at a temperature of about 23 °C to 100 °C to afford compounds of Formula 2.3, wherein R², R³, R⁴ and R⁵ are as originally defined, as shown in *e*. Compounds of Formula 1.4, wherein R², R³, R⁴ and R⁵ are as originally defined, can be prepared by the method shown in **Scheme 2, step f.** The compound of Formula 2.3, wherein R², R³, R⁴ and R⁵ are as originally defined, can be treated with a base, such as potassium hydroxide (KOH), in a solvent, such as water, at a temperature of about 23 °C to 60 °C to afford compounds of Formula 1.4, wherein R², R³, R⁴ and R⁵ are as originally defined, as shown in *f*.

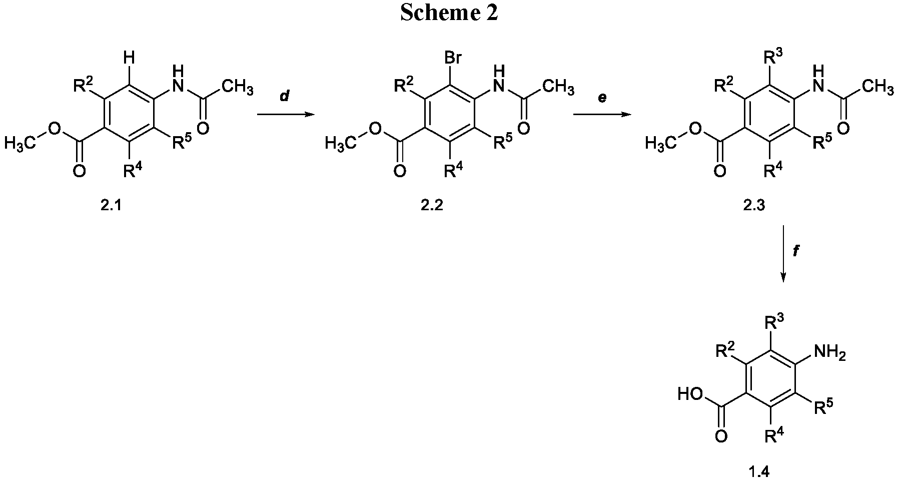

Alternatively, compounds of Formula 1.4, wherein R², R³, R⁴ and R⁵ are as originally defined, can be prepared by the method shown in Scheme 3, steps g-n. Compounds of Formula 3.2, wherein R², R³, R⁴ and R⁵ are as originally defined, can be prepared by the method shown in Scheme 3, step g. The compound of Formula 3.1, wherein R², R³, R⁴ and R⁵ are as originally defined, can be treated with hydrogen bromide (HBr), in the presence of sodium nitrite (NaNO₂), in a solvent, such as acetic acid, at a temperature of about 23 °C to 85 °C to afford compounds of Formula 3.2, wherein R², R³, R⁴ and R⁵ are as originally defined, as shown in g. Compounds of Formula 3.3, wherein R², R³, R⁴ and R⁵ are as originally defined, can be prepared by the method shown in **Scheme 3, step *h*.** The compound of Formula 3.2, wherein R², R³, R⁴ and R⁵ are as originally defined, can be treated with a metal catalyst, such as iron (Fe⁰), in the presence of an ammonium salt such as ammonium chloride (NH₄Cl), in a solvent mixture, such as 1:1 ethanol (EtOH):H₂O, at a temperature of about 23 °C to 70 °C to afford compounds of Formula 3.3, wherein R², R³, R⁴ and R⁵ are as originally defined, as shown in ***h*.** Alternatively, compounds of Formula 3.3, wherein R², R³, R⁴ and R⁵ are as originally defined, can be prepared by the method shown in **Scheme 3, step *i*.** The compound of Formula 3.4, wherein R², R³, R⁴ and R⁵ are as originally defined, can be treated with a halogenating reagent, such as *N*-bromosuccinimide (NBS), in a solvent, such as N,N-dimethylformamide (DMF), at a temperature of about 0 °C to 23 °C to afford compounds of Formula 3.3, wherein R², R³, R⁴ and R⁵ are as originally defined, as shown in ***i*.** Compounds of Formula 3.5, wherein R², R³, R⁴ and R⁵ are as originally defined, can be prepared by the method shown in **Scheme 3, stepj.** The compound of Formula 3.3, wherein R², R³, R⁴ and R⁵ are as originally defined, can be treated with a metal cyanide, such as CuCN, in a solvent, such as N-methyl-2-pyrrolidone (NMP), at a temperature of about 23 °C to 180 °C under microwave irradiation to afford compounds of Formula 3.5, wherein R², R³, R⁴ and R⁵ are as originally defined, as shown in ***j***. Alternatively, compounds of Formula 3.5, wherein R², R³, R⁴ and R⁵ are as originally defined, can be prepared by the method shown in **Scheme 3, step *k*.** The compound of Formula 3.3, wherein R², R³, R⁴ and R⁵ are as originally defined, can be treated with a metal cyanide, such as zinc(II) cyanide (Zn(CN)₂), in the presence of a metal catalyst, such as tetrakis(triphenylphosphine)-palladium(0) (Pd(PPh₃)₄), in a solvent, such as DMF, at a temperature of about 23 °C to 120 °C to afford compounds of Formula 3.4, wherein R², R³, R⁴ and R⁵ are as originally defined, as shown in *k*. Compounds of Formula 1.4, wherein R², R³, R⁴ and R⁵ are as originally defined, can be prepared by the method shown in **Scheme 3, step *l*.** The compound of Formula 3.5, wherein R², R³, R⁴ and R⁵ are as originally defined, can be treated with a base, such as potassium hydroxide (KOH), in a solvent, such as H₂O, at a temperature of about 23 °C to 120 °C to afford compounds of Formula 1.4, wherein R², R³, R⁴ and R⁵ are as originally defined, as shown in ***l*.** Alternatively, compounds of Formula 3.6, wherein R², R³, R⁴ and R⁵ are as originally defined, can be prepared by the method shown in **Scheme 3, step *m.*** The compound of Formula 3.3, wherein R², R³, R⁴ and R⁵ are as originally defined, can be treated with carbon monoxide (CO) gas in the presence of a metal catalyst, such as palladium(II) acetate, in the presence of a ligand, such as 1,4-bis(diphenylphosphanyl)butane, with a base, such as triethylamine (TEA), in a solvent, such as methanol, at a pressure of about 400 psi and a temperature of about 23 °C to 125 °C to afford compounds of Formula 3.6, wherein R², R³, R⁴ and R⁵ are as originally defined, as shown in ***m.*** Compounds of Formula 1.4, wherein R², R³, R⁴ and R⁵ are as originally defined, can be prepared by the method shown in **Scheme 3, step *n.*** The compound of Formula 3.6, wherein R², R³, R⁴ and R⁵ are as originally defined, can be treated with a base, such a lithium hydroxide (LiOH), in a solvent mixture, such as 3:2:1 THF:MeOH:water, at a temperature of about 23 °C to 125 °C to afford compounds of Formula 1.4, wherein R², R³, R⁴ and R⁵ are as originally defined, as shown in ***n.***

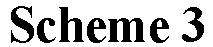

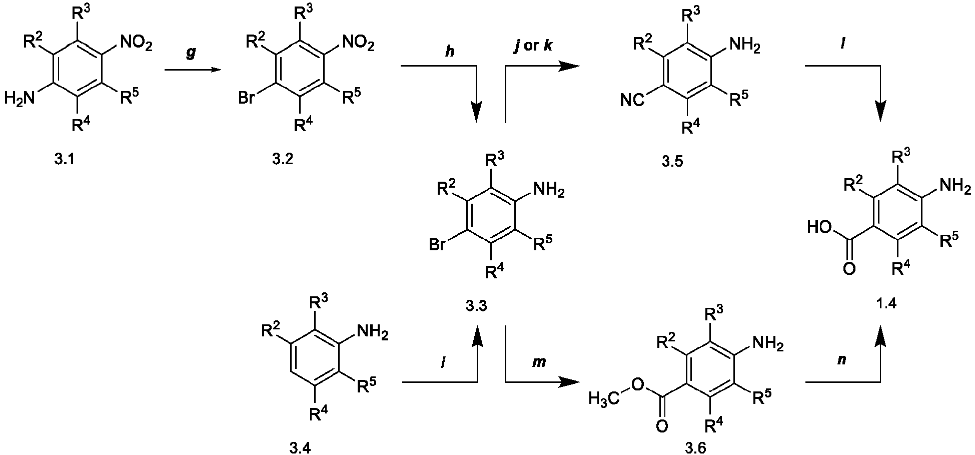

Compounds of Formula 4.1, wherein R¹, R², R³, R⁴ and R⁵ are as originally defined, can be prepared by the method shown in **Scheme 4, step *o.*** The compound of Formula 1.4, wherein R², R³, R⁴ and R⁵ are as originally defined, can be treated with an alcohol, such as R¹-OH, wherein R¹ is as originally defined, in the presence of a peptide coupling regent, such as 1-ethyl-3-(3-dimethylaminopropyl)carbodiimide (EDCI), *N,N*'-dicyclohexylcarbodiimide (DCC) or benzotriazol-1-yl-oxytripyrrolidinophosphonium hexafluorophosphate (PyBOP), and a catalyst, such as dimethylamino pyridine (DMAP) or *N*-ethyl-*N*-isopropylpropan-2-amine (DIPEA), in a solvent, such as dichloromethane (DCM), at a temperature of about 0 °C to ambient temperature, to afford compounds of Formula 4.1, wherein R¹, R², R³, R⁴ and R⁵ are as originally defined, as shown in ***o*.** Alternatively, compounds of Formula 4.1, wherein R¹, R², R³, R⁴ and R⁵ are as originally defined, can be prepared by the method shown in **Scheme 4, step *p*.** The compound of Formula 1.4, wherein R², R³, R⁴ and R⁵ are as originally defined, can be treated with an alkylating agent, such as R¹-Br, wherein R¹ is as originally defined, in the presence of a base, such as potassium carbonate (K₂CO₃), in a solvent, such as DMF, at a temperature of about 23 °C, to afford compounds of Formula 4.1, wherein R¹, R², R³, R⁴ and R⁵ are as originally defined, as shown in***p***.

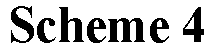

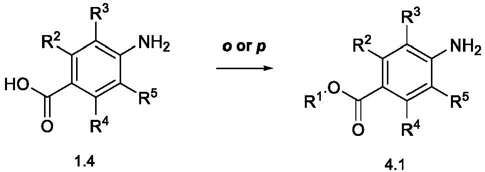

Compounds of Formula 5.2, wherein R¹, R², R³, R⁴, R⁵, R⁷ and R⁸ are as originally defined, can be prepared by the method shown in **Scheme 5, step *q*.** The compound of Formula 4.1, wherein R¹, R², R³, R⁴ and R⁵ are as originally defined, can be treated with an amine, such as a compound of Formula 5.1, wherein R⁷ and R⁸ are as originally defined, in a solvent, such as toluene, at a temperature of about 23 °C to 90 °C to afford compounds of Formula 5.2, wherein R¹, R², R³, R⁴, R⁵, R⁷ and R⁸ are as originally defined, as shown in ***q.***

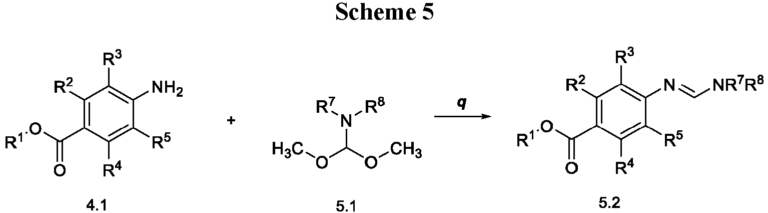

Alternatively, compounds of Formula 5.2, wherein R¹, R², R³, R⁴, R⁵, R⁷ and R⁸ are as originally defined, can be prepared by the method shown in **Scheme 6, steps** r-s. Compounds of Formula 6.1, wherein R¹, R², R³, R⁴ and R⁵ are as originally defined, and Z is an alkyl group, can be prepared by the method shown in **Scheme** 6, **step** r. The compound of Formula 4.1, wherein R¹, R², R³, R⁴ and R⁵ are as originally defined, can be treated with a trialkyl orthoformate (CH(OZ)₃), wherein Z is an alkyl group, such as trimethyl orthoformate or triethyl orthoformate, in the presence of an acid catalyst, such as *p*-toluenesulfonic acid monohydrate (pTsOH-H₂O), at a temperature of about reflux (~100 °C or ~140 °C, respectively) to afford compounds of Formula 6.1, wherein R¹, R², R³, R⁴ and R⁵ are as originally defined, and Z is an alkyl group, as shown in ***r*.** Compounds of Formula 5.2, wherein R¹, R², R³, R⁴, R⁵, R⁷ and R⁸ are as originally defined, can be prepared by the method shown in **Scheme 6, step *s*.** The compound of Formula 6.1, wherein R¹, R², R³, R⁴ and R⁵ are as originally defined, and Z is an alkyl group, can be treated with an amine, such as a compound of Formula 6.2, wherein R⁷ and R⁸ are as originally defined, in a solvent, such as DCM, at a temperature of about 23 °C to 40 °C to afford compounds of Formula 5.2, wherein R¹, R², R³, R⁴, R⁵, R⁷ and R⁸ are as originally defined, as shown in s.

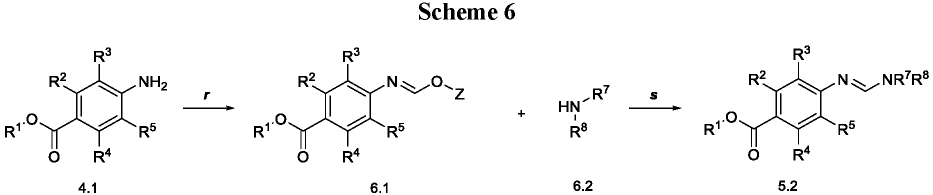

Compounds of Formula 7.2, wherein R¹, R², R³, R⁴, R⁵, R⁶, R⁷ and R⁸ are as originally defined, can be prepared by the method shown in **Scheme 7, step** t. The compound of Formula 4.1, wherein R¹, R², R³, R⁴, and R⁵ are as previously defined, can be treated with an amide, such as a compound of Formula 7.1, wherein R⁶, R⁷ and R⁸ are as originally defined, in the presence of a dehydrating reagent, such as phosphoryl trichloride (POCl₃), in a solvent, such as toluene, at a temperature of about 23 °C to reflux (~110 °C) to afford compounds of Formula 7.2, wherein R¹, R², R³, R⁴, R⁵, R⁶, R⁷ and R⁸ are as originally defined, as shown in *t.*

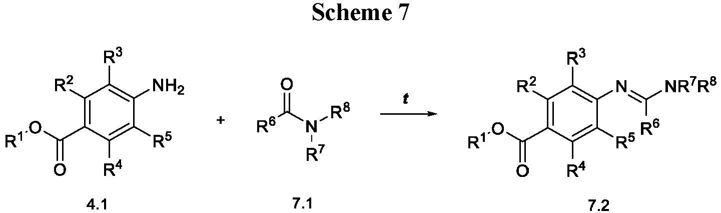

Compounds of Formula 8.2, wherein R¹, R², R³, R⁴, R⁵, R⁷ and R⁸ are as originally defined, can be prepared by the method shown in **Scheme 8, steps u-v.** Compounds of Formula 8.1, wherein R¹, R², R³, R⁴ and R⁵ are as previously defined, can be prepared by the method shown in **Scheme 8, step *u.*** The compound of Formula 4.1, wherein R¹, R², R³, R⁴ and R⁵ are as previously defined, can be treated with thiophosgene in the presence of a base, such as sodium bicarbonate (NaHCO₃), in a solvent mixture, such as 1:1 DCM:H₂O, at a temperature of about 23 °C to afford compounds of Formula 8.1, wherein R¹, R², R³, R⁴ and R⁵ are as previously defined, as shown in *u.* Compounds of Formula 8.2, wherein R¹, R², R³, R⁴, R⁵, R⁷ and R⁸ are as previously defined, can be prepared by the method shown in **Scheme 8, step** *v*. The compound of Formula 8.1, wherein R¹, R², R³, R⁴ and R⁵ are as previously defined, can be treated with an amine, such as a compound of Formula 6.2, wherein R⁷ and R⁸ are as originally defined, in a solvent, such as DCM, at a temperature of about 23 °C to afford compounds of Formula 8.2, wherein R¹, R², R³, R⁴, R⁵, R⁷ and R⁸ are as originally defined, as shown in *v.*

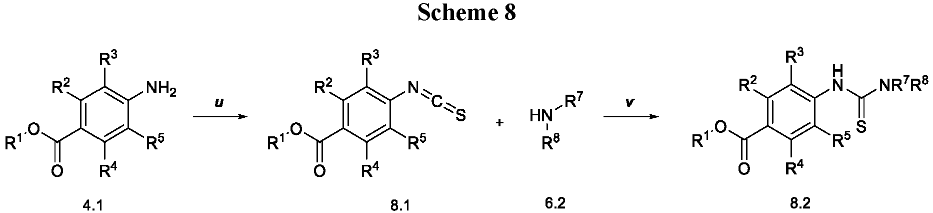

Alternatively, compounds of Formula 9.3, wherein R¹, R², R³, R⁴, R⁵, R⁷ and R⁸ are as originally defined, can be prepared by the method shown in **Scheme 9, steps** w-x. Compounds of Formula 9.1, wherein R¹, R², R³, R⁴ and R⁵ are as originally defined, can be prepared by the method shown in **Scheme 9, step w.** The compound of Formula 4.1, wherein R¹, R², R³, R⁴ and R⁵ are as originally defined, can be treated with trimethyl orthoformate in the presence of an acid catalyst, such as *p*-toluenesulfonic acid monohydrate (pTsOH-H₂O), at a temperature of about reflux (~100 °C) to afford compounds of Formula 9.1, wherein R¹, R², R³, R⁴ and R⁵ are as originally defined, as shown in w. Compounds of Formula 9.3, wherein R¹, R², R³, R⁴, R⁵, R⁷ and R⁸ are as originally defined, can be prepared by the method shown in **Scheme 9, step *x*.** The compound of Formula 9.1, wherein R¹, R², R³, R⁴ and R⁵ are as originally defined, can be treated with an amine, such as a compound of Formula 9.2, wherein R⁷ and R⁸ are as originally defined, in the presence of a base, such as triethylamine, in a solvent mixture, such as 1:1 methanol: 1,4-dioxane, at a temperature of about 23 °C to reflux (~80 °C) to afford compounds of Formula 9.3, wherein R¹, R², R³, R⁴, R⁵, R⁷ and R⁸ are as originally defined, as shown in x.

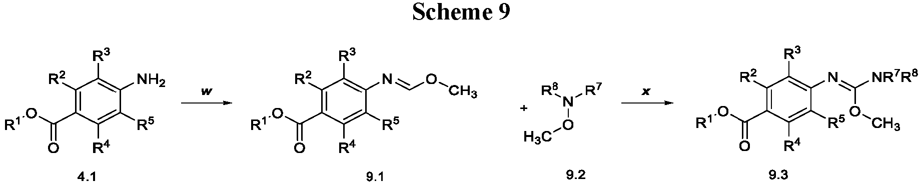

Compounds of Formula 10.2, wherein R², R³, R⁴, R⁵, R⁶, R⁷ and R⁸ are as originally defined, can be prepared by the method shown in Scheme 10, step y. The compound of Formula 10.1, wherein R², R³, R⁴, R⁵, R⁶, R⁷ and R⁸ are as originally defined, can be treated with a base, such as sodium hydroxide (NaOH), in a solvent, such as MeOH, at a temperature of about 23 °C to 60 °C to afford compounds of Formula 10.2, wherein R², R³, R⁴, R⁵, R⁶, R⁷ and R⁸ are as originally defined, as shown in*y*.

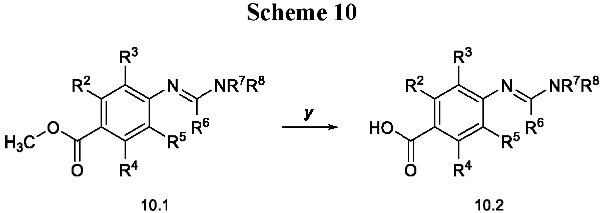

Compounds of Formula 11.1, wherein R¹, R², R³, R⁴, R⁵, R⁷ and R⁸ are as originally defined, can be prepared by the method shown in Scheme 11, step z. The compound of Formula 8.2, wherein R¹, R², R³, R⁴, R⁵, R⁷ and R⁸ are as originally defined, can be treated with an alkylating agent, such as iodomethane, in the presence of a base, such as potassium carbonate (K₂CO₃), in a solvent, such as acetone, at a temperature of about 23 °C to afford compounds of Formula 11.1, wherein R¹, R², R³, R⁴, R⁵, R⁷ and R⁸ are as originally defined, as shown in *z*.

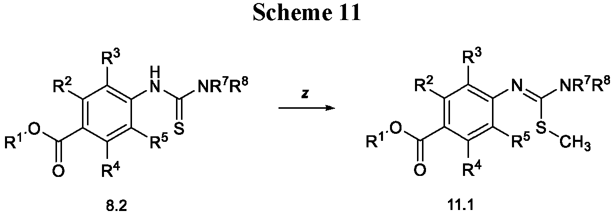

Compounds of Formula 12.1, wherein R¹, R², R³, R⁴, R⁵, R⁷ and R⁸ are as originally defined, can be prepared by the method shown in **Scheme 12, step *aa.*** The compound of Formula 5.2, wherein R¹, R², R³, R⁴, R⁵, R⁷ and R⁸ are as originally defined, can be treated with a protic acid (HX), such as hydrochloric acid (HCl), hydrobromic acid (HBr), acetic acid (HOAc), trifluoroacetic acid, para-toluenesulfonic acid (pTsOH), or citric acid, in a solvent, such as heptane or ethyl acetate, at a temperature of about 23 °C to afford compounds of Formula 12.1, wherein R¹, R², R³, R⁴, R⁵, R⁷ and R⁸ are as originally defined, as shown in *aa.*

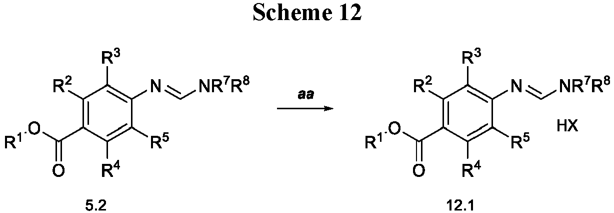

### EXAMPLES

### Example 1A: Preparation of methyl 4-amino-5-iodo-2-methylbenzoate.

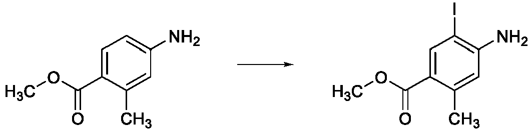

To a solution of methyl 4-amino-2-methylbenzoate (0.29 g, 1.76 mmol) in DMF (1.5 mL) were added sodium periodate (0.14 g, 0.70 mmol) and I₂ (74 mg, 1.41 mmol), respectively. The reaction mixture was stirred at 50 °C for 3 h. The reaction mixture was diluted with a saturated sodium thiosulfate solution (5 mL). Solids were then filtered and dried. The crude product was triturated with EtOAc (1 mL), and pentane (9 mL) to afford the title compound (0.22 g, 43% yield) as a pink solid: ¹H NMR (400 MHz, CDCl₃) δ 8.27 (s, 1H), 6.54 (s, 1H), 4.38 (brs, 2H), 3.84 (s, 3H), 2.50 (s, 3H); ESIMS *m*/*z* 292 ([M+H]⁺).

### Example 1B: Preparation of methyl 4-acetamido-5-bromo-2-methoxybenzoate.

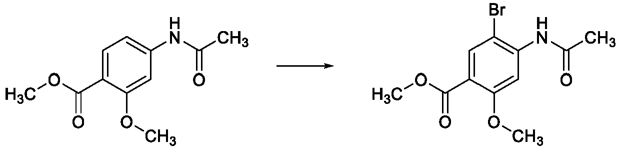

To a solution of methyl 4-acetamido-2-methoxybenzoate (4.04 g, 18.1 mmol) in DMF (80 mL) at 0 °C was added N-bromosuccinimide (3.22 g, 18.1 mmol). The mixture was stirred at 0 °C and allowed to slowly warm to room temp while stirring overnight. The mixture was then diluted with water, and a precipitate formed. The precipitate was filtered off and washed with additional water. The precipitate was dried under vacuum, providing an impure product. The crude product was purified by flash column chromatography (silica gel (SiO₂), 0→100% ethyl acetate in hexane) to afford the title compound (3.89 g, 12.9 mmol, 71% yield) as a white solid: ¹H NMR (400 MHz, CDCl₃) δ 8.32 (s, 1H), 8.04 (s, 1H), 7.76 (s, 1H), 3.93 (s, 3H), 3.87 (s, 3H), 2.28 (s, 3H); ¹³C NMR (101 MHz, CDCl₃) δ 166.28, 162.47, 157.58, 137.80, 132.74, 113.36, 102.13, 99.53, 54.06, 49.79, 22.92; ESIMS *m*/*z* 304 [(M+H)⁺].

### Example IC: Preparation of 4-bromo-5-methyl-2-(trifluoromethyl)aniline.

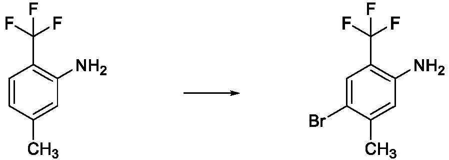

In a 25 mL vial, a solution of 5-methyl-2-(trifluoromethyl)aniline (1.00 g, 5.71 mmol) was prepared in DMF (18 mL). The reaction was cooled to 0 °C in an ice water bath. N-bromosuccinimide (1.02 g, 5.71 mmol) was then added in one portion. The reaction was allowed to stir overnight, slowly warming to ambient temperature as the ice melted. After 18 h, the reaction was quenched with water (50 mL) and diluted with EtOAc (50 mL). The layers were separated, and the aqueous layer was extracted with EtOAc (3 x 50 mL). The combined organic layers were then washed with brine (3 x 100 mL), dried over MgSO₄, filtered, and concentrated to afford the title compound (1.31 g, 5.16 mmol, 90% yield) as a dark yellow oil that was used without further purification: ¹H NMR (400 MHz, CDCl₃) δ 7.54 (s, 1H), 6.63 (s, 1H), 4.09 (s, 2H), 2.32 (s, 3H); ¹⁹F NMR (376 MHz, CDCl₃) δ -62.58; HRMS-ESI *(m*/*z)* [M+H]⁺ calcd for C₈H₈BrF₃N, 253.9787; found, 253.9778.

### Example 2A: Preparation of methyl 4-amino-2,5-dimethylbenzoate.

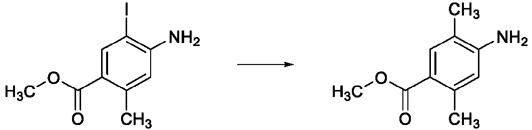

To a solution of methyl 4-amino-5-iodo-2-methylbenzoate (0.22 g, 0.75 mmol) in 1,4-dioxane (5 mL), was added cesium carbonate (0.98 g, 3.02 mmol) and it was degassed for 5 min. PdCl₂(dppf)DCM (0.061 g, 0.07 mmol) and trimethylboroxine (0.23 g, 1.88 mmol) were then added, and the reaction mixture was heated to 120 °C for 1 h under microwave radiation. The reaction mixture was diluted with water (15 mL) and extracted with EtOAc (2 × 40 mL). The combined organic layers were dried over anhydrous Na₂SO₄, filtered and concentrated under reduced pressure. The crude product was purified by flash column chromatography (silica gel (SiO₂), 20→25% ethyl acetate in hexane) to afford the title compound (0.11 g, 84% yield) as a brown solid: ESIMS *m*/*z* 180 ([M+H]⁺).

### Example 2B: Preparation of methyl 4-acetamido-2-methoxy-5-methylbenzoate.

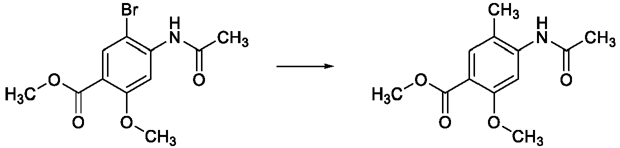

Methyl 4-acetamido-5-bromo-2-methoxybenzoate (2.00 g, 6.62 mmol), methylboronic acid (0.594 g, 9.93 mmol), XPhosPd G3 (0.112 g, 0.132 mmol), and potassium phosphate tribasic (2.81 g, 13.2 mmol) were dissolved/suspended in 1,4-dioxane (30.1 mL)/water (3.01 mL) and heated to 100 °C. The mixture was stirred for 4 h at 100 °C. The mixture was cooled to room temperature (UPLC shows ~50 % conversion), diluted with DCM and water. The mixture was then passed through a phase separator, and the products extracted with DCM. The crude product was purified by flash column chromatography (silica gel (SiO₂), 0→100% ethyl acetate in hexane) to afford the title compound (658 mg, 2.77 mmol, 42% yield) as a white solid and 866 mg (43%) of recovered starting material: ¹H NMR (400 MHz, CDCl₃) δ 8.01 (s, 1H), 7.70 - 7.63 (m, 1H), 7.11 (s, 1H), 3.90 (s, 3H), 3.87 (s, 3H), 2.25 (s, 3H), 2.22 (s, 3H); ¹³C NMR (101 MHz, CDCl₃) δ 167.27, 165.10, 157.74, 139.71, 132.51, 131.72, 115.99, 103.58, 55.11, 50.80, 23.93, 15.45; ESIMS m/z 236 [(M-H)⁻].

### Example 3A: Preparation of 4-amino-2,5-dimethylbenzoic acid

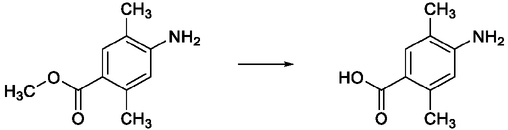

To a solution of methyl 4-amino-2,5-dimethylbenzoate (0.11 g, 0.69 mmol) in THF: MeOH:H₂O (3:2:1) (2 mL) was added LiOH (0.073 mg, 3.07 mmol), and the reaction mixture was stirred at 70 °C for 16 h. The reaction mixture was then acidified with acetic acid (0.5 mL). The precipitated solids were filtered and dried to afford the title compound (0.062 g, 68% yield) as a pale yellow solid: ¹H NMR (400 MHz, CDCl₃) δ 7.82 (s, 1H), 6.48 (s, 1H), 3.97 (brs, 2H), 2.54 (s, 3H), 2.14 (s, 3H); ESIMS *m*/*z* 166 ([M+H]⁺).

### Example 3B: Preparation of 4-amino-2-methoxy-5-methylbenzoic acid.

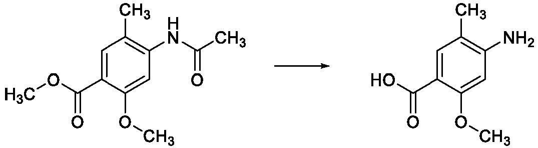

In a 50 mL round-bottomed flask, methyl 4-acetamido-2-methoxy-5-methylbenzoate (0.658 g, 2.77 mmol) was dissolved/suspended in a 6M aqueous KOH solution. To the suspension at room temp was added MeOH (5 mL). The mixture was then heated to 60 °C and stirred overnight. The reaction was cooled to rt, diluted with water, and carefully acidified to pH ~ 4-5 with 6N HCl (dropwise). The products were extracted with EtOAc (3x). The combined organic layers were then dried with Na₂SO₄, filtered and concentrated to afford the title compound (437 mg, 2.41 mmol, 87% yield) as an off-white solid: ¹H NMR (500 MHz, CDCl₃) δ 7.84 (s, 1H), 6.25 (s, 1H), 4.19 (s, 3H), 3.98 (s, 3H), 2.11 (s, 3H); ¹³C NMR (126 MHz, CDCl₃) δ 165.97, 158.27, 151.07, 135.66, 115.42, 106.65, 96.62, 56.49, 16.15; ESIMS *m*/*z* 182 [(M+H)⁺].

### Example 4: Preparation of 1-bromo-5-chloro-2-methyl-4-nitrobenzene.

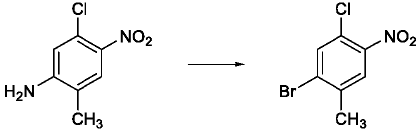

To a solution of 5-chloro-2-methyl-4-nitroaniline (5.3 g, 28.49 mmol) in acetic acid (53 mL) was added aq. HBr (7.7 mL) at room temperature. NaNO₂ (1.96 g, 28.49 mmol) was then added over 45 min. The reaction mixture was stirred at 85 °C for 2 h. After 2 h, the reaction mixture was cooled to room temperature and poured into ice water (100 mL). The obtained solid was filtered, washed with water (100 mL), and dried to afford the title compound (5.5 g, 74% yield) as a pale yellow solid: ¹H NMR (400 MHz, CDCl₃) δ 7.79 (s, 1H), 7.52 (s, 1H), 2.45 (s, 3H).

### Example 5: Preparation of 4-bromo-2-chloro-5-methylaniline.

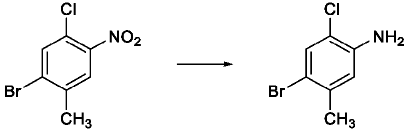

Fe⁰ powder (12.1 g, 220.8 mmol) and NH₄Cl (11.7 g, 220.8 mmol) were added to a solution of 1-bromo-5-chloro-2-methyl-4-nitrobenzene (5.5 g, 22.08 mmol) in EtOH:H₂O (55 mL, 1:1) at room temperature. The reaction mixture was stirred at 70 °C for 30 min. The reaction mixture was then cooled to room temperature, and the solvent was concentrated under reduced pressure. The crude material was diluted with water (30 mL), filtered and the solid was washed with EtOAc (30 mL). The aqueous layer was extracted with EtOAc (2 x 30 mL). The combined organic layers were dried over anhydrous Na₂SO₄, filtered and concentrated under reduced pressure. The crude product was purified via flash column chromatography (silica gel (SiO₂), 3→5% ethyl acetate in petroleum ether) to afford the title compound (2.8 g, 58% yield) as an off-white solid: ¹H NMR (400 MHz, DMSO-*d*₆) δ 7.38 (s, 1H), 6.65 (s, 1H), 3.96 (brs, 2H), 2.27 (s, 1H); ESIMS *m*/*z 220* ([M+H]⁺).

### Example 6A: Preparation of 4-amino-2,5-dichlorobenzonitrile.

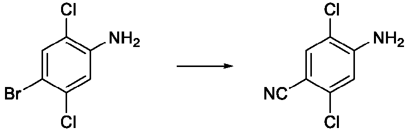

To a solution of 4-bromo-2,5-dichloroaniline (2 g, 8.33 mmol) in NMP (20 mL) was added CuCN (2.2 g, 24.99 mmol) and the reaction mixture was heated to 180 °C for 1.5 h under microwave irradiation. The reaction mixture was poured into ice cold water (30 mL) and was extracted with EtOAc (3 x 60 mL). The organic layer was dried over anhydrous Na₂SO₄, filtered and concentrated under reduced pressure to obtain the crude product. The crude product was purified by column chromatography (silica gel (SiO₂), 15→20% ethyl acetate in petroleum ether) to afford the title compound (1 g, 64% yield) as a pale yellow solid: ¹H NMR (400 MHz, CDCl₃) δ 7.83 (s, 1H), 6.92 (s, 1H), 6.73 (brs, 2H); ESIMS *m*/*z* 187 ([M+H]⁺).

### Example 6B: Preparation of 4-amino-2,5-dimethylbenzonitrile.

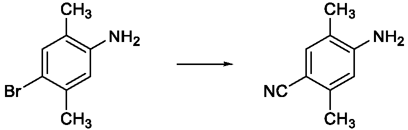

A solution of 4-bromo-2,5-dimethylaniline (15 g, 75.00 mmol) and Zn(CN)₂ (9.6 g, 82.50 mmol) in DMF (150 mL) was degassed for 10 min. Tetrakis(triphenylphosphine)-palladium(0) (12.9 g, 11.25 mmol) was then added, and the reaction mixture was heated to 120 °C for 2 days in a sealed tube. After 2 d, the reaction mixture was poured into ice cold water (400 mL) and extracted with EtOAc (3 × 600 mL). The combined organic layers were dried over anhydrous Na₂SO₄, filtered and concentrated under reduced pressure. The crude product was purified by column chromatography (silica gel (SiO₂), 15→20% ethyl acetate in petroleum ether) to afford the title compound (5.7 g, 52% yield) as pale yellow solid: ¹H NMR (400 MHz, CDCl₃) δ 7.25 (s, 1H), 6.50 (s, 1H), 3.98 (brs, 2H), 2.40 (s, 3H), 2.11 (s, 3H); ESIMS *m*/*z* 147 ([M+H]⁺).

### Example 6C: Preparation of methyl 4-amino-5-methoxy-2-methylbenzoate.

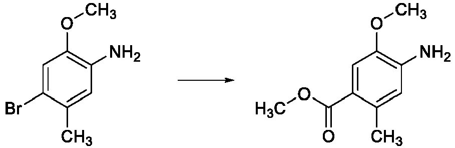

A solution of 4-bromo-2-methoxy-5-methylaniline (2.0 g, 9.3 mmol), palladium(II) acetate (0.302 g, 1.345 mmol), 1,4-bis(diphenylphosphanyl)butane (1.19 g, 2.79 mmol) and triethylamine (2.6 mL, 19 mmol) was prepared in MeOH (20 mL) in a 45 mL Parr reactor. The reactor was sealed and purged with CO (3 cycles to 50-100 psi). The reactor was then filled with CO to 400 psi, placed in a heating block, and heated to 130 °C for 24 h. The crude material was concentrated, and the crude residue was dissolved in water (10 mL) and EtOAc (40 mL) and filtered through celite. The aqueous layer was extracted with EtOAc (3 x 20 mL). The combined organic layers were washed with brine (10 mL), dried over MgSO₄, filtered and concentrated. The crude product was purified by column chromatography (silica gel (SiO₂), 0→40% ethyl acetate in petroleum ether) to afford the title compound (363 mg, 20% yield) as a rose red solid: ¹H NMR (400 MHz, CDCl₃) δ 7.42 (s, 1H), 6.50 (s, 1H), 4.12 (s, 2H), 3.87 (s, 3H), 3.84 (s, 3H), 2.49 (s, 3H); ESIMS *m*/*z* 196 ([M+H]⁺).

### Example 7A: Preparation of 4-amino-2,5-dichlorobenzoic acid.

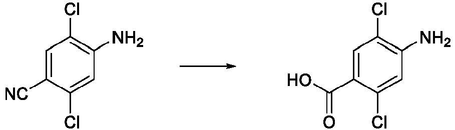

To a solution of 4-amino-2,5-dichlorobenzonitrile (1 g, 5.37 mmol) in water (10 mL) was added KOH (6.0 g, 107.52 mmol) at room temperature, and the reaction mixture was heated to 120 °C for 2 days in a sealed tube. After 2 d, the reaction mixture was extracted with EtOAc (2 × 25 mL). The aqueous layer was acidified with acetic acid (12 mL) and was extracted with 10% MeOH in DCM (2 × 75 mL). The combined organic layers were dried over anhydrous Na₂SO₄, filtered and concentrated under reduced pressure to afford the title compound (0.7 g, 63% yield) as a pale yellow solid which was used in the next step without further purification: ¹H NMR (400 MHz, CDCl₃) δ 7.61 (s, 1H), 6.77 (s, 1H), 5.89 (brs, 2H); ESIMS *m*/*z* 206 ([M+H]⁺).

### Example 7B: Preparation of 4-amino-5-methoxy-2-methylbenzoic acid.

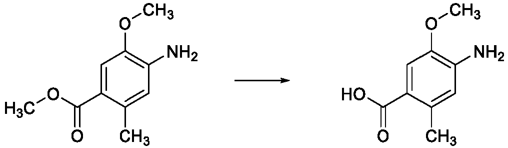

A solution of methyl 4-amino-5-methoxy-2-methylbenzoate (155 mg, 0.794 mmol) and lithium hydroxide (86 mg, 3.6 mmol) was prepared in 3:2:1 THF:MeOH:water (2.4 mL). The resulting dark purple reaction was stirred at 70 °C overnight. 1M HCl was then carefully added to acidify the reaction to ~pH = 4, and a solid precipitated. The aqueous layer was extracted with EtOAc (3 x 30 mL). The combined organic layers were dried over anhydrous MgSO₄, filtered and concentrated under reduced pressure to afford the title compound (92 mg, 64% yield) as a dark green solid which was used in the next step without further purification: ¹H NMR (400 MHz, DMSO-*d*₆) δ 11.95 (s, 1H), 7.29 (s, 1H), 6.44 (s, 1H), 5.40 (s, 2H), 3.75 (s, 3H), 2.37 (s, 3H); ¹³C NMR (126 MHz, DMSO-*d*₆) δ 168.71, 143.69, 142.34, 134.93, 116.12, 115.89, 113.25, 55.76, 21.98; IR (thin film) 3500, 3396, 2935, 2836, 1669, 1608, 1529, 1451, 1364, 1258, 1217, 1081, 1022, 867 cm⁻¹; HRMS-ESI *(m*/*z)* [M+H]⁺ calcd for C₉H₁₂NO₃, 182.0812; found, 182.0812.

### Example 8A: Preparation of 1-(p-tolyl)propan-1-ol.

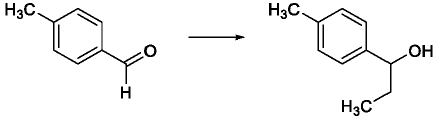

In a 250 mL flask, a solution of 4-methylbenzaldehyde (0.736 mL, 6.24 mmol) was prepared in diethyl ether (31.2 mL) and cooled to 0 °C in an ice bath. To this clear solution was added ethylmagnesium bromide (1M in THF, 7.49 mL, 7.49 mmol) dropwise over 5 min, and the resulting solution was stirred overnight, slowly warming to rt as the ice bath melted. After 18 h, TLC indicated consumption of starting material and conversion to a more polar product. The reaction was quenched with saturated aqueous NH₄Cl (50 mL) and extracted with diethyl ether (3 x 50 mL). The combined organic layers were passed through a phase separator and concentrated to a clear oil. The crude material was purified by flash column chromatography (silica gel (SiO₂), 0→50% ethyl acetate in hexanes) to afford the title compound (476 mg, 1.89 mmol, 51% yield) as a clear colorless oil: ¹H NMR (500 MHz, CDCl₃) δ 7.24 - 7.20 (m, 2H), 7.15 (d, *J=* 7.9 Hz, 2H), 4.54 (ddd, *J=* 7.0, 4.7, 1.6 Hz, 1H), 2.34 (s, 3H), 1.88 - 1.68 (m, 3H), 0.90 (t, *J* = 7.4 Hz, 3H); ¹³C NMR (126 MHz, CDCl₃) δ 141.64, 137.15, 129.08, 125.93, 75.89, 31.80, 21.11, 10.20; IR (thin film) 3340, 2962, 2926, 1454, 1097, 1039, 1012, 815 cm⁻¹.

### Example 8B: Preparation of (R)-1-(p-tolyl)ethan-1-ol.

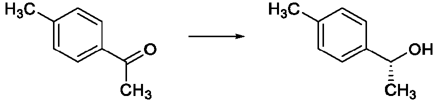

In a 100 mL flask, a solution of 1-(p-tolyl)ethan-1-one (0.747 mL, 5.59 mmol) and (*S*)-1-methyl-3,3-diphenyltetrahydro-1H,3H-pyrrolo[1,2-c][1,3,2]oxazaborole ((S)-CBS catalyst, 1M in toluene, 1.118 mL, 1.118 mmol) was prepared in toluene (37.3 mL) and cooled to 0 °C in an ice/water bath. BH₃-DMS (2M in THF, 3.49 mL, 6.99 mmol) was then added over 2 min via syringe, and the ice bath was removed. The reaction was stirred at rt. After 1 hr, TLC indicated consumption of starting material. Methanol (2.27 mL, 55.9 mmol) was added slowly, and the reaction was concentrated to afford a Clear colorless oil. The crude material was purified by flash column chromatography (silica gel (SiO₂), 0→50% ethyl acetate in hexanes) to afford the title compound (784 mg, 5.76 mmol, quant. yield) as a Clear colorless oil: ¹H NMR (500 MHz, CDCl₃) δ 7.26 (d, *J= 8.0* Hz, 2H), 7.16 (d, *J=* 7.9 Hz, 2H), 4.86 (qd, *J* = 6.4, 2.7 Hz, 1H), 2.34 (s, 3H), 1.78 (d, *J=* 3.1 Hz, 1H), 1.48 (d, *J=* 6.5 Hz, 3H); ¹³C NMR (126 MHz, CDCl₃) δ 142.88, 137.16, 129.17, 125.35, 70.26, 25.08, 21.09; IR (thin film) 3341, 2971, 1513, 1071, 1009, 897, 816 cm⁻¹.

### Example 9A: Preparation of 4-methylbenzyl 4-amino-2,5-dimethylbenzoate.

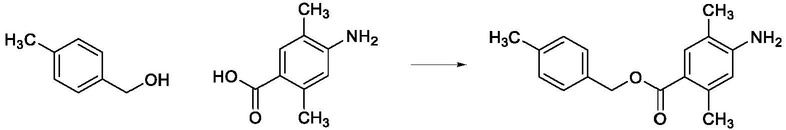

In a 20 mL vial, p-tolylmethanol (222 mg, 1.82 mmol), 4-amino-2,5-dimethylbenzoic acid (150 mg, 0.908 mmol) and DMAP (11.1 mg, 0.091 mmol) were dissolved in DCM (4.45 mL) and cooled to 0 °C in an ice/water bath. After ~5 min, EDC (211 mg, 1.36 mmol) was added in one portion, and the resulting pale yellow reaction was stirred overnight, slowly warming to rt as the ice melted. After 18 h, TLC indicated consumption of starting material. The reaction was concentrated to afford an oil. The crude material was purified by flash column chromatography (C18 reverse phase, 50→100% acetonitrile in water) to afford the title compound (192 mg, 0.712 mmol, 78% yield) as an off white semisolid: ¹H NMR (400 MHz, CDCl₃) δ 7.74 (s, 1H), 7.36 - 7.29 (m, 2H), 7.18 *(d, J=* 7.8 Hz, 2H), 6.46 (s, 1H), 5.25 (s, 2H), 3.88 (s, 2H), 2.52 (s, 3H), 2.36 (s, 3H), 2.12 (s, 3H); ¹³C NMR (101 MHz, CDCl₃) δ 167.17, 148.26, 140.79, 137.70, 133.84, 133.80, 129.17, 128.24, 118.63, 117.06, 77.22, 65.78, 21.98, 21.20, 16.59; HRMS-ESI (*m*/*z*) [M+H]⁺ calcd for C₁₇H₂₀NO₂, 270.1489; found, 270.1477.

### Example 9B: Preparation of 2-methylbenzyl 4-amino-2,5-dimethylbenzoate.

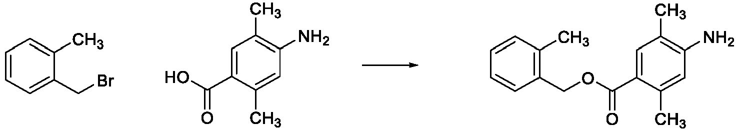

To a solution of 4-amino-2,5-dimethylbenzoic acid (4.2 g, 25.45 mmol) in DMF (40 mL), were added 1-(bromomethyl)-2-methylbenzene (3.5 mL, 25.45 mmol) and K₂CO₃ (3.8 g, 27.99 mmol), respectively. The reaction mixture was stirred at room temperature for 3 h. The reaction mixture was then poured into ice cold water (100 mL) and extracted with EtOAc (2 × 200 mL). The combined organic layers were dried over anhydrous Na₂SO₄, filtered and concentrated under reduced pressure. The crude product was purified by flash column chromatography (silica gel (SiO₂), 10→15% ethyl acetate in hexanes) to afford the title compound (3.8 g, 55% yield) as an off white solid: ¹H NMR (400 MHz, CDCl₃) δ 7.74 (s, 1H), 7.40 (d, 1H), 7.20 (m, 3H), 6.47 (s, 1H), 5.29 (s, 2H), 3.89 (brs, 2H), 2.52 (s, 3H), 2.40 (s, 3H), 2.11 (s, 3H); ESIMS *m*/*z* 270 ([M+H]⁺).

### Example 9C: Preparation of 1-(p-tolyl)propyl 4-amino-2,5-dimethylbenzoate.

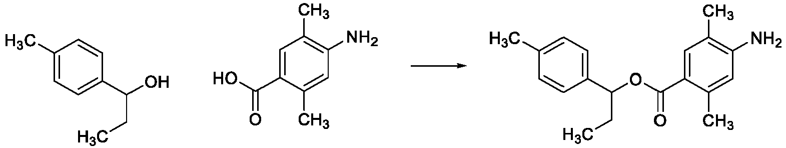

To a 20 mL vial containing 4-amino-2,5-dimethylbenzoic acid (200 mg, 1.21 mmol) was added 2-(p-tolyl)propan-2-ol (364 mg, 2.42 mmol) and PyBOP (945 mg, 1.82 mmol). DCM (12.1 mL) was added followed by *N*-ethyl-*N*-isopropylpropan-2-amine (844 µl, 4.84 mmol) dropwise over 45 seconds. After 10 min, most of the solids solubilized and the resultant pale pink colored reaction was stirred at room temperature overnight. After 18 h, the reaction was filtered and concentrated to a brown oil. The crude material was purified by flash column chromatography (C18 reverse phase, 50→100% acetonitrile in water) to afford the title compound (107 mg, 0.36 mmol, 30% yield) as an orange oil: ¹H NMR (500 MHz, CDCl₃) δ 7.77 (s, 1H), 7.33 7.27 (m, 2H), 7.18 7.10 (m, 2H), 6.44 (s, 1H), 5.82 (t, *J* = 6.8 Hz, 1H), 3.87 (s, 2H), 2.51 (s, 3H), 2.32 (s, 3H), 2.14 (s, 3H), 2.03 (dt, *J* = 13.7, 7.5 Hz, 1H), 1.90 (tt, *J* = 13.7, 7.4 Hz, 1H), 0.94 (t, *J* = 7.4 Hz, 3H); ¹³C NMR (126 MHz, CDCl₃) δ 166.70, 148.20, 140.65, 138.30, 137.17, 133.76, 129.02, 126.50, 118.97, 118.60, 117.06, 76.92, 29.66, 22.08, 21.14, 16.70, 10.17; IR (thin film) 3376, 2967, 2927, 1689, 1624, 1562, 1253, 1156, 1053, 814 cm^{-1;} HRMS-ESI *(m*/*z)* [M+H]⁺ calcd for C₁₉H₂₄NO₂, 298.1802; found, 298.1801.

### Example 10A: Preparation of 4-methylbenzyl (E)-4-(((ethyl(methyl)amino)methylene)amino)-2,5-dimethylbenzoate.

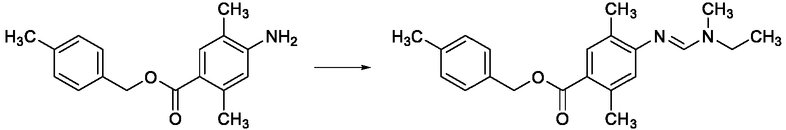

In a 100 mL round-bottomed flask, a solution of 4-methylbenzyl 4-amino-2,5-dimethylbenzoate (359 mg, 1.33 mmol) was prepared in toluene (26.6 mL). N-(dimethoxymethyl)-N-methylethanamine (532 mg, 4.00 mmol) was then added, and the resulting solution was fitted with a reflux condenser, heated to 80 °C and stirred for 48 h. After 48 h, the solution was concentrated to an oil. The crude material was purified by flash column chromatography (C18 reverse phase, 30→100% acetonitrile in water) to afford the title compound (333 mg, 0.98 mmol, 74% yield) as a brown oil: ¹H NMR (400 MHz, CDCl₃) δ 7.79 (s, 1H), 7.45 (s, 1H), 7.33 (d, *J=* 7.9 Hz, 2H), 7.17 (d, *J* = 7.8 Hz, 2H), 6.56 (s, 1H), 5.26 (s, 2H), 3.39 (bd, *J=* 67.1 Hz, 2H), 2.99 (s, 3H), 2.55 (s, 3H), 2.35 (s, 3H), 2.22 (s, 3H), 1.20 (t, *J* = 7.1 Hz, 3H); ¹³C NMR (101 MHz, CDCl₃) δ 167.49, 154.64, 151.69, 139.49, 137.70, 133.73, 132.81, 129.17, 128.79, 128.27, 122.55, 121.90, 65.92, 47.85, 32.02, 21.80, 21.18, 17.41, 14.37; ESIMS *m*/*z* 339 [(M+H)⁺].

### Example 10B: Preparation of 4-methylbenzyl (E)-4-(((diethylamino)methylene)amino)-2,5-dimethylbenzoate.

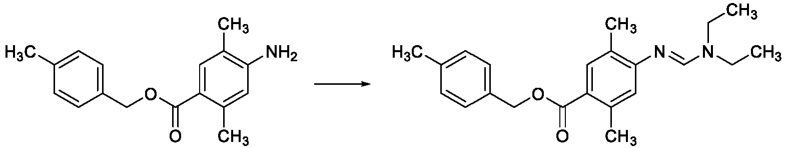

In a 20 mL vial, 4-methylbenzyl 4-amino-2,5-dimethylbenzoate (100 mg, 0.37 mmol) was dissolved in triethyl orthoformate (2 mL, 12.00 mmol) and then *p*-toluenesulfonic acid monohydrate (7.06 mg, 0.03 mmol) was added. The reaction was heated to reflux (140 ⁰C) and stirred for 3 h. After 3 h, TLC indicated near complete consumption of starting material. The reaction was quenched with saturated aqueous NaHCO₃ (10 mL) and extracted with DCM (3 x 10 mL). The combined organic phases were passed through a phase separator and concentrated to a pale yellow oil. The residue was redissolved in DCM (0.371 mL) and diethylamine (0.058 mL, 0.55 mmol) was added dropwise via syringe. The solution was heated to 40 °C and stirred for 3 h. The reaction was quenched with water (10 mL) and extracted with DCM (3 x 10 mL). The combined organic phases were passed through a phase separator and concentrated. The crude material was purified by flash column chromatography (C18 reverse phase, 30→100% acetonitrile in water) to afford the title compound (75.8 mg, 0.21 mmol, 58% yield) as a brown oil: ¹H NMR (500 MHz, CDCl₃) δ 7.79 *(t, J=* 1.4 Hz, 1H), 7.42 (s, 1H), 7.38 7.30 (m, 2H), 7.18 (dt, J= 6.6, 1.7 Hz, 2H), 6.55 (s, 1H), 5.26 (s, 2H), 3.40 (d, *J* = 94.6 Hz, 4H), 2.55 (s, 3H), 2.36 (s, 3H), 2.22 (s, 3H), 1.22 (t, *J=* 7.1 Hz, 6H); IR (thin film) 2970, 2927, 1707, 1629, 1592, 1549, 1371, 1250, 1110, 1047 cm⁻¹; HRMS-ESI *(m*/*z)* [M+H]⁺ calcd for C₂₂H₂₉N₂O₂, 353.2224; found, 353.2227.

### Reference Example 10C: Preparation of 4-methylbenzyl (E)-2,5-dimethyl-4-(piperidin-2-ylideneamino)benzoate. (compound not according to the attached claims)

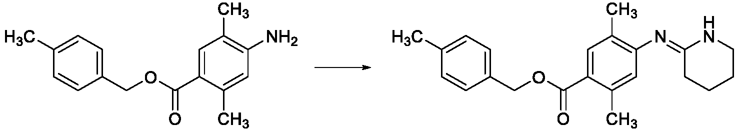

In a 20 mL vial, a solution of piperidin-2-one (0.103 mL, 1.11 mmol) was prepared in toluene (9 mL) under N₂. Phosphoryl trichloride (0.052 mL, 0.55 mmol) was then added, and the cloudy reaction was stirred at rt for 2 h. 4-methylbenzyl-4-amino-2,5-dimethylbenzoate (150 mg, 0.55 mmol) was then added, and the reaction was fitted with a reflux condenser and heated at reflux (110 ⁰C) for 3 h. The resulting clear golden-colored reaction was then cooled to rt, neutralized to pH 7 with 10% aq. NaOH, and diluted with toluene (20 mL). The crude reaction was stirred overnight. The layers were separated and the aqueous layers were washed with ethyl acetate (3 x 20 mL). The combined organic layers were washed with brine, dried over Na₂SO₄, filtered, and concentrated to an oil. A SCX column (equilibrated with DCM, DMF, MeOH) was used to purify the crude material. The material was loaded in DCM, and the column was flushed with DCM and MeOH to elute undesired components. Flushing the SCX column with 7N NH₃ in MeOH afforded the title compound (95.0 mg, 0.27 mmol, 49% yield) as a yellow oil: ¹H NMR (600 MHz, CDCl₃) δ 7.79 (s, 1H), 7.34 (d, *J* = 7.8 Hz, 2H), 7.19 (d, *J* = 7.8 Hz, 2H), 6.63 (s, 1H), 5.27 (s, 2H), 4.39 (s, 1H), 3.22 (d, *J=* 100.6 Hz, 2H), 2.59 (d, *J= 31.6* Hz, 2H), 2.52 (s, 3H), 2.36 (s, 3H), 2.07 (s, 3H), 1.76 (dh, *J* = 8.3, 4.0, 3.3 Hz, 4H); ¹³C NMR (151 MHz, CDCl₃) δ 167.43, 155.09, 152.08, 139.70, 137.84, 133.52, 133.42, 129.21, 128.35, 127.29, 125.05, 123.33, 66.08, 42.55, 30.85, 23.06, 21.70, 21.22, 21.14, 17.02; ESIMS *m*/*z* 351 [(M+H)⁺].

### Example 10D: Preparation of 4-methylbenzyl 4-(3,3-diethylthioureido)-2,5-dimethylbenzoate.

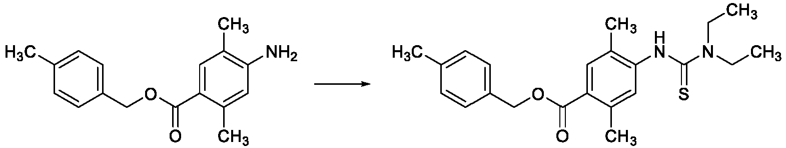

In a 20 mL vial, a solution of 4-methylbenzyl 4-amino-2,5-dimethylbenzoate (100 mg, 0.37 mmol) and sodium bicarbonate (312 mg, 3.71 mmol) was prepared in DCM (1.24 mL) and water (1.24 mL). To this solution was added thiophosgene (31.3 µL, 0.40 mmol) dropwise via syringe. The resulting orange biphasic mixture was stirred vigorously at rt for 2 h. After 2 h, TLC showed complete consumption of starting material. The biphasic mixture was diluted with water (5 mL) and DCM (5 mL), passed through a phase separator, and concentrated to afford a pale yellow oil. The crude material was redissolved in DCM (1.24 mL) and diethylamine (77 µL, 0.74 mmol) was then added in one portion via syringe. The resulting solution was stirred at rt for 1 h. After 1 h, the solution was concentrated to an oil. The crude material was purified by flash column chromatography (silica gel (SiO₂), 0→50% ethyl acetate in hexanes) to afford the title compound (140.0 mg, 0.36 mmol, 98% yield) as a white semisolid: ¹H NMR (400 MHz, CDCl₃) δ 7.81 (s, 1H), 7.36 - 7.28 (m, 2H), 7.23 - 7.15 (m, 3H), 6.75 (s, 1H), 5.27 (s, 2H), 3.76 (q, *J* = 7.1 Hz, 4H), 2.54 (s, 3H), 2.36 (s, 3H), 2.23 (s, 3H), 1.31 (t, *J* = 7.1 Hz, 6H); ¹³C NMR (101 MHz, CDCl₃) δ 180.90, 167.00, 141.68, 138.92, 138.00, 133.26, 133.19, 130.80, 129.63, 129.26, 128.37, 127.11, 66.42, 45.82, 21.54, 21.21, 17.59, 12.70; IR (thin film) 3240, 2974, 1713, 1516, 1258, 1141, 1055, 806, 728 cm⁻¹; HRMS-ESI *(m*/*z)* [M+H]⁺ calcd for C₂₂H₂₉N₂O₂S, 385.1944; found, 385.1950.

### Example 12: Preparation of 3-(trifluoromethyl)benzyl (Z)-4-(((ethyl(methyl)amino)(methylthio)methylene)amino)-2,5-dimethylbenzoate.

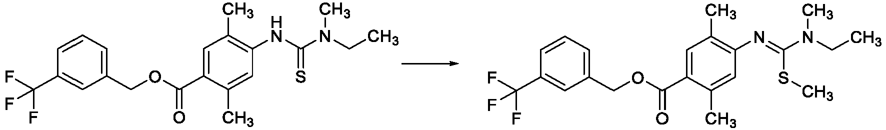

A solution of 3-(trifluoromethyl)benzyl 4-(3-ethyl-3-methylthioureido)-2,5-dimethylbenzoate (0.050 g, 0.118 mmol) was prepared in acetone (1.18 mL). To this solution was added K₂CO₃ (0.033 g, 0.24 mmol) and iodomethane (10 µL, 0.16 mmol). The mixture was then stirred at ambient temperature for 18 h. The reaction was then diluted with ethyl acetate (50 mL), filtered through celite and concentrated to an oil. The crude material was purified by flash column chromatography (silica gel (SiO₂), 0→70% ethyl acetate in hexanes) to afford the title compound (49 mg, 0.11 mmol, 95% yield) as a clear oil: ¹H NMR (500 MHz, CDCl₃) δ 7.82-7.78 (m, 1H), 7.71 *(d, J=* 1.8 Hz, 1H), 7.64 *(d, J=* 7.6 Hz, 1H), 7.59 (d, *J=* 7.8 Hz, 1H), 7.51 (t, *J=* 7.7 Hz, 1H), 6.68 (s, 1H), 5.35 (s, 2H), 3.57 (q, *J* = 7.1 Hz, 2H), 3.08 (s, 3H), 2.54 (s, 3H), 2.14 (s, 3H), 1.94 (s, 3H), 1.20 (t, *J* = 7.0 Hz, 3H); ¹⁹F NMR (471 MHz, CDCl₃) δ -62.60; ESIMS *m*/*z* 439 [(M+H)⁺].

### Example 13: Preparation of 3-(trifluoromethyl)benzyl 4-(((ethyl(methyl)amino)methylene)amino)-2,3-dimethylbenzoate hydrochloride.

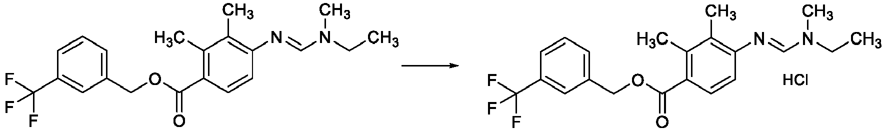

3-(trifluoromethyl)benzyl (E)-4-(((ethyl(methyl)amino)methylene)amino)-2,3-dimethylbenzoate was dissolved in heptane and transferred to a separtory funnel. 2N HCl was added, and the resulting layers were separated. The heptane layer was discarded and the aqueous layer was extracted with ethyl acetate. The organic layer was concentrated to afford 3-(trifluoromethyl)benzyl 4-(((ethyl(methyl)amino)methylene)amino)-2,3-dimethylbenzoate hydrochloride (237 mg, 0.553 mmol) as a light brown solid and ~2:1 mixture of *E:Z* isomers: ¹H NMR (500 MHz, CDCl₃) δ 12.64-12.53 (m, 1H), 7.95-7.86 (m, 1H), 7.70-7.65 (m, 1H), 7.65-7.57 (m, 3H), 7.56-7.49 (m, 1H), 7.28-7.22 (m, 0.6H), 5.37 (s, 2H), 4.00 (q, *J* = 7.2 Hz, 0.6H), 3.64 (q, *J* = 7.2 Hz, 1.4H), 3.50 (s, 2H), 3.33 (s, 1H), 2.44 (s, 3H), 2.38 (s, 3H), 1.36-1.28 (m, 3H); ¹⁹F NMR (471 MHz, CDCl₃) δ -62.64; HRMS-ESI (*m*/*z*) [M+H]⁺ calcd for C₂₁H₂₃F₃N₂O₂, 393.1784, found 393.1793; m.p. 172-176 °C.

### GENERAL BIOLOGICAL EXPERIMENTAL DETAILS

### Example A: Evaluation of Fungicidal Activity: Septoria Leaf Blotch of Wheat (Zymoseptoria tritici; Bayer code SEPTTR):

Technical grades of materials were dissolved in acetone, which were then mixed with nine volumes of water (H₂O) containing 110 ppm Triton X-100. The fungicide solutions were applied onto wheat seedlings using an automated booth sprayer to run-off. All sprayed plants were allowed to air dry prior to further handling. All fungicides were evaluated using the aforementioned method for their activity vs. all target diseases, unless stated otherwise.

Wheat plants (variety 'Yuma') were grown from seed in a greenhouse in soil-less potting mix until the first leaf was fully emerged, with 7-10 seedlings per pot. These plants were inoculated with an aqueous spore suspension of *Zymoseptoria tritici* either 3 days prior to fungicide treatment (3 day curative; 3DC) or 1 day after fungicide treatment (1 day protectant; 1DP). After inoculation the plants were kept in 100% relative humidity for three days to permit spores to germinate and infect the leaf. The plants were then transferred to a greenhouse for disease to develop. When disease symptoms were fully expressed on the 1st leaves of untreated plants, infection levels were assessed on a scale of 0 to 100 percent disease severity. Percent disease control was calculated using the ratio of disease severity on treated plants relative to untreated plants.

### Example B: Evaluation of FungicidalActivity: Wheat Brown Rust (Puccinia triticina; Synonym: Puccinia recondita f. sp. tritici; Bayer code PUCCRT):

Wheat plants (variety 'Yuma') were grown from seed in a greenhouse in soil-less potting mix until the first leaf was fully emerged, with 7-10 seedlings per pot. These plants were inoculated with an aqueous spore suspension of *Puccinia triticina* after fungicide treatments. After inoculation, the plants were kept in a dark dew room with 100% relative humidity overnight to permit spores to germinate and infect the leaf. The plants were then transferred to a greenhouse for disease to develop. Fungicide formulation, application and disease assessment followed the procedures as described in the Example A.

### Example C: Evaluation of Fungicidal Activity: Asian Soybean Rust (Phakopsora pachyrhizi; Bayer code PHAKPA):

Technical grades of materials were dissolved in acetone, which were then mixed with nine volumes of H₂O containing 0.011% Tween 20. The fungicide solutions were applied onto soybean seedlings using an automated booth sprayer to run-off. All sprayed plants were allowed to air dry prior to further handling.

Soybean plants (variety 'Williams 82') were grown in soil-less potting mix, with one plant per pot. Ten-day-old seedlings were used for testing. Plants were inoculated as described in example A. Plants were incubated for 24 h in a dark dew room with 100% relative humidity then transferred to a growth room for disease to develop. Fungicide formulation and application were made as described in the Example A. When disease symptoms were fully expressed, disease severity was assessed on the sprayed leaves on a scale of 0 to 100 percent. Percent disease control was calculated using the ratio of disease severity on treated plants relative to untreated plants.

### Example D: Evaluation of Fungicidal Activity: Leaf Blotch of Barley (Rhynchosporium secalis; Bayer code RHYNSE):

Barley plants (variety 'Harrington') were grown from seed in a greenhouse in soil-less potting mix until the first leaf was fully emerged, with 7-10 seedlings per pot. These plants were inoculated with an aqueous spore suspension of *Rhynchosporium secalis* after fungicide treatments. After inoculation the plants were kept in a dark dew room with 100% relative humidity for two days to permit spores to germinate and infect the leaf. The plants were then transferred to a greenhouse for disease to develop. Fungicide formulation and application were made as described in the Example A. Disease assessment was conducted as described in Example A.

### Example E: Evaluation of Fungicidal Activity: Spot Blotch of Barley (Cochliobolus sativus; Bayer code COCHSA):

Barley seedlings (variety Harrington) were propagated in soil-less potting mix, with each pot having 8 to 12 plants, and used for testing when first leaf was fully emerged. Test plants were inoculated with a spore suspension of *Cochliobolus sativus* 24 hr after fungicide treatments. After inoculation the plants were kept in 100% relative humidity for two days to permit spores to germinate and infect the leaf. The plants were then transferred to a greenhouse for disease to develop. Fungicide formulation, application and disease assessment followed the procedures as described in Example A.

**Table 1. Compound Structure, Preparation Method, and Appearance**

| **Cmpd. No.** | **Structure** | **As Prepared According To** | **Appearance** |
|---|---|---|---|
| 1 | 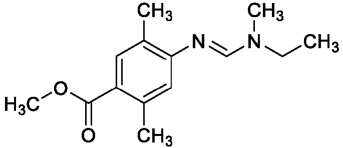 | Example 9A; Example 10A. | Orange liquid |
| 3 | 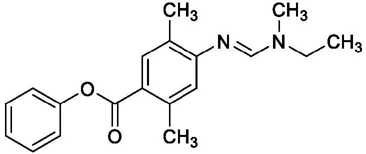 | Example 9A; Example 10A. | Clear colorless oil |
| 4 | 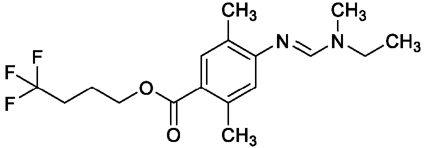 | Example 9A; Example 10A. | Clear colorless oil |
| 5 | 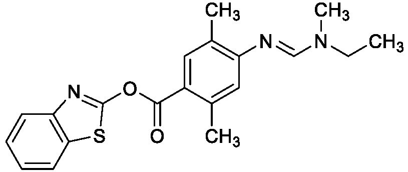 | Example 9A; Example 10A. | Yellow solid |
| 6 | 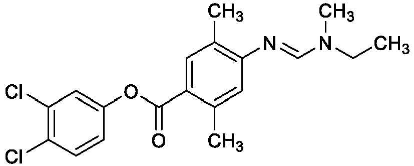 | Example 9A; Example 10A. | Yellow oil |
| 7 | 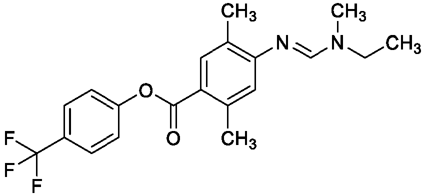 | Example 9A; Example 10A. | White solid |
| 8 | 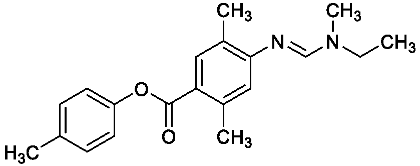 | Example 9A; Example 10A. | Clear colorless oil |
| 9 | 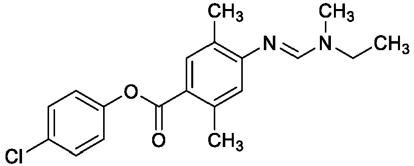 | Example 9A; Example 10A. | White solid |
| 10 | 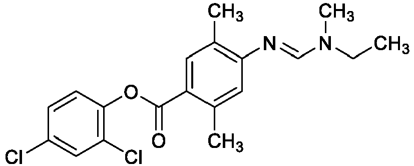 | Example 9A; Example 10A. | Yellow solid |
| 11 | 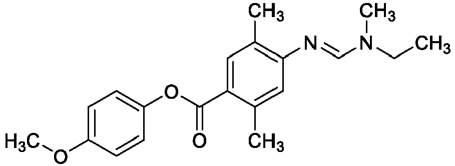 | Example 9A; Example 10A. | Orange solid |
| 12 | 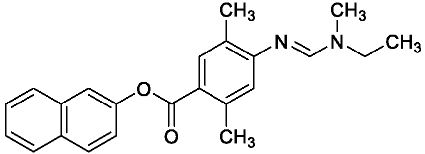 | Example 9A; Example 10A. | Yellow oil |
| 13 | 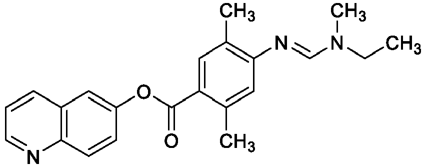 | Example 9A; Example 10A. | Yellow oil |
| 14 | 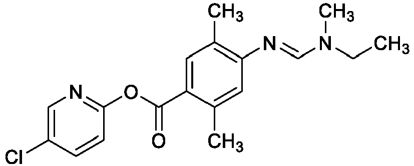 | Example 9A; Example 10A. | Yellow oil |
| 15 | 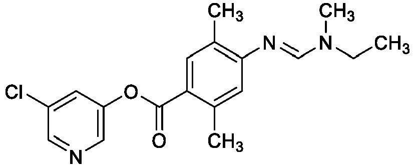 | Example 9A; Example 10A. | Yellow oil |
| 16 | 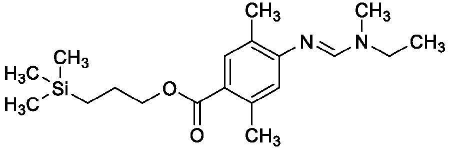 | Example 9A; Example 10A. | Orange oil |
| 17 | 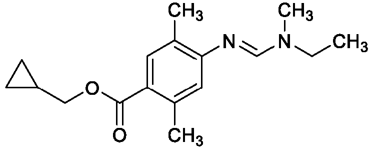 | Example 9A; Example 10A. | Orange oil |
| 18 | 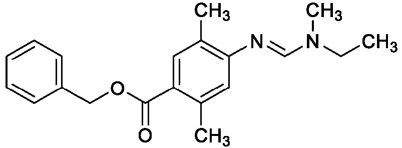 | Example 9A; Example 10A. | Orange oil |
| 19 | 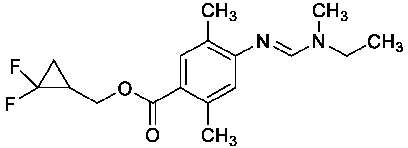 | Example 9A; Example 10A. | Orange oil |
| 20 | 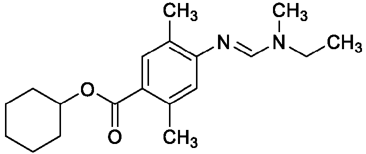 | Example 9A; Example 10A. | Orange oil |
| 21 | 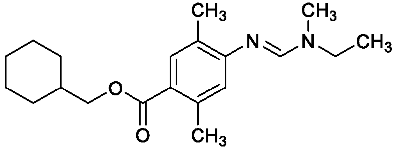 | Example 9A; Example 10A. | Orange oil |
| 22 | 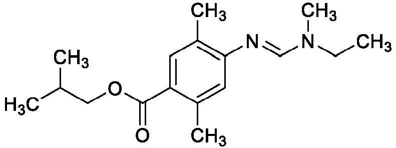 | Example 9A; Example 10A. | Orange oil |
| 23 | 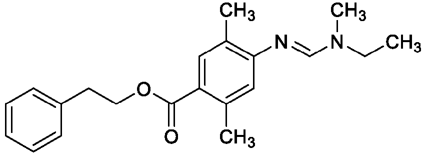 | Example 9A; Example 10A. | Dark oil |
| 24 | 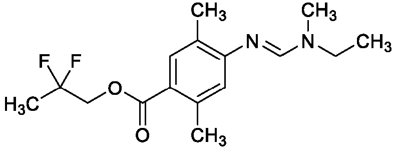 | Example 9A; Example 10A. | Orange oil |
| 25 | 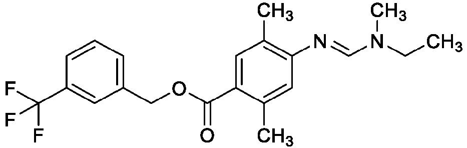 | Example 9B; Example 10A. | Yellow oil |
| 26 | 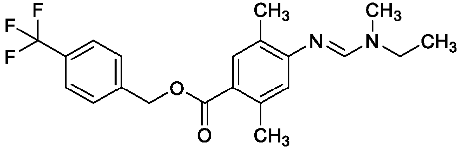 | Example 9A; Example 10A. | Yellow oil |
| 27 | 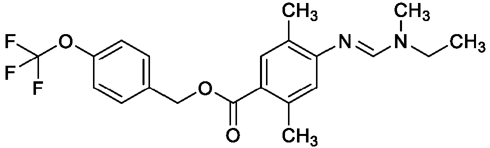 | Example 9A; Example 10A. | Yellow oil |
| 28 | 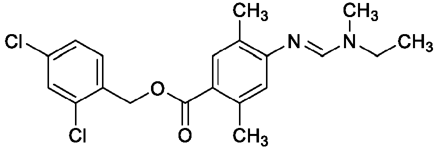 | Example 9A; Example 10A. | Yellow oil |
| 29 | 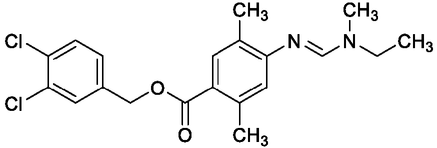 | Example 9A; Example 10A. | Yellow oil |
| 30 | 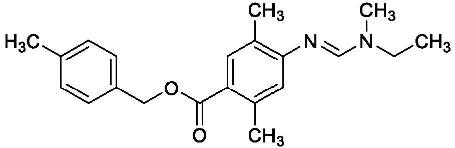 | Example 9B; Example 10A. | Orange oil |
| 31 | 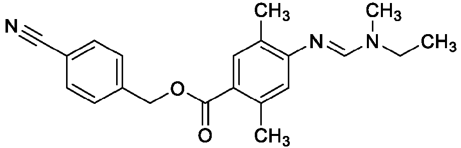 | Example 9A; Example 10A. | White semisolid |
| 32 | 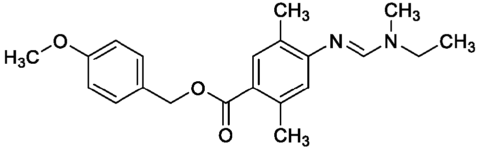 | Example 9A; Example 10A. | Yellow oil |
| 33 | 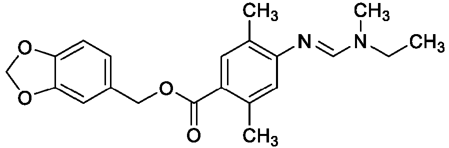 | Example 9A; Example 10A. | Yellow oil |
| 34 | 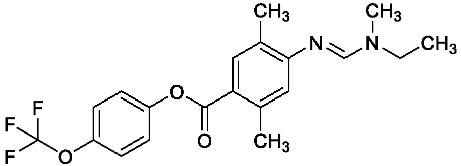 | Example 9A; Example 10A. | White solid |
| 35 | 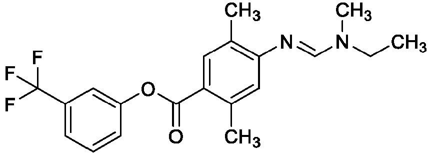 | Example 9A; Example 10A. | Yellow oil |
| 36 | 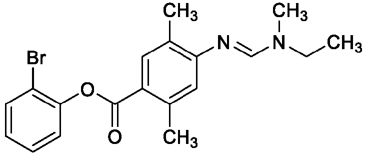 | Example 9A; Example 10A. | Yellow oil |
| 37 | 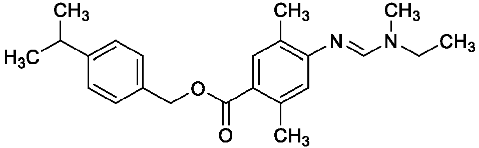 | Example 9A; Example 10A. | Brown oil |
| 38 | 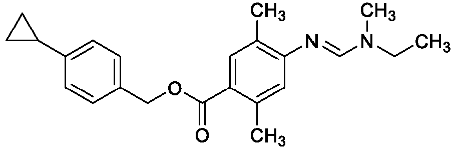 | Example 9A; Example 10A. | Brown oil |
| 39 | 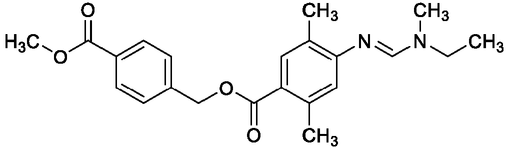 | Example 9A; Example 10A. | Brown oil |
| 40 | 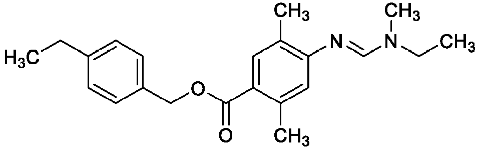 | Example 9A; Example 10A. | Brown oil |
| 41 | 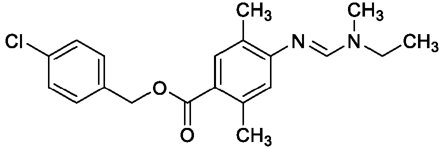 | Example 9A; Example 10A. | Brown oil |
| 42 | 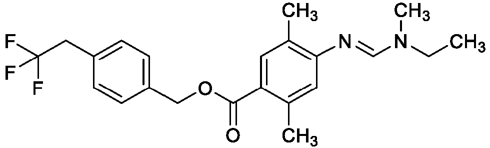 | Example 9A; Example 10A. | Brown oil |
| 43 | 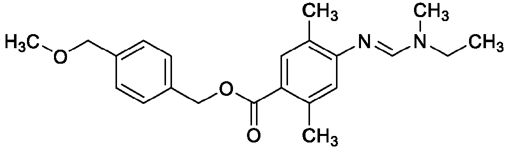 | Example 9A; Example 10A. | Brown oil |
| 44 | 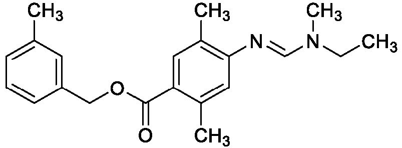 | Example 9B; Example 10A. | Brown oil |
| 45 | 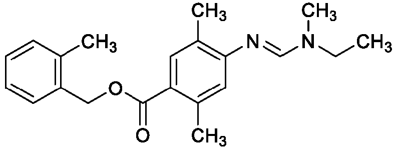 | Example 9B; Example 10A. | Brown oil |
| 46 | 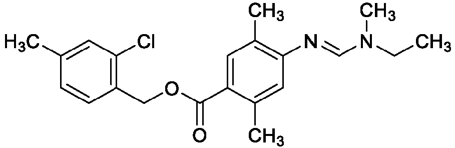 | Example 9A; Example 10A. | Brown oil |
| 47 | 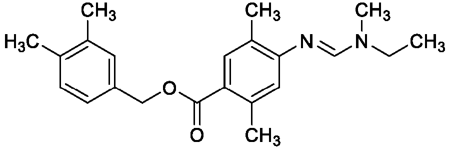 | Example 9A; Example 10A. | Brown oil |
| 48 | 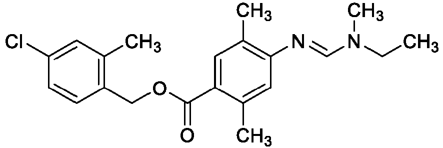 | Example 9A; Example 10A. | Brown oil |
| 49 | 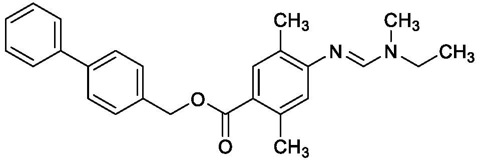 | Example 9A; Example 10A. | Brown oil |
| 50 | 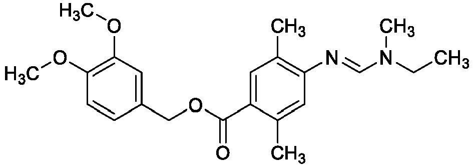 | Example 9A; Example 10A. | Brown oil |
| 51 | 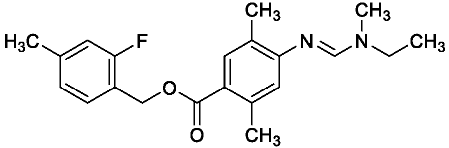 | Example 9A; Example 10A. | Brown oil |
| 52 | 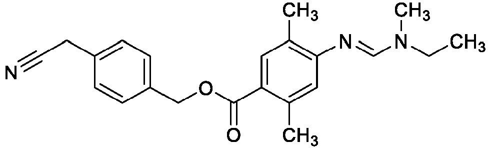 | Example 9A; Example 10A. | Brown oil |
| 53 | 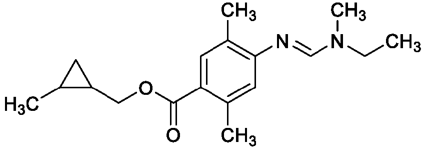 | Example 9A; Example 10A. | Clear colorless oil |
| 54 | 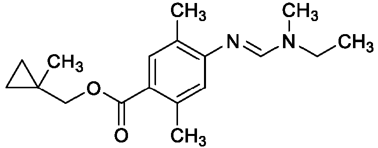 | Example 9A; Example 10A. | Clear colorless oil |
| 55 | 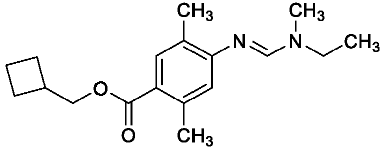 | Example 9A; Example 10A. | Clear colorless oil |
| 56 | 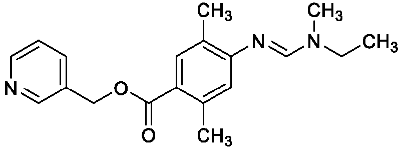 | Example 9A; Example 10A. | Clear colorless oil |
| 57 | 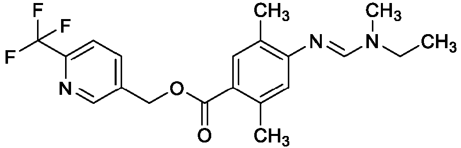 | Example 9A; Example 10A. | Clear colorless oil |
| 58 | 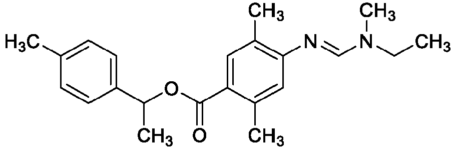 | Example 9C; Example 10A. | Brown oil |
| 59 | 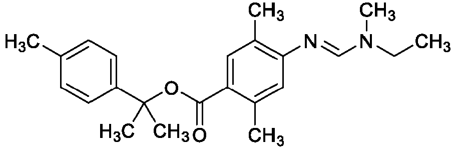 | Example 9C; Example 10A. | Brown oil |
| 60 | 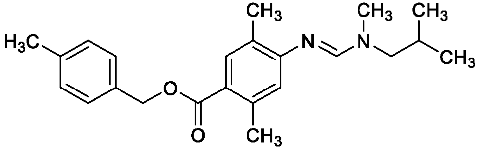 | Example 9B; Example 10B. | Dark brown semisolid |
| 61 | 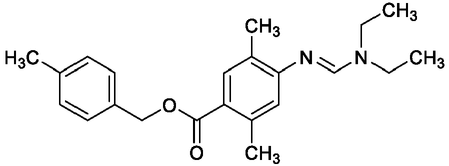 | Example 9B; Example 10B. | Brown oil |
| 62 | 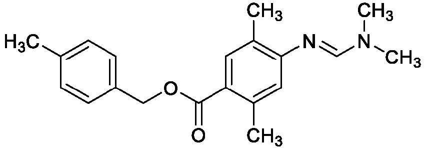 | Example 9B; Example 10B. | Orange oil |
| 63 | 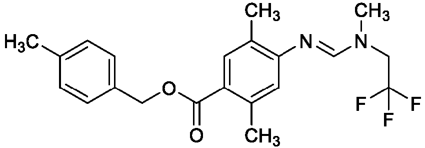 | Example 9B; Example 10B. | Yellow oil |
| 64 | 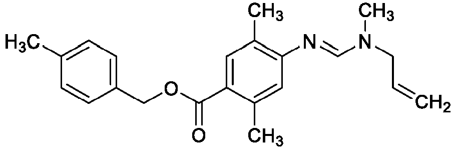 | Example 9B; Example 10B. | Orange oil |
| 65 | 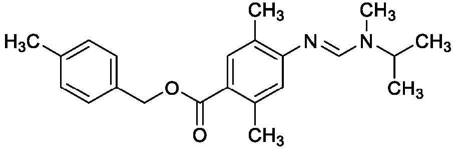 | Example 9B; Example 10B. | Yellow oil |
| 66 | 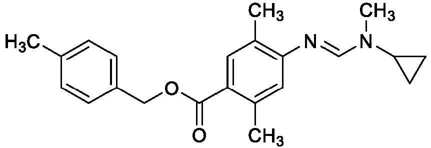 | Example 9B; Example 10B. | Orange oil |
| 67 | 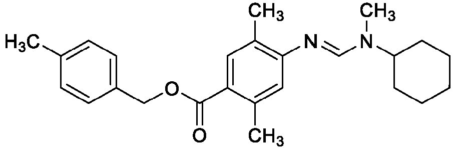 | Example 9B; Example 10B. | Off white semisolid |
| 68 | 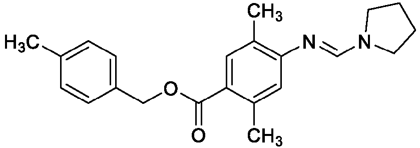 | Example 9B; Example 10B. | Brown oil |
| 69 | 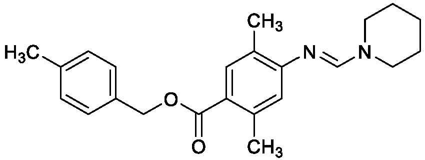 | Example 9B; Example 10B. | Orange oil |
| 70 | 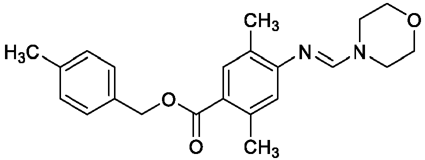 | Example 9B; Example 10B. | Yellow oil |
| 72 | 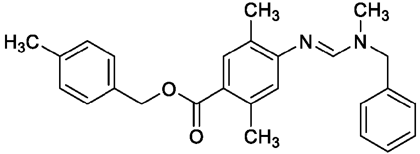 | Example 9B; Example 10B. | Yellow oil |
| 73 | 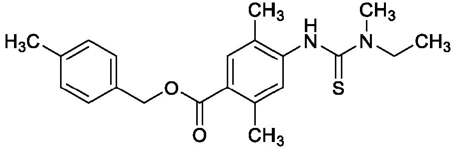 | Example 9B; Example 10D. | Off white semisolid |
| 74 | 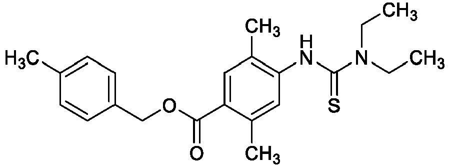 | Example 9B; Example 10D. | White semisolid |
| 75 | 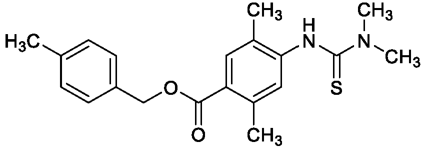 | Example 9B; Example 10D. | White semisolid |
| 76 | 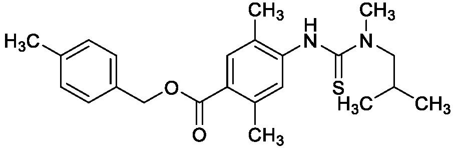 | Example 9B; Example 10D. | Pale rose semisolid |
| 77 | 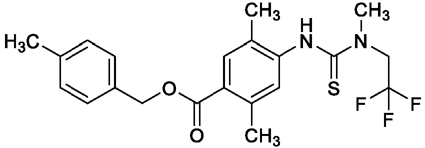 | Example 9B; Example 10D. | White semisolid |
| 78 | 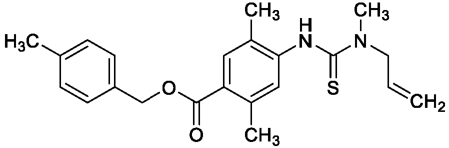 | Example 9B; Example 10D. | White semisolid |
| 79 | 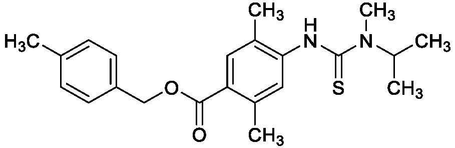 | Example 9B; Example 10D. | White semisolid |
| 80 | 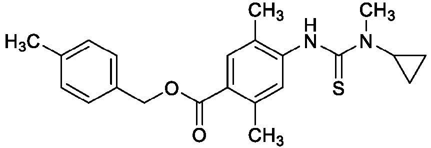 | Example 9B; Example 10D. | White semisolid |
| 81 | 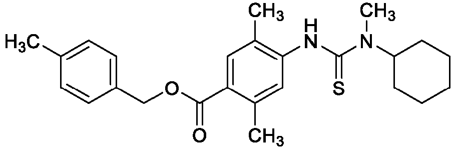 | Example 9B; Example 10D. | Clear colorless oil |
| 82 | 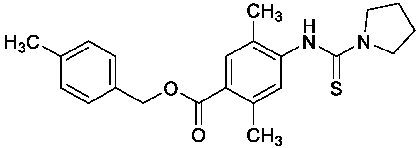 | Example 9B; Example 10D. | White semisolid |
| 83 | 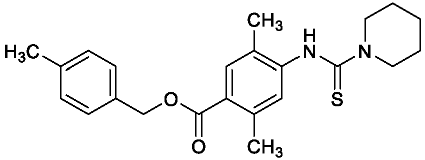 | Example 9B; Example 10D. | White semisolid |
| 84 | 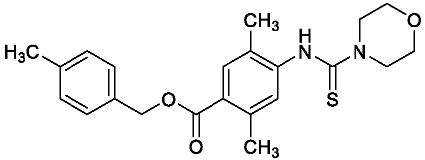 | Example 9B; Example 10D. | White semisolid |
| 86 | 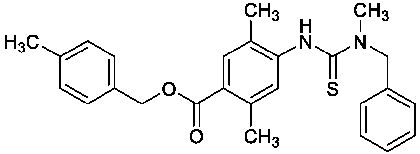 | Example 9B; Example 10D. | White semisolid |
| 89 | 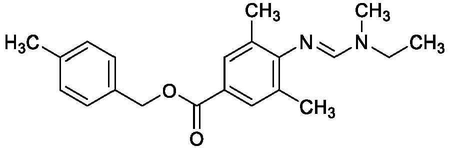 | Example 9A; Example 10A. | Yellow oil |
| 90 | 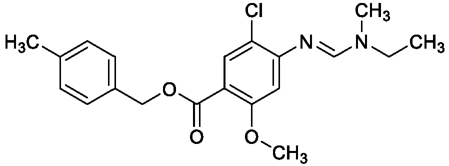 | Example 9A; Example 10A. | Orange oil |
| 91 | 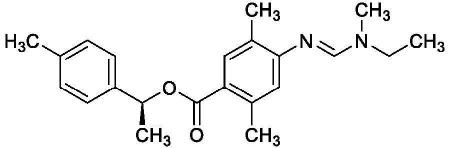 | Example 8B; Example 9C; Example 10A. | Orange oil |
| 92 | 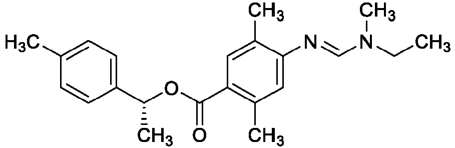 | Example 8B; Example 9C; Example 10A. | Orange oil |
| 94 | 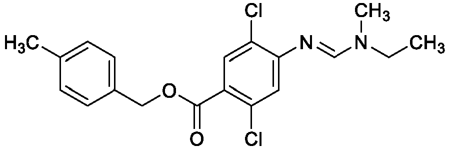 | Example 9A; Example 10A. | Brown oil |
| 95 | 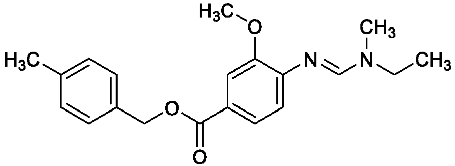 | Example 9A; Example 10A. | Yellow oil |
| 96 | 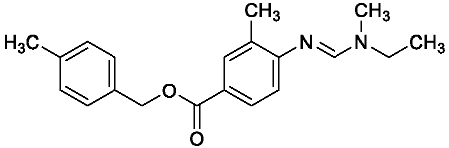 | Example 9A; Example 10A. | Off white semisolid |
| 97 | 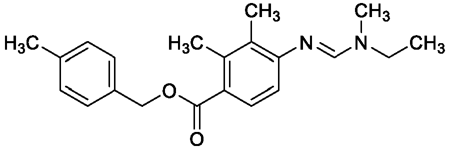 | Example 9B; Example 10A. | Yellow oil |
| 98 | 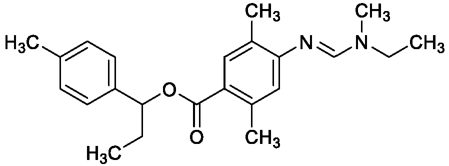 | Example 8A; Example 9C; Example 10A. | Yellow oil |
| 99 | 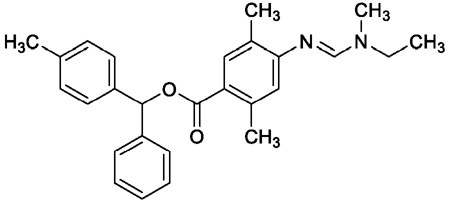 | Example 8A; Example 9C; Example 10A. | Yellow oil |
| 100 | 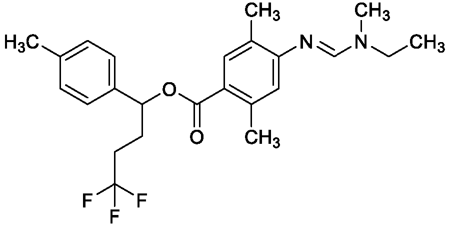 | Example 8A; Example 9C; Example 10A. | Yellow oil |
| 101 | 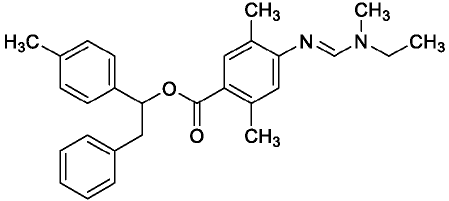 | Example 8A; Example 9C; Example 10A. | Yellow oil |
| 103 | 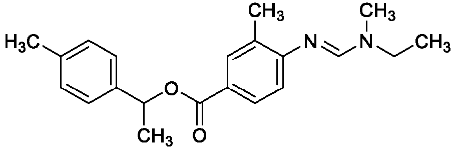 | Example 8A; Example 9C; Example 10A. | Yellow oil |
| 105 | 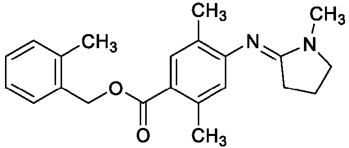 | Example 9B; Example 10C. | Pale yellow sticky liquid |
| 106 | 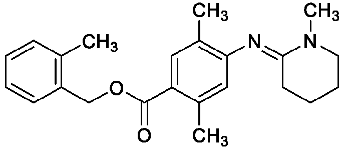 | Example 9B; Example 10C. | Off white solid |
| 107 | 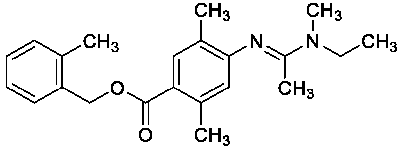 | Example 9B; Example 10C. | Pale yellow gummy liquid |
| 109 | 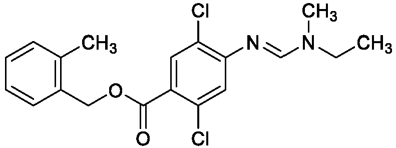 | Example 9A; Example 10A. | Pale yellow semisolid |
| 110 | 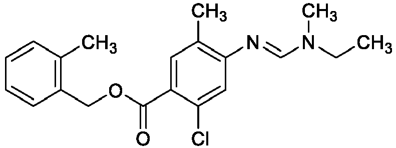 | Example 9A; Example 10A. | Brown gummy liquid |
| 111 | 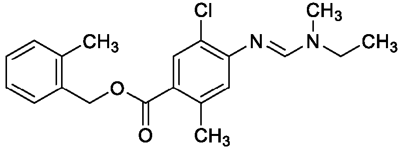 | Example 9A; Example 10A. | Pale brown liquid |
| 112 | 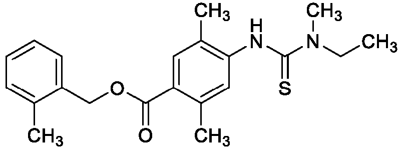 | Example 9B; Example 10D. | White semisolid |
| 113 | 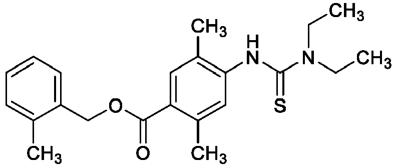 | Example 9B; Example 10D. | White semisolid |
| 114 | 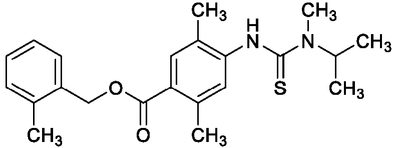 | Example 9B; Example 10D. | White semisolid |
| 115 | 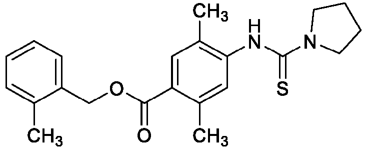 | Example 9B; Example 10D. | White semisolid |
| 116 | 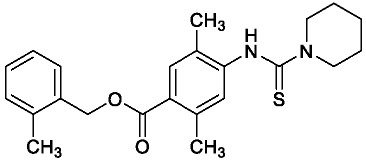 | Example 9B; Example 10D. | White semisolid |
| 117 | 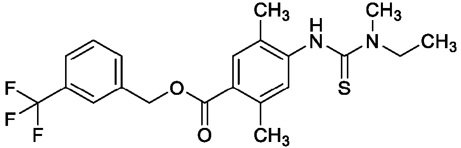 | Example 9B; Example 10D. | White semisolid |
| 118 | 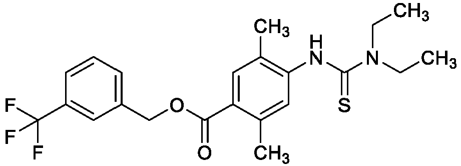 | Example 9B; Example 10D. | White semisolid |
| 119 | 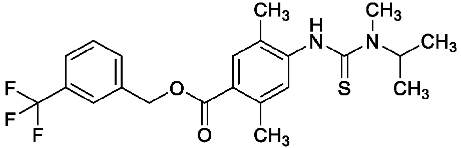 | Example 9B; Example 10D. | White semisolid |
| 120 | 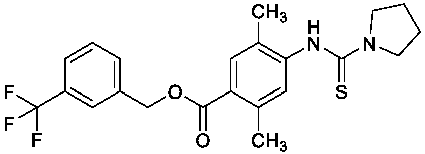 | Example 9B; Example 10D. | White semisolid |
| 121 | 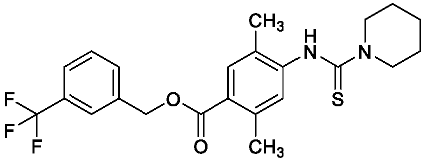 | Example 9B; Example 10D. | White semisolid |
| 122 | 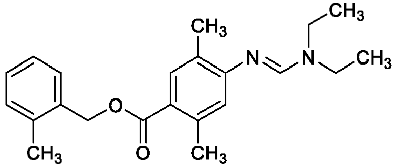 | Example 9B; Example 10B. | Pale yellow oil |
| 123 | 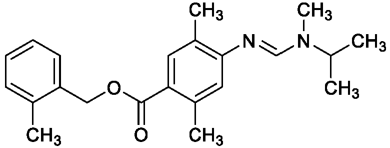 | Example 9B; Example 10B. | Pale yellow oil |
| 124 | 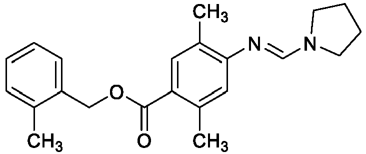 | Example 9B; Example 10B. | Pale yellow oil |
| 125 | 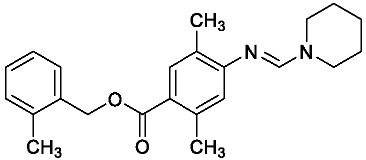 | Example 9B; Example 10B. | Pale yellow oil |
| 126 | 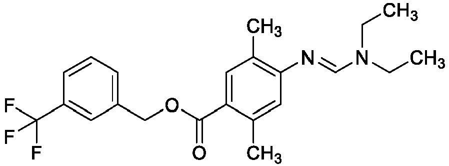 | Example 9B; Example 10B. | Clear colorless oil |
| 127 | 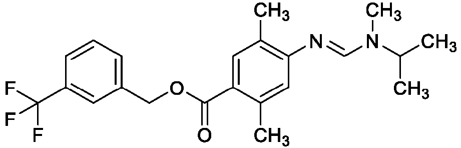 | Example 9B; Example 10B. | Clear colorless oil |
| 128 | 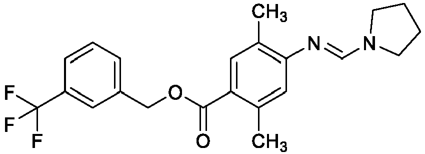 | Example 9B; Example 10B. | Pale yellow oil |
| 129 | 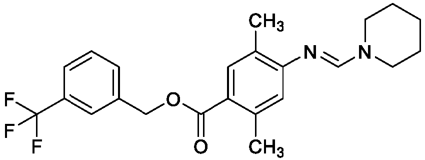 | Example 9B; Example 10B. | Pale yellow oil |
| 130 | 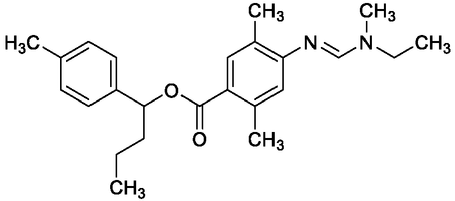 | Example 8A; Example 9C; Example 10A. | Orange oil |
| 131 | 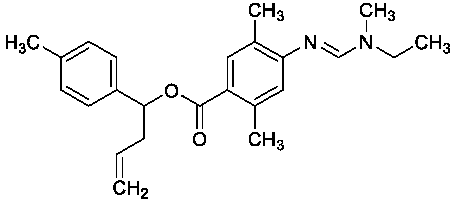 | Example 8A; Example 9C; Example 10A. | Orange oil |
| 132 | 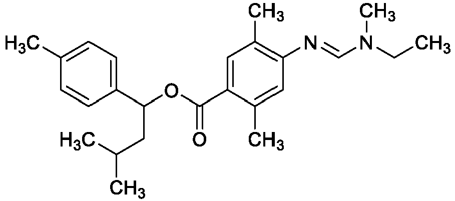 | Example 8A; Example 9C; Example 10A. | Orange oil |
| 133 | 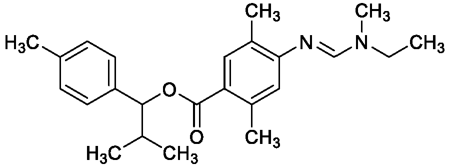 | Example 8A; Example 9C; Example 10A. | Orange oil |
| 134 | 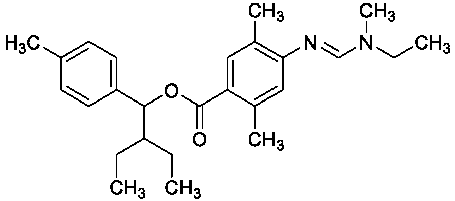 | Example 8A; Example 9C; Example 10A. | Orange oil |
| 135 | 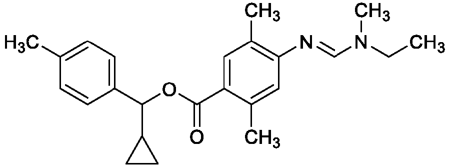 | Example 8A; Example 9C; Example 10A. | Orange oil |
| 136 | 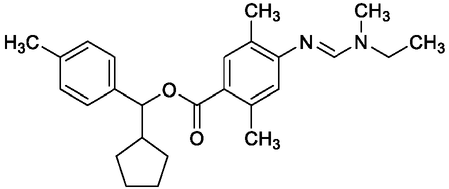 | Example 8A; Example 9C; Example 10A. | Orange oil |
| 137 | 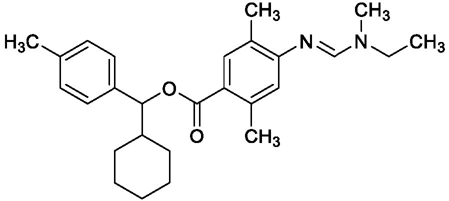 | Example 8A; Example 9C; Example 10A. | Orange oil |
| 138 | 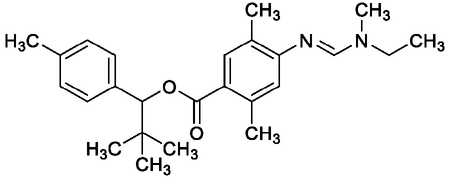 | Example 8A; Example 9C; Example 10A. | Orange oil |
| 139 | 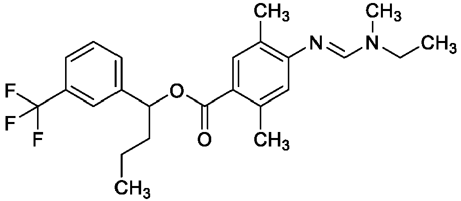 | Example 8A; Example 9C; Example 10A. | Orange oil |
| 140 | 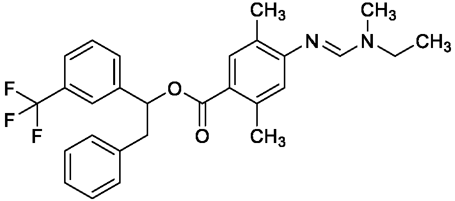 | Example 8A; Example 9C; Example 10A. | Orange oil |
| 141 | 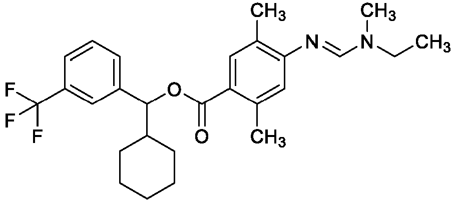 | Example 8A; Example 9C; Example 10A. | Orange oil |
| 142 | 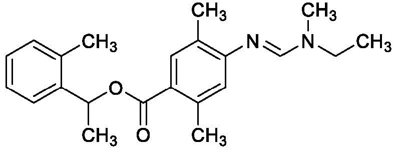 | Example 8A; Example 9C; Example 10A. | Orange oil |
| 143 | 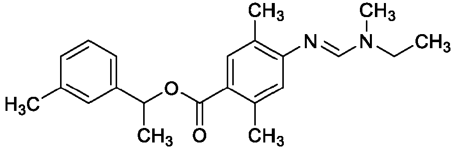 | Example 8A; Example 9C; Example 10A. | Orange oil |
| 144 | 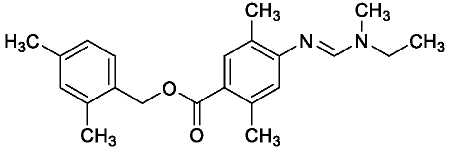 | Example 9A; Example 10A. | Pale yellow oil |
| 145 | 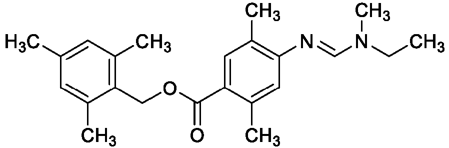 | Example 9A; Example 10A. | Off white solid |
| 146 | 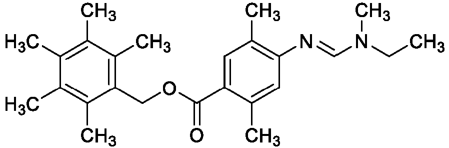 | Example 9A; Example 10A. | Waxy pale yellow semisolid |
| 147 | 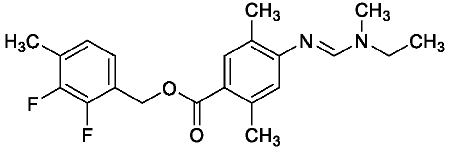 | Example 9A; Example 10A. | Pale yellow oil |
| 148 | 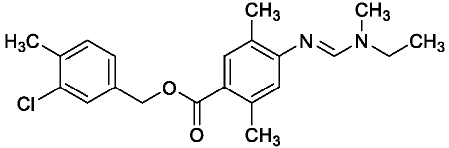 | Example 9A; Example 10A. | Pale yellow oil |
| 149 | 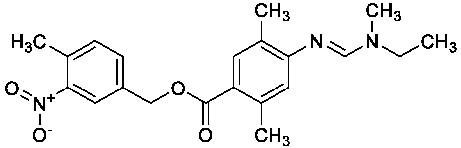 | Example 9A; Example 10A. | Brown oil |
| 150 | 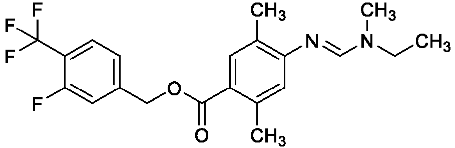 | Example 9A; Example 10A. | Brown solid |
| 151 | 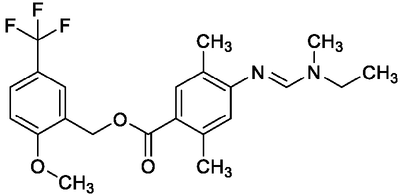 | Example 9A; Example 10A. | Brown solid |
| 152 | 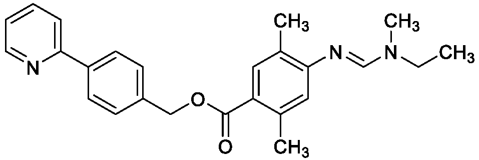 | Example 9A; Example 10A. | Brown oil |
| 153 | 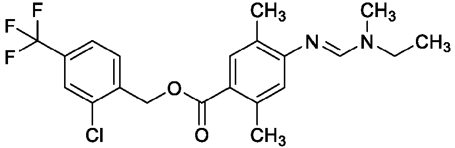 | Example 9A; Example 10A. | Brown oil |
| 154 | 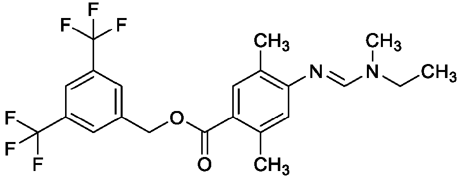 | Example 9A; Example 10A. | Brown solid |
| 155 | 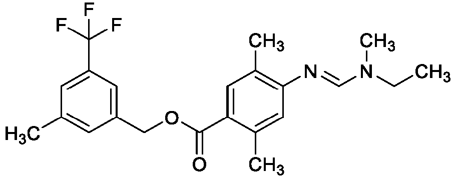 | Example 9A; Example 10A. | Tan solid |
| 156 | 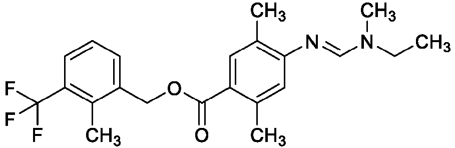 | Example 9A; Example 10A. | Light brown solid |
| 157 | 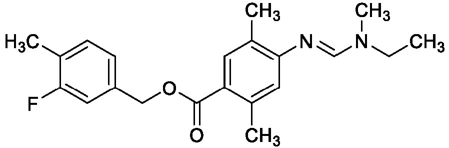 | Example 9A; Example 10A. | Brown oil |
| 158 | 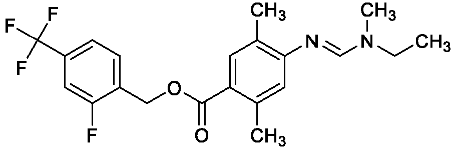 | Example 9A; Example 10A. | Light brown semisolid |
| 159 | 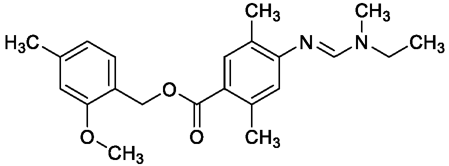 | Example 9A; Example 10A. | Light brown oil |
| 160 | 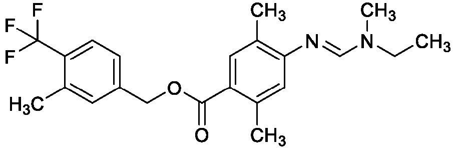 | Example 9A; Example 10A. | Tan semisolid |
| 161 | 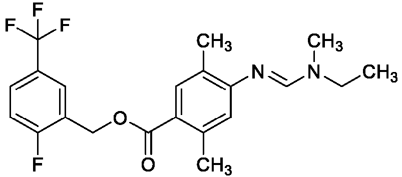 | Example 9A; Example 10A. | Tan solid |
| 162 | 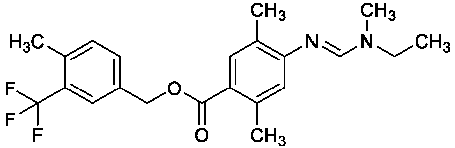 | Example 9A; Example 10A. | Off white solid |
| 163 | 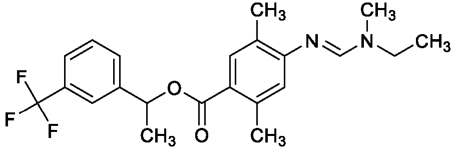 | Example 9C; Example 10A. | Brown oil |
| 164 | 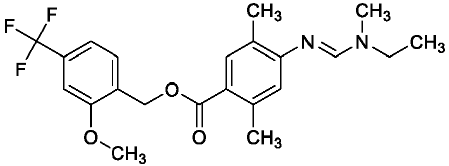 | Example 9A; Example 10A. | Light brown oil |
| 165 | 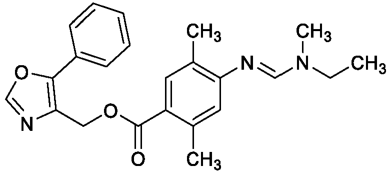 | Example 9A; Example 10A. | Light brown oil |
| 166 | 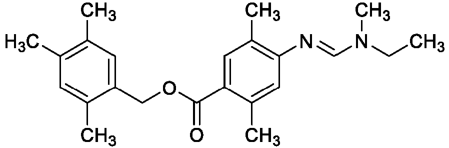 | Example 9A; Example 10A. | Brown oil |
| 167 | 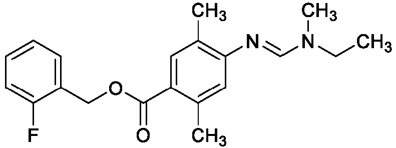 | Example 9B; Example 10A. | Brown oil |
| 168 | 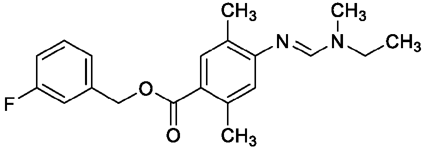 | Example 9A; Example 10A. | Clear oil |
| 169 | 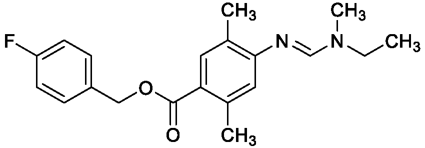 | Example 9A; Example 10A. | Brown oil |
| 170 | 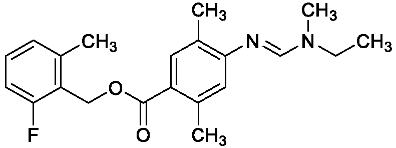 | Example 9A; Example 10A. | Brown oil |
| 171 | 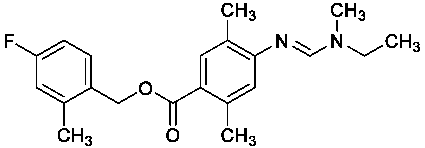 | Example 9A; Example 10A. | Off white solid |
| 172 | 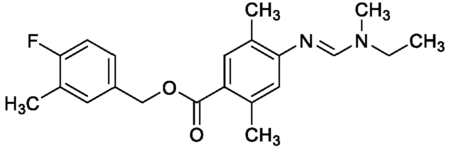 | Example 9A; Example 10A. | Brown oil |
| 173 | 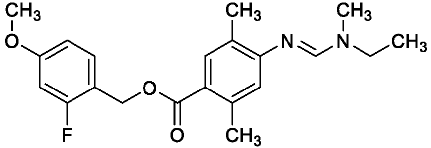 | Example 9A; Example 10A. | Brown oil |
| 174 | 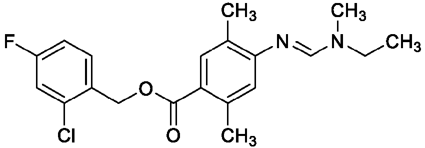 | Example 9A; Example 10A. | Brown oil |
| 175 | 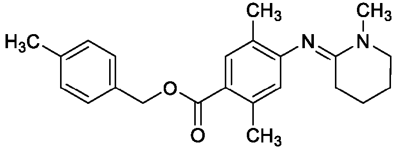 | Example 9A; Example 10C. | Yellow oil |
| 176 | 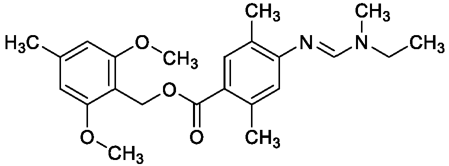 | Example 9A; Example 10A. | Pale yellow solid |
| 177 | 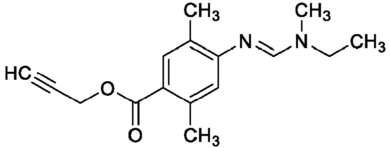 | Example 9A; Example 10A. | Light brown solid |
| 178 | 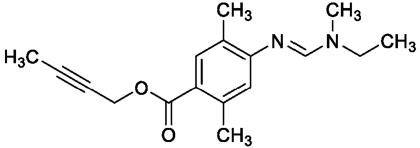 | Example 9A; Example 10A. | Pale yellow oil |
| 179 | 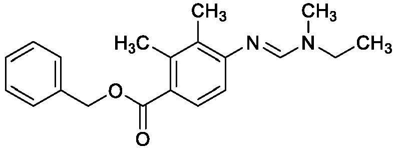 | Example 9A; Example 10A. | Yellow oil |
| 180 | 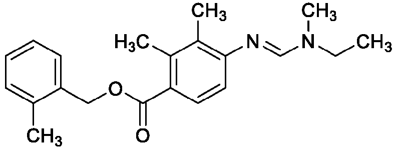 | Example 9A; Example 10A. | Yellow solid |
| 181 | 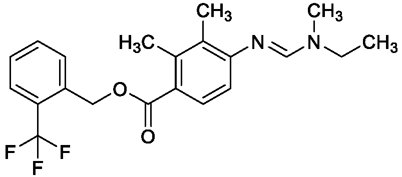 | Example 9A; Example 10A. | Yellow oil |
| 182 | 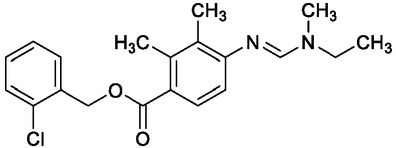 | Example 9A; Example 10A. | Yellow oil |
| 183 | 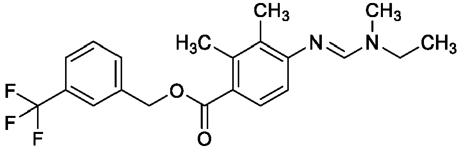 | Example 9B; Example 10A. | Yellow oil |
| 184 | 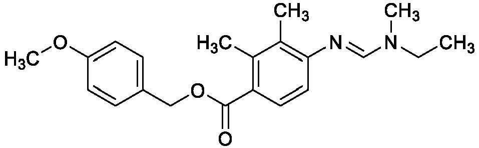 | Example 9A; Example 10A. | Yellow oil |
| 185 | 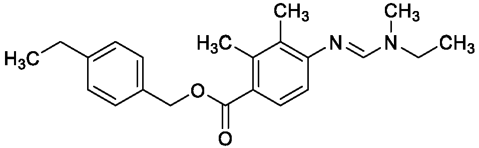 | Example 9A; Example 10A. | Yellow oil |
| 186 | 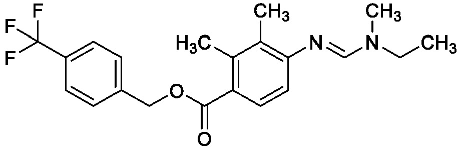 | Example 9A; Example 10A. | Yellow solid |
| 187 | 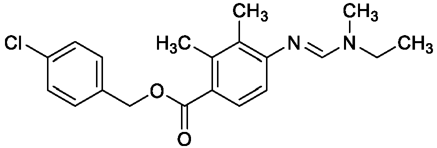 | Example 9A; Example 10A. | Yellow solid |
| 188 | 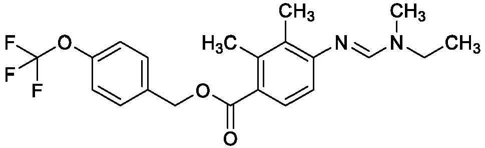 | Example 9A; Example 10A. | Yellow solid |
| 189 | 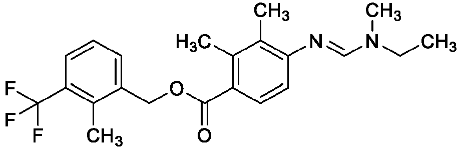 | Example 9A; Example 10A. | Light yellow solid |
| 190 | 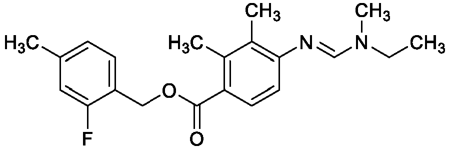 | Example 9A; Example 10A. | Yellow solid |
| 191 | 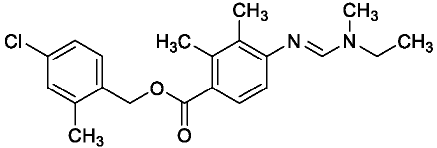 | Example 9A; Example 10A. | Light yellow solid |
| 192 | 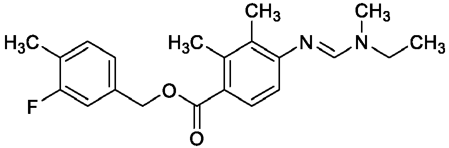 | Example 9A; Example 10A. | Dark yellow-brown oil |
| 193 | 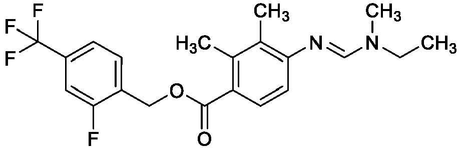 | Example 9A; Example 10A. | Dark yellow solid |
| 194 | 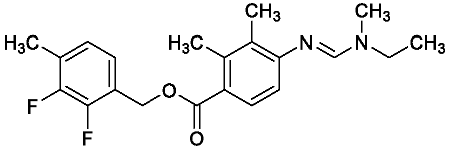 | Example 9A; Example 10A. | Dark yellow solid |
| 195 | 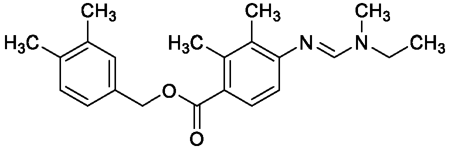 | Example 9A; Example 10A. | Dark yellow oil |
| 196 | 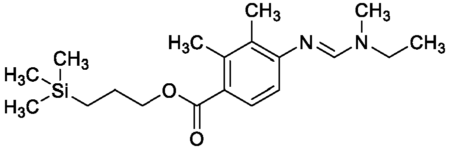 | Example 9A; Example 10A. | Yellow oil |
| 197 | 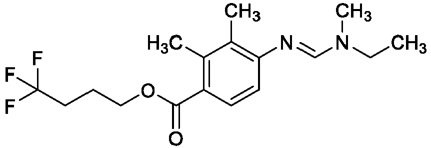 | Example 9A; Example 10A. | Dark brown oil |
| 198 | 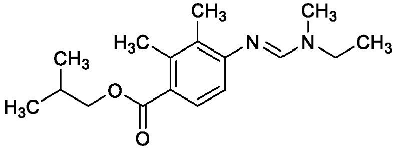 | Example 9A; Example 10A. | Dark brown oil |
| 199 | 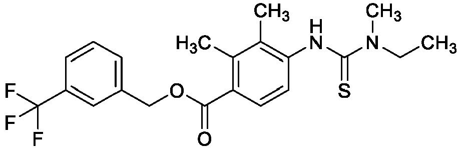 | Example 9B; Example 10D. | Dark yellow oil |
| 200 | 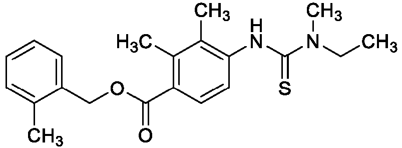 | Example 9A; Example 10D. | Yellow solid |
| 201 | 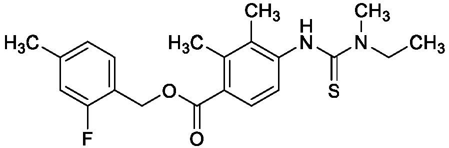 | Example 9A; Example 10D. | Yellow solid |
| 202 | 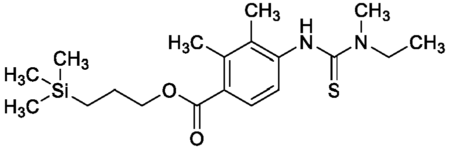 | Example 9A; Example 10D. | Dark yellow oil |
| 203 | 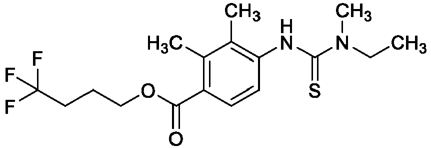 | Example 9A; Example 10D. | Yellow solid |
| 204 | 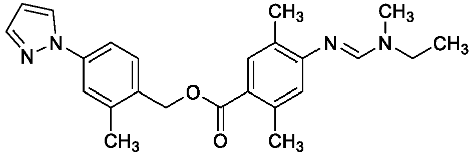 | Example 9A; Example 10A. | Light brown oil |
| 205 | 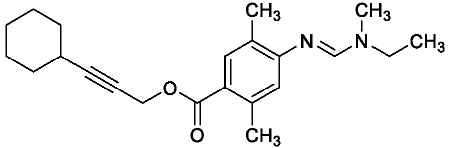 | Example 9A; Example 10A. | Pale yellow oil |
| 206 | 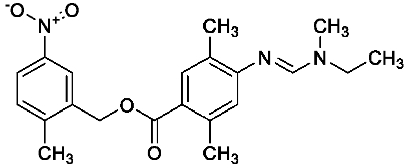 | Example 9A; Example 10A. | Light orange solid |
| 207 | 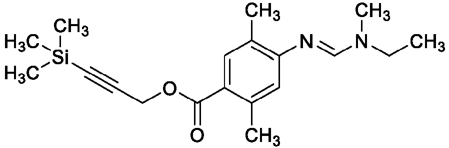 | Example 9A; Example 10A. | Pale yellow oil |
| 208 | 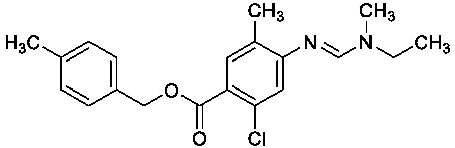 | Example 9A; Example 10A. | Brown oil |
| 209 | 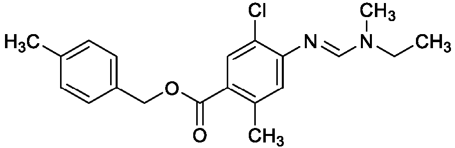 | Example 9A; Example 10A. | Brown oil |
| 210 | 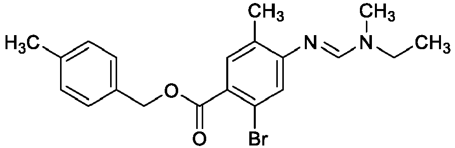 | Example 9A; Example 10A. | Pale brown oil |
| 212 | 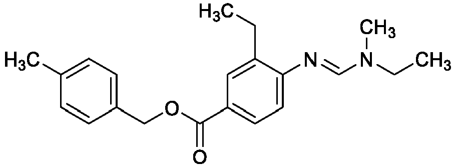 | Example 9A; Example 10A. | Pale yellow oil |
| 213 | 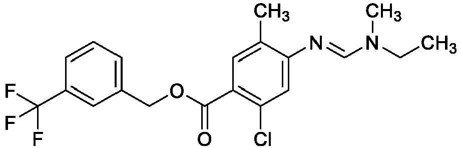 | Example 9A; Example 10A. | Pale yellow oil |
| 214 | 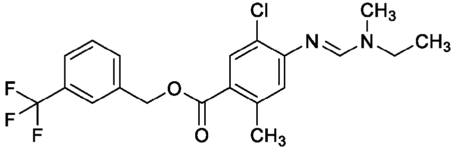 | Example 9A; Example 10A. | Clear colorless oil |
| 215 | 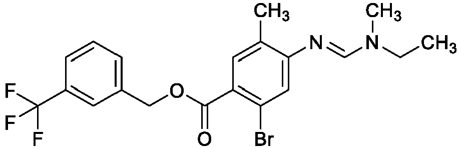 | Example 9A; Example 10A. | White semisolid |
| 217 | 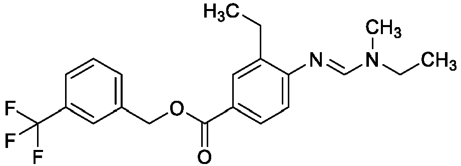 | Example 9A; Example 10A. | Pale yellow oil |
| 218 | 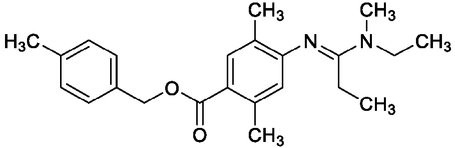 | Example 9B; Example 10C. | Yellow oil |
| 219 | 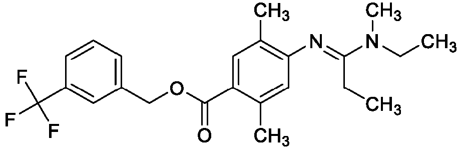 | Example 9B; Example 10C. | Pale yellow oil |
| 220 | 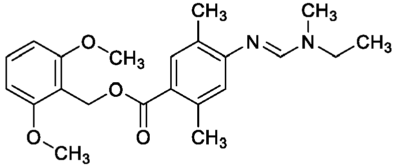 | Example 9A; Example 10A. | Off white solid |
| 221 | 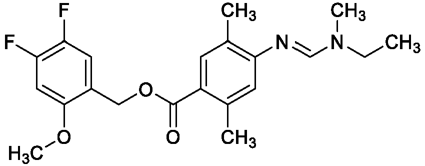 | Example 9A; Example 10A. | Pale yellow oil |
| 222 | 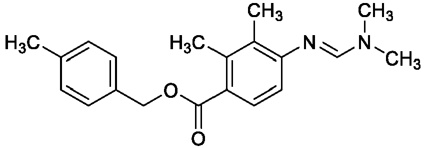 | Example 9B; Example 10B. | Yellow oil |
| 223 | 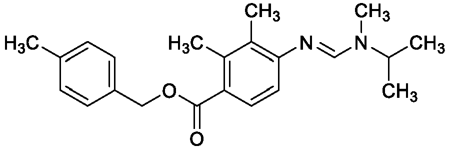 | Example 9B; Example 10B. | Clear oil |
| 224 | 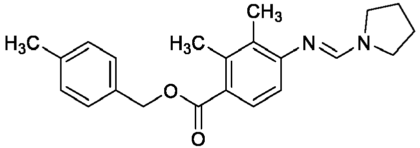 | Example 9B; Example 10B. | Clear oil |
| 225 | 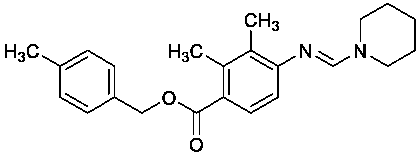 | Example 9B; Example 10B. | Clear oil |
| 226 | 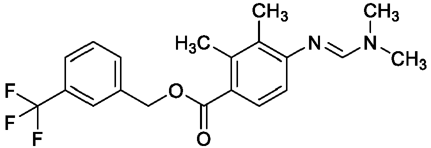 | Example 9B; Example 10B. | Light yellow oil |
| 227 | 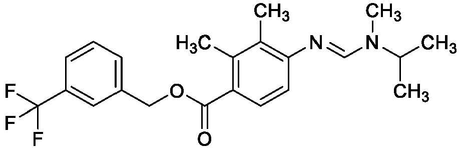 | Example 9B; Example 10B. | Clear oil |
| 228 | 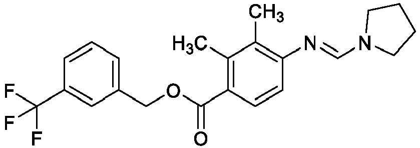 | Example 9B; Example 10B. | Light yellow oil |
| 229 | 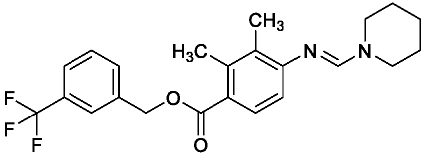 | Example 9B; Example 10B. | Light yellow oil |
| 230 | 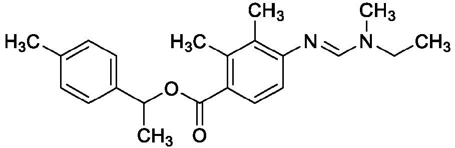 | Example 9C; Example 10A. | Yellow oil |
| 231 | 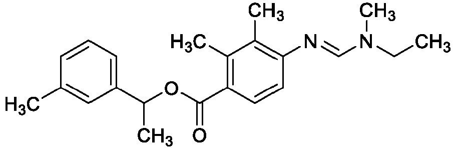 | Example 9C; Example 10A. | Yellow oil |
| 232 | 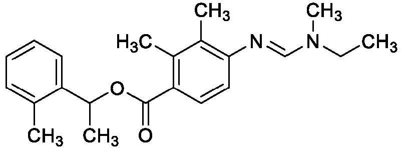 | Example 9C; Example 10A. | Yellow oil |
| 233 | 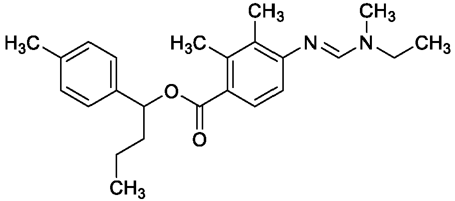 | Example 8A; Example 9C; Example 10A. | Yellow oil |
| 234 | 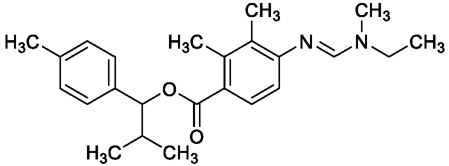 | Example 8A; Example 9C; Example 10A. | Yellow oil |
| 235 | 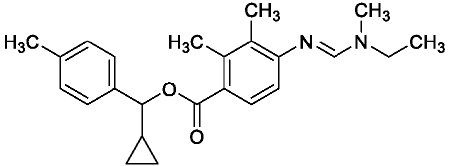 | Example 8A; Example 9C; Example 10A. | Yellow oil |
| 236 | 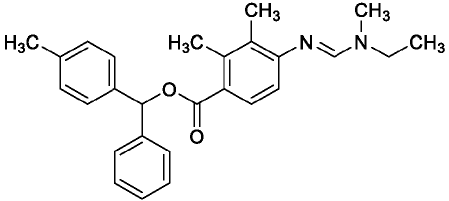 | Example 8A; Example 9C; Example 10A. | Yellow oil |
| 237 | 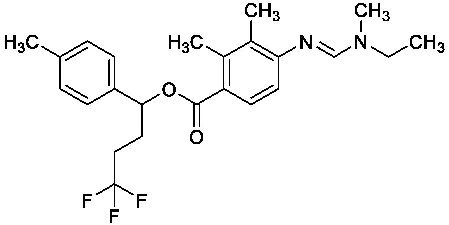 | Example 8A; Example 9C; Example 10A. | Yellow oil |
| 238 | 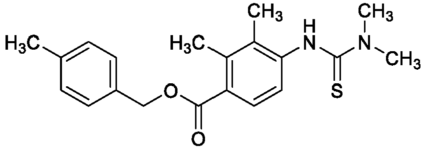 | Example 9B; Example 10D. | Yellow solid |
| 239 | 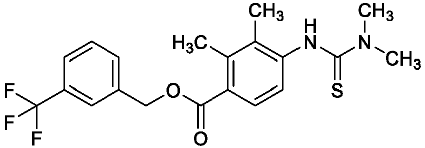 | Example 9B; Example 10D. | Yellow solid |
| 240 | 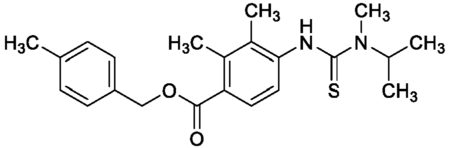 | Example 9B; Example 10D. | Yellow solid |
| 241 | 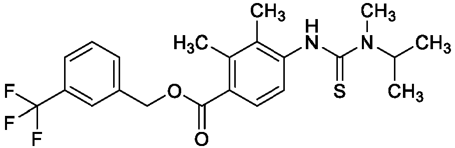 | Example 9B; Example 10D. | Off white solid |
| 242 | 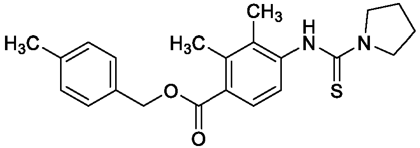 | Example 9B; Example 10D. | Yellow solid |
| 243 | 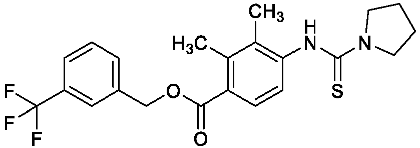 | Example 9B; Example 10D. | White solid |
| 244 | 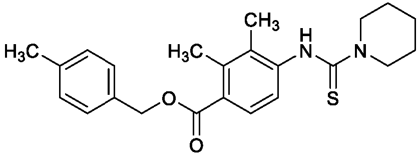 | Example 9B; Example 10D. | Off white solid |
| 245 | 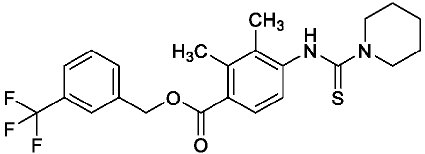 | Example 9B; Example 10D. | Off white solid |
| 246 | 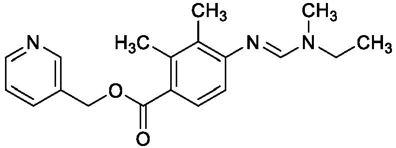 | Example 9A; Example 10A. | Dark yellow oil |
| 247 | 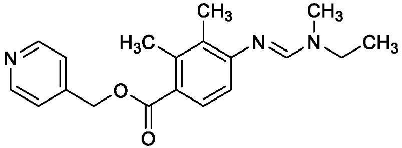 | Example 9A; Example 10A. | Thick yellow oil |
| 248 | 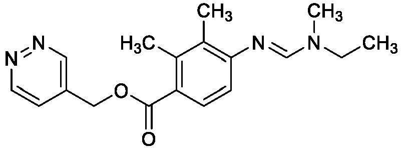 | Example 9A; Example 10A. | Brown oil |
| 249 | 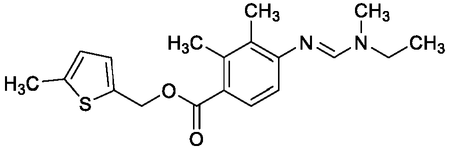 | Example 9A; Example 10A. | Yellow oil |
| 250 | 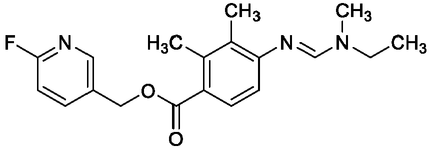 | Example 9A; Example 10A. | Dark yellow solid |
| 251 | 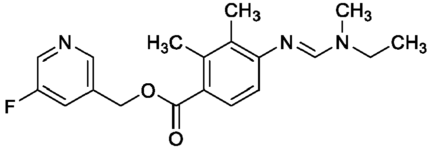 | Example 9A; Example 10A. | Yellow oil |
| 252 | 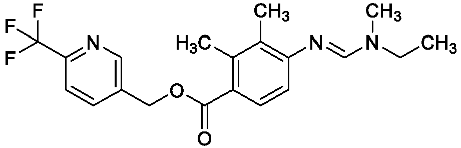 | Example 9A; Example 10A. | Light yellow solid |
| 253 | 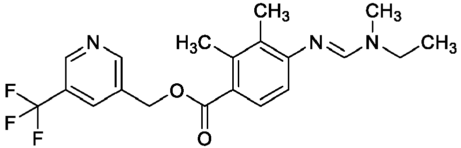 | Example 9A; Example 10A. | Yellow oil |
| 254 | 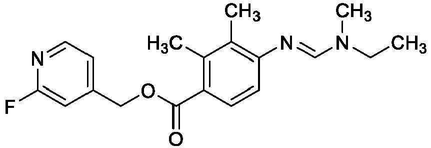 | Example 9A; Example 10A. | Off white solid |
| 255 | 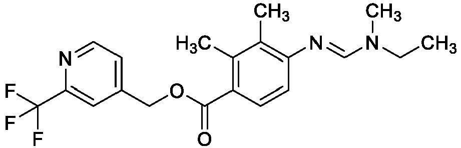 | Example 9A; Example 10A. | Off white solid |
| 256 | 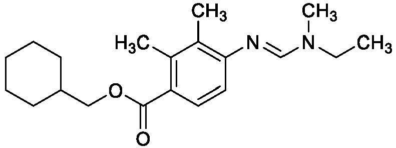 | Example 9A; Example 10A. | Clear oil |
| 257 | 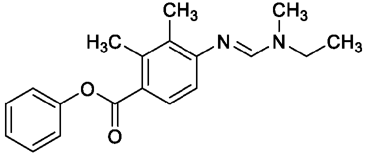 | Example 9A; Example 10A. | Light yellow oil |
| 258 | 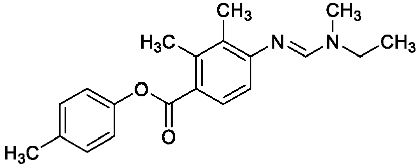 | Example 9A; Example 10A. | Light yellow oil |
| 259 | 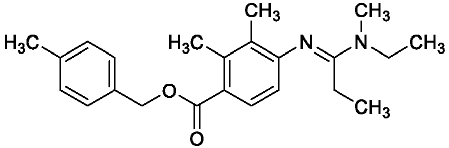 | Example 9B; Example 10C. | Light yellow oil |
| 260 | 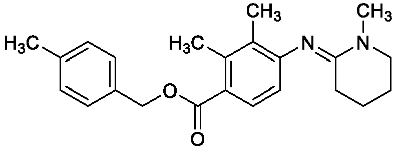 | Example 9B; Example 10C. | White solid |
| 261 | 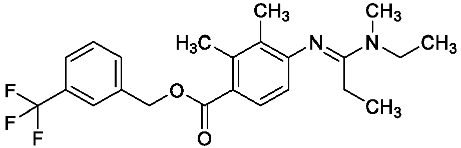 | Example 9B; Example 10C. | Clear oil |
| 262 | 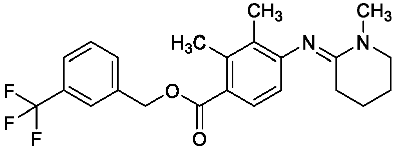 | Example 9B; Example 10C. | Yellow oil |
| 263 | 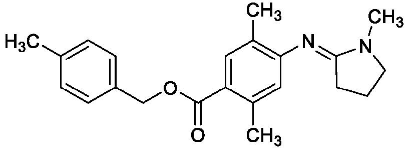 | Example 9B; Example 10C. | Brown oil |
| 264 | 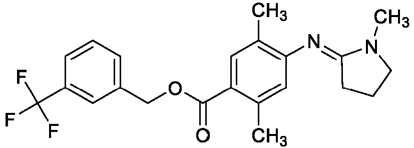 | Example 9B; Example 10C. | Pale yellow oil |
| 265 | 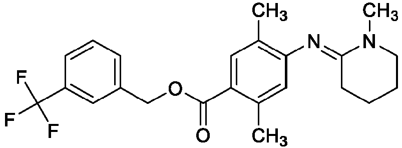 | Example 9B; Example 10C. | Pale yellow oil |
| 266 | 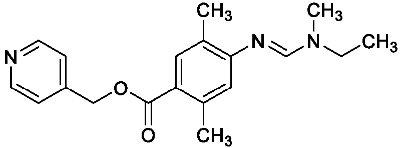 | Example 9A; Example 10A. | Yellow oil |
| 267 | 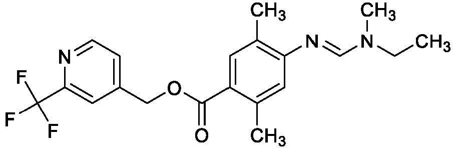 | Example 9A; Example 10A. | Off white solid |
| 268 | 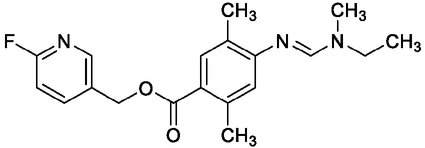 | Example 9A; Example 10A. | Yellow oil |
| 269 | 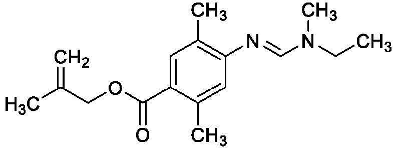 | Example 9A; Example 10A. | Yellow oil |
| 270 | 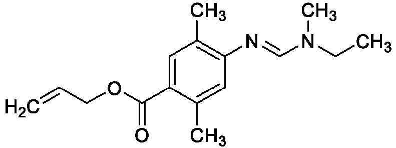 | Example 9A; Example 10A. | Yellow oil |
| 271 | 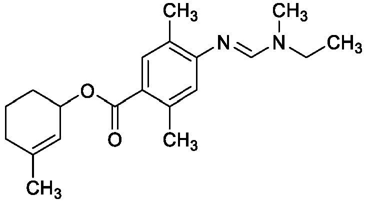 | Example 9A; Example 10A. | Yellow oil |
| 272 | 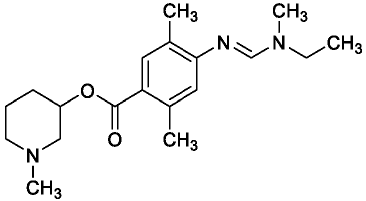 | Example 9A; Example 10A. | Yellow oil |
| 273 | 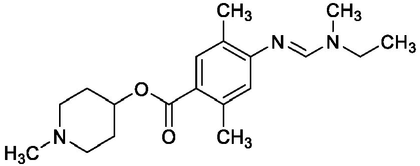 | Example 9A; Example 10A. | Yellow oil |
| 274 | 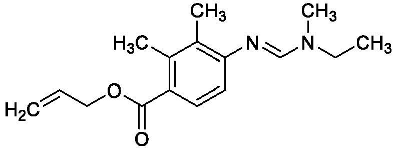 | Example 9A; Example 10A. | Dark yellow oil |
| 275 | 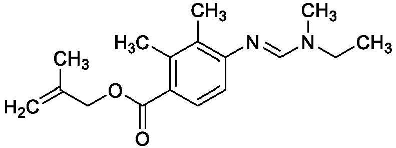 | Example 9A; Example 10A. | Yellow oil |
| 276 | 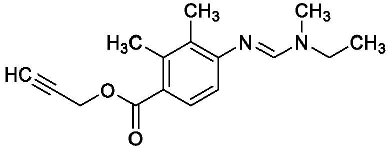 | Example 9A; Example 10A. | Brown oil |
| 277 | 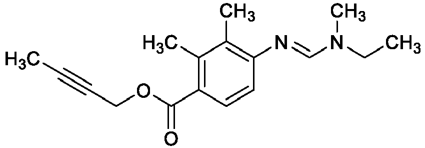 | Example 9A; Example 10A. | Yellow oil |
| 278 | 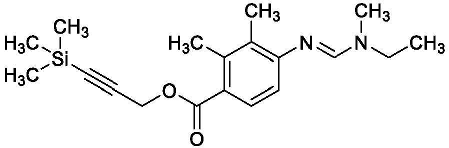 | Example 9A; Example 10A. | Yellow oil |
| 279 | 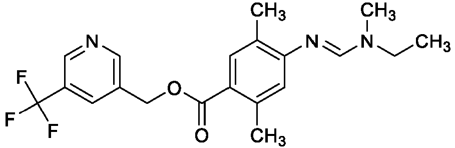 | Example 9A; Example 10A. | Off white solid |
| 280 | 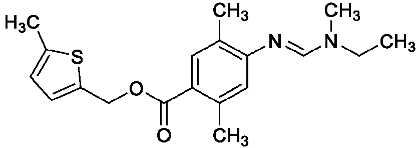 | Example 9A; Example 10A. | Yellow oil |
| 285 | 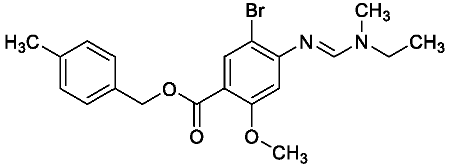 | Example 1B; Example 3B; Example 9A; Example 10A. | Sticky wax |
| 286 | 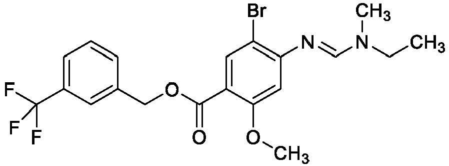 | Example 1B; Example 3B; Example 9A; Example 10A. | White solid |
| 287 | 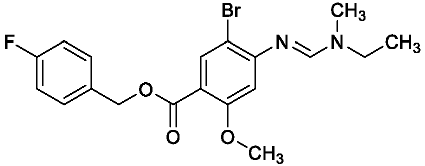 | Example 1B; Example 3B; Example 9A; Example 10A. | Yellow solid |
| 288 | 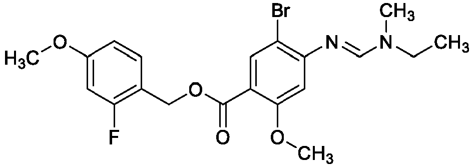 | Example 1B; Example 3B; Example 9A; Example 10A. | White solid |
| 289 | 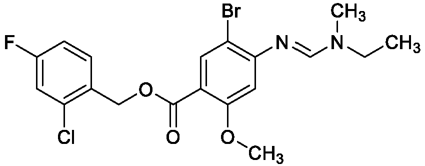 | Example 1B; Example 3B; Example 9A; Example 10A. | White solid |
| 290 | 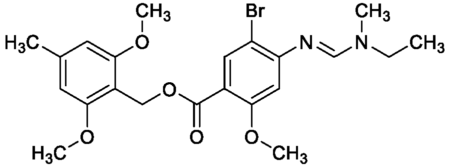 | Example 1B; Example 3B; Example 9A; Example 10A. | White solid |
| 291 | 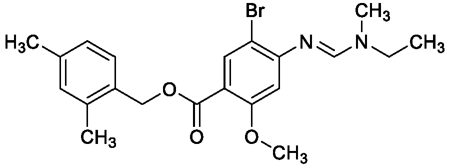 | Example 1B; Example 3B; Example 9A; Example 10A. | White solid |
| 292 | 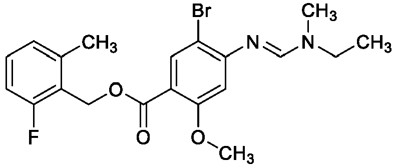 | Example 1B; Example 3B; Example 9A; Example 10A. | Sticky wax |
| 293 | 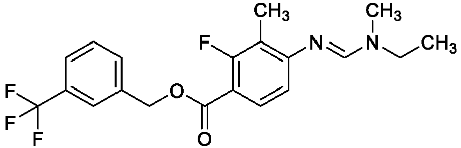 | Example 6A; Example 7A; Example 9A; Example 10A. | Yellow oil |
| 294 | 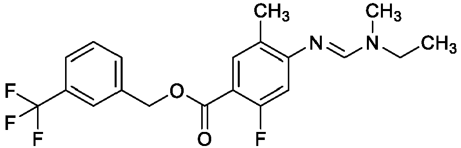 | Example 6A; Example 7A; Example 9A; Example 10A. | Yellow oil |
| 295 | 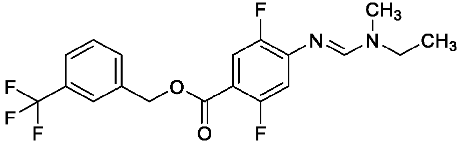 | Example 6A; Example 7A; Example 9A; Example 10A. | Light yellow solid |
| 296 | 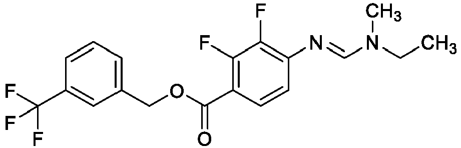 | Example 6A; Example 7A; Example 9A; Example 10A. | Light yellow oil |
| 297 | 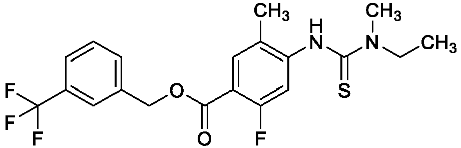 | Example 6A; Example 7A; Example 9A; Example 10D. | White solid |
| 298 | 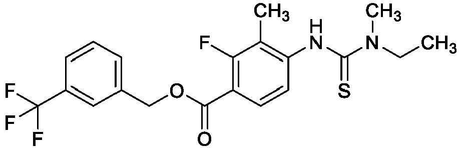 | Example 6A; Example 7A; Example 9A; Example 10D. | White solid |
| 299 | 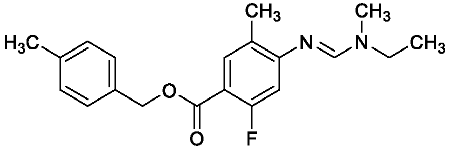 | Example 6A; Example 7A; Example 9A; Example 10A. | Brown oil |
| 300 | 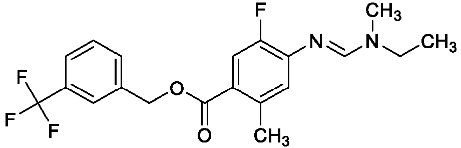 | Example 6A; Example 7A; Example 9A; Example 10A. | Yellow oil |
| 301 | 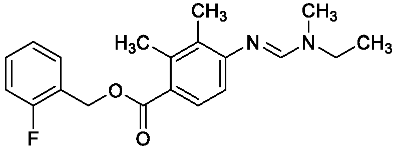 | Example 9B; Example 10A. | Dark orange oily solid |
| 302 | 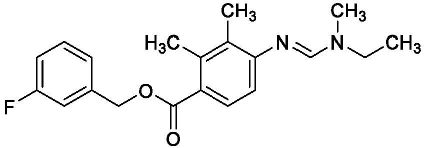 | Example 9A; Example 10A. | Dark orange oily solid |
| 303 | 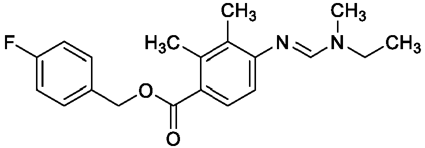 | Example 9A; Example 10A. | Dark orange oil |
| 304 | 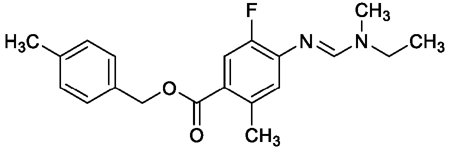 | Example 6A; Example 7A; Example 9A; Example 10A. | Yellow oil |
| 305 | 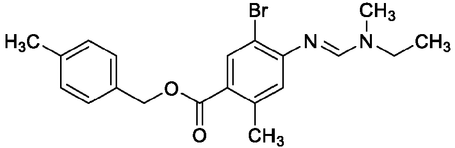 | Example 9A; Example 10A. | Yellow oil |
| 306 | 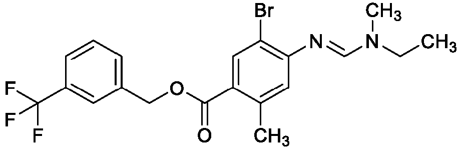 | Example 9A; Example 10A. | White solid |
| 307 | 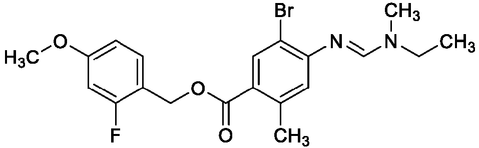 | Example 9A; Example 10A. | Pale yellow oil |
| 308 | 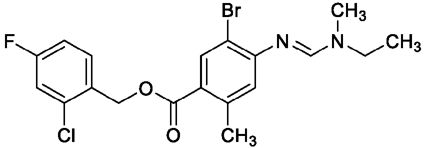 | Example 9A; Example 10A. | White solid |
| 309 | 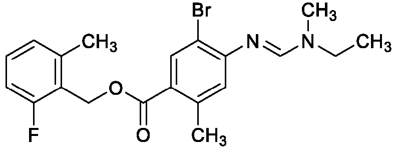 | Example 9A; Example 10A. | Clear oil |
| 310 | 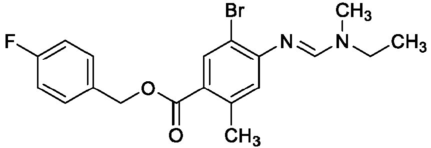 | Example 9A; Example 10A. | White solid |
| 311 | 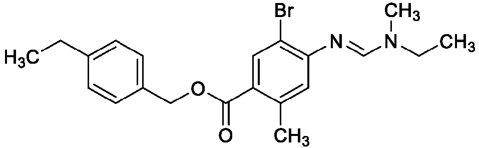 | Example 9A; Example 10A. | Yellow oil |
| 312 | 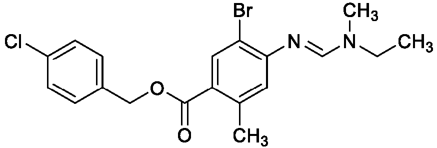 | Example 9A; Example 10A. | Yellow oil |
| 313 | 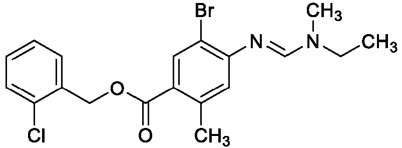 | Example 9A; Example 10A. | White solid |
| 314 | 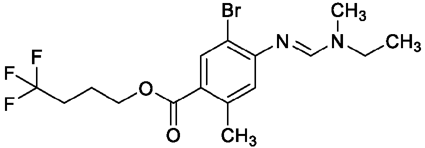 | Example 9A; Example 10A. | Clear oil |
| 315 | 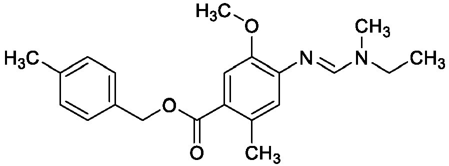 | Example 6C; Example 7B; Example 9A; Example 10A. | Yellow oil |
| 316 | 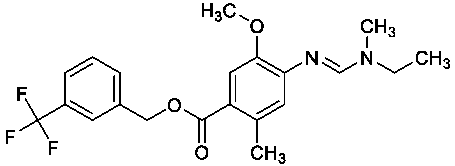 | Example 6C; Example 7B; Example 9A; Example 10A. | Yellow oil |
| 317 | 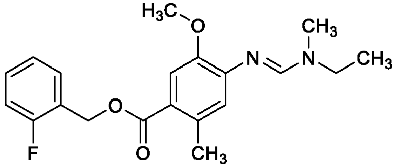 | Example 6C; Example 7B; Example 9A; Example 10A. | Yellow oil |
| 318 | 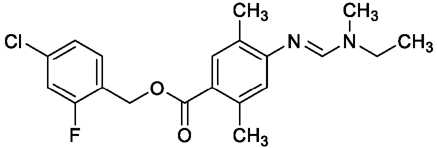 | Example 9A; Example 10A. | Yellow oil |
| 319 | 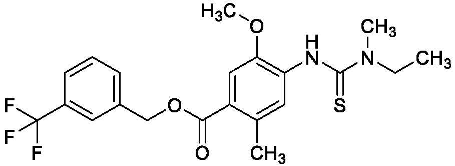 | Example 6C; Example 7B; Example 9A; Example 10D. | Dark yellow oil |
| 320 | 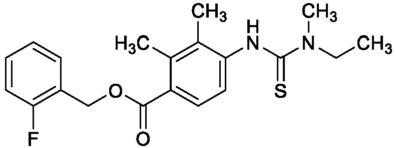 | Example 9B; Example 10D. | Light yellow solid |
| 321 | 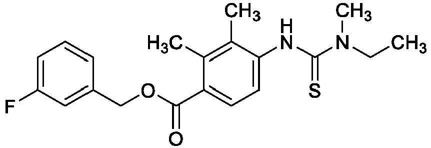 | Example 9A; Example 10D. | Yellow oil |
| 322 | 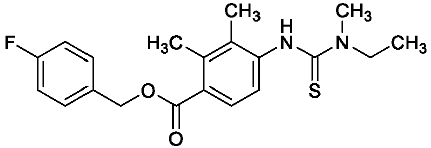 | Example 9A; Example 10D. | Yellow oil |
| 323 | 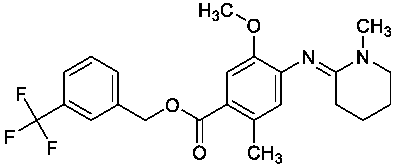 | Example 6C; Example 7B; Example 9A; Example 10C. | Clear rusty brown oil |
| 324 | 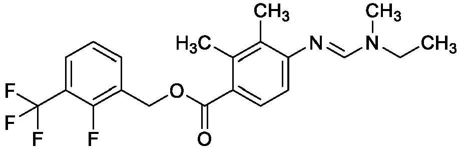 | Example 9A; Example 10A. | White solid |
| 325 | 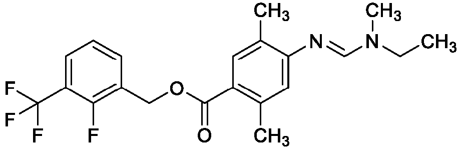 | Example 9A; Example 10A. | Pale yellow solid |
| 326 | 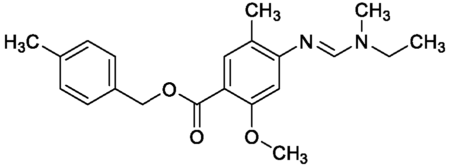 | Example 1B; Example 2B; Example 3B; Example 9A; Example 10A. | Clear colorless oil |
| 327 | 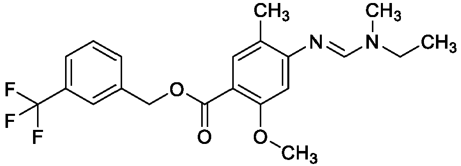 | Example 1B; Example 2B; Example 3B; Example 9A; Example 10A. | Clear colorless oil |
| 328 | 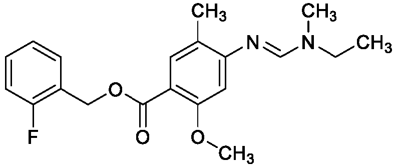 | Example 1B; Example 2B; Example 3B; Example 9A; Example 10A. | Clear colorless oil |
| 329 | 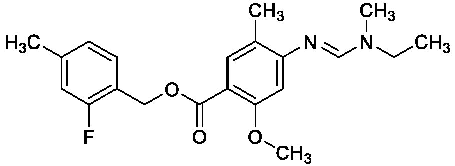 | Example 1B; Example 2B; Example 3B; Example 9A; Example 10A. | Clear colorless oil |
| 330 | 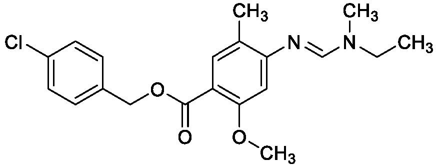 | Example 1B; Example 2B; Example 3B; Example 9A; Example 10A. | Clear colorless oil |
| 331 | 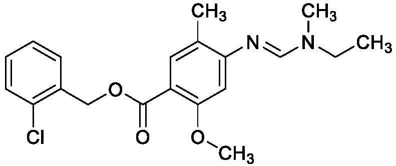 | Example 1B; Example 2B; Example 3B; Example 9A; Example 10A. | Clear colorless oil |
| 332 | 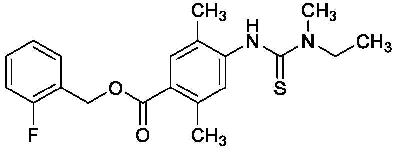 | Example 9B; Example 10D. | Off white solid |
| 333 | 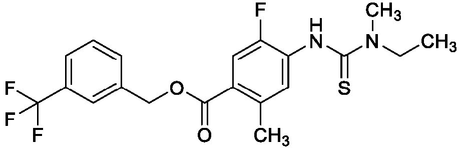 | Example 6A; Example 7A; Example 9A; Example 10D. | White solid |
| 334 | 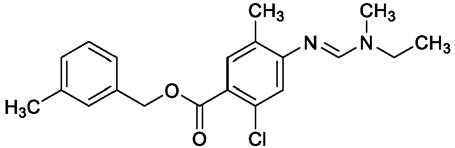 | Example 6A; Example 7A; Example 9A; Example 10A. | Orange liquid |
| 335 | 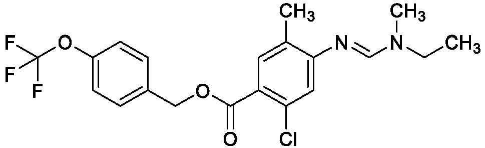 | Example 6A; Example 7A; Example 9A; Example 10A. | White solid |
| 336 | 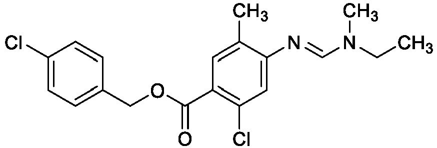 | Example 6A; Example 7A; Example 9A; Example 10A. | Pale brown solid |
| 337 | 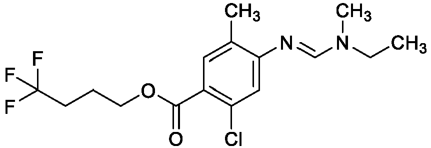 | Example 6A; Example 7A; Example 9A; Example 10A. | Pale brown gummy liquid |
| 338 | 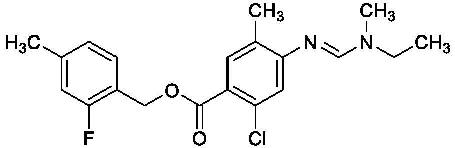 | Example 6A; Example 7A; Example 9A; Example 10A. | Brown solid |
| 339 | 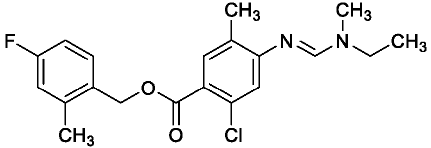 | Example 6A; Example 7A; Example 9A; Example 10A. | Off white solid |
| 340 | 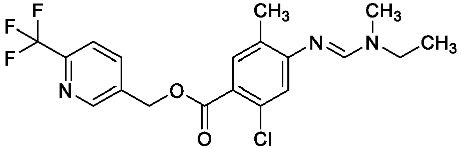 | Example 6A; Example 7A; Example 9A; Example 10A. | Off white solid |
| 341 | 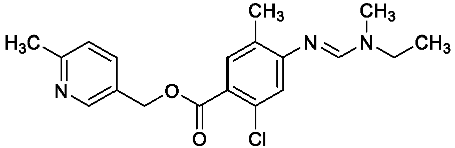 | Example 6A; Example 7A; Example 9A; Example 10A. | Off white solid |
| 342 | 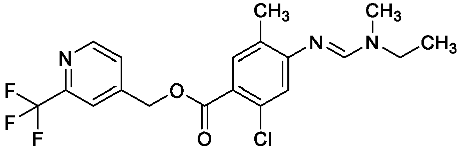 | Example 6A; Example 7A; Example 9A; Example 10A. | Off white solid |
| 343 | 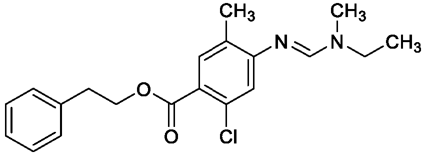 | Example 6A; Example 7A; Example 9A; Example 10A. | Yellow liquid |
| 344 | 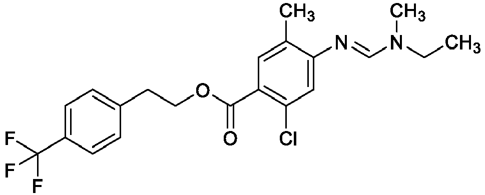 | Example 6A; Example 7A; Example 9A; Example 10A. | Off white solid |
| 345 | 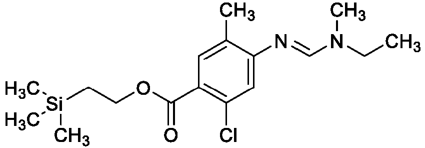 | Example 6A; Example 7A; Example 9A; Example 10A. | Yellow liquid |
| 346 | 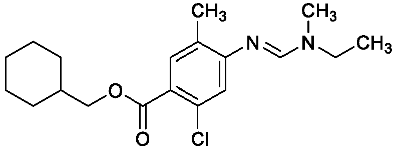 | Example 6A; Example 7A; Example 9A; Example 10A. | Pale reddish gummy liquid |
| 347 | 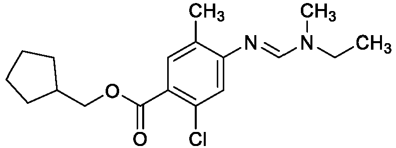 | Example 6A; Example 7A; Example 9A; Example 10A. | Brown solid |
| 348 | 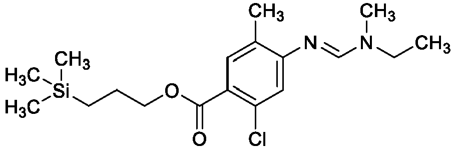 | Example 6A; Example 7A; Example 9A; Example 10A. | Pale yellow liquid |
| 349 | 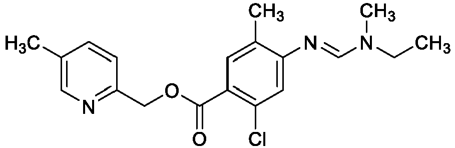 | Example 6A; Example 7A; Example 9A; Example 10A. | Brown gummy liquid |
| 350 | 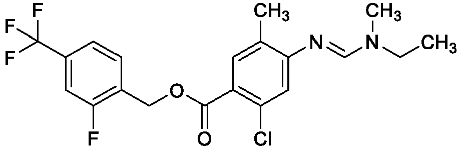 | Example 6A; Example 7A; Example 9A; Example 10A. | Brown liquid |
| 351 | 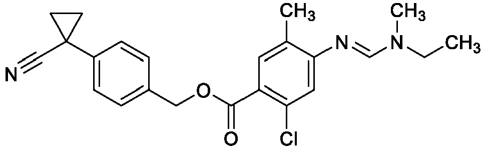 | Example 6A; Example 7A; Example 9A; Example 10A. | Pale brown solid |
| 352 | 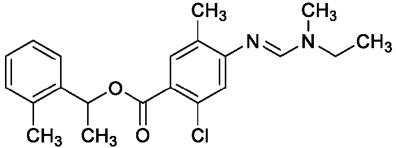 | Example 6A; Example 7A; Example 9C; Example 10A. | Orange gummy liquid |
| 353 | 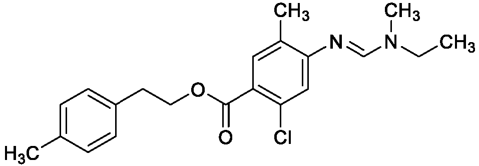 | Example 6A; Example 7A; Example 9A; Example 10A. | Pale orange liquid |
| 354 | 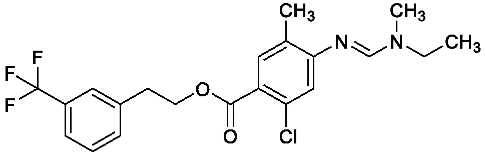 | Example 6A; Example 7A; Example 9A; Example 10A. | Pale yellow solid |
| 355 | 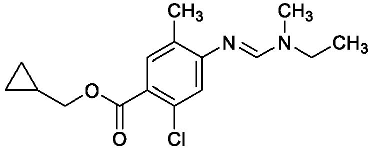 | Example 6A; Example 7A; Example 9A; Example 10A. | Off white sticky solid |
| 356 | 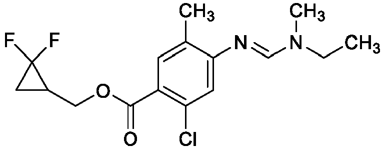 | Example 6A; Example 7A; Example 9A; Example 10A. | Pale brown liquid |
| 357 | 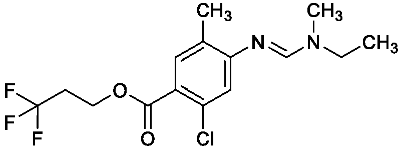 | Example 6A; Example 7A; Example 9A; Example 10A. | Off white solid |
| 358 | 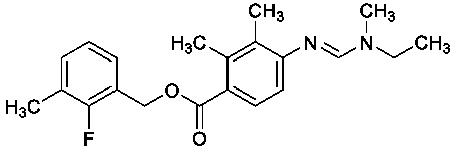 | Example 9A; Example 10A. | Light yellow oil |
| 359 | 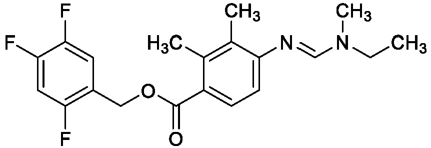 | Example 9A; Example 10A. | Light yellow oil |
| 360 | 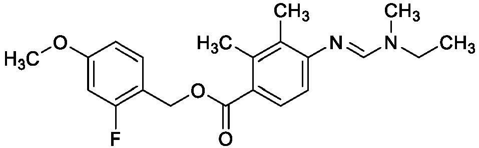 | Example 9A; Example 10A. | Light yellow semisolid |
| 361 | 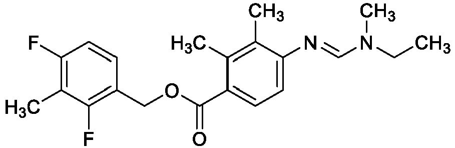 | Example 9A; Example 10A. | Light yellow oil |
| 362 | 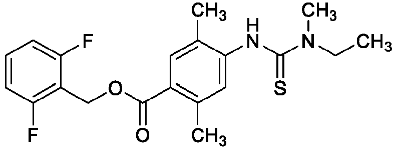 | Example 9A; Example 10D. | Off white solid |
| 363 | 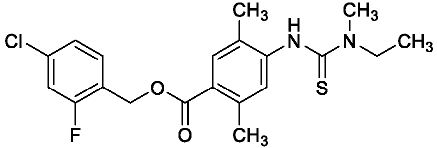 | Example 9A; Example 10D. | Off white solid |
| 364 | 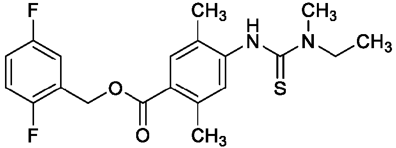 | Example 9A; Example 10D. | Off white solid |
| 365 | 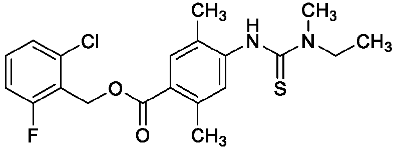 | Example 9A; Example 10D. | Off white solid |
| 366 | 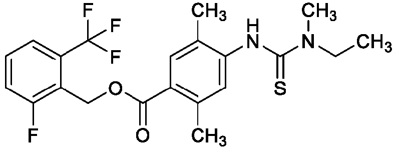 | Example 9A; Example 10D. | White solid |
| 367 | 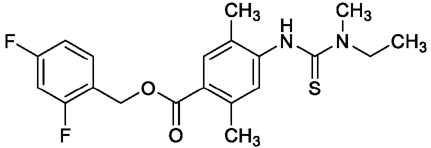 | Example 9A; Example 10D. | White solid |
| 368 | 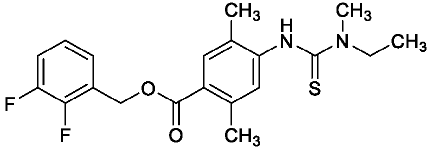 | Example 9A; Example 10D. | Off white solid |
| 369 | 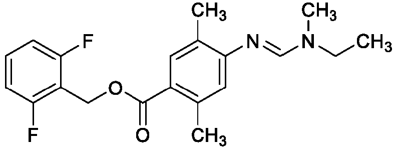 | Example 9A; Example 10A. | Yellow oil |
| 370 | 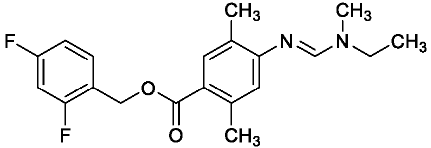 | Example 9A; Example 10A. | Yellow oil |
| 371 | 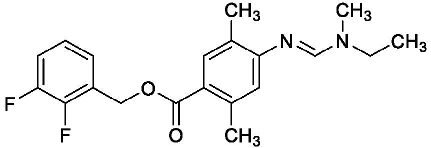 | Example 9A; Example 10A. | Yellow oil |
| 372 | 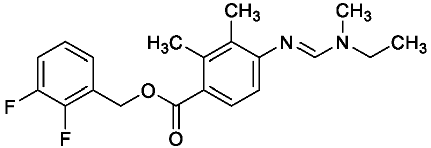 | Example 9A; Example 10A. | Yellow oil |
| 373 | 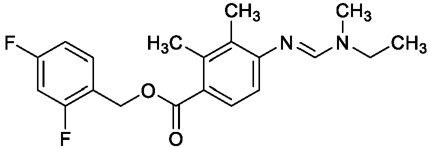 | Example 9A; Example 10A. | Yellow oil |
| 374 | 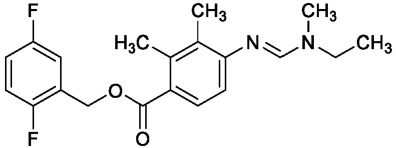 | Example 9A; Example 10A. | Yellow oil |
| 375 | 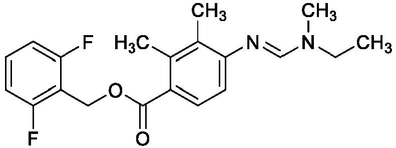 | Example 9A; Example 10A. | Yellow oil |
| 376 | 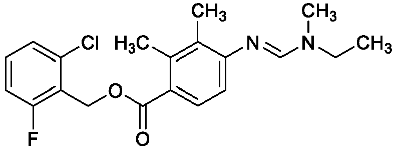 | Example 9A; Example 10A. | Yellow semisolid |
| 377 | 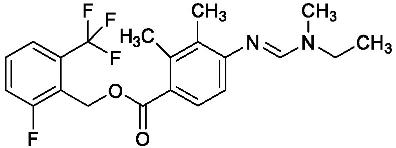 | Example 9A; Example 10A. | Yellow oil |
| 378 | 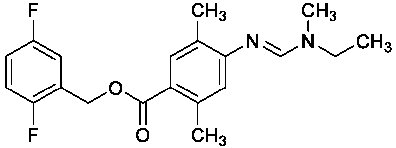 | Example 9A; Example 10A. | Off white waxy solid |
| 379 | 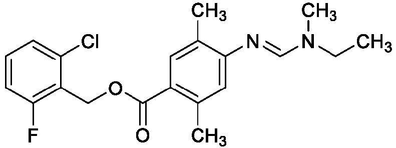 | Example 9A; Example 10A. | Yellow oil |
| 380 | 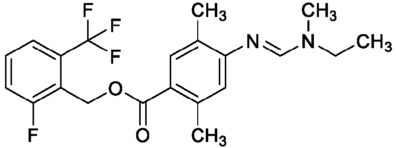 | Example 9A; Example 10A. | Yellow oil |
| 381 | 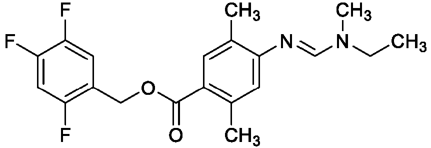 | Example 9A; Example 10A. | Orange oil |
| 382 | 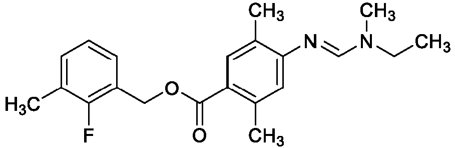 | Example 9A; Example 10A. | Orange oil |
| 383 | 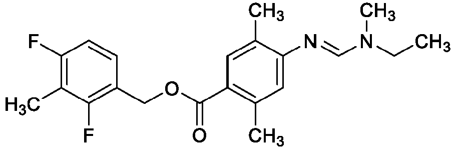 | Example 9A; Example 10A. | Yellow waxy solid |
| 384 | 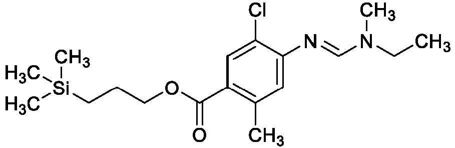 | Example 4; Example 5; Example 6A; Example 7A; Example 9A; Example 10A. | Pale brown gummy liquid |
| 385 | 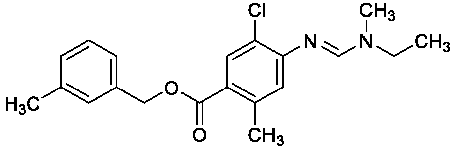 | Example 4; Example 5; Example 6A; Example 7A; Example 9A; Example 10A. | Brown liquid |
| 386 | 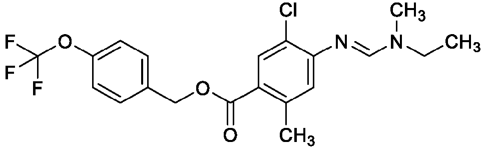 | Example 4; Example 5; Example 6A; Example 7A; Example 9A; Example 10A. | Pale brown gummy solid |
| 387 | 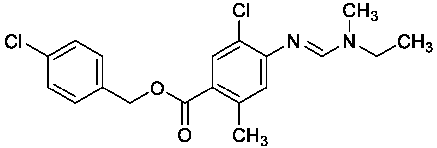 | Example 4; Example 5; Example 6A; Example 7A; Example 9A; Example 10A. | Pale brown solid |
| 388 | 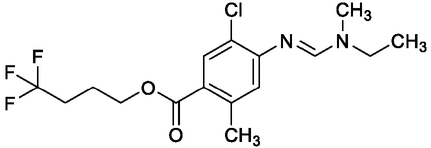 | Example 4; Example 5; Example 6A; Example 7A; Example 9A; Example 10A. | Brown liquid |
| 389 | 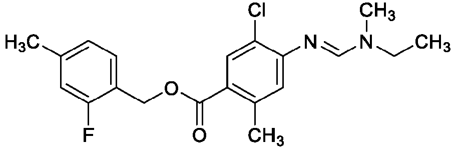 | Example 4; Example 5; Example 6A; Example 7A; Example 9A; Example 10A. | Pale brown sticky solid |
| 390 | 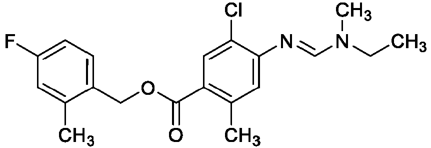 | Example 4; Example 5; Example 6A; Example 7A; Example 9A; Example 10A. | Off white solid |
| 391 | 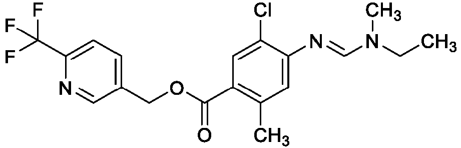 | Example 4; Example 5; Example 6A; Example 7A; Example 9A; Example 10A. | Pale brown solid |
| 392 | 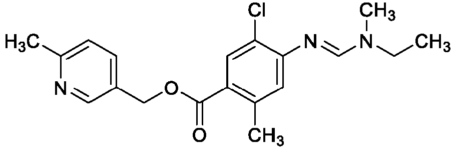 | Example 4; Example 5; Example 6A; Example 7A; Example 9A; Example 10A. | Pale yellow solid |
| 393 | 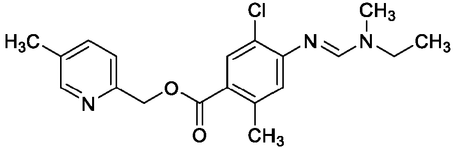 | Example 4; Example 5; Example 6A; Example 7A; Example 9A; Example 10A. | Pale brown gummy liquid |
| 394 | 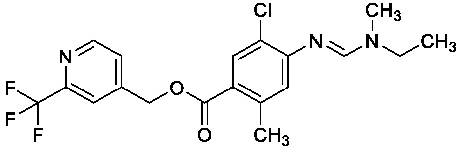 | Example 4; Example 5; Example 6A; Example 7A; Example 9A; Example 10A. | Off white solid |
| 395 | 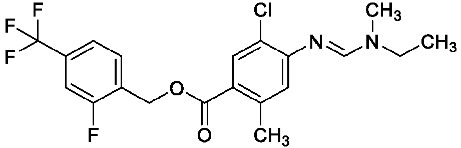 | Example 4; Example 5; Example 6A; Example 7A; Example 9A; Example 10A. | Pale brown solid |
| 396 | 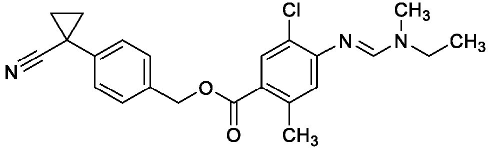 | Example 4; Example 5; Example 6A; Example 7A; Example 9A; Example 10A. | Pale yellow solid |
| 397 | 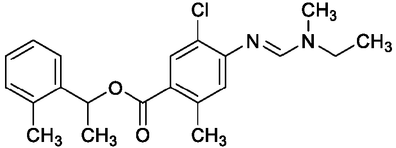 | Example 4; Example 5; Example 6A; Example 7A; Example 9C; Example 10A. | Brown gummy liquid |
| 398 | 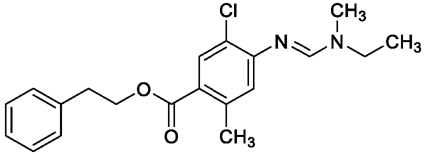 | Example 4; Example 5; Example 6A; Example 7A; Example 9A; Example 10A. | Pale brown solid |
| 399 | 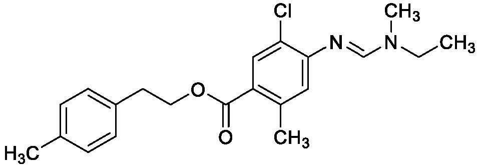 | Example 4; Example 5; Example 6A; Example 7A; Example 9A; Example 10A. | Ash color solid |
| 400 | 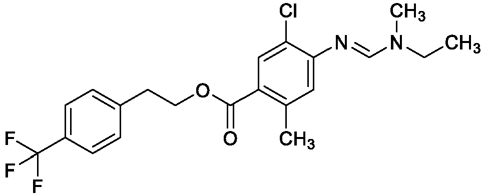 | Example 4; Example 5; Example 6A; Example 7A; Example 9A; Example 10A. | Off white solid |
| 401 | 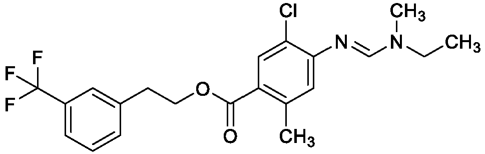 | Example 4; Example 5; Example 6A; Example 7A; Example 9A; Example 10A. | Off white solid |
| 402 | 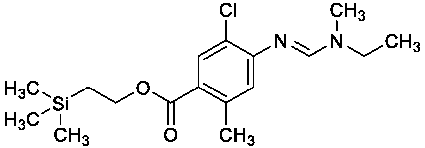 | Example 4; Example 5; Example 6A; Example 7A; Example 9A; Example 10A. | Off white solid |
| 403 | 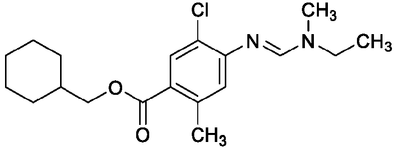 | Example 4; Example 5; Example 6A; Example 7A; Example 9A; Example 10A. | Off white solid |
| 404 | 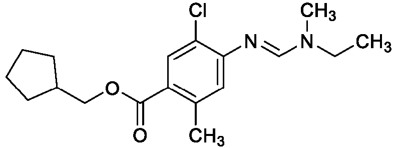 | Example 4; Example 5; Example 6A; Example 7A; Example 9A; Example 10A. | Pale brown sticky solid |
| 405 | 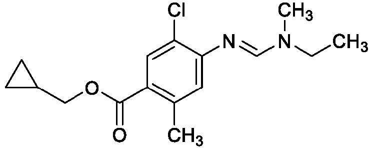 | Example 4; Example 5; Example 6A; Example 7A; Example 9A; Example 10A. | Brown sticky solid |
| 406 | 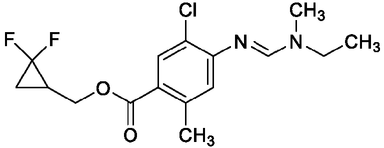 | Example 4; Example 5; Example 6A; Example 7A; Example 9A; Example 10A. | Brown solid |
| 407 | 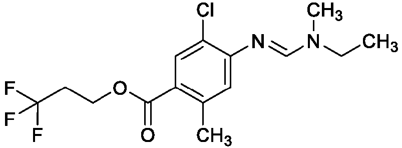 | Example 4; Example 5; Example 6A; Example 7A; Example 9A; Example 10A. | Off white solid |
| 408 | 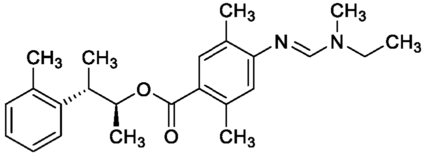 | Example 9C; Example 10A. | Brown oil |
| 409 | 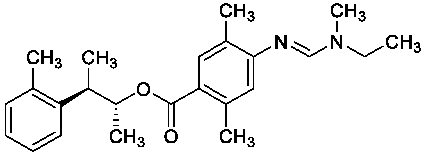 | Example 9C; Example 10A. | Brown oil |
| 410 | 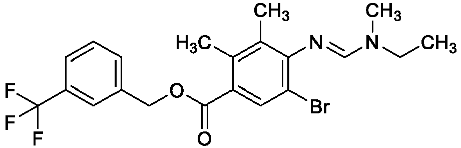 | Example 1B; Example 9A; Example 10A. | Clear oil |
| 411 | 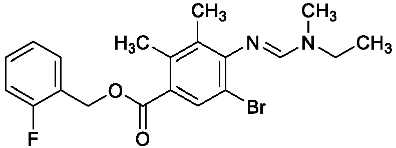 | Example 1B; Example 9A; Example 10A. | Clear oil |
| 412 | 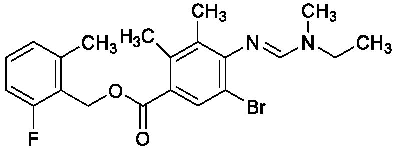 | Example 1B; Example 9A; Example 10A. | Clear oil |
| 413 | 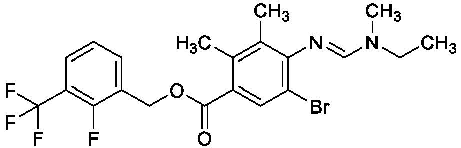 | Example 1B; Example 9A; Example 10A. | Clear oil |
| 414 | 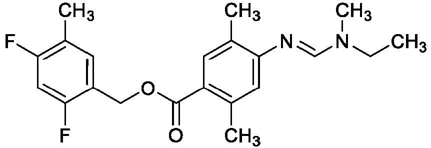 | Example 9A; Example 10A. | Tan semisolid |
| 415 | 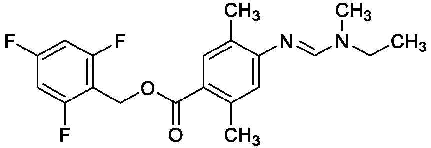 | Example 9A; Example 10A. | Dark brown oil |
| 416 | 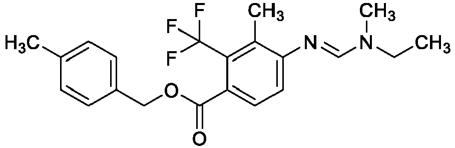 | Example 1C; Example 6C; Example 7B; Example 9A; Example 10A. | Brown oil |
| 417 | 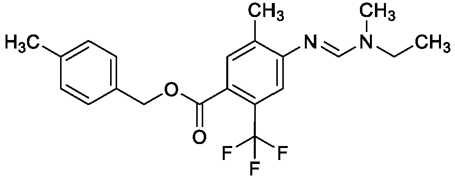 | Example 1C; Example 6C; Example 7B; Example 9A; Example 10A. | Brown oil |
| 418 | 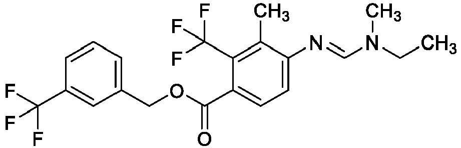 | Example 1C; Example 6C; Example 7B; Example 9A; Example 10A. | Yellow oil |
| 419 | 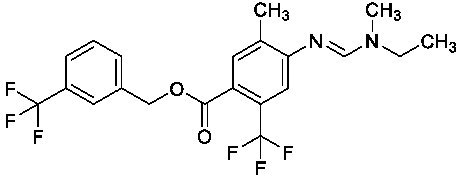 | Example 1C; Example 6C; Example 7B; Example 9A; Example 10A. | Off white solid |
| 420 | 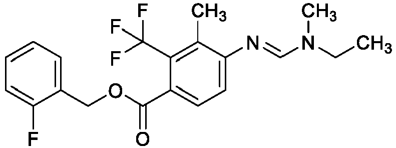 | Example 1C; Example 6C; Example 7B; Example 9A; Example 10A. | Brown oil |
| 421 | 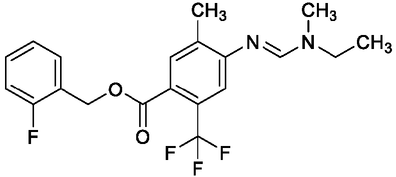 | Example 1C; Example 6C; Example 7B; Example 9A; Example 10A. | Brown oil |
| 422 | 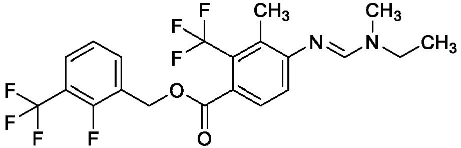 | Example 1C; Example 6C; Example 7B; Example 9A; Example 10A. | Brown oil |
| 423 | 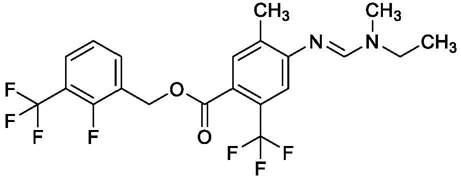 | Example 1C; Example 6C; Example 7B; Example 9A; Example 10A. | Brown oil |
| 424 | 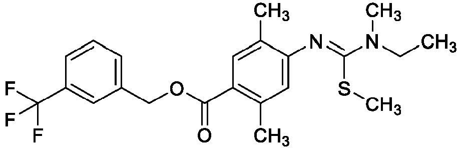 | Example 9B; Example 10D; Example 12. | Clear oil |
| 425 | 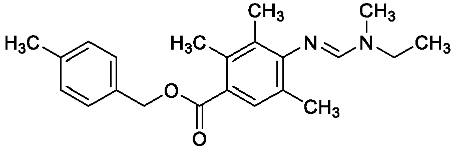 | Example 1B; Example 2B; Example 3B; Example 9A; Example 10A. | Clear oil |
| 426 | 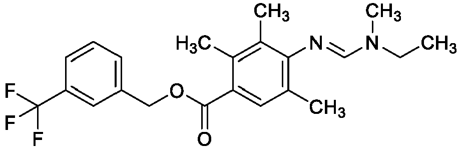 | Example 1B; Example 2B; Example 3B; Example 9A; Example 10A. | Colorless oil |
| 427 | 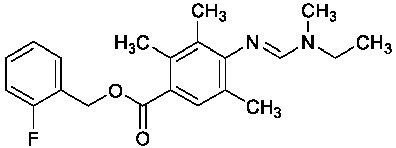 | Example 1B; Example 2B; Example 3B; Example 9A; Example 10A. | Colorless oil |
| 428 | 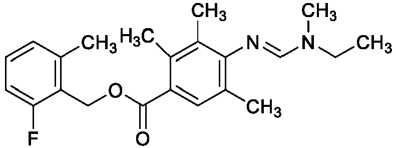 | Example 1B; Example 2B; Example 3B; Example 9A; Example 10A. | Colorless oil |
| 429 | 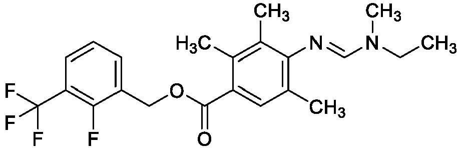 | Example 1B; Example 2B; Example 3B; Example 9A; Example 10A. | Colorless oil |
| 430 | 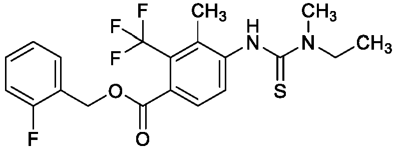 | Example 1C; Example 6C; Example 7B; Example 9A; Example 10D. | Yellow solid |
| 431 | 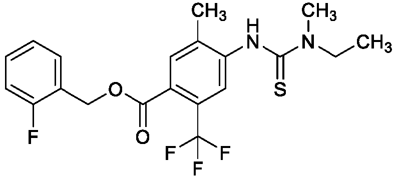 | Example 1C; Example 6C; Example 7B; Example 9A; Example 10D. | Off white semisolid |
| 432 | 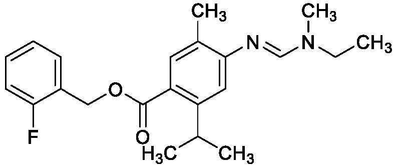 | Example 1C; Example 6C; Example 7B; Example 9A; Example 10A. | Yellow oil |
| 433 | 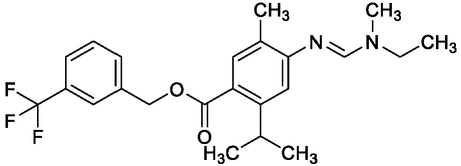 | Example 1C; Example 6C; Example 7B; Example 9A; Example 10A. | Yellow oil |
| 434 | 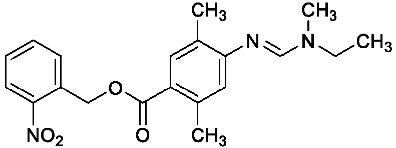 | Example 9A; Example 10A. | Yellow solid |
| 435 | 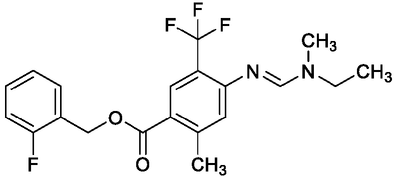 | Example 1C; Example 6C; Example 7B; Example 9A; Example 10A. | Off white solid |
| 436 | 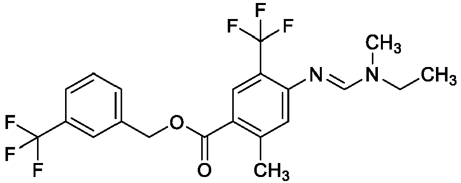 | Example 1C; Example 6C; Example 7B; Example 9A; Example 10A. | Light yellow oil |
| 437 | 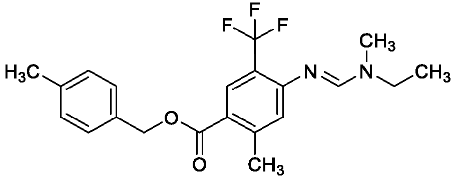 | Example 1C; Example 6C; Example 7B; Example 9A; Example 10A. | Light yellow oil |
| 438 | 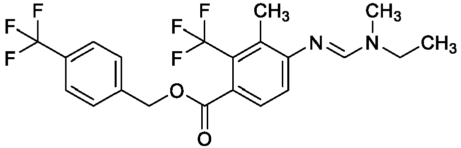 | Example 1C; Example 6C; Example 7B; Example 9A; Example 10A. | Light yellow oil |
| 439 | 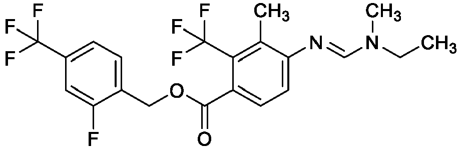 | Example 1C; Example 6C; Example 7B; Example 9A; Example 10A. | Light yellow oil |
| 440 | 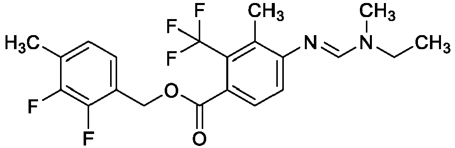 | Example 1C; Example 6C; Example 7B; Example 9A; Example 10A. | Light yellow oil |
| 441 | 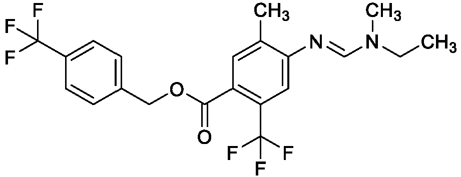 | Example 1C; Example 6C; Example 7B; Example 9A; Example 10A. | Off white solid |
| 442 | 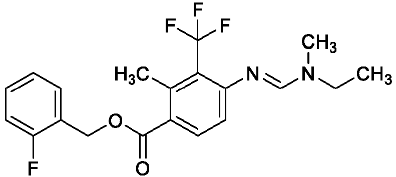 | Example 1C; Example 6C; Example 7B; Example 9A; Example 10A. | Light yellow oil |
| 443 | 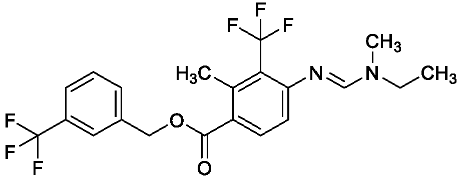 | Example 1C; Example 6C; Example 7B; Example 9A; Example 10A. | Light yellow oil |
| 444 | 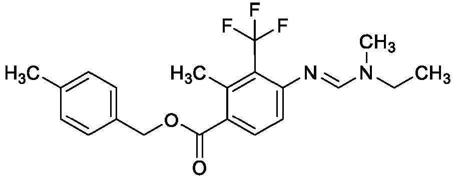 | Example 1C; Example 6C; Example 7B; Example 9A; Example 10A. | Light yellow oil |
| 445 | 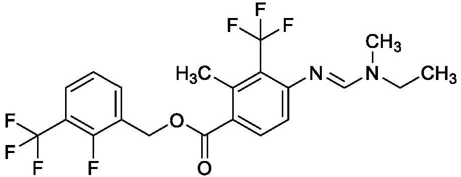 | Example 1C; Example 6C; Example 7B; Example 9A; Example 10A. | Light yellow oil |
| 446 | 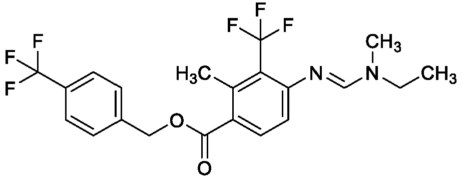 | Example 1C; Example 6C; Example 7B; Example 9A; Example 10A. | Off white solid |
| 447 | 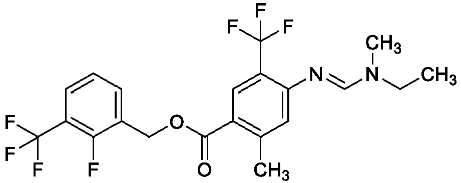 | Example 1C; Example 6C; Example 7B; Example 9A; Example 10A. | Light yellow oil |
| 448 | 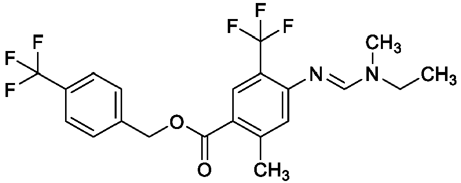 | Example 1C; Example 6C; Example 7B; Example 9A; Example 10A. | Light yellow oil |
| 449 | 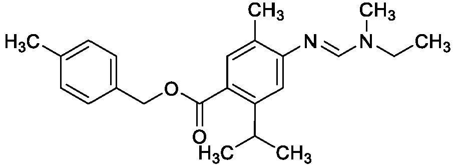 | Example 1C; Example 6C; Example 7B; Example 9A; Example 10A. | Clear oil |
| 450 | 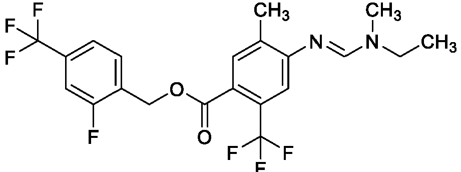 | Example 1C; Example 6C; Example 7B; Example 9A; Example 10A. | Light yellow solid |
| 451 | 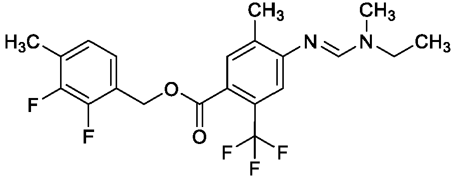 | Example 1C; Example 6C; Example 7B; Example 9A; Example 10A. | Light yellow semisolid |
| 452 | 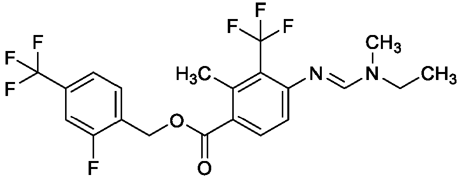 | Example 1C; Example 6C; Example 7B; Example 9A; Example 10A. | Light yellow solid |
| 453 | 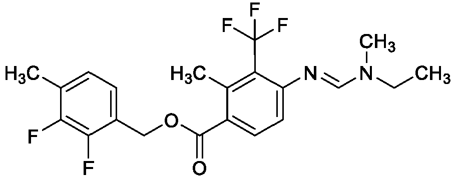 | Example 1C; Example 6C; Example 7B; Example 9A; Example 10A. | Light yellow semisolid |
| 454 | 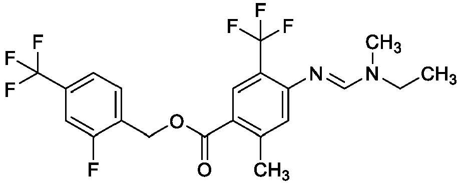 | Example 1C; Example 6C; Example 7B; Example 9A; Example 10A. | Light yellow solid |
| 455 | 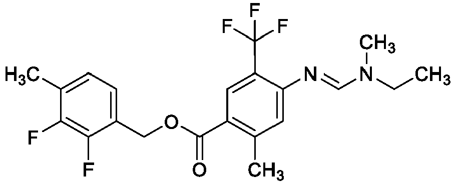 | Example 1C; Example 6C; Example 7B; Example 9A; Example 10A. | Yellow oil |
| 456 | 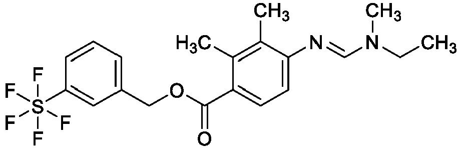 | Example 9A; Example 10A. | Light yellow oil |
| 457 | 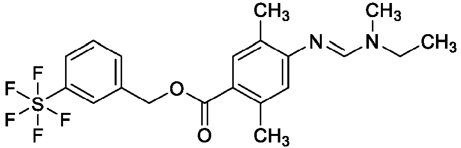 | Example 9A; Example 10A. | Light yellow oil |
| 458 | 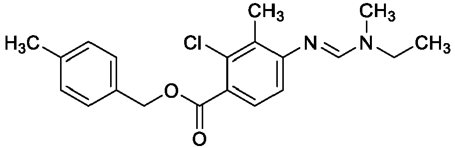 | Example 6C; Example 7B; Example 9A; Example 10A. | Beige semisolid |
| 459 | 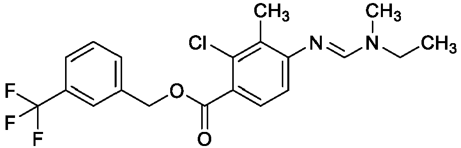 | Example 6C; Example 7B; Example 9A; Example 10A. | Beige oil |
| 460 | 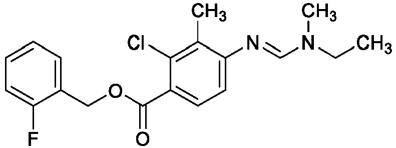 | Example 6C; Example 7B; Example 9A; Example 10A. | Orange oil |
| 461 | 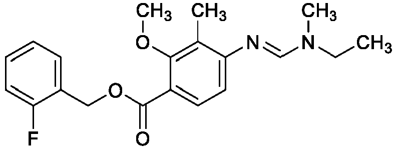 | Example 1C; Example 6C; Example 7B; Example 9A; Example 10A. | Clear oil |
| 462 | 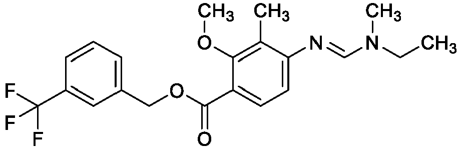 | Example 1C; Example 6C; Example 7B; Example 9A; Example 10A. | Yellow oil |
| 463 | 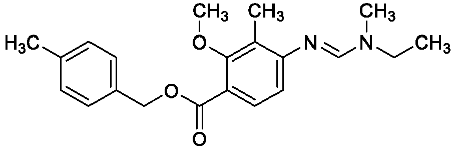 | Example 1C; Example 6C; Example 7B; Example 9A; Example 10A. | Clear oil |
| 464 | 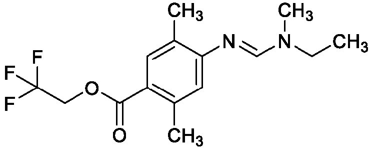 | Example 9A; Example 10A. | Off white powder |
| 465 | 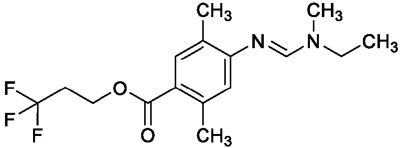 | Example 9A; Example 10A. | Off white semisolid |
| 466 | 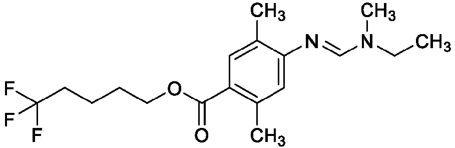 | Example 9A; Example 10A. | Orange oil |
| 467 | 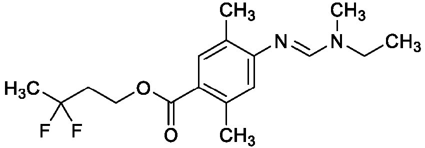 | Example 9A; Example 10A. | Brown oil |
| 468 | 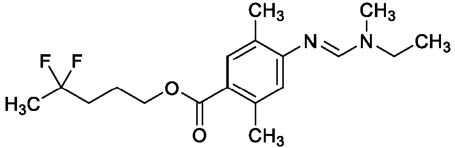 | Example 9A; Example 10A. | Brown oil |
| 469 | 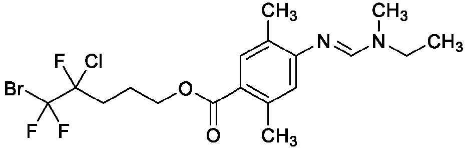 | Example 9A; Example 10A. | Brown oil |
| 470 | 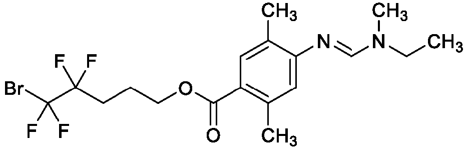 | Example 9A; Example 10A. | Brown oil |
| 471 | 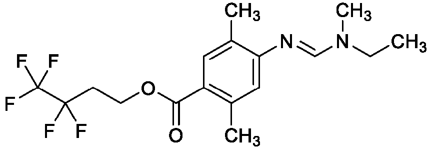 | Example 9A; Example 10A. | Off white semisolid |
| 472 | 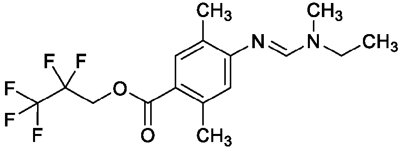 | Example 9A; Example 10A. | Off white semisolid |
| 473 | 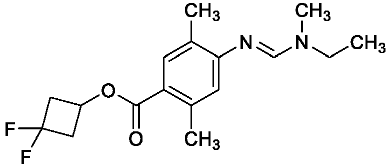 | Example 9A; Example 10A. | Off white semisolid |
| 474 | 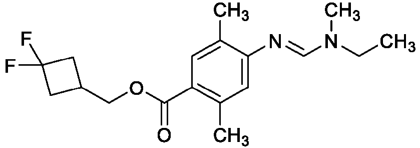 | Example 9A; Example 10A. | Brown oil |
| 475 | 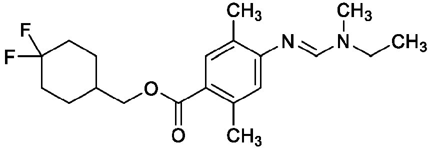 | Example 9A; Example 10A. | Off white semisolid |
| 476 | 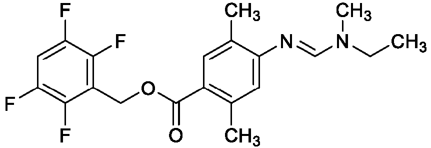 | Example 9A; Example 10A. | Off white semisolid |
| 477 | 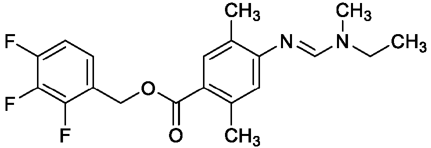 | Example 9A; Example 10A. | Brown oil |
| 478 | 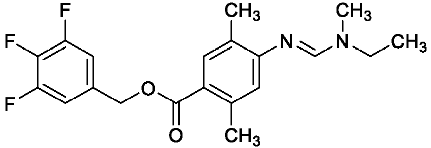 | Example 9A; Example 10A. | Off white powder |
| 479 | 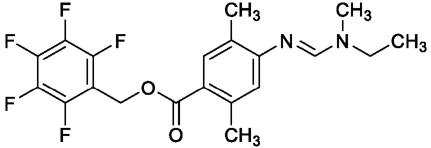 | Example 9A; Example 10A. | Brown oil |
| 480 | 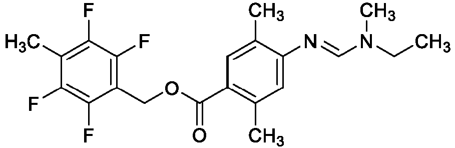 | Example 9A; Example 10A. | Brown oil |
| 481 | 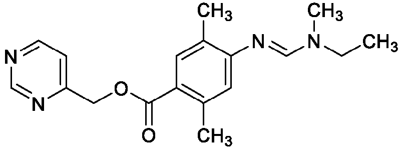 | Example 9A; Example 10A. | Brown oil |
| 482 | 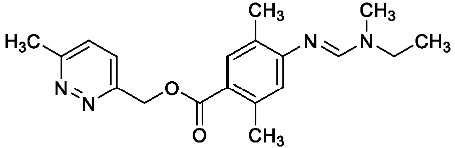 | Example 9A; Example 10A. | Brown semisolid |
| 483 | 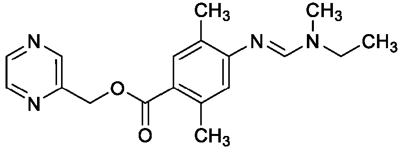 | Example 9A; Example 10A. | Brown oil |
| 484 | 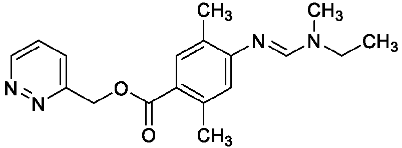 | Example 9A; Example 10A. | Dark brown oil |
| 485 | 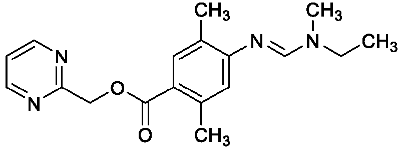 | Example 9A; Example 10A. | Brown oil |
| 486 | 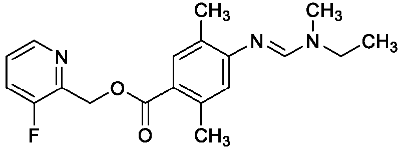 | Example 9A; Example 10A. | Dark brown oil |
| 487 | 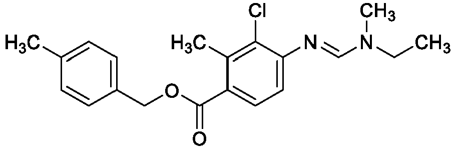 | Example 4; Example 5; Example 6C; Example 7B; Example 9A; Example 10A. | Brown oil |
| 488 | 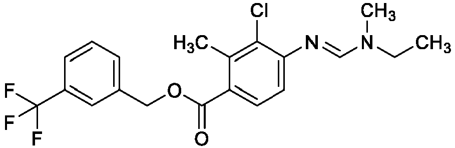 | Example 4; Example 5; Example 6C; Example 7B; Example 9A; Example 10A. | Brown oil |
| 489 | 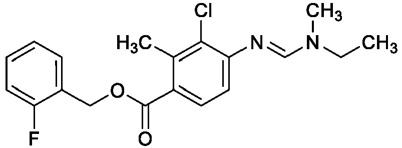 | Example 4; Example 5; Example 6C; Example 7B; Example 9A; Example 10A. | Brown semisolid |
| 490 | 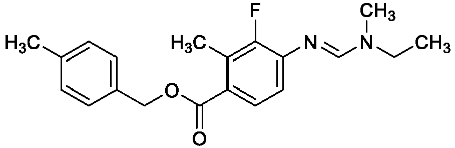 | Example 4; Example 5; Example 6C; Example 7B; Example 9A; Example 10A. | Orange oil |
| 491 | 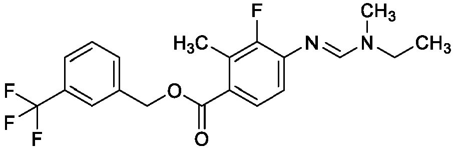 | Example 4; Example 5; Example 6C; Example 7B; Example 9A; Example 10A. | Orange oil |
| 492 | 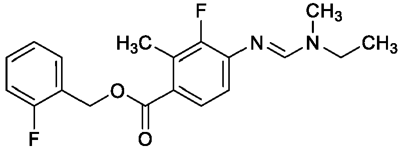 | Example 4; Example 5; Example 6C; Example 7B; Example 9A; Example 10A. | Orange oil |
| 493 | 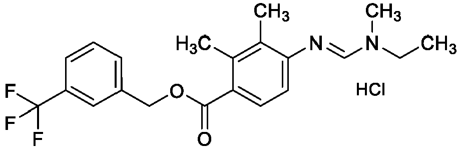 | Example 9B; Example 10B; Example 13. | Light brown solid |
| 494 | 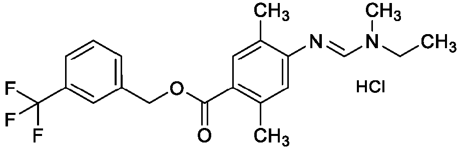 | Example 9B; Example 10B; Example 13. | Off white solid |
| 495 | 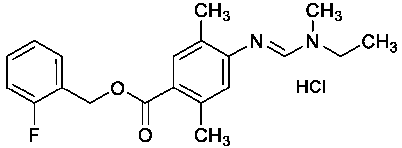 | Example 9B; Example 10B; Example 13. | Off white solid |
| 496 | 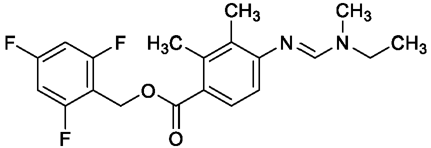 | Example 9A; Example 10A. | Off white semisolid |
| 497 | 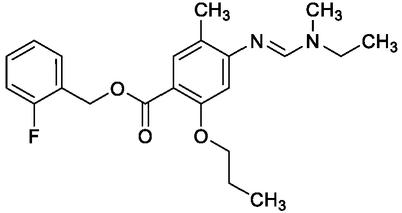 | Example 1C; Example 6C; Example 7B; Example 9A; Example 10A. | Light yellow oil |
| 498 | 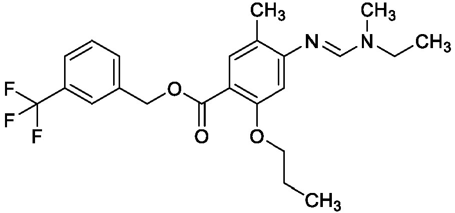 | Example 1C; Example 6C; Example 7B; Example 9A; Example 10A. | Light yellow oil |
| 499 | 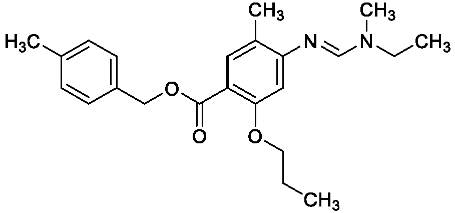 | Example 1C; Example 6C; Example 7B; Example 9A; Example 10A. | Light yellow oil |
| 500 | 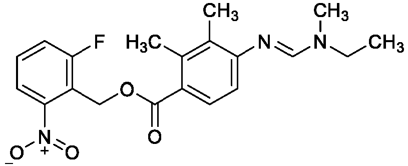 | Example 9A; Example 10A. | Yellow oil |
| 501 | 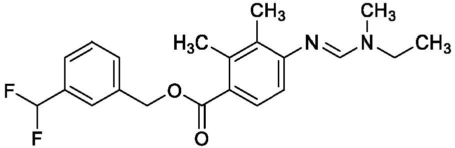 | Example 9A; Example 10A. | Clear oil |
| 502 | 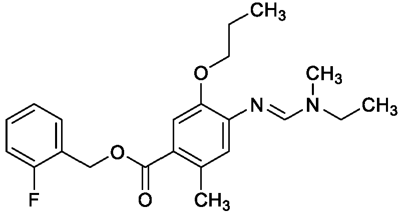 | Example 1C; Example 6C; Example 7B; Example 9A; Example 10A. | Light yellow oil |
| 503 | 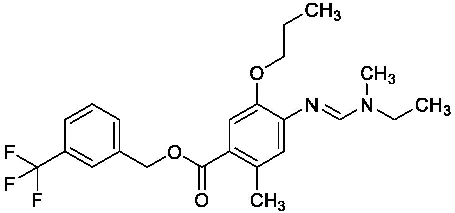 | Example 1C; Example 6C; Example 7B; Example 9A; Example 10A. | Light yellow oil |
| 504 | 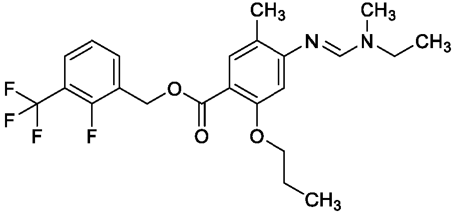 | Example 1C; Example 6C; Example 7B; Example 9A; Example 10A. | Light yellow oil |
| 505 | 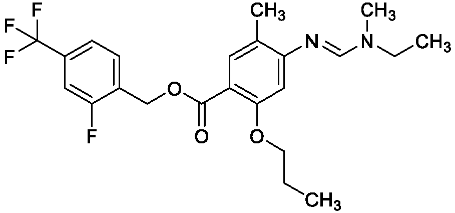 | Example 1C; Example 6C; Example 7B; Example 9A; Example 10A. | Light yellow oil |
| 506 | 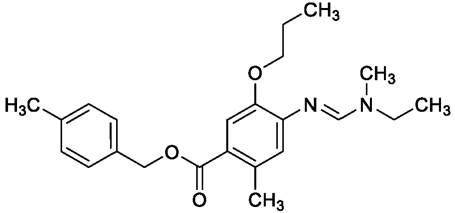 | Example 1C; Example 6C; Example 7B; Example 9A; Example 10A. | Light yellow oil |
| 507 | 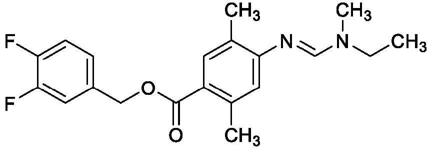 | Example 9A; Example 10A. | Brown oil |
| 508 | 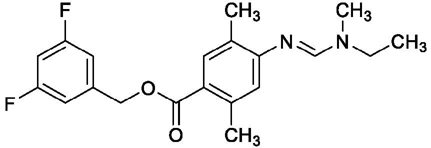 | Example 9A; Example 10A. | Brown oil |
| 509 | 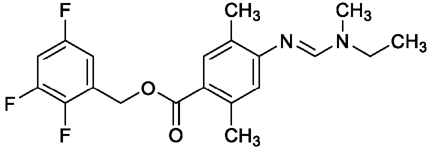 | Example 9A; Example 10A. | Off white semisolid |
| 510 | 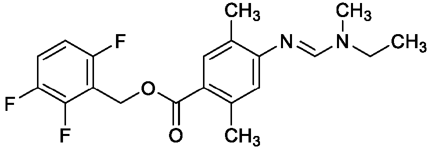 | Example 9A; Example 10A. | Orange semisolid |
| 511 | 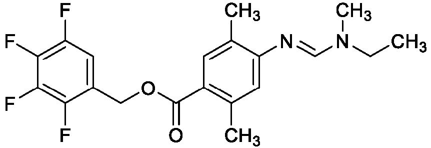 | Example 9A; Example 10A. | Off white semisolid |
| 512 | 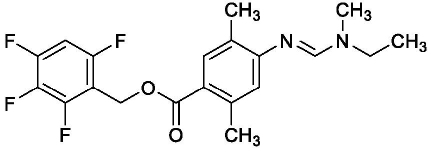 | Example 9A; Example 10A. | Brown oil |
| 513 | 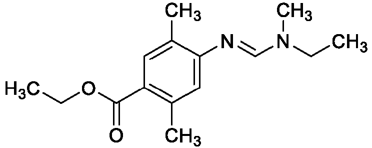 | Example 9A; Example 10A. | Brown oil |
| 514 | 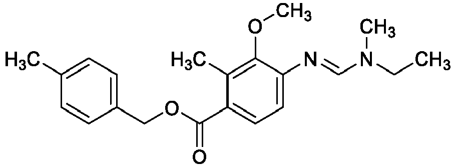 | Example 1C; Example 6C; Example 7B; Example 9A; Example 10A. | Yellow oil |
| 515 | 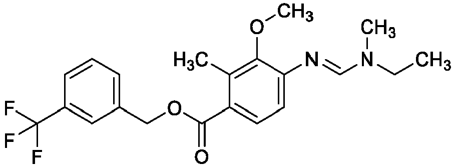 | Example 1C; Example 6C; Example 7B; Example 9A; Example 10A. | Yellow oil |
| 516 | 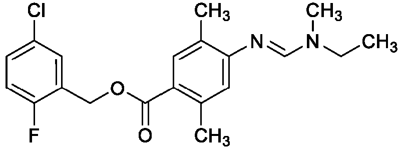 | Example 9A; Example 10A. | Yellow oil |
| 517 | 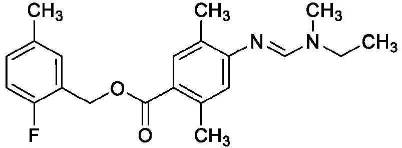 | Example 9A; Example 10A. | Yellow oil |
| 518 | 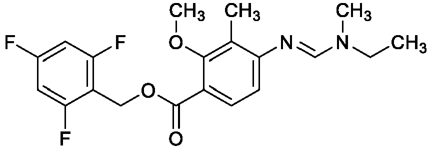 | Example 1C; Example 6C; Example 7B; Example 9A; Example 10A. | Off white solid |
| 519 | 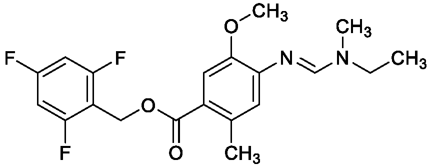 | Example 6C; Example 7B; Example 9A; Example 10A. | Light yellow oil |
| 520 | 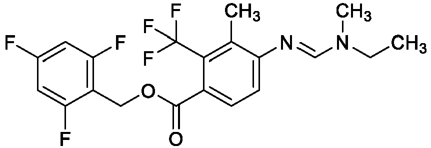 | Example 1C; Example 6C; Example 7B; Example 9A; Example 10A. | Light yellow oil |
| 521 | 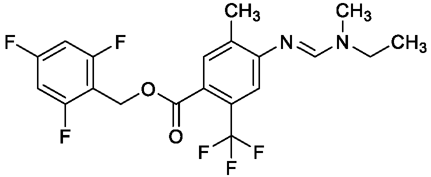 | Example 1C; Example 6C; Example 7B; Example 9A; Example 10A. | Light yellow oil |
| 522 | 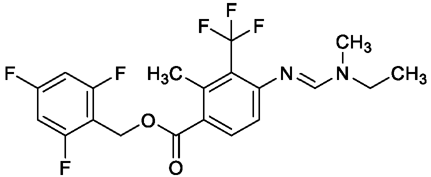 | Example 1C; Example 6C; Example 7B; Example 9A; Example 10A. | White solid |
| 523 | 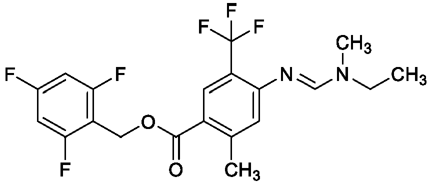 | Example 1C; Example 6C; Example 7B; Example 9A; Example 10A. | White solid |
| 524 | 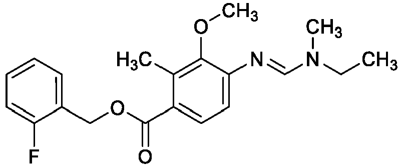 | Example 1C; Example 6C; Example 7B; Example 9A; Example 10A. | Yellow oil |
| 525 | 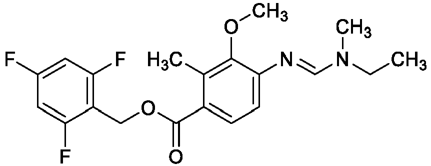 | Example 1C; Example 6C; Example 7B; Example 9A; Example 10A. | Yellow oil |
| 526 | 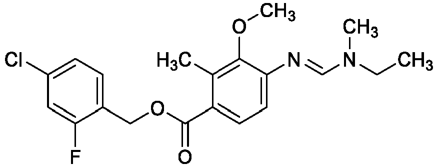 | Example 1C; Example 6C; Example 7B; Example 9A; Example 10A. | Yellow oil |
| 527 | 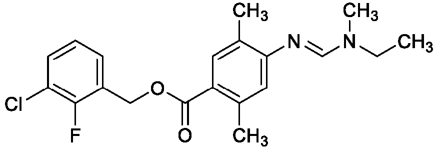 | Example 9A; Example 10A. | Yellow oil |
| 528 | 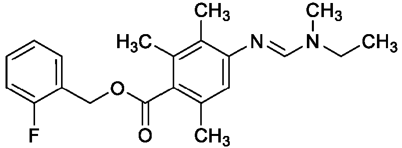 | Example 1C; Example 6C; Example 7B; Example 9A; Example 10A. | Yellow semisolid |
| 530 | 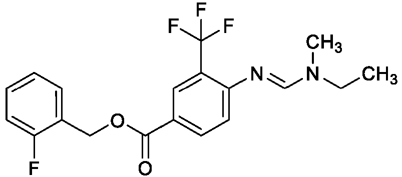 | Example 9A; Example 10A. | Brown oil |
| 531 | 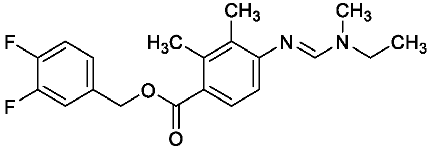 | Example 9A; Example 10A. | Yellow oil |
| 532 | 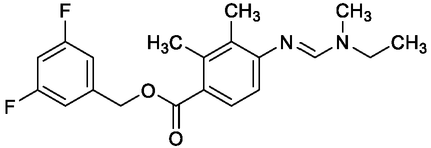 | Example 9A; Example 10A. | Yellow oil |
| 533 | 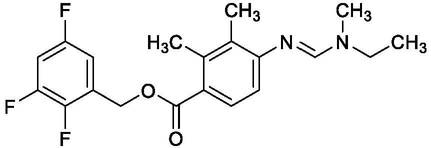 | Example 9A; Example 10A. | Yellow oil |
| 534 | 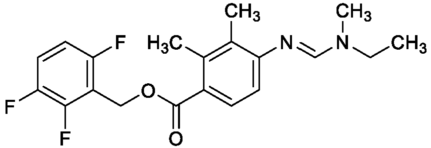 | Example 9A; Example 10A. | Yellow oil |
| 535 | 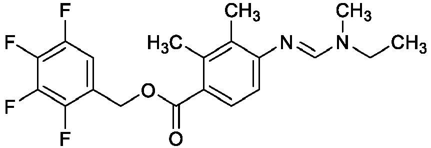 | Example 9A; Example 10A. | Yellow oil |
| 536 | 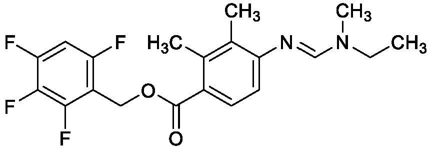 | Example 9A; Example 10A. | Yellow oil |
| 537 | 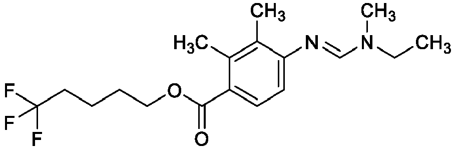 | Example 9A; Example 10A. | Yellow oil |
| 538 | 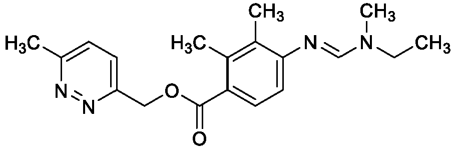 | Example 9A; Example 10A. | Yellow oil |
| 539 | 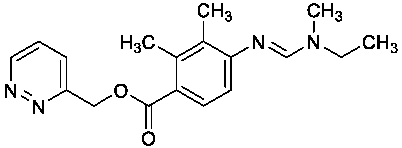 | Example 9A; Example 10A. | Yellow oil |
| 540 | 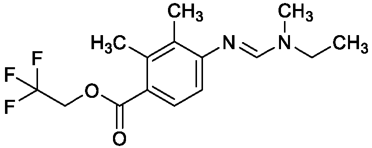 | Example 9A; Example 10A. | Yellow oil |
| 541 | 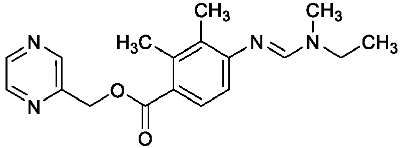 | Example 9A; Example 10A. | Yellow oil |
| 542 | 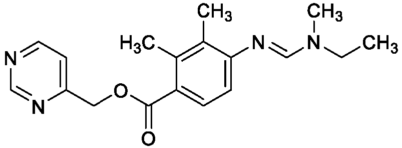 | Example 9A; Example 10A. | Yellow oil |
| 543 | 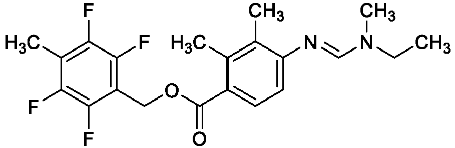 | Example 9A; Example 10A. | Yellow oil |
| 544 | 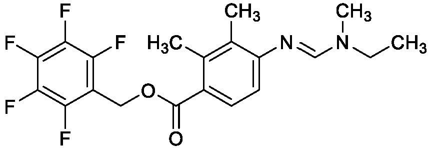 | Example 9A; Example 10A. | Yellow oil |
| 545 | 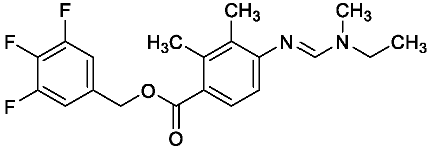 | Example 9A; Example 10A. | Yellow oil |
| 546 | 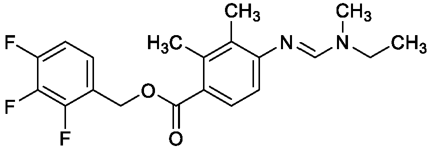 | Example 9A; Example 10A. | Yellow oil |
| 547 | 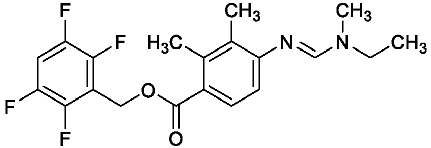 | Example 9A; Example 10A. | Yellow oil |
| 548 | 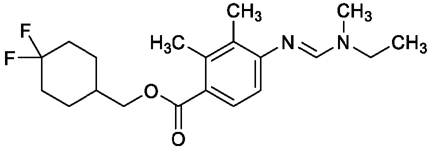 | Example 9A; Example 10A. | Yellow oil |
| 549 | 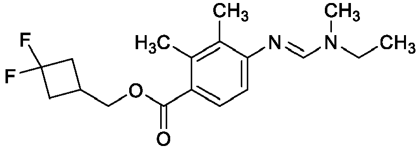 | Example 9A; Example 10A. | Yellow oil |
| 550 | 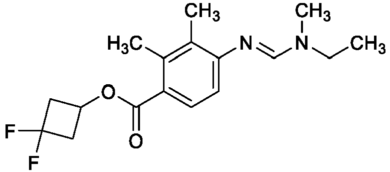 | Example 9A; Example 10A. | Yellow oil |
| 551 | 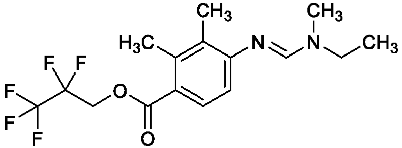 | Example 9A; Example 10A. | Yellow oil |
| 552 | 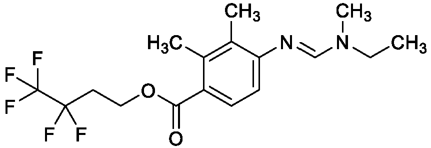 | Example 9A; Example 10A. | Yellow oil |
| 553 | 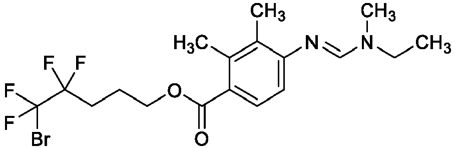 | Example 9A; Example 10A. | Yellow oil |
| 554 | 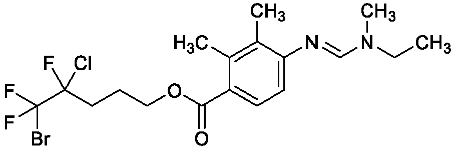 | Example 9A; Example 10A. | Yellow oil |
| 555 | 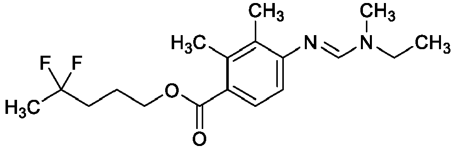 | Example 9A; Example 10A. | Yellow oil |
| 556 | 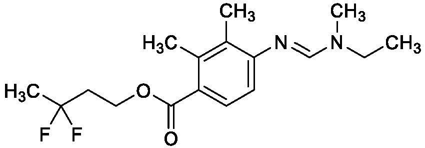 | Example 9A; Example 10A. | Yellow oil |
| 557 | 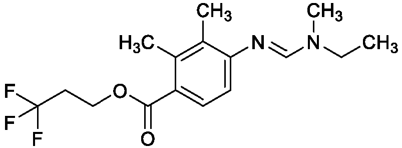 | Example 9A; Example 10A. | Yellow oil |
| 558 | 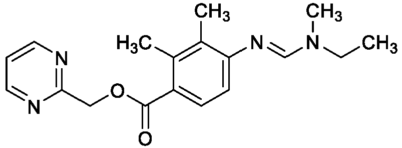 | Example 9A; Example 10A. | Yellow oil |
| 559 | 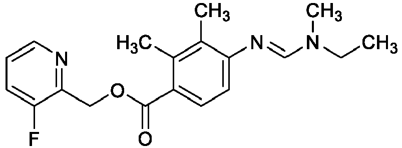 | Example 9A; Example 10A. | Yellow oil |
| 560 | 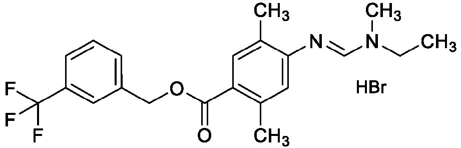 | Example 9B; Example 10A; Example 13. | Off white solid |
| 561 | 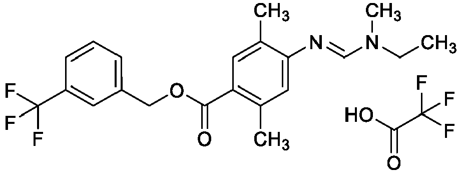 | Example 9B; Example 10A; Example 13. | Off white solid |
| 562 | 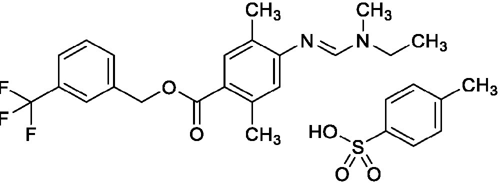 | Example 9B; Example 10A; Example 13. | Brown oil |
| 563 | 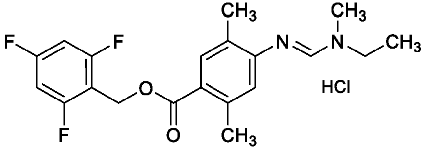 | Example 9B; Example 10A; Example 13. | Off white solid |

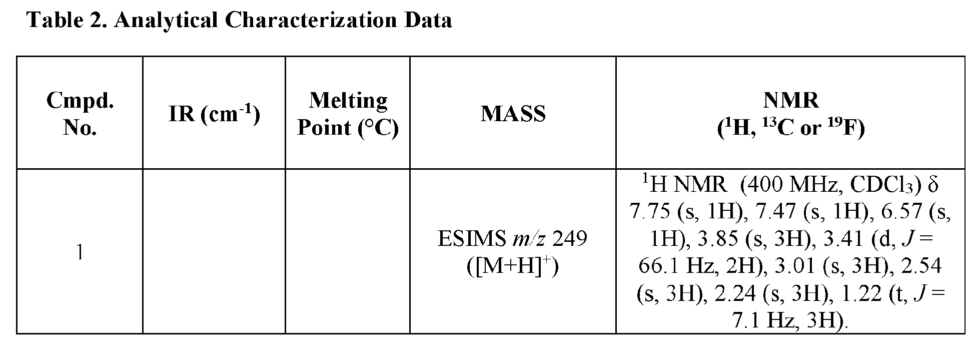

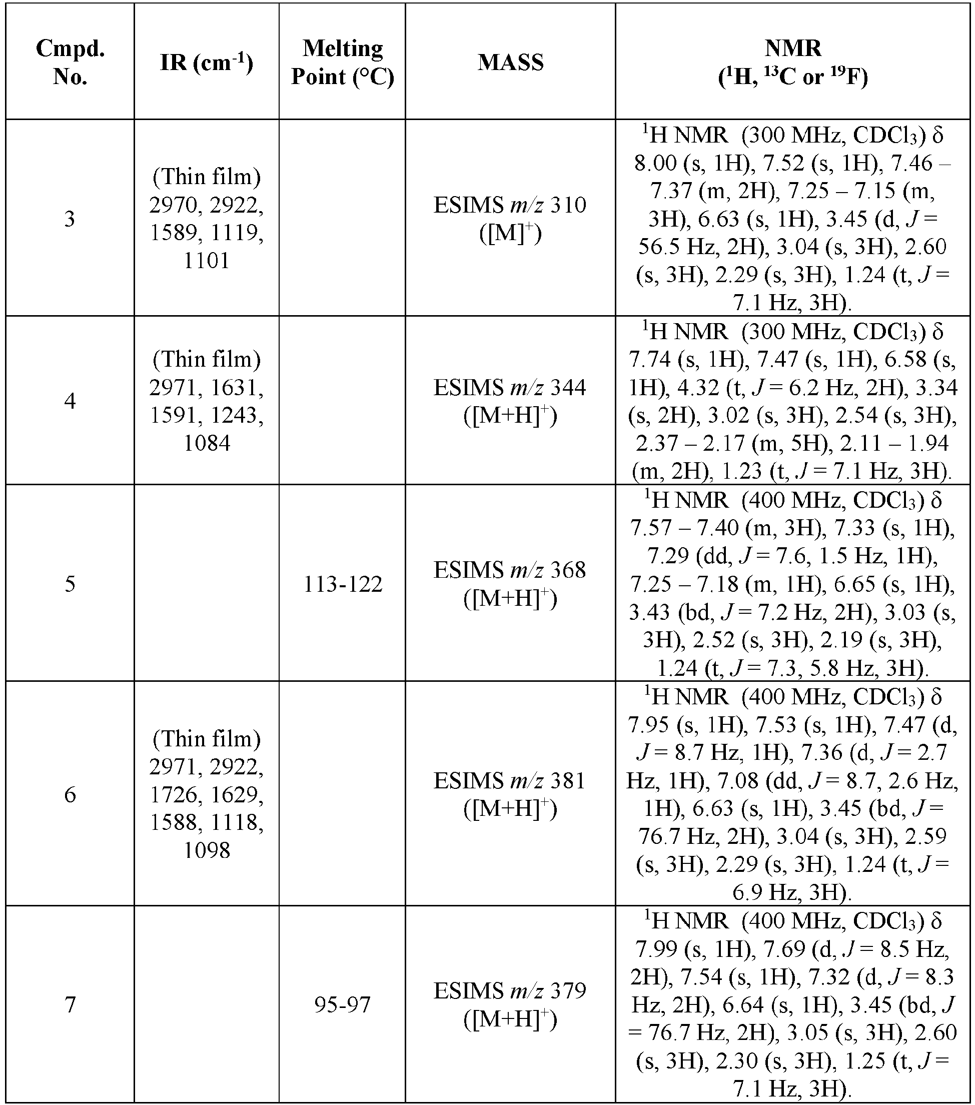

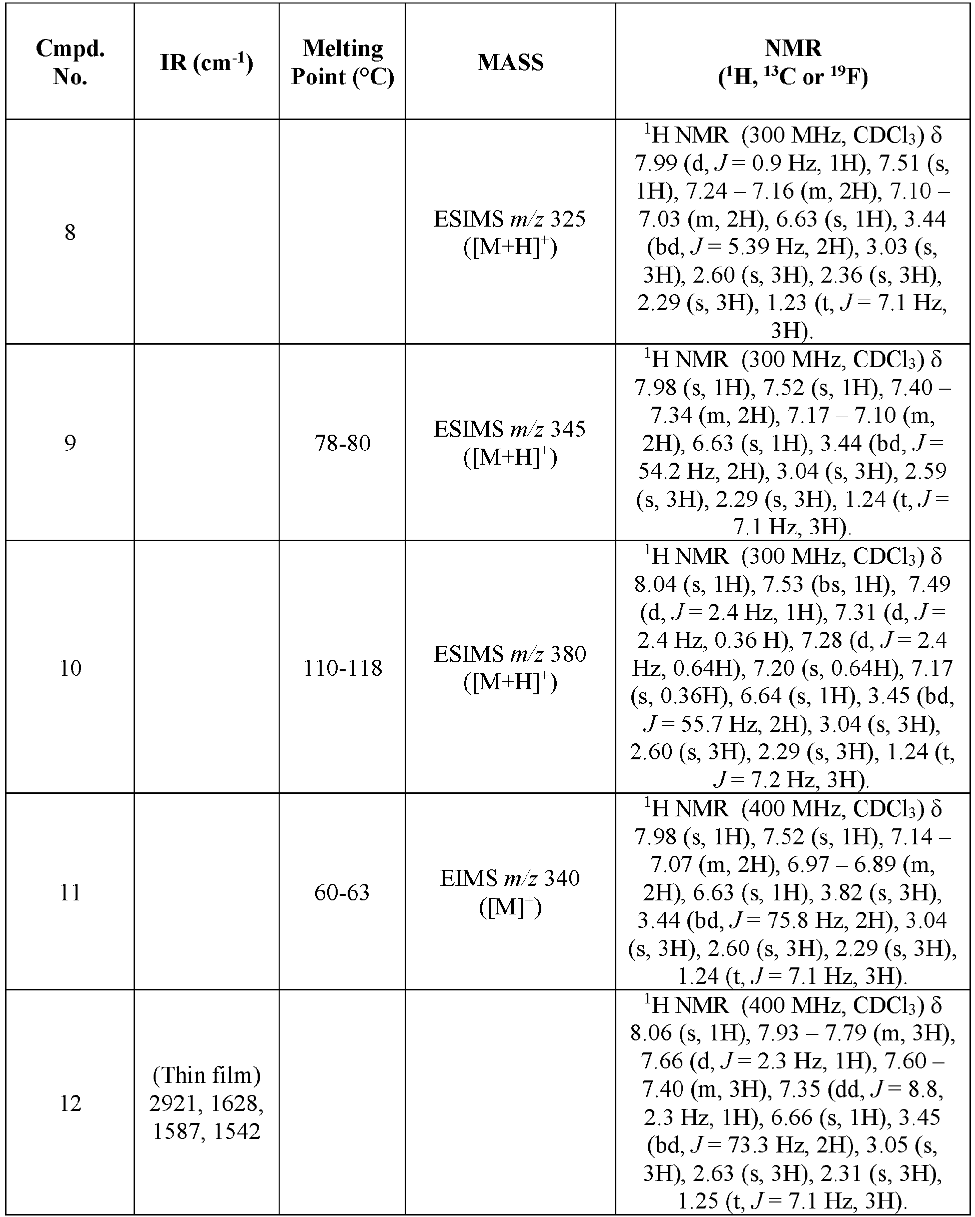

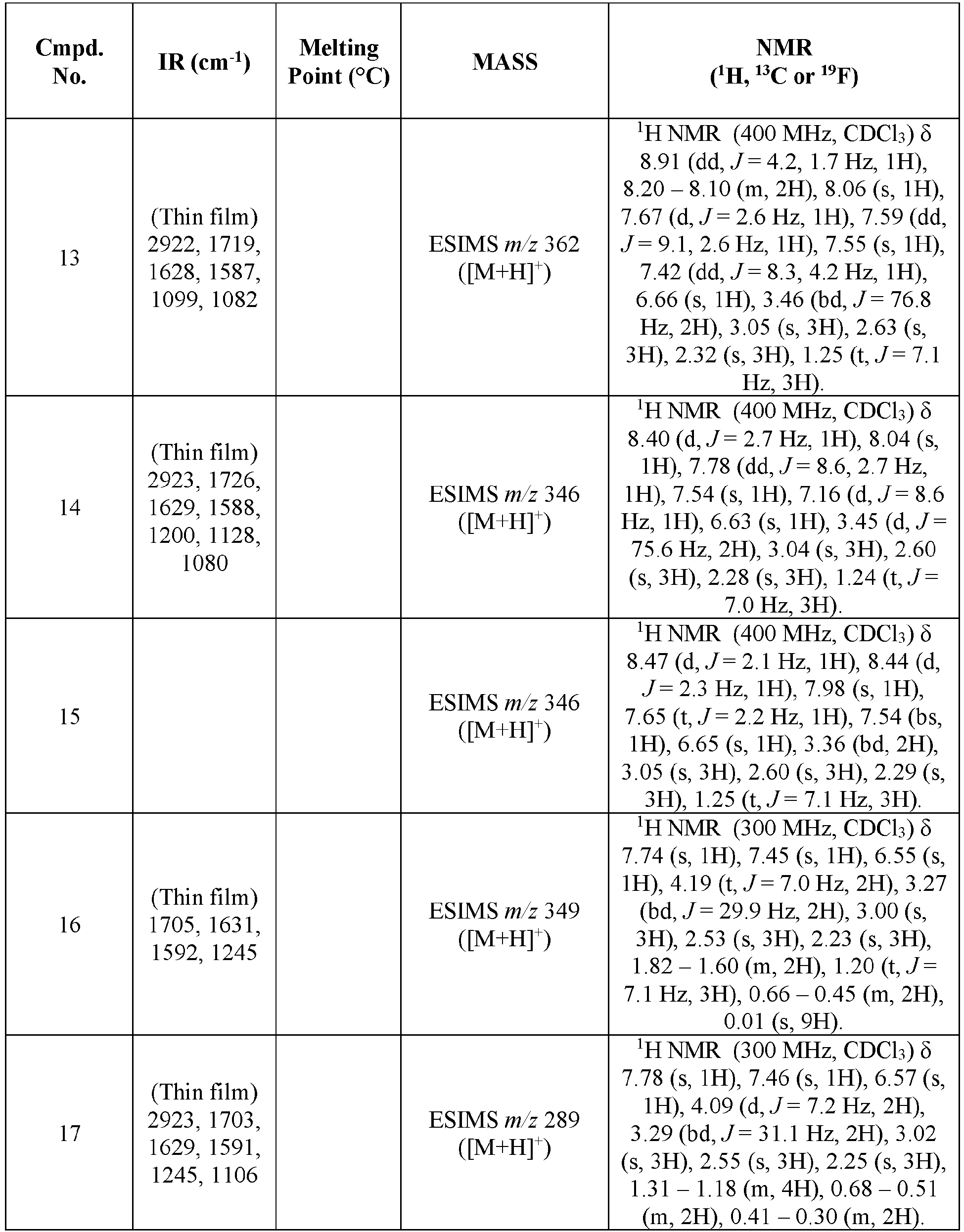

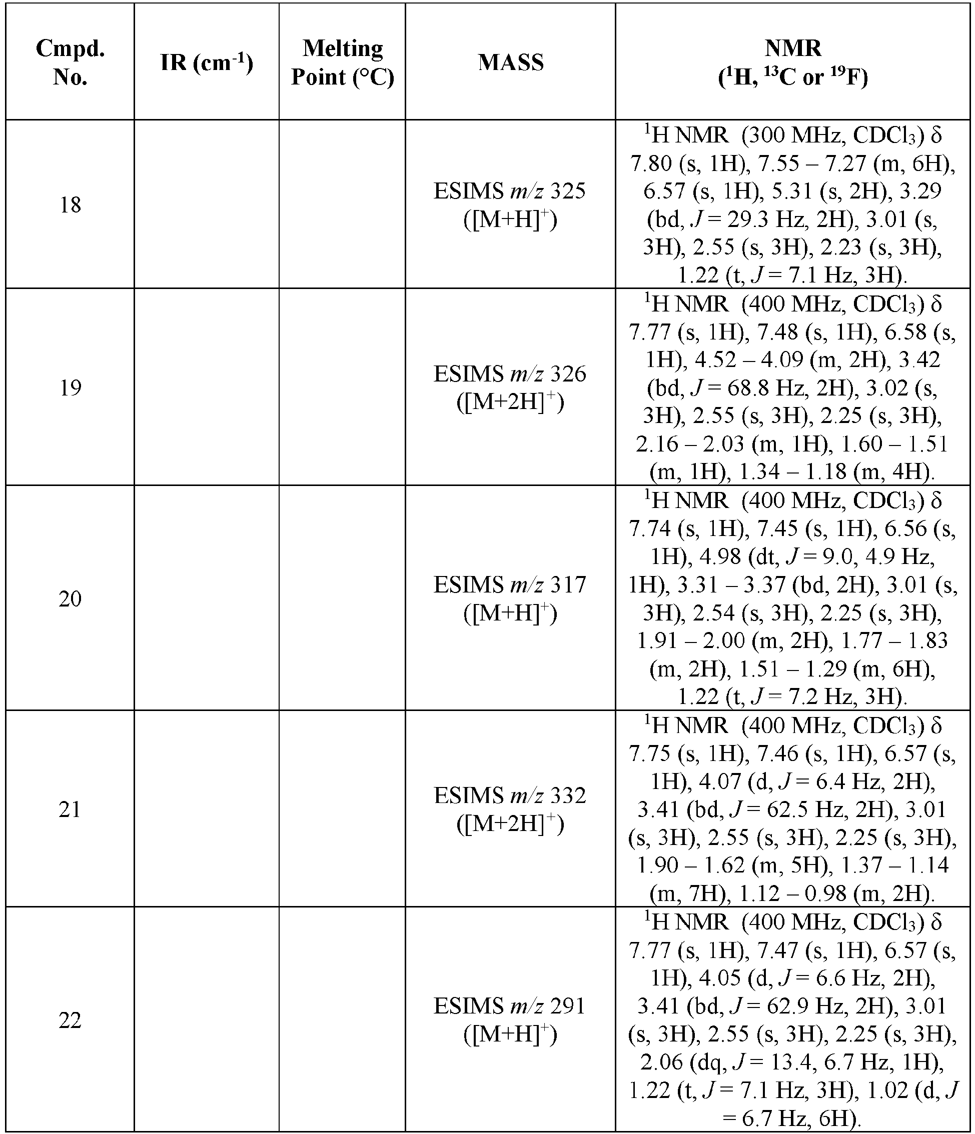

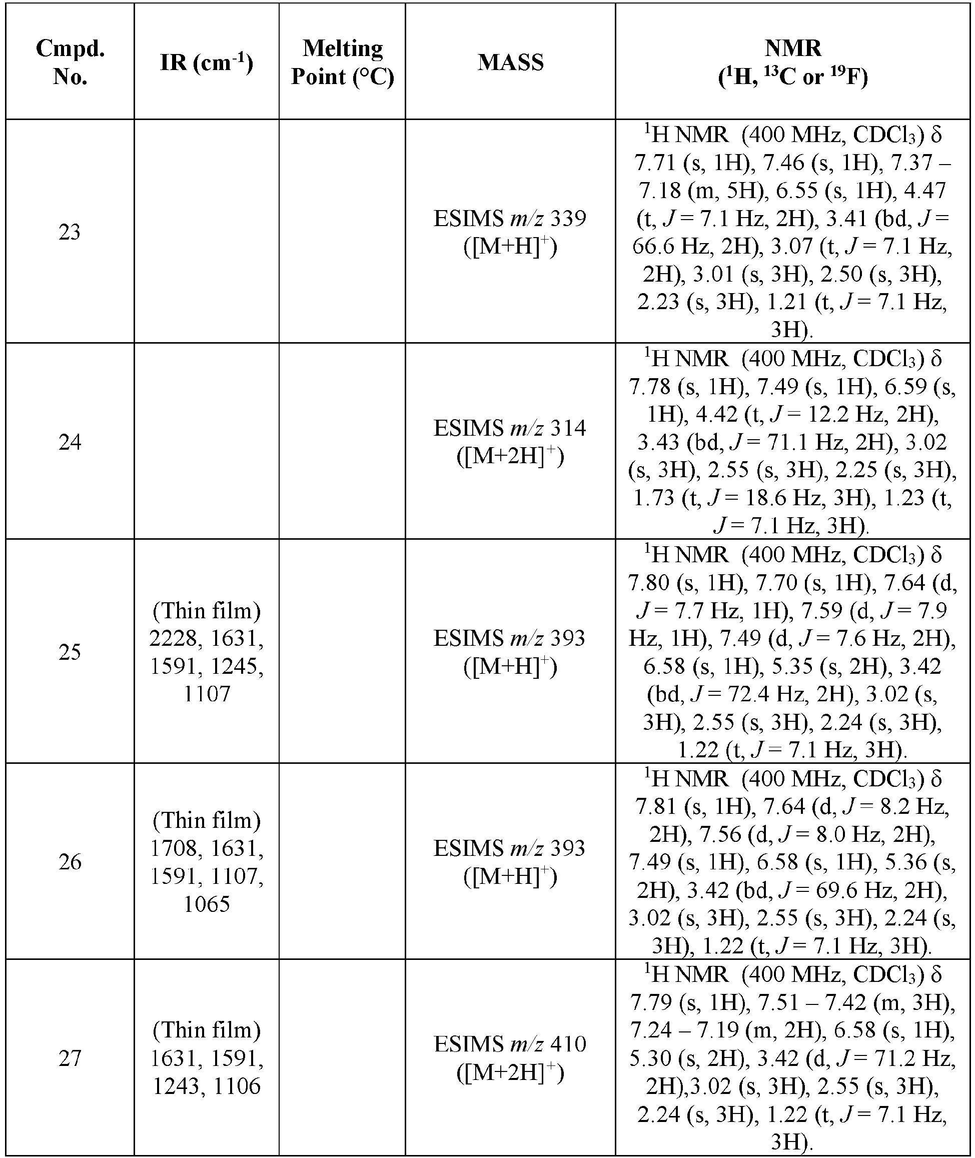

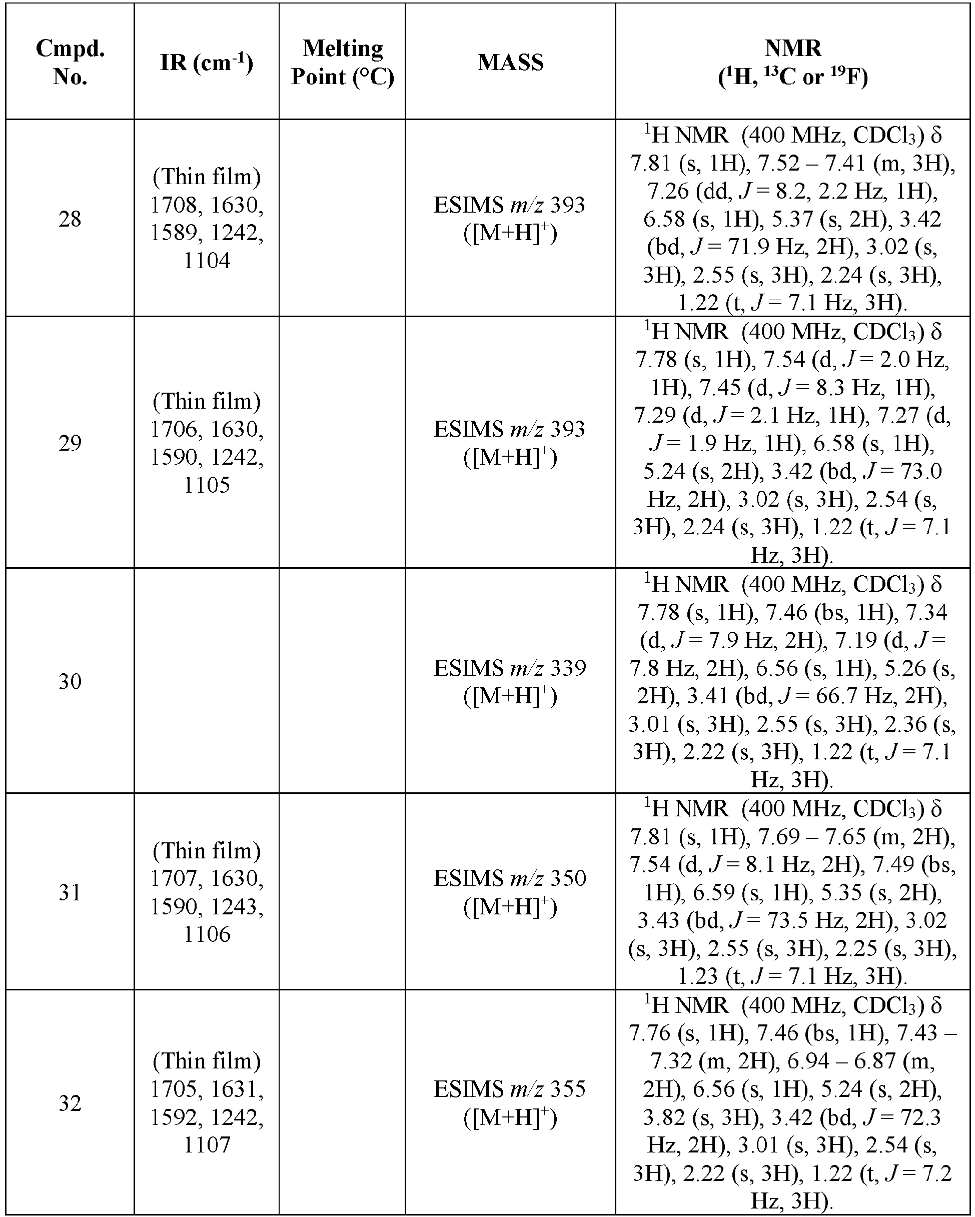

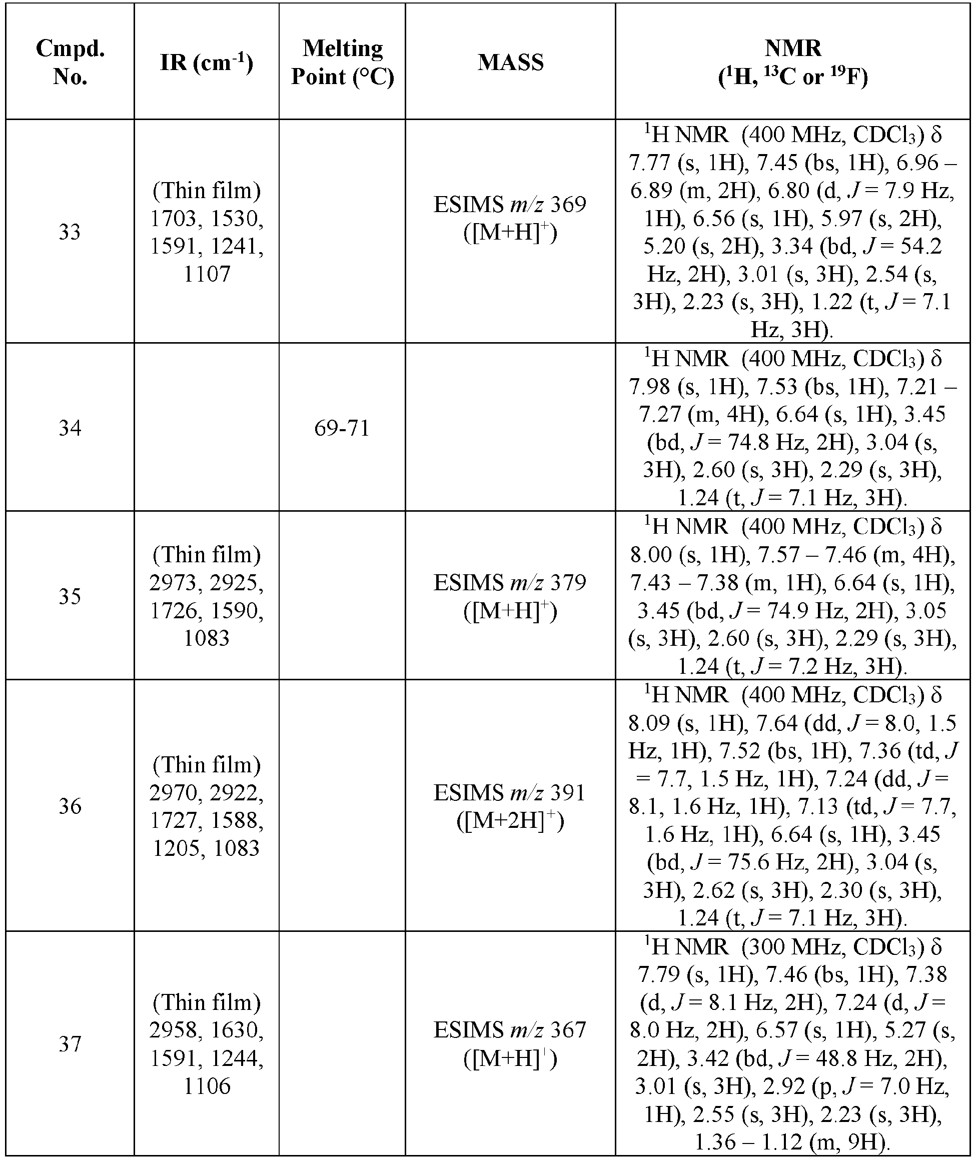

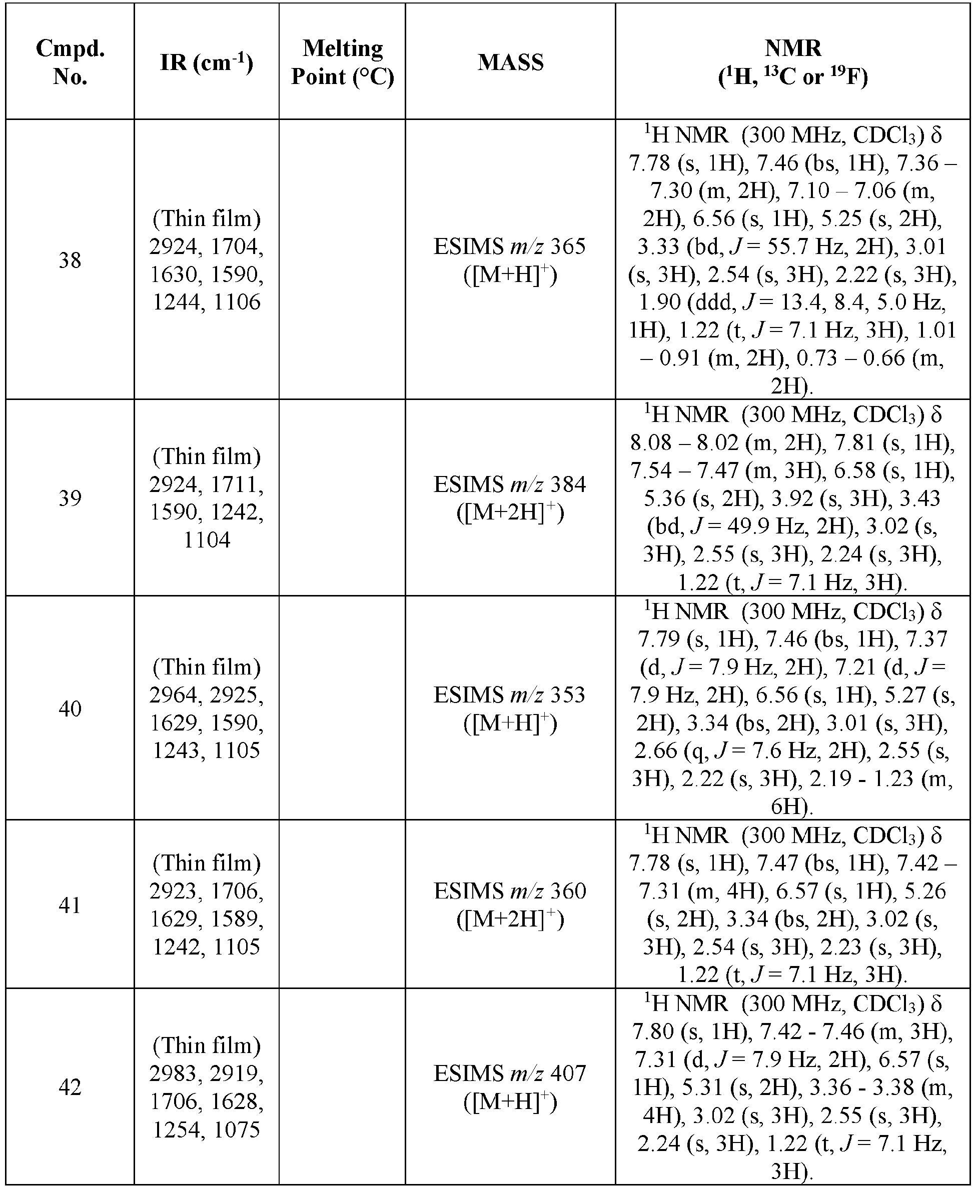

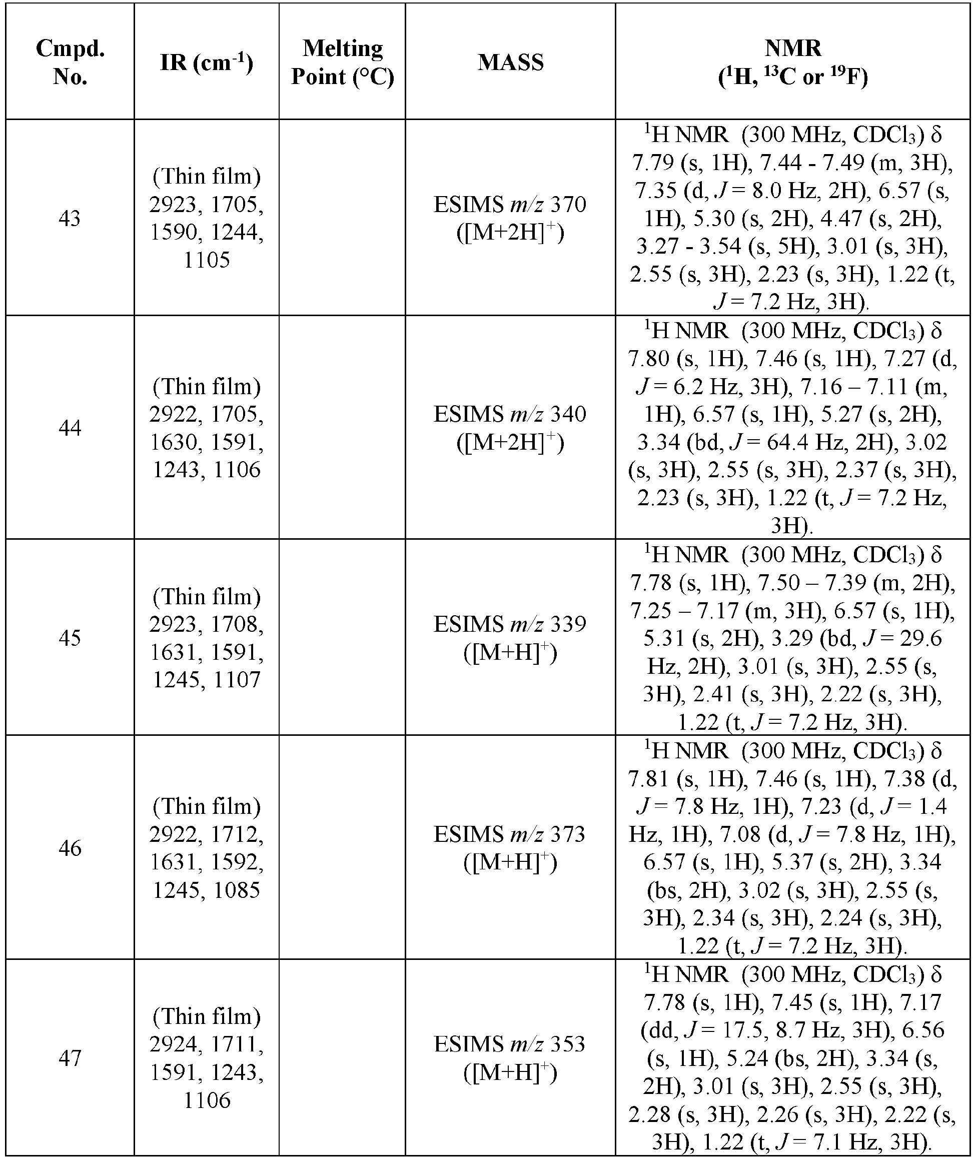

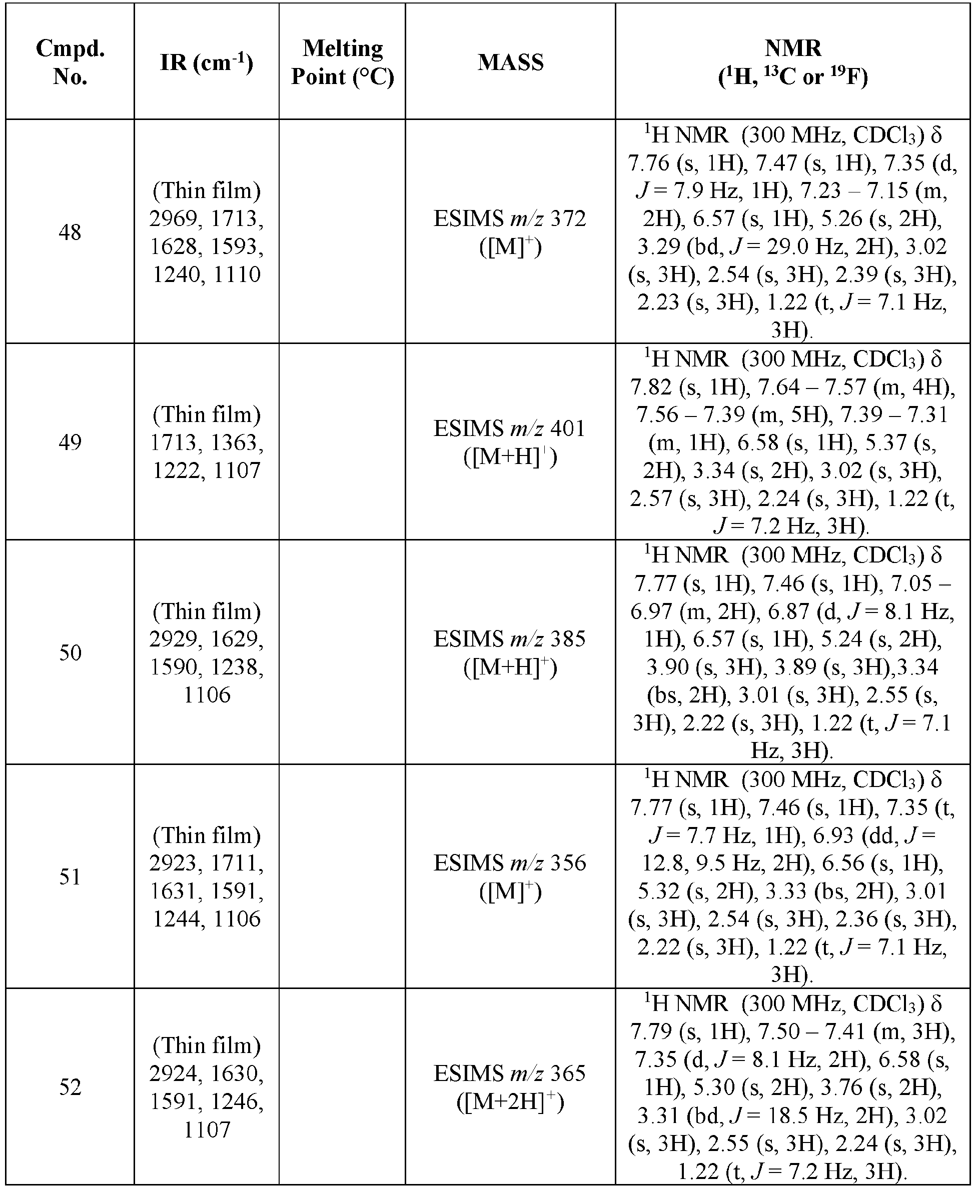

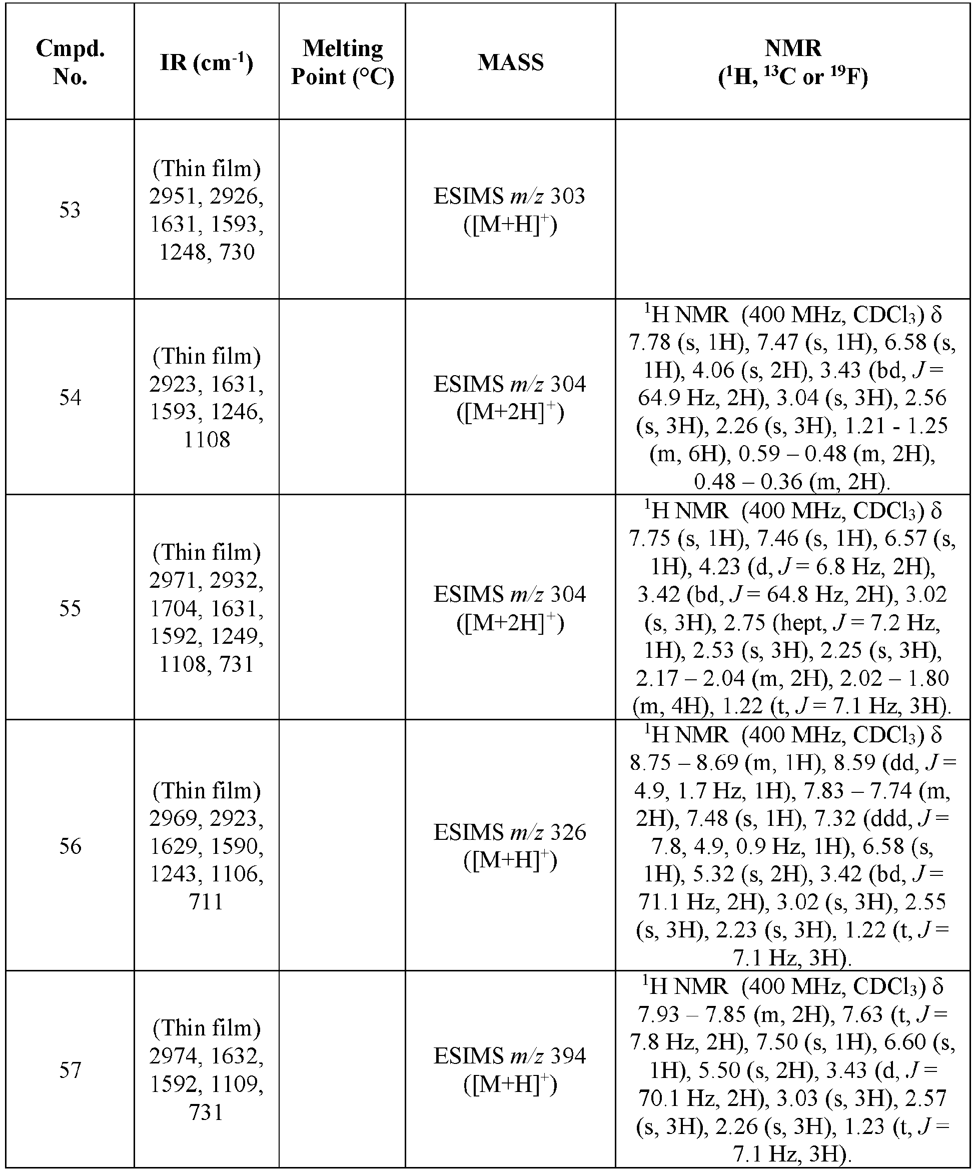

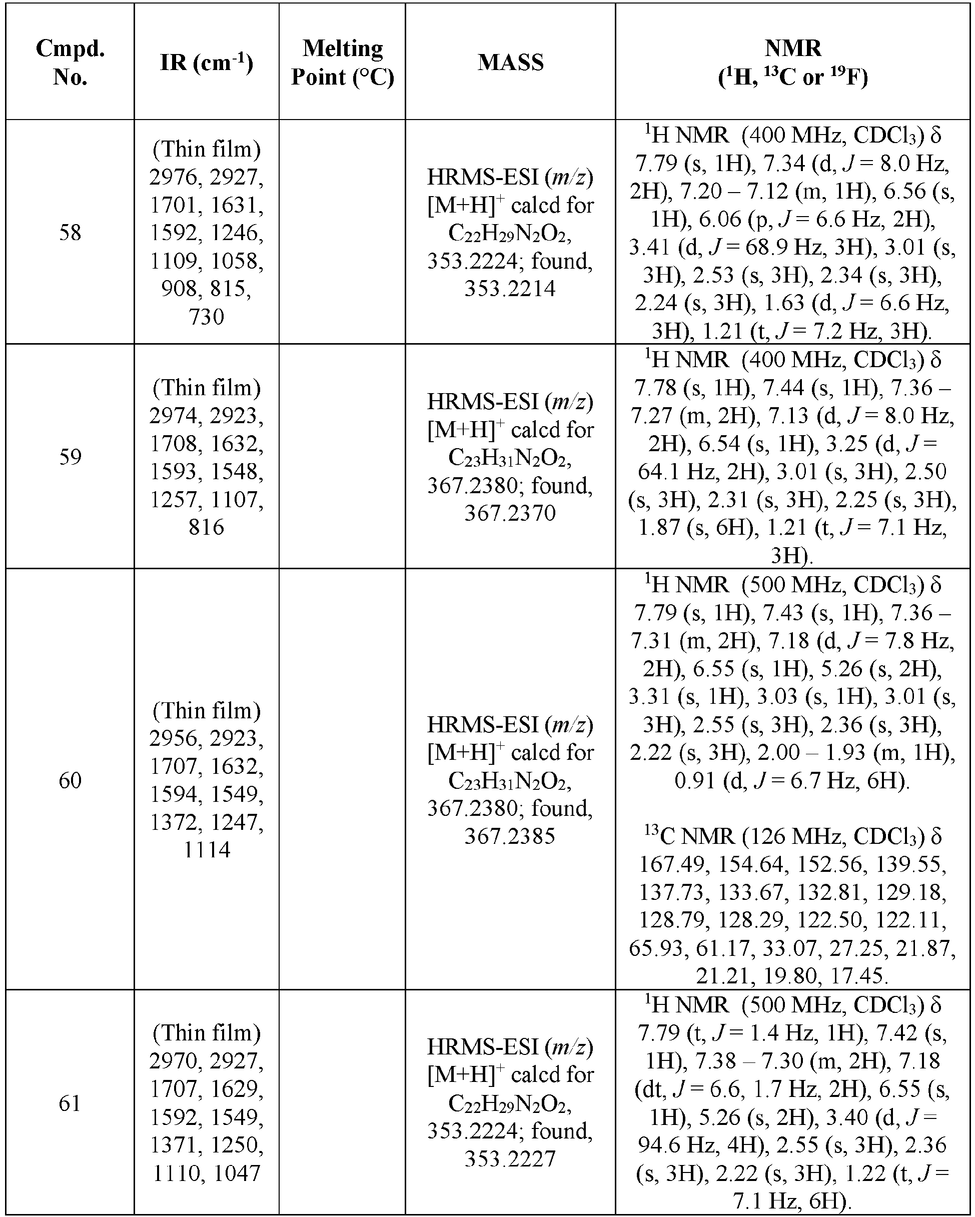

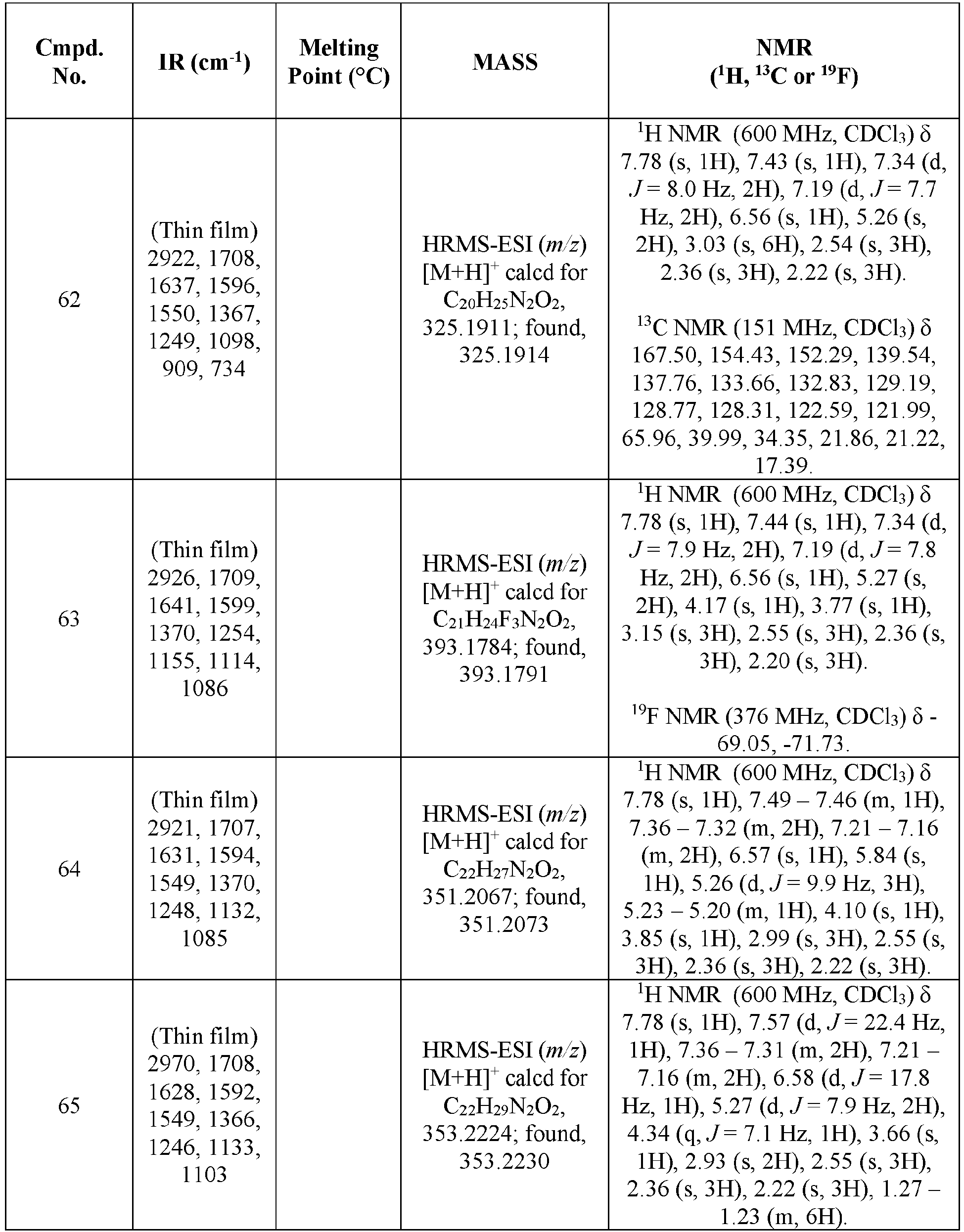

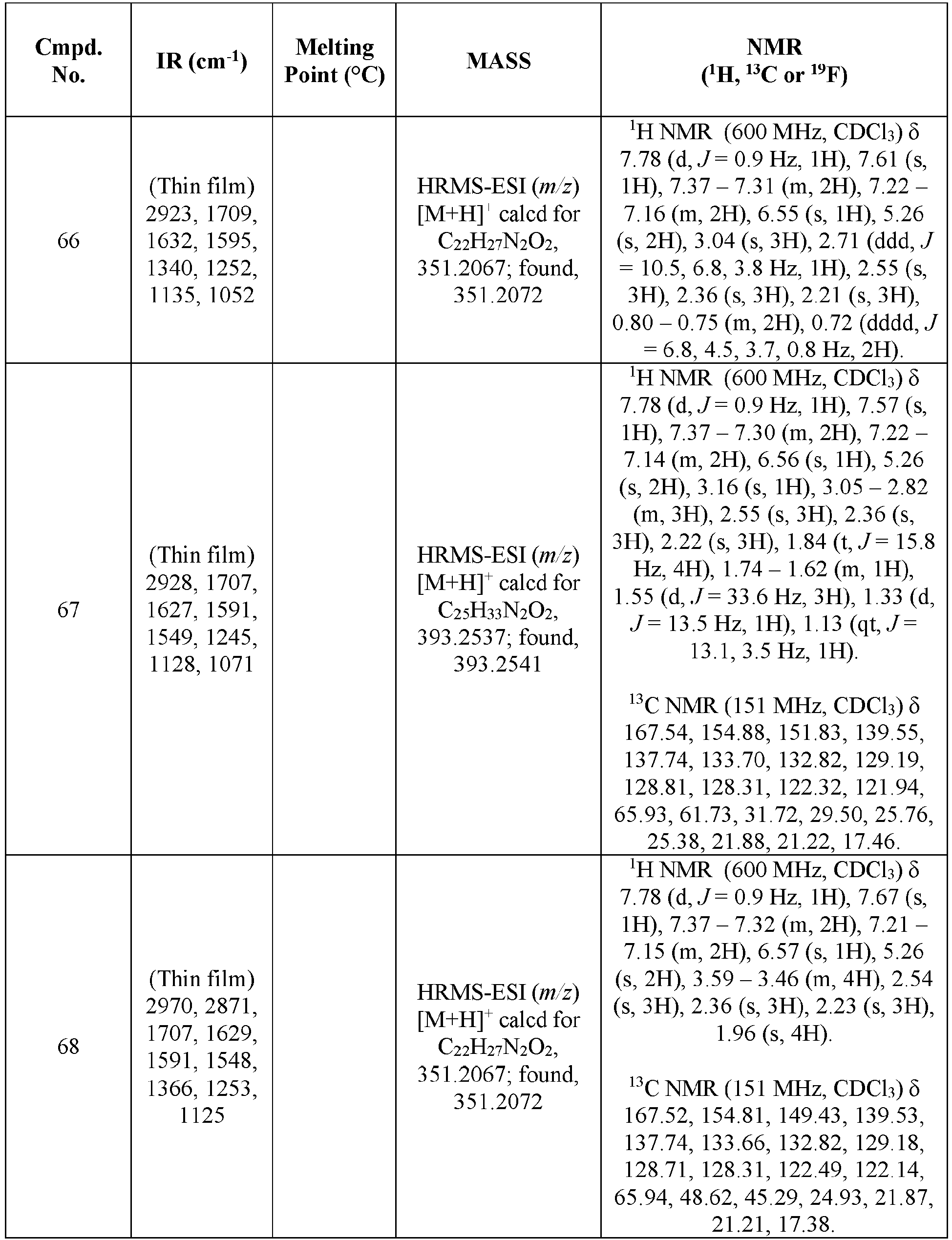

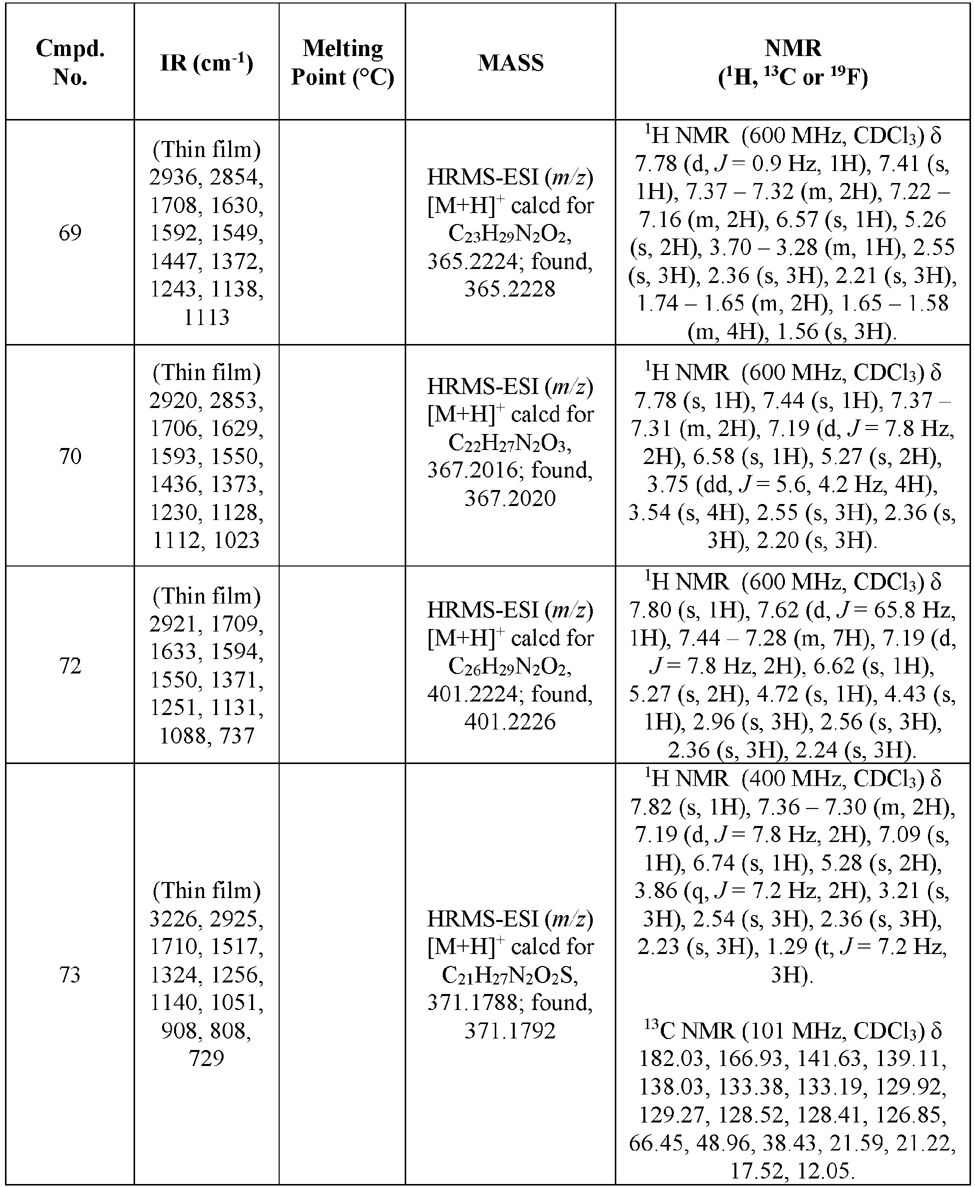

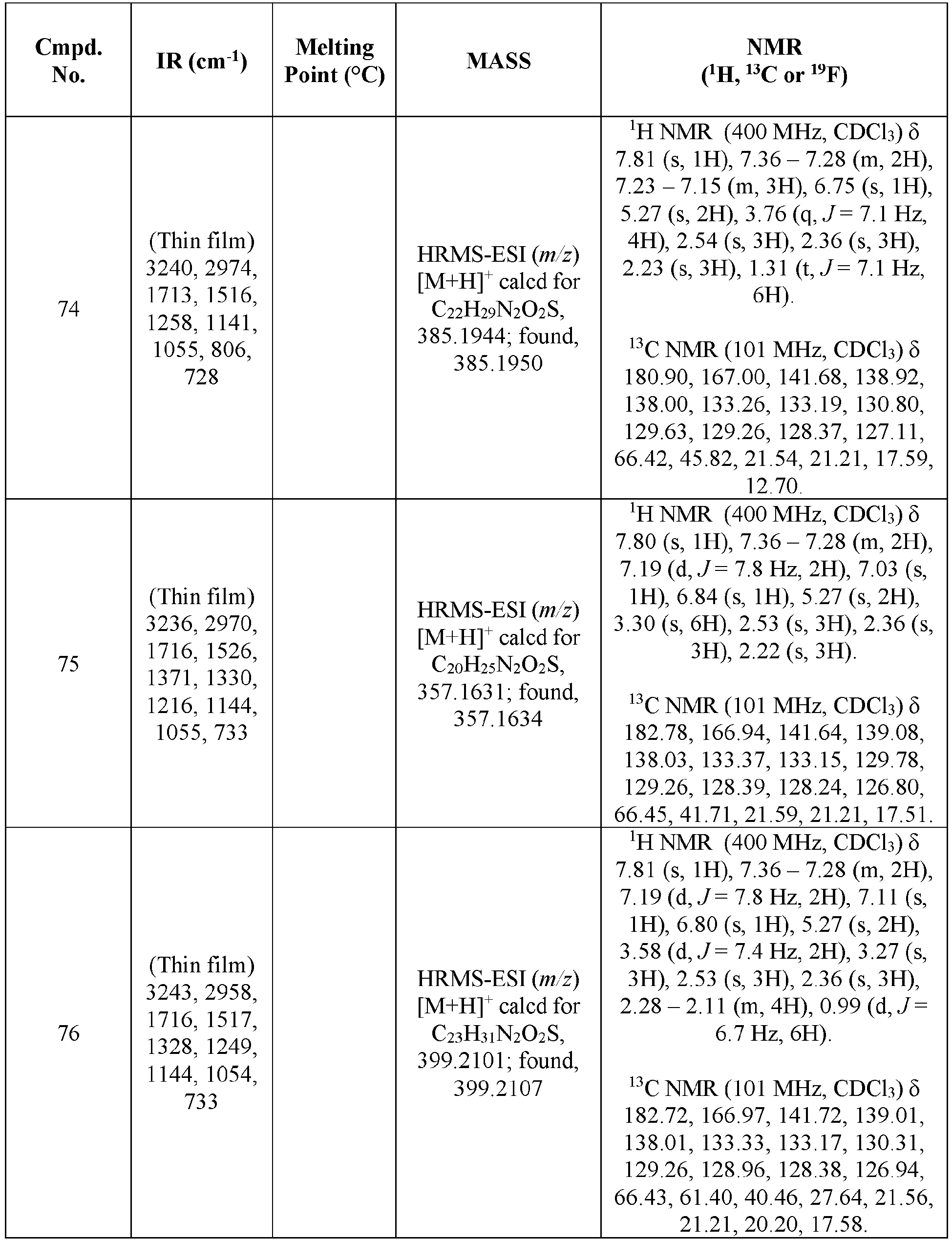

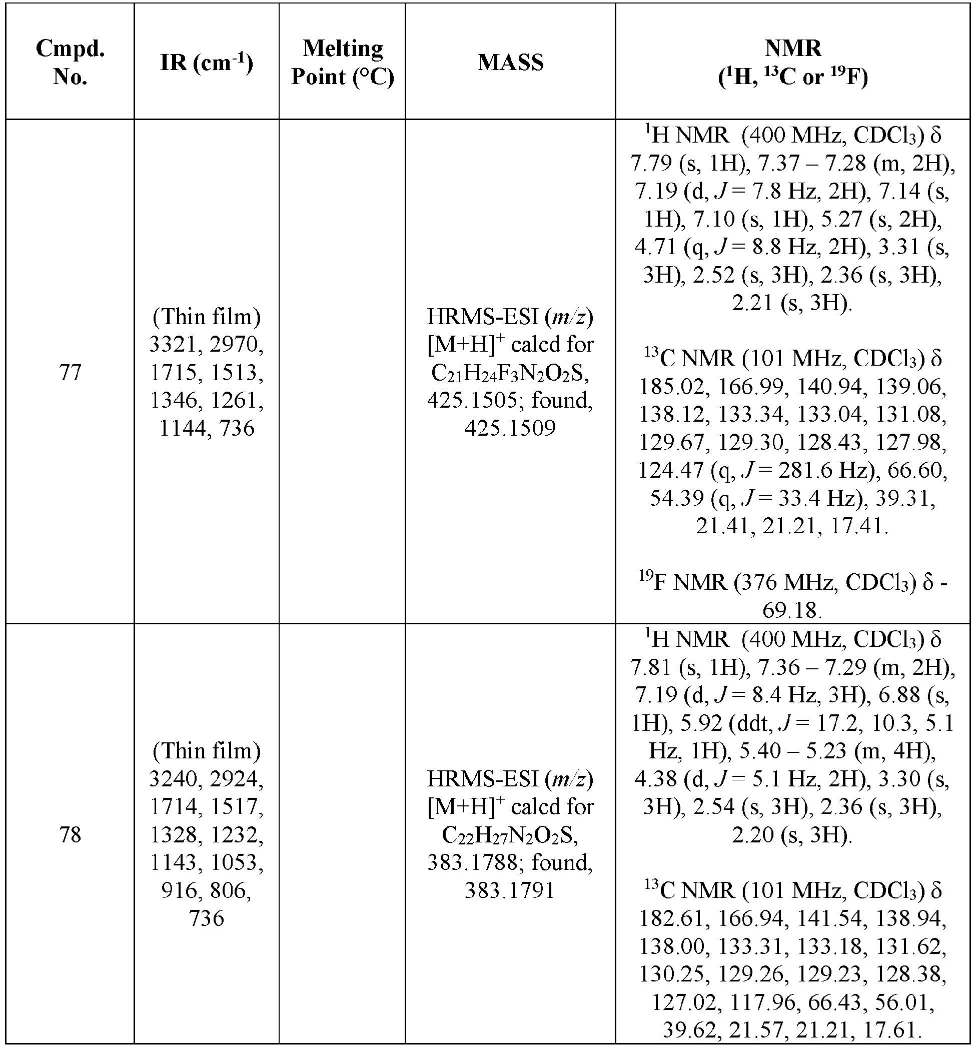

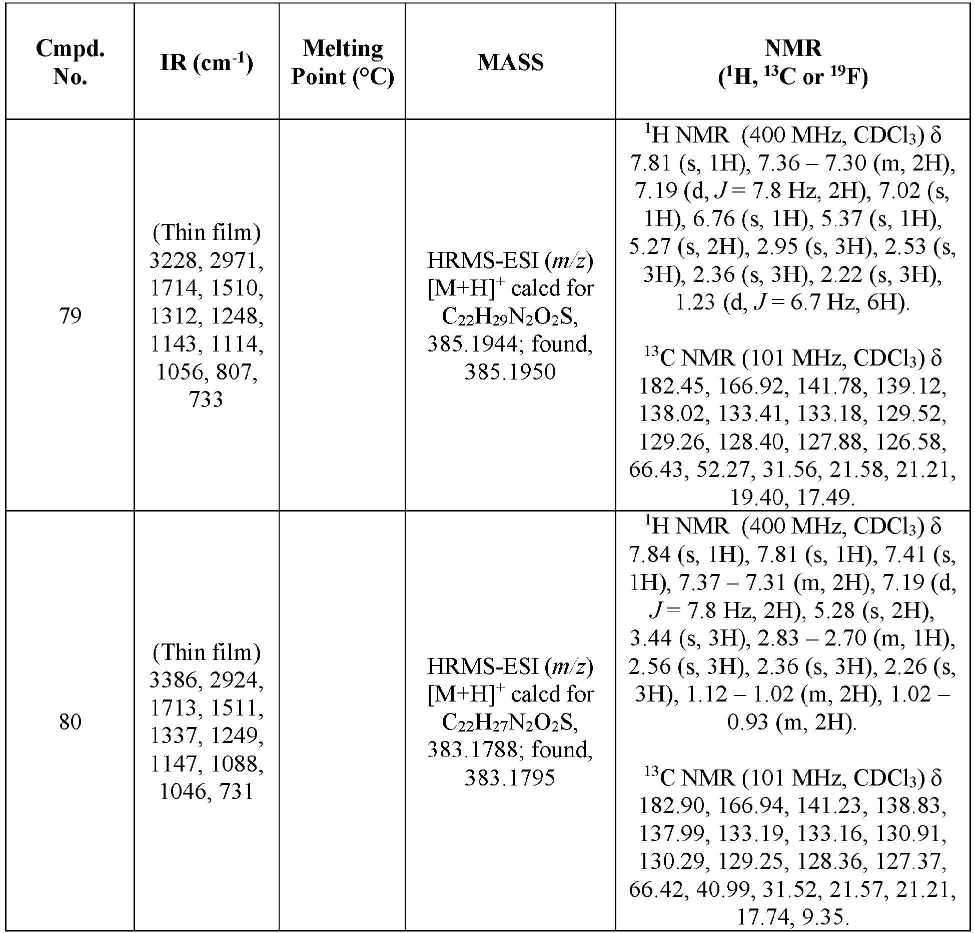

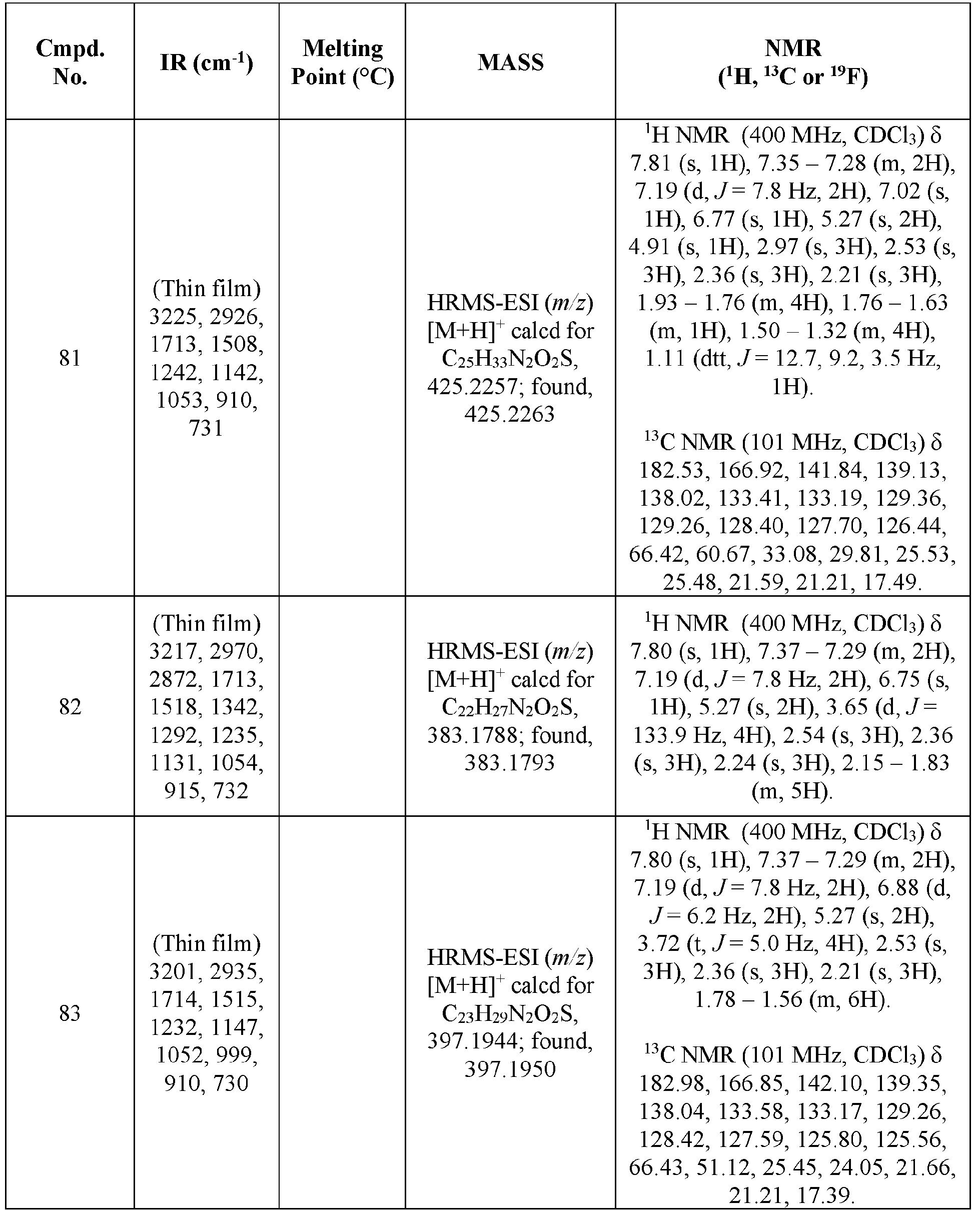

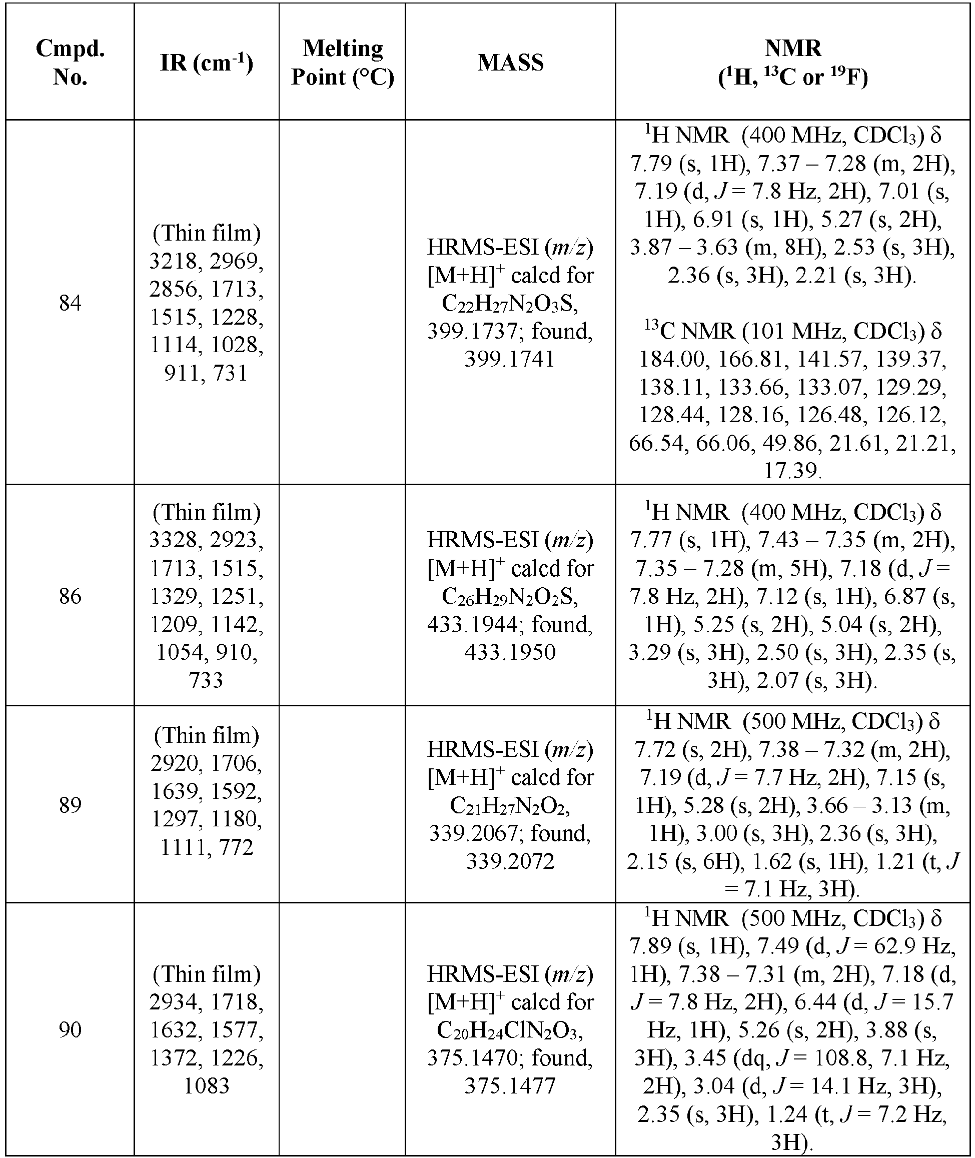

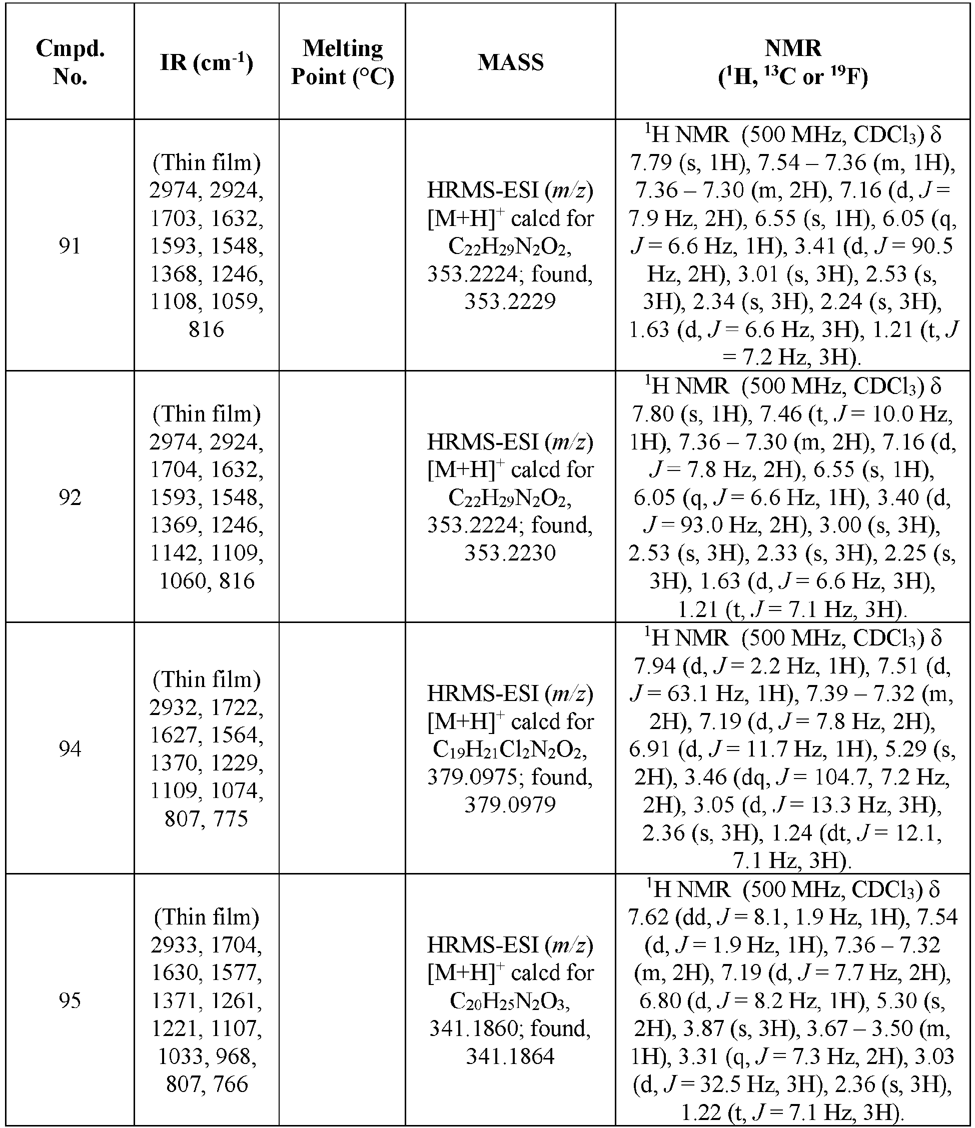

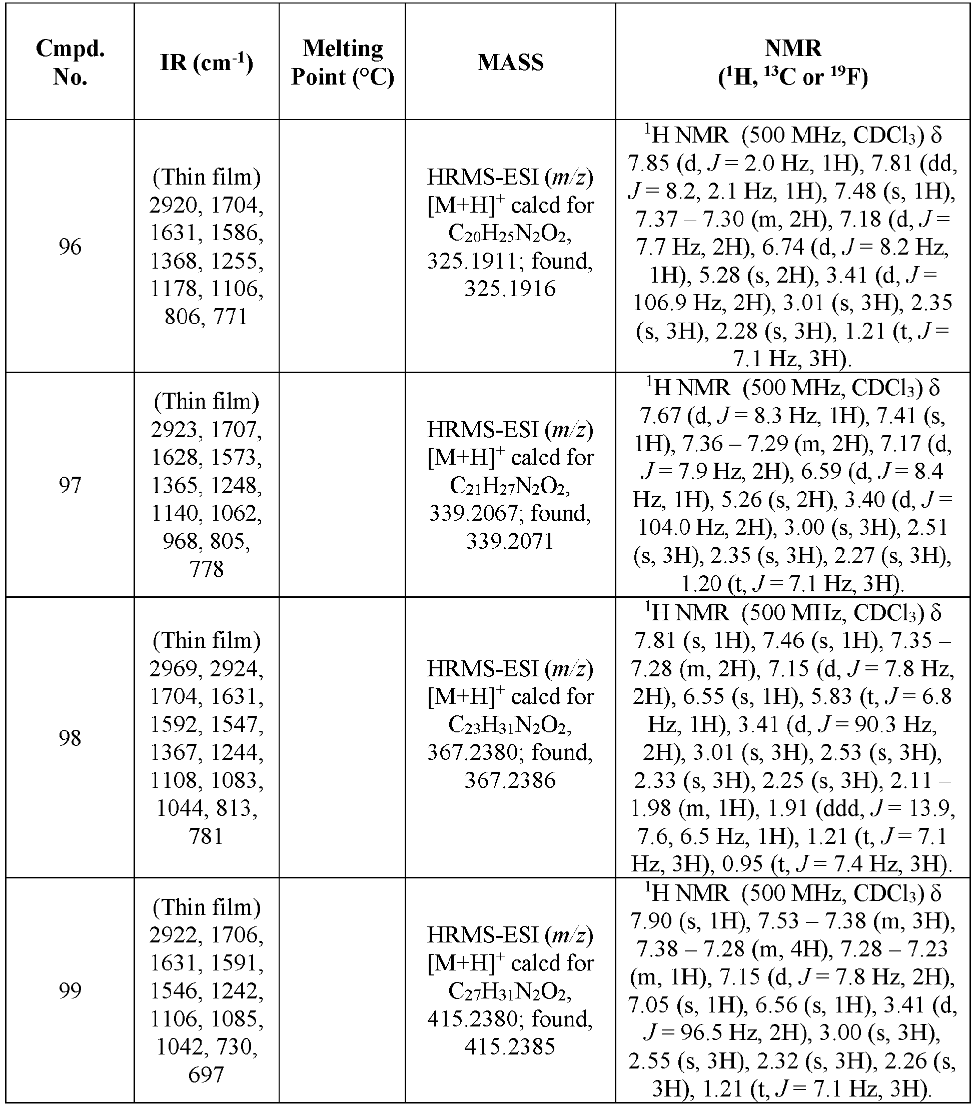

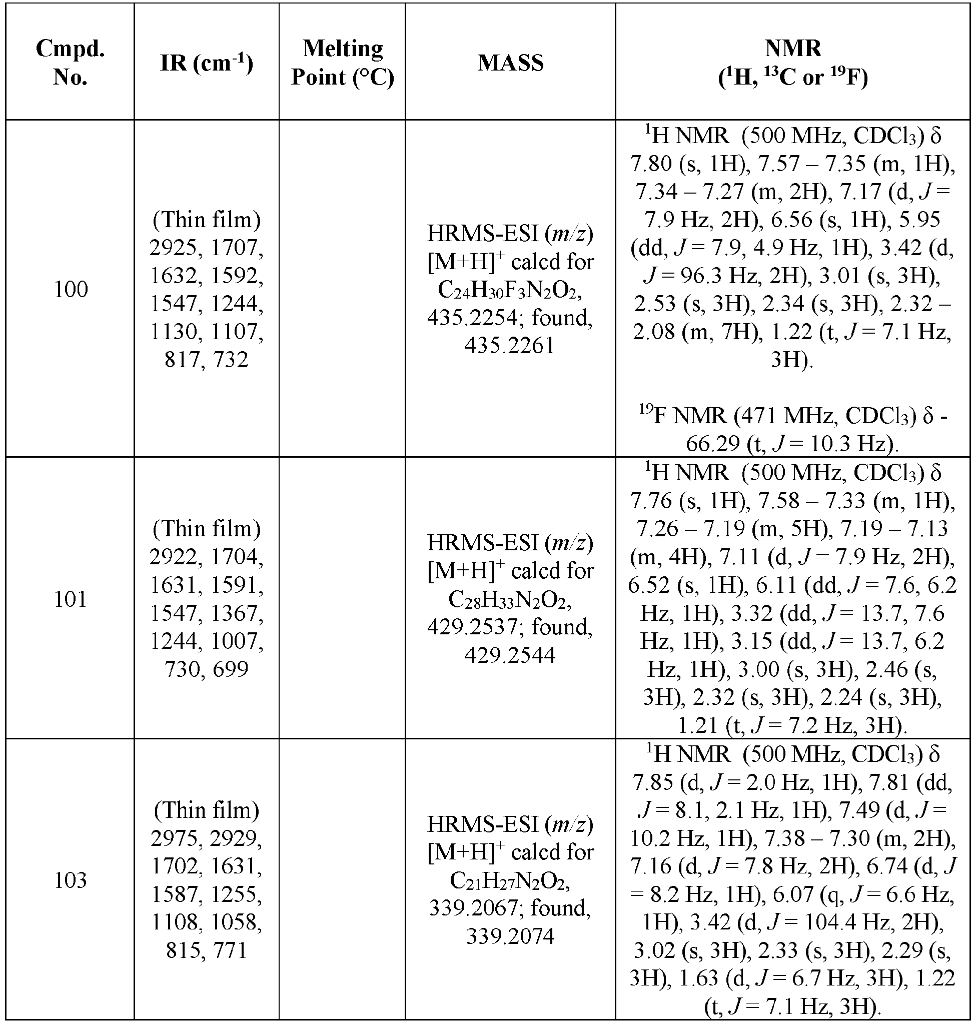

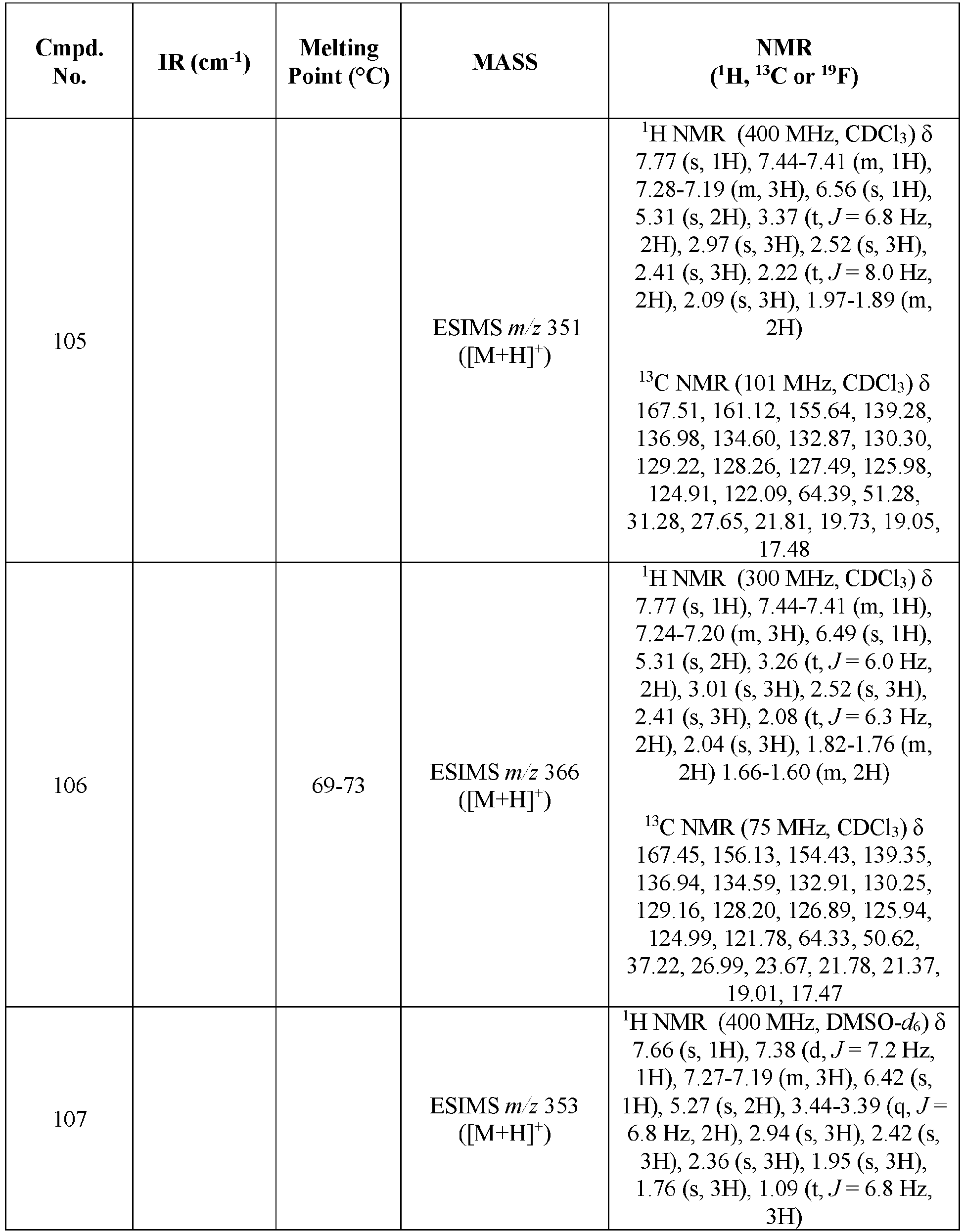

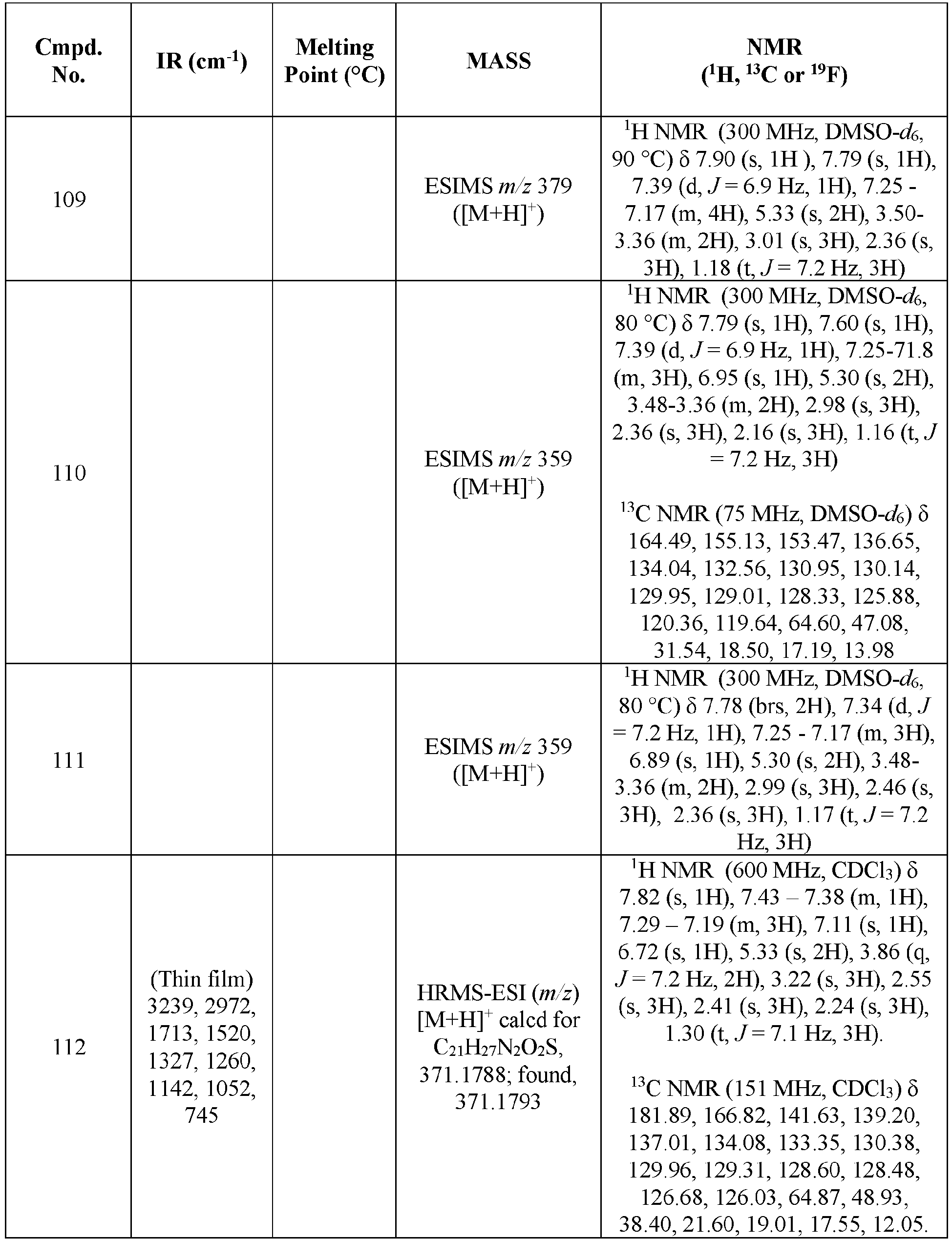

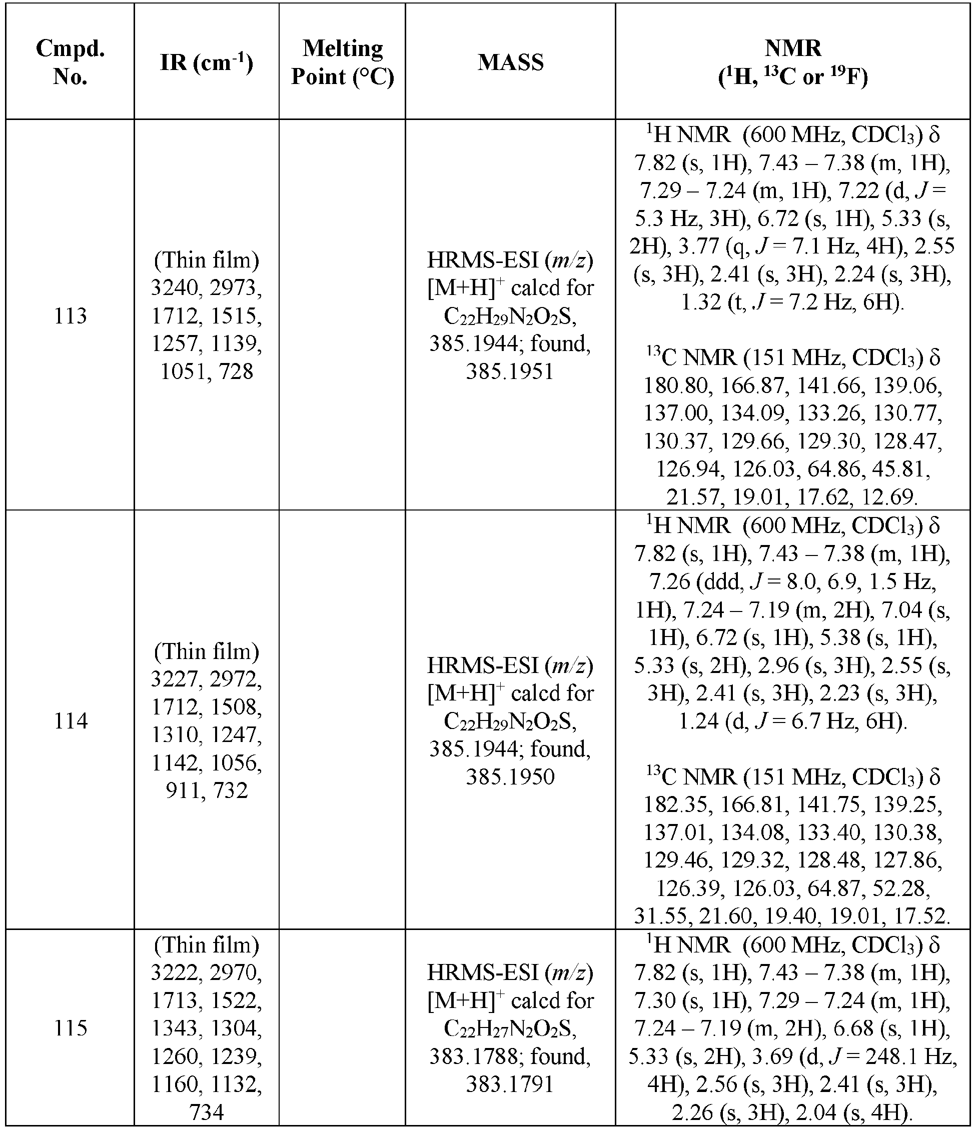

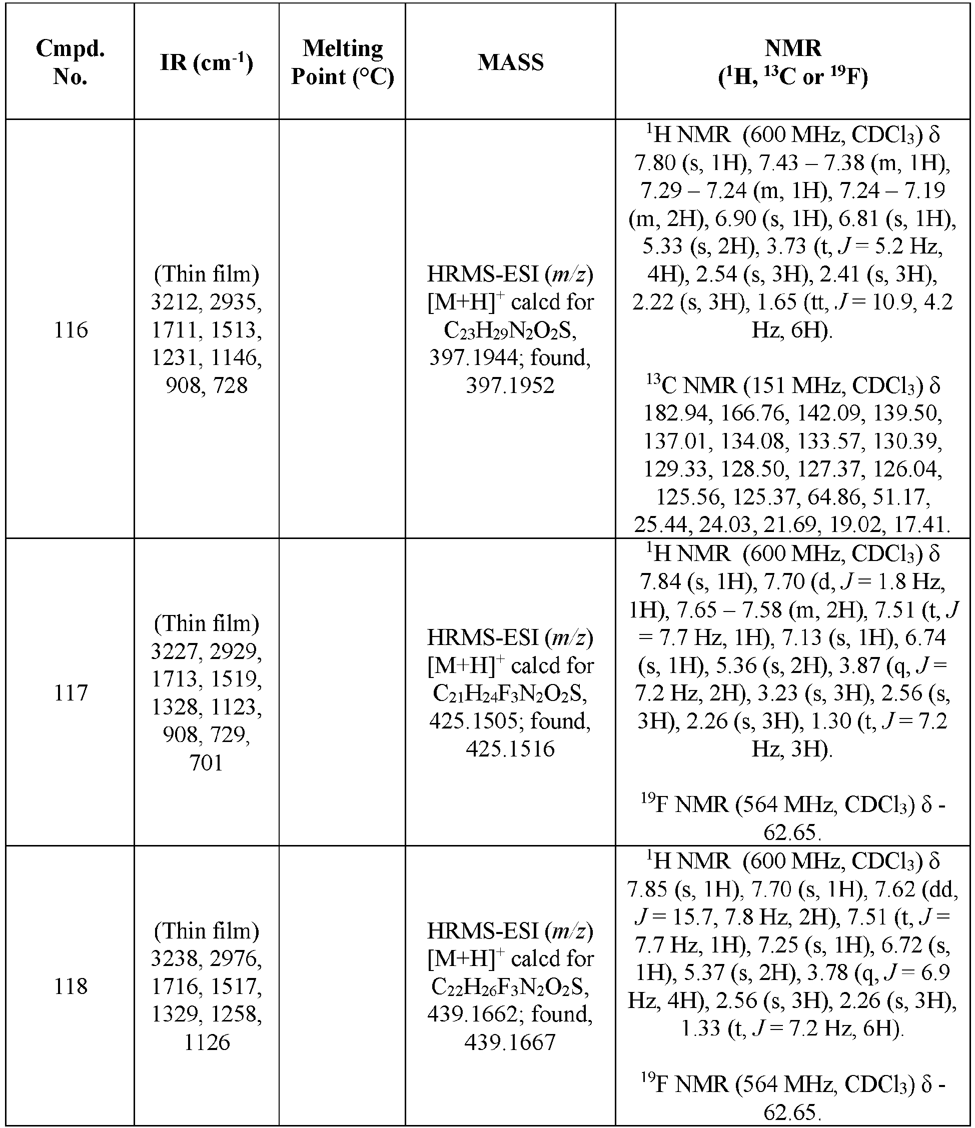

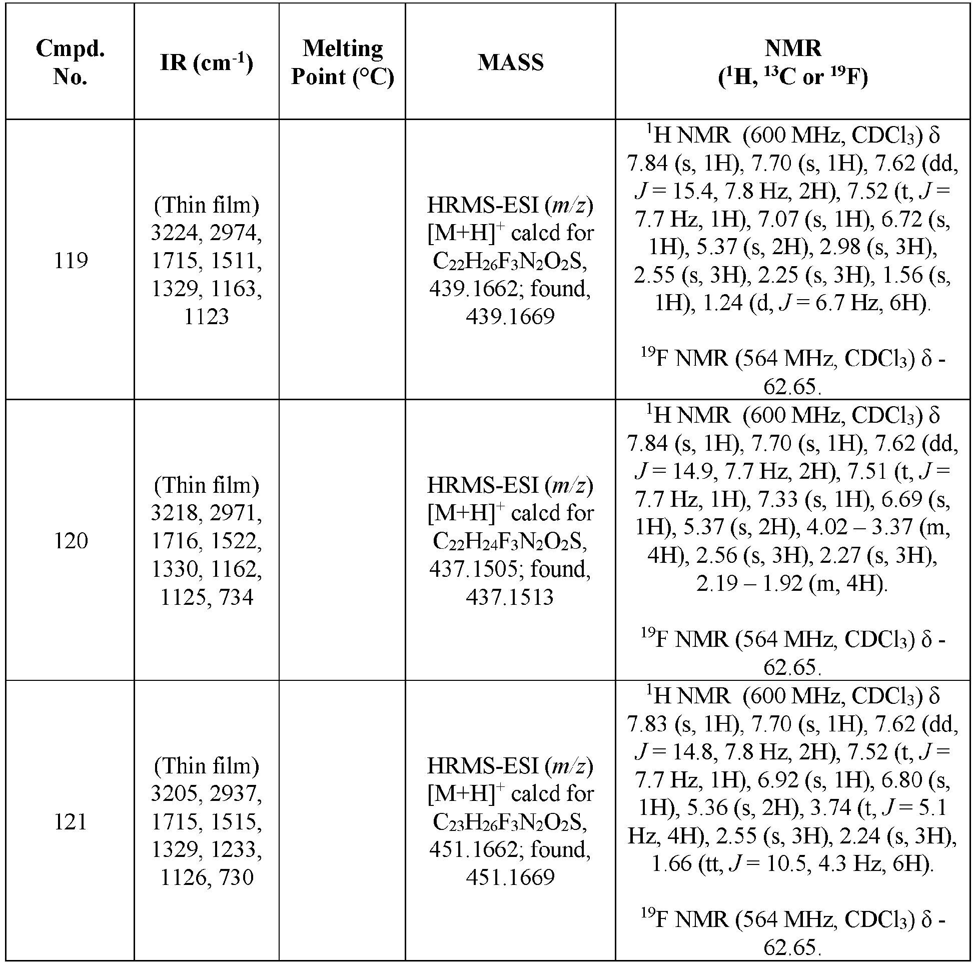

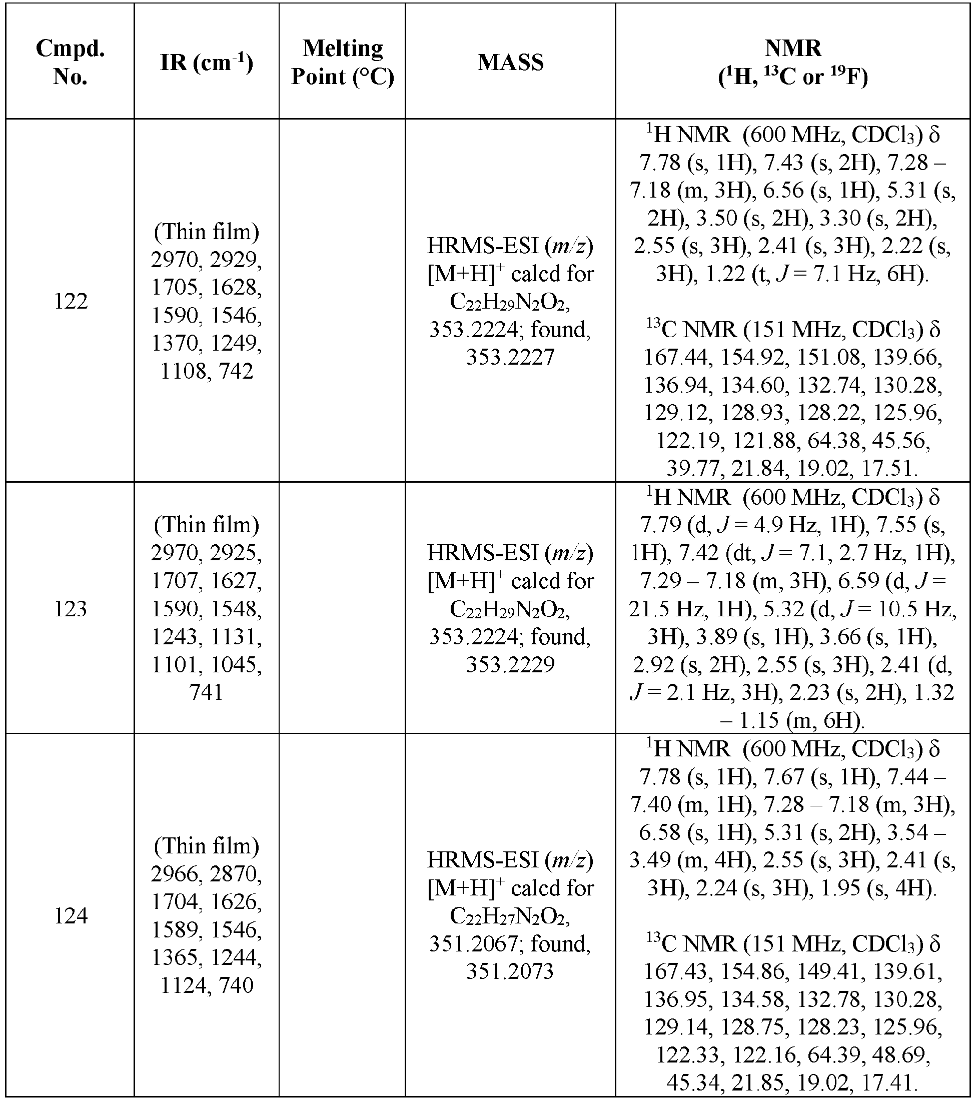

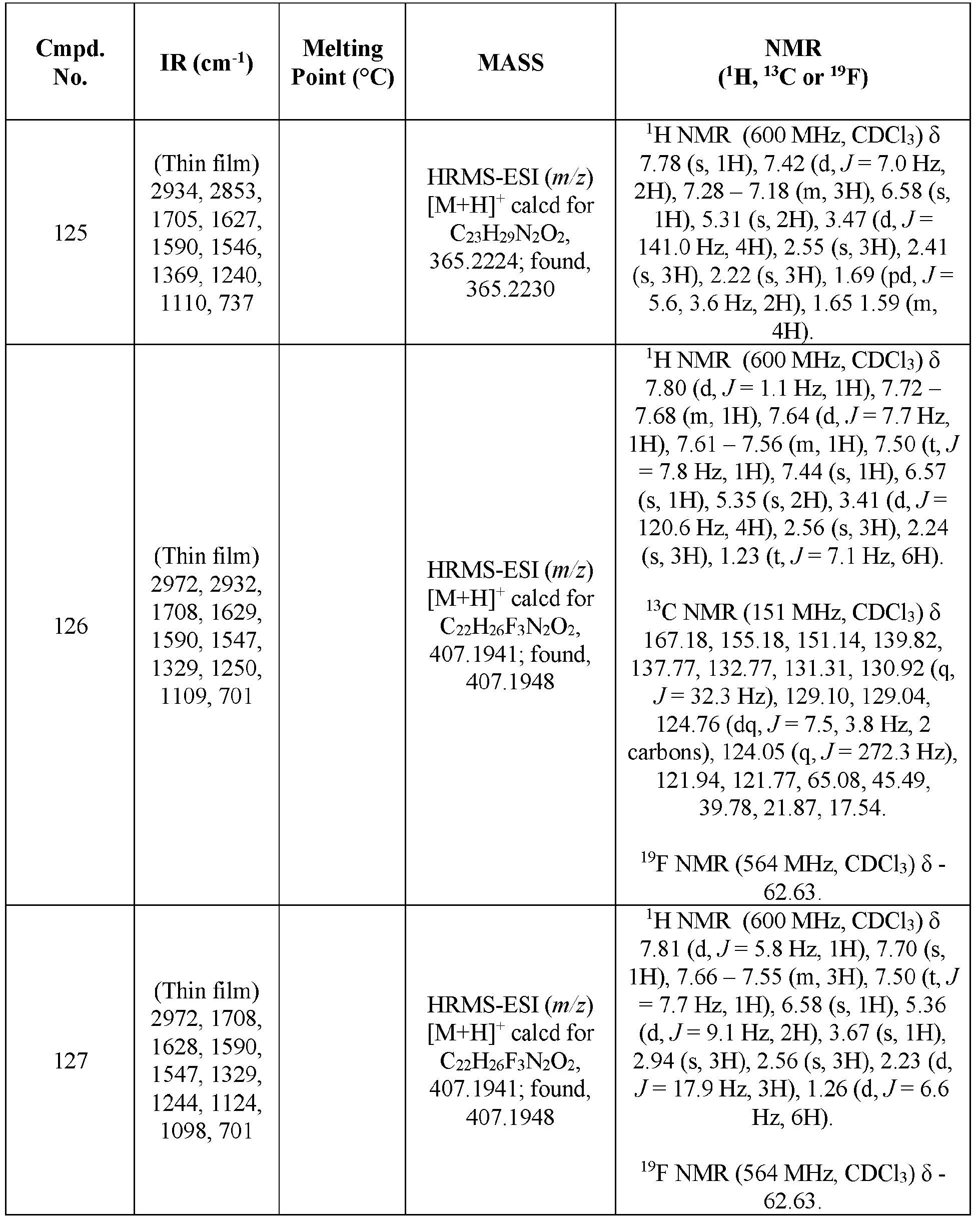

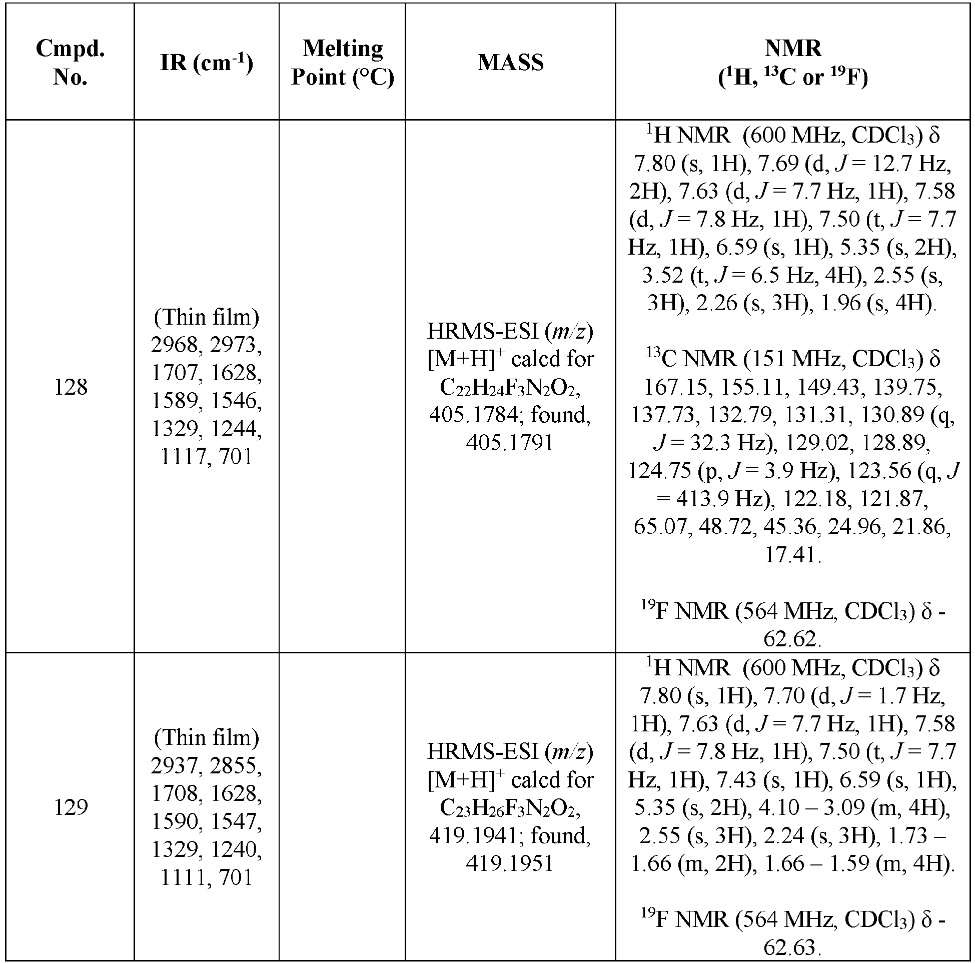

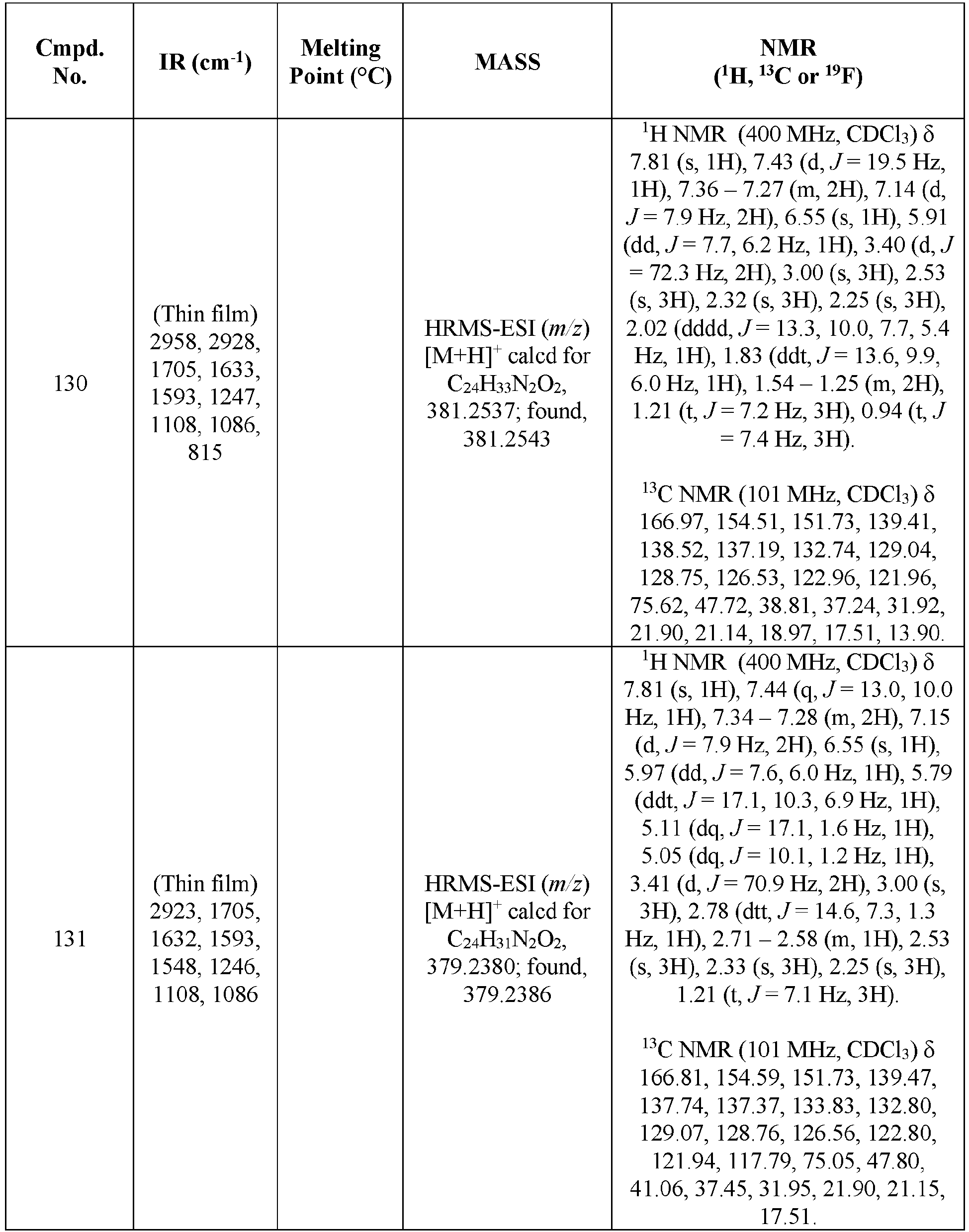

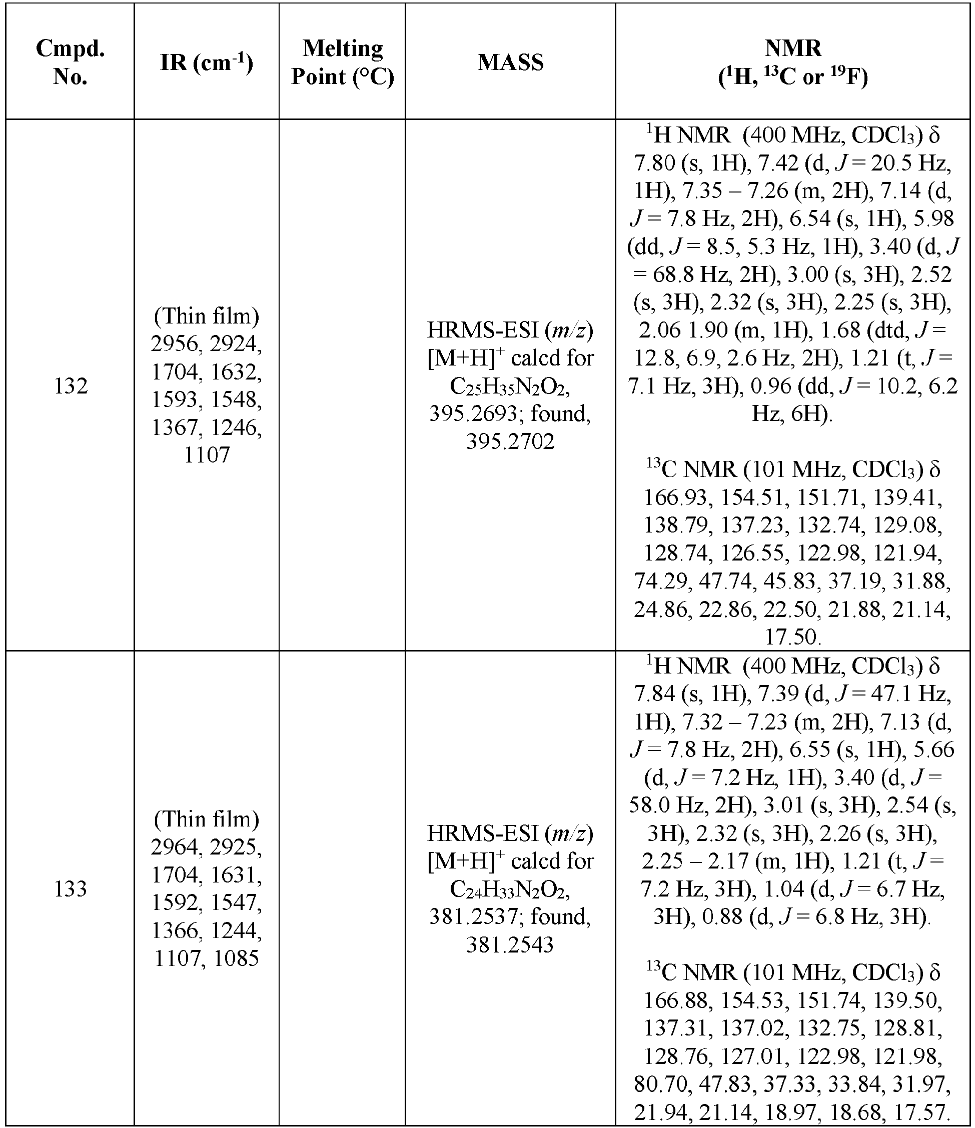

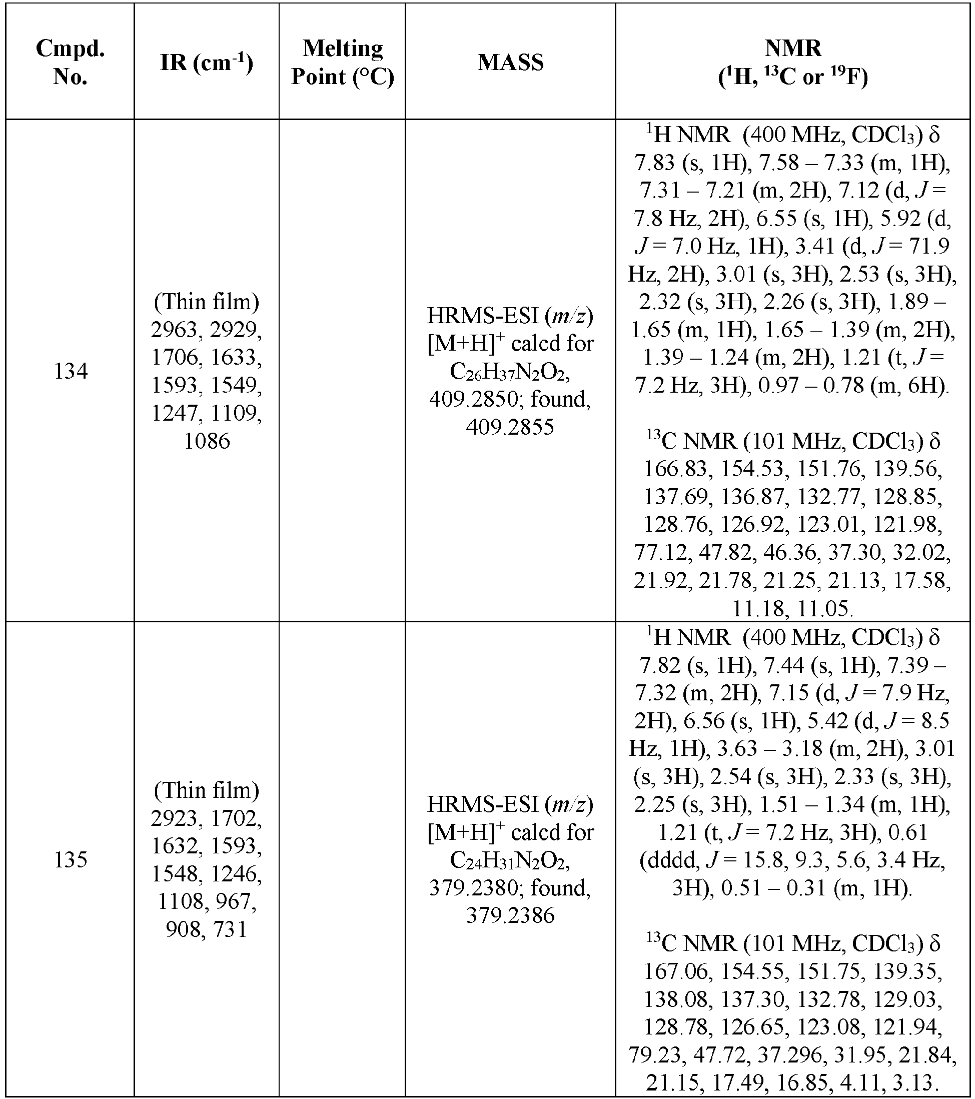

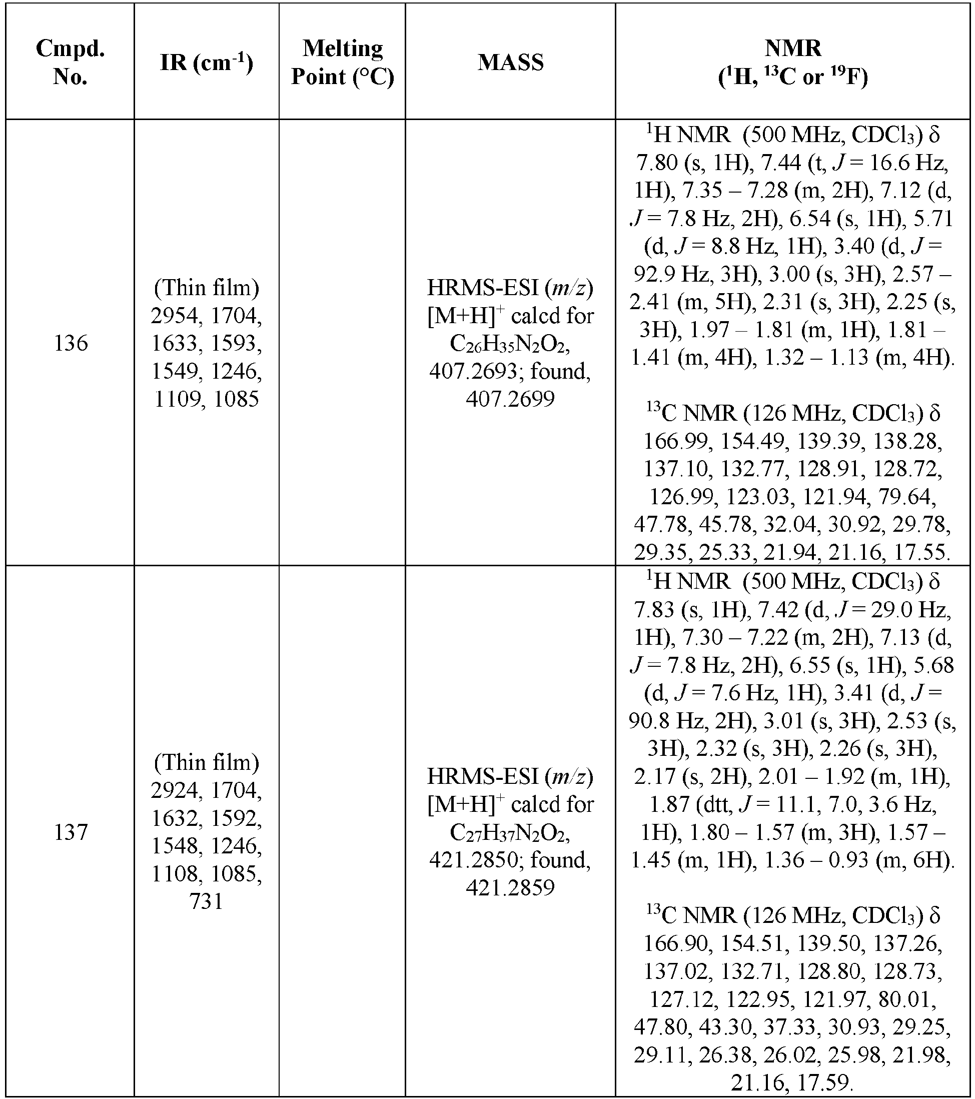

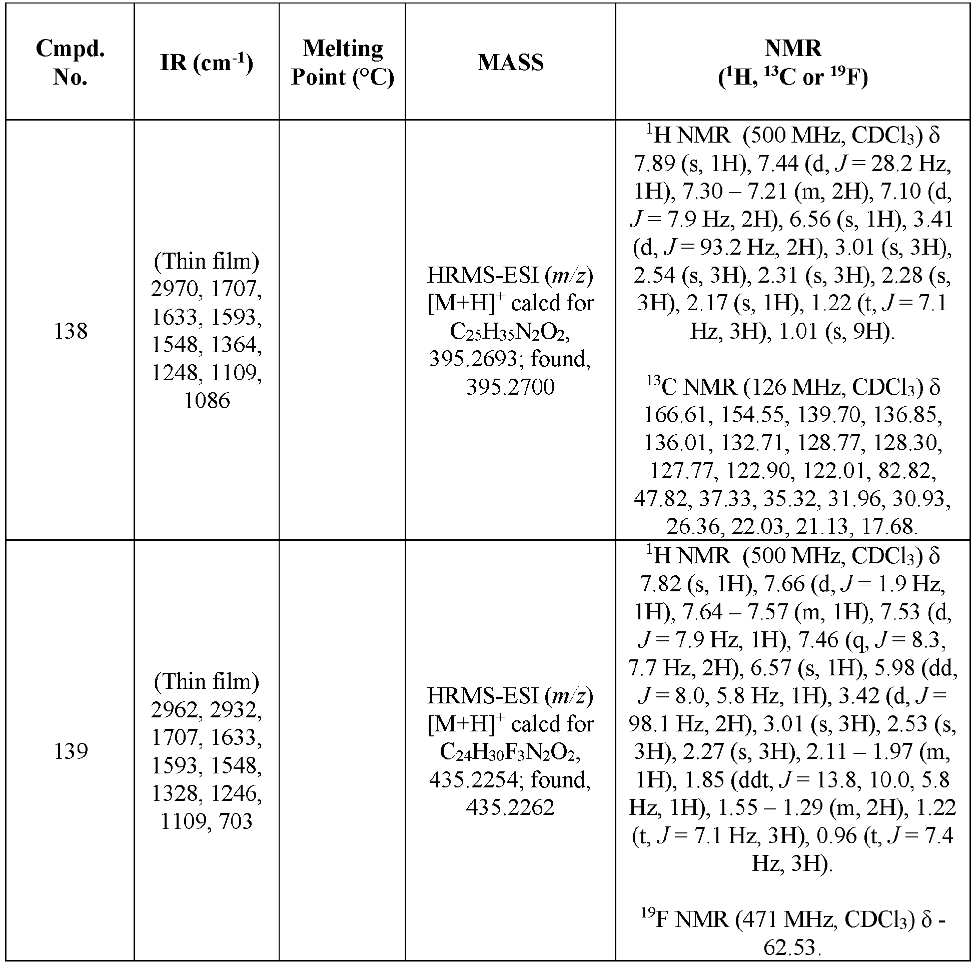

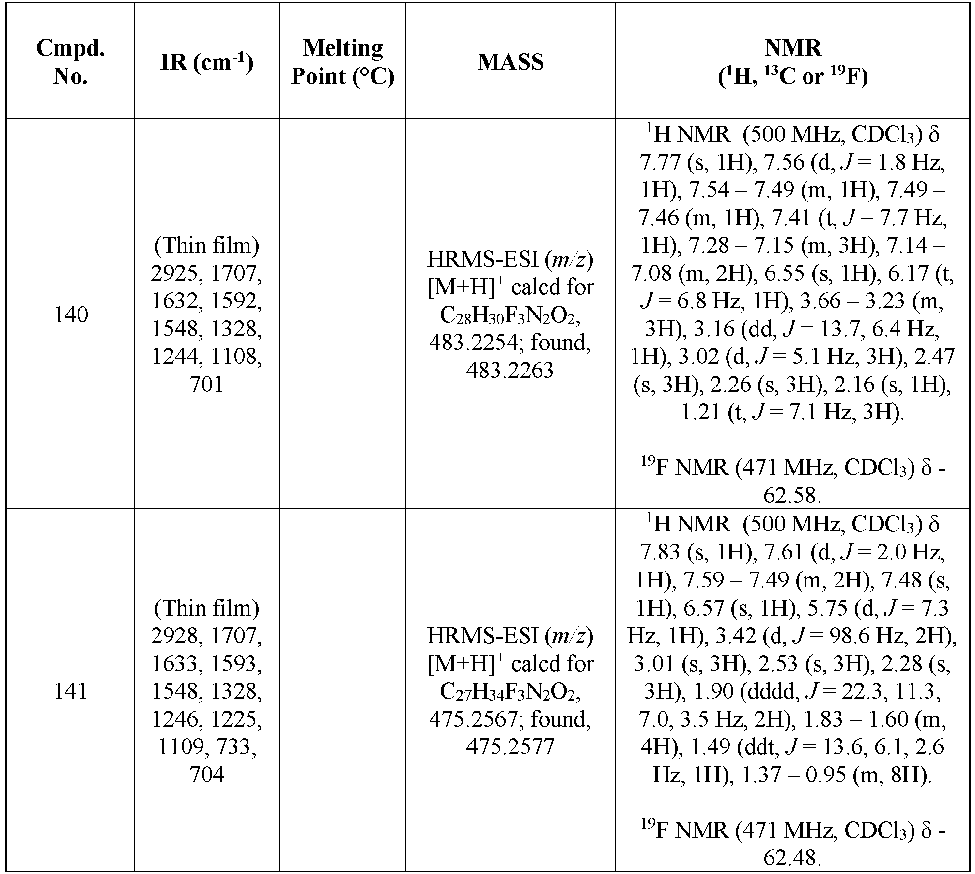

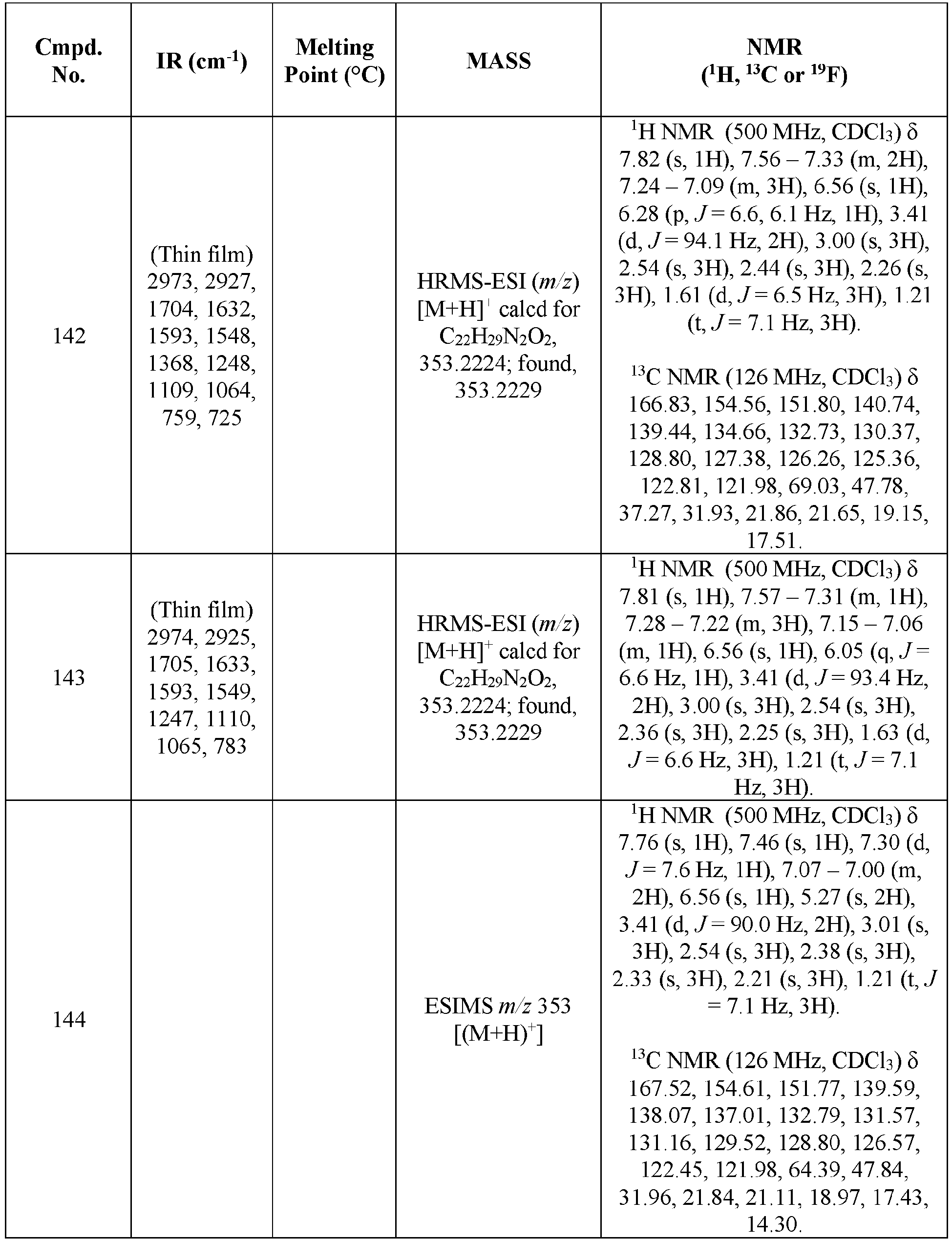

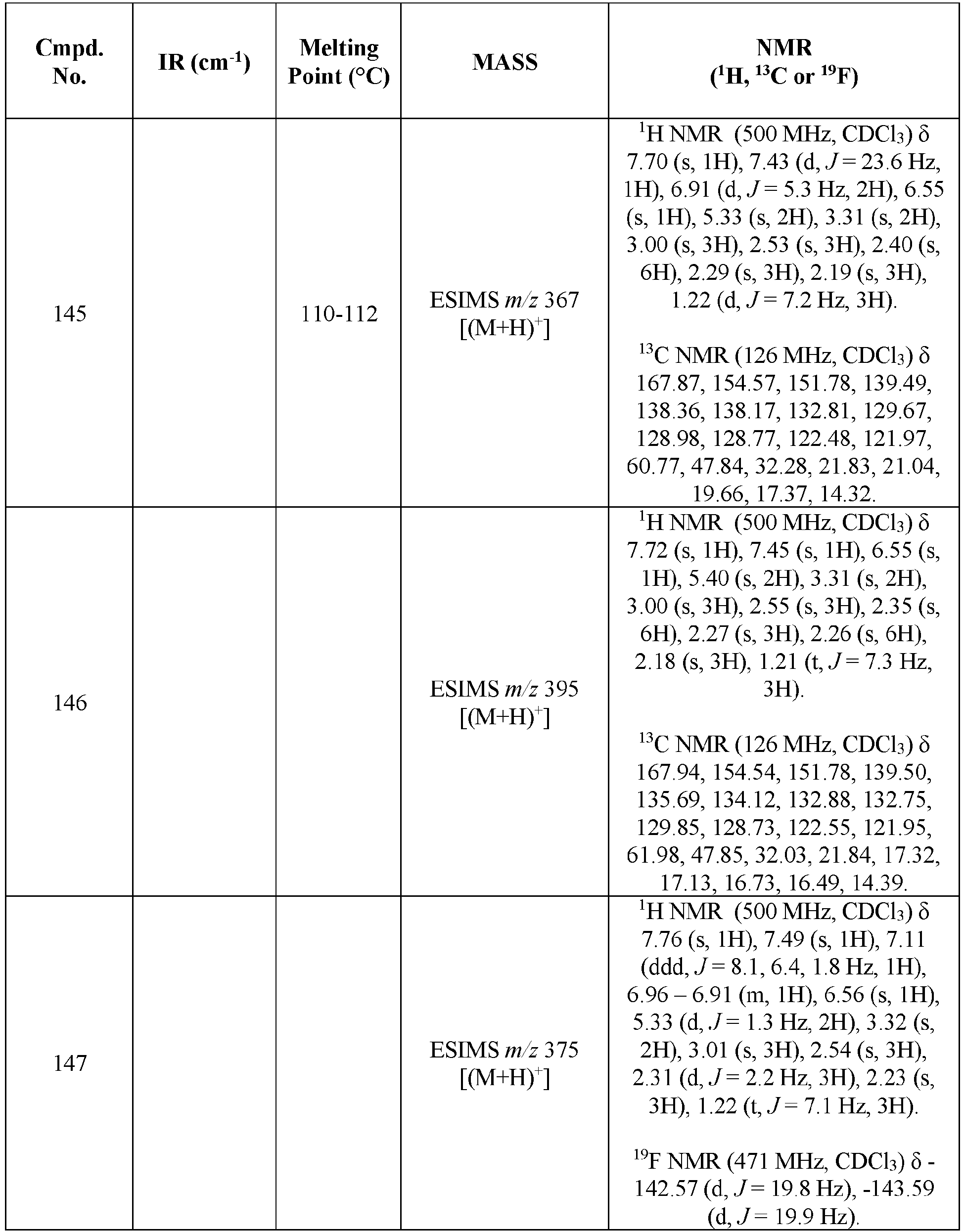

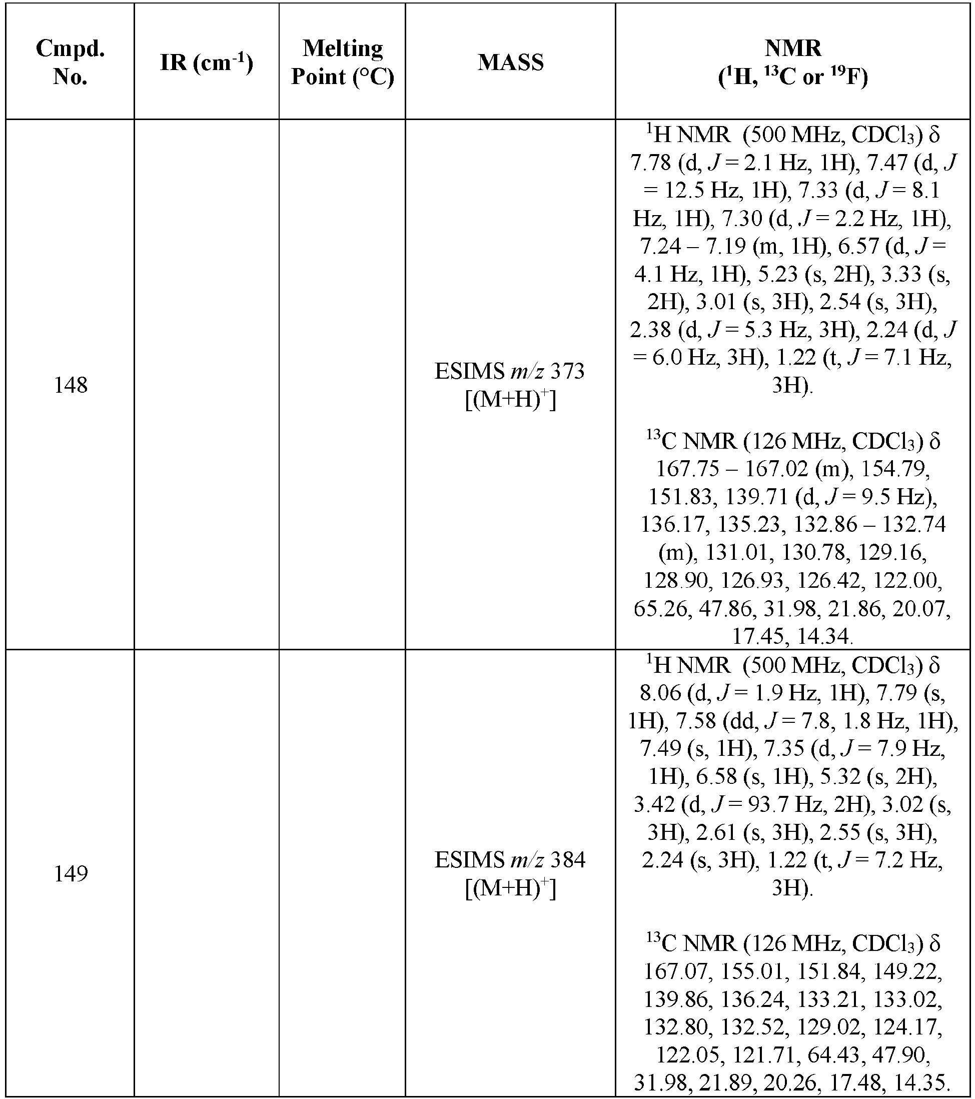

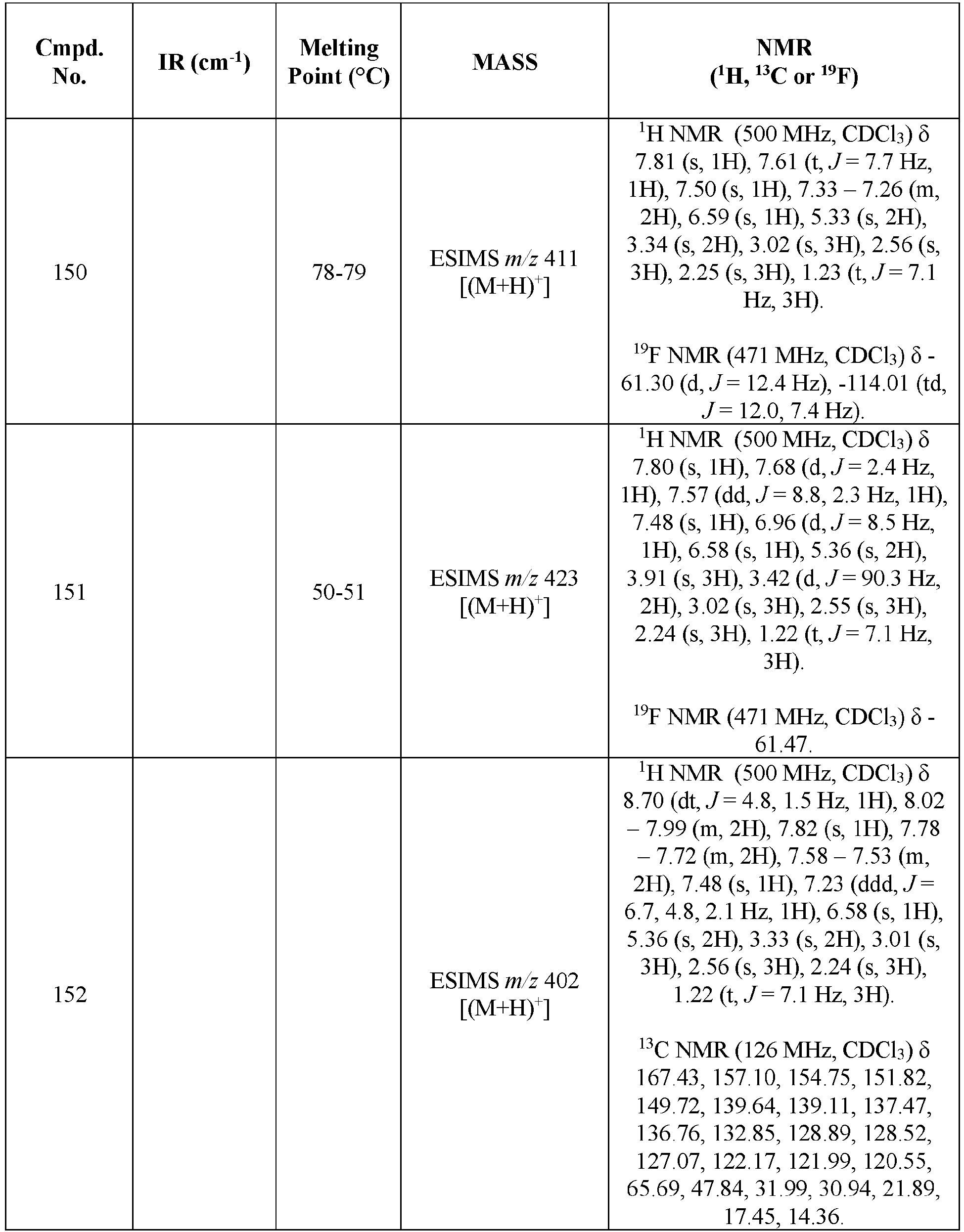

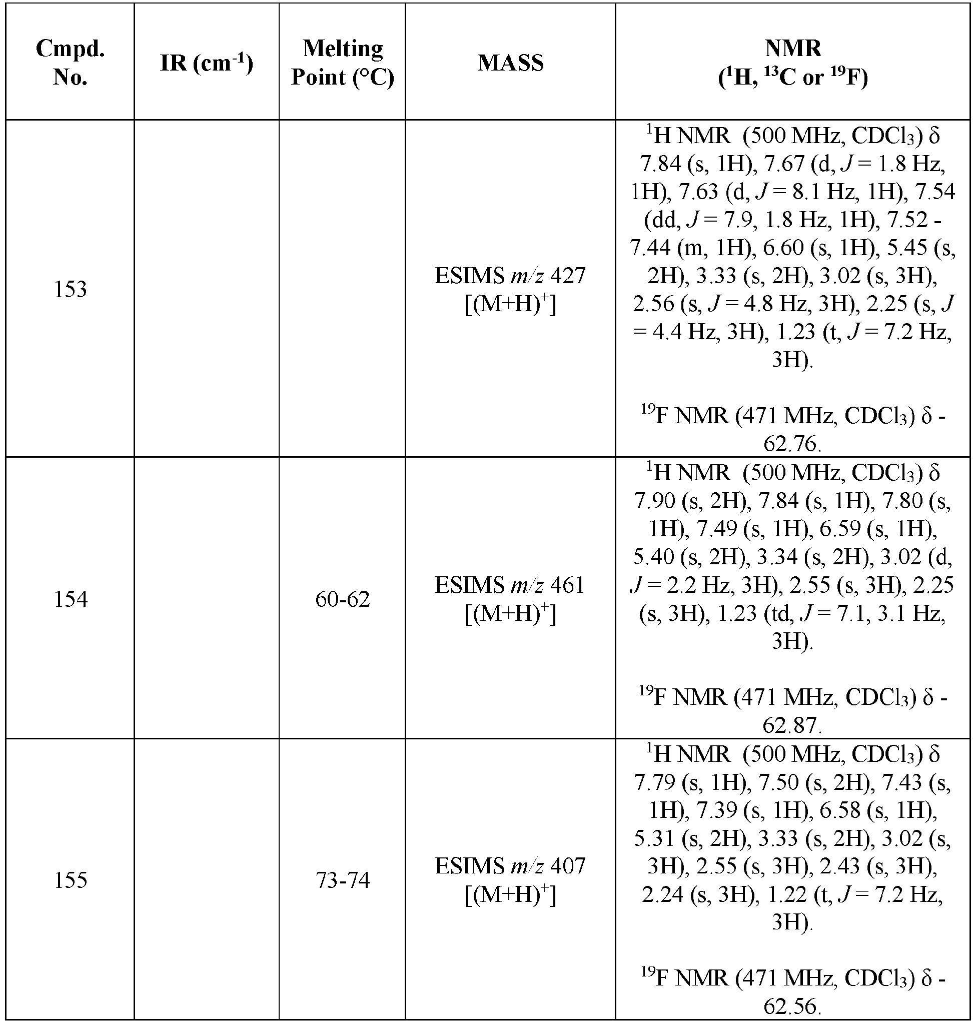

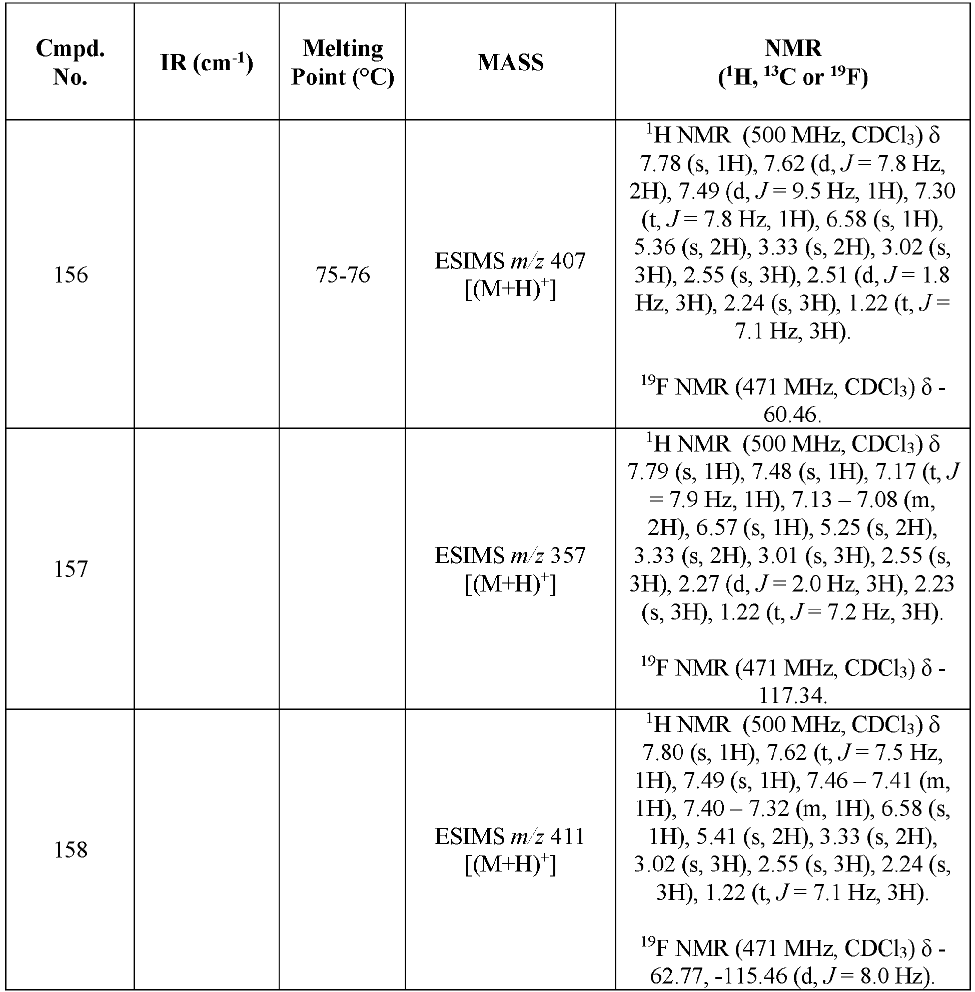

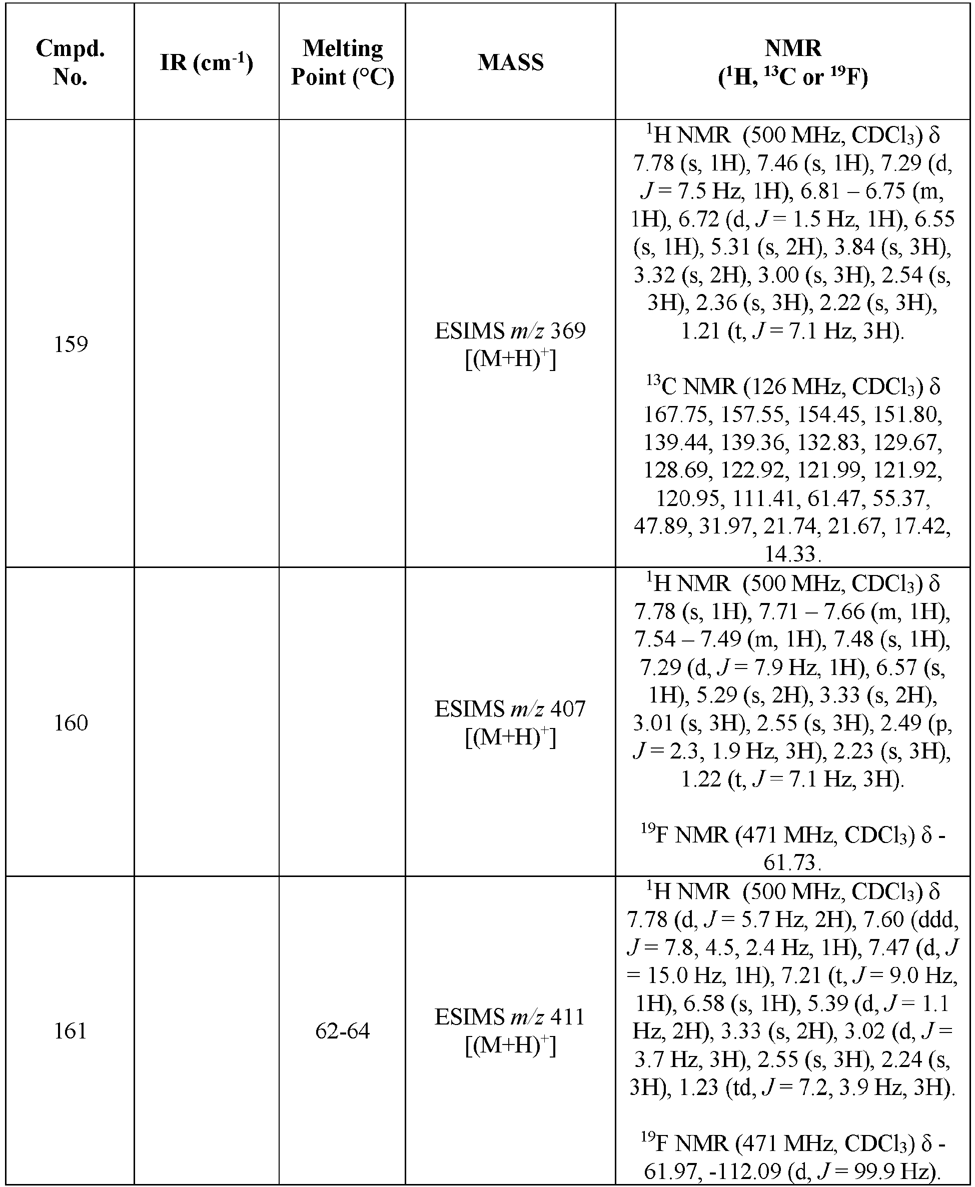

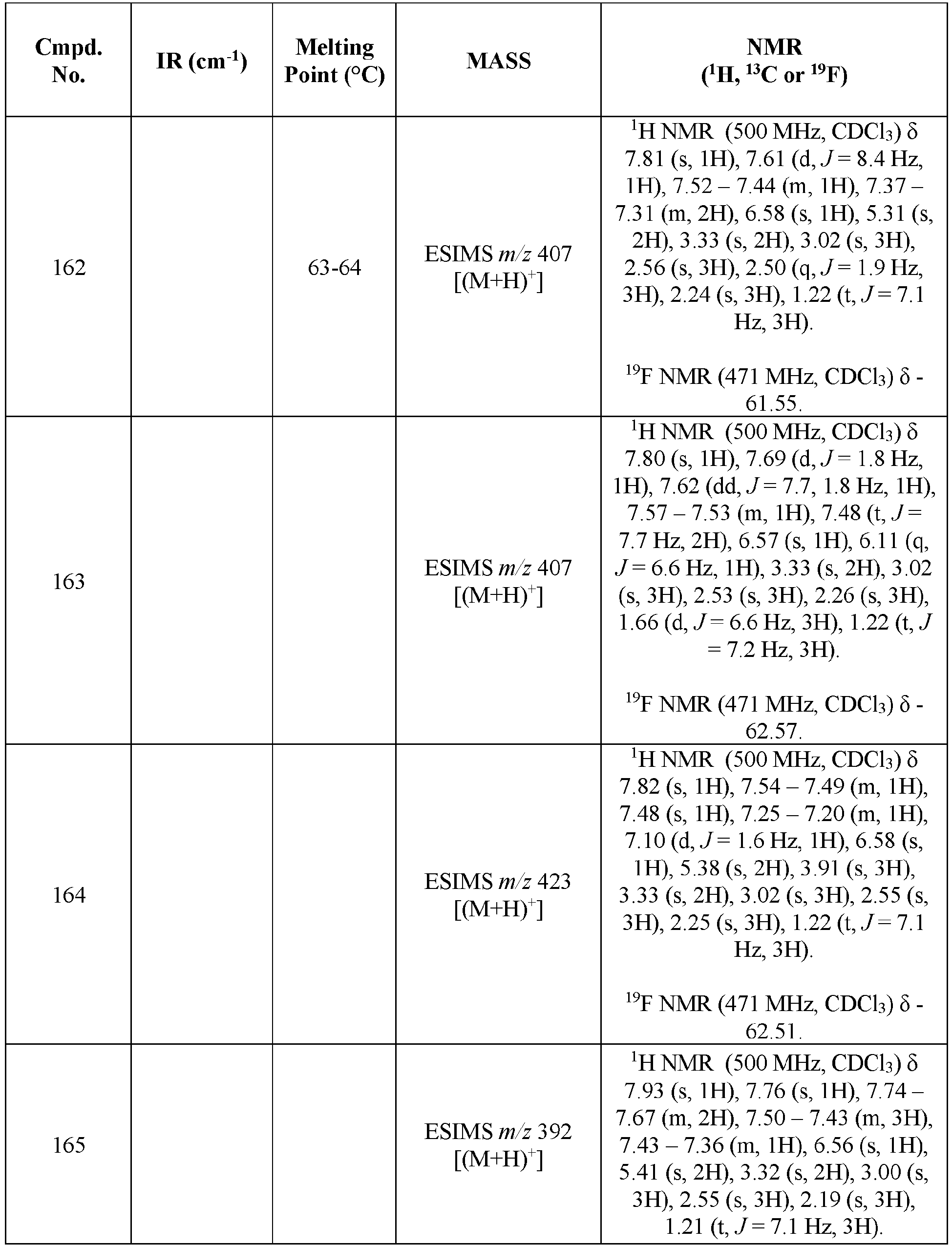

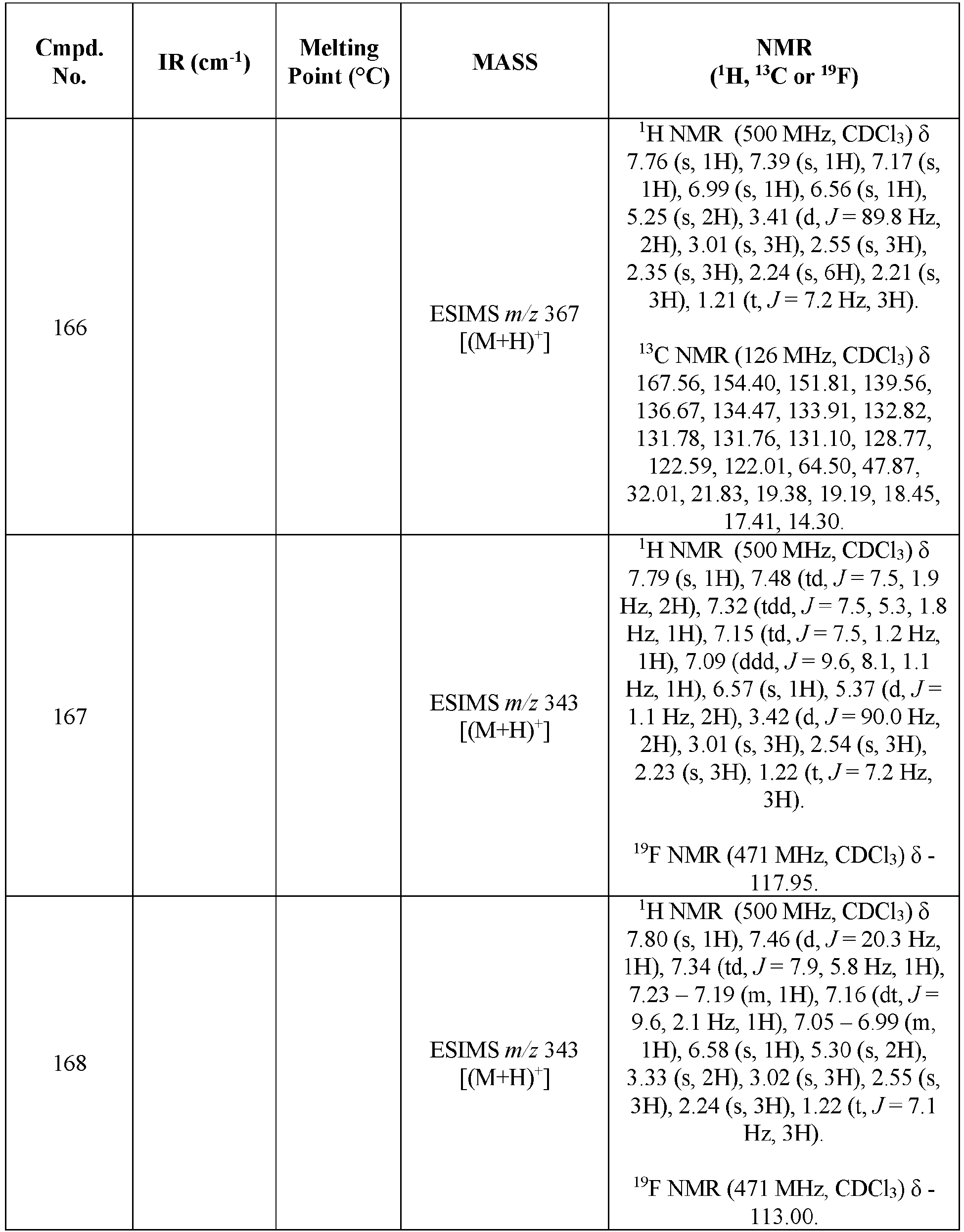

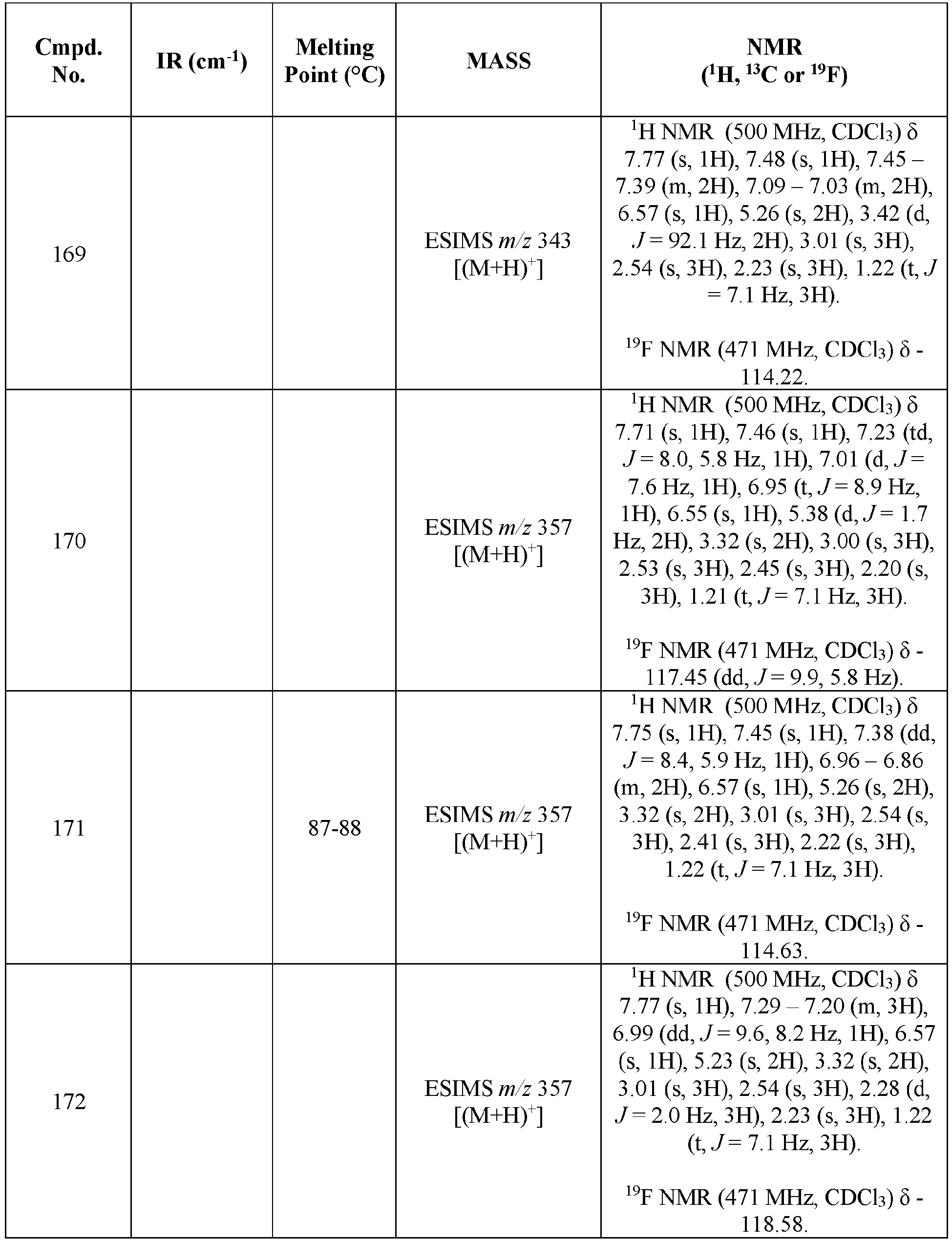

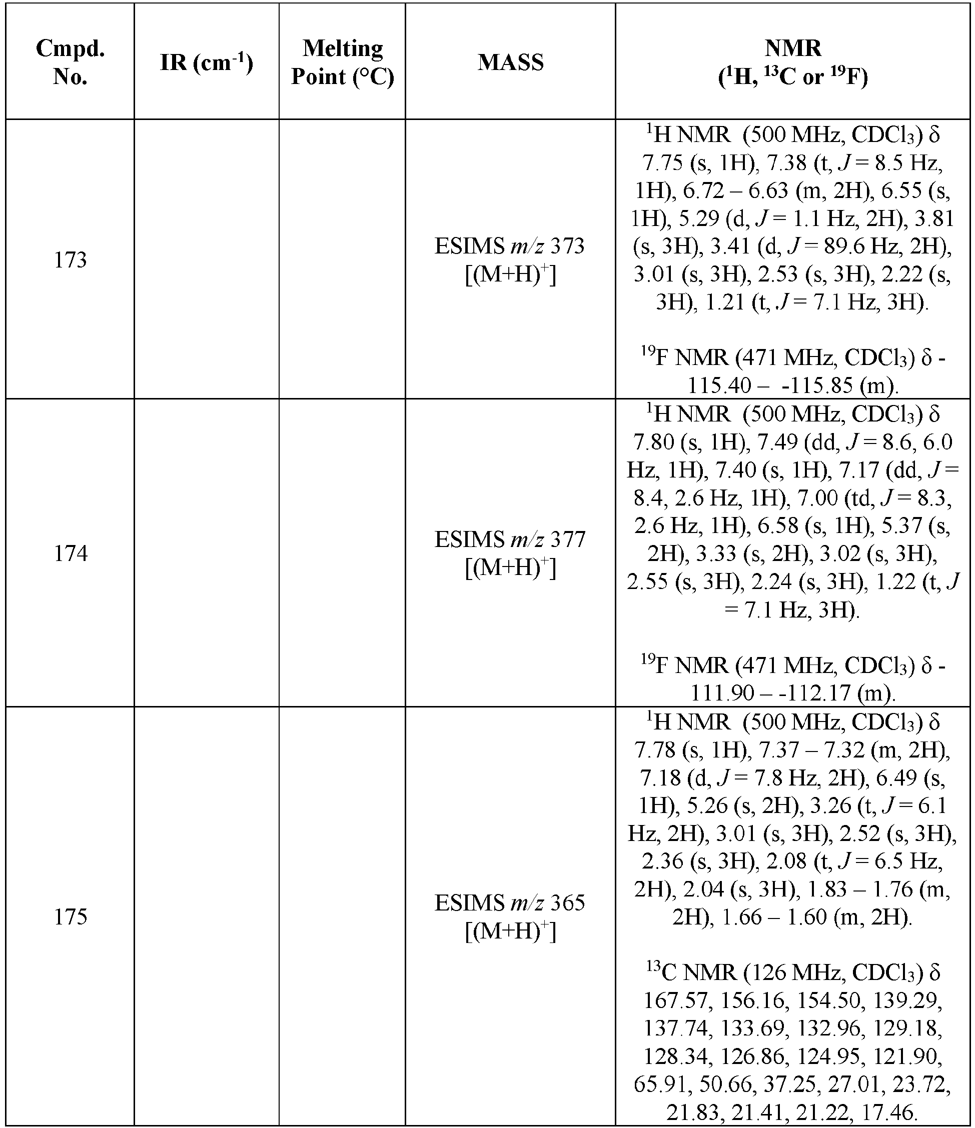

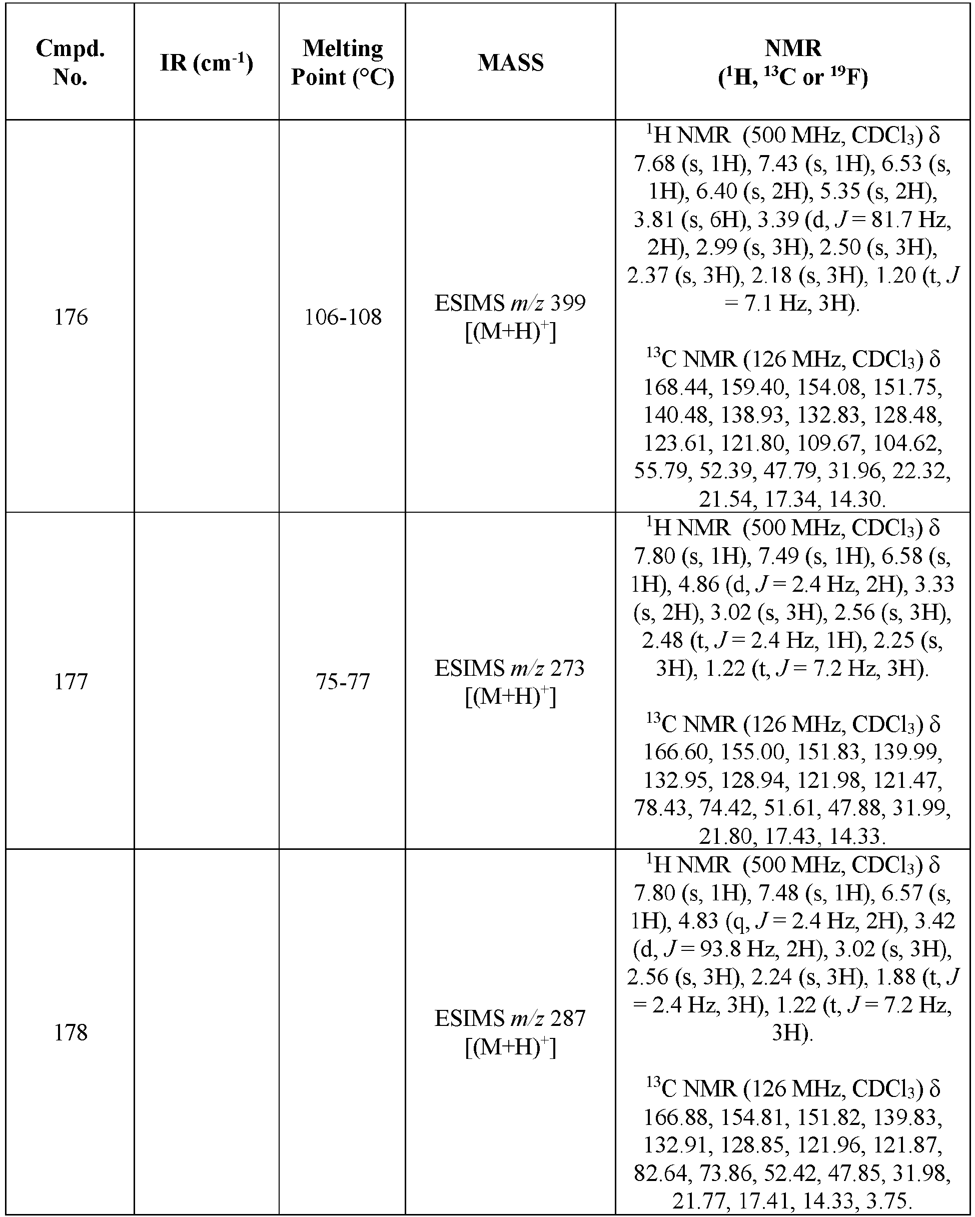

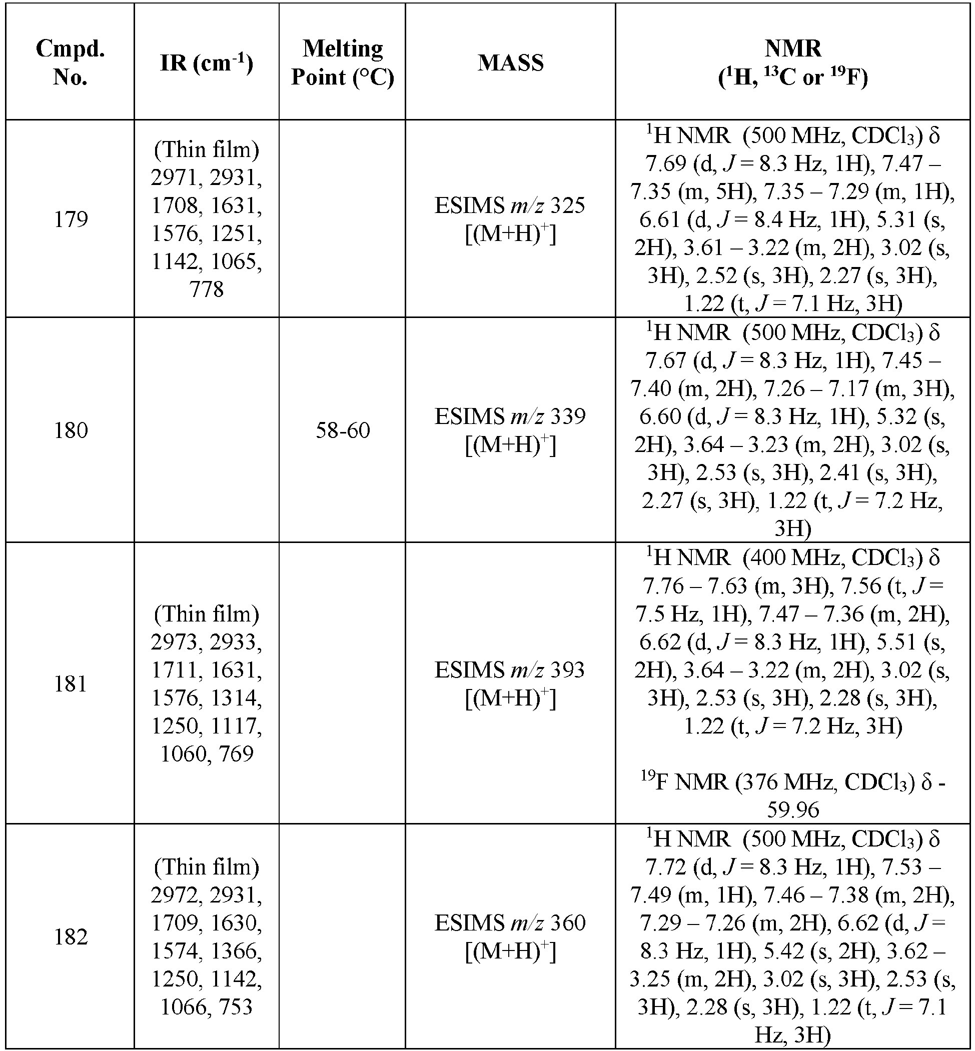

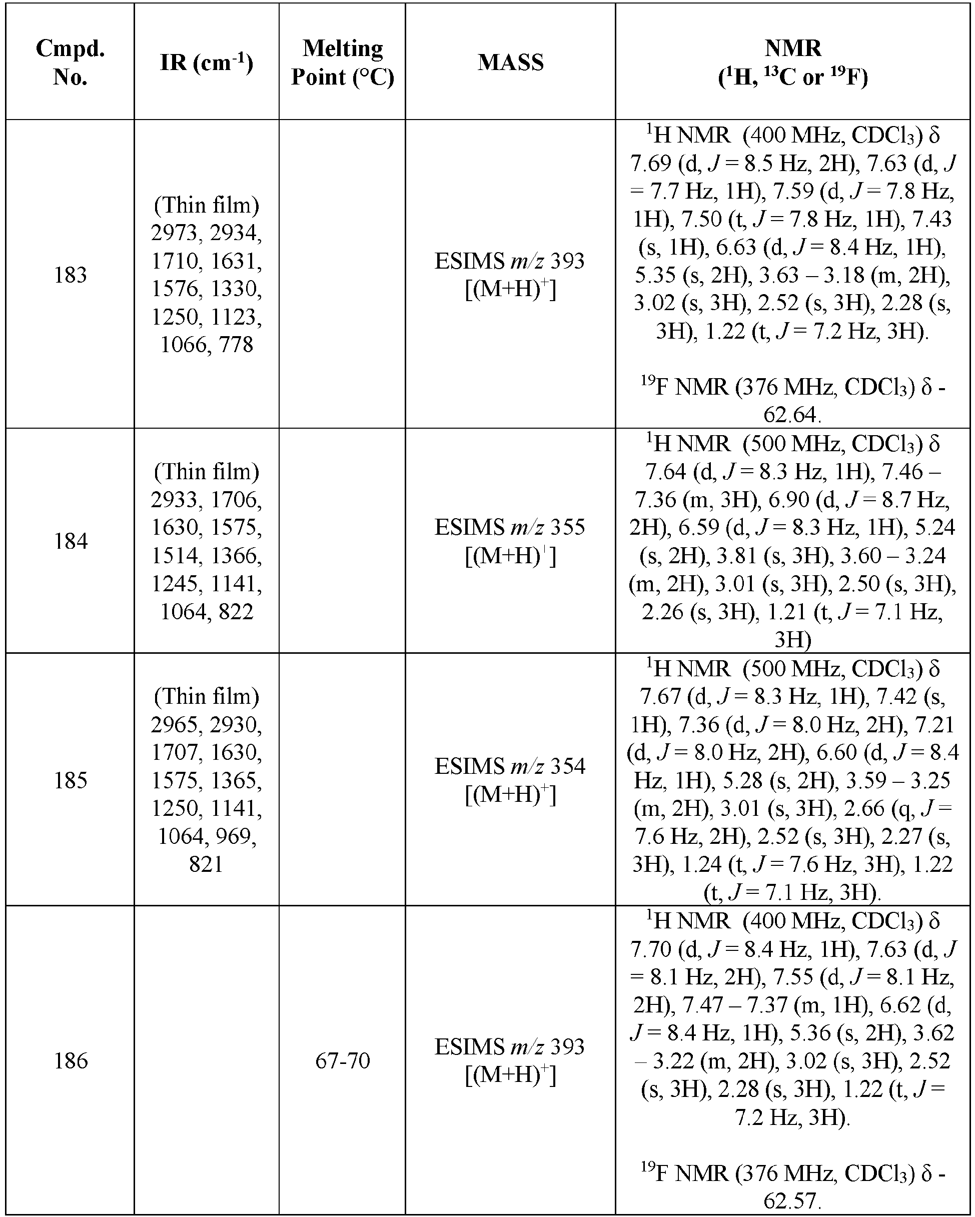

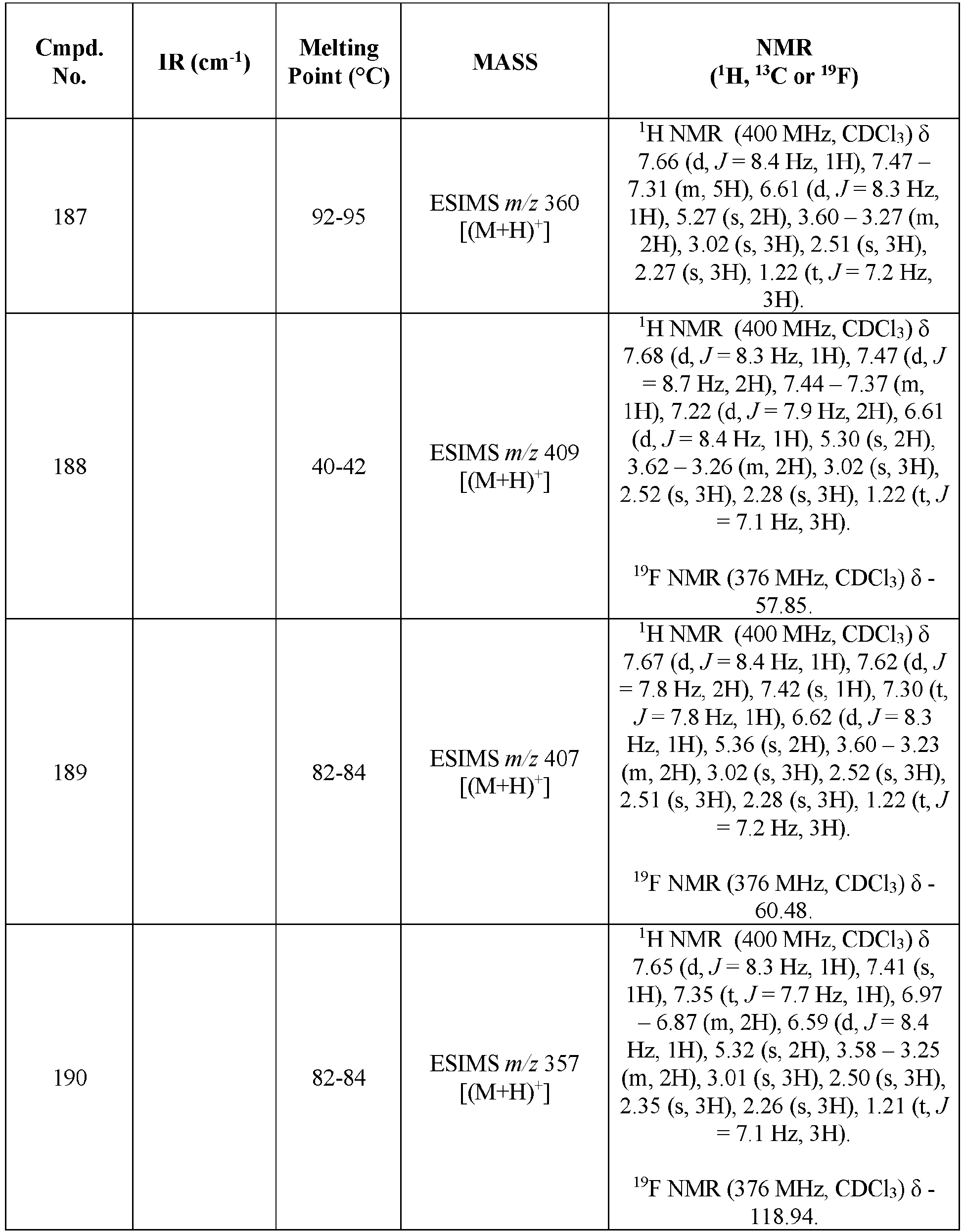

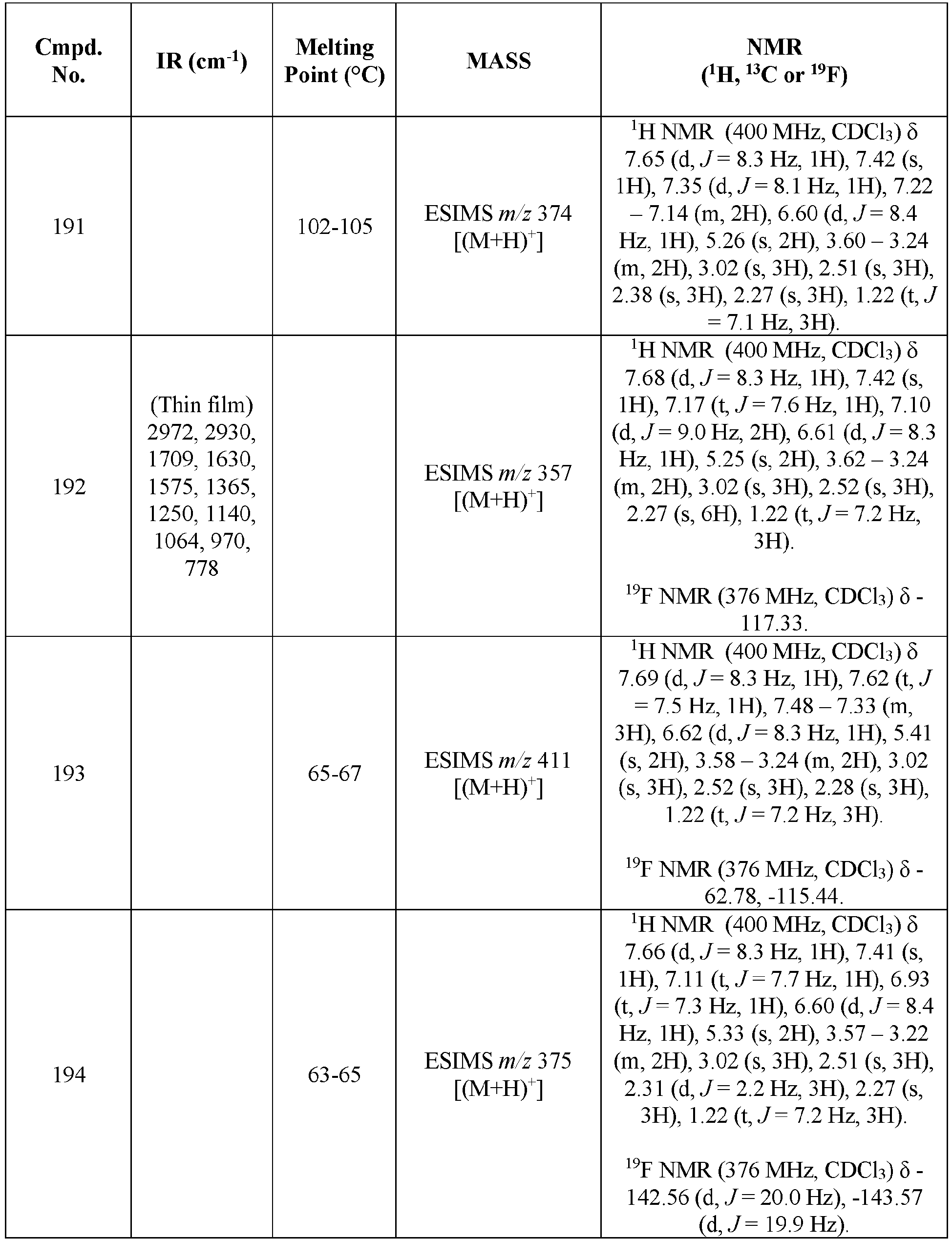

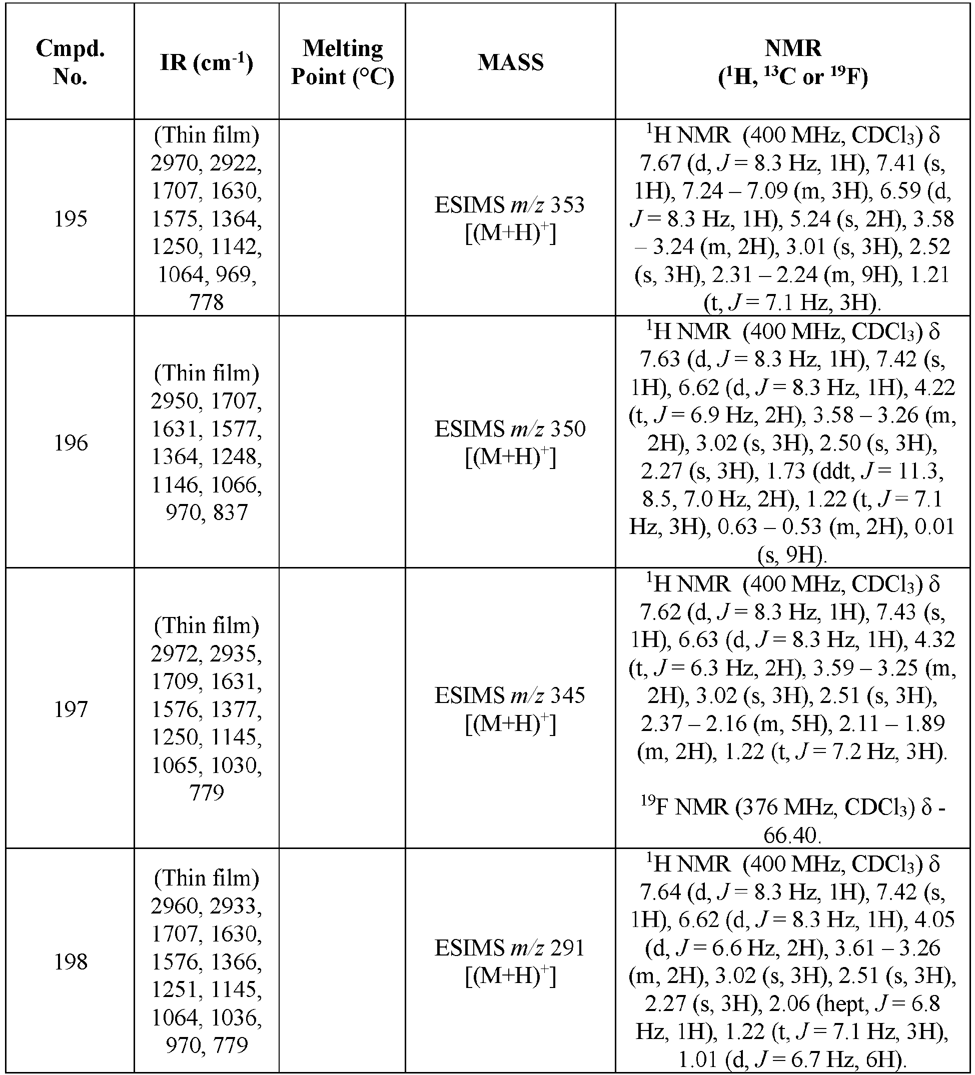

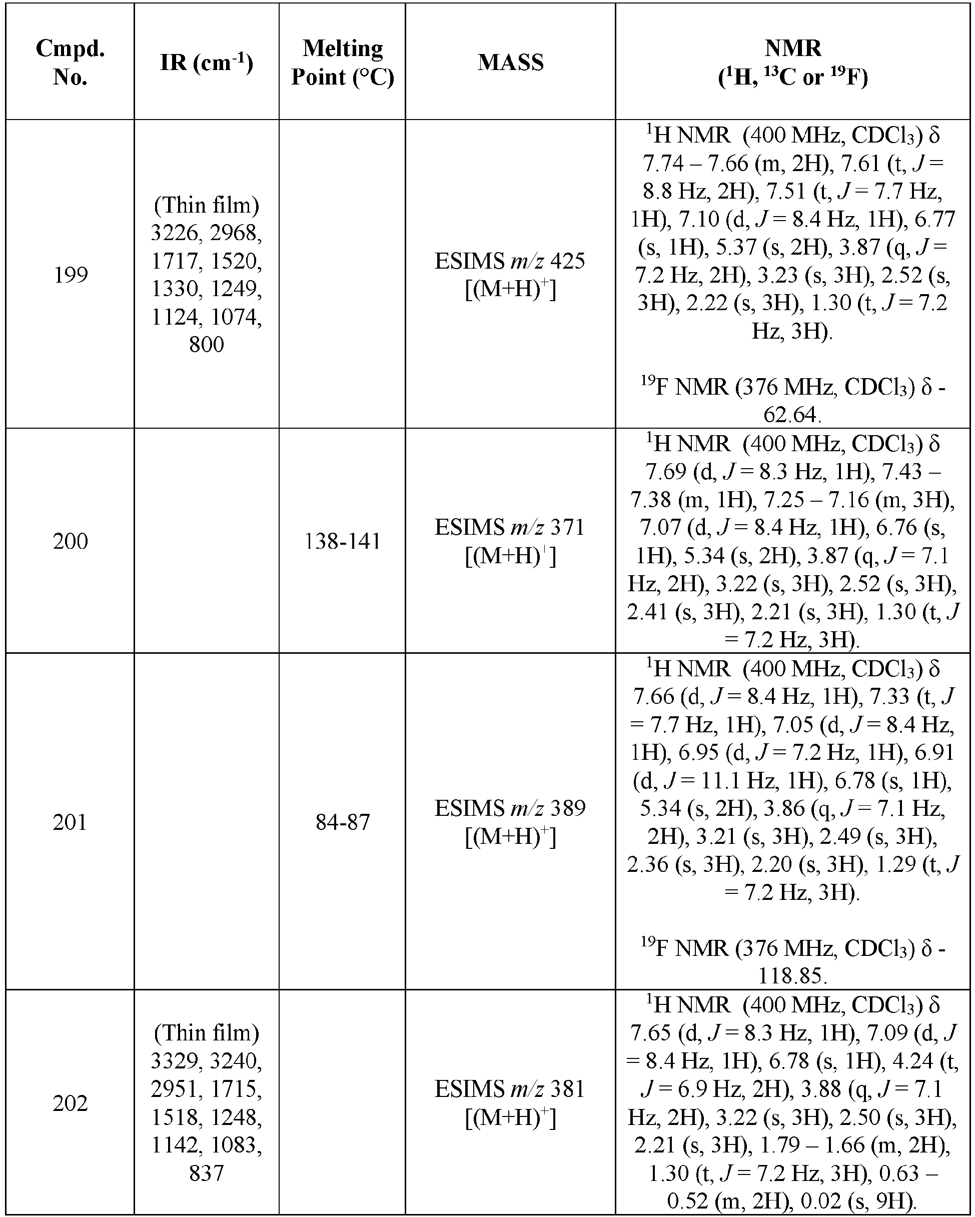

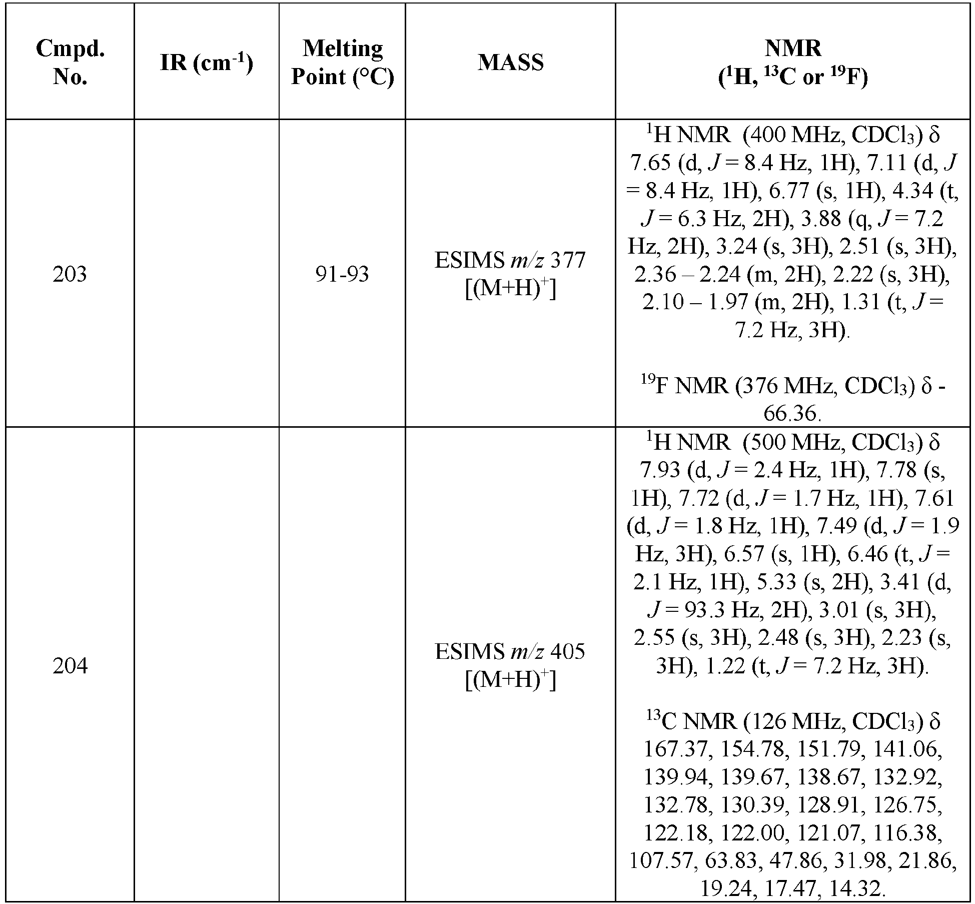

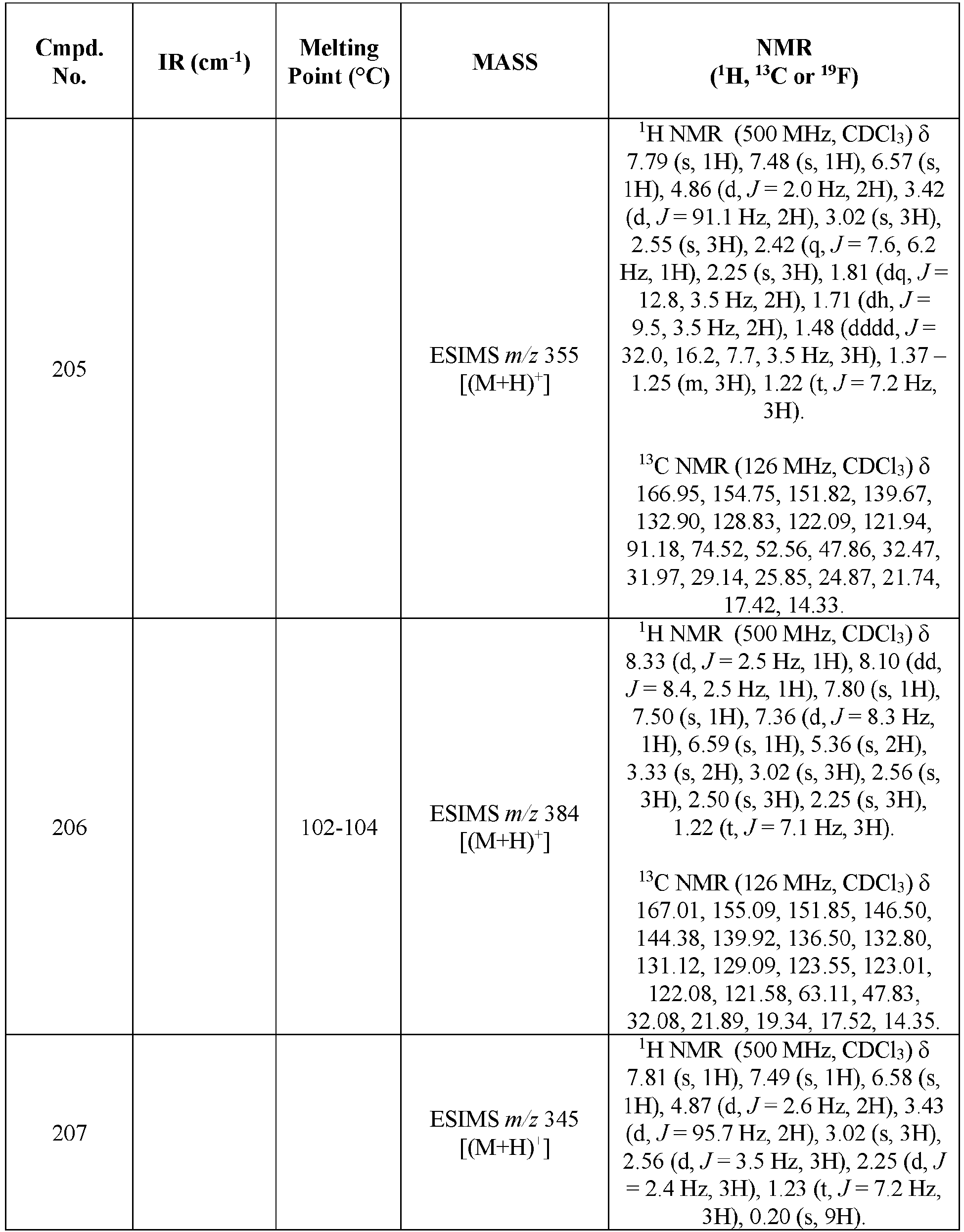

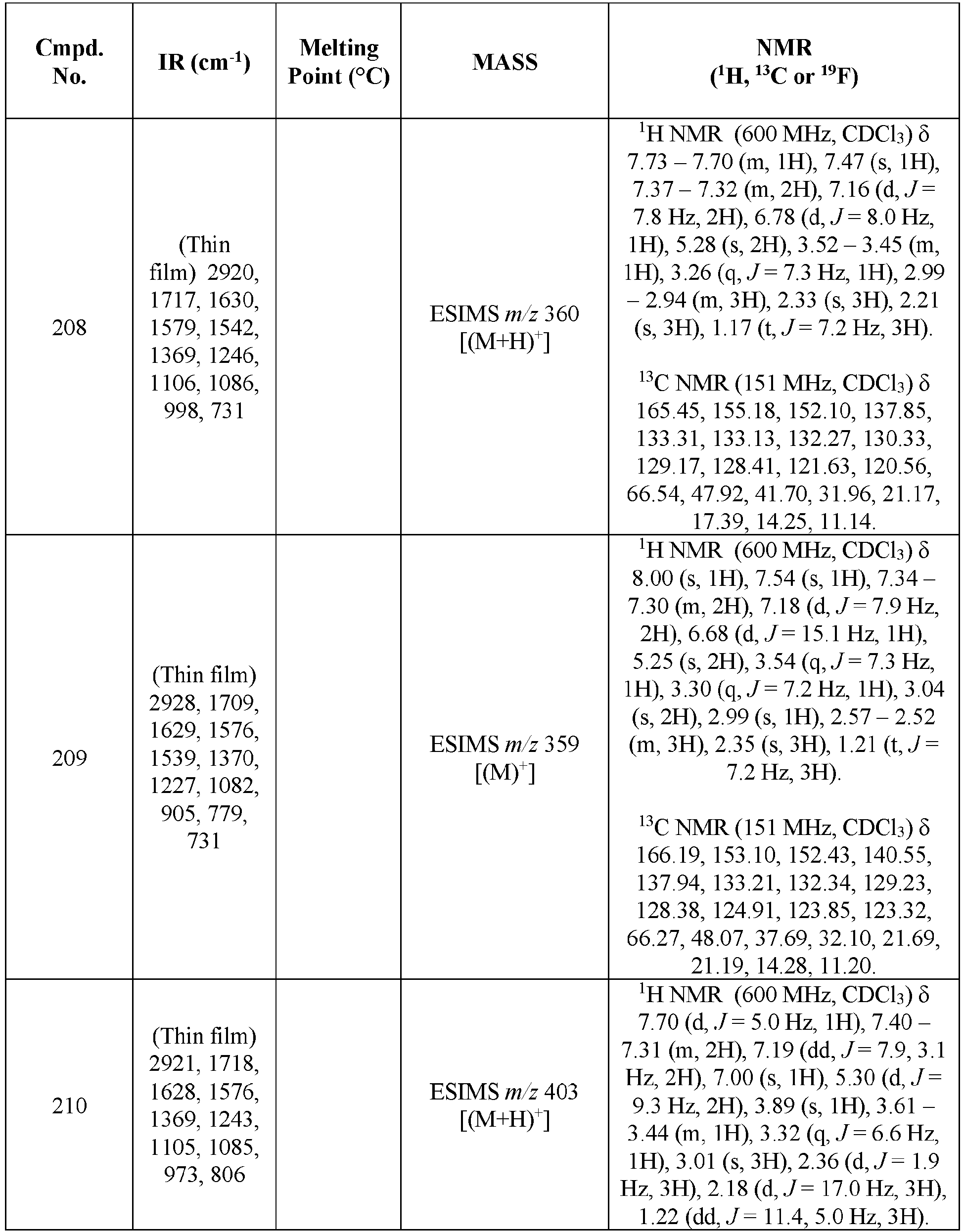

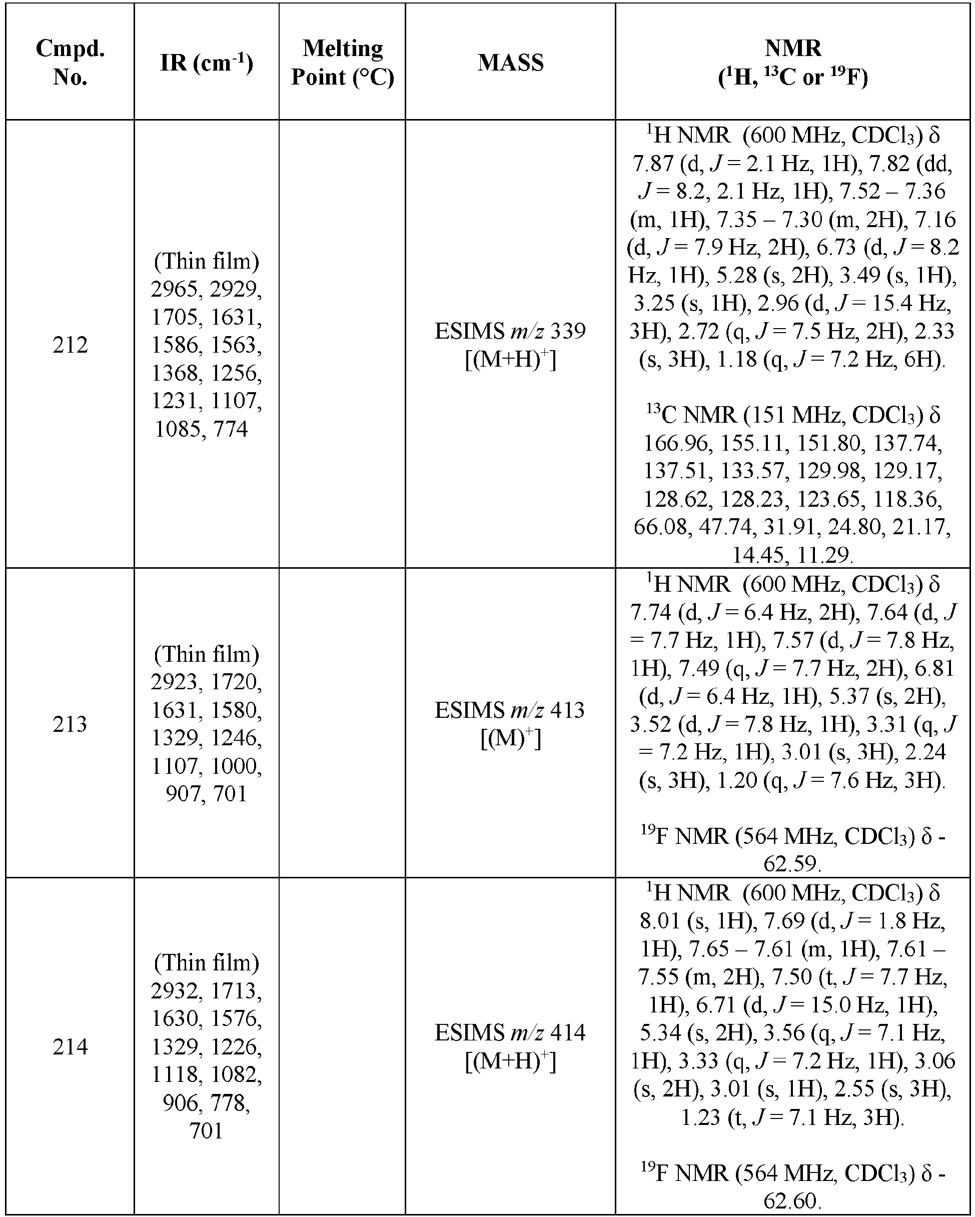

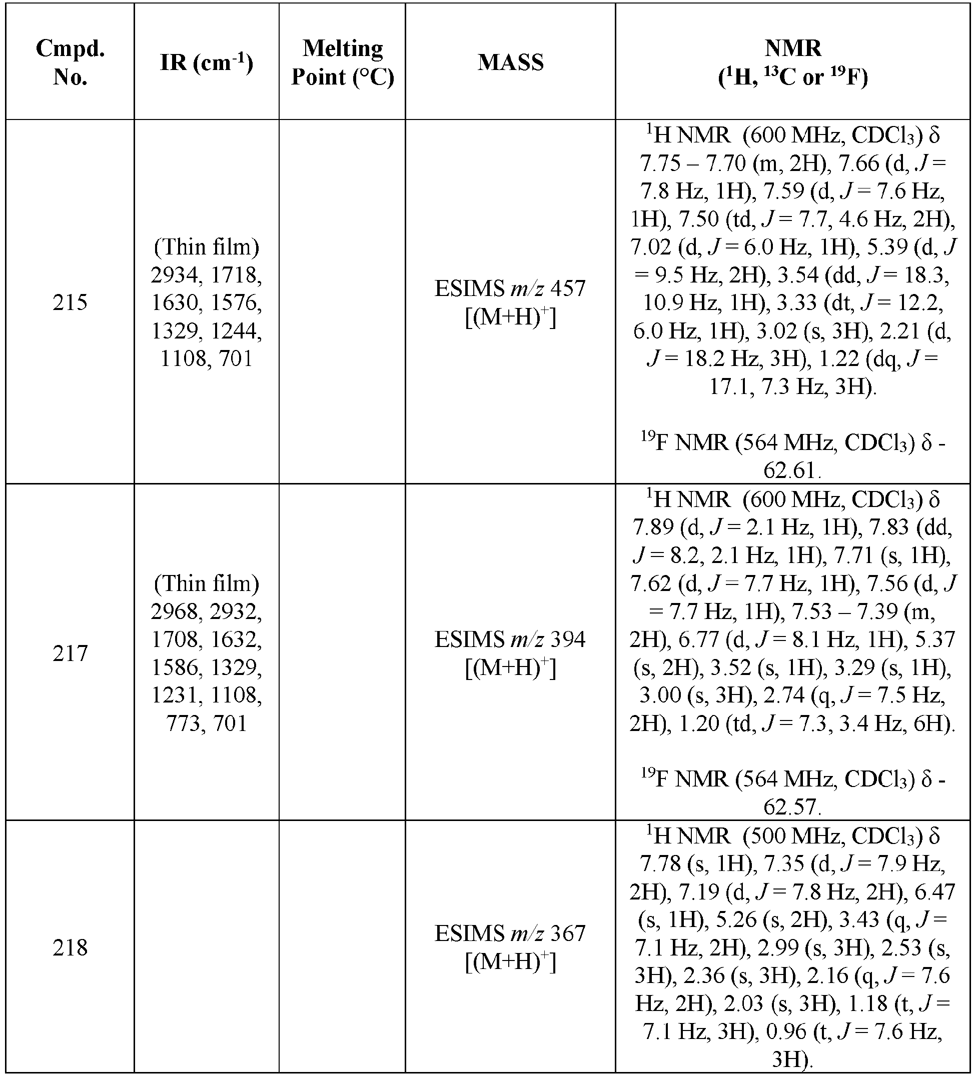

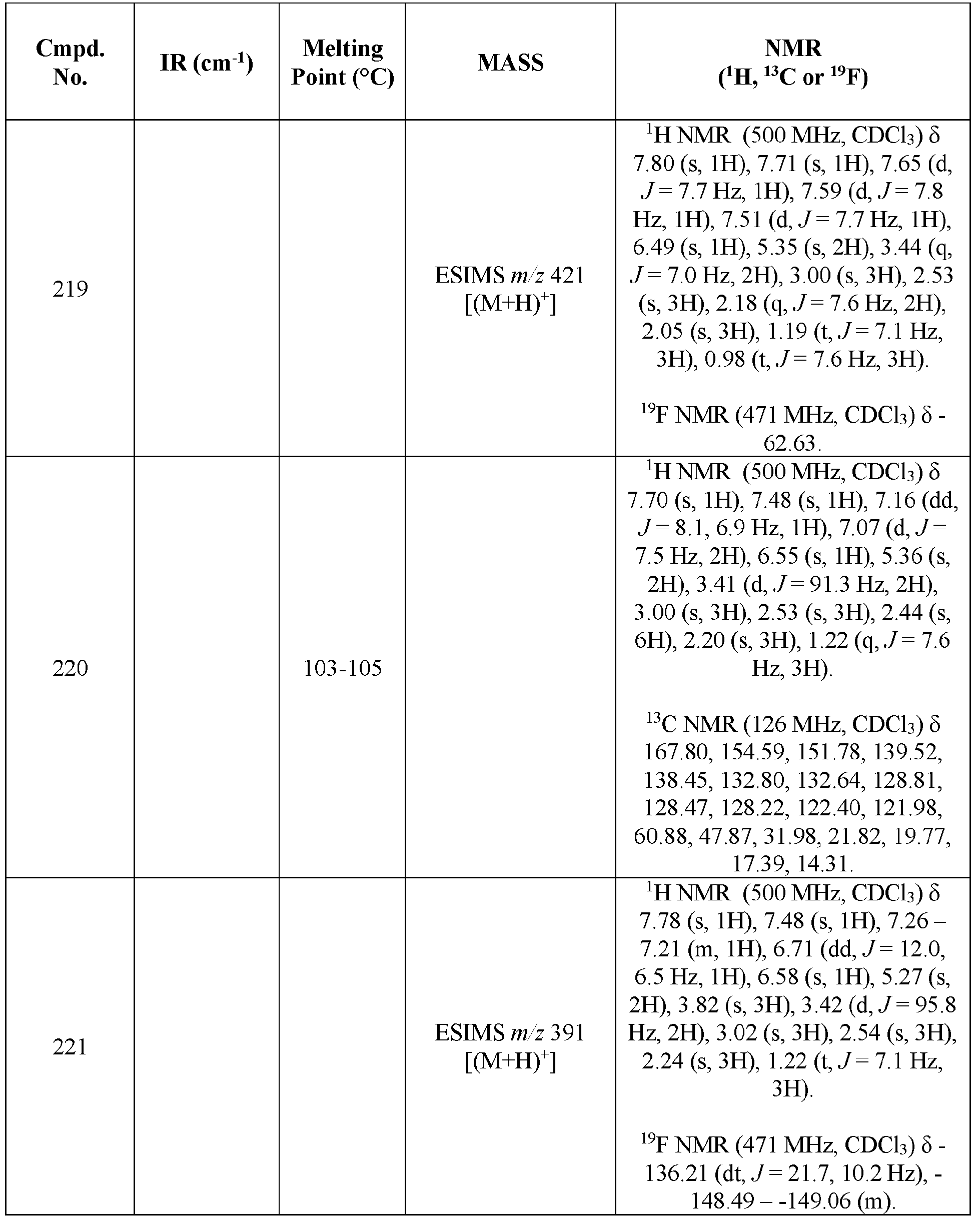

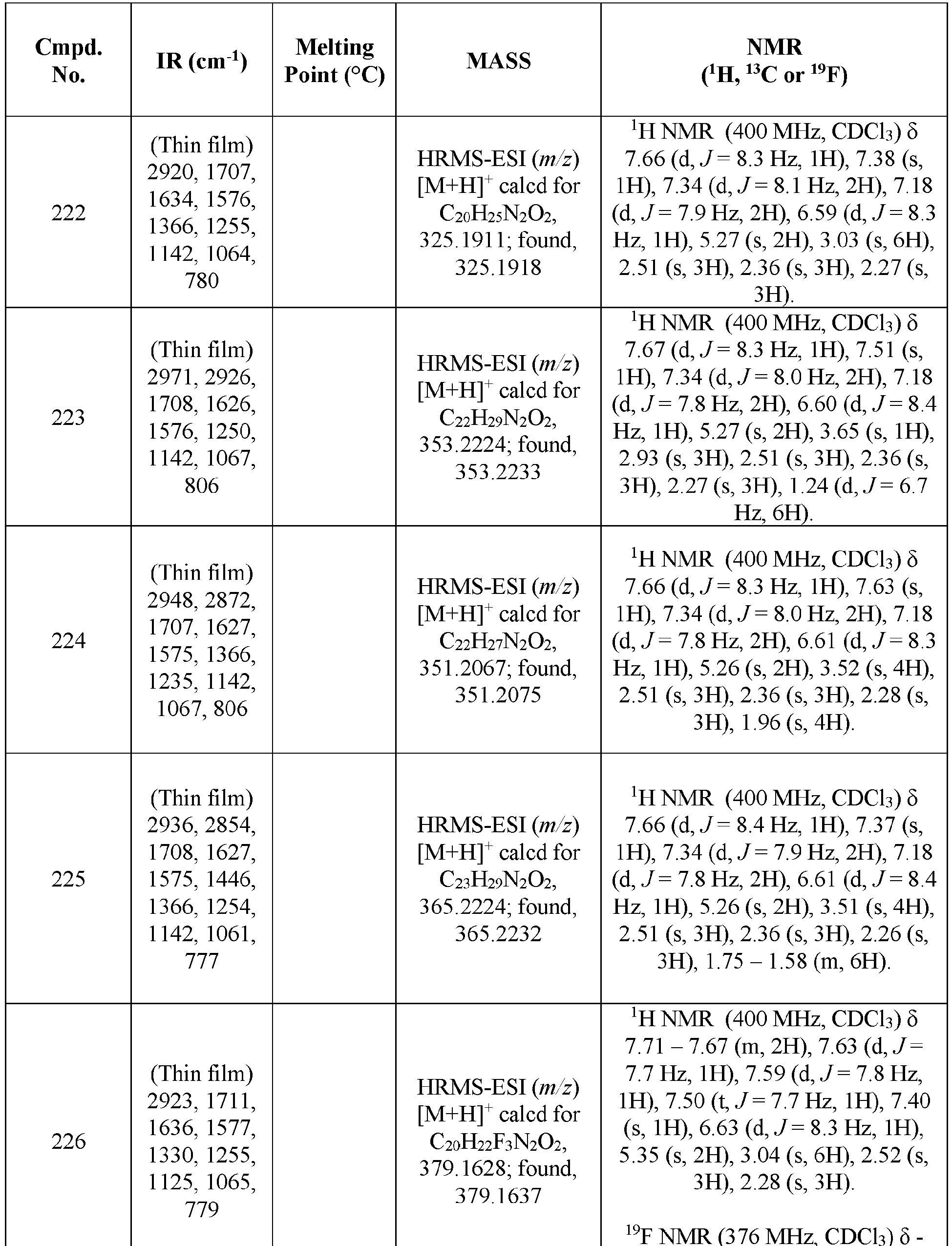

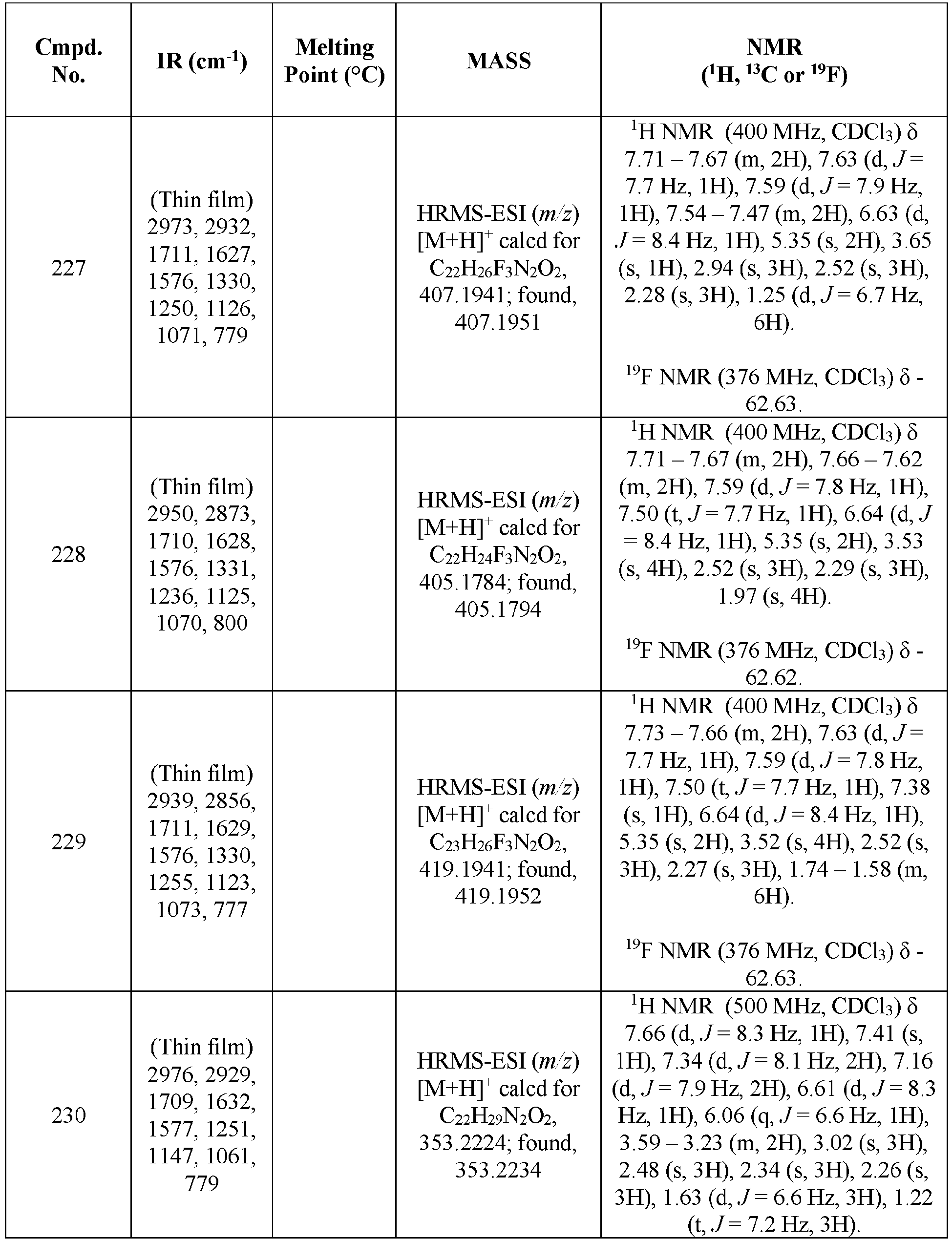

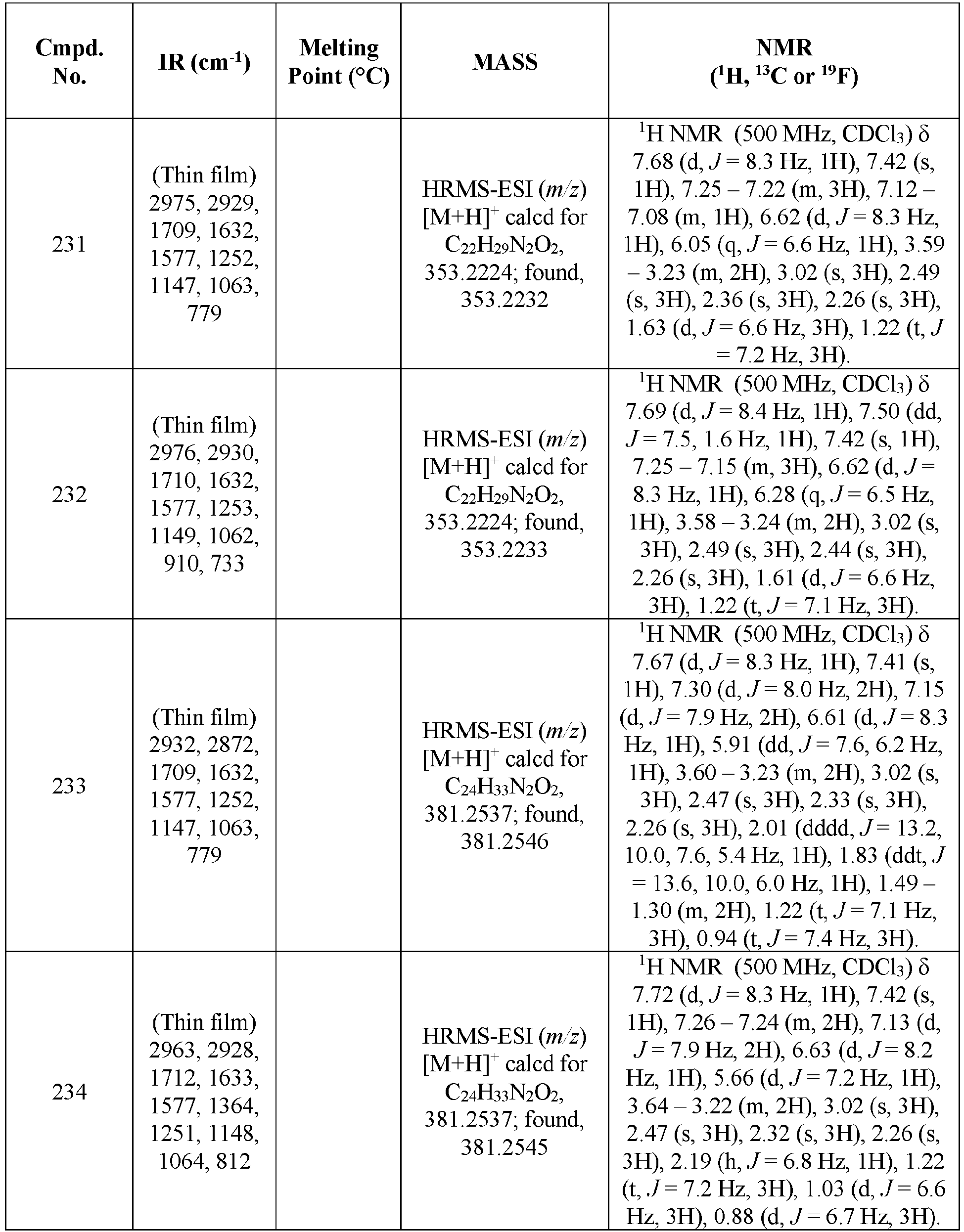

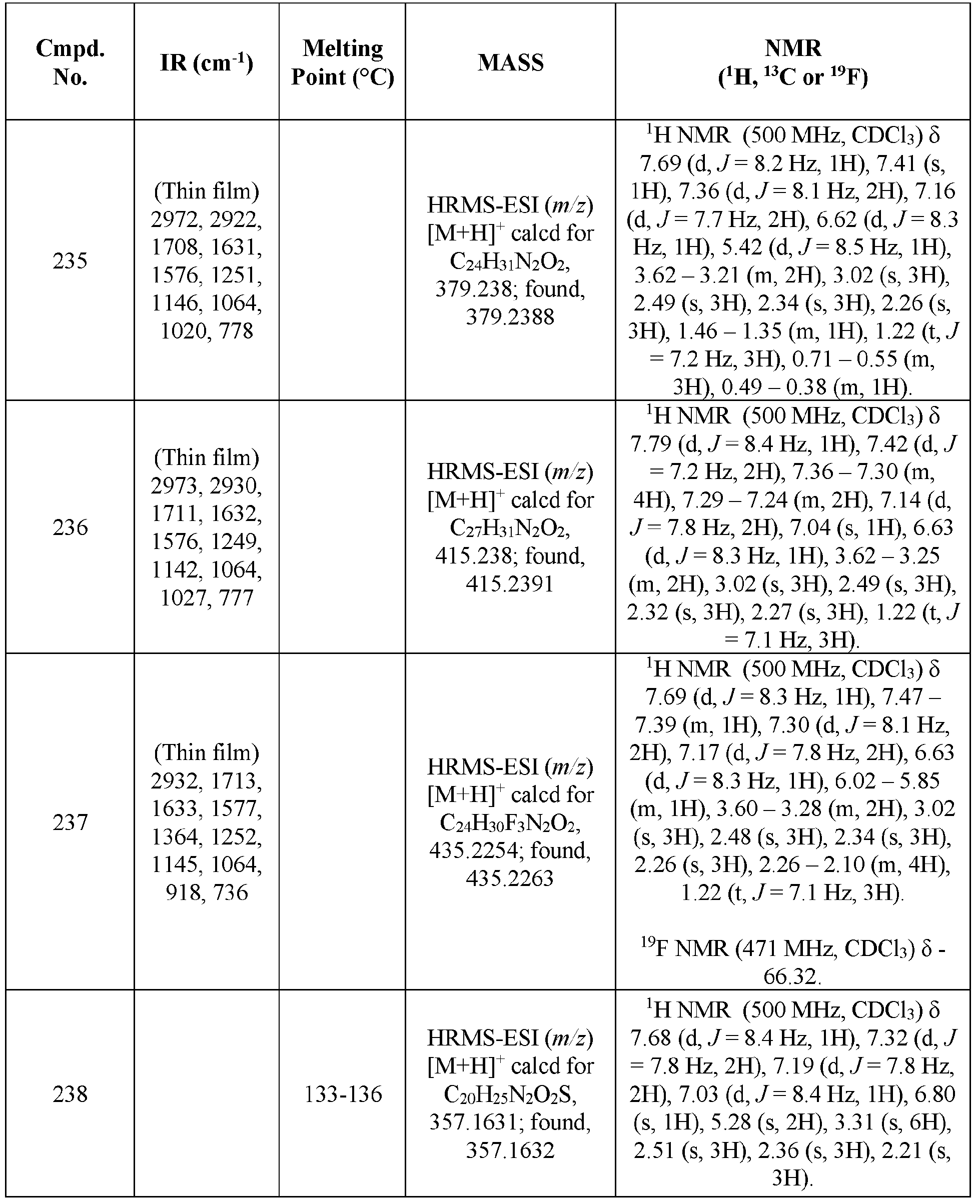

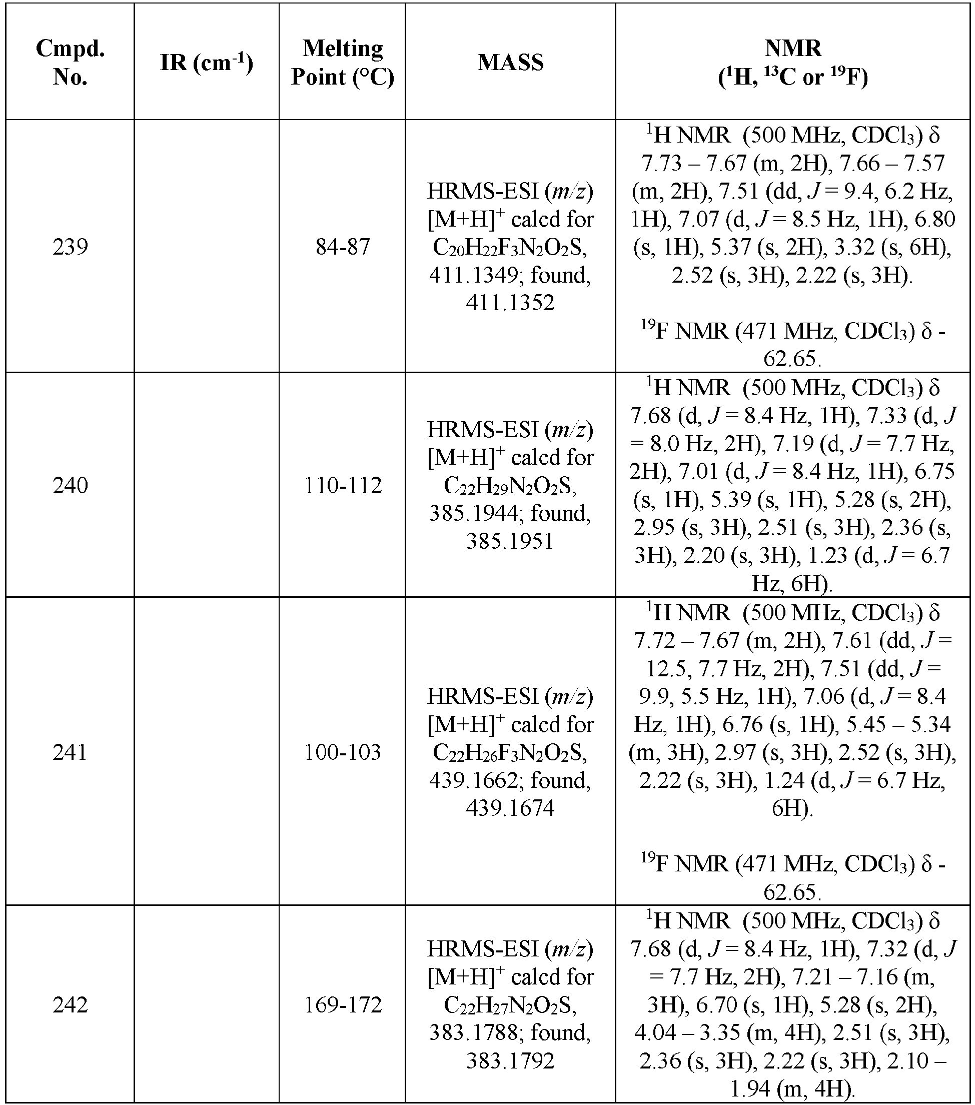

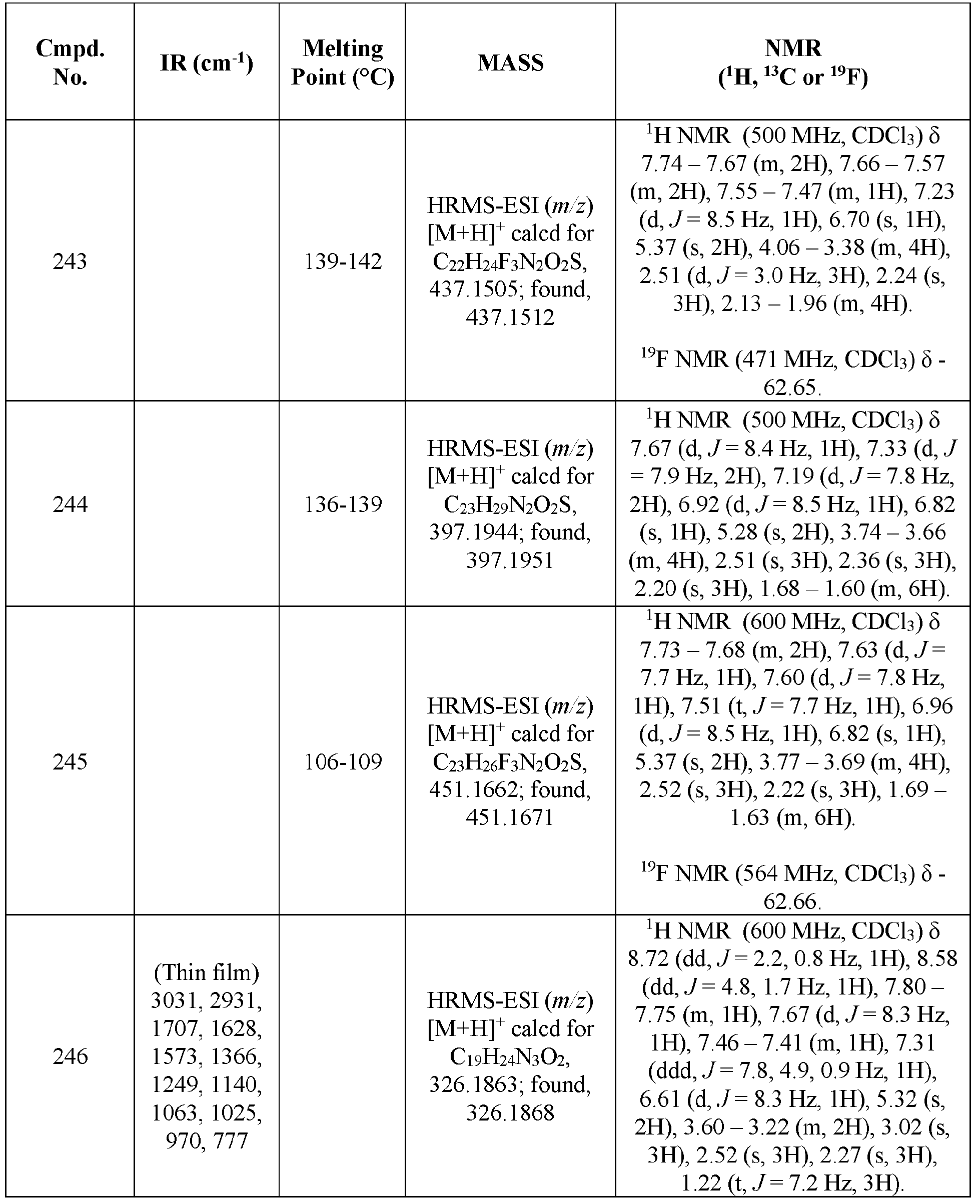

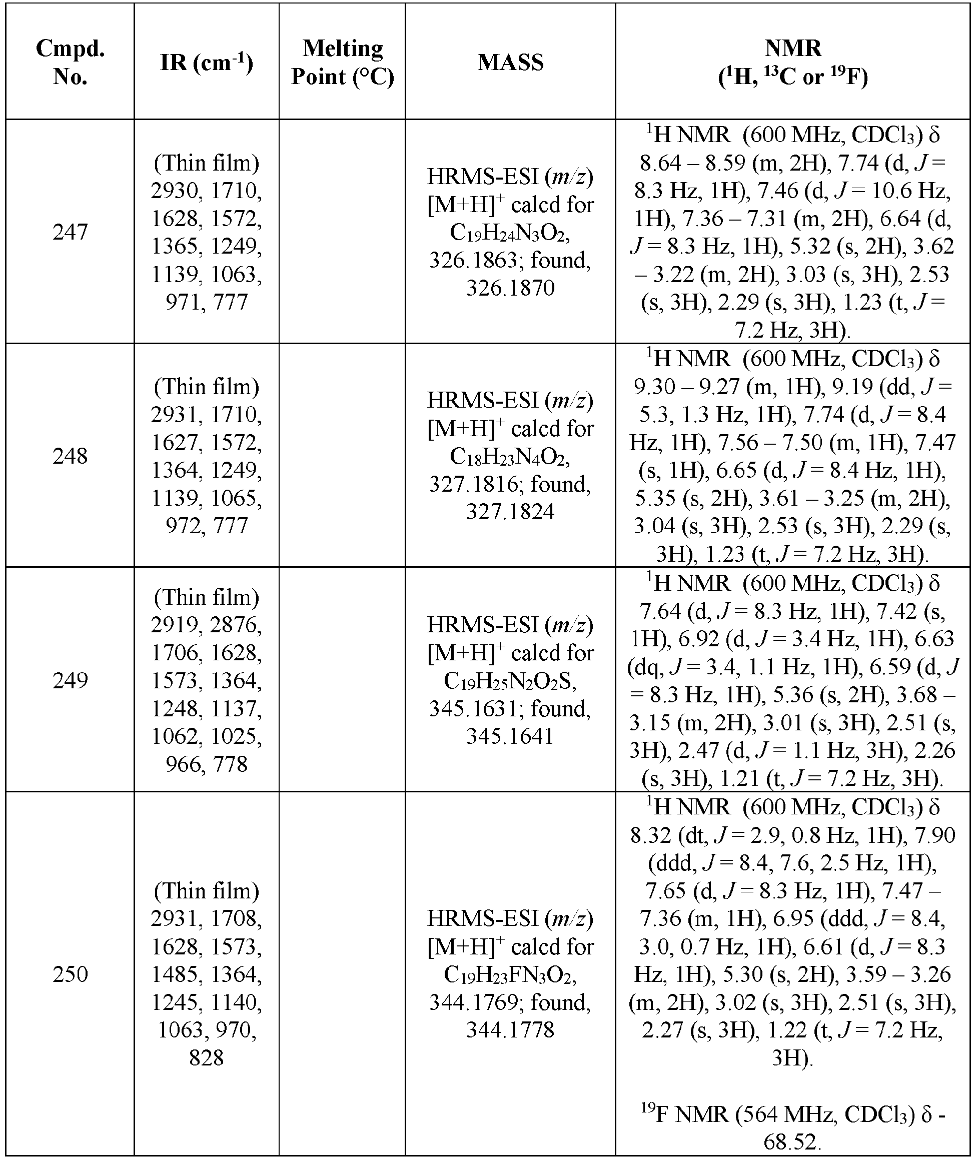

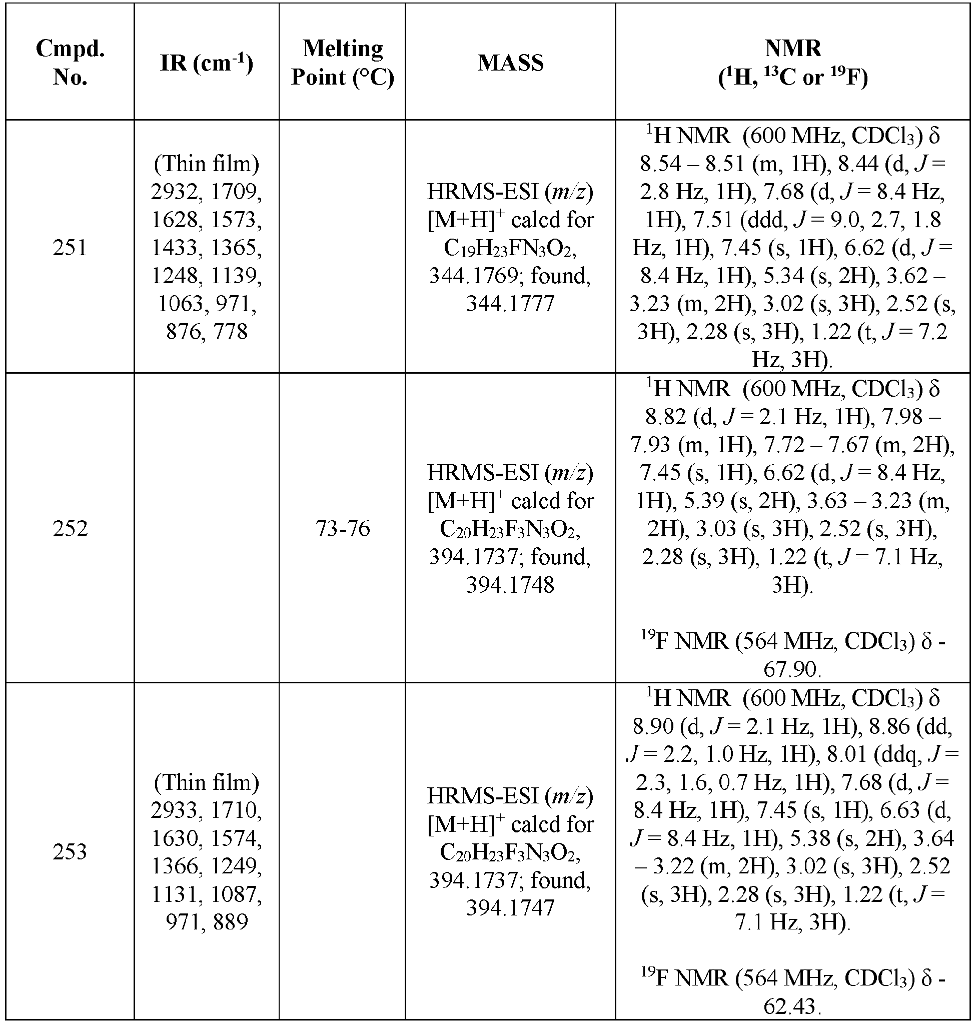

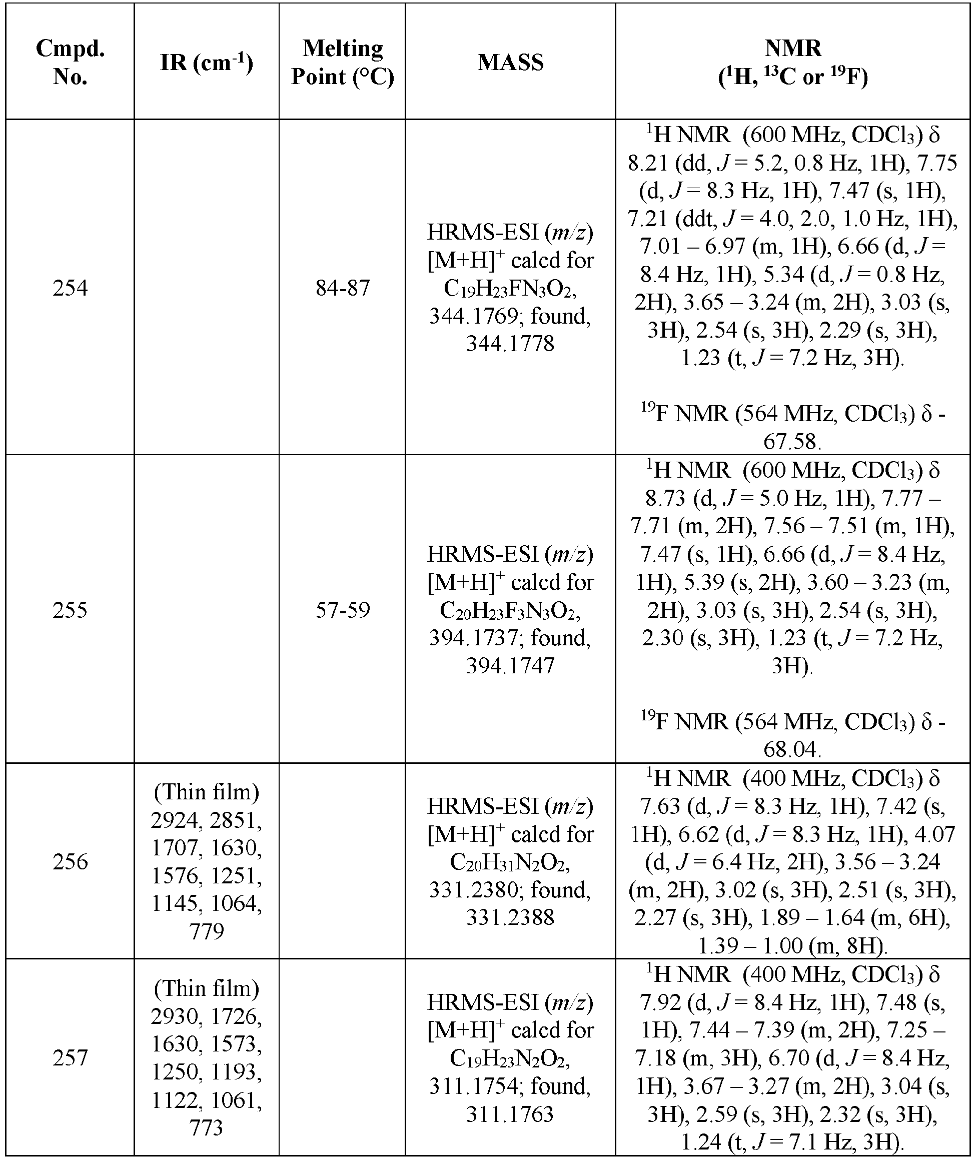

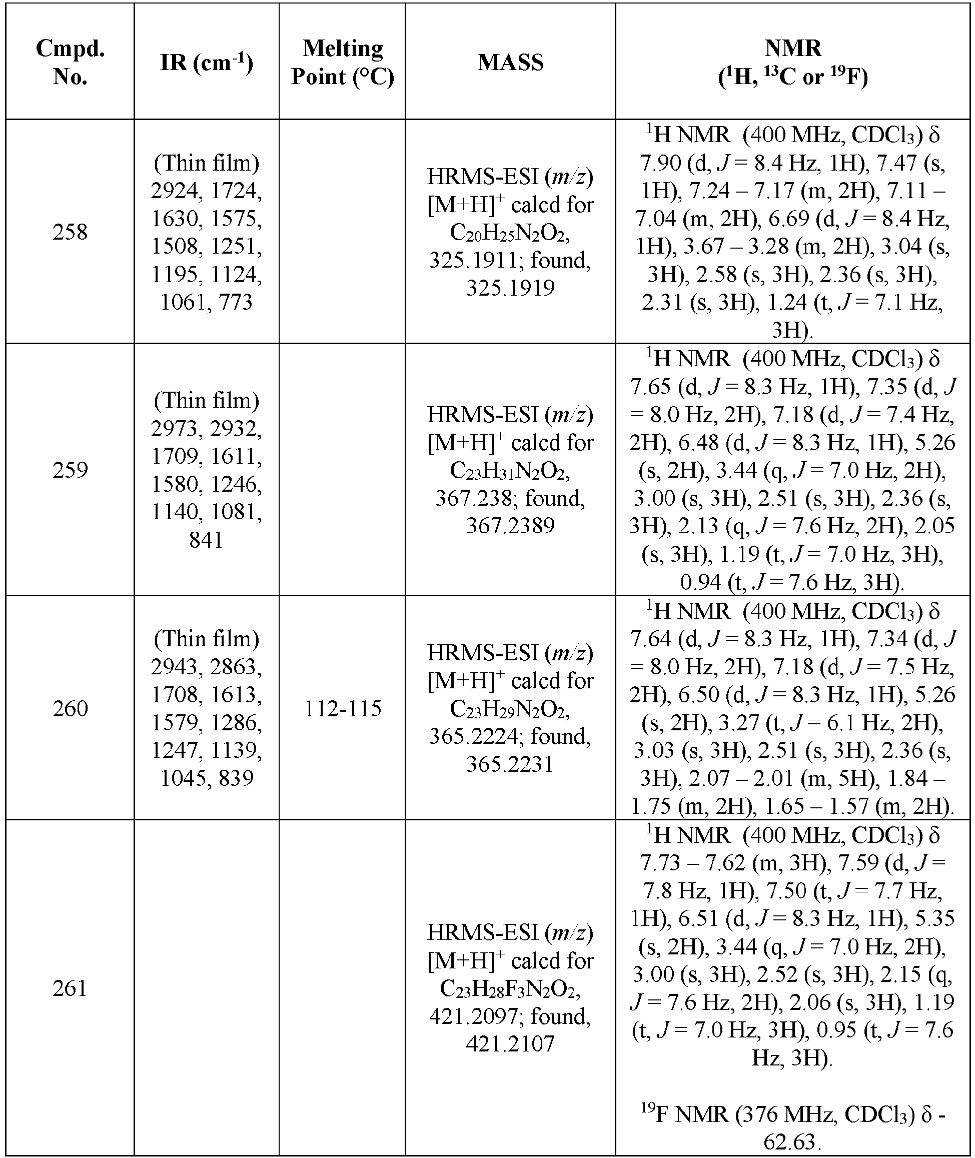

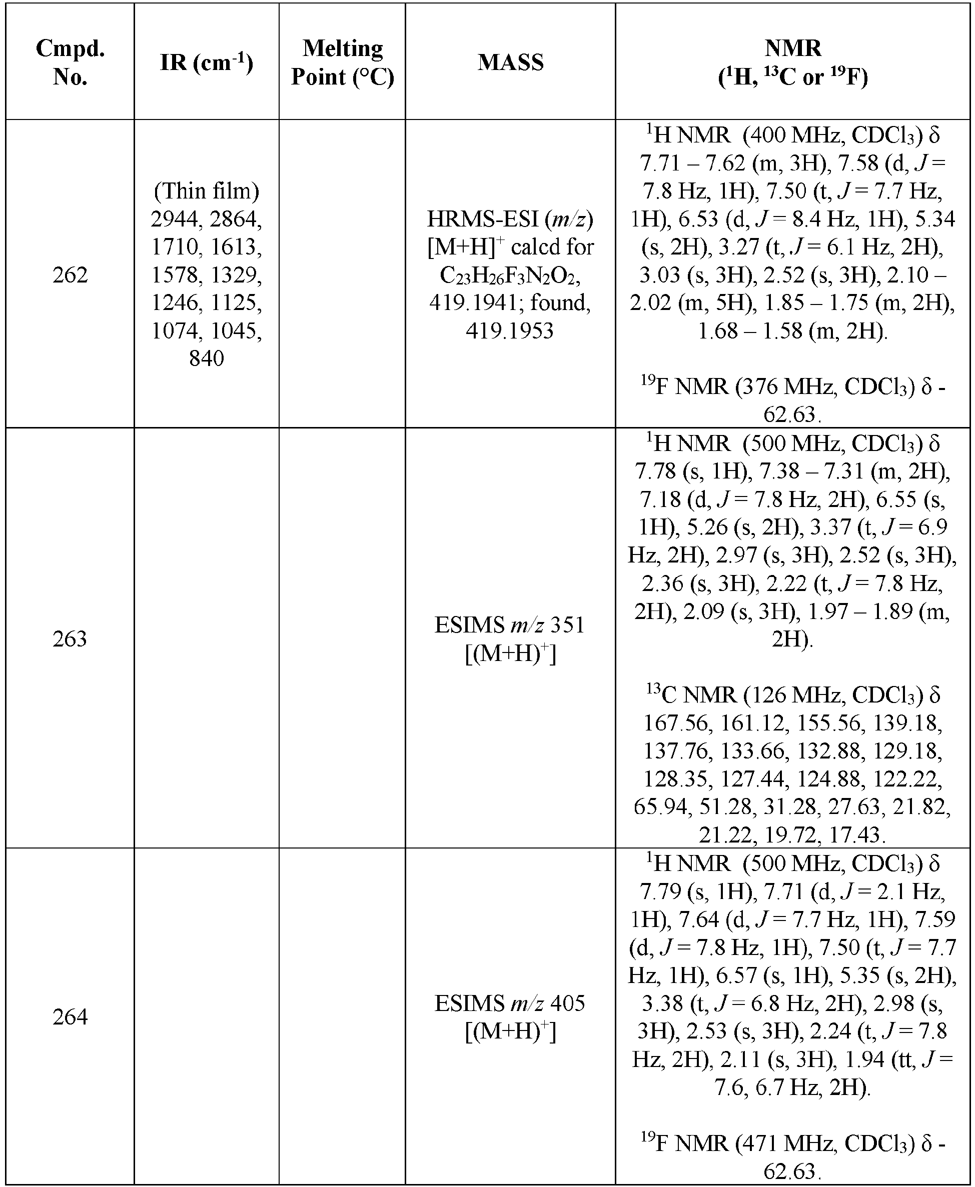

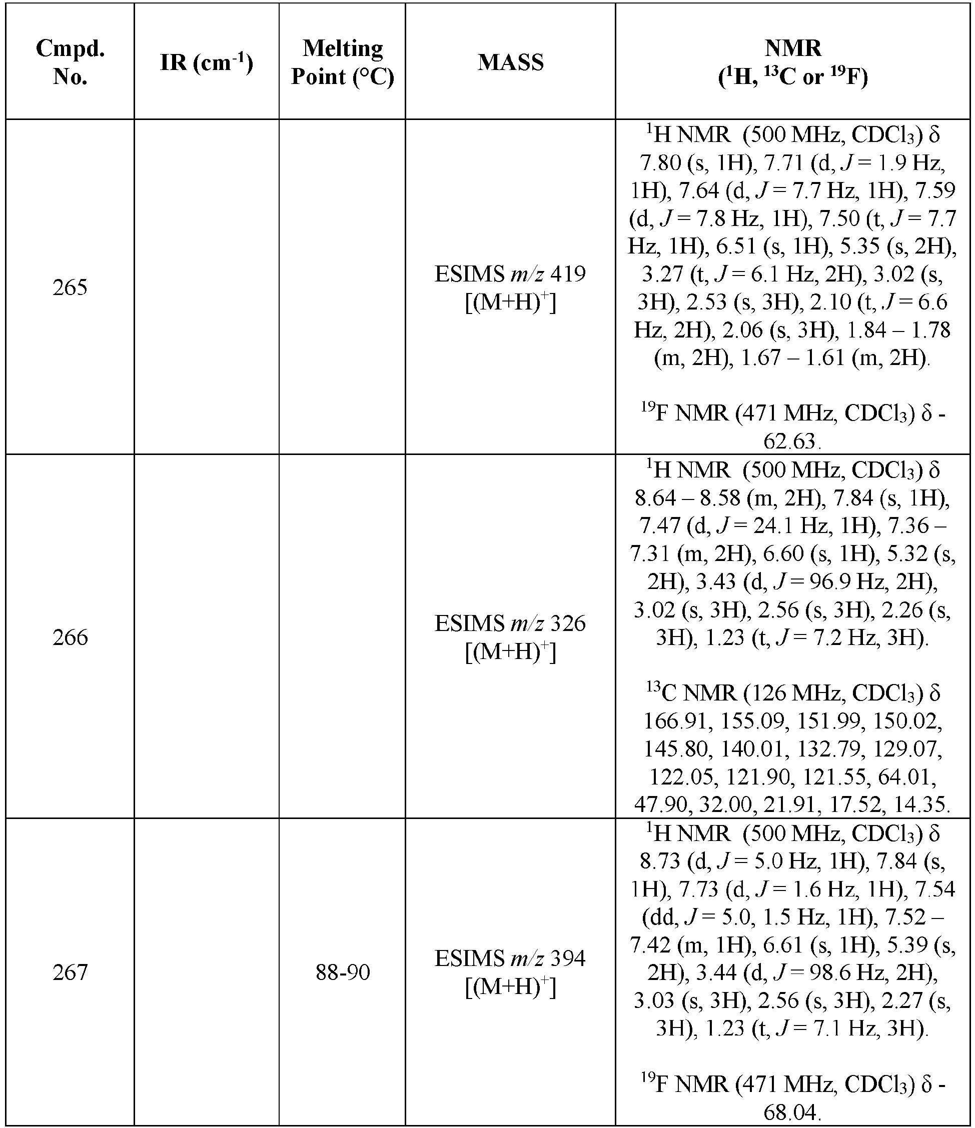

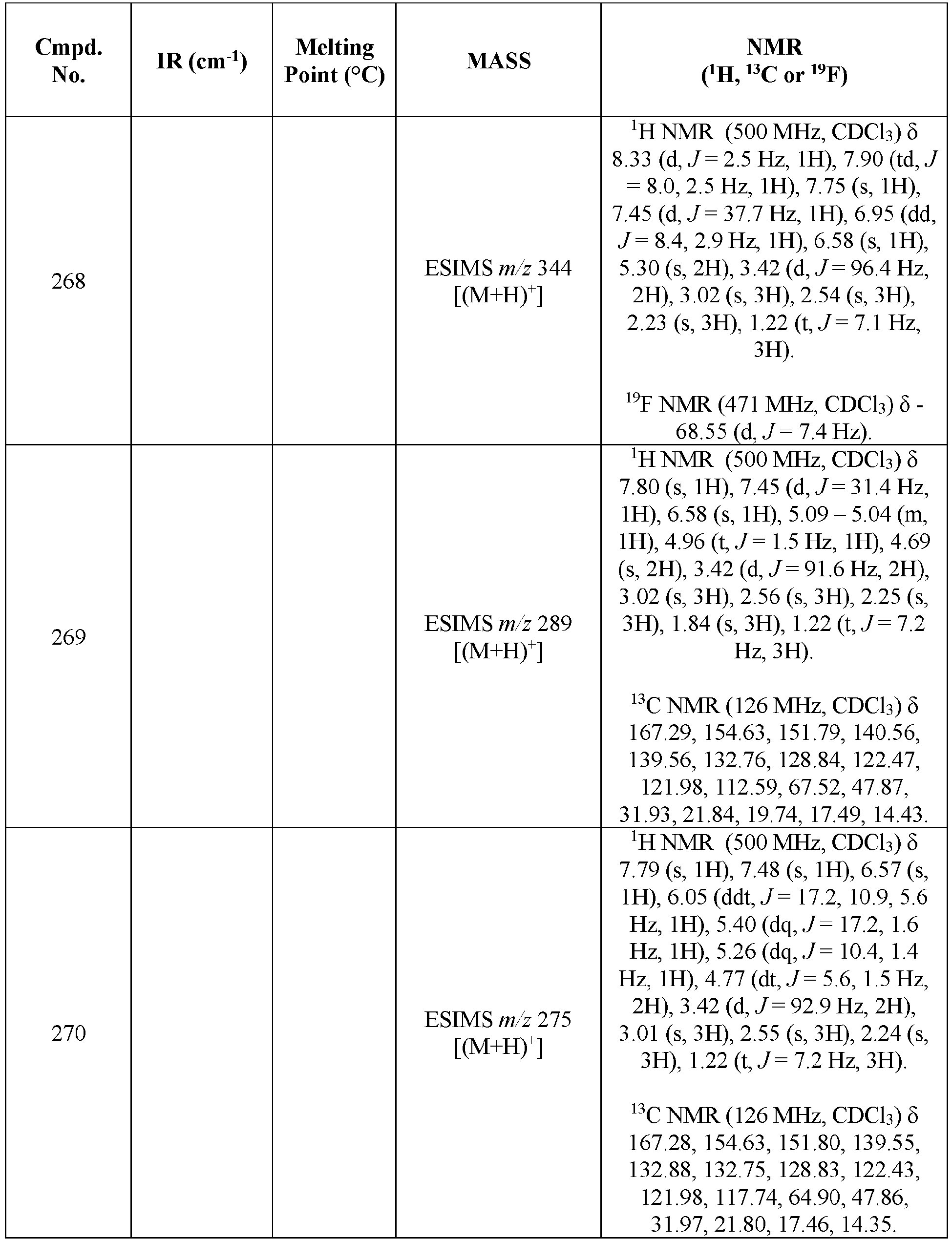

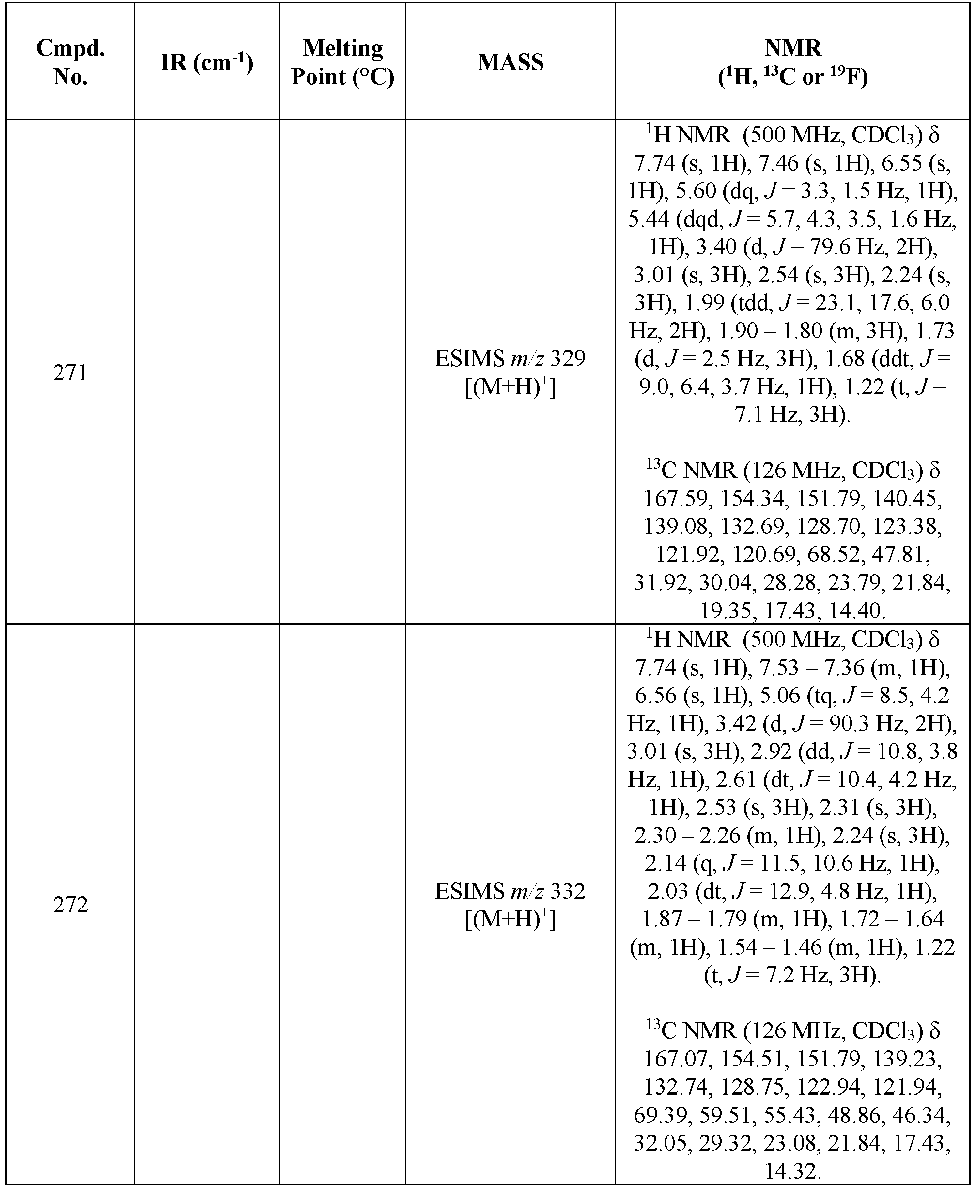

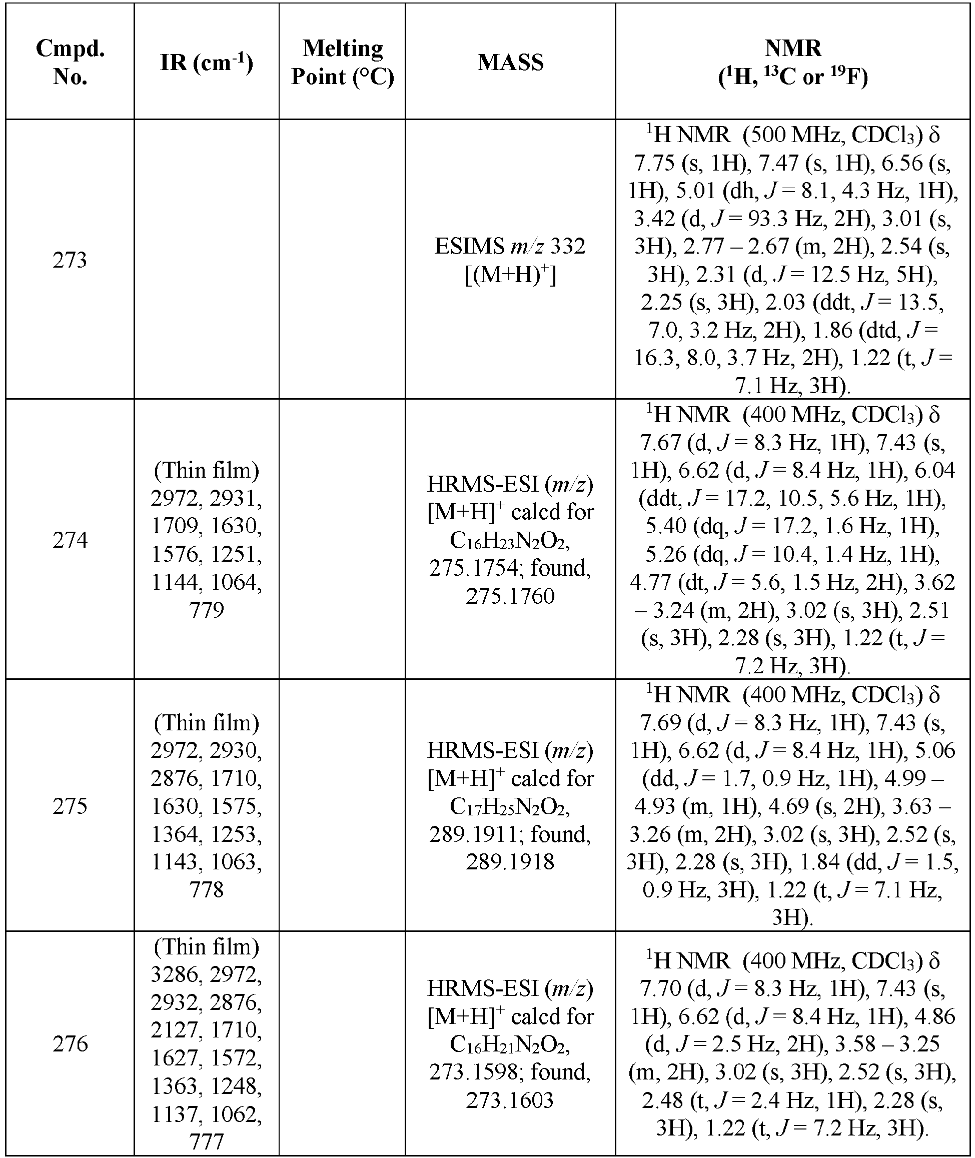

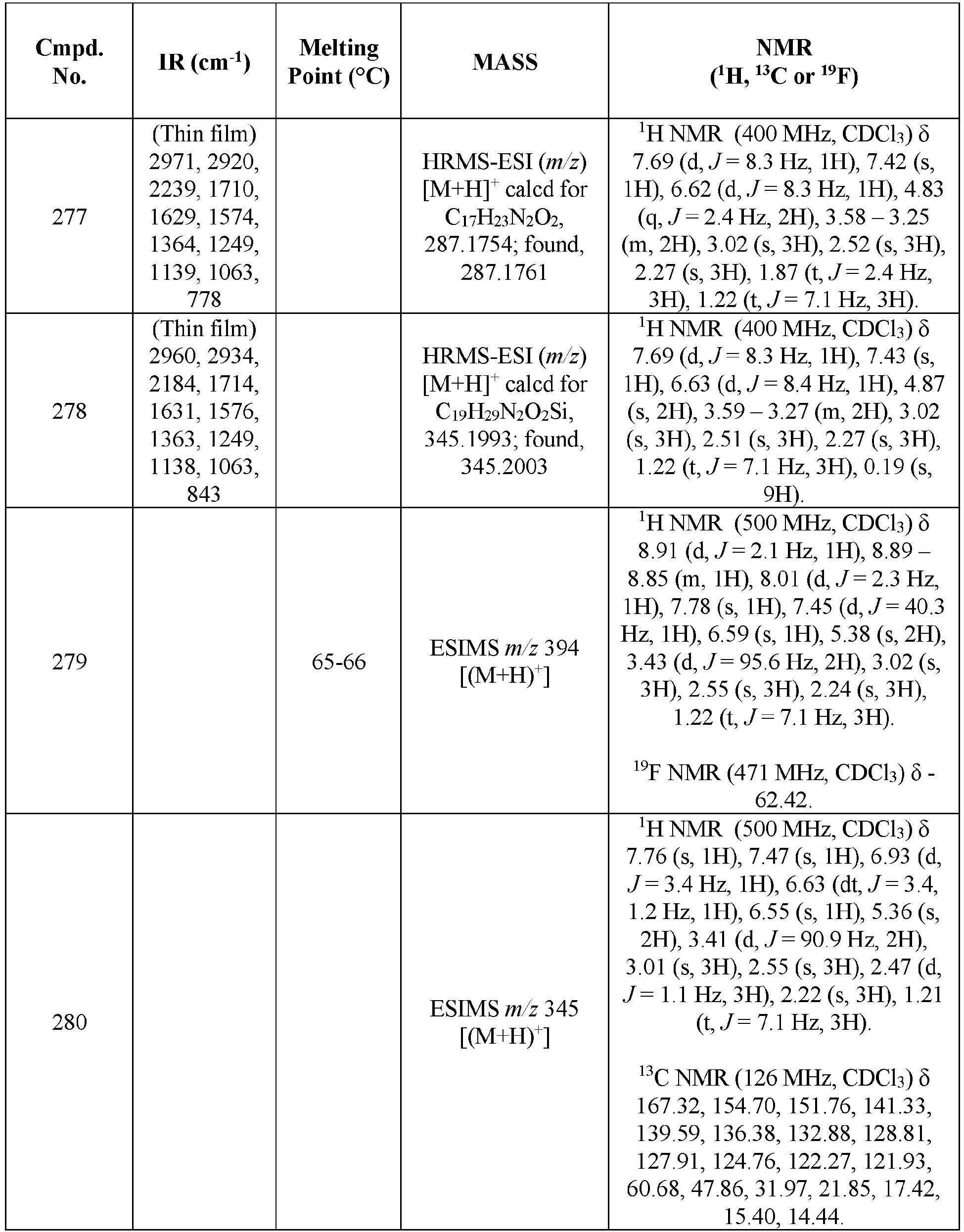

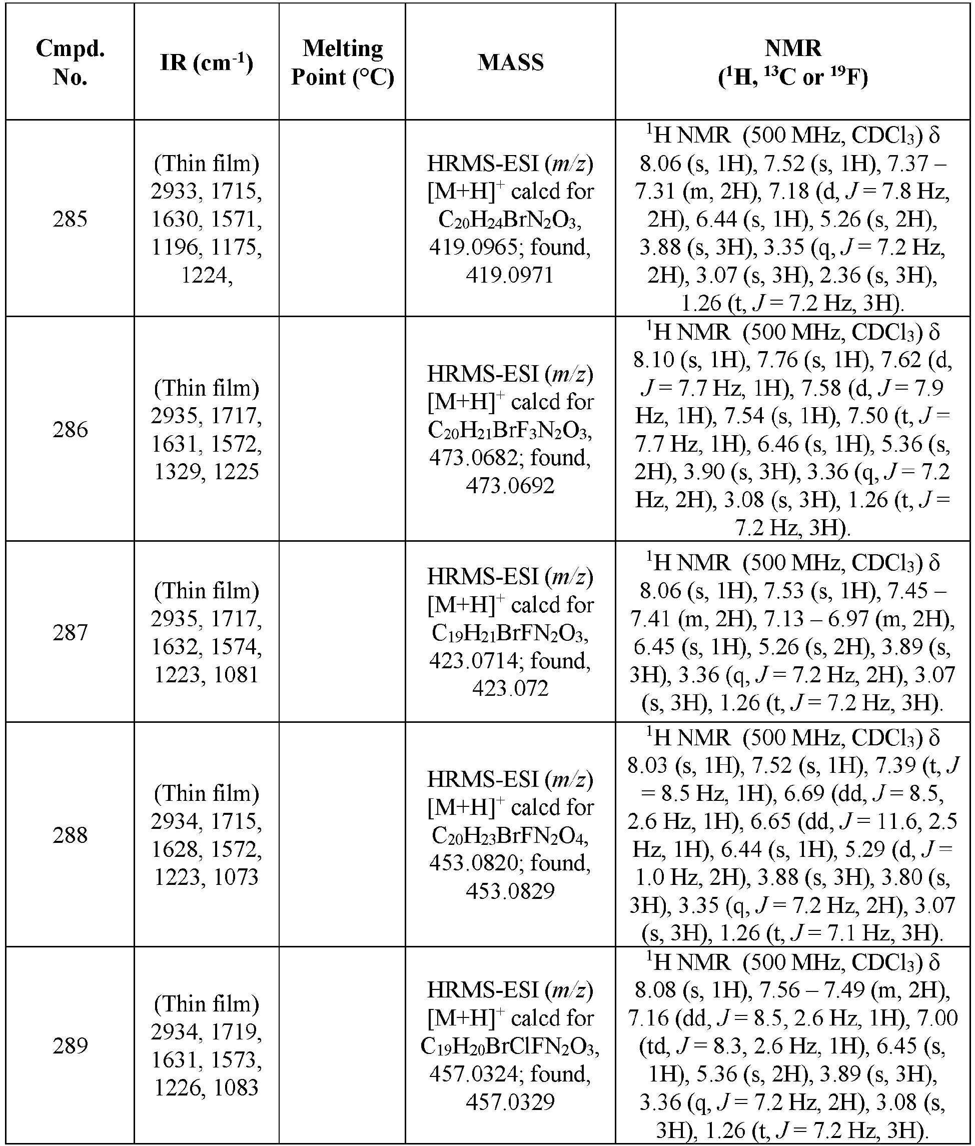

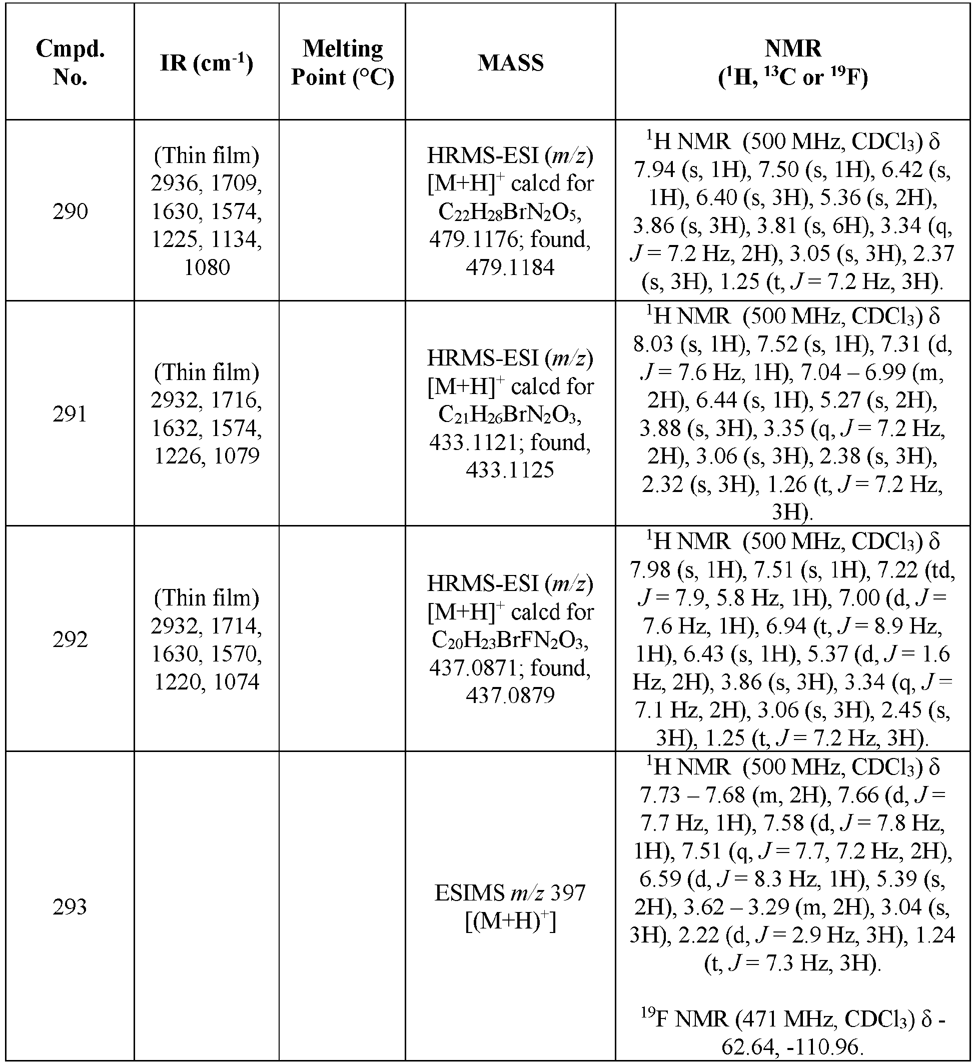

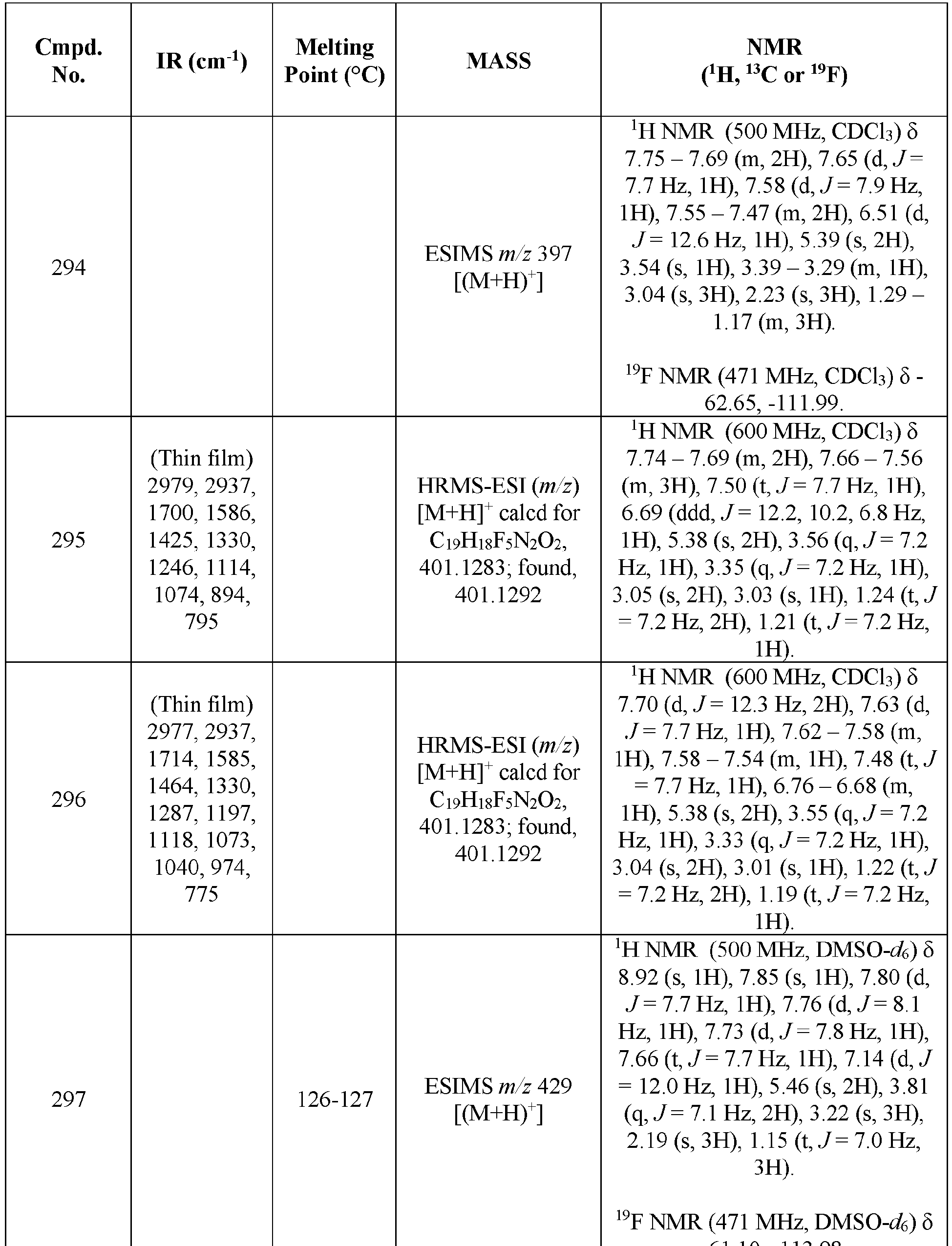

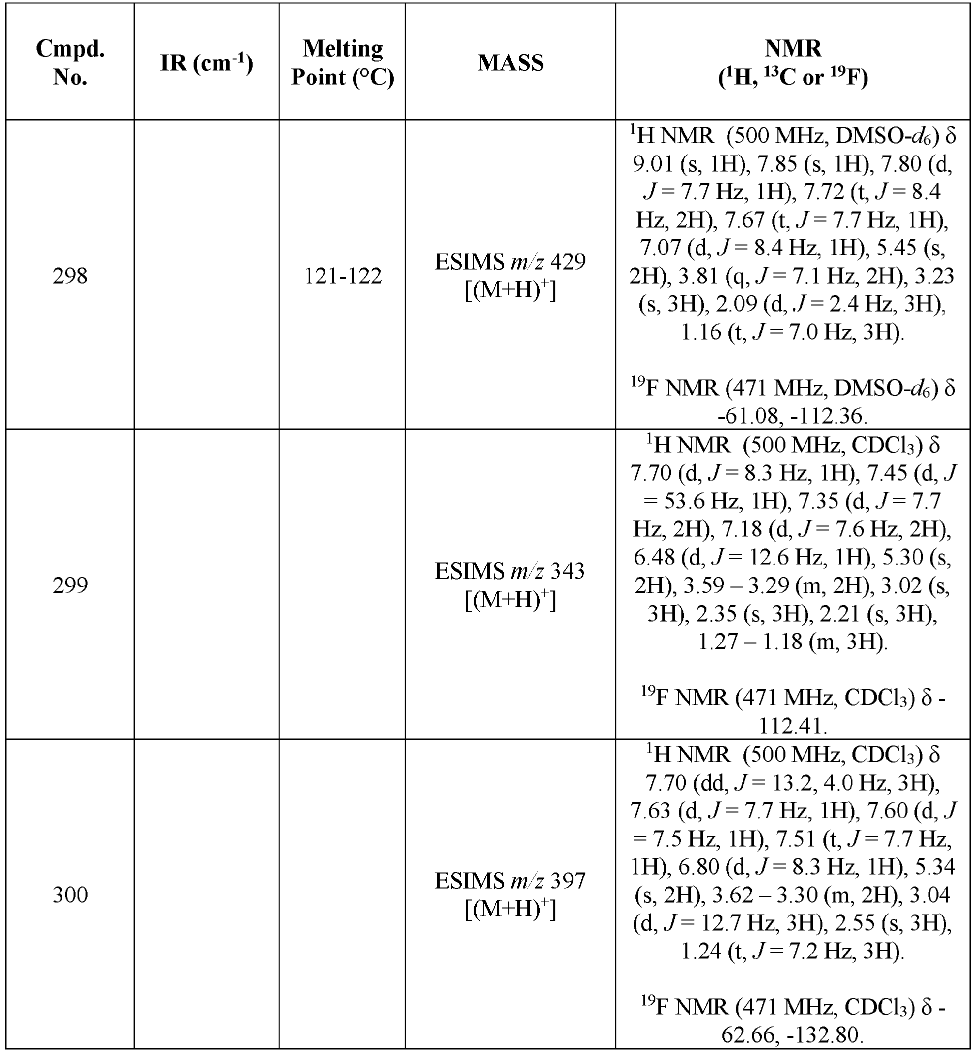

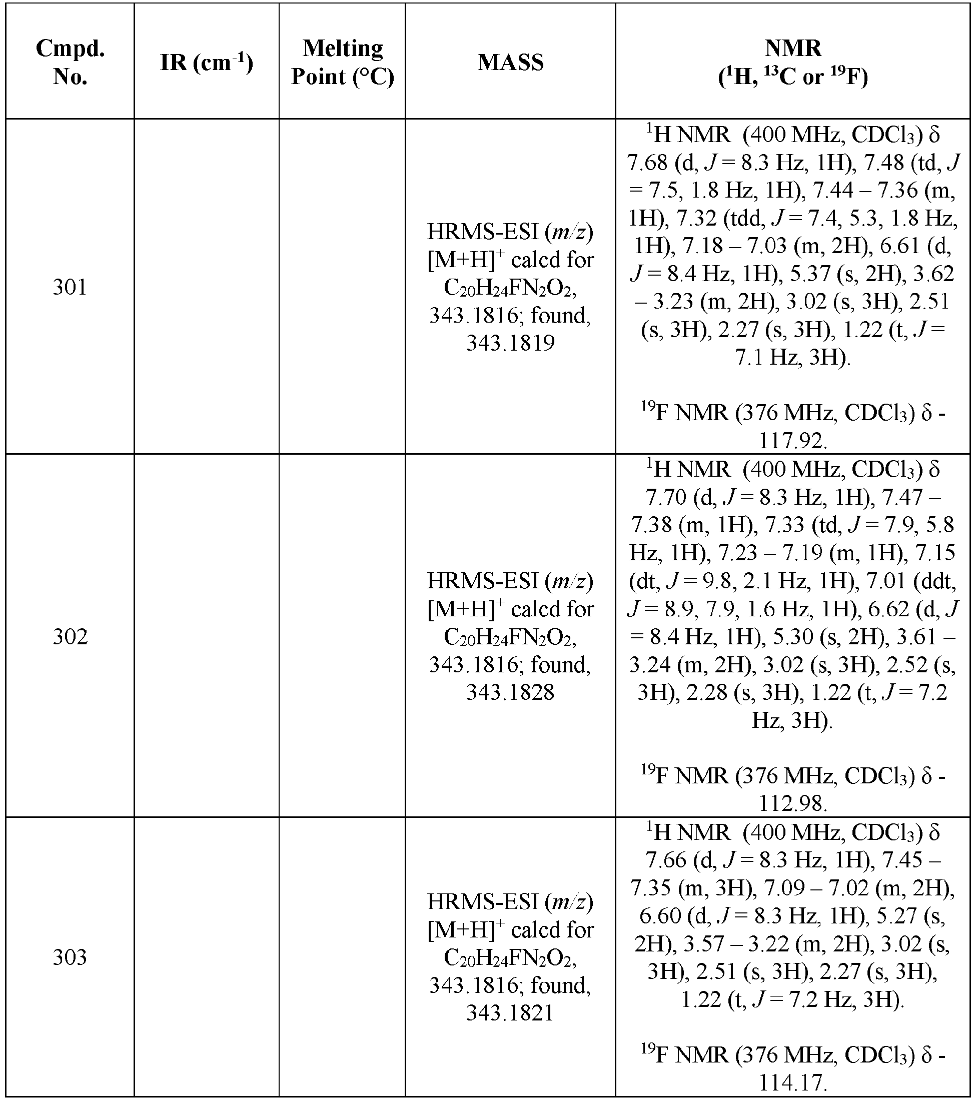

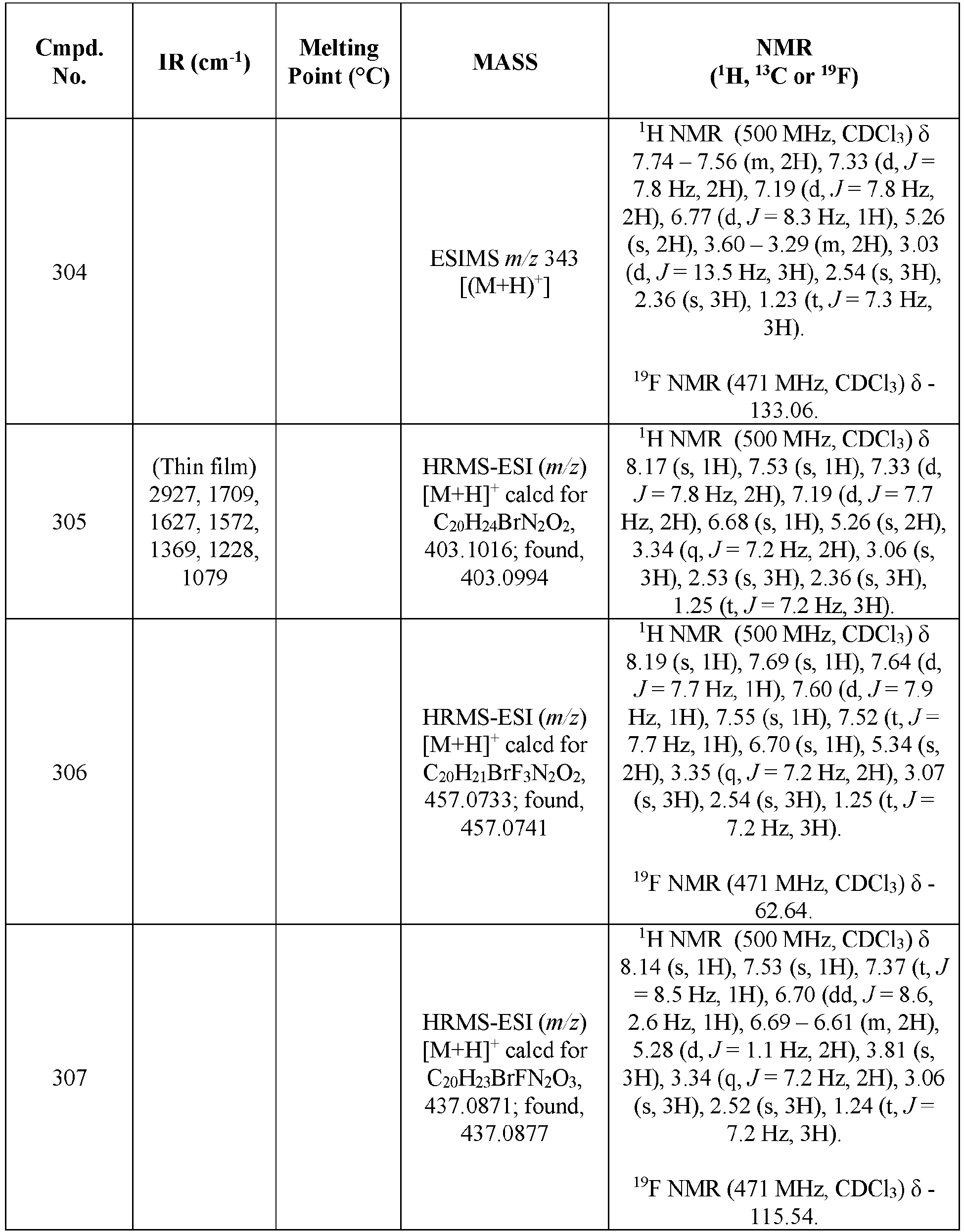

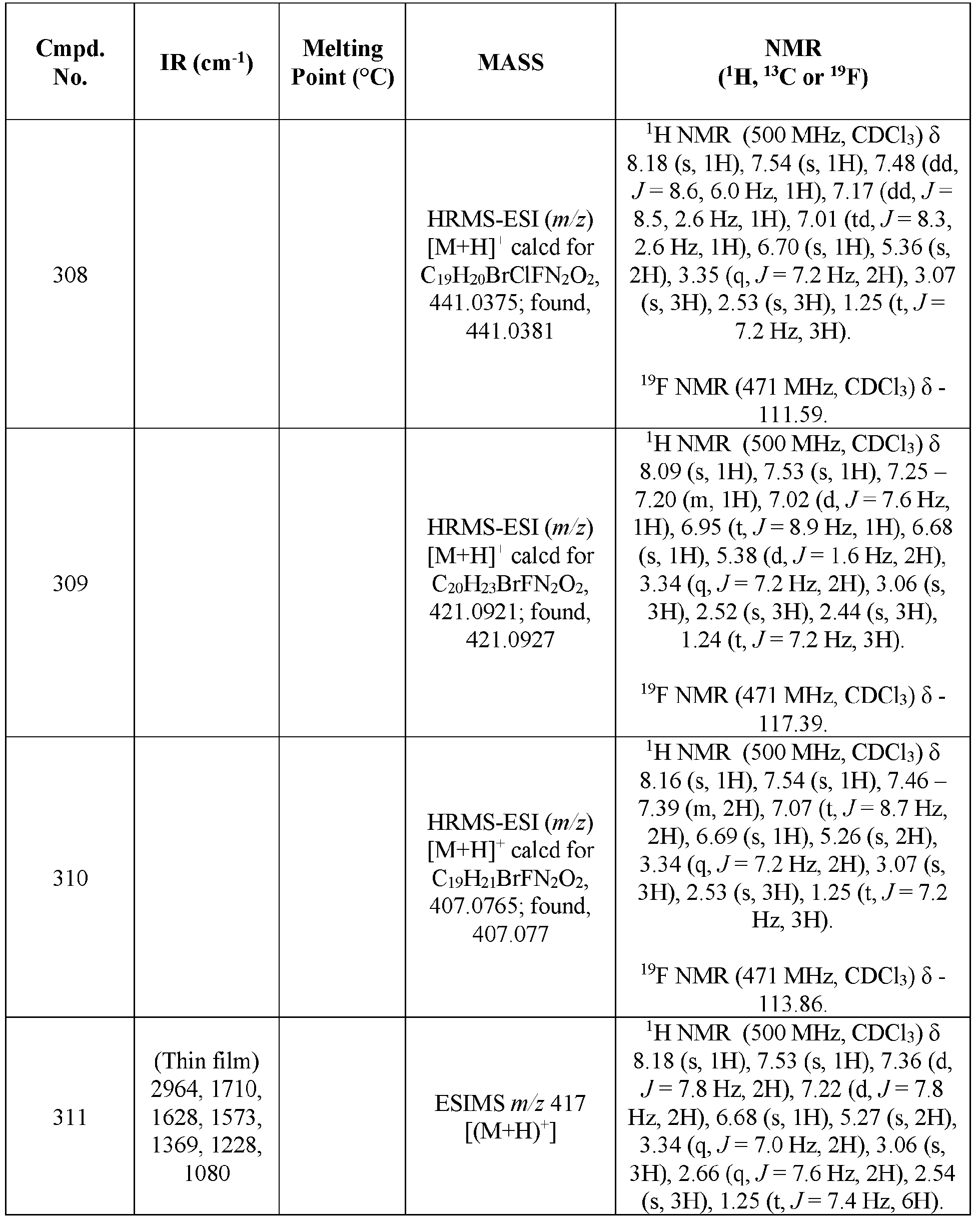

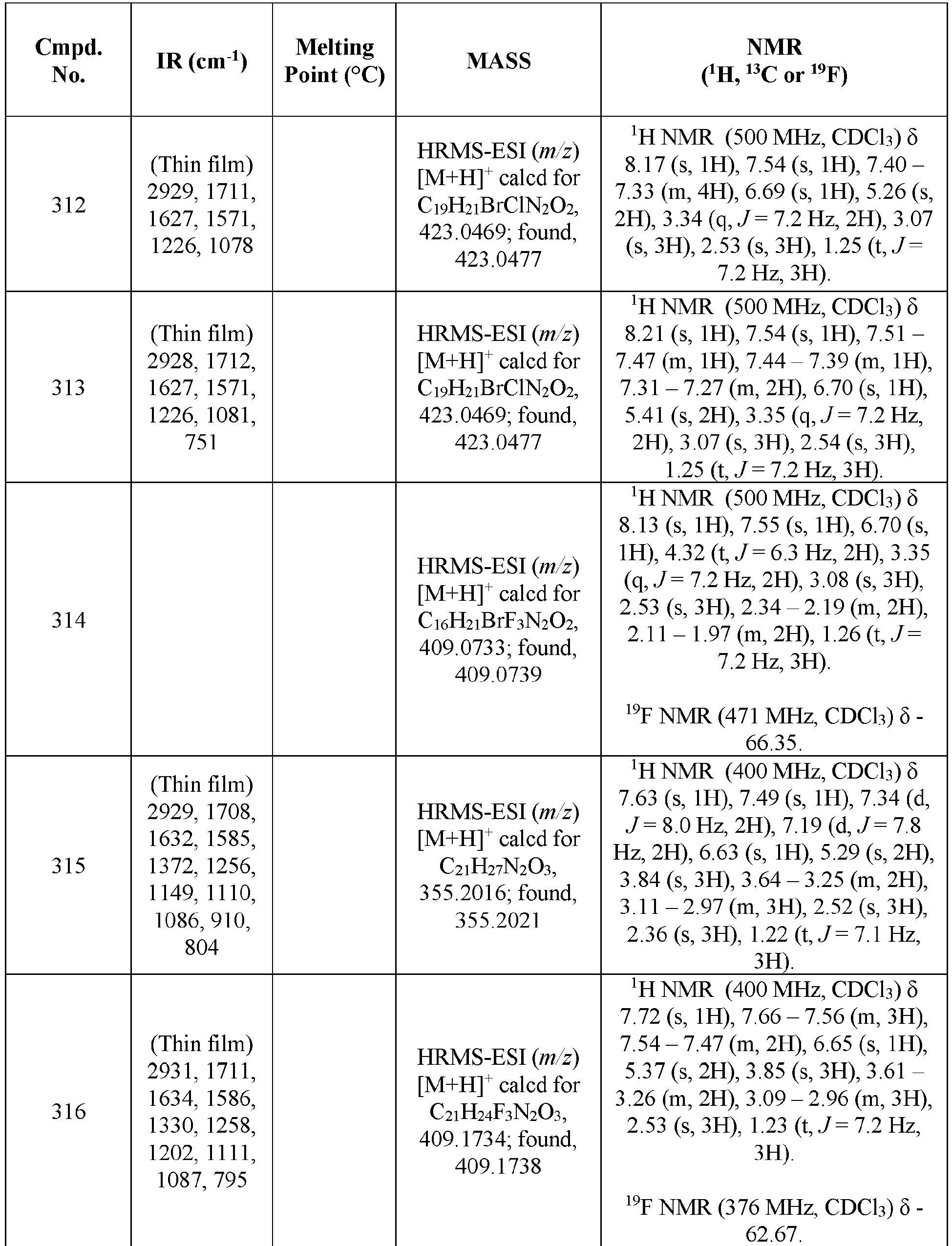

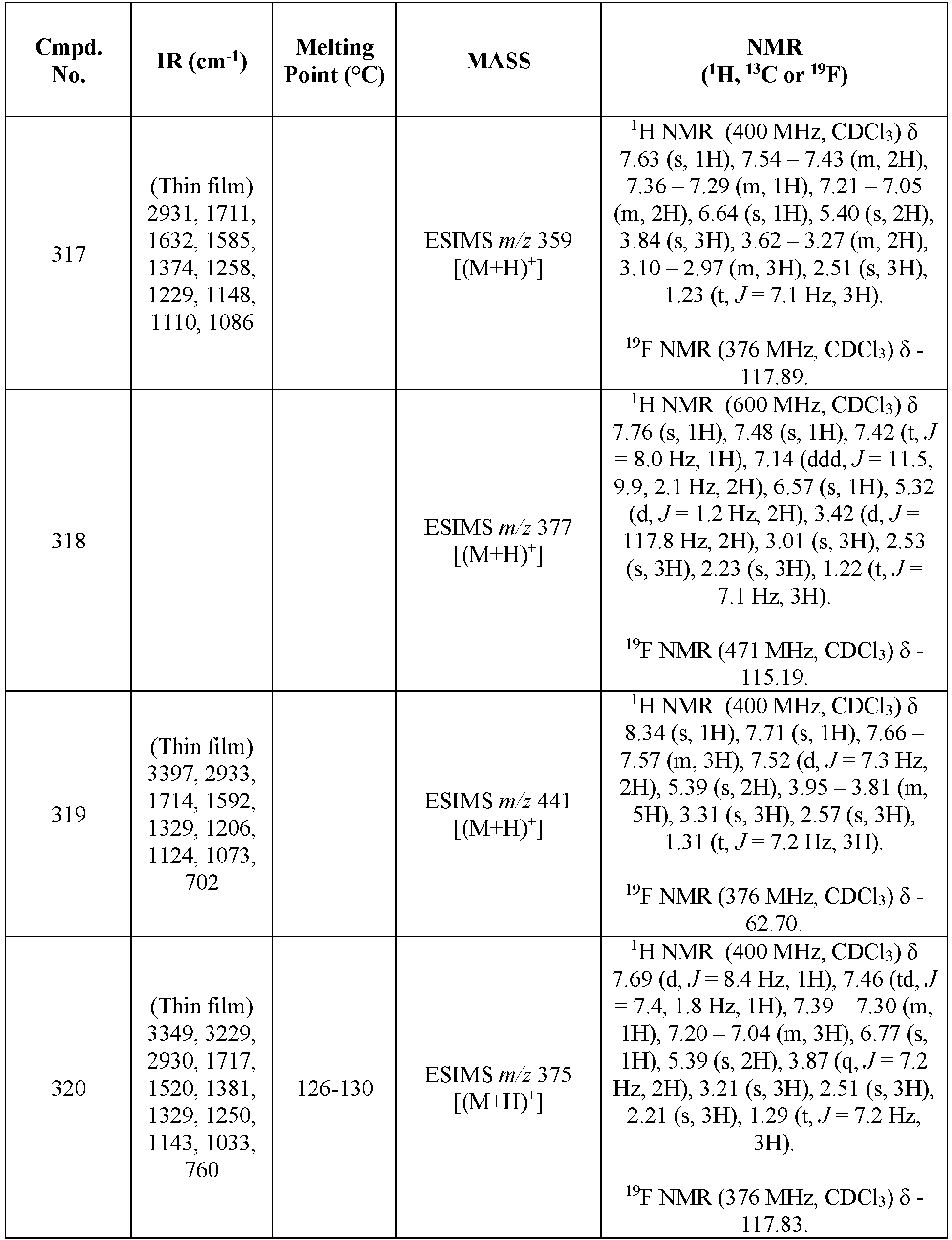

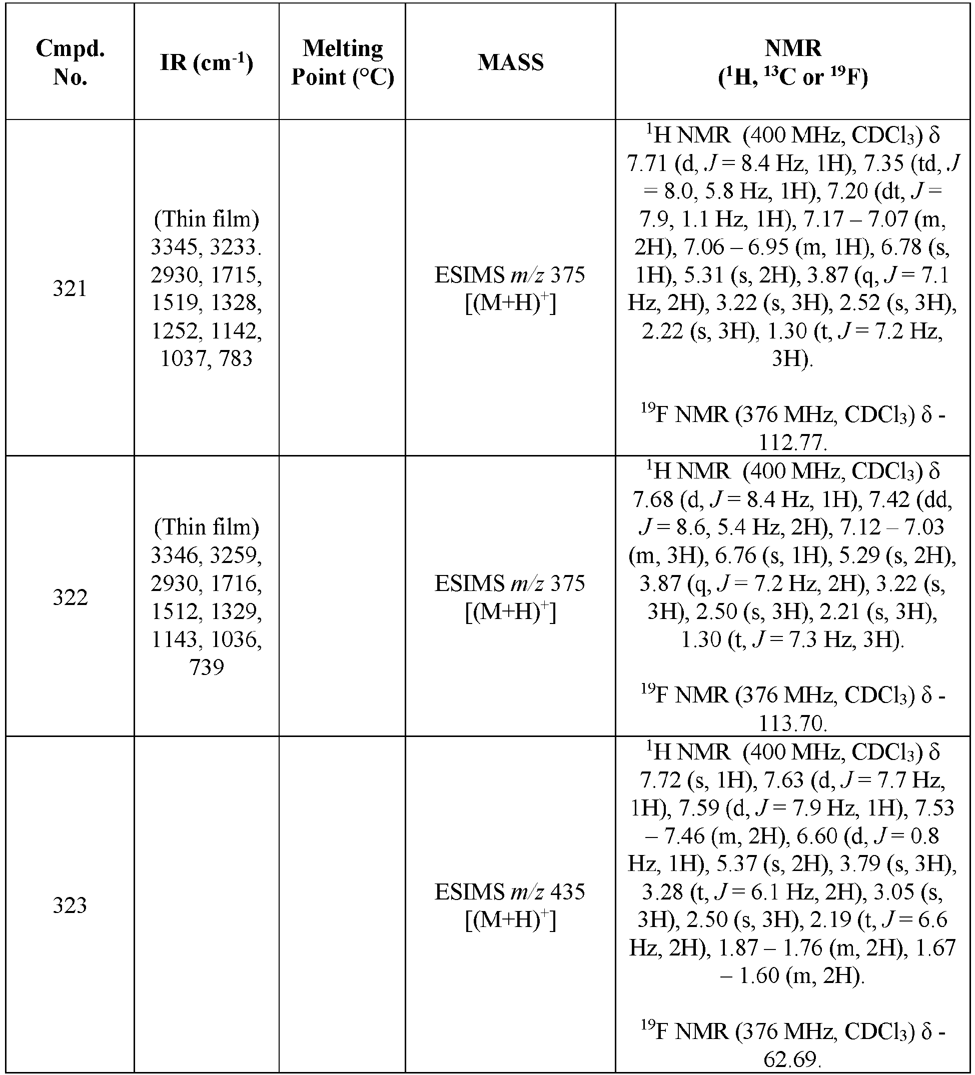

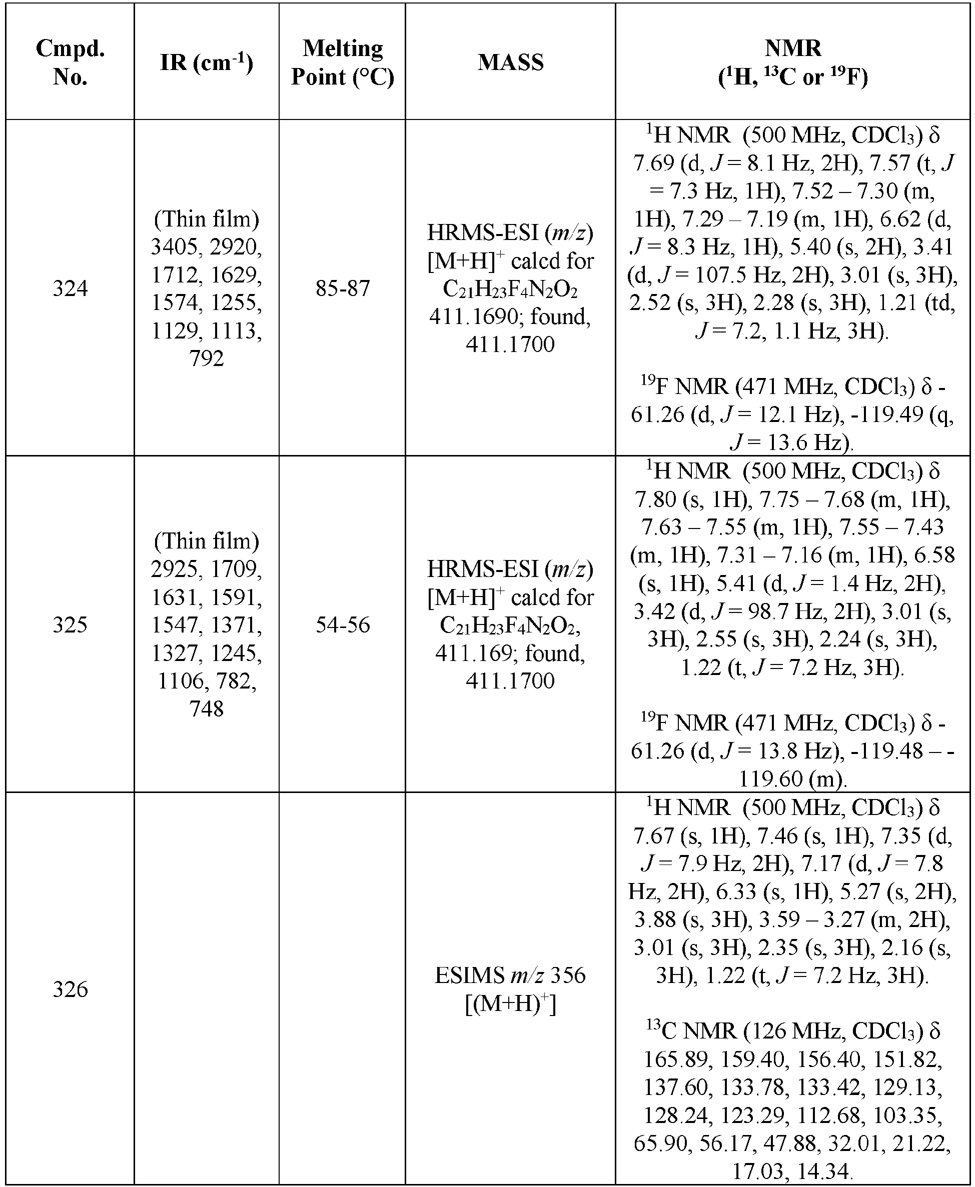

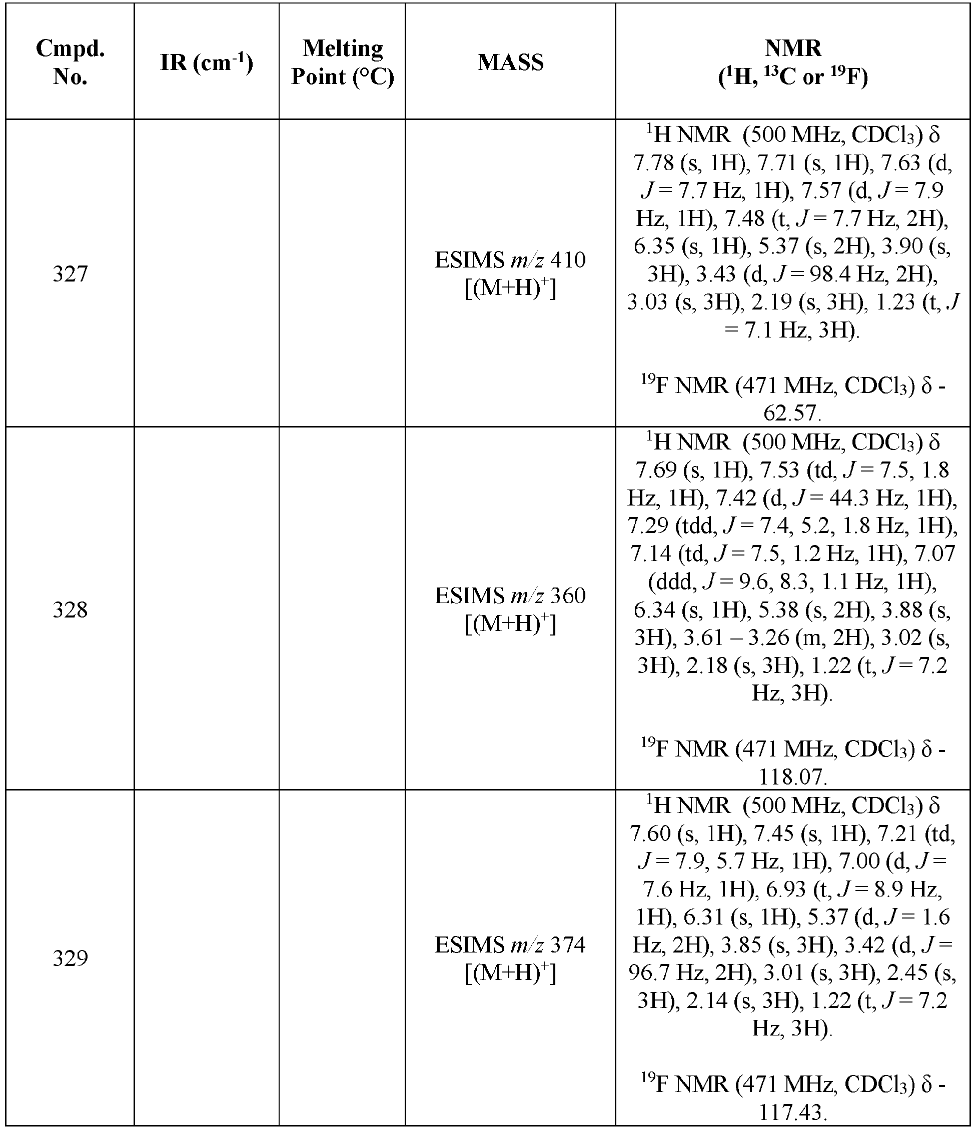

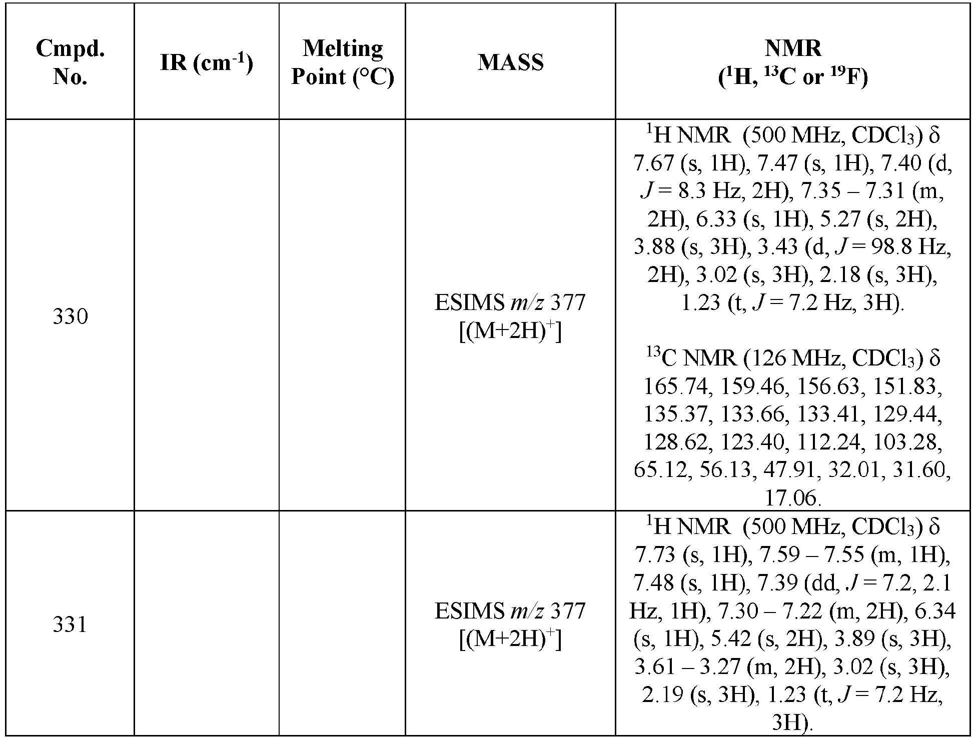

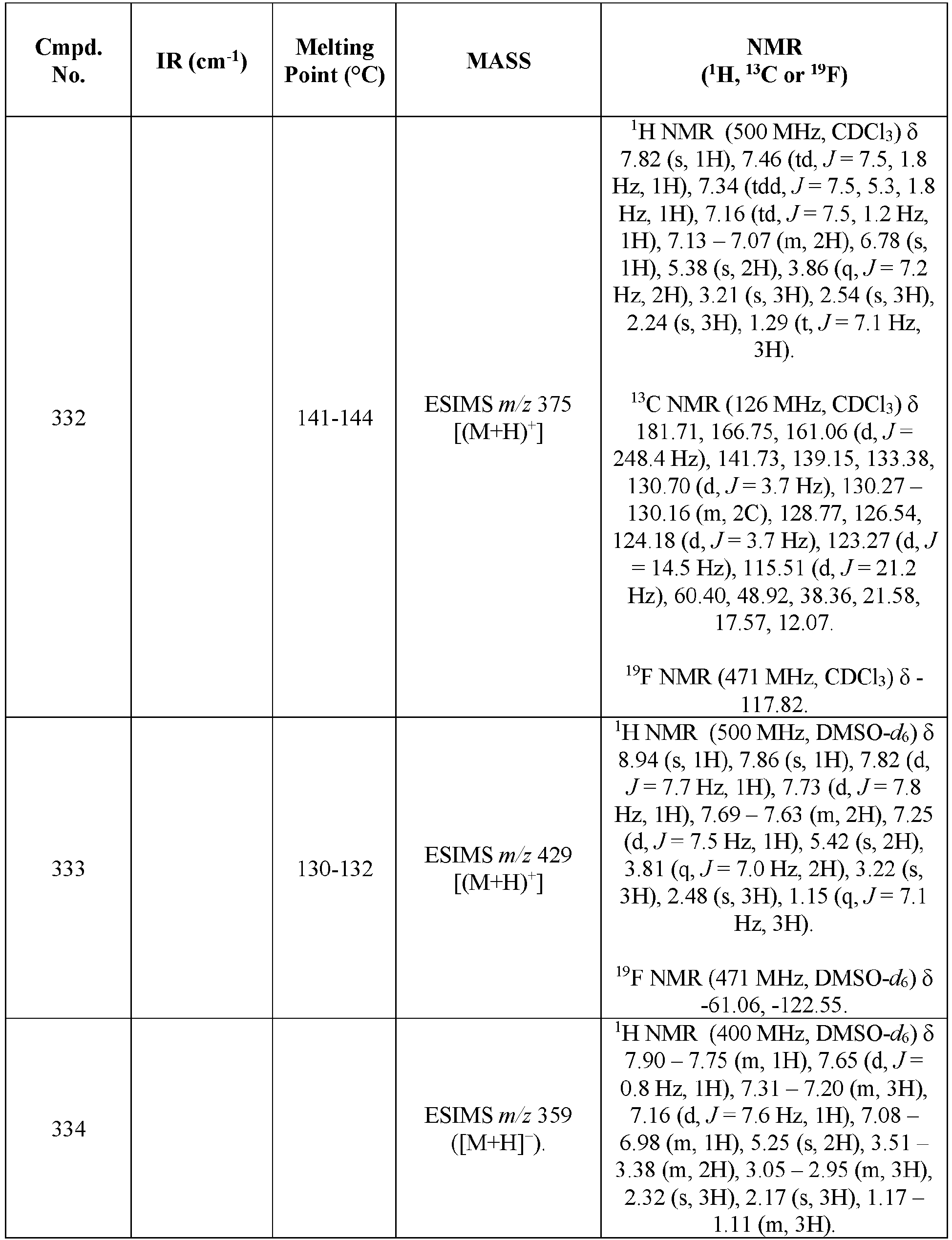

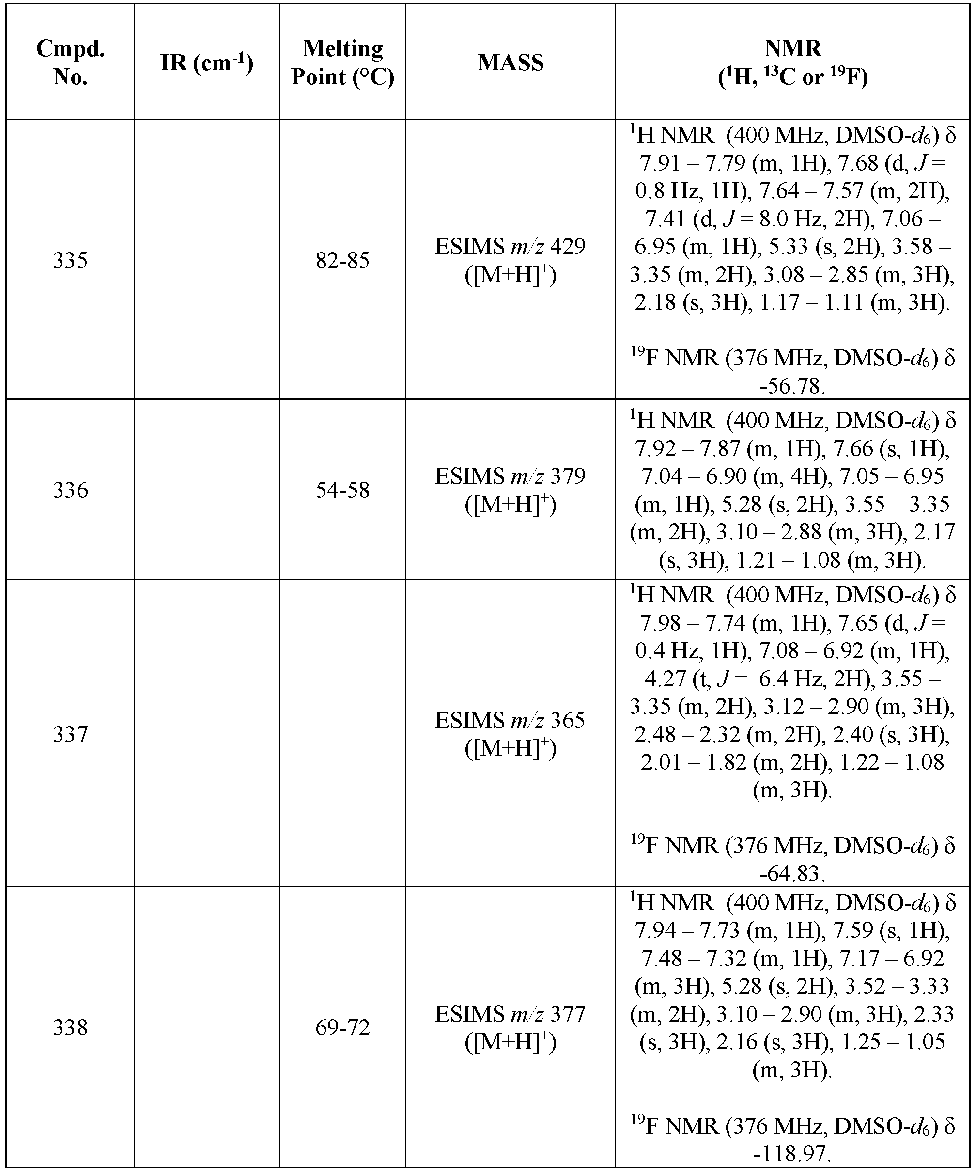

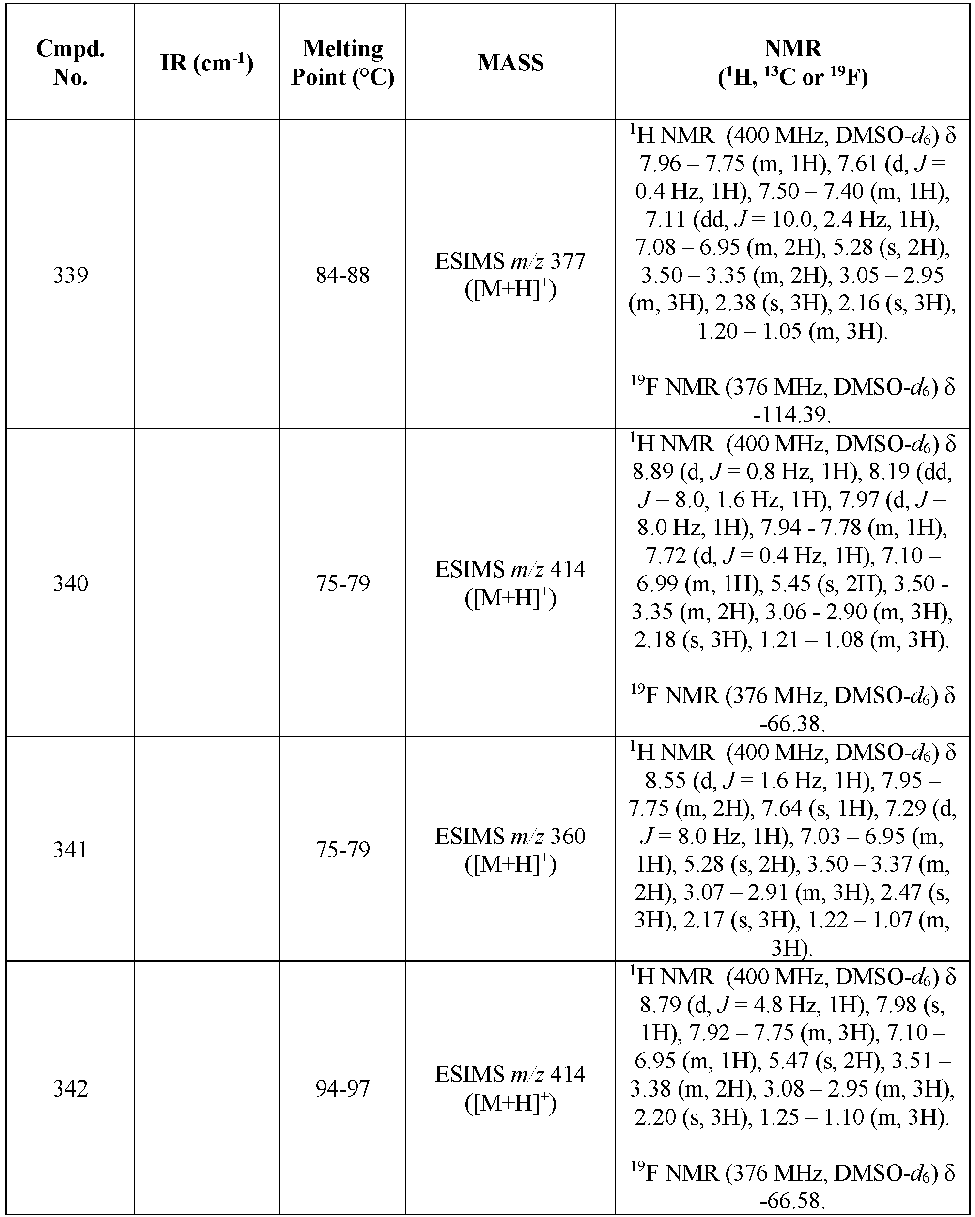

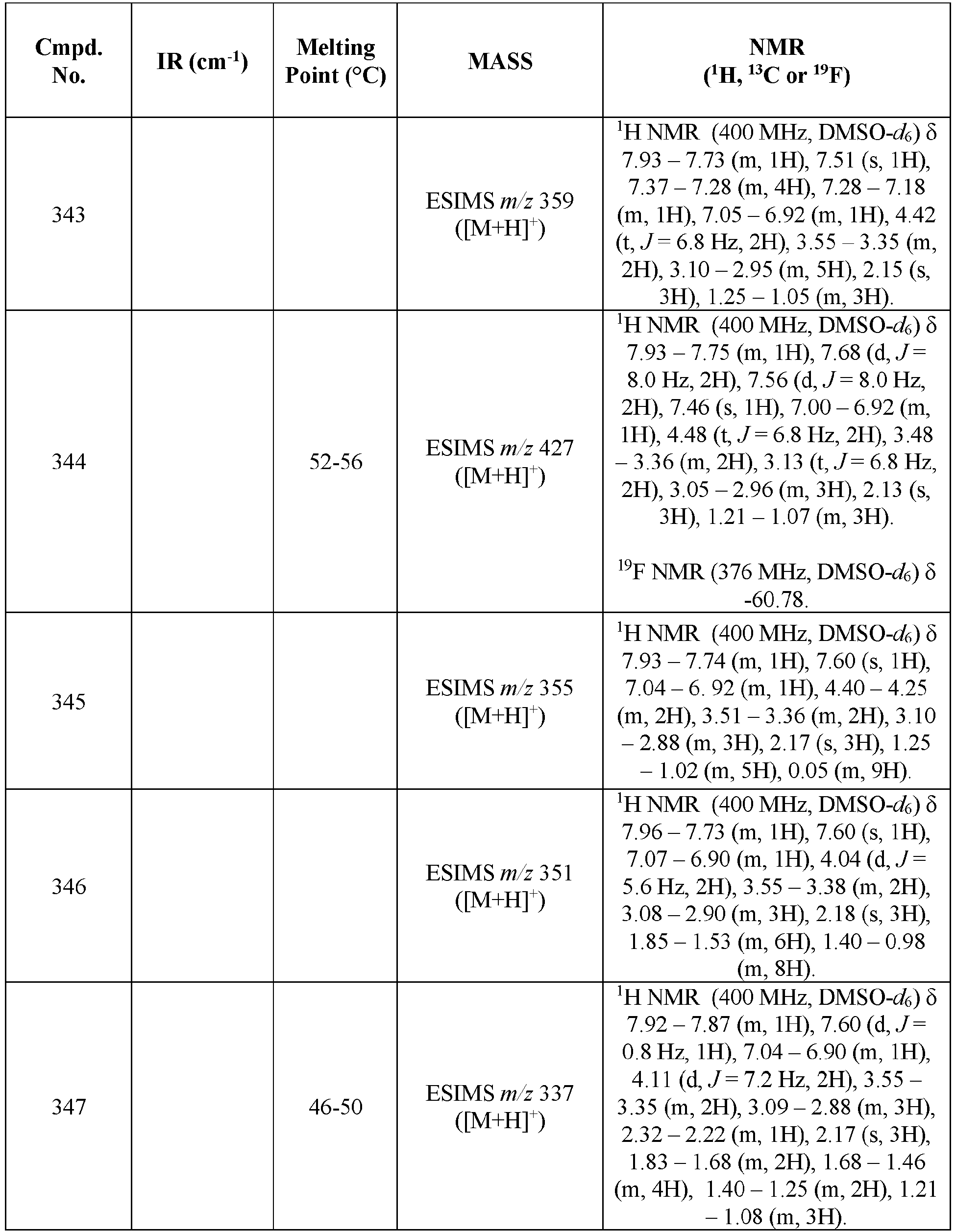

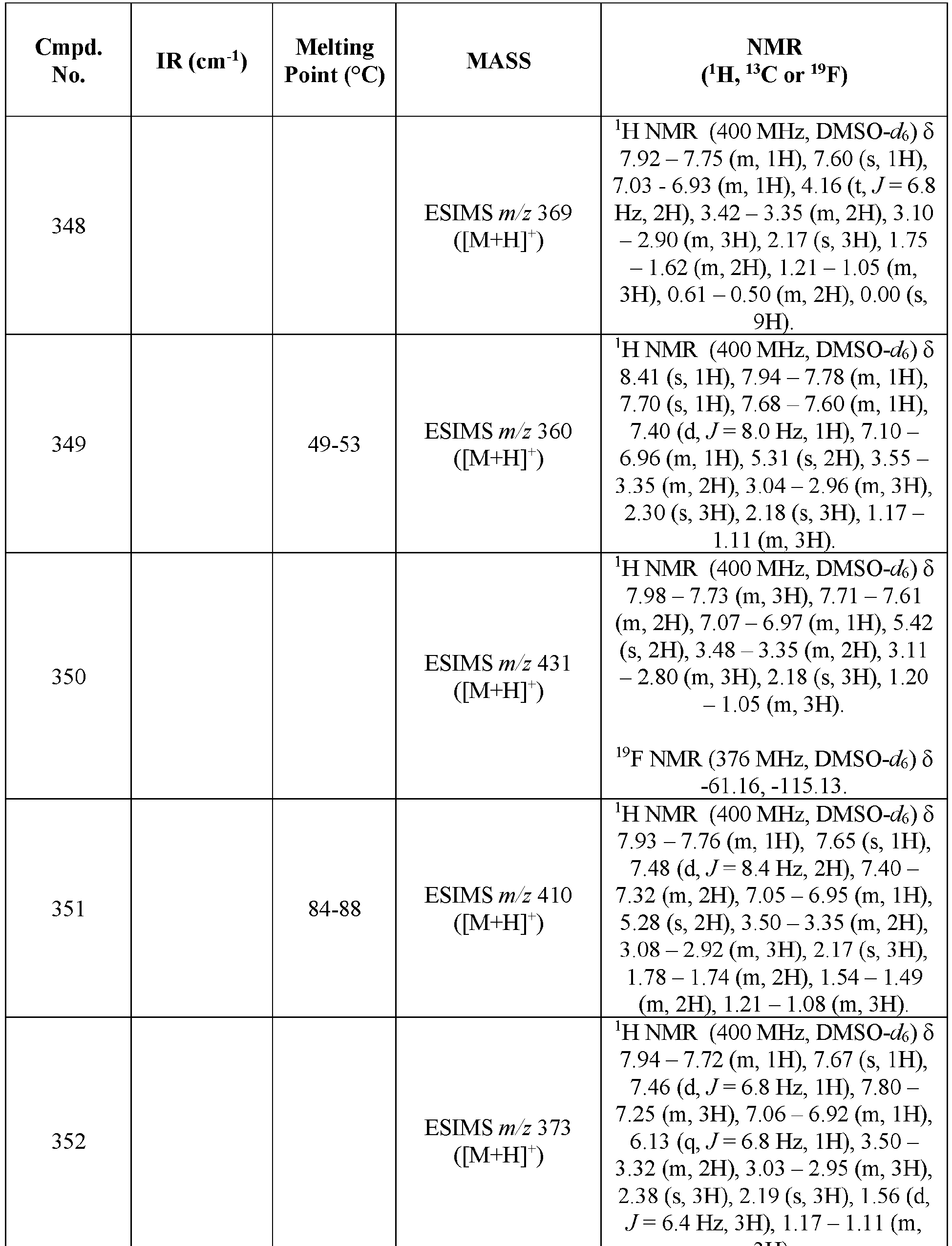

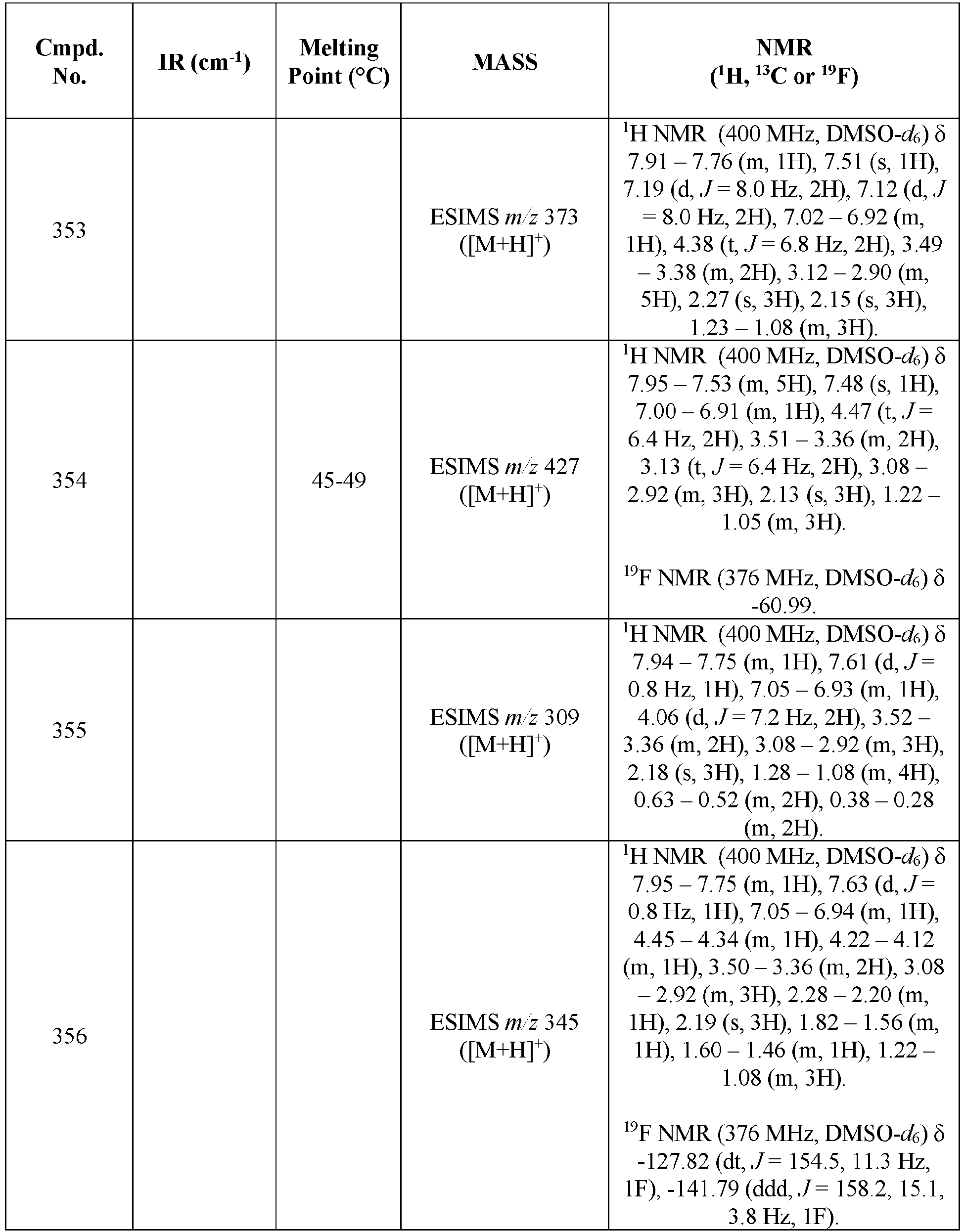

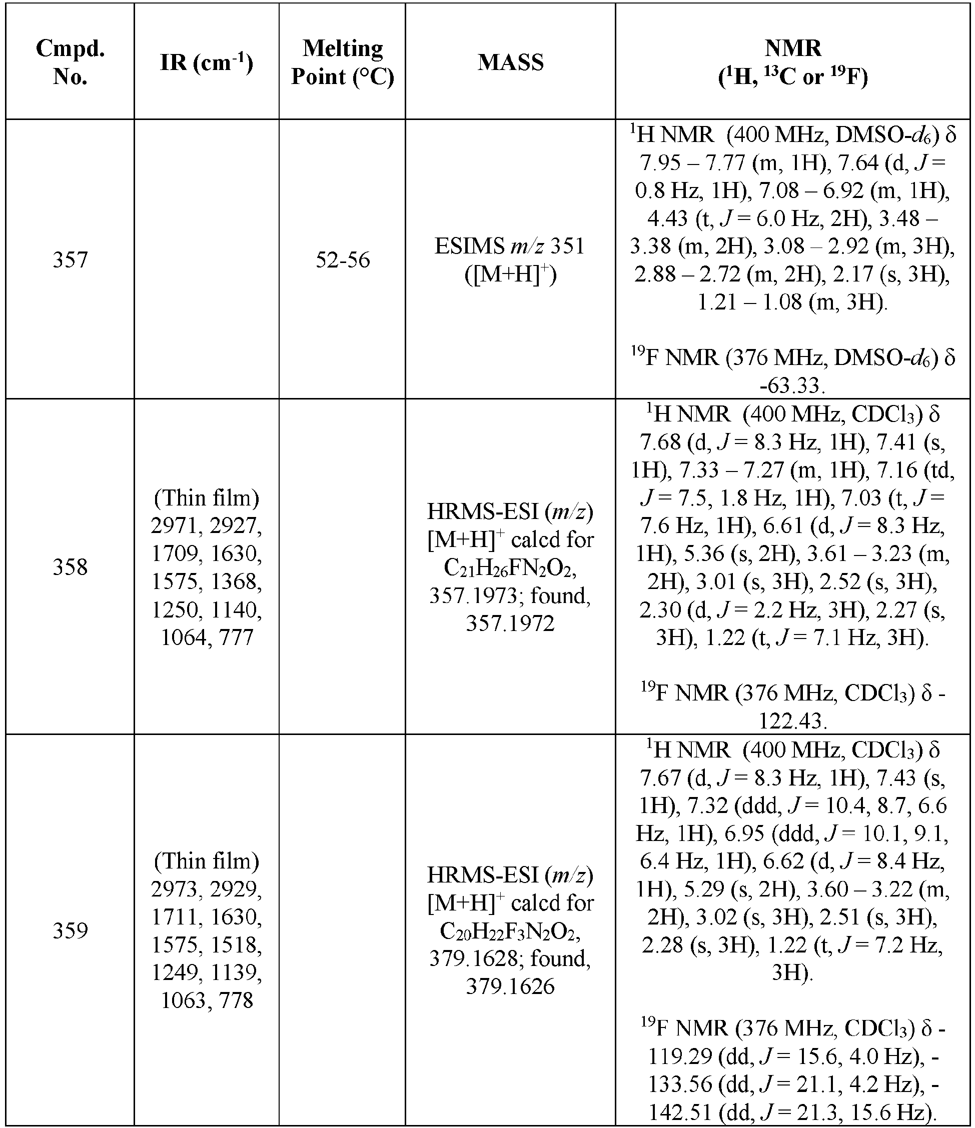

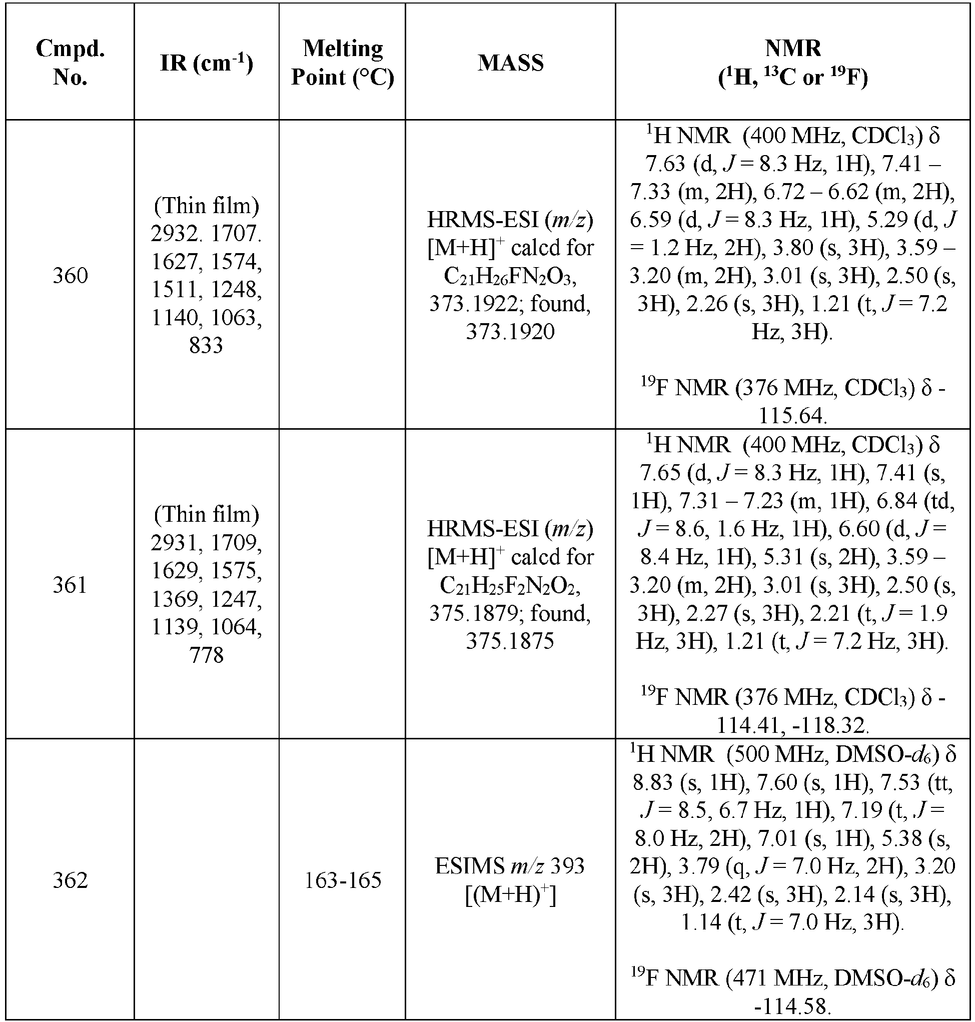

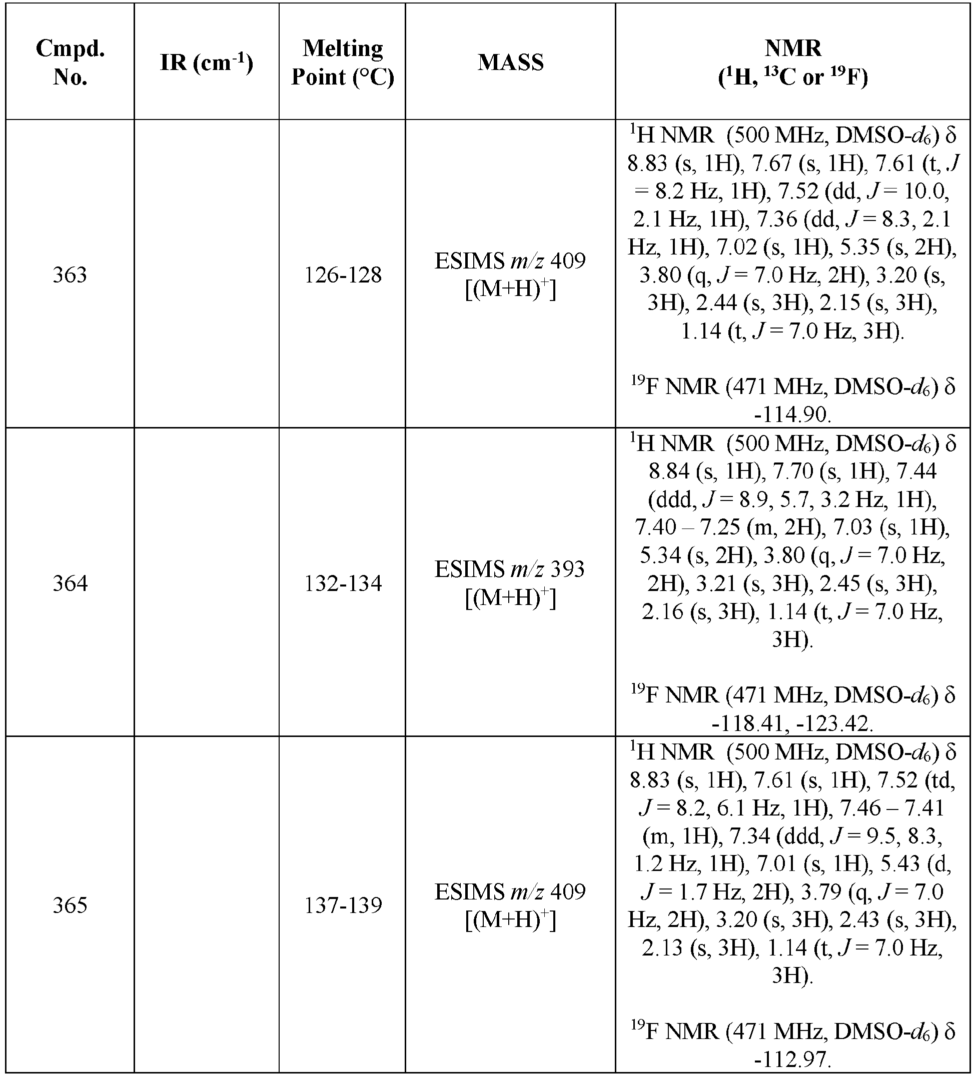

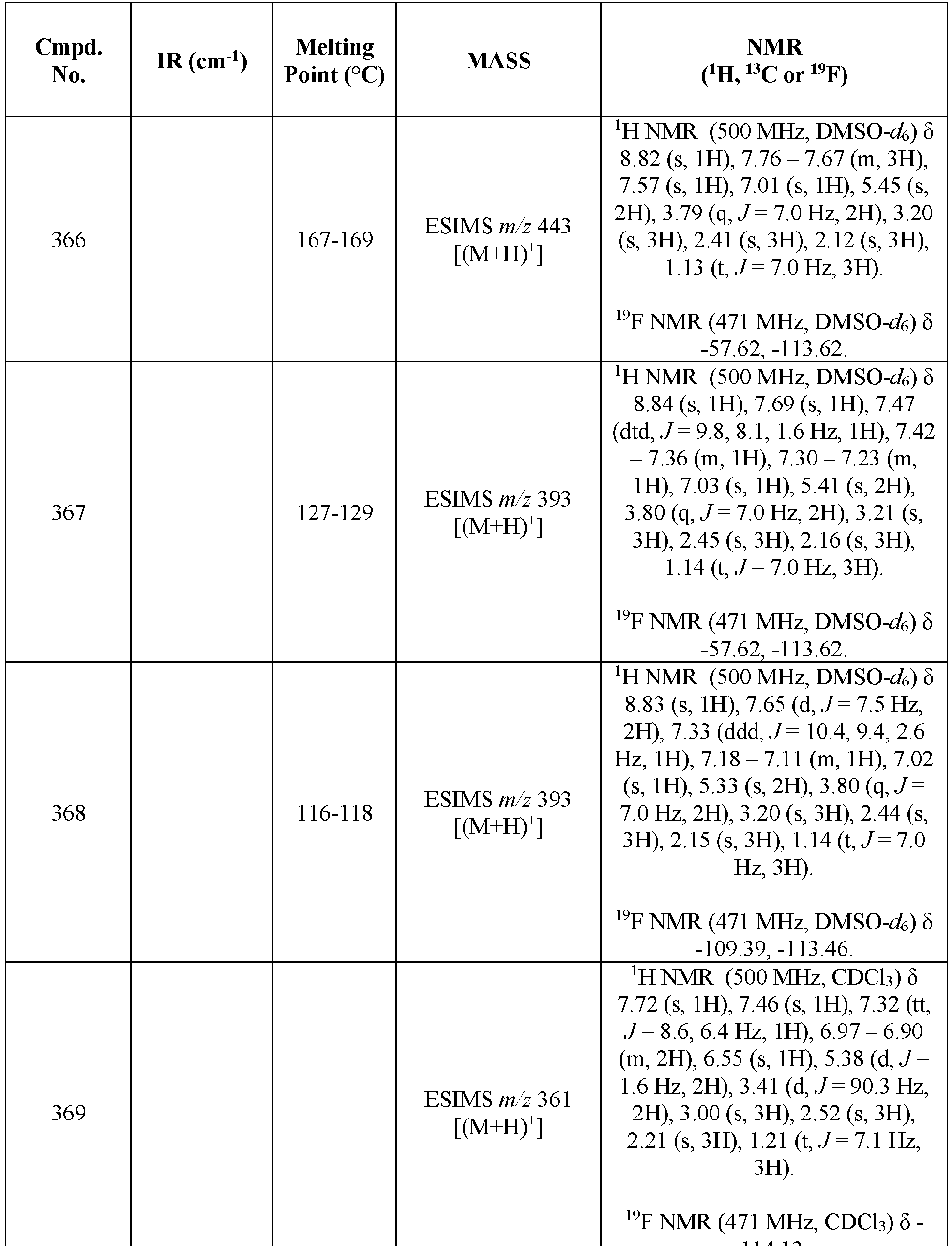

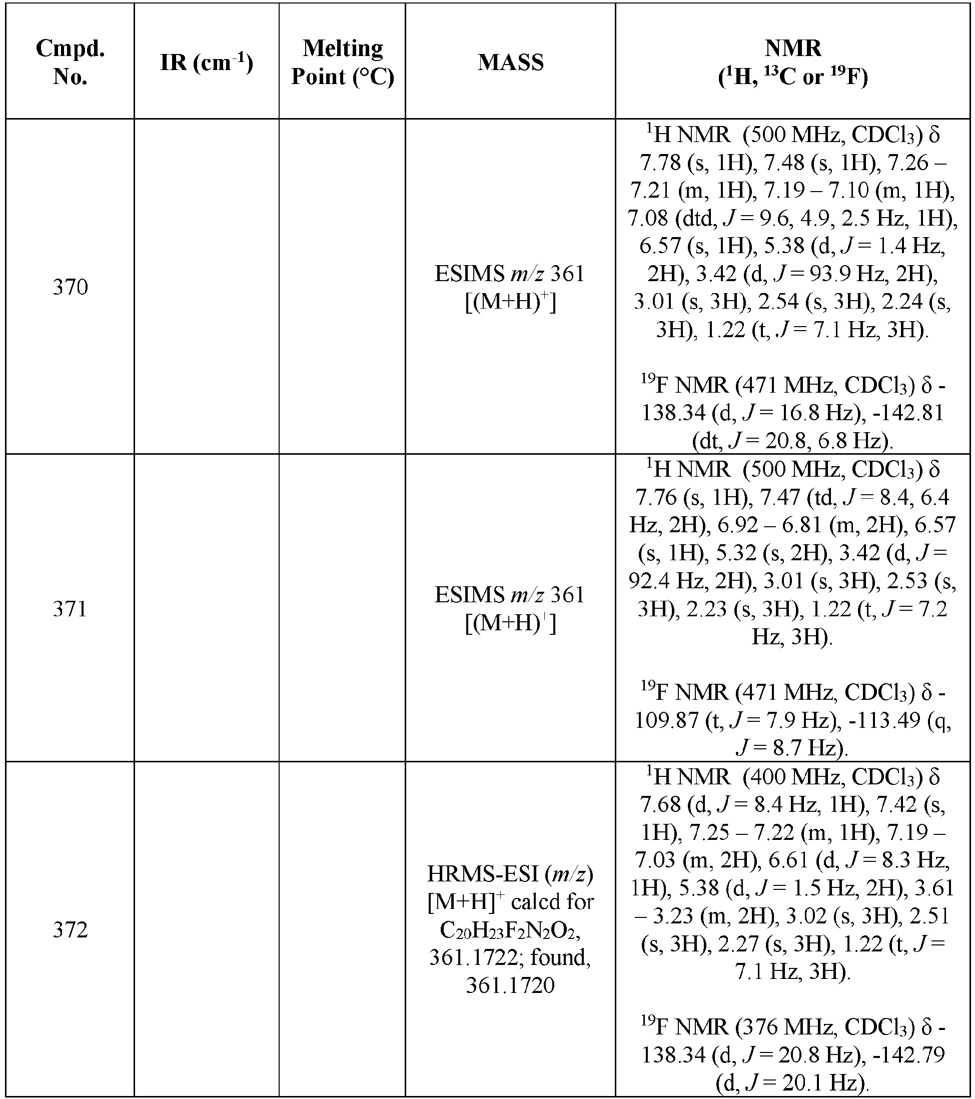

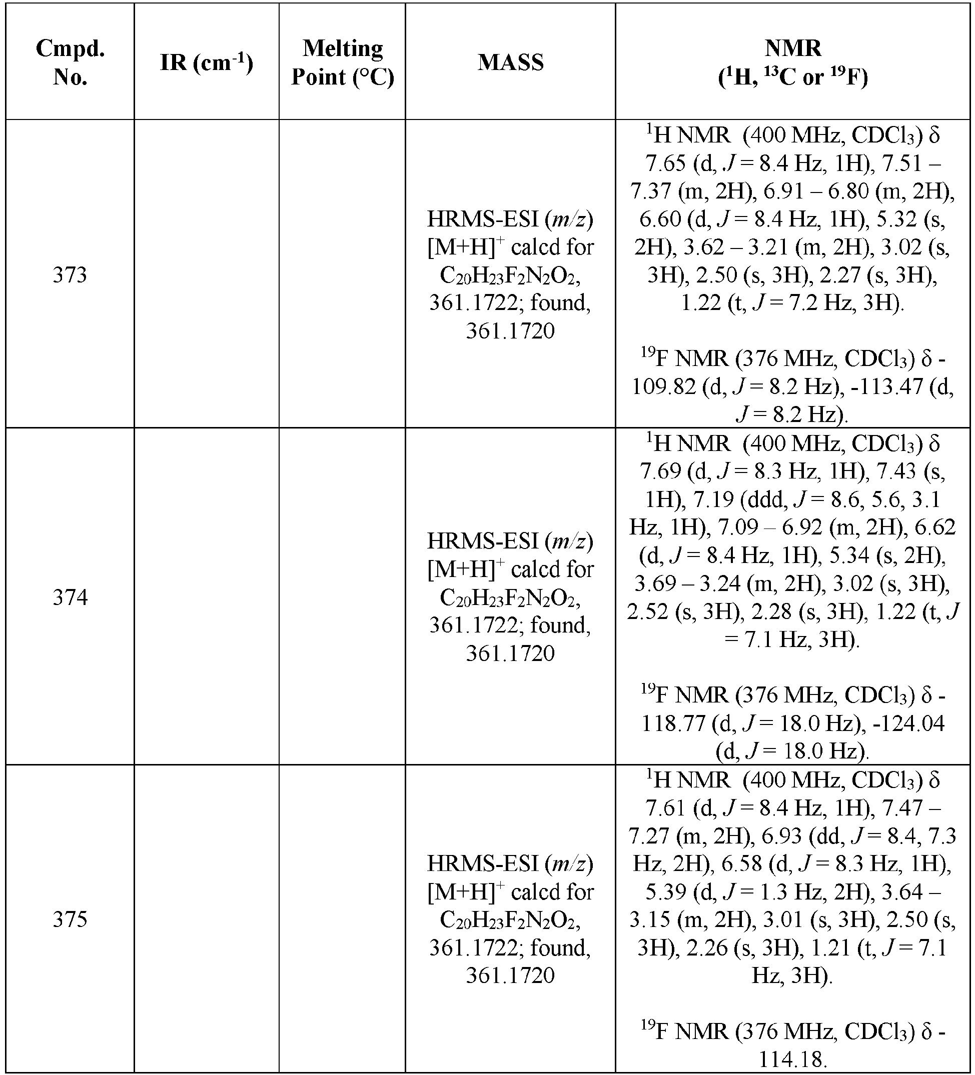

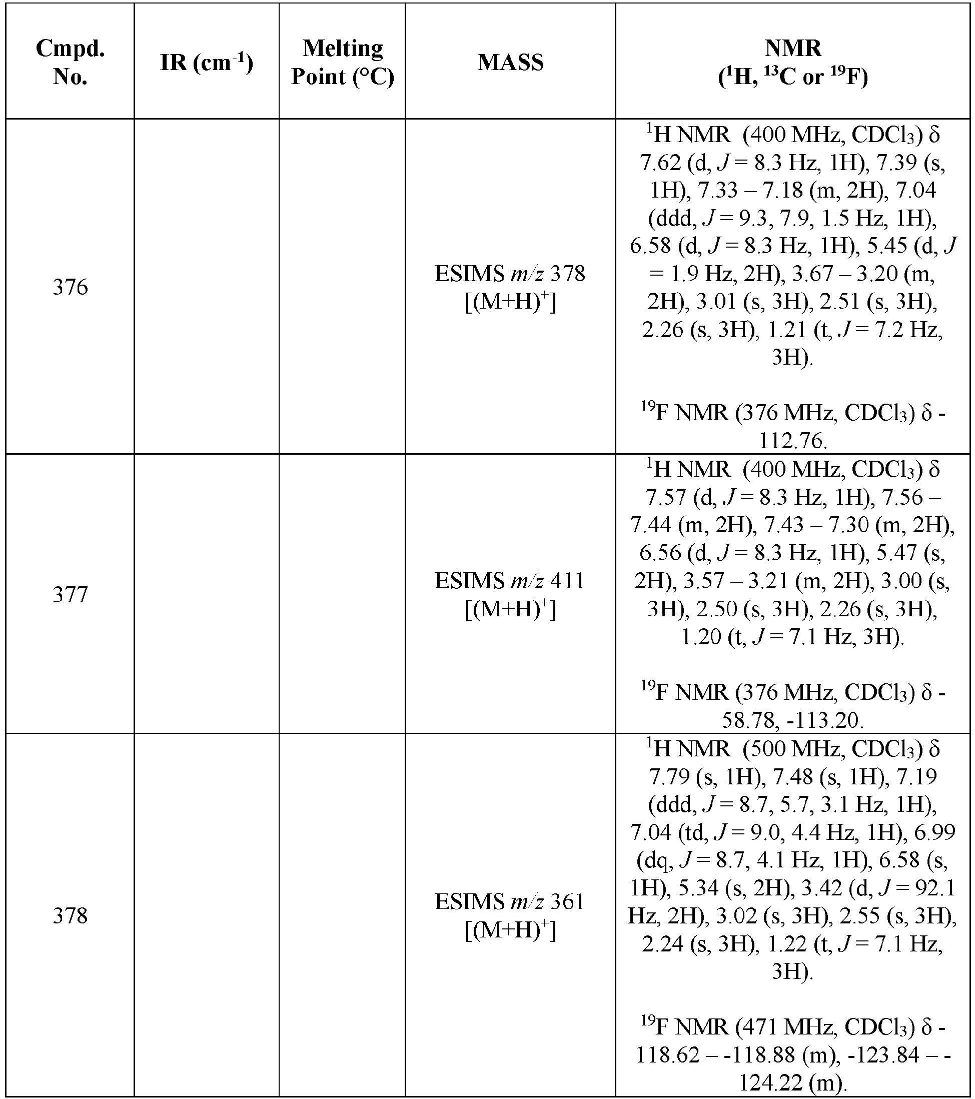

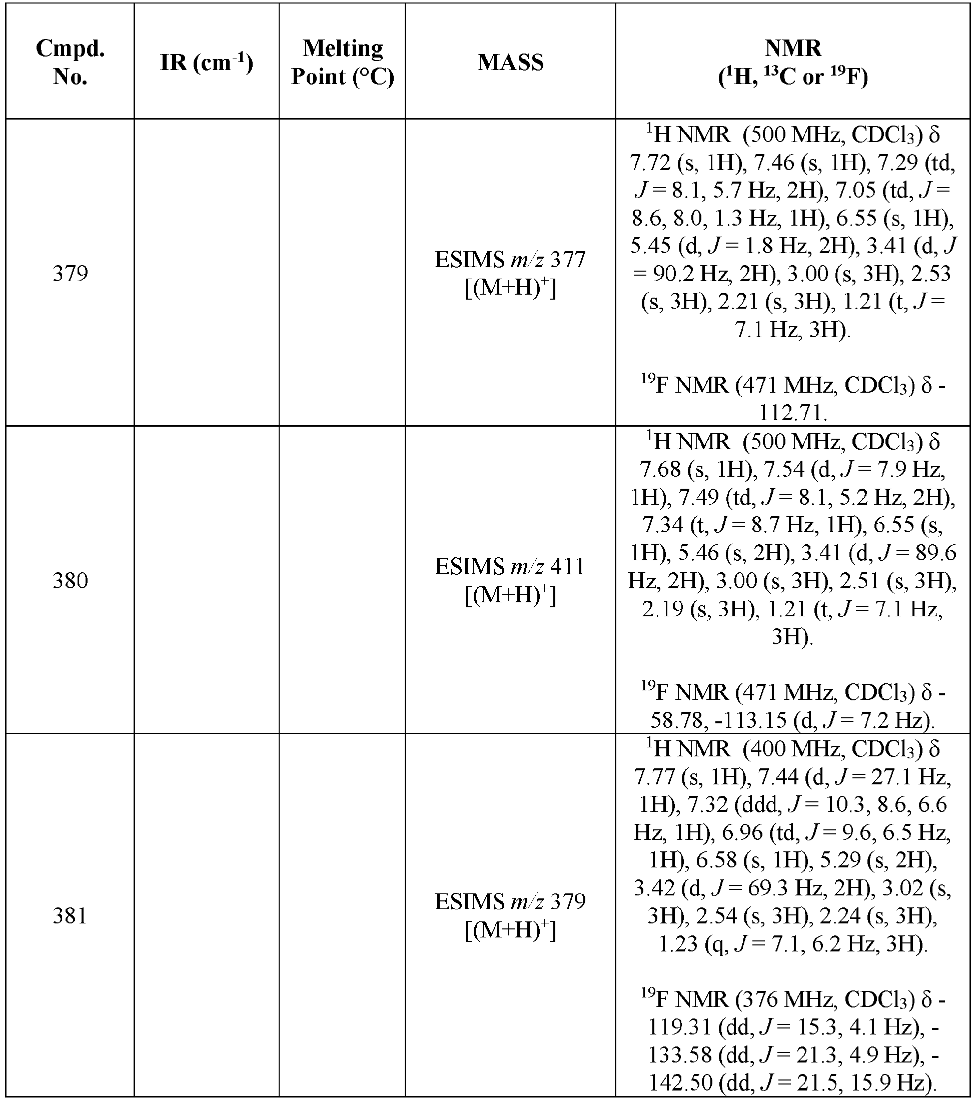

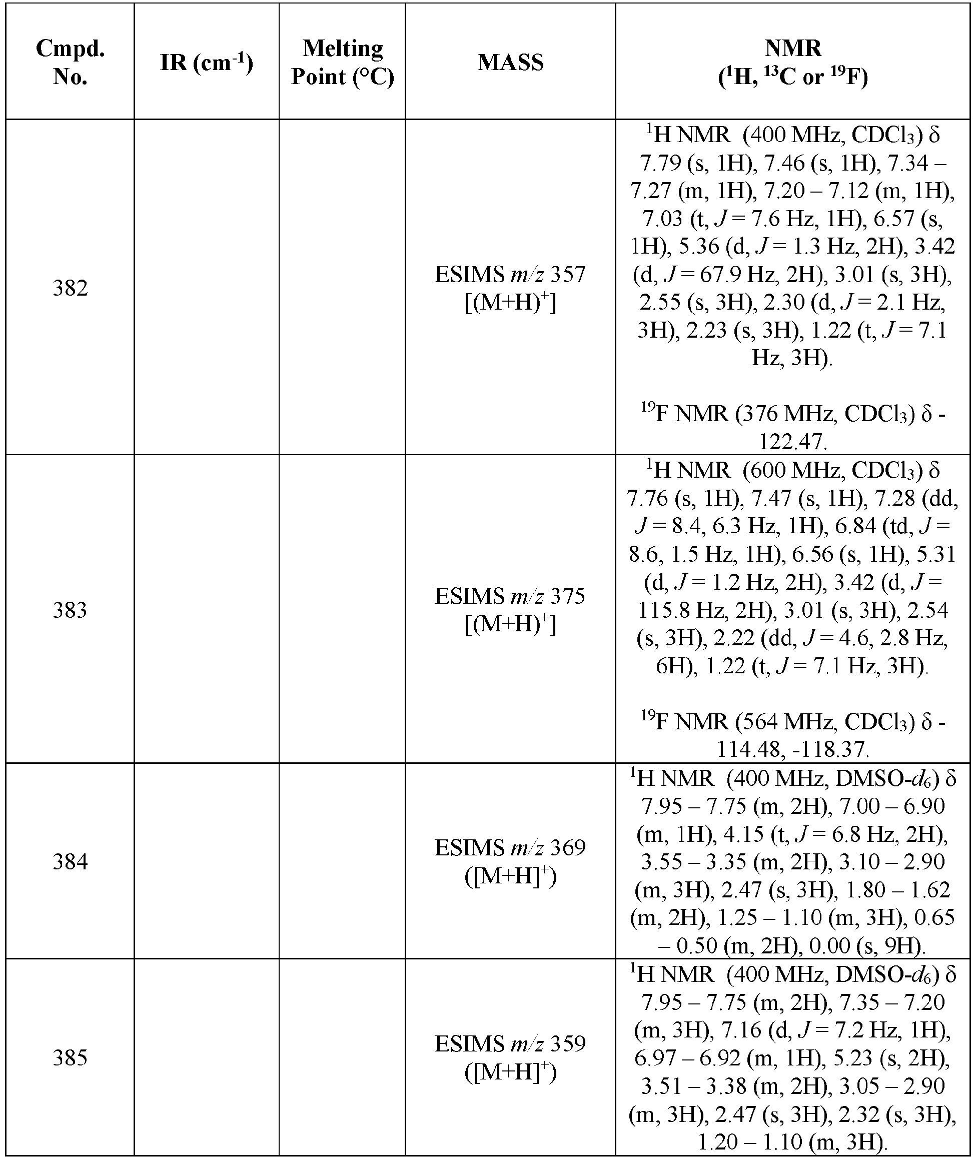

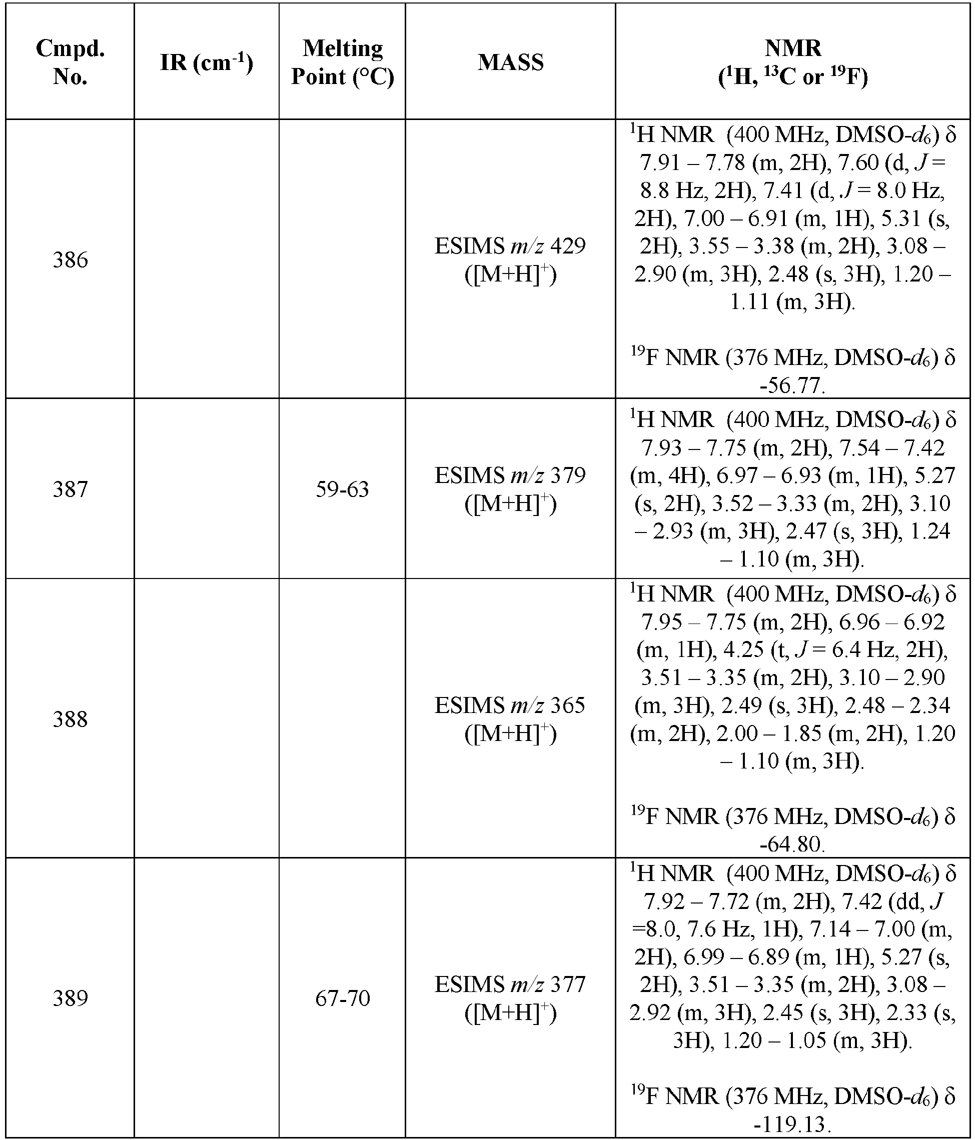

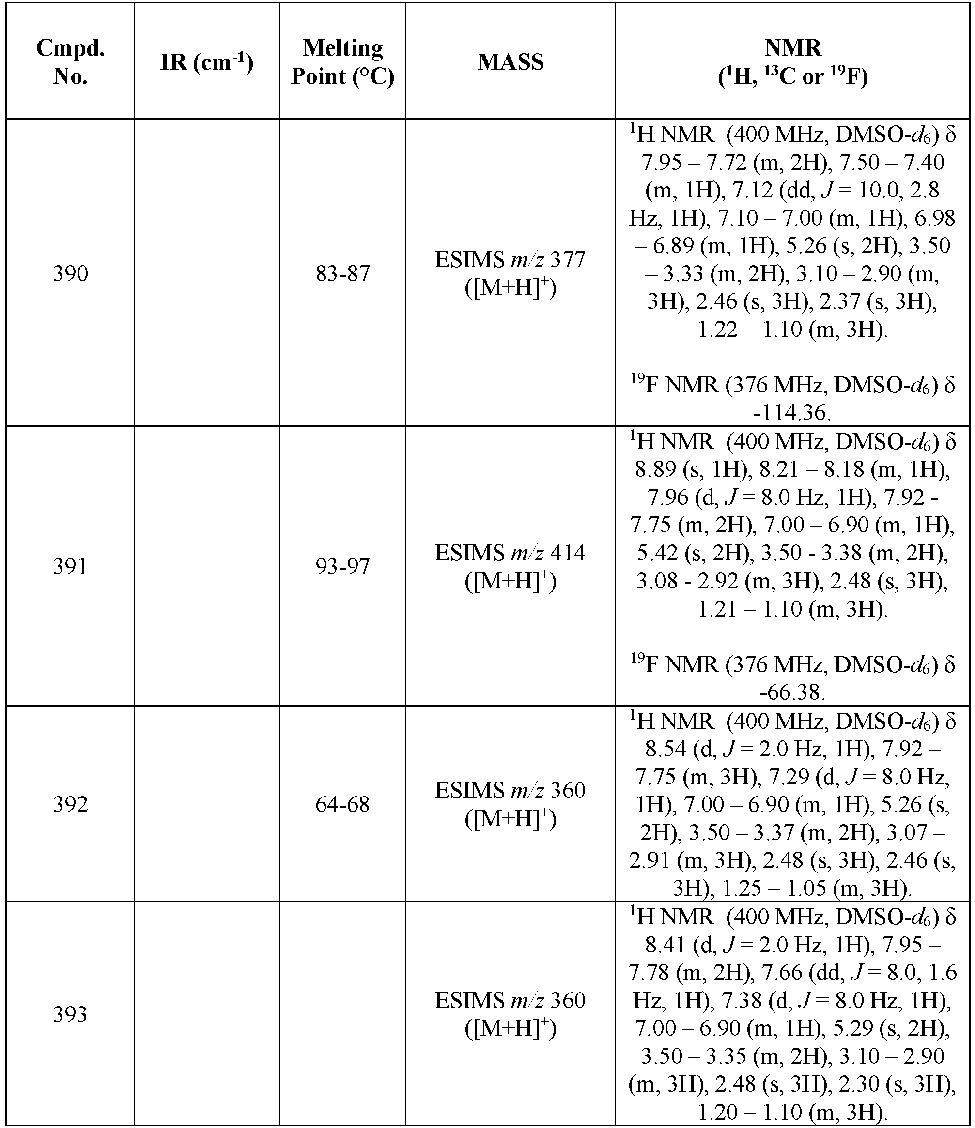

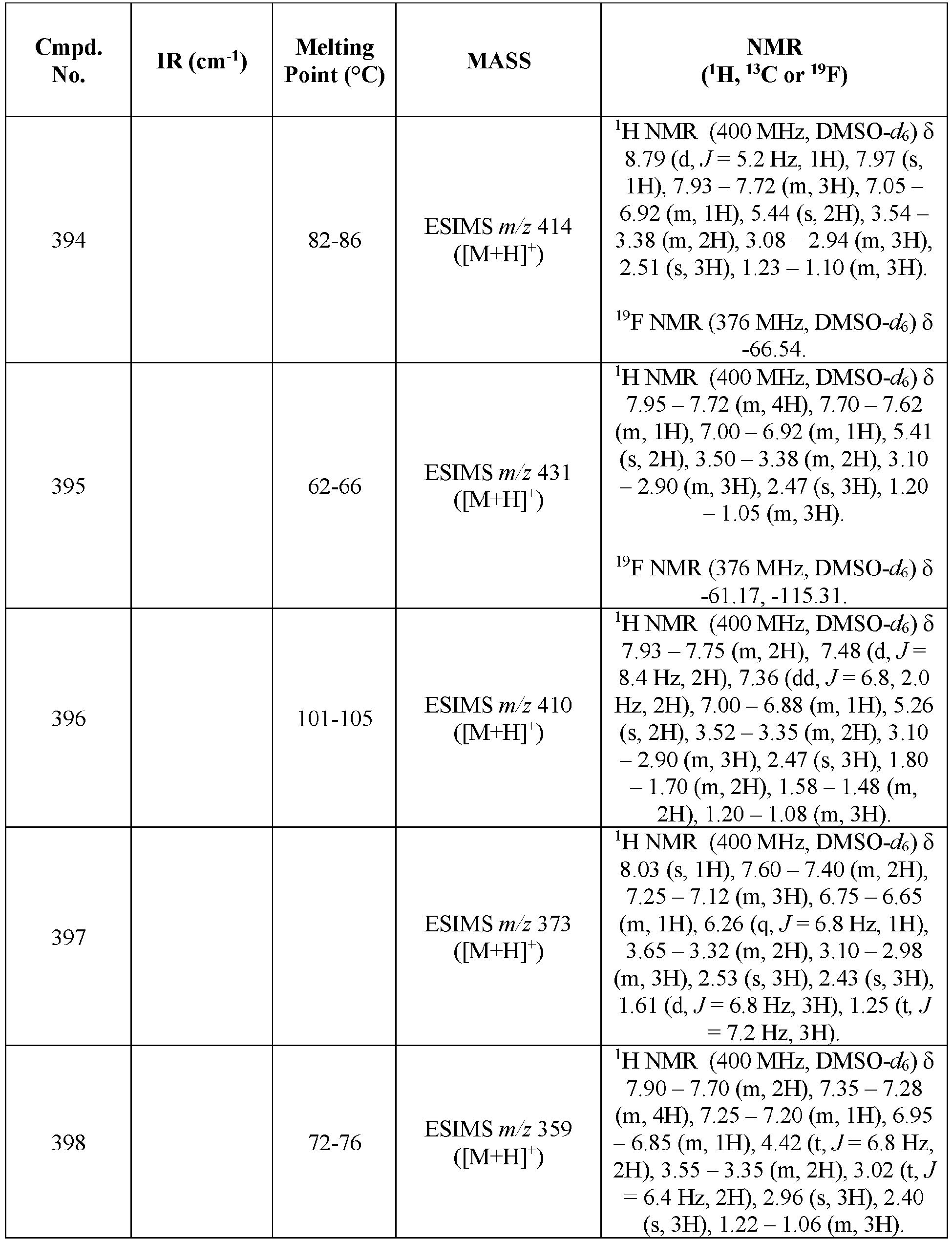

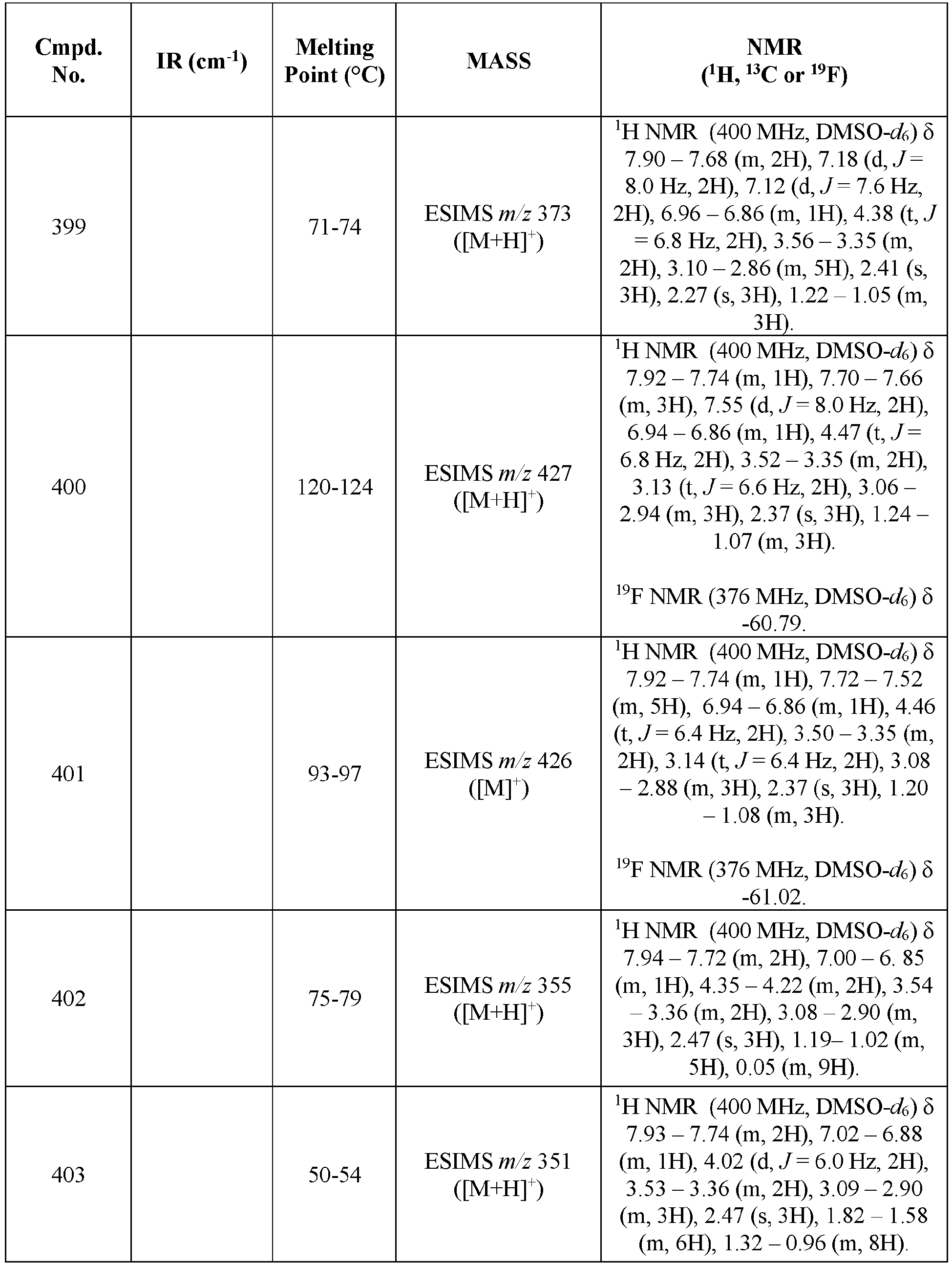

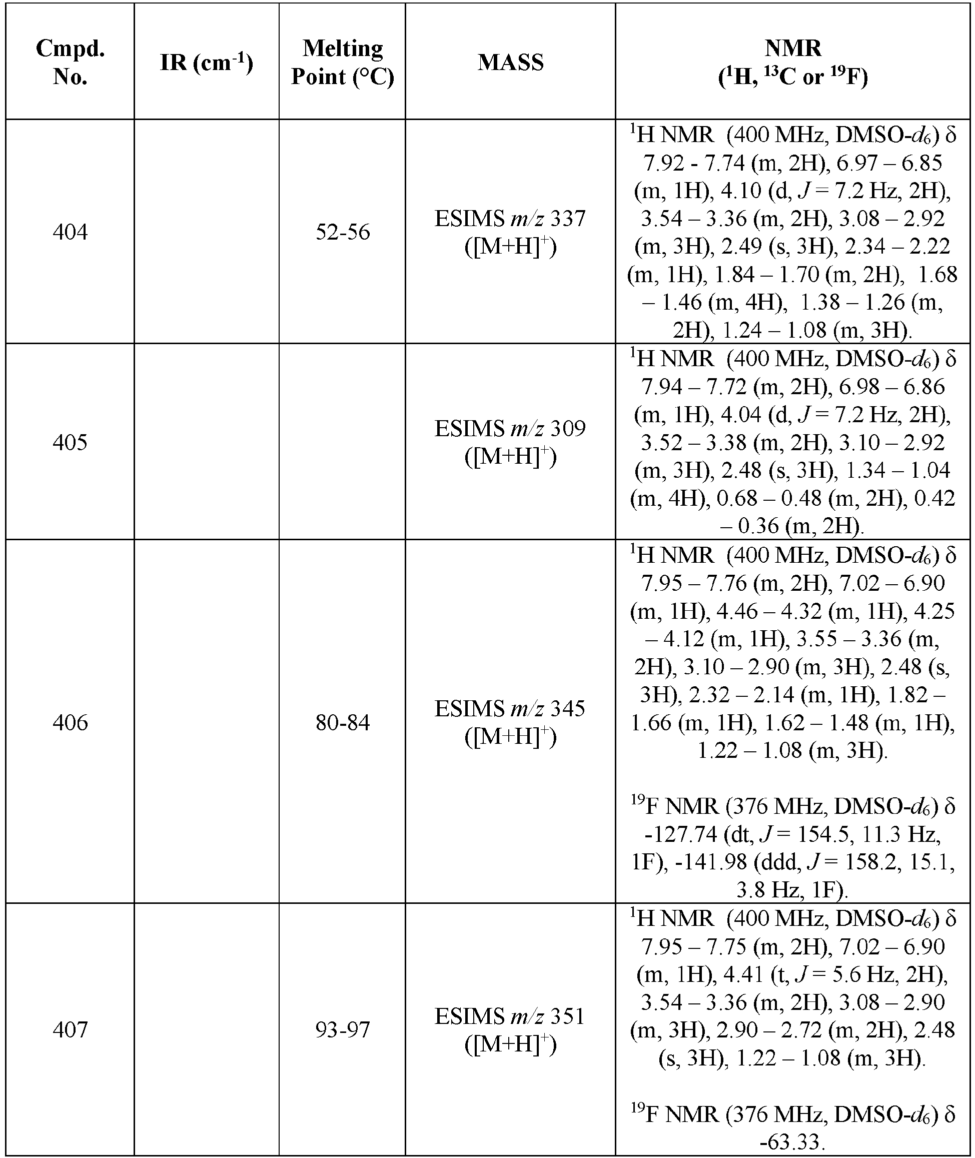

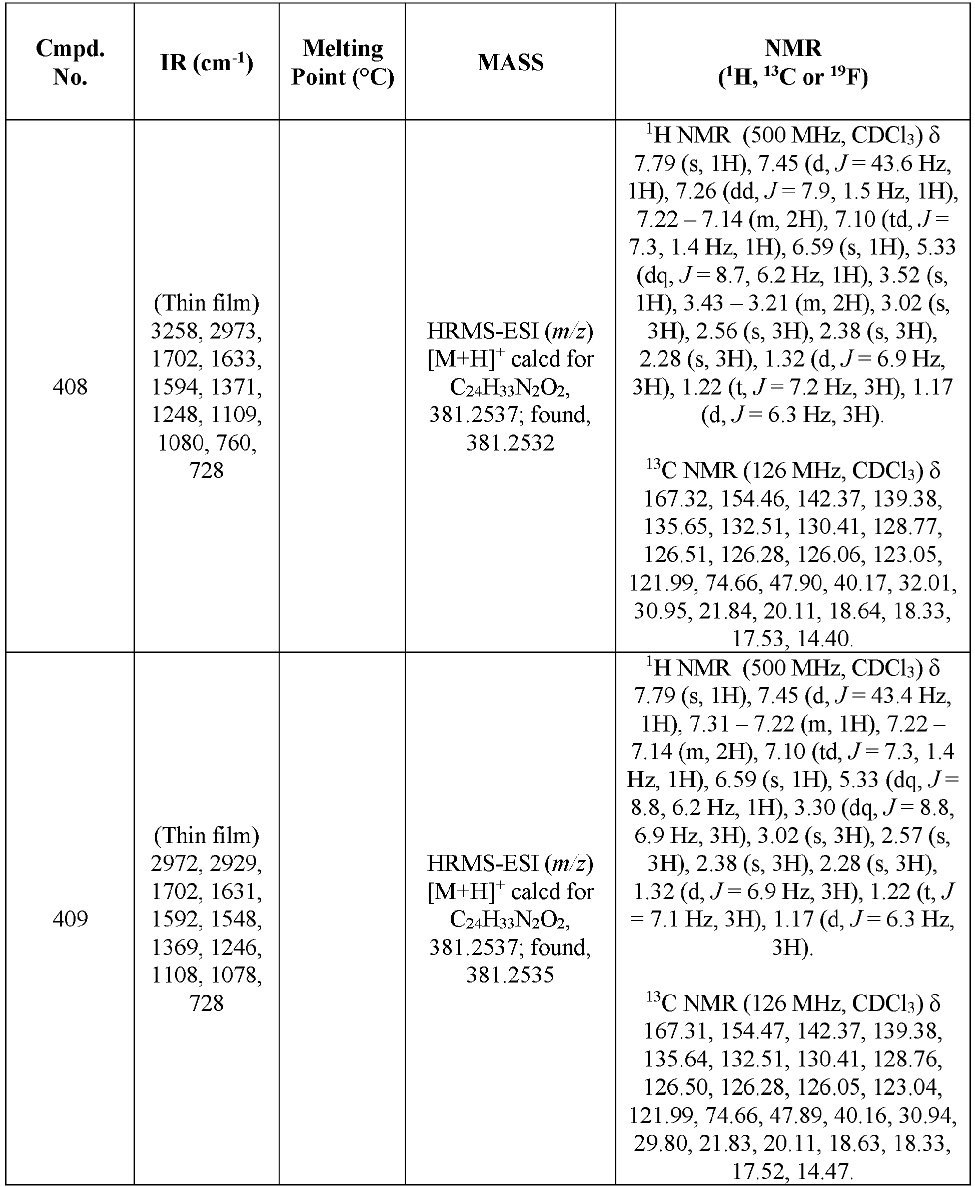

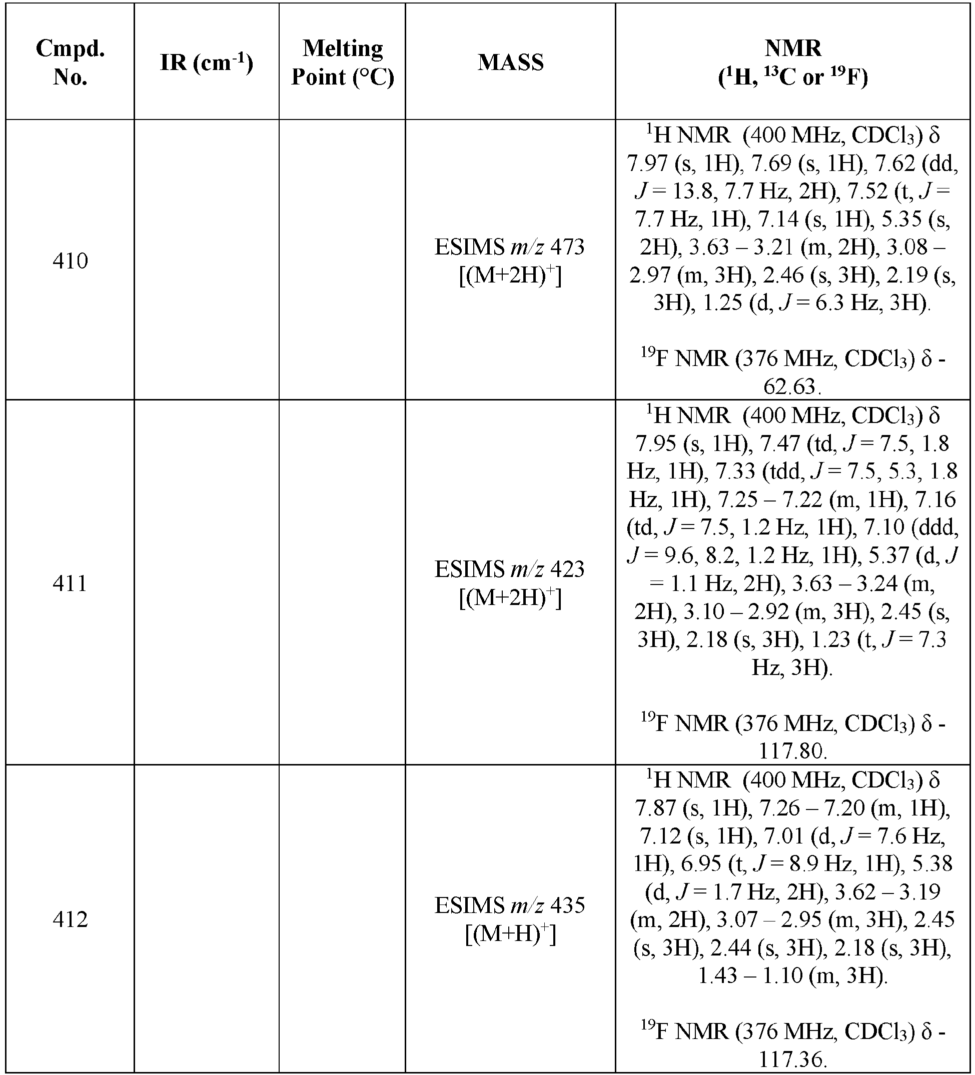

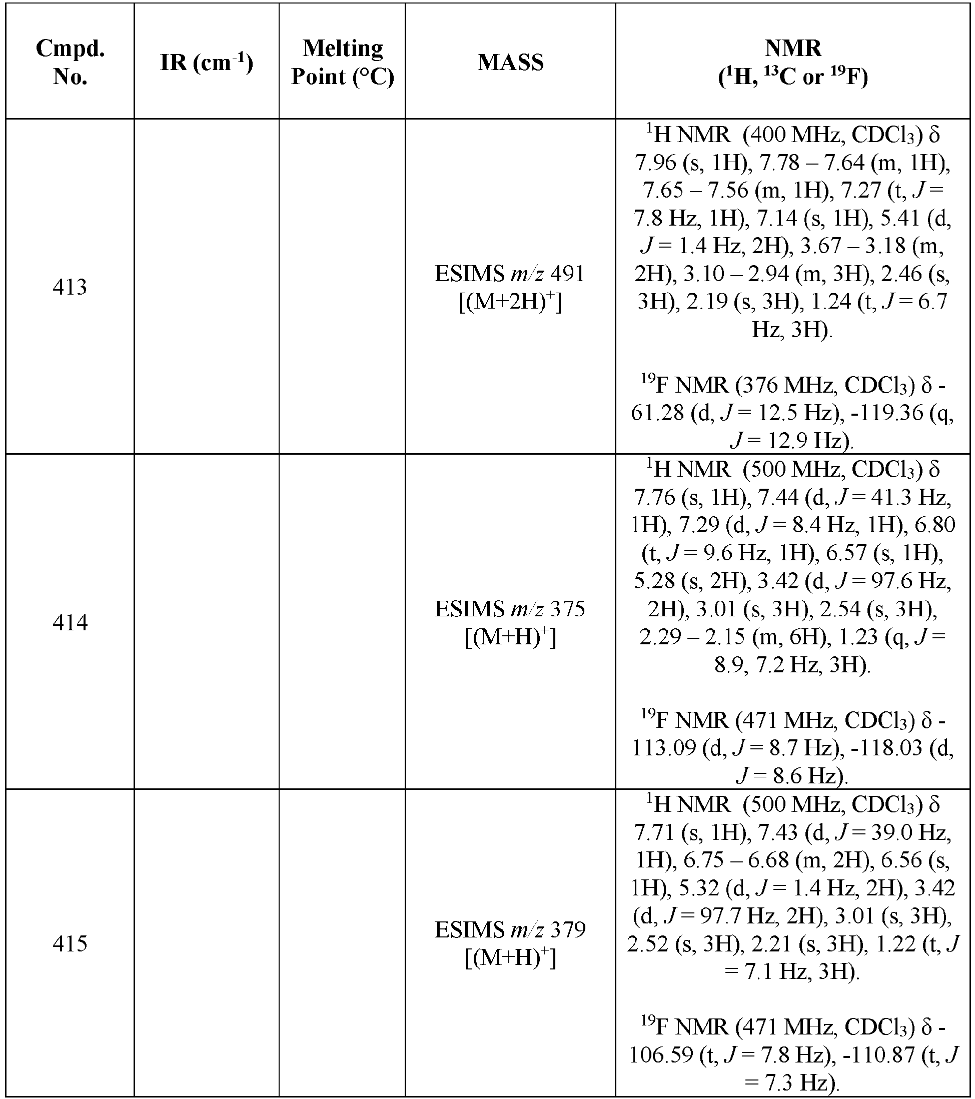

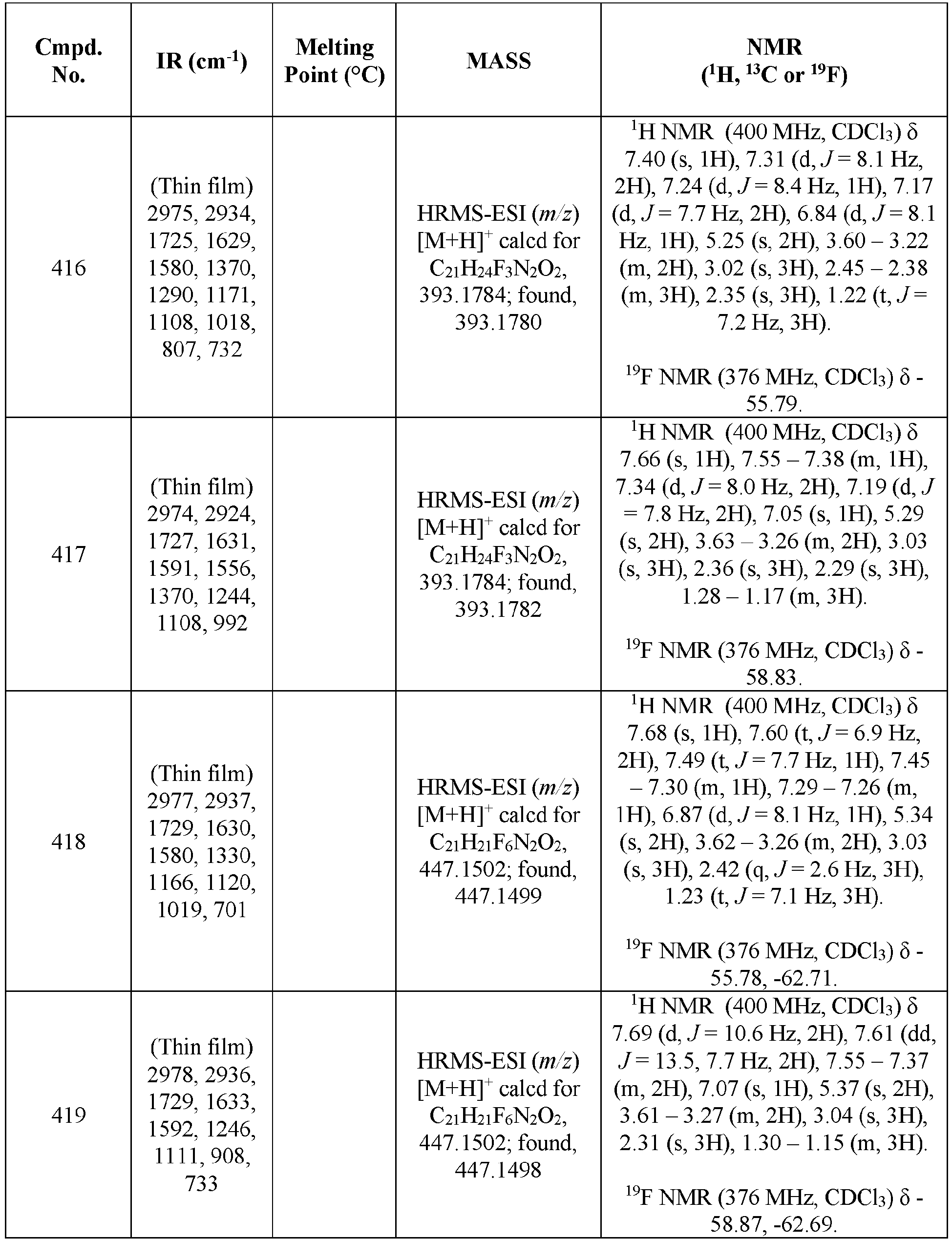

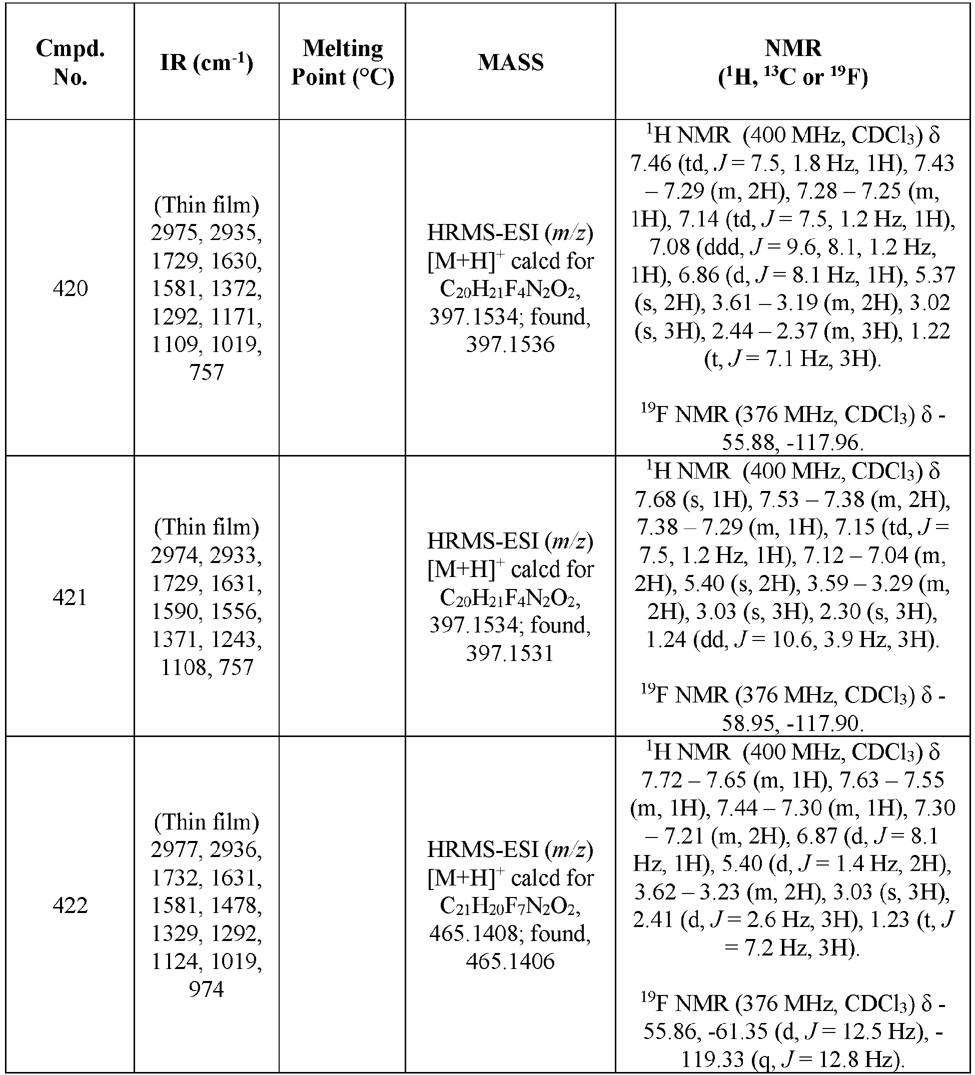

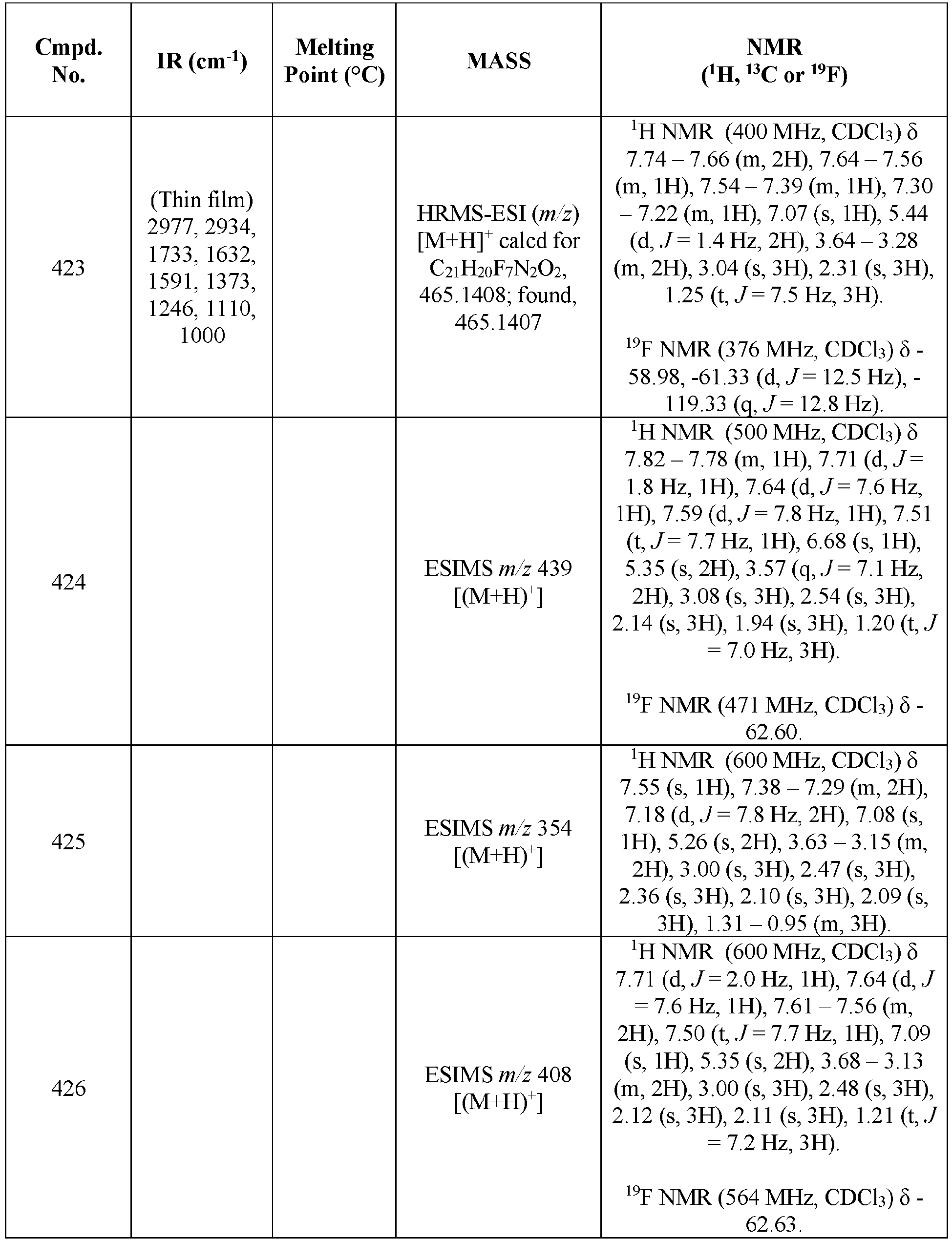

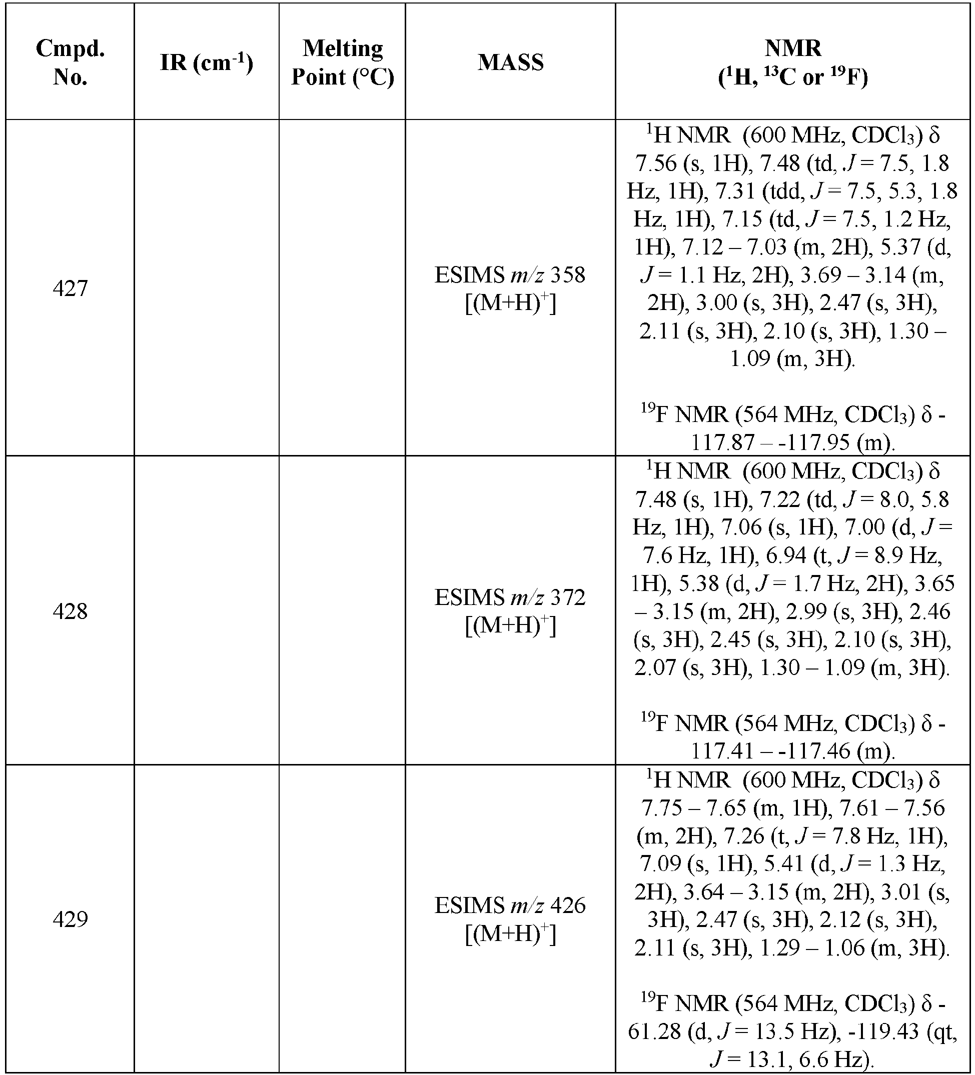

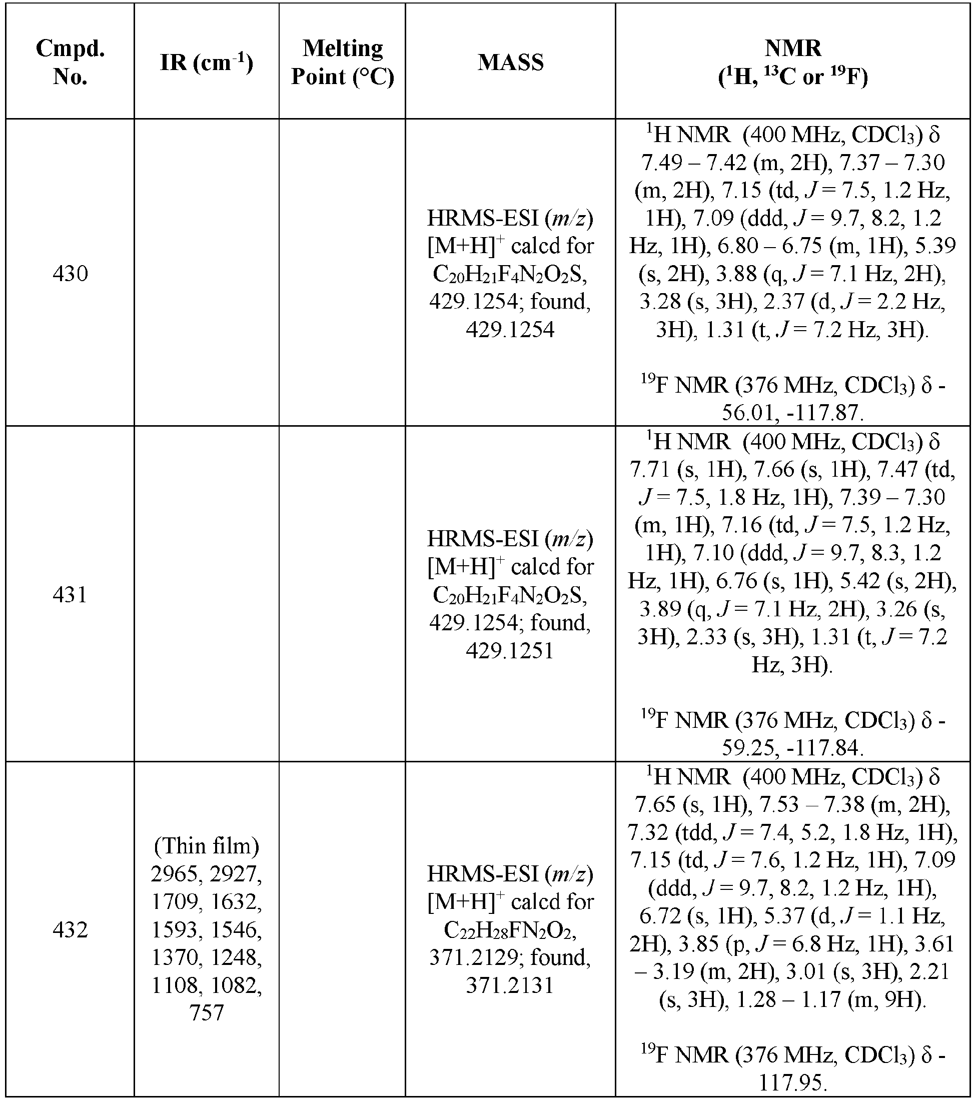

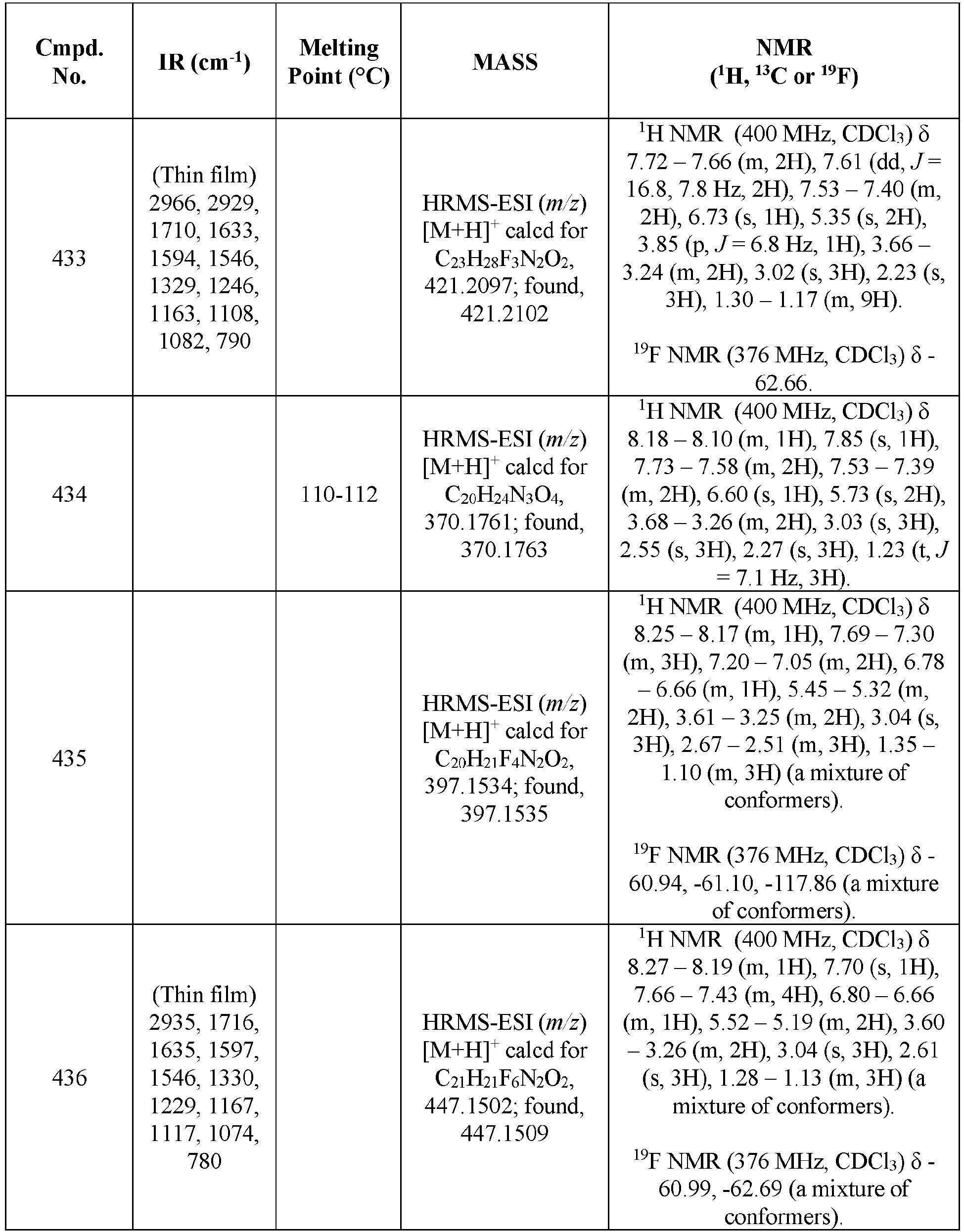

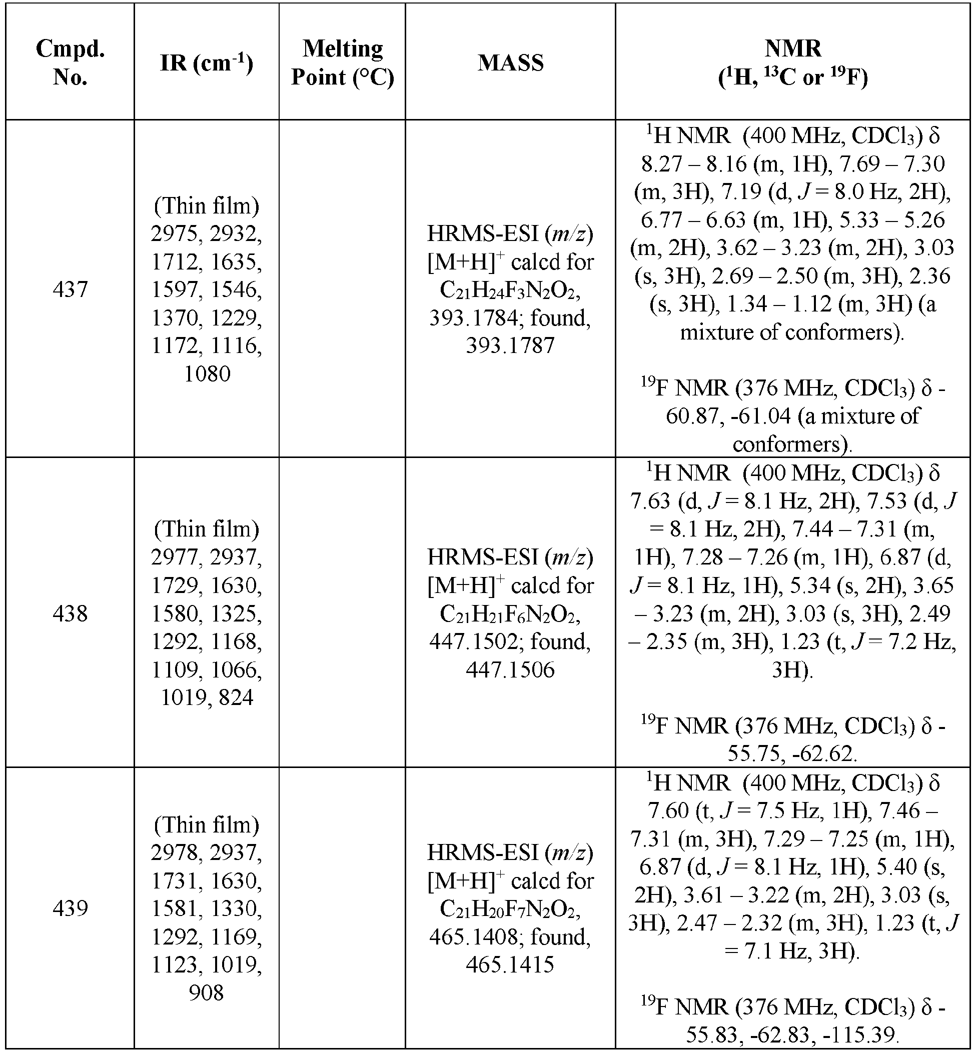

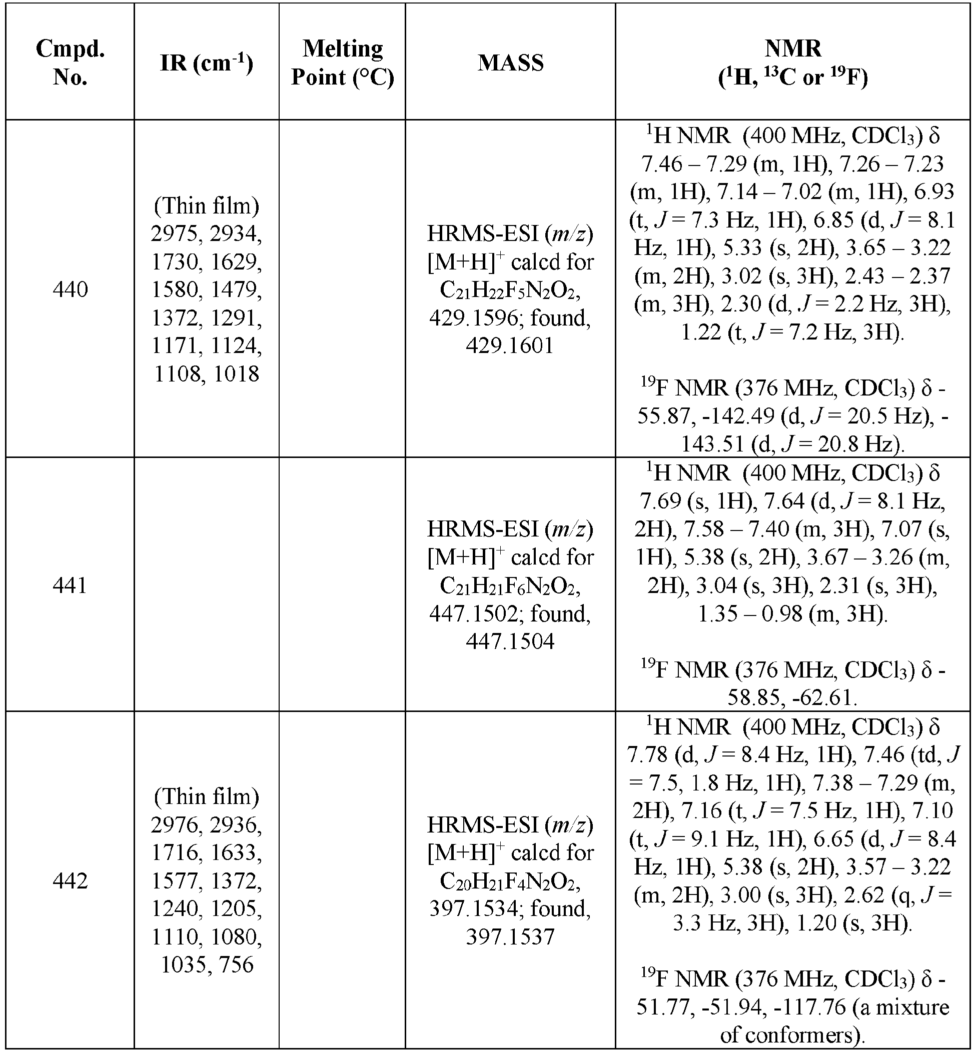

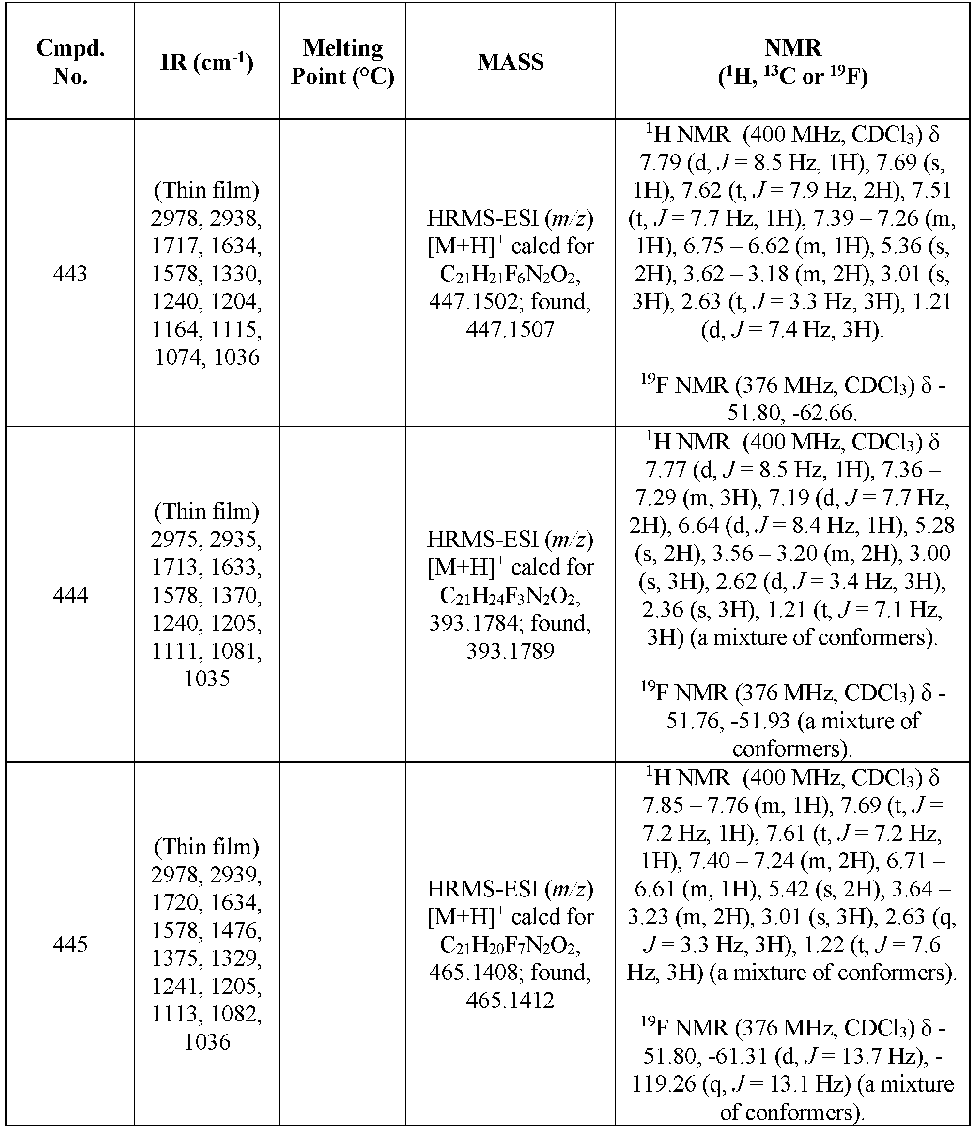

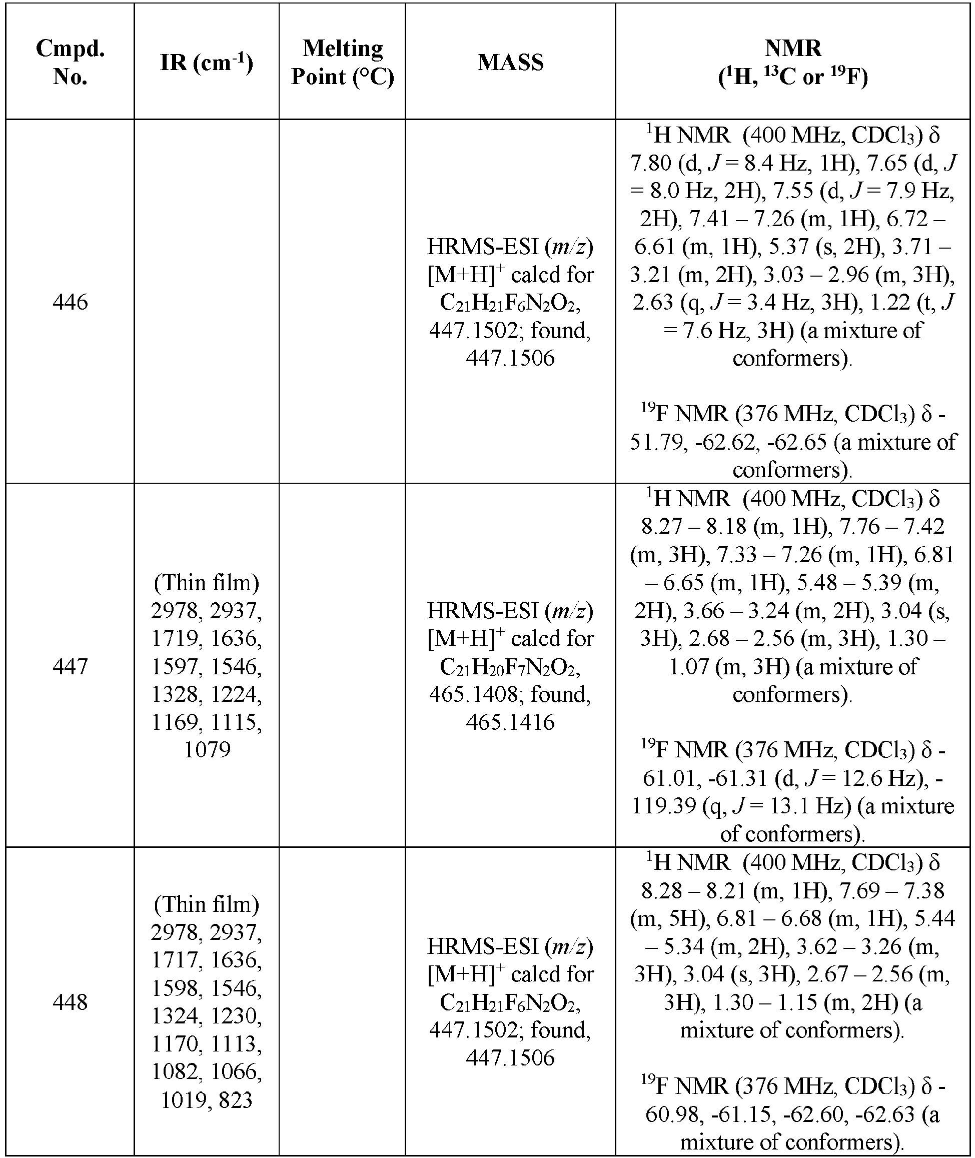

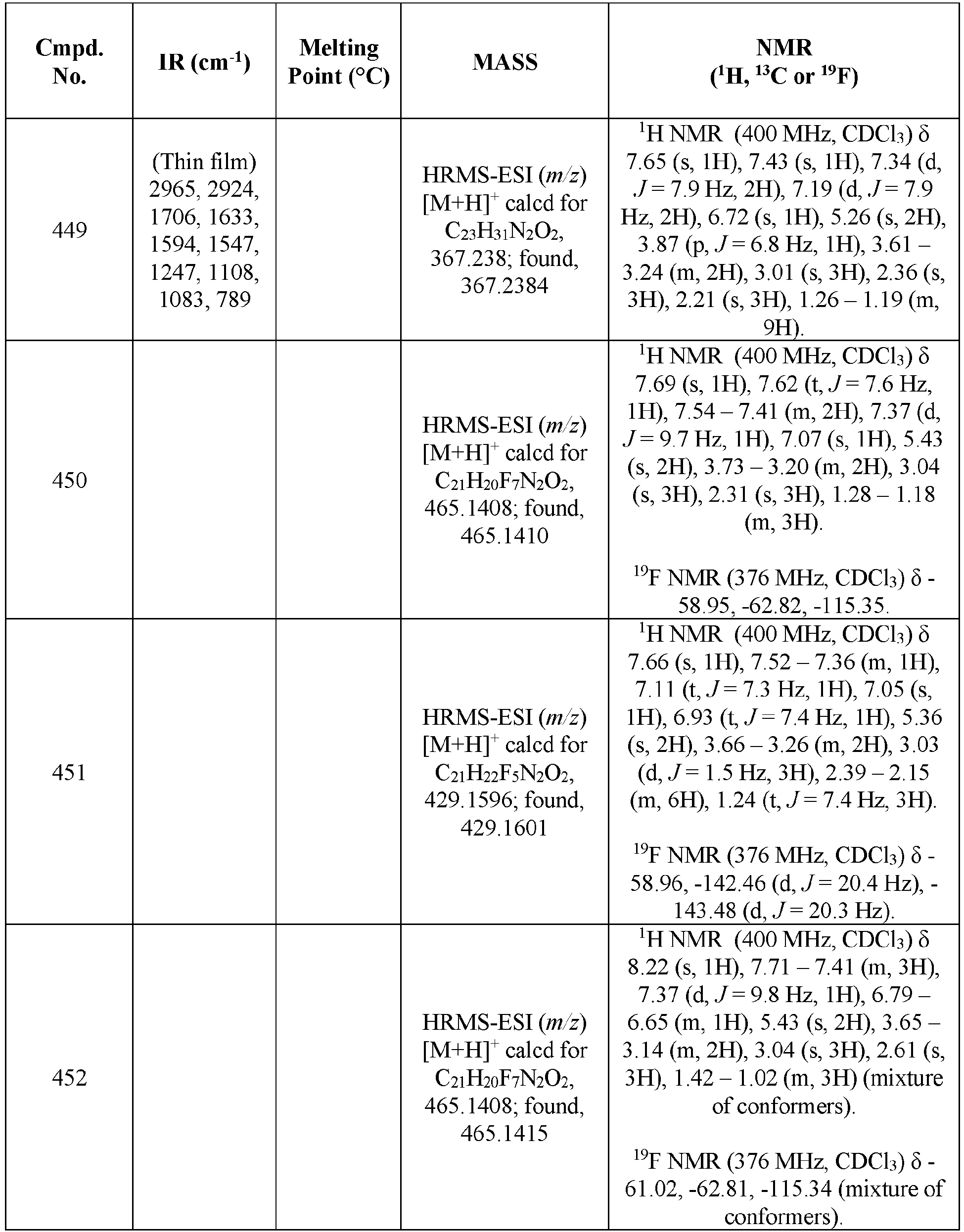

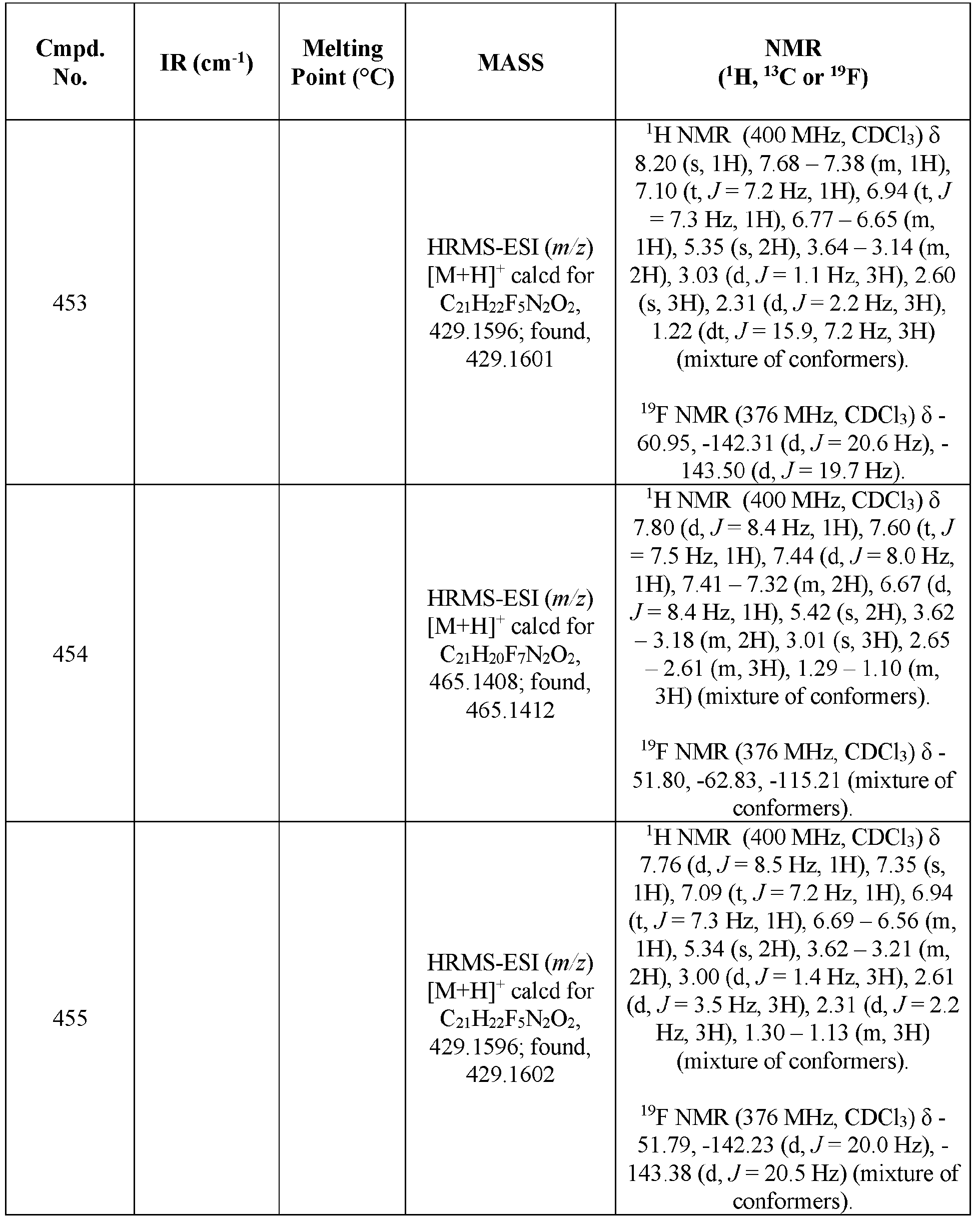

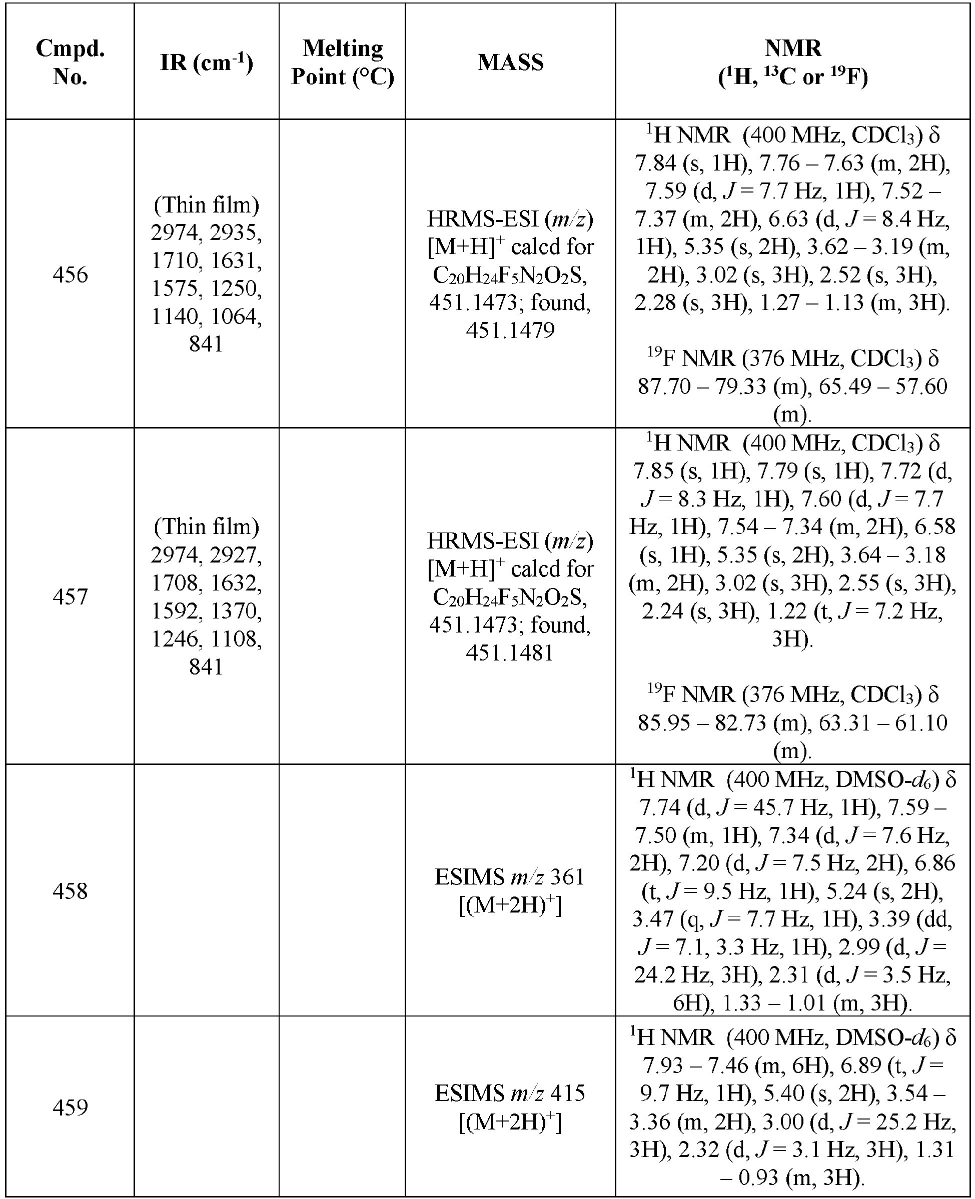

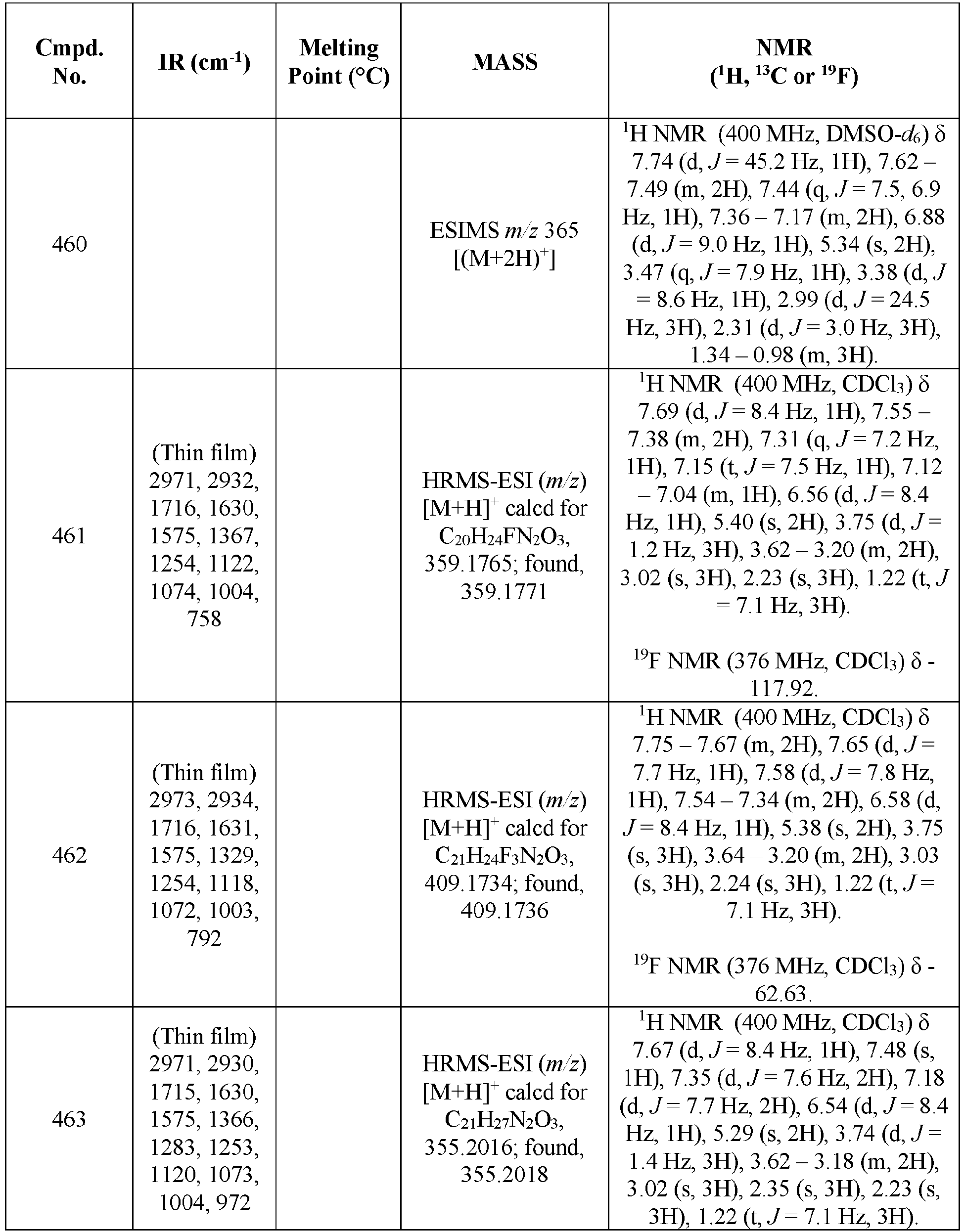

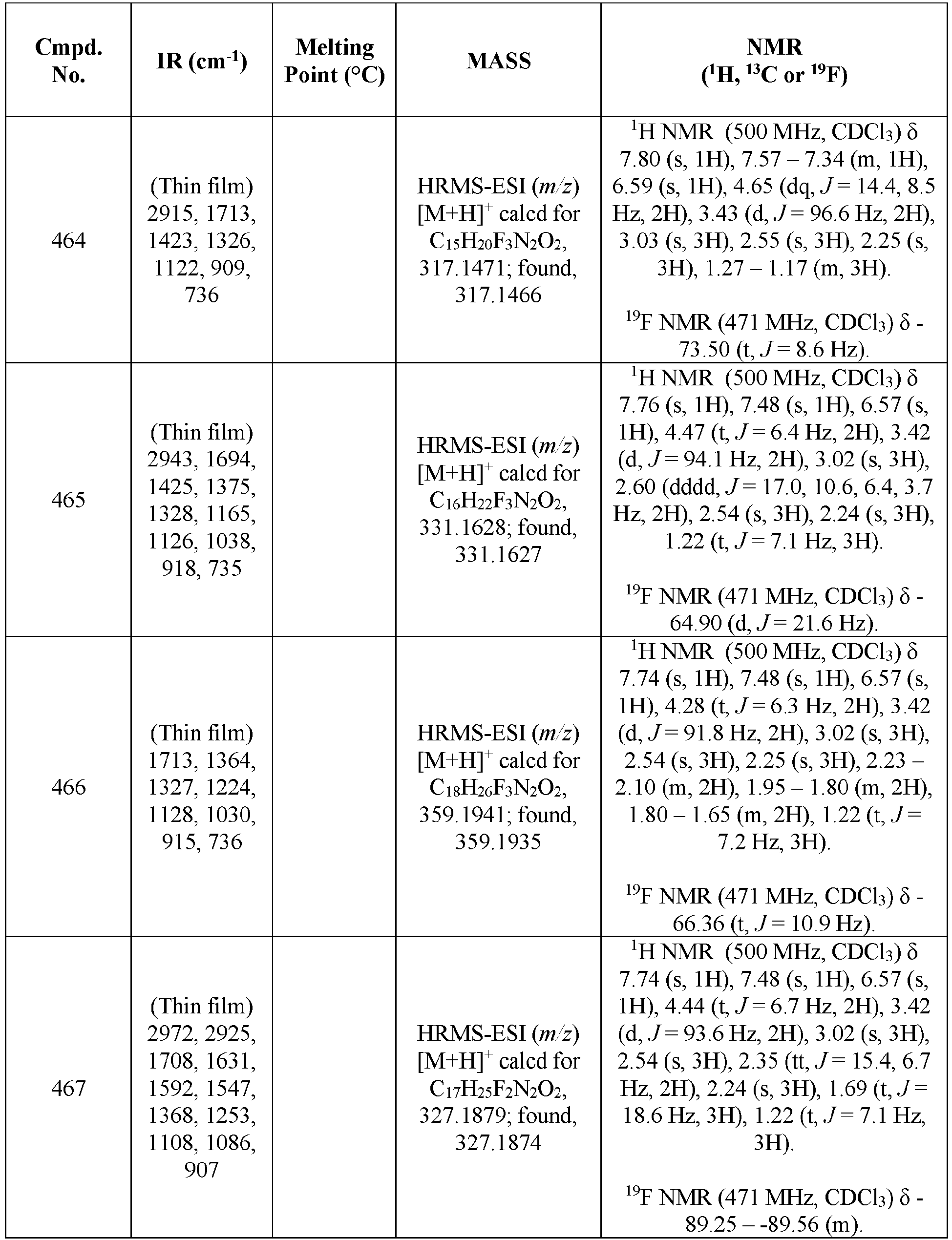

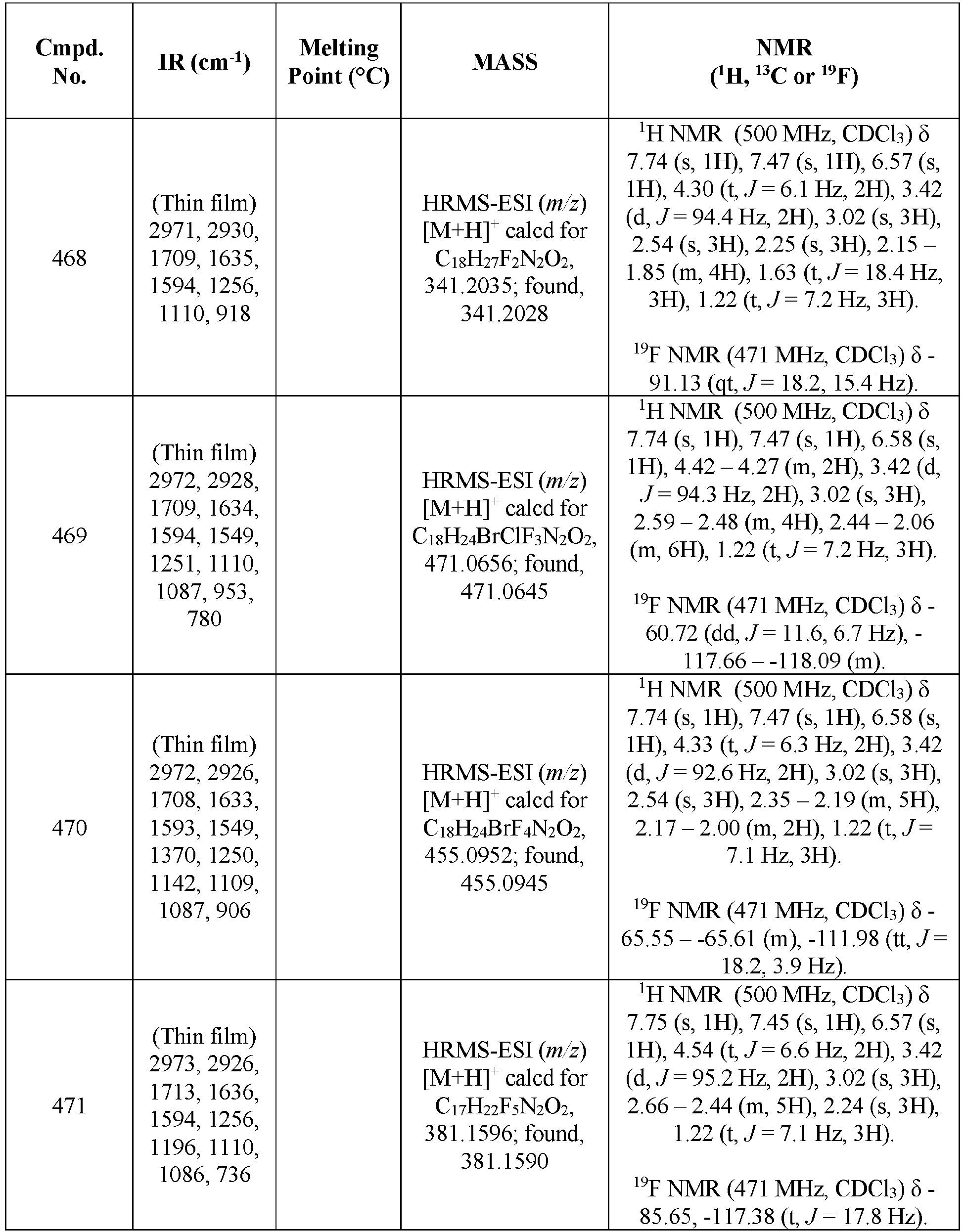

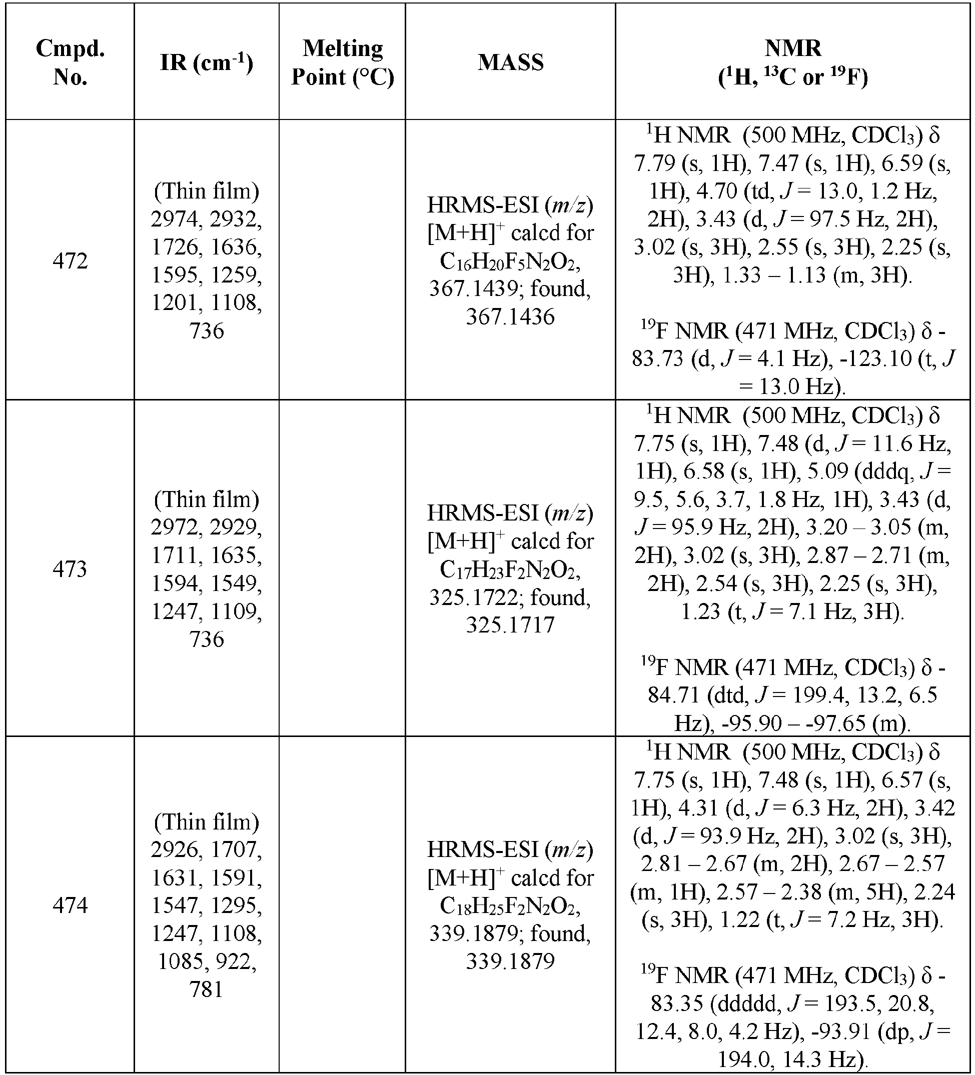

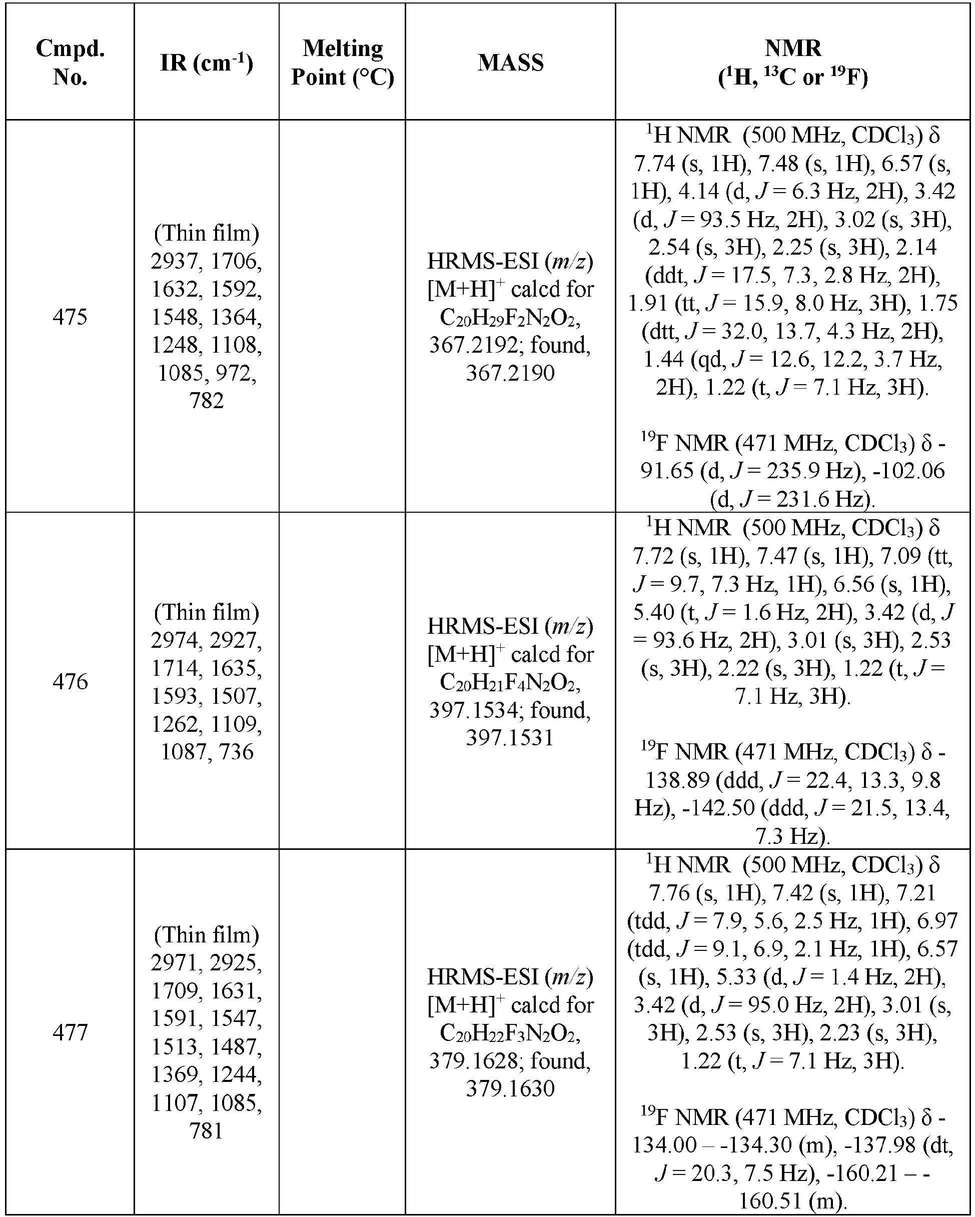

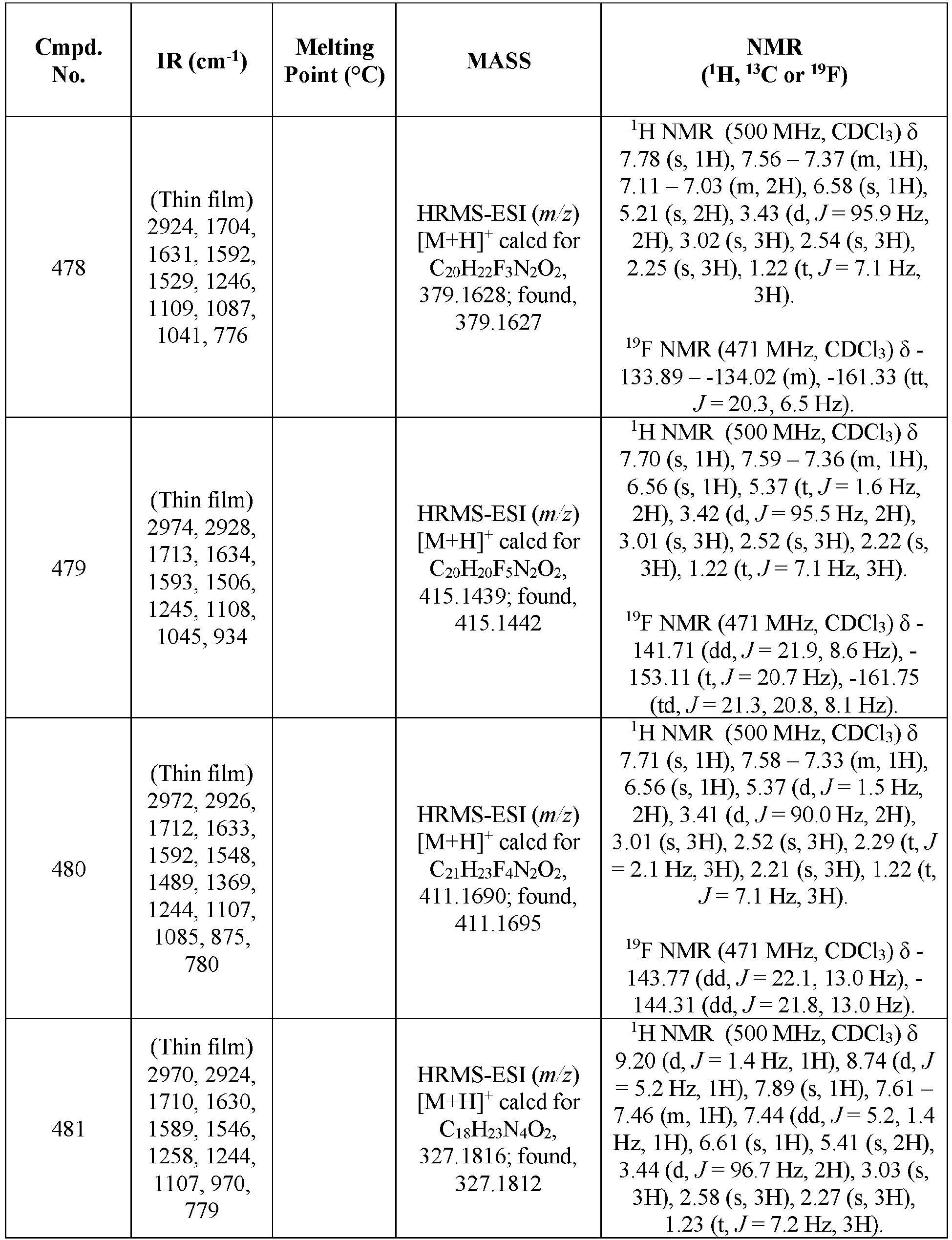

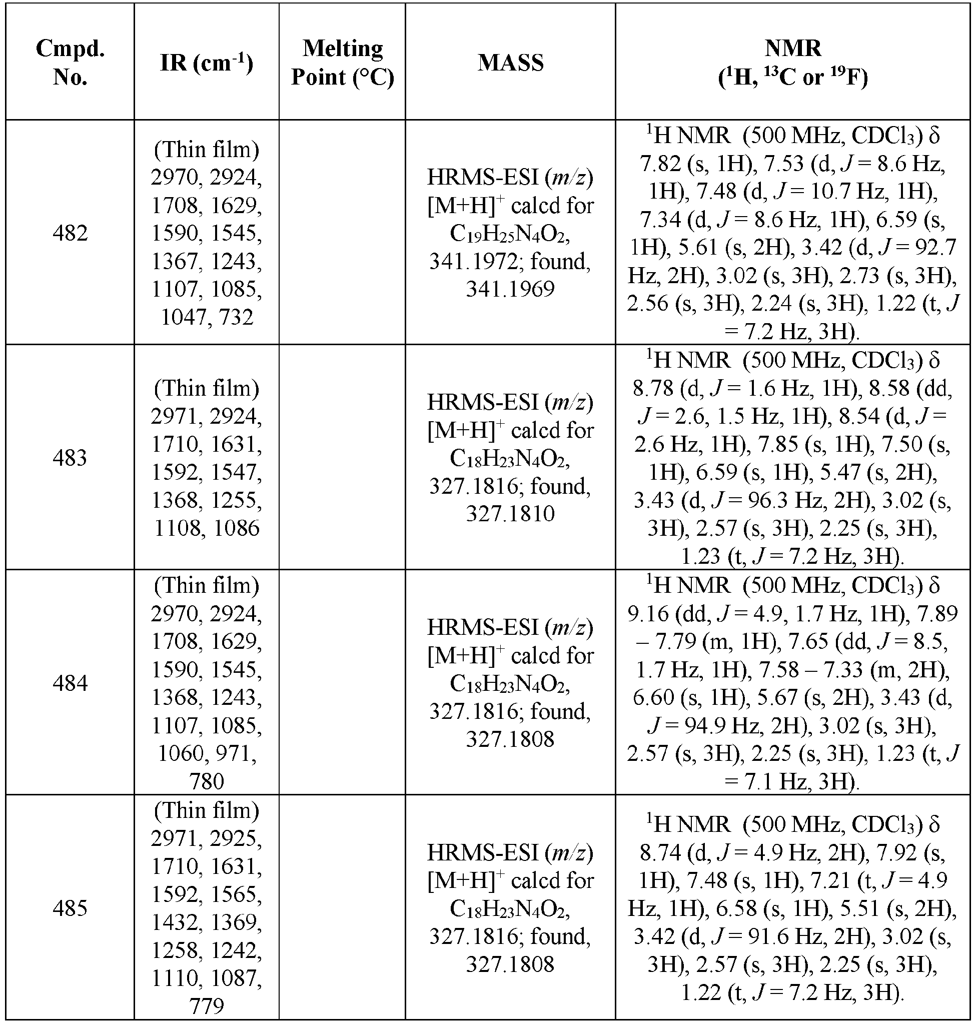

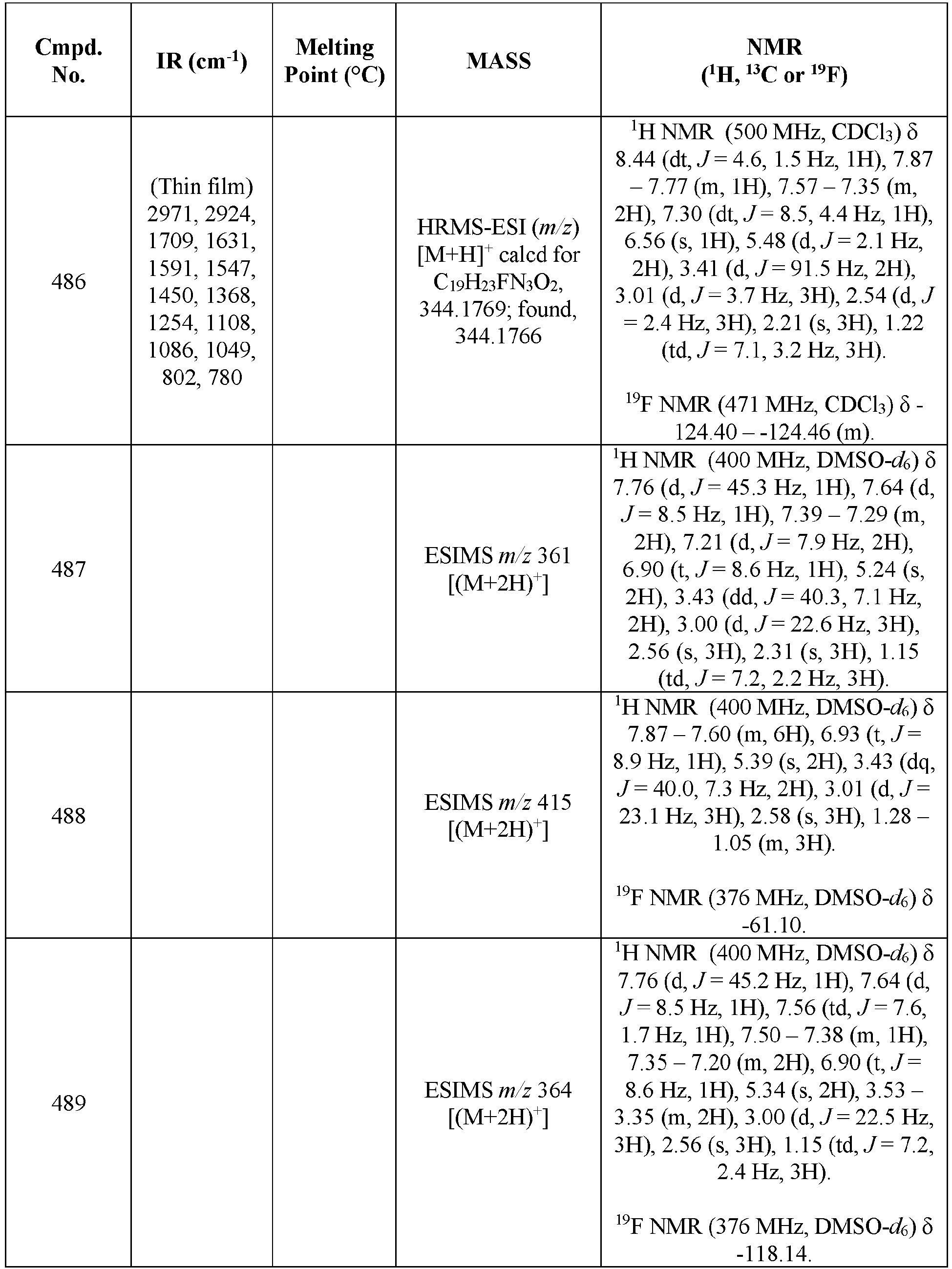

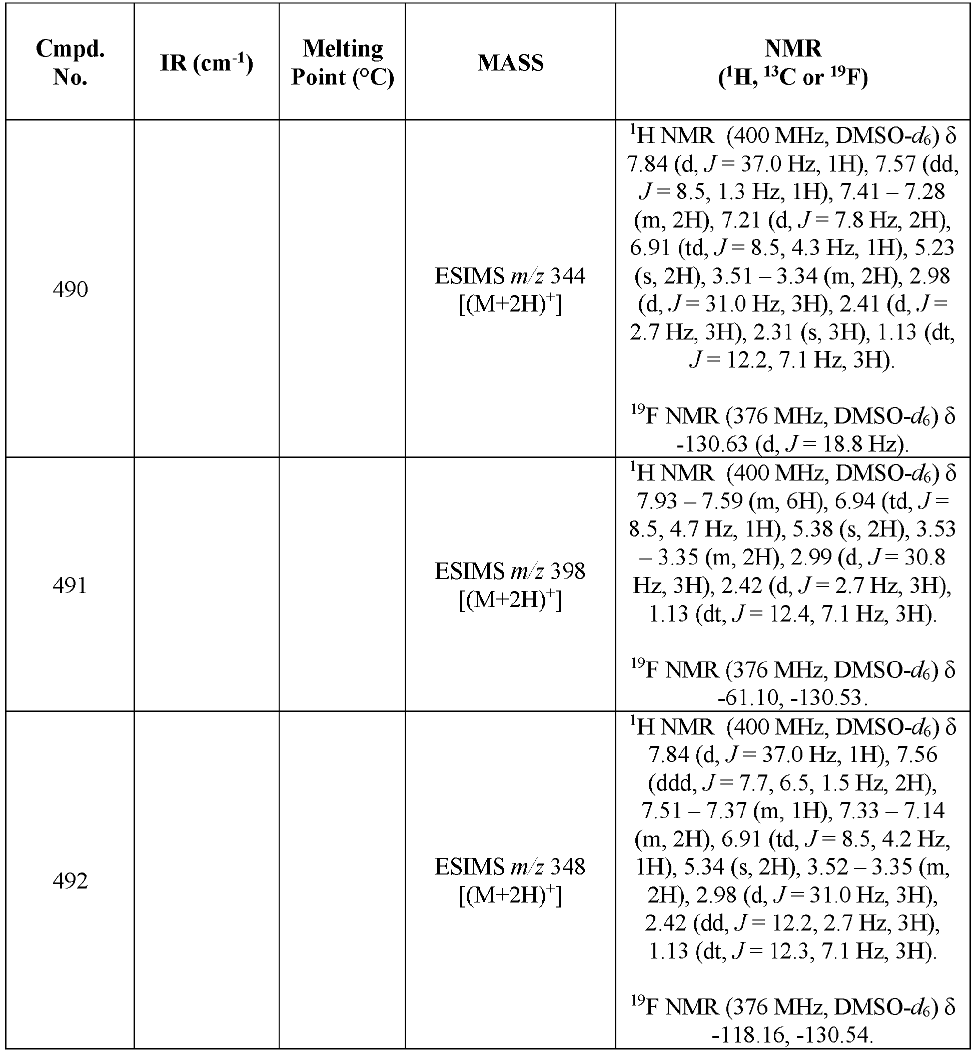

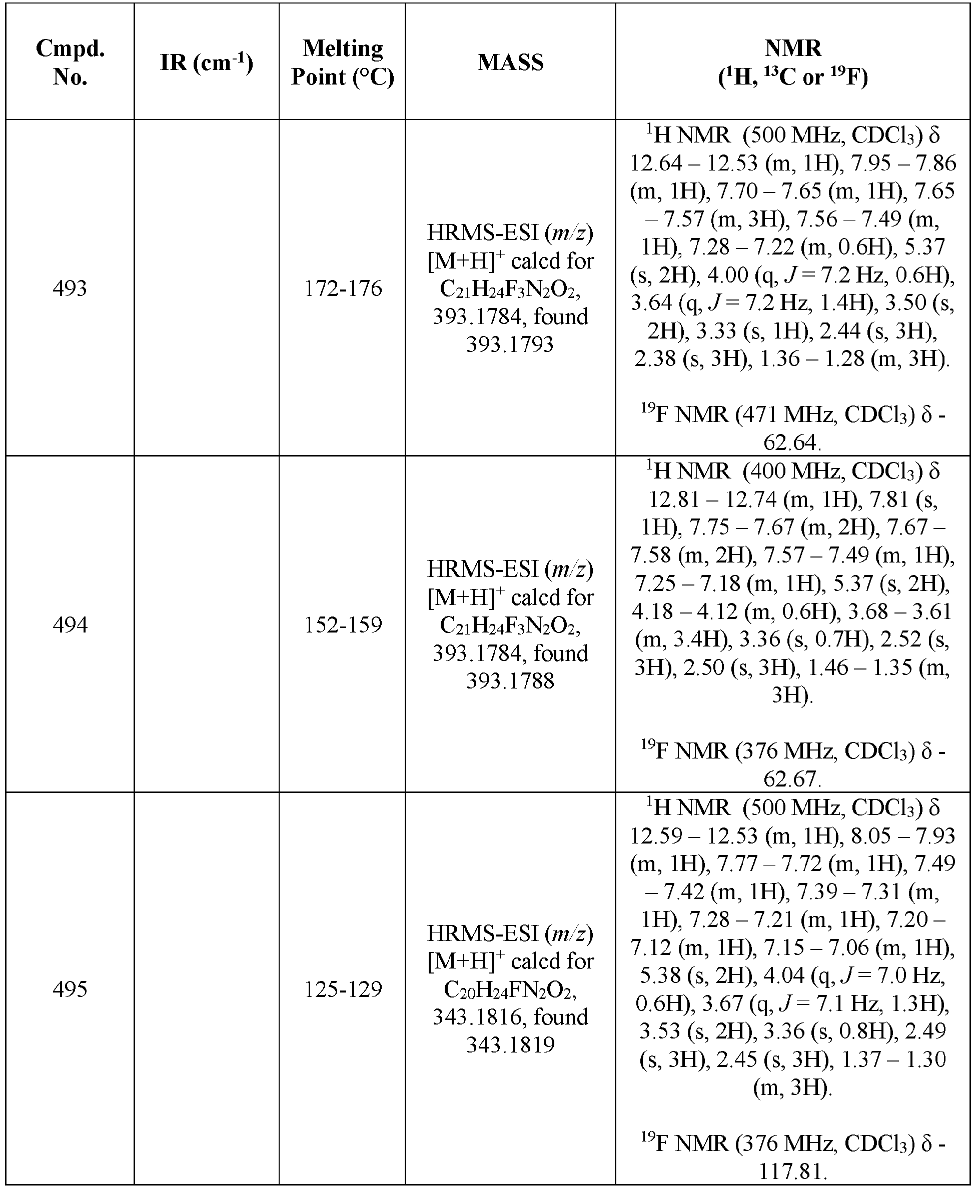

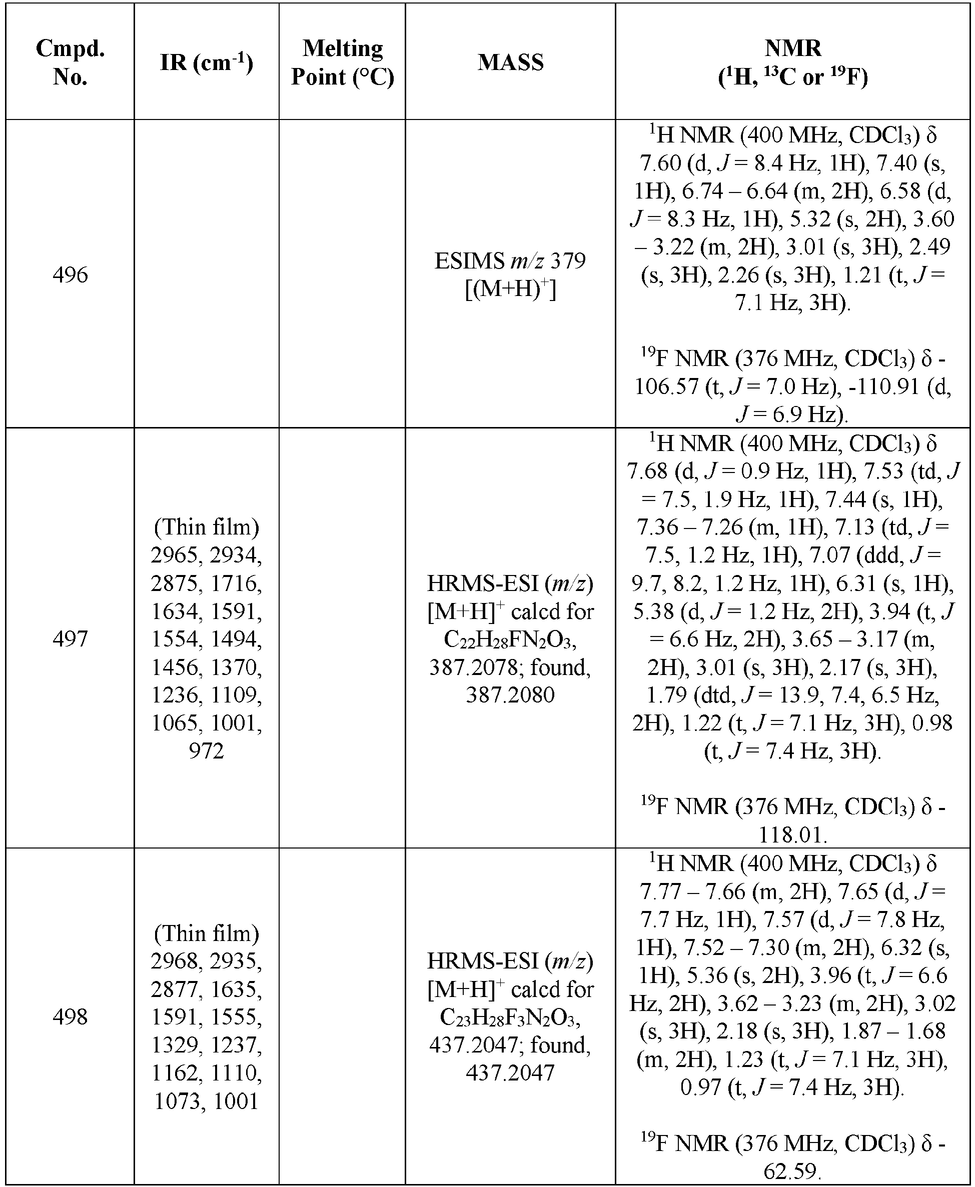

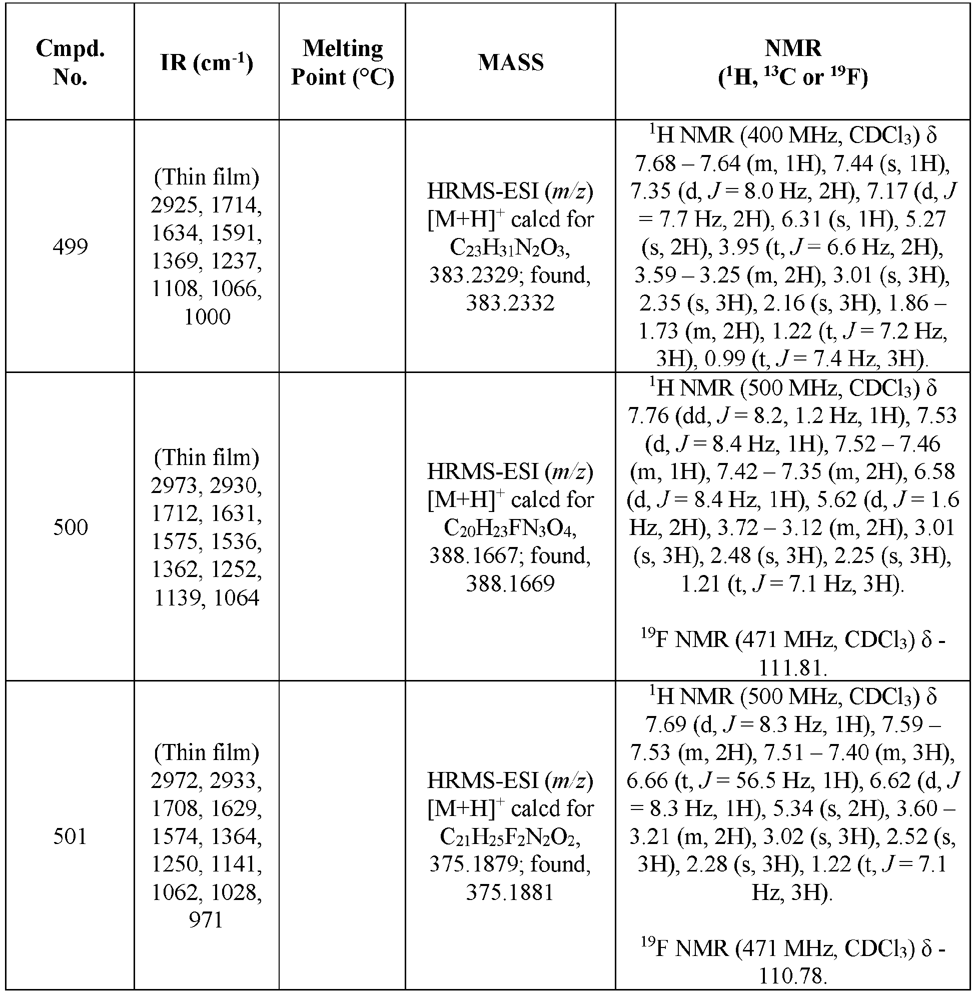

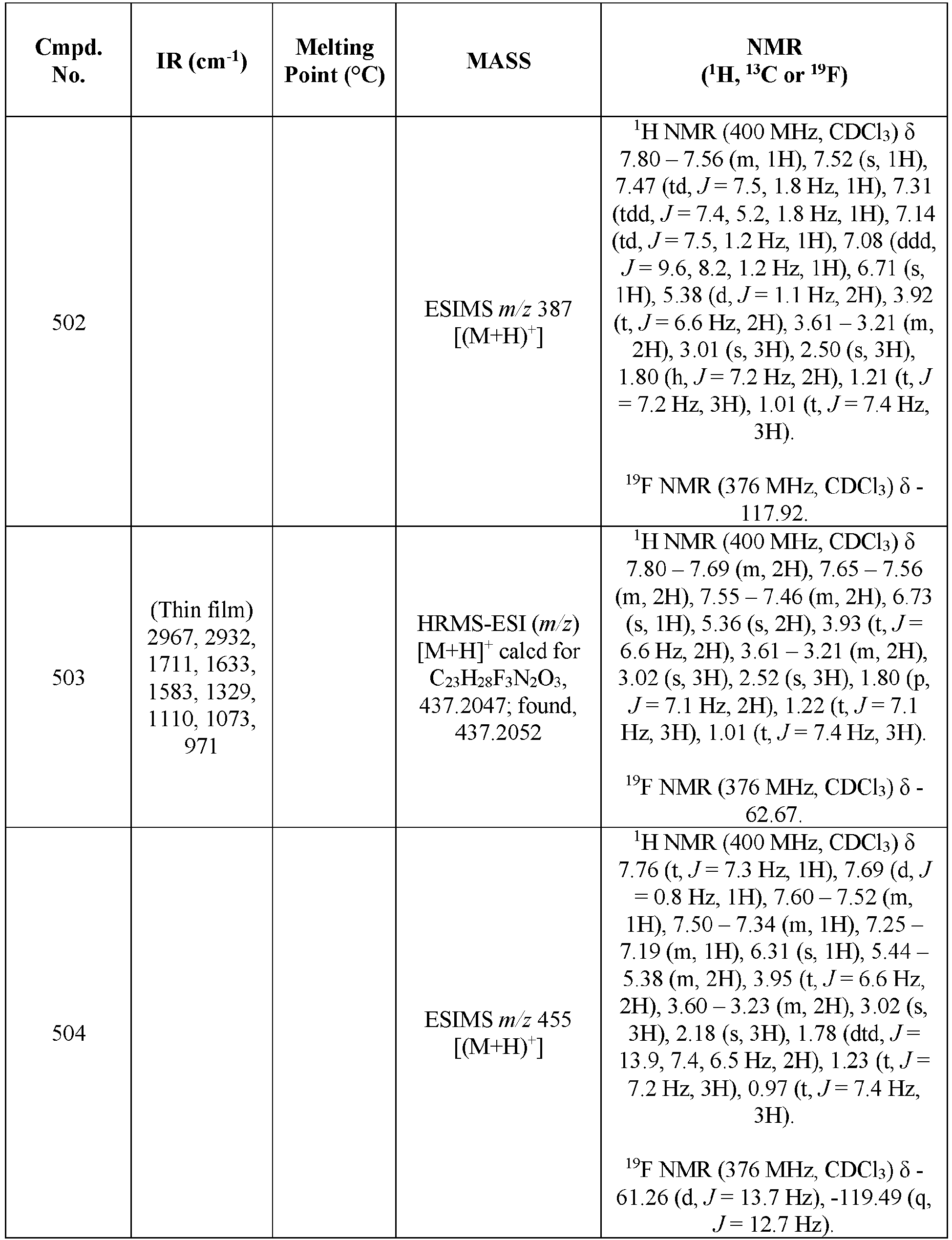

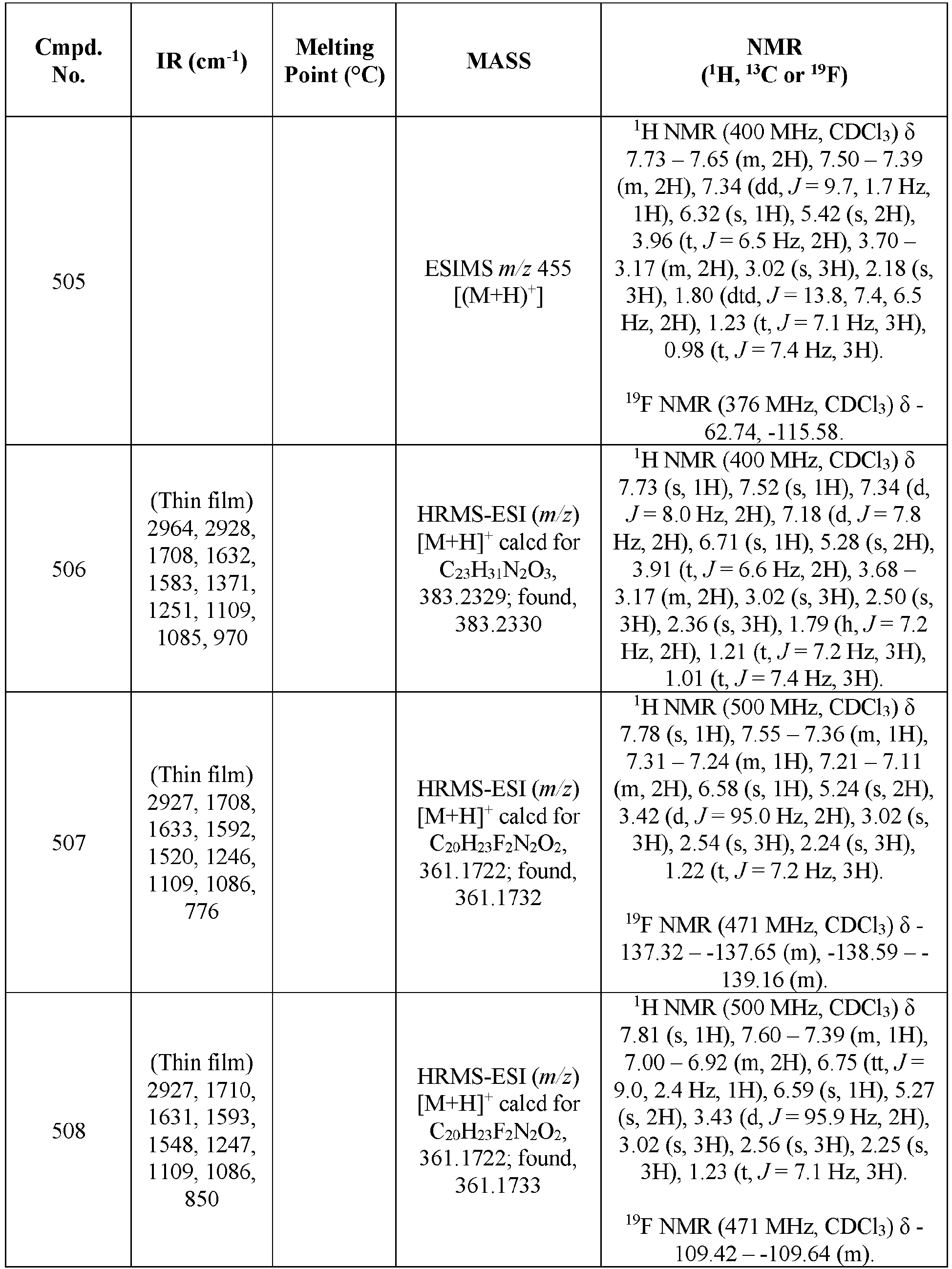

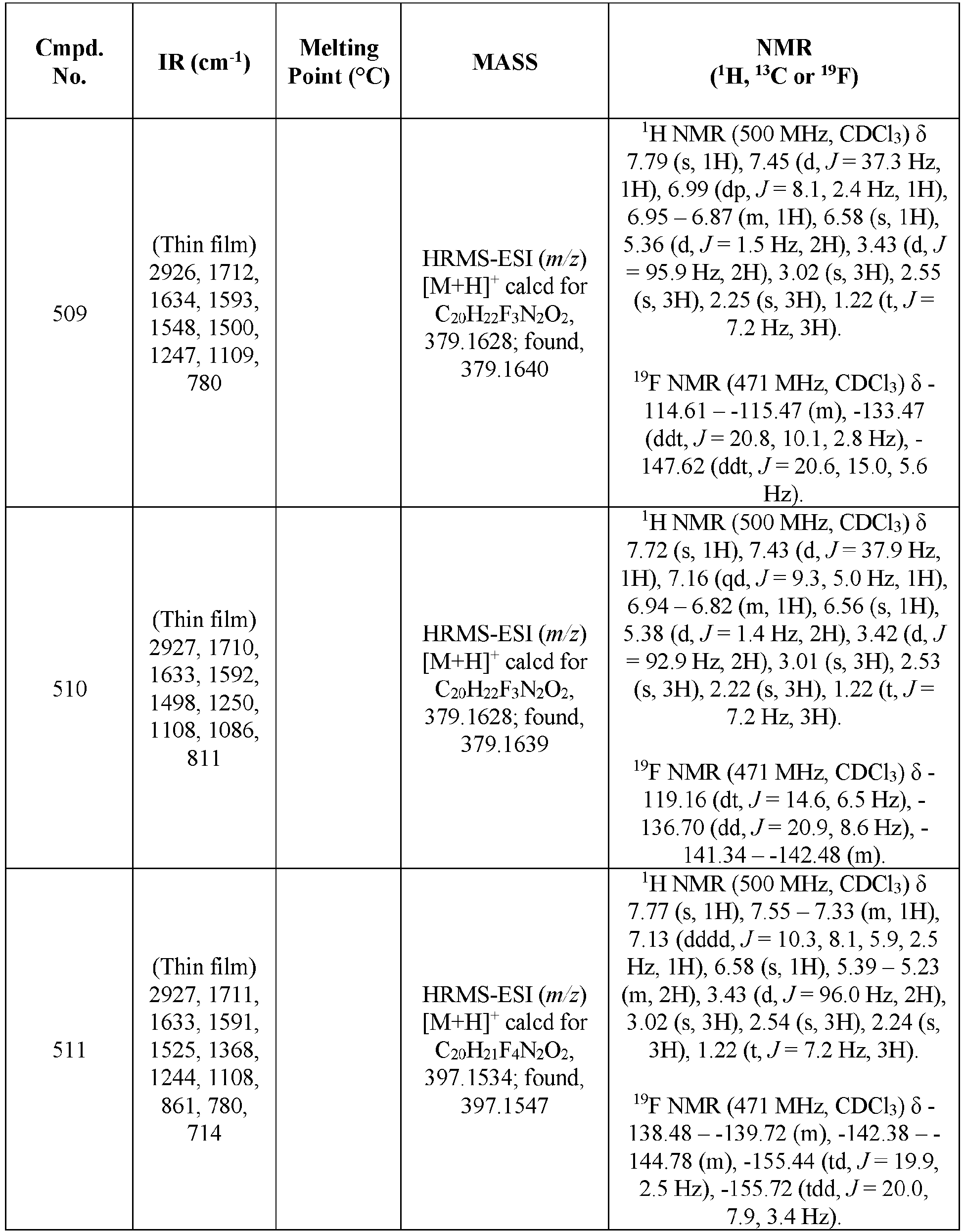

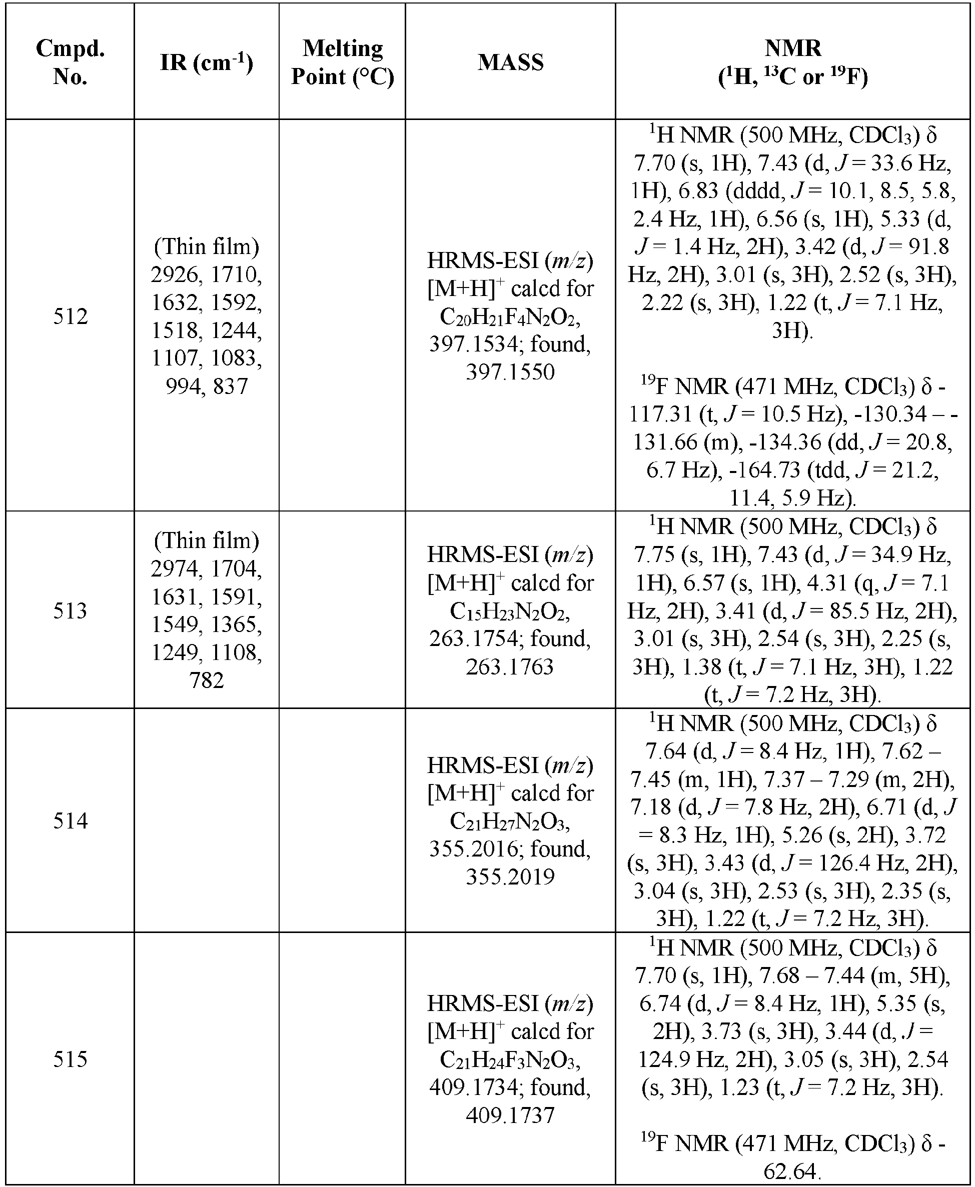

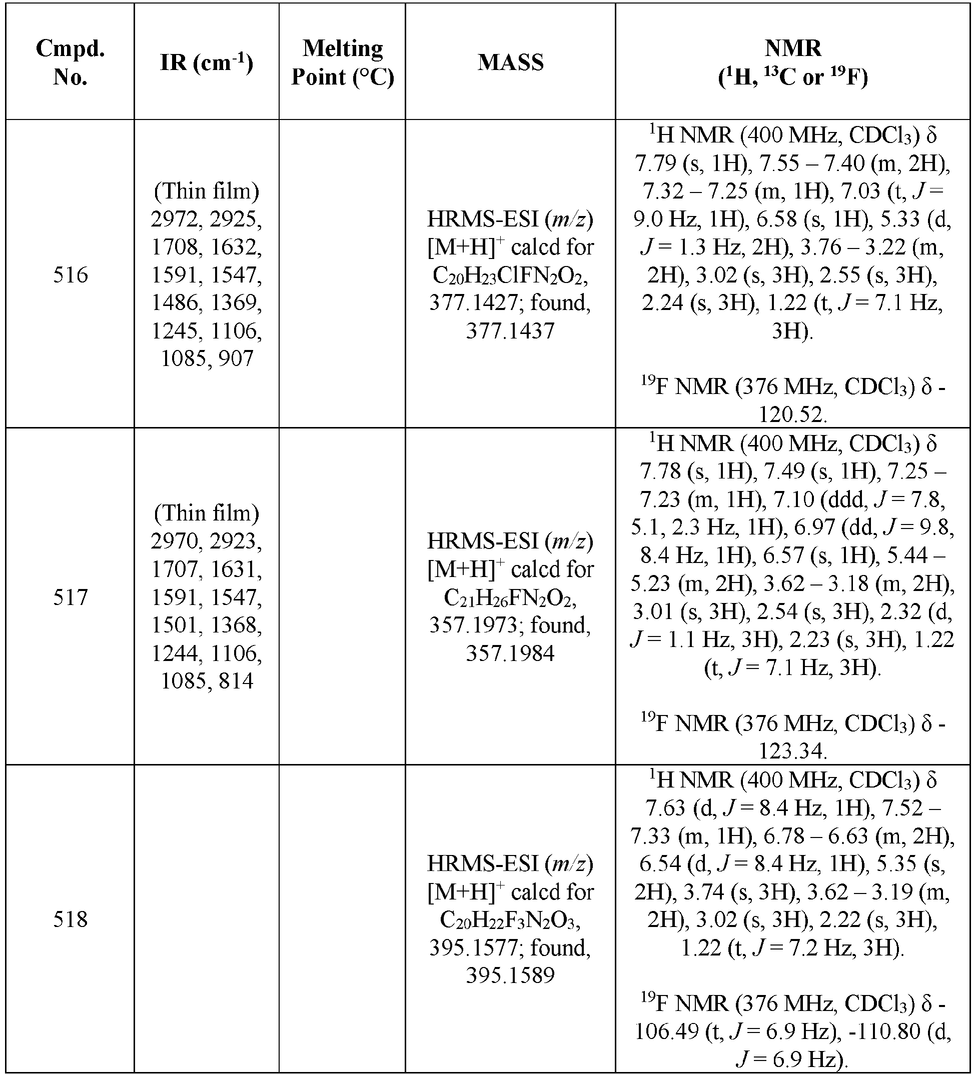

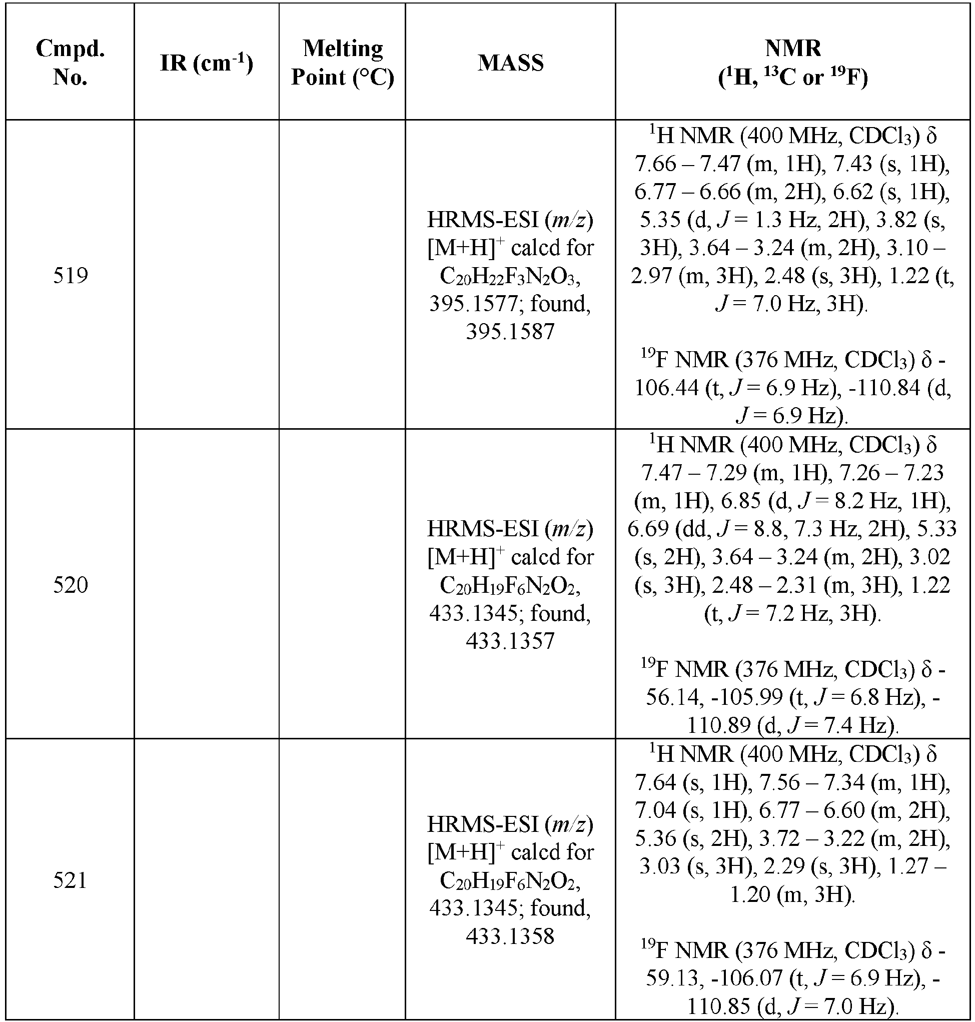

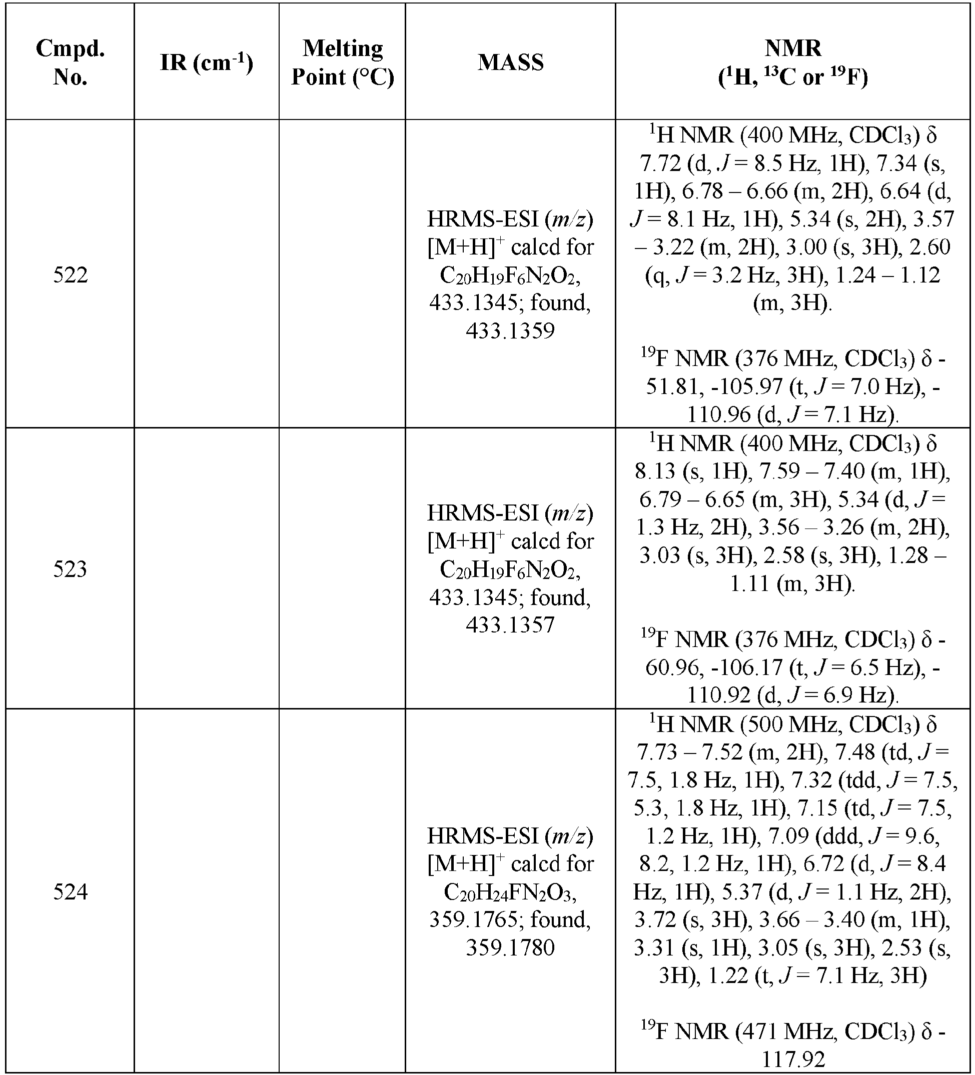

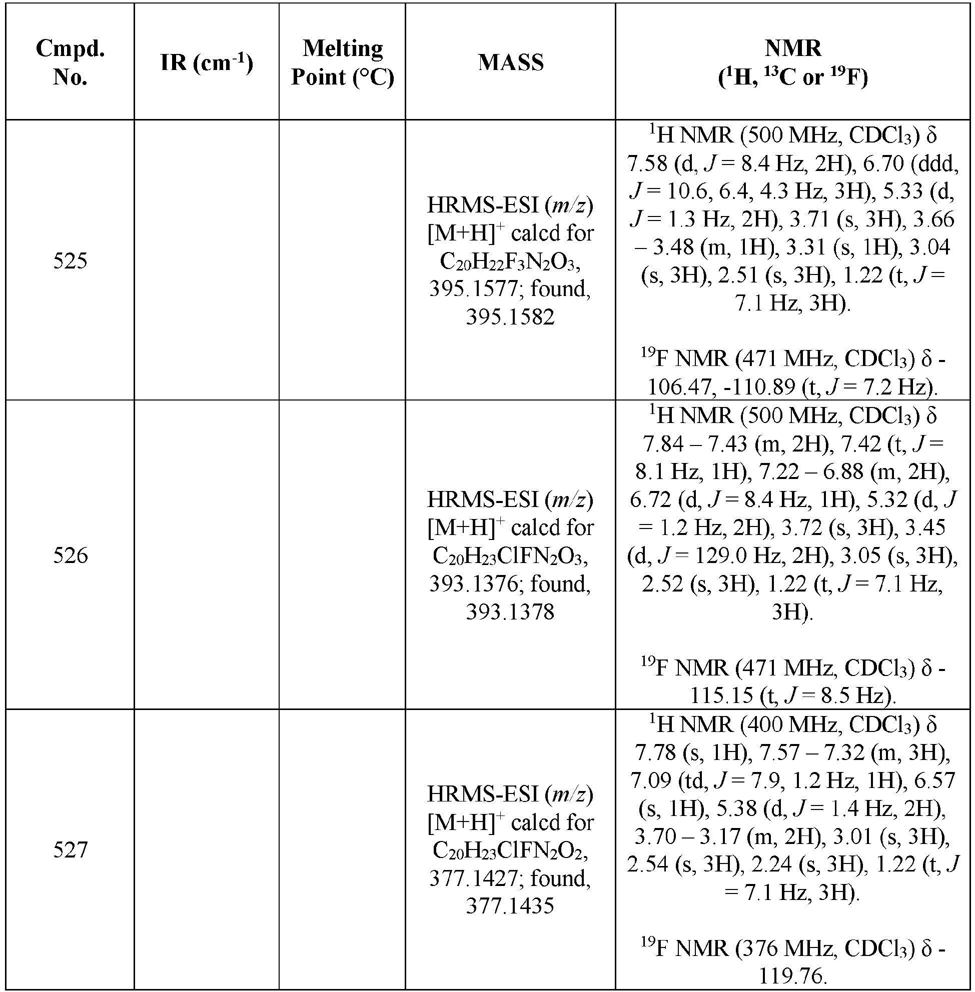

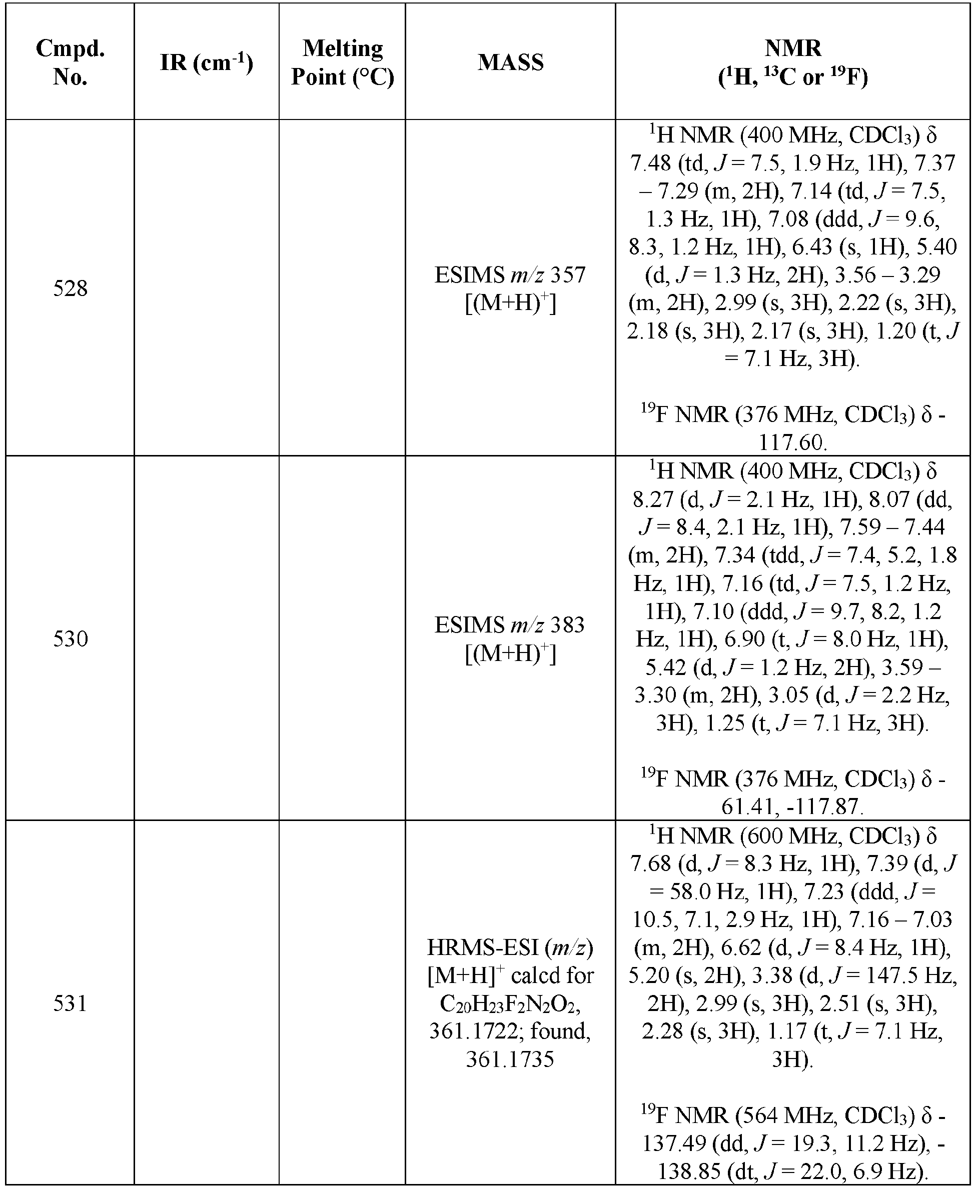

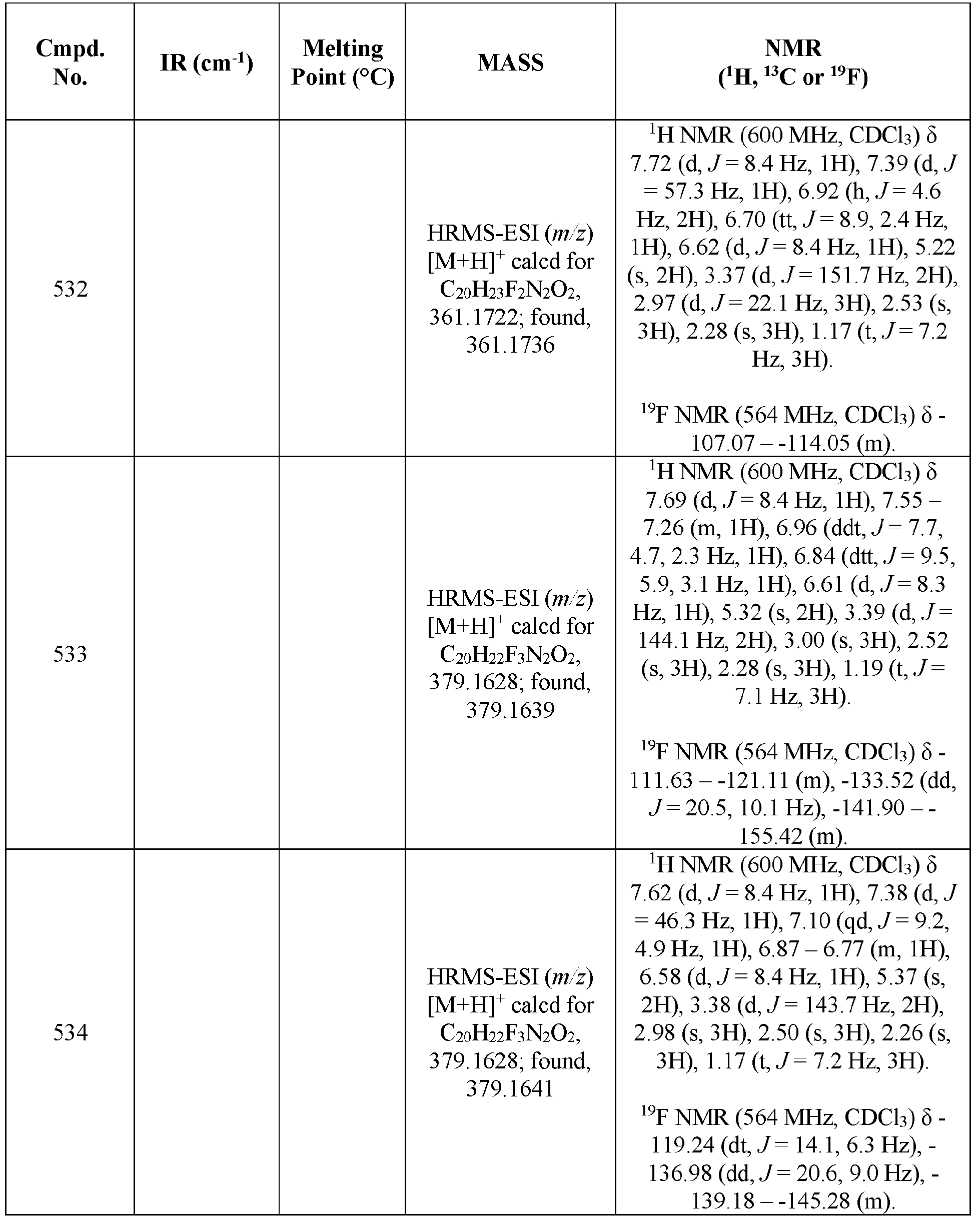

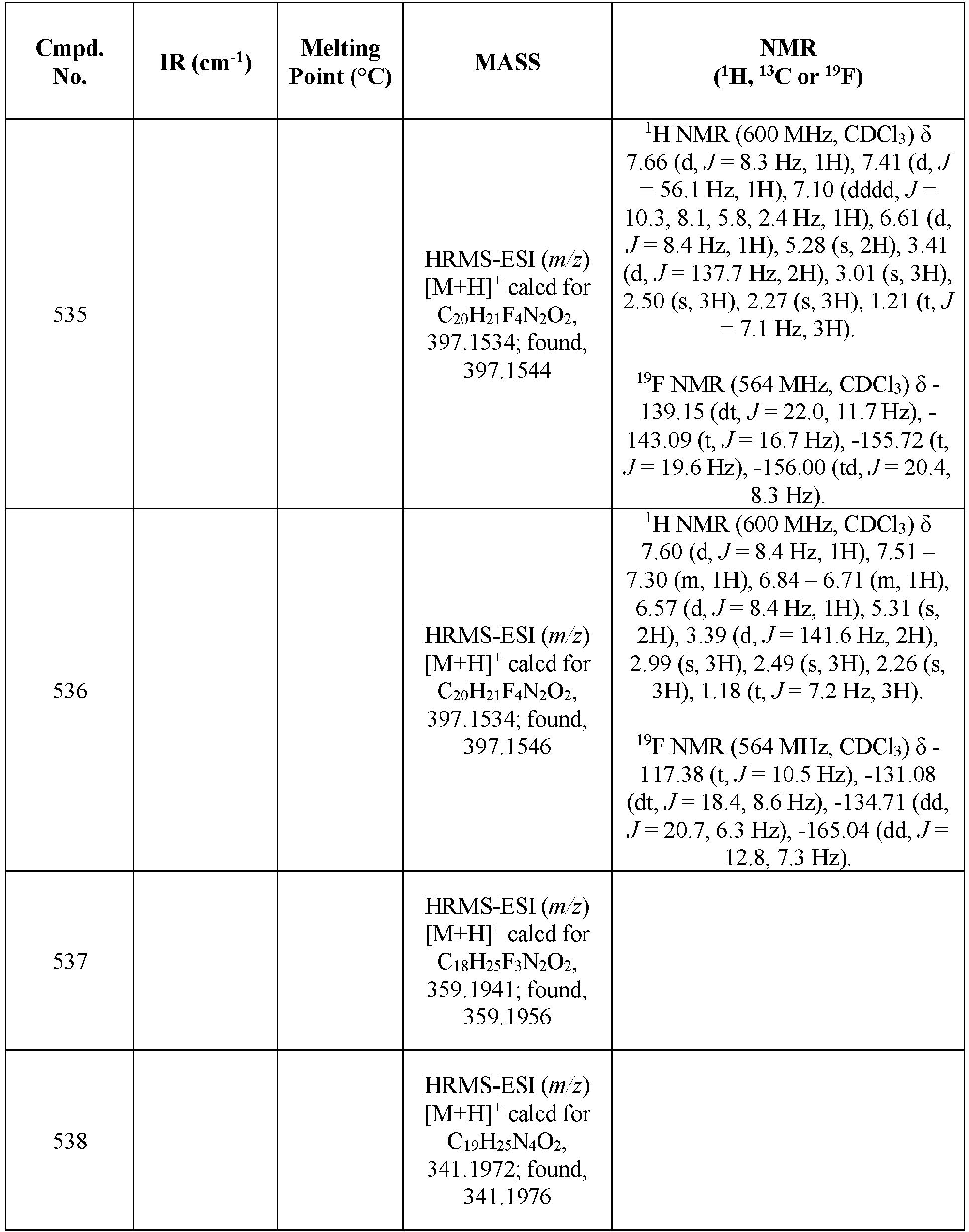

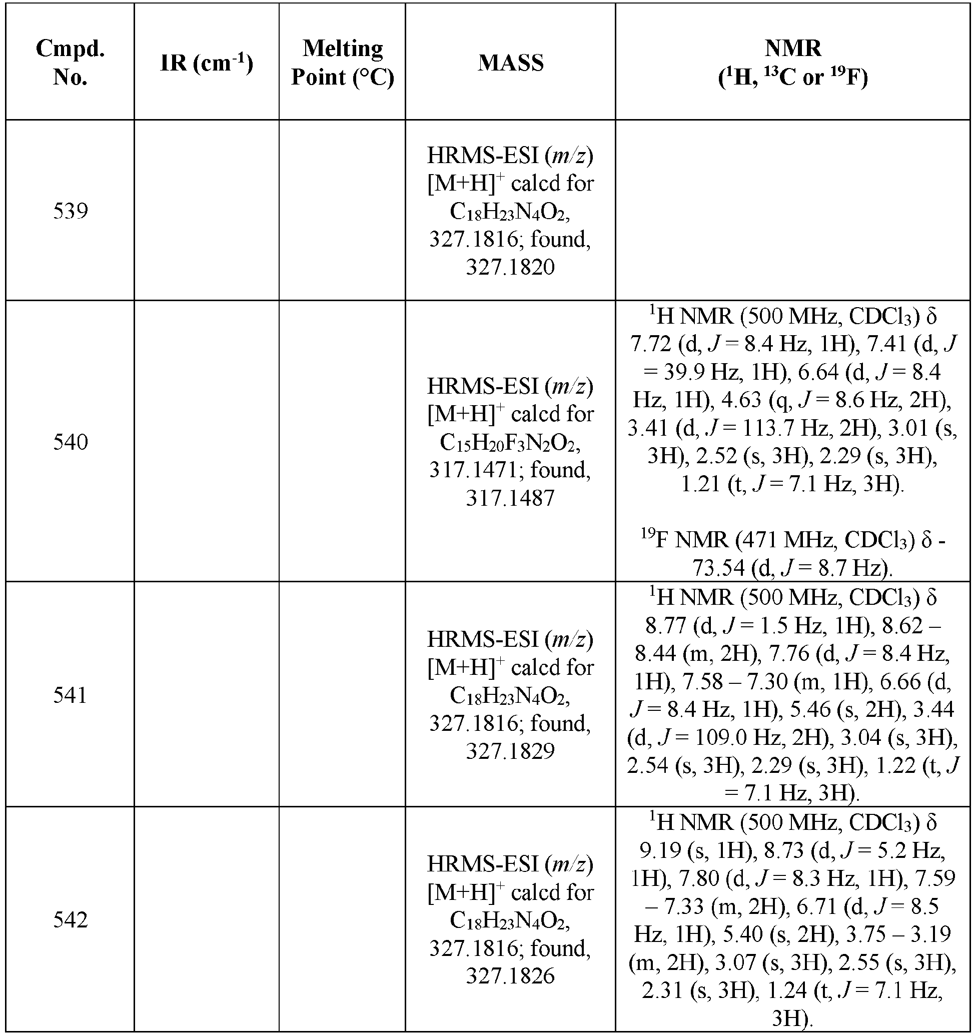

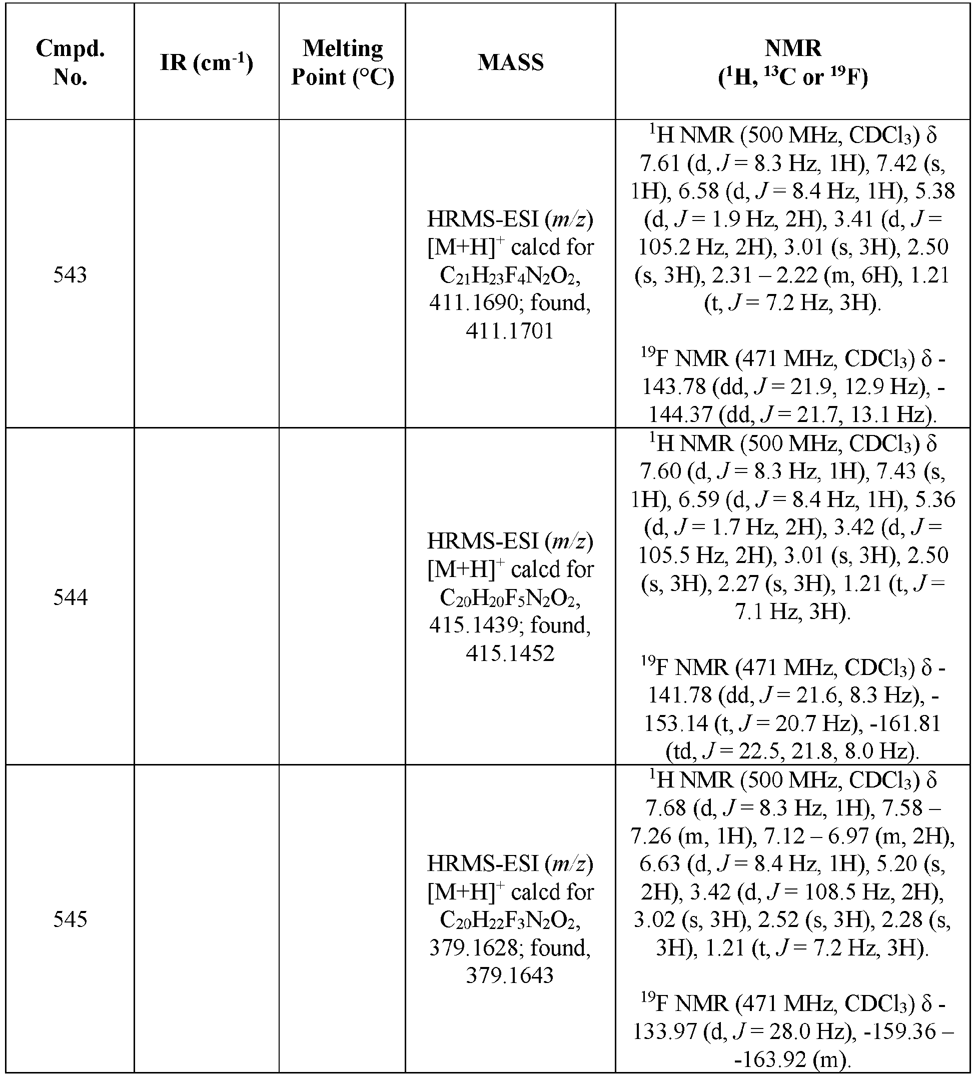

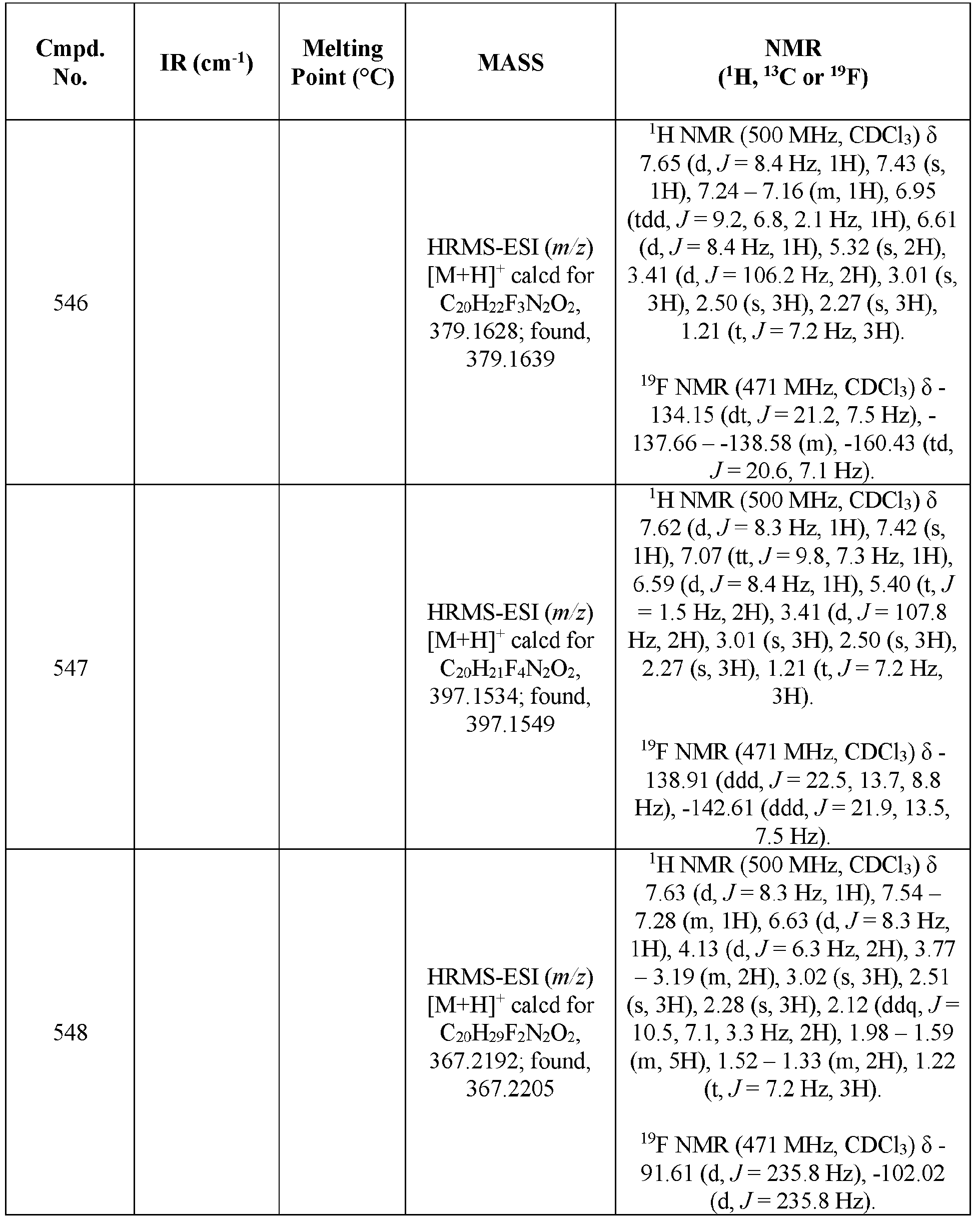

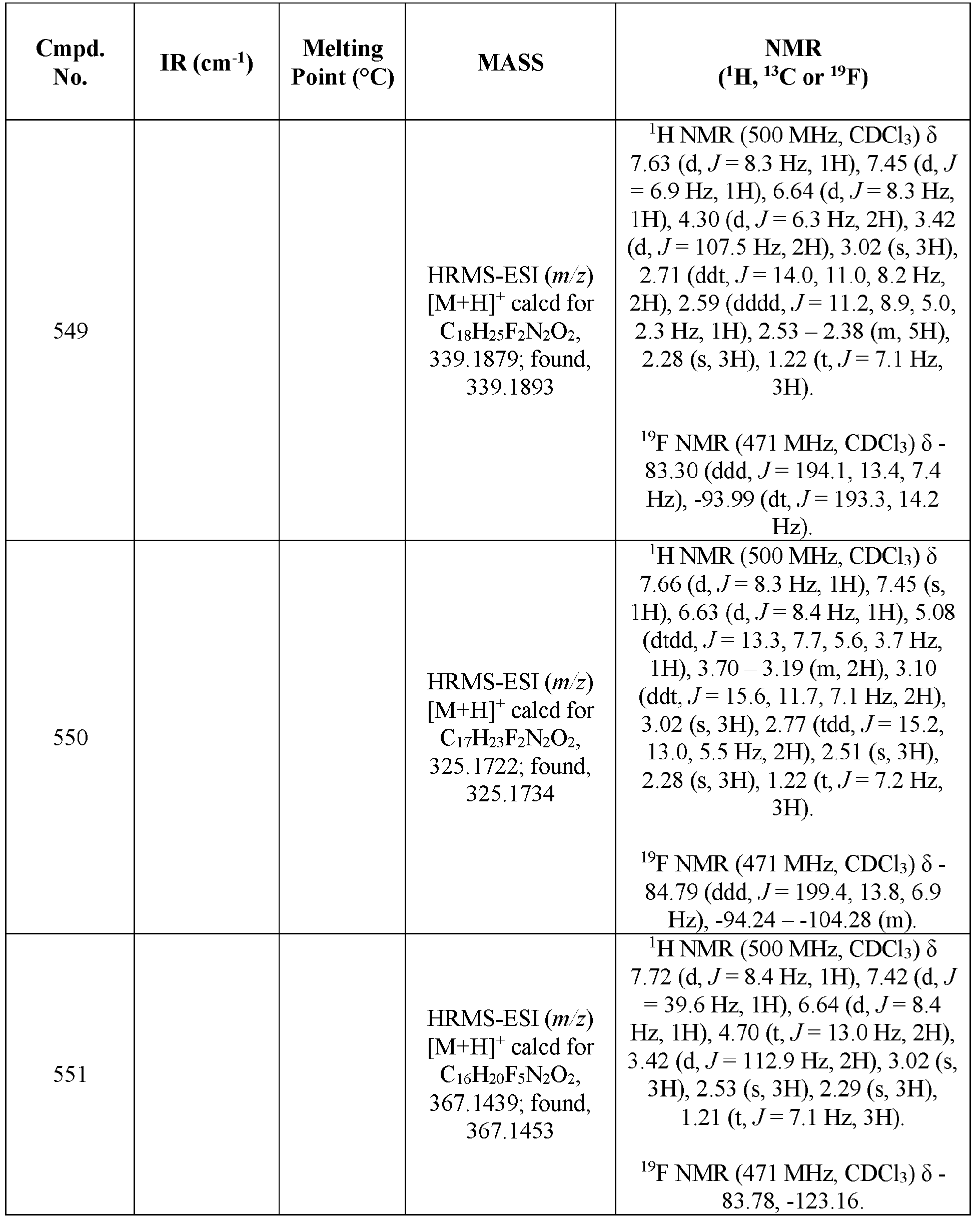

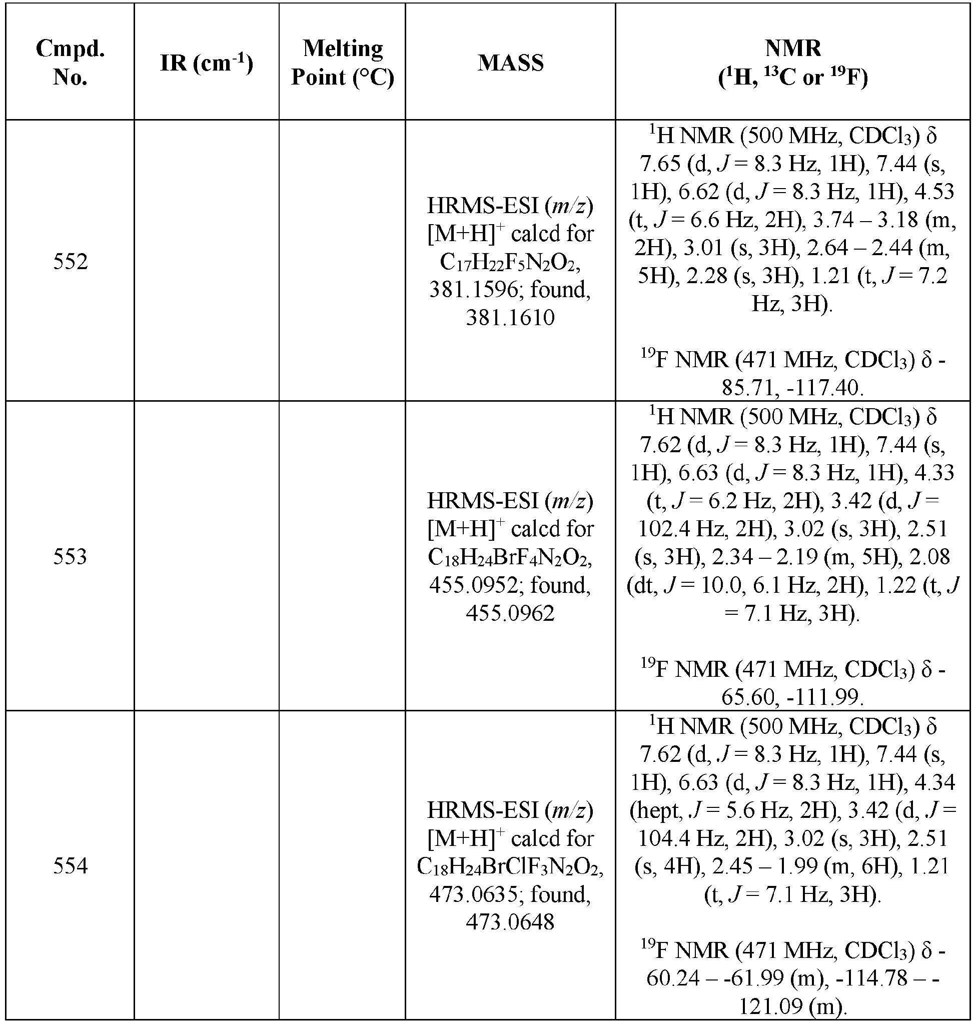

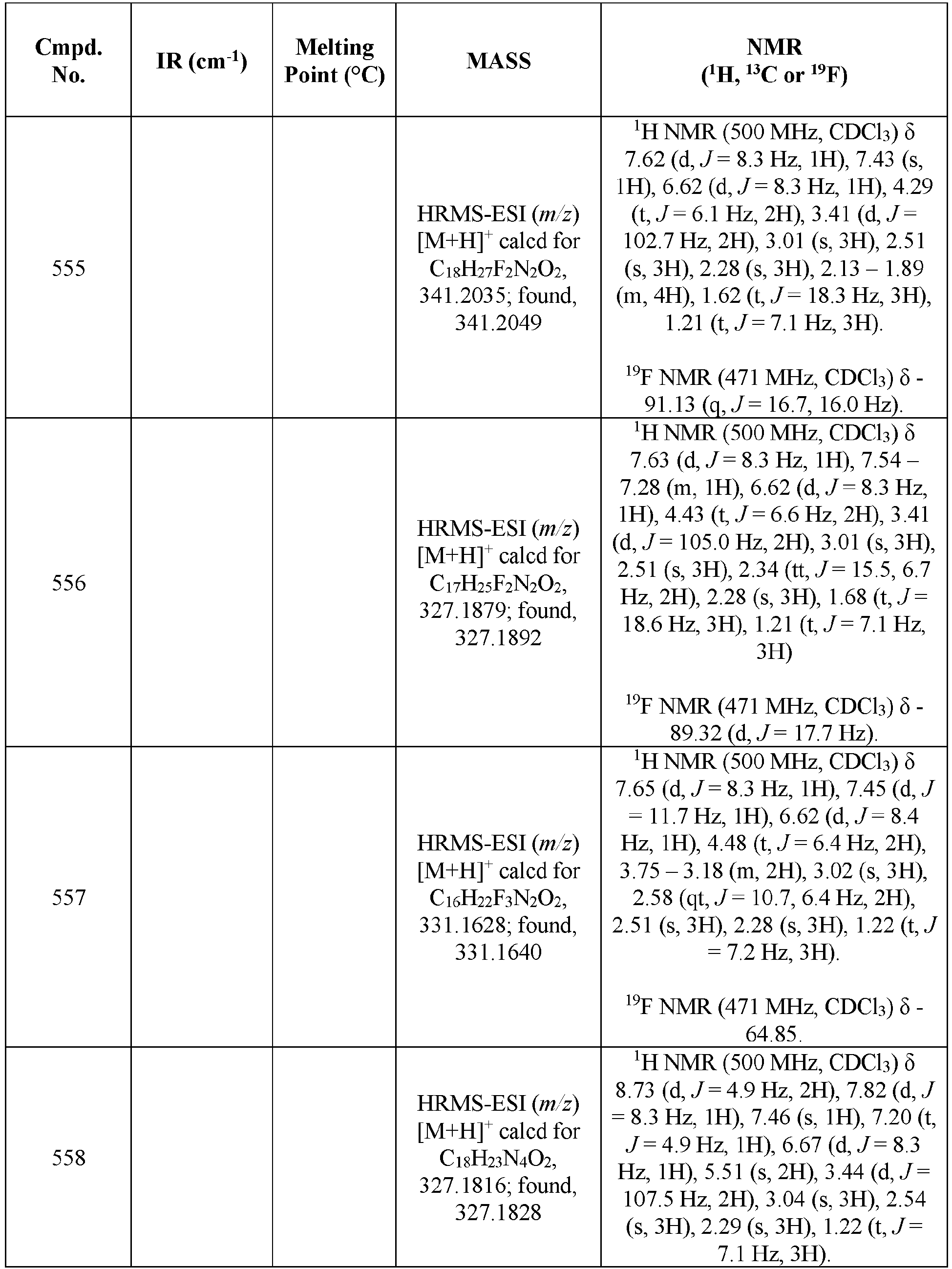

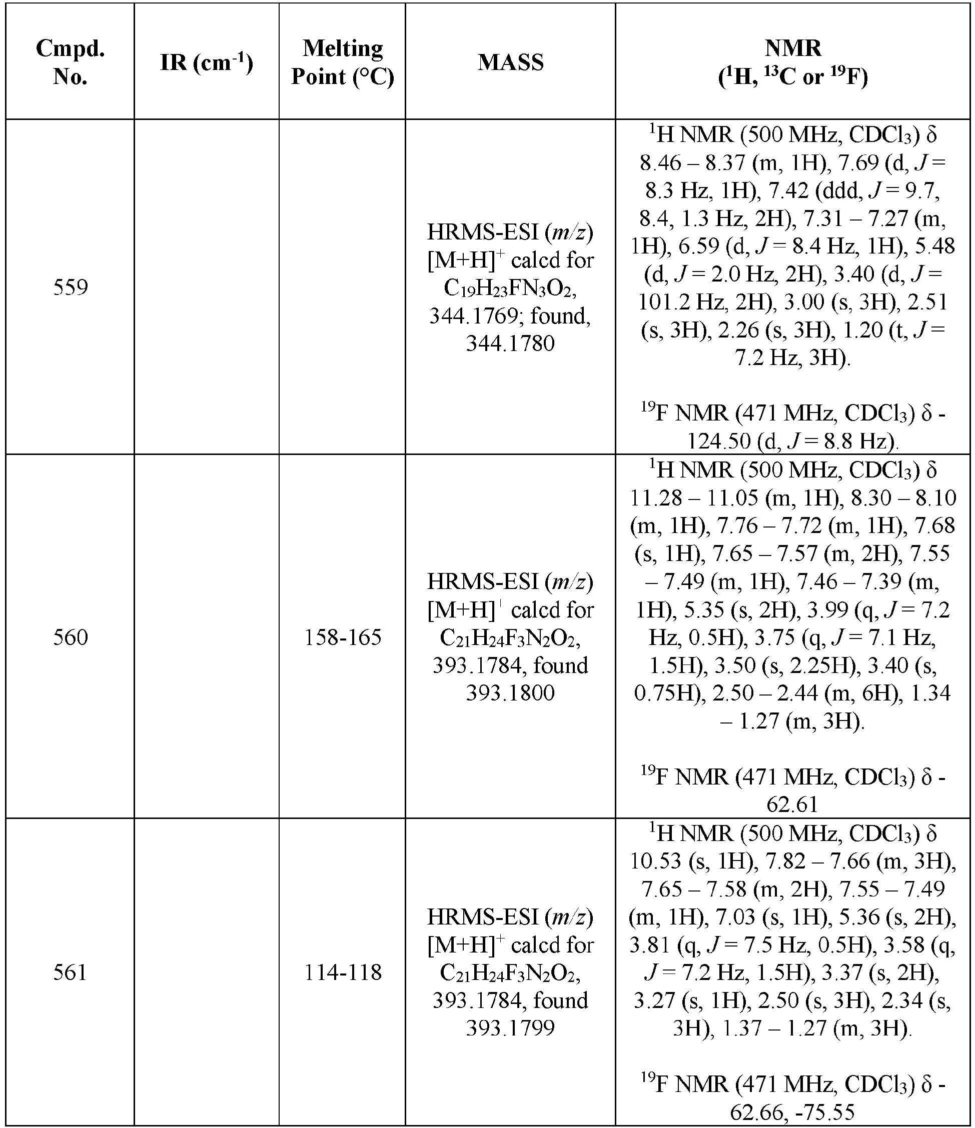

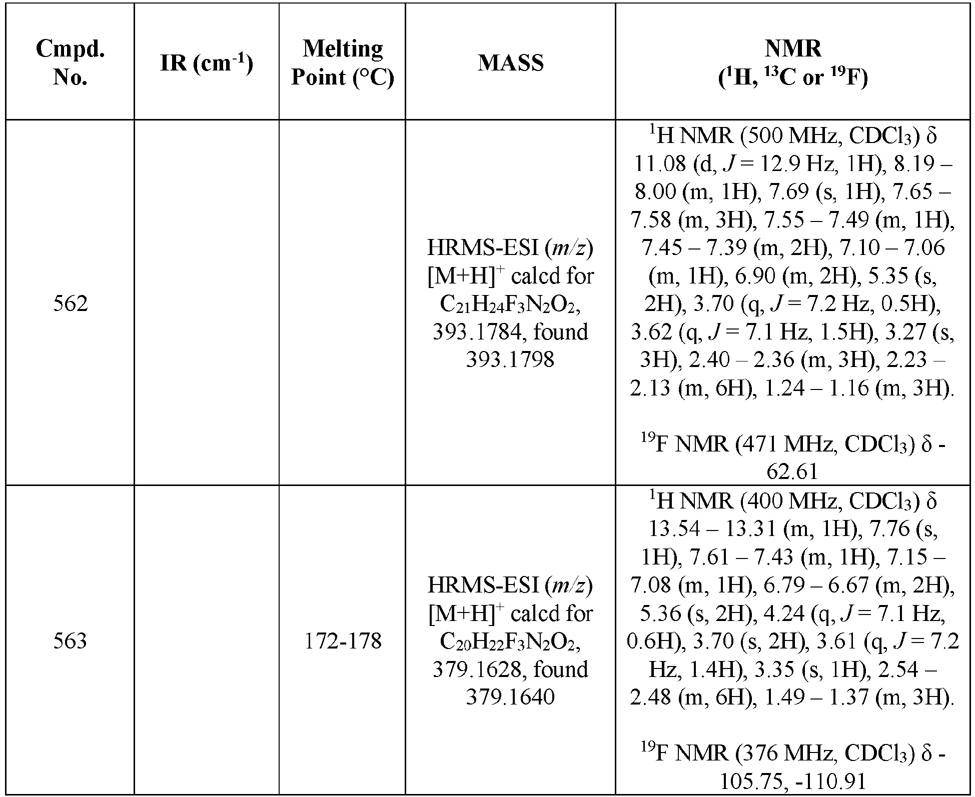

**Table 3. Biological Testing Rating Scale**

| **Rating Table for Fungal Pathogens** | |
|---|---|
| **% Disease Control** | **Rating** |
| >85% | A |
| 50 - 85% | B |
| 20 - <50% | C |
| <20% | D |
| Not Tested | E |

**Table 4. Biological Activity - COCHSA, PHAKPA, PUCCRT, RHYNSE, and SEPTTR Disease Control in Applications at 200 ppm**

| **Cmpd. No.** | **% Disease Control Rating** | | | | | | |
|---|---|---|---|---|---|---|---|
| | **CD-1DP** | | | | | **CD-3DC** | |
| | **COCHSA** | **PHAKPA** | **PUCCRT** | **RHYNSE** | **SEPTTR** | **PHAKPA** | **SEPTTR** |
| | **200 ppm** | **200 ppm** | **200 ppm** | **200 ppm** | **200 ppm** | **200 ppm** | **200 ppm** |
| 1 | E | B | A | E | D | A | D |
| 3 | D | A | A | E | C | A | B |
| 4 | C | A | A | E | D | A | D |
| 5 | D | E | D | E | C | E | D |
| 6 | C | C | A | E | D | A | D |
| 7 | B | B | A | E | C | A | c |
| 8 | B | A | A | E | A | A | D |
| 9 | A | A | A | A | B | A | B |
| 10 | C | A | A | A | B | A | C |
| 11 | A | B | A | B | A | A | C |
| 12 | A | C | B | A | A | B | B |
| 13 | D | E | A | E | C | E | C |
| 14 | D | D | C | E | D | B | D |
| 15 | D | D | D | E | C | B | D |
| 16 | B | A | A | A | A | A | B |
| 17 | C | A | A | E | D | A | D |
| 18 | A | A | A | A | B | A | A |
| 19 | C | A | A | E | D | A | D |
| 20 | B | A | A | E | C | A | D |
| 21 | B | A | A | A | C | A | B |
| 22 | B | A | A | C | C | A | C |
| 23 | A | A | A | E | C | A | D |
| 24 | B | A | A | E | D | A | D |
| 25 | A | A | A | A | B | A | C |
| 26 | A | A | A | A | A | A | B |
| 27 | A | A | A | A | A | A | B |
| 28 | A | A | A | A | A | A | B |
| 29 | A | A | A | A | B | A | B |
| 30 | A | A | A | A | A | A | A |
| 31 | A | A | A | E | C | A | C |
| 32 | A | A | A | B | B | A | C |
| 33 | A | A | A | A | A | A | B |
| 34 | B | C | A | E | D | B | D |
| 35 | D | A | A | E | C | A | D |
| 36 | D | A | A | A | A | A | D |
| 37 | A | A | A | E | A | A | B |
| 38 | B | A | A | E | A | A | A |
| 39 | A | C | D | E | C | C | B |
| 40 | A | A | A | E | A | A | A |
| 41 | A | A | A | E | A | A | A |
| 42 | A | A | A | E | A | A | A |
| 43 | A | A | A | E | B | A | C |
| 44 | A | A | A | E | A | A | A |
| 45 | A | A | A | E | A | A | A |
| 46 | B | A | A | E | A | A | B |
| 47 | A | A | A | E | A | A | B |
| 48 | A | A | A | E | A | A | A |
| 49 | B | A | A | E | A | A | B |
| 50 | B | B | A | E | C | A | B |
| 51 | A | A | A | E | A | A | A |
| 52 | A | A | A | E | A | A | A |
| 53 | E | A | E | E | E | A | E |
| 54 | E | A | E | E | E | A | E |
| 55 | C | A | A | E | C | A | B |
| 56 | D | B | A | E | D | A | D |
| 57 | E | A | A | A | A | A | C |
| 58 | D | A | A | E | B | A | D |
| 59 | D | D | D | E | D | D | D |
| 60 | D | D | D | E | D | C | C |
| 61 | D | A | A | E | C | A | C |
| 62 | A | A | A | E | B | A | B |
| 63 | D | D | D | E | D | C | D |
| 64 | B | C | A | E | B | B | A |
| 65 | A | A | A | A | A | A | A |
| 66 | B | B | A | E | B | A | B |
| 67 | D | D | D | E | D | D | D |
| 68 | A | A | D | A | B | A | A |
| 69 | A | A | A | A | A | A | B |
| 70 | D | D | D | E | C | D | D |
| 72 | D | D | D | E | D | C | D |
| 73 | A | A | A | E | B | B | D |
| 74 | D | D | D | E | D | D | D |
| 75 | D | D | D | E | D | D | D |
| 76 | D | D | D | E | D | D | D |
| 77 | D | D | D | E | D | D | C |
| 78 | D | D | D | E | D | D | D |
| 79 | C | A | B | E | B | A | B |
| 80 | D | C | C | E | B | C | C |
| 81 | D | C | D | E | D | D | C |
| 82 | C | B | C | E | B | C | C |
| 83 | B | B | A | E | B | B | A |
| 84 | D | D | D | E | D | D | D |
| 86 | D | D | D | E | D | D | C |
| 89 | D | C | D | E | D | D | C |
| 90 | C | D | D | E | C | D | D |
| 91 | B | A | C | E | B | A | A |
| 92 | D | A | B | E | D | A | D |
| 94 | D | C | D | E | C | D | D |
| 95 | D | C | D | E | D | D | C |
| 96 | D | C | B | E | D | A | D |
| 97 | D | A | A | C | D | A | B |
| 98 | D | B | A | E | A | A | B |
| 99 | D | C | D | E | A | D | D |
| 100 | D | C | B | E | A | C | D |
| 101 | D | C | C | E | B | D | D |
| 103 | D | A | B | E | B | A | D |
| 105 | D | C | A | E | D | C | C |
| 106 | D | A | A | E | B | C | B |
| 107 | D | A | A | E | A | A | B |
| 109 | D | C | D | E | D | D | B |
| 110 | D | A | A | E | B | A | A |
| 111 | D | A | A | E | A | A | C |
| 112 | B | A | A | A | A | A | B |
| 113 | D | A | D | D | D | A | C |
| 114 | D | A | A | D | D | A | B |
| 115 | C | A | A | C | D | B | D |
| 116 | B | A | A | C | D | A | B |
| 117 | C | A | A | A | A | A | A |
| 118 | D | A | B | D | D | B | B |
| 119 | D | A | A | D | D | A | A |
| 120 | D | A | A | C | D | A | C |
| 121 | C | A | A | B | A | A | B |
| 122 | D | A | A | C | D | A | D |
| 123 | B | A | A | A | B | A | C |
| 124 | C | A | A | B | A | A | D |
| 125 | A | A | A | A | A | A | B |
| 126 | D | A | A | D | D | A | A |
| 127 | C | A | A | A | A | A | A |
| 128 | C | A | A | B | A | A | B |
| 129 | B | A | A | A | A | A | A |
| 130 | C | C | A | A | A | B | C |
| 131 | D | C | A | B | A | A | D |
| 132 | D | D | B | A | A | C | D |
| 133 | D | C | A | D | B | C | D |
| 134 | D | D | B | D | A | D | D |
| 135 | D | D | C | D | D | B | D |
| 136 | C | D | A | B | B | D | D |
| 137 | C | D | D | D | B | D | D |
| 138 | D | D | A | D | D | D | D |
| 139 | D | D | A | A | C | D | B |
| 140 | D | D | D | D | B | D | D |
| 141 | D | D | D | D | D | D | D |
| 142 | A | A | A | A | A | A | D |
| 143 | A | A | A | A | A | A | D |
| 144 | A | A | A | E | A | A | D |
| 145 | A | A | A | E | A | A | D |
| 146 | C | B | A | E | B | B | D |
| 147 | A | A | A | E | A | A | D |
| 148 | A | A | A | E | A | A | D |
| 149 | A | B | A | E | A | B | D |
| 150 | A | A | A | E | A | A | D |
| 151 | C | D | A | E | D | D | D |
| 152 | C | B | A | E | A | C | D |
| 153 | A | A | A | E | A | A | D |
| 154 | D | C | A | E | D | D | D |
| 155 | D | A | A | E | B | A | D |
| 156 | A | A | A | E | A | A | B |
| 157 | A | A | A | E | A | A | B |
| 158 | A | A | A | A | A | A | B |
| 159 | B | A | A | E | A | A | D |
| 160 | B | A | A | E | A | A | A |
| 161 | D | A | A | E | A | A | B |
| 162 | A | A | A | E | A | A | B |
| 163 | B | A | A | E | A | A | A |
| 164 | A | A | A | E | A | A | D |
| 165 | D | C | A | E | D | D | D |
| 166 | A | A | A | E | B | A | D |
| 167 | E | A | A | A | A | A | B |
| 168 | E | A | A | A | A | A | c |
| 169 | E | A | A | A | A | A | B |
| 170 | E | A | A | A | A | A | C |
| 171 | E | A | A | A | A | A | B |
| 172 | E | A | A | A | A | A | B |
| 173 | E | A | A | A | A | A | C |
| 174 | E | A | A | A | A | A | B |
| 175 | D | C | A | E | D | B | D |
| 176 | E | D | D | D | D | D | D |
| 177 | E | A | A | C | D | A | D |
| 178 | E | A | A | A | D | A | D |
| 179 | D | A | A | E | B | A | D |
| 180 | B | A | A | E | C | A | D |
| 181 | D | A | A | E | B | A | D |
| 182 | B | A | A | E | A | A | C |
| 183 | D | A | A | E | A | A | D |
| 184 | D | A | A | E | C | A | D |
| 185 | D | A | A | E | A | A | D |
| 186 | D | A | A | E | A | A | C |
| 187 | C | A | A | E | A | A | D |
| 188 | D | A | A | E | A | A | D |
| 189 | C | A | A | E | A | A | D |
| 190 | D | A | A | E | A | A | C |
| 191 | D | A | A | E | A | A | C |
| 192 | D | A | A | E | B | A | D |
| 193 | D | A | A | E | A | A | C |
| 194 | D | A | A | E | B | A | D |
| 195 | D | A | A | E | A | A | C |
| 196 | D | A | A | E | A | A | D |
| 197 | D | A | A | E | C | A | D |
| 198 | C | A | A | E | D | A | D |
| 199 | D | A | A | E | B | A | D |
| 200 | D | A | A | E | C | A | D |
| 201 | D | A | A | E | B | A | D |
| 202 | D | A | A | E | B | A | D |
| 203 | D | A | A | E | D | A | D |
| 204 | E | B | A | A | A | B | B |
| 205 | E | A | A | A | A | A | C |
| 206 | E | B | A | A | B | B | D |
| 207 | E | A | A | A | B | A | B |
| 208 | E | B | A | B | A | B | B |
| 209 | E | B | A | B | B | A | C |
| 210 | E | C | A | A | A | B | B |
| 212 | E | B | A | D | C | A | D |
| 213 | E | A | A | C | B | A | B |
| 214 | E | C | A | C | B | B | C |
| 215 | E | A | A | A | D | A | B |
| 217 | E | C | A | D | B | A | D |
| 218 | E | B | A | D | D | B | D |
| 219 | E | B | A | D | D | B | D |
| 220 | E | A | A | A | A | A | D |
| 221 | E | A | A | A | A | B | C |
| 222 | D | A | B | E | D | A | D |
| 223 | D | A | A | E | B | A | D |
| 224 | D | A | A | E | C | A | D |
| 225 | D | A | A | E | B | A | D |
| 226 | D | A | A | E | D | A | D |
| 227 | D | A | A | E | A | A | D |
| 228 | D | A | A | E | D | A | D |
| 229 | D | A | A | E | D | A | D |
| 230 | E | A | A | D | B | A | B |
| 231 | E | A | A | D | B | A | B |
| 232 | E | A | A | D | A | A | D |
| 233 | E | A | A | C | A | A | B |
| 234 | E | A | A | D | A | A | D |
| 235 | E | C | D | D | C | C | D |
| 236 | E | A | A | D | A | B | D |
| 237 | E | A | A | D | A | B | C |
| 238 | E | A | A | D | D | A | D |
| 239 | E | A | A | D | A | B | A |
| 240 | E | A | A | D | C | A | D |
| 241 | E | A | A | D | B | A | D |
| 242 | E | B | D | D | D | B | D |
| 243 | E | A | A | D | D | B | D |
| 244 | E | A | A | D | D | A | D |
| 245 | E | A | A | D | C | A | C |
| 246 | E | B | A | D | D | B | D |
| 247 | E | A | A | D | D | A | D |
| 248 | E | D | D | D | D | D | D |
| 249 | E | D | D | D | D | A | D |
| 250 | E | A | A | D | D | A | D |
| 251 | E | B | A | D | D | A | D |
| 252 | E | A | A | D | D | A | D |
| 253 | E | A | A | D | D | A | D |
| 254 | E | A | A | D | D | A | D |
| 255 | E | A | A | D | D | B | D |
| 256 | E | A | A | D | D | A | D |
| 257 | E | C | A | D | D | A | D |
| 258 | E | C | A | D | D | A | D |
| 259 | E | A | A | D | D | B | D |
| 260 | E | B | D | D | D | B | D |
| 261 | E | B | A | D | D | B | D |
| 262 | E | C | B | D | D | C | D |
| 263 | E | C | C | D | D | C | D |
| 264 | E | B | A | D | D | C | C |
| 265 | E | A | A | D | D | B | D |
| 266 | E | A | A | A | D | A | D |
| 267 | E | A | A | A | B | A | D |
| 268 | E | A | A | A | C | A | D |
| 269 | E | A | A | A | C | A | D |
| 270 | E | A | A | A | D | A | D |
| 271 | E | C | D | D | D | C | D |
| 272 | E | B | E | E | E | B | E |
| 273 | E | B | A | D | D | B | D |
| 274 | E | B | B | D | D | A | D |
| 275 | E | A | A | D | D | A | D |
| 276 | E | C | C | B | D | B | D |
| 277 | E | A | B | D | C | A | D |
| 278 | E | A | A | D | B | A | D |
| 279 | E | A | A | B | B | A | D |
| 280 | E | A | A | D | D | A | D |
| 285 | E | D | B | D | C | D | D |
| 286 | E | D | D | D | D | D | D |
| 287 | E | C | A | D | C | D | D |
| 288 | E | D | A | D | B | C | D |
| 289 | E | D | D | D | C | D | D |
| 290 | E | D | D | D | D | D | D |
| 291 | E | D | D | D | D | D | D |
| 292 | E | A | B | D | C | B | D |
| 293 | E | D | D | D | D | C | D |
| 294 | E | D | D | D | D | C | D |
| 295 | E | D | D | D | D | D | D |
| 296 | E | C | D | D | D | C | D |
| 297 | E | D | D | D | D | C | D |
| 298 | E | D | D | D | D | C | D |
| 299 | E | D | D | D | D | B | D |
| 300 | E | C | A | C | D | B | D |
| 301 | E | A | A | D | C | A | D |
| 302 | E | A | A | D | D | A | D |
| 303 | E | A | A | D | D | A | D |
| 304 | E | A | A | D | D | A | D |
| 305 | E | B | A | C | B | B | D |
| 306 | E | B | A | D | D | B | D |
| 307 | E | B | A | D | D | B | D |
| 308 | E | B | A | B | D | B | D |
| 309 | E | A | A | A | D | A | D |
| 310 | E | B | A | A | C | B | D |
| 311 | E | A | B | B | B | A | C |
| 312 | E | C | B | C | B | C | C |
| 313 | E | A | A | D | D | A | D |
| 314 | E | D | A | D | D | C | D |
| 315 | E | A | A | C | D | A | D |
| 316 | E | A | A | C | D | A | D |
| 317 | E | A | A | D | C | A | D |
| 318 | E | A | A | A | A | A | D |
| 319 | E | A | A | D | D | A | D |
| 320 | E | A | A | D | D | A | D |
| 321 | E | A | D | D | C | A | D |
| 322 | E | A | A | D | D | A | D |
| 323 | E | A | D | D | D | C | D |
| 324 | E | A | A | B | B | A | D |
| 325 | E | A | A | A | A | A | D |
| 326 | E | B | A | A | B | A | C |
| 327 | E | A | A | A | A | A | C |
| 328 | E | A | A | A | C | A | C |
| 329 | E | A | A | A | C | A | C |
| 330 | E | C | A | A | A | B | D |
| 331 | E | A | A | A | C | A | C |
| 332 | E | A | A | A | C | A | D |
| 333 | E | D | A | A | A | D | A |
| 334 | E | B | D | D | D | A | D |
| 335 | E | D | D | A | C | D | C |
| 336 | E | D | B | B | D | C | C |
| 337 | E | D | D | D | D | A | D |
| 338 | E | A | B | D | D | A | C |
| 339 | E | A | B | B | B | A | C |
| 340 | E | D | D | D | D | D | D |
| 341 | E | D | D | D | D | D | D |
| 342 | E | D | B | C | D | D | D |
| 343 | E | B | A | B | D | A | D |
| 344 | E | D | D | B | D | D | D |
| 345 | E | A | A | C | B | A | D |
| 346 | E | C | A | D | D | A | D |
| 347 | E | A | C | C | D | A | D |
| 348 | E | C | A | C | D | B | C |
| 349 | E | D | D | D | D | D | D |
| 350 | E | D | D | B | B | B | B |
| 351 | E | D | D | A | B | D | C |
| 352 | E | C | A | A | C | B | C |
| 353 | E | D | D | D | D | D | D |
| 354 | E | D | B | A | D | D | D |
| 355 | E | A | D | B | D | A | D |
| 356 | E | C | D | D | D | A | D |
| 357 | E | C | D | D | D | A | D |
| 358 | E | A | A | C | D | A | D |
| 359 | E | A | A | C | D | A | D |
| 360 | E | A | A | D | D | A | D |
| 361 | E | A | A | B | B | A | B |
| 362 | E | A | A | A | D | A | D |
| 363 | E | A | A | A | B | A | B |
| 364 | E | A | A | A | D | A | D |
| 365 | E | A | A | A | C | A | D |
| 366 | E | A | A | B | D | A | D |
| 367 | E | A | A | A | D | A | D |
| 368 | E | A | A | A | B | A | B |
| 369 | E | A | A | A | C | A | D |
| 370 | E | A | A | A | B | A | B |
| 371 | E | A | A | A | B | A | B |
| 372 | E | A | A | B | D | A | D |
| 373 | E | A | A | C | C | A | C |
| 374 | E | A | A | C | D | A | D |
| 375 | E | A | A | D | D | A | D |
| 376 | E | A | A | D | D | A | D |
| 377 | E | A | A | D | D | A | D |
| 378 | E | A | A | A | B | A | D |
| 379 | E | A | A | A | B | A | C |
| 380 | E | A | A | A | B | A | C |
| 381 | E | A | A | A | A | A | C |
| 382 | E | A | A | A | A | A | B |
| 383 | E | A | A | A | B | A | D |
| 384 | E | D | A | A | D | B | D |
| 385 | E | B | A | A | C | A | D |
| 386 | E | C | D | A | C | B | D |
| 387 | E | D | A | A | C | D | D |
| 388 | E | C | D | C | D | D | D |
| 389 | E | A | A | A | C | A | D |
| 390 | E | B | C | A | C | B | D |
| 391 | E | D | C | A | C | D | D |
| 392 | E | D | D | A | D | D | D |
| 393 | E | D | A | B | D | D | D |
| 394 | E | D | D | B | D | D | D |
| 395 | E | D | C | A | B | B | D |
| 396 | E | D | C | A | D | D | D |
| 397 | E | D | B | A | B | D | D |
| 398 | E | D | A | A | D | C | D |
| 399 | E | D | D | B | D | D | D |
| 400 | E | D | D | D | D | D | D |
| 401 | E | D | D | A | D | D | D |
| 402 | E | B | A | A | D | B | D |
| 403 | E | C | A | A | D | B | D |
| 404 | E | D | B | D | D | B | D |
| 405 | E | B | B | B | D | A | D |
| 406 | E | C | C | A | D | B | D |
| 407 | E | C | D | D | D | B | D |
| 408 | E | A | A | A | A | A | A |
| 409 | E | A | A | A | A | A | B |
| 410 | E | D | D | D | D | C | D |
| 411 | E | D | D | D | D | D | D |
| 412 | E | D | D | C | D | D | D |
| 413 | E | D | D | D | D | D | B |
| 414 | E | A | A | A | A | A | A |
| 415 | E | A | A | A | A | A | A |
| 416 | E | A | A | D | C | A | D |
| 417 | E | A | A | A | A | A | B |
| 418 | E | B | A | C | C | B | D |
| 419 | E | A | A | A | B | A | D |
| 420 | E | A | A | D | C | A | D |
| 421 | E | A | A | A | A | A | D |
| 422 | E | A | A | D | B | A | D |
| 423 | E | A | A | A | A | A | B |
| 424 | E | C | A | C | D | D | D |
| 425 | E | A | D | D | B | A | D |
| 426 | E | A | C | D | D | B | D |
| 427 | E | A | C | D | B | A | D |
| 428 | E | A | D | C | B | A | D |
| 429 | E | A | A | D | B | A | D |
| 430 | E | B | C | D | B | A | D |
| 431 | E | A | A | A | B | A | C |
| 432 | E | A | A | A | A | A | B |
| 433 | E | A | A | A | A | A | B |
| 434 | E | A | A | A | B | B | D |
| 435 | E | D | B | D | D | D | D |
| 436 | E | D | D | D | D | B | D |
| 437 | E | C | D | D | D | D | D |
| 438 | E | A | A | B | B | A | D |
| 439 | E | A | A | B | A | A | C |
| 440 | E | A | A | D | D | A | D |
| 441 | E | D | A | A | A | D | B |
| 442 | E | A | A | C | A | A | D |
| 443 | E | A | A | B | D | A | D |
| 444 | E | A | A | B | D | A | D |
| 445 | E | A | A | D | D | A | D |
| 446 | E | A | A | D | C | A | D |
| 447 | E | D | D | D | D | C | D |
| 448 | E | D | D | D | D | D | D |
| 449 | E | E | E | E | E | E | E |
| 450 | E | A | A | A | A | A | C |
| 451 | E | A | A | A | A | A | C |
| 452 | E | D | D | D | D | D | D |
| 453 | E | D | D | D | D | D | D |
| 454 | E | A | A | C | B | A | C |
| 455 | E | A | A | D | C | A | D |
| 456 | E | A | A | A | B | A | D |
| 457 | E | A | A | A | A | A | D |
| 458 | E | B | A | C | D | A | D |
| 459 | E | D | A | C | C | A | C |
| 460 | E | A | A | D | D | A | D |
| 461 | E | A | A | D | D | A | D |
| 462 | E | A | A | D | D | A | D |
| 463 | E | A | A | D | C | A | C |
| 464 | E | A | A | B | D | A | D |
| 465 | E | A | A | A | C | A | C |
| 466 | E | A | A | A | D | A | D |
| 467 | E | A | A | A | D | A | B |
| 468 | E | A | A | A | B | A | D |
| 469 | E | A | A | A | A | A | A |
| 470 | E | A | A | A | A | A | A |
| 471 | E | A | A | B | C | A | B |
| 472 | E | A | A | C | D | A | C |
| 473 | E | A | A | A | D | A | B |
| 474 | E | A | A | A | B | A | D |
| 475 | E | A | A | A | A | A | D |
| 476 | E | A | A | A | A | A | A |
| 477 | E | A | A | A | A | A | B |
| 478 | E | A | A | A | A | A | D |
| 479 | E | A | A | A | A | A | D |
| 480 | E | A | A | A | A | A | B |
| 481 | E | B | A | A | D | A | B |
| 482 | E | B | C | C | D | C | D |
| 483 | E | A | A | D | D | A | D |
| 484 | E | B | A | C | D | B | D |
| 485 | E | B | A | C | D | B | D |
| 486 | E | A | A | B | D | A | D |
| 487 | E | A | A | D | D | A | D |
| 488 | E | C | A | B | D | B | D |
| 489 | E | A | A | D | D | A | D |
| 490 | E | D | D | D | D | C | D |
| 491 | E | D | B | D | D | C | D |
| 492 | E | A | B | D | D | A | D |
| 493 | E | A | A | A | C | A | B |
| 494 | E | A | A | A | A | A | B |
| 495 | E | A | A | A | A | A | A |
| 496 | C | A | A | A | A | A | B |
| 497 | E | D | A | B | D | C | D |
| 498 | E | D | A | D | D | C | D |
| 499 | E | D | A | B | B | B | D |
| 500 | E | A | A | C | C | A | D |
| 501 | C | A | A | A | A | A | D |
| 502 | E | D | B | D | D | D | D |
| 503 | E | D | D | C | D | D | D |
| 504 | E | A | A | D | D | A | D |
| 505 | E | D | D | A | B | D | C |
| 506 | E | D | C | D | D | D | D |
| 507 | E | A | A | A | A | A | D |
| 508 | E | A | A | A | A | A | D |
| 509 | E | A | A | A | A | A | D |
| 510 | E | A | A | A | A | A | D |
| 511 | E | A | A | A | A | A | D |
| 512 | E | A | A | A | A | A | D |
| 513 | E | C | A | D | D | B | D |
| 514 | E | A | A | D | D | A | D |
| 515 | E | A | E | E | E | A | E |
| 516 | E | A | A | A | A | A | D |
| 517 | E | A | A | B | A | A | D |
| 518 | E | A | A | D | D | A | D |
| 519 | E | A | A | A | A | A | D |
| 520 | E | A | A | D | D | A | D |
| 521 | E | A | A | A | A | A | B |
| 522 | E | A | A | D | B | A | D |
| 523 | E | D | D | D | D | D | D |
| 524 | E | A | E | E | E | A | E |
| 525 | E | A | A | D | C | A | D |
| 526 | E | A | A | D | C | A | D |
| 527 | E | A | A | A | A | A | C |
| 528 | E | A | A | A | A | A | A |
| 530 | E | D | D | D | D | C | D |
| 531 | E | A | A | B | D | A | D |
| 532 | E | A | A | C | D | A | D |
| 533 | E | A | A | B | D | A | D |
| 534 | E | A | A | D | D | A | D |
| 535 | E | A | A | B | D | A | D |
| 536 | E | A | A | D | D | A | D |
| 537 | E | E | E | E | E | E | E |
| 538 | E | E | E | E | E | E | E |
| 539 | E | E | E | E | E | E | E |
| 540 | E | B | B | D | D | A | D |
| 541 | E | C | D | D | D | A | D |
| 542 | E | C | D | C | D | B | D |
| 543 | E | A | A | A | C | A | D |
| 544 | E | A | A | D | D | A | D |
| 545 | E | A | A | D | D | A | D |
| 546 | E | A | A | D | D | A | D |
| 547 | E | A | A | B | D | A | D |
| 548 | E | A | A | D | D | A | D |
| 549 | E | A | A | D | D | A | C |
| 550 | E | A | A | C | D | A | D |
| 551 | E | A | A | D | D | A | D |
| 552 | E | A | A | B | D | A | D |
| 553 | E | A | A | D | D | A | D |
| 554 | E | A | A | B | C | A | D |
| 555 | E | A | A | D | D | A | D |
| 556 | E | A | A | C | D | A | D |
| 557 | E | E | E | E | E | E | E |
| 558 | E | D | D | D | D | B | D |
| 559 | E | A | B | D | D | A | D |
| 560 | E | A | A | A | A | A | B |
| 561 | E | A | A | A | A | A | D |
| 562 | E | A | A | A | B | A | C |
| 563 | E | A | A | A | A | A | B |

| | | | | | | | |
|---|---|---|---|---|---|---|---|
| *Cmpd. No. - Compound Number *COCHSA - Spot Blotch of Barley (*Cochliobolus sativus*) *PHAKPA - Asian Soybean Rust (*Phakopsora pachyrhizi*) *PUCCRT - Wheat Brown Rust (*Puccinia triticina*) *RHYNSE - Leaf Blotch of Barley (*Rhynochosporium commune*) *SEPTTR - Septoria Leaf Blotch of Wheat (*Zymoseptoria tritici*) *1DP - 1 Day Protectant *3DC - 3 Day Curative *ppm - Parts Per Million | | | | | | | |

## Claims

1. A compound of Formula I: 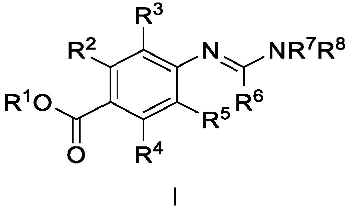 wherein
R¹ is selected from the group consisting of C₁-C₈ alkyl, C₁-C₈ substituted alkyl, C₂-C₈ alkenyl, C₂-C₈ substituted alkenyl, C₂-C₈ alkynyl, C₂-C₈ substituted alkynyl, C₃-C₈ cycloalkyl, C₃-C₈ substituted cycloalkyl, C₃-C₈ heterocycloalkyl, C₃-C₈ substituted heterocycloalkyl, C₅-C₇ heteroaryl, Cs-C₇ substituted heteroaryl, phenyl, substituted phenyl, benzyl, and substituted benzyl;
each R², R⁴, and R⁶ independently is selected from the group consisting of hydrogen, halogen, cyano, nitro, C₁-C₈ alkyl, C₁-C₈ substituted alkyl, C₂-C₈ alkenyl, C₂-C₈ substituted alkenyl, C₂-C₈ alkynyl, C₂-C₈ substituted alkynyl, C₁-C₈ alkoxy, and C₁-C₈ substituted alkoxy;
R³ is selected from the group consisting of consisting of halogen, cyano, C₁-C₈ alkyl, C₁-C₈ substituted alkyl, C₂-C₈ alkenyl, C₂-C₈ substituted alkenyl, C₂-C₈ alkynyl, C₂-C₈ substituted alkynyl, C₁-C₈ alkoxy, and C₁-C₈ substituted alkoxy;
R⁶ is selected from the group consisting of hydrogen, C₁-C₈ alkyl, C₁-C₈ substituted alkyl, C₂-C₈ alkenyl, C₂₋-C₈ substituted alkenyl, C₂-C₈ alkynyl, C₂-C₈ substituted alkynyl, C₁-C₈ alkoxy, C₁-C₈ substituted alkoxy, thiol, alkylthio, and substituted alkylthio;
or R⁶ and R⁷ may be covalently bonded together to form a saturated or unsaturated C₃-C₈ heterocycloalkyl or C₃-C₈ substituted heterocycloalkyl group;
each R⁷ and R⁸ independently is selected from the group consisting of C₁-C₈ alkyl, C₁-C₈ substituted alkyl, C₂-C₈ alkenyl, C₂-C₈ substituted alkenyl, C₂-C₈ alkynyl, C₂-C₈ substituted alkynyl, C₃-C₈ cycloalkyl, C₃-C₈ substituted cycloalkyl, benzyl, and substituted benzyl;
or R⁷ and R⁸ may be covalently bonded together to form a saturated or unsaturated C₃-C₈ heterocycloalkyl or C₃-C₈ substituted heterocycloalkyl group;
wherein any and all heterocyclic rings may contain up to three heteroatoms selected from the group consisting of O, N, and S;
or a tautomer or salt thereof,
with the proviso that the compound is not ethyl 4-(((dimethylamino)methylene)amino)-3,5-dimethylbenzoate hydrochloride, or methyl 3-bromo-4-[(*E*)-dimethylaminomethylideneamino]benzoate.

2. The compound of claim 1, wherein R¹ is selected from the group consisting of C₁-C₈ alkyl, C₁-C₈ substituted alkyl, C₂-C₈ alkynyl, C₂-C₈ substituted alkynyl, C₃-C₈ cycloalkyl, C₃-C₈ substituted cycloalkyl, phenyl, substituted phenyl, benzyl, and substituted benzyl, preferably wherein R¹ is selected from the group consisting of benzyl and substituted benzyl.

3. The compound of claims 1 or 2, wherein R² and R⁵ are both hydrogen.

4. The compound of claim 3, wherein R³ and R⁴ are independently selected from the group consisting of halogen, C₁-C₈ alkyl, C₁-C₈ substituted alkyl, and C₁-C₈ alkoxy, preferably wherein R³ and R⁴ are both CH₃.

5. The compound of claims 1 or 2, wherein R⁴ and R⁵ are both hydrogen.

6. The compound of claim 5, wherein R² and R³ are independently selected from the group consisting of halogen, C₁-C₈ alkyl, C₁-C₈ substituted alkyl, and C₁-C₈ alkoxy, preferably wherein R² and R³ are both CH₃.

7. The compound of any one of claims 1-6, wherein each R⁷ and R⁸ independently is selected from the group consisting of C₁-C₈ alkyl, C₁-C₈ substituted alkyl, C₂-C₈ alkenyl, C₃-C₈ cycloalkyl, benzyl, and substituted benzyl; and/or
wherein R⁶ is selected from the group consisting of hydrogen, C₁-C₈ alkyl, C₁-C₈ substituted alkyl, thiol, alkylthio, and substituted alkylthio.

8. A compound according to any one of the preceding claims that is selected from the following table:
| **Cmpd. No.** | **Structure** |
|---|---|
| 1 | 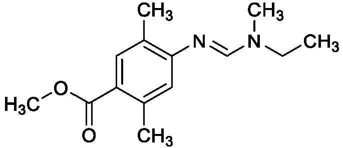 |
| 3 | 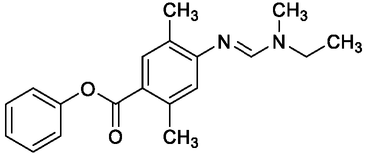 |
| 4 | 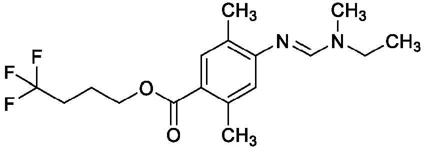 |
| 5 | 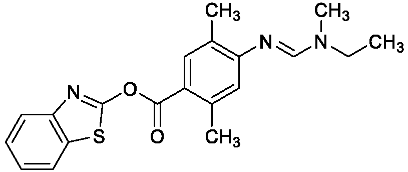 |
| 6 | 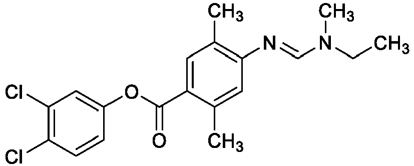 |
| 7 | 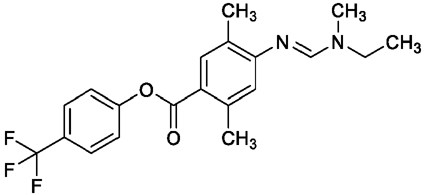 |
| 8 | 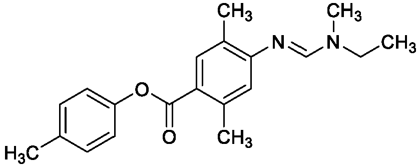 |
| 9 | 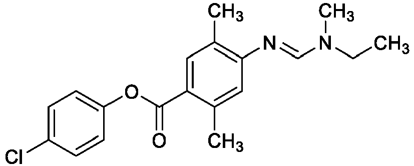 |
| 10 | 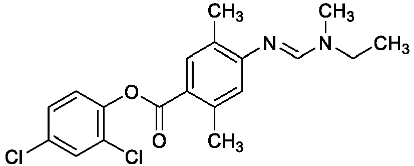 |
| 11 | 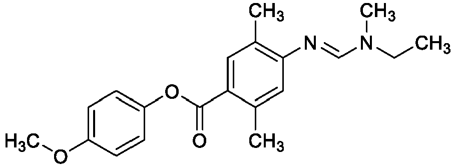 |
| 12 | 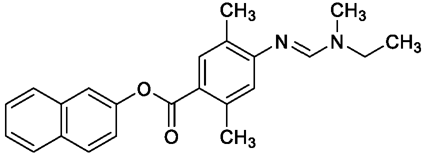 |
| 13 | 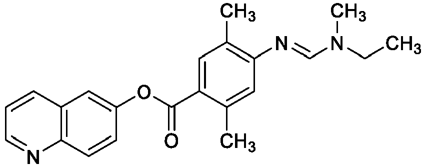 |
| 14 | 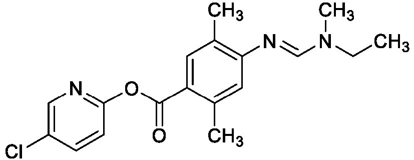 |
| 15 | 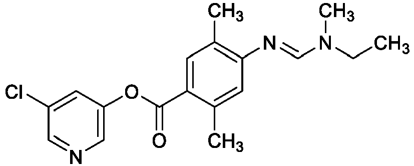 |
| 16 | 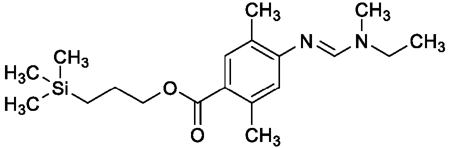 |
| 17 | 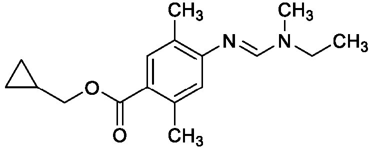 |
| 18 | 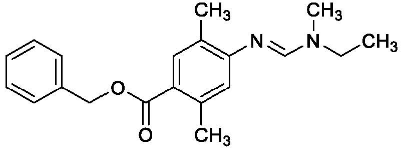 |
| 19 | 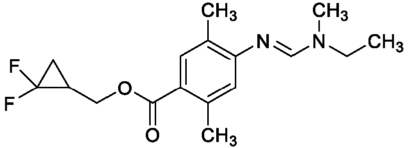 |
| 20 | 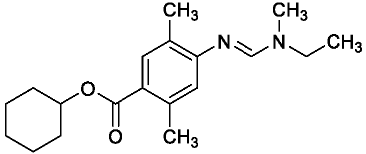 |
| 21 | 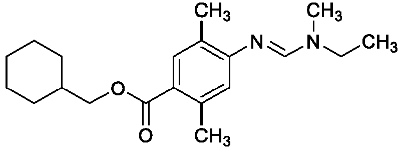 |
| 22 | 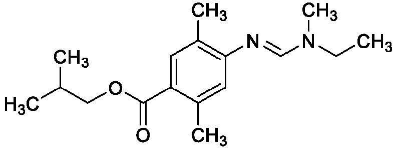 |
| 23 | 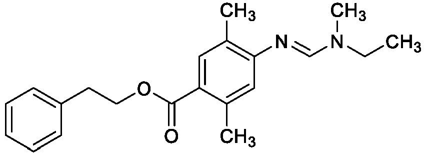 |
| 24 | 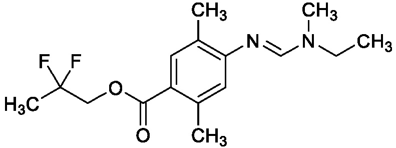 |
| 25 | 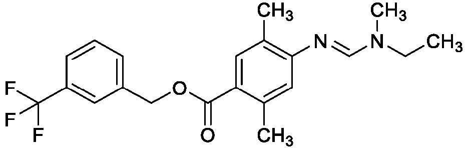 |
| 26 | 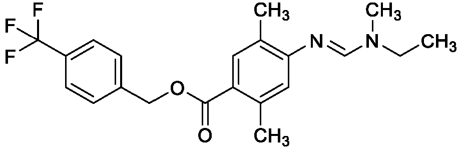 |
| 27 | 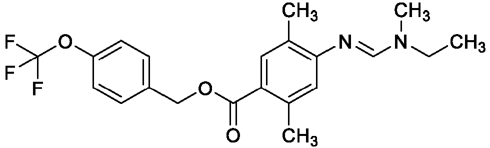 |
| 28 | 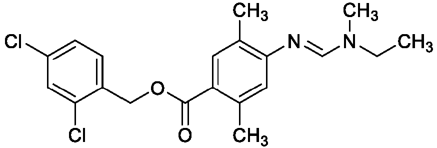 |
| 29 | 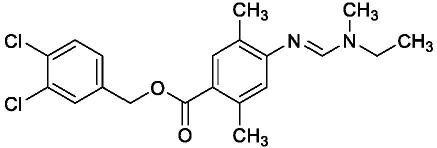 |
| 30 | 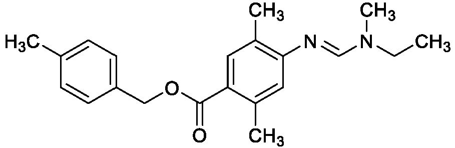 |
| 31 | 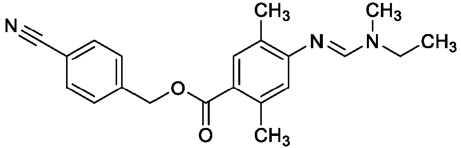 |
| 32 | 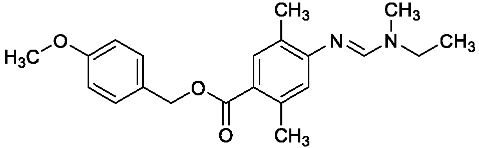 |
| 33 | 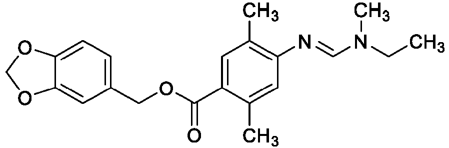 |
| 34 | 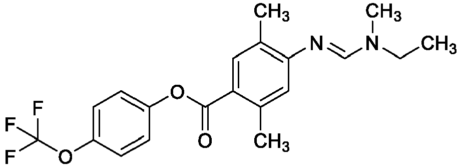 |
| 35 | 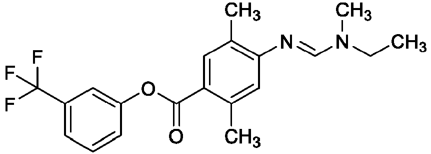 |
| 36 | 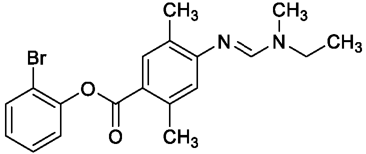 |
| 37 | 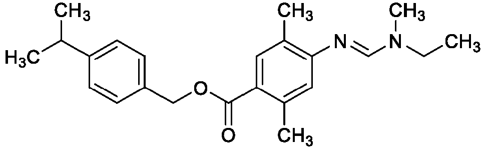 |
| 38 | 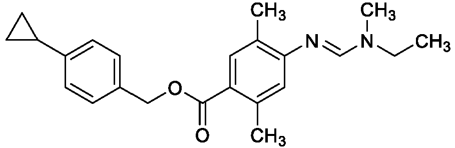 |
| 39 | 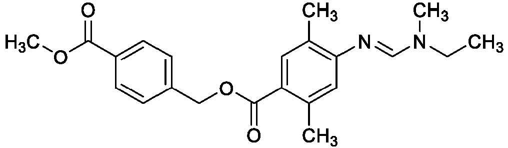 |
| 40 | 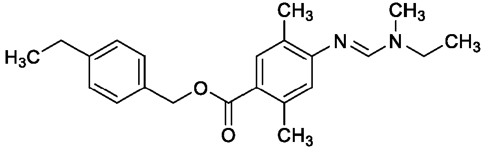 |
| 41 | 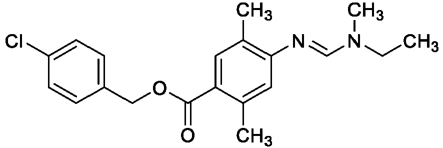 |
| 42 | 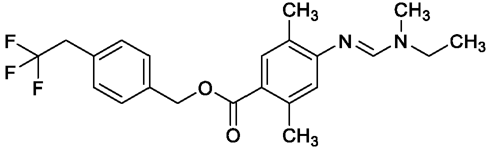 |
| 43 | 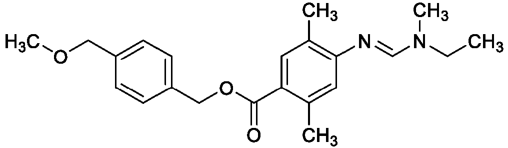 |
| 44 | 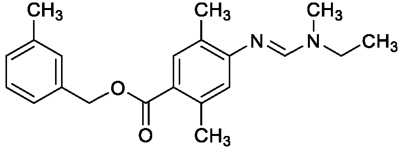 |
| 45 | 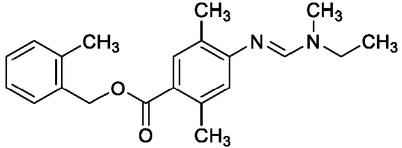 |
| 46 | 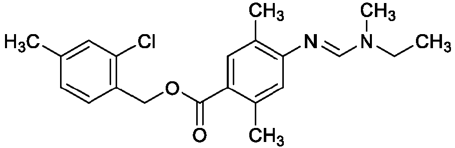 |
| 47 | 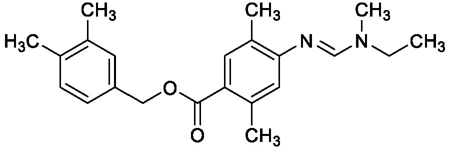 |
| 48 | 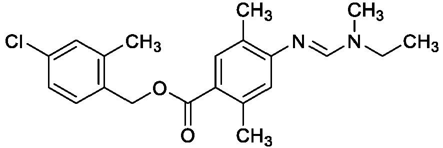 |
| 49 | 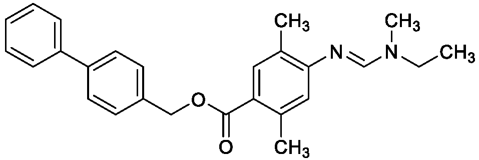 |
| 50 | 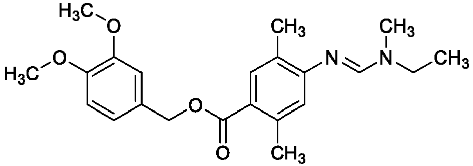 |
| 51 | 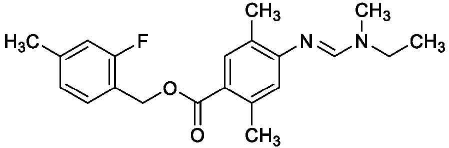 |
| 52 | 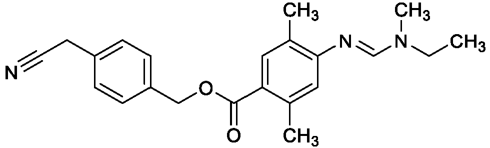 |
| 53 | 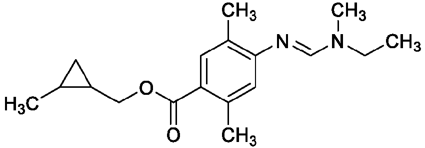 |
| 54 | 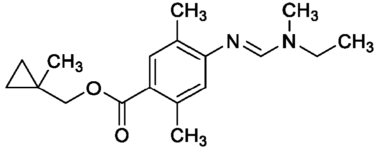 |
| 55 | 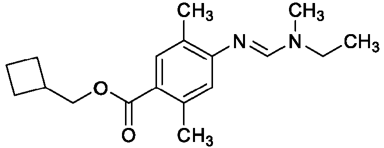 |
| 56 | 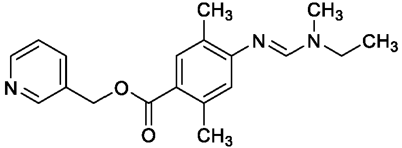 |
| 57 | 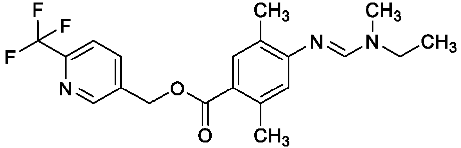 |
| 58 | 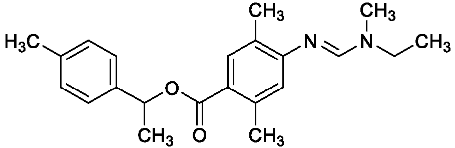 |
| 59 | 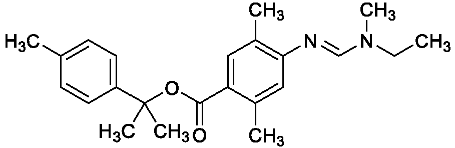 |
| 60 | 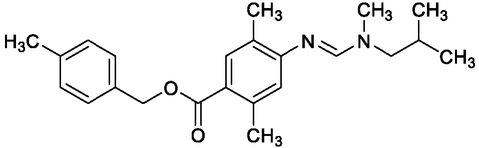 |
| 61 | 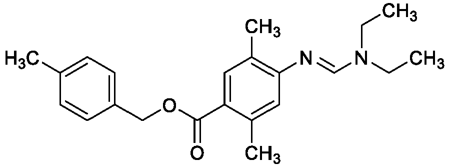 |
| 62 | 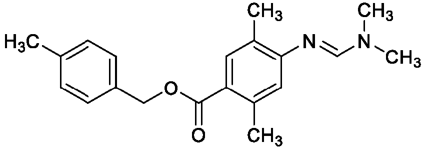 |
| 63 | 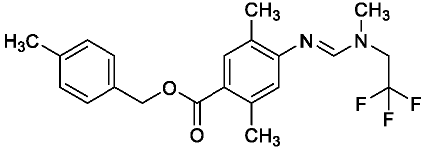 |
| 64 | 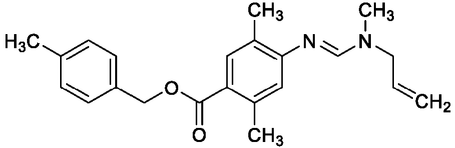 |
| 65 | 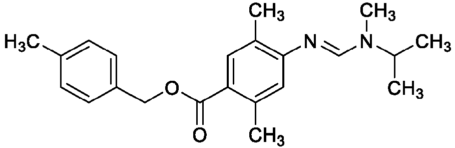 |
| 66 | 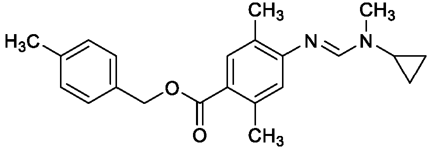 |
| 67 | 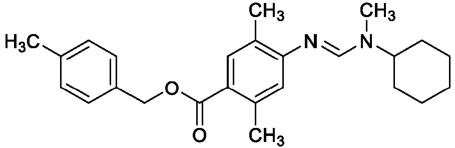 |
| 68 | 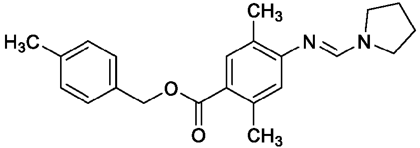 |
| 69 | 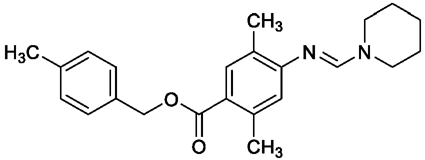 |
| 70 | 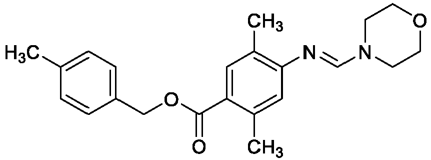 |
| 72 | 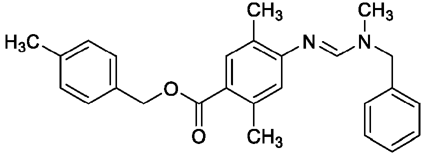 |
| 73 | 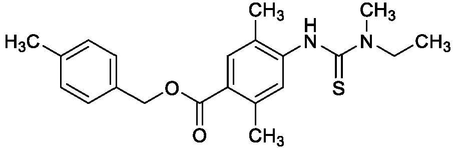 |
| 74 | 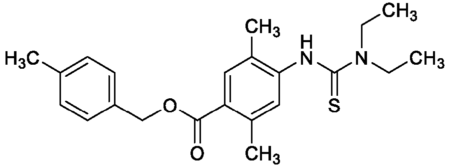 |
| 75 | 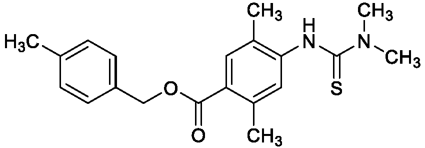 |
| 76 | 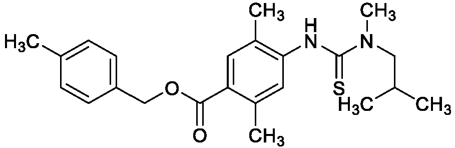 |
| 77 | 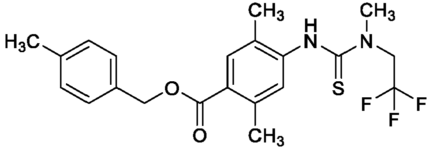 |
| 78 | 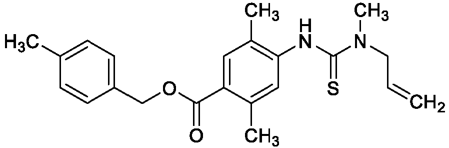 |
| 79 | 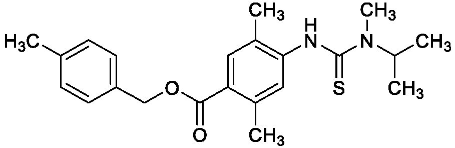 |
| 80 | 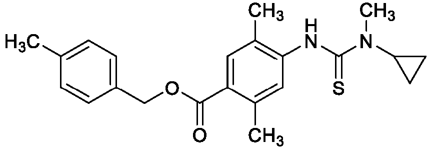 |
| 81 | 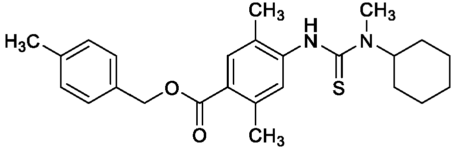 |
| 82 | 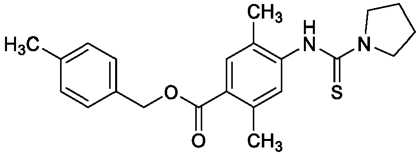 |
| 83 | 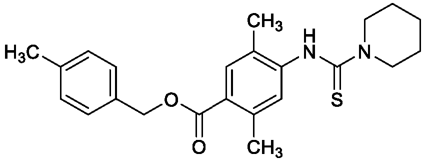 |
| 84 | 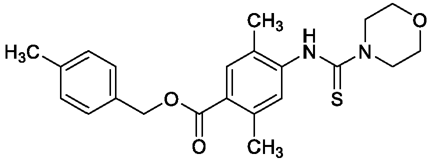 |
| 86 | 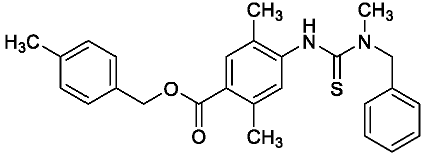 |
| | |
| | |
| 89 | 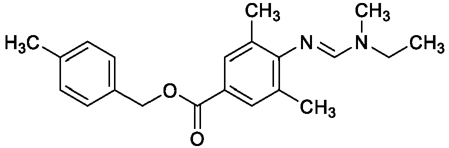 |
| 90 | 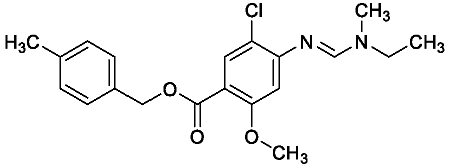 |
| 91 | 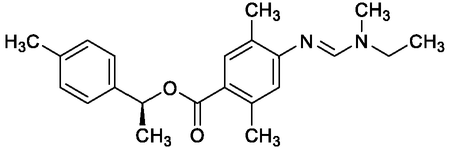 |
| 92 | 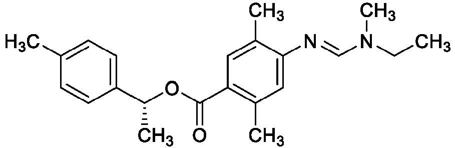 |
| | |
| 94 | 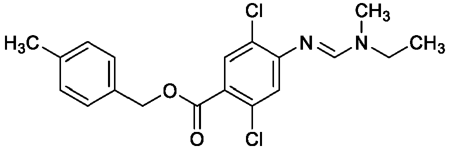 |
| 95 | 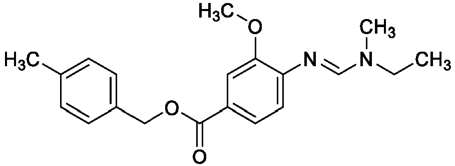 |
| 96 | 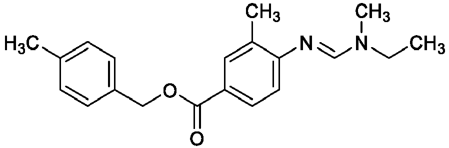 |
| 97 | 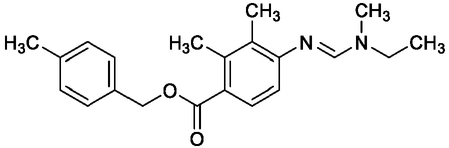 |
| 98 | 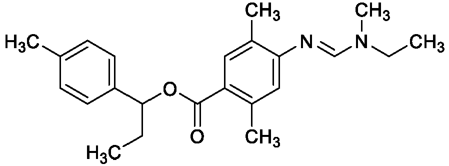 |
| 99 | 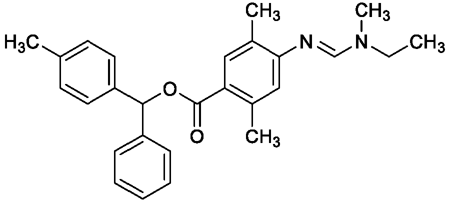 |
| 100 | 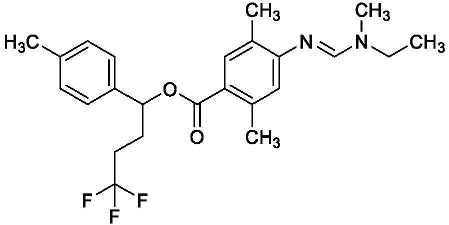 |
| 101 | 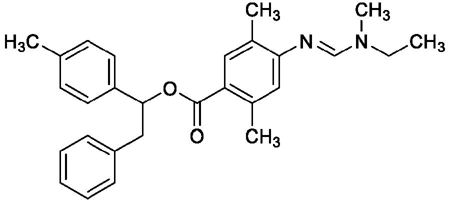 |
| | |
| 103 | 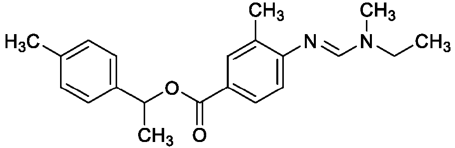 |
| 105 | 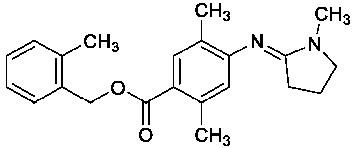 |
| 106 | 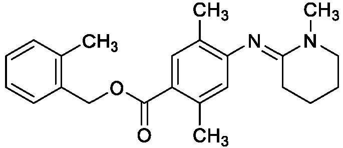 |
| 107 | 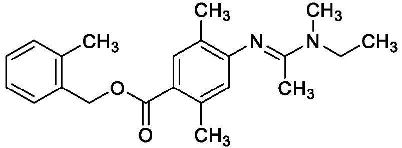 |
| 109 | 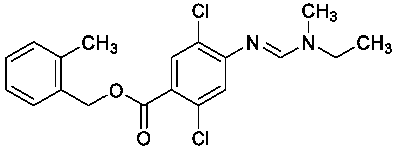 |
| 110 | 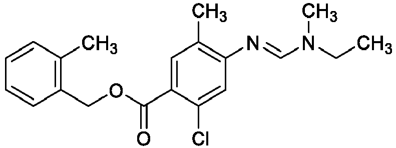 |
| 111 | 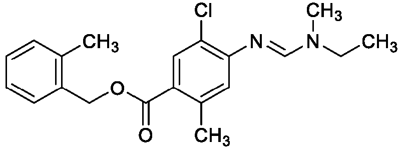 |
| 112 | 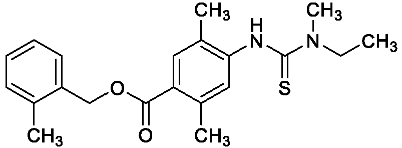 |
| 113 | 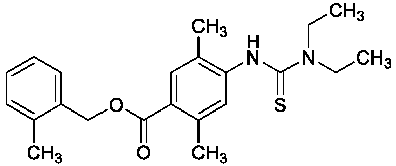 |
| 114 | 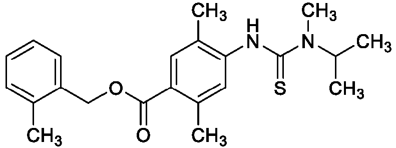 |
| 115 | 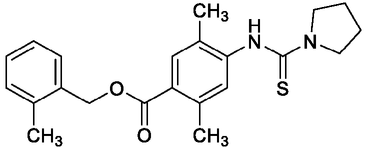 |
| 116 | 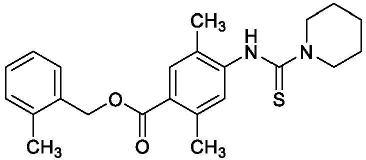 |
| 117 | 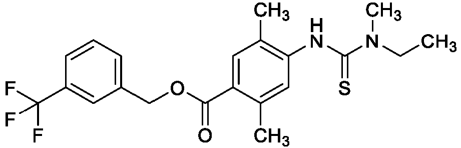 |
| 118 | 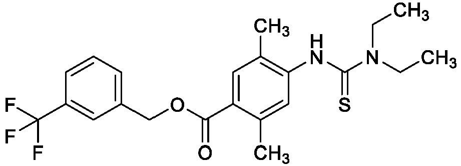 |
| 119 | 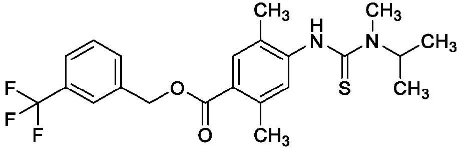 |
| 120 | 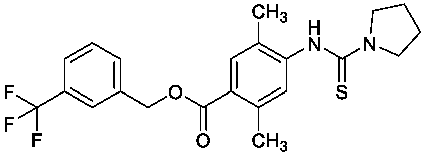 |
| 121 | 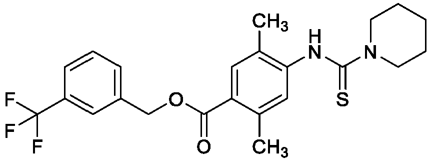 |
| 122 | 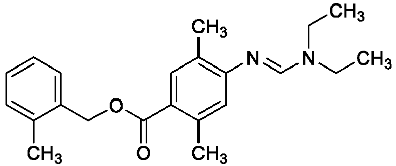 |
| 123 | 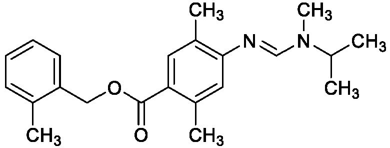 |
| 124 | 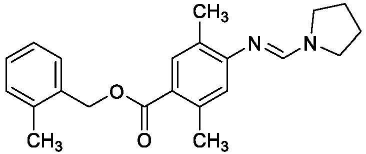 |
| 125 | 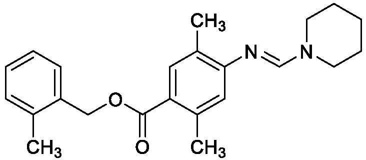 |
| 126 | 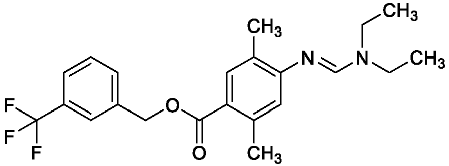 |
| 127 | 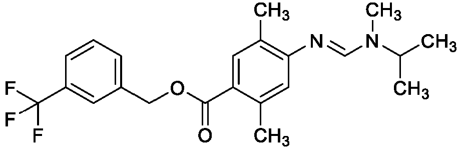 |
| 128 | 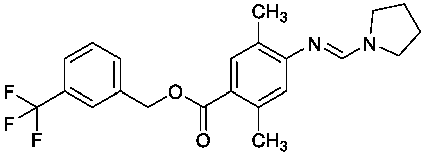 |
| 129 | 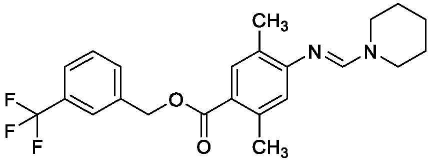 |
| 130 | 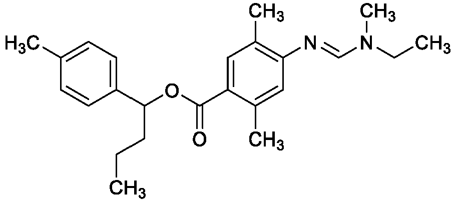 |
| 131 | 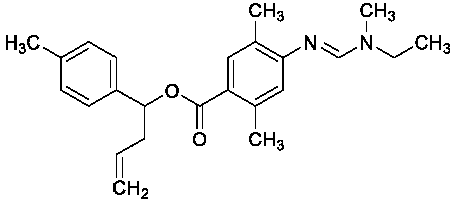 |
| 132 | 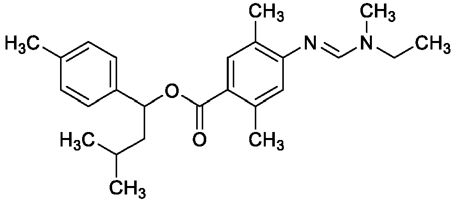 |
| 133 | 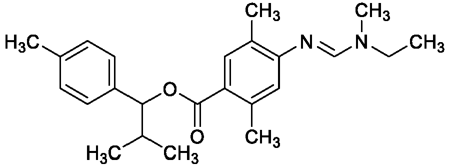 |
| 134 | 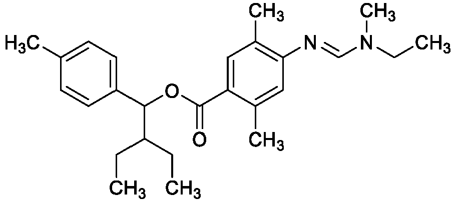 |
| 135 | 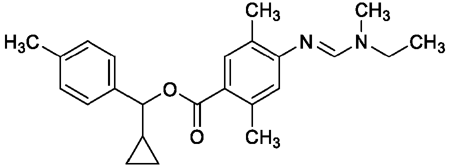 |
| 136 | 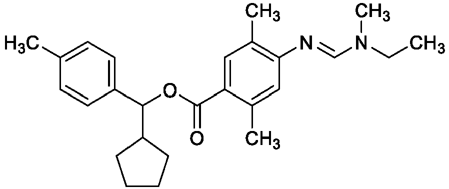 |
| 137 | 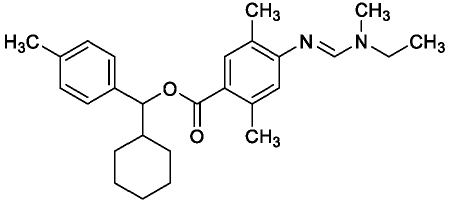 |
| 138 | 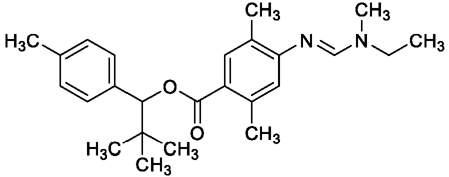 |
| 139 | 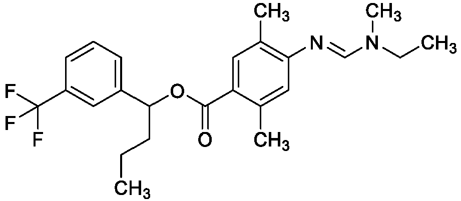 |
| 140 | 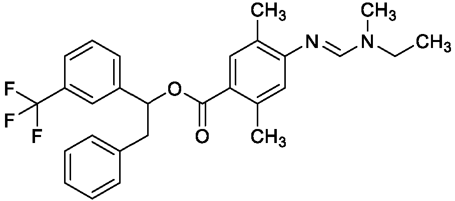 |
| 141 | 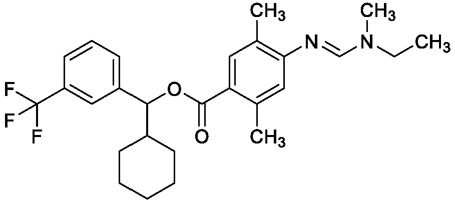 |
| 142 | 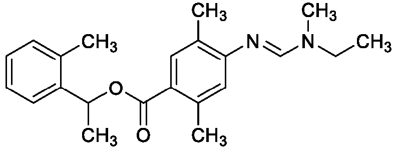 |
| 143 | 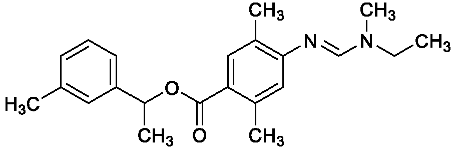 |
| 144 | 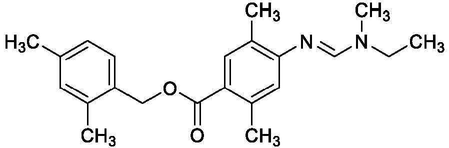 |
| 145 | 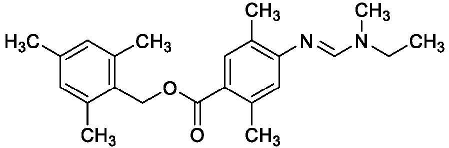 |
| 146 | 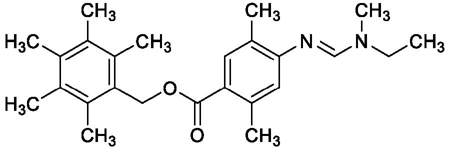 |
| 147 | 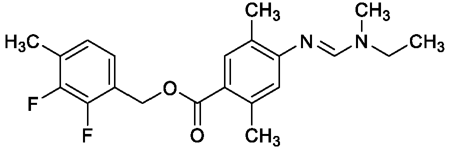 |
| 148 | 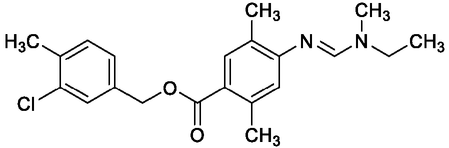 |
| 149 | 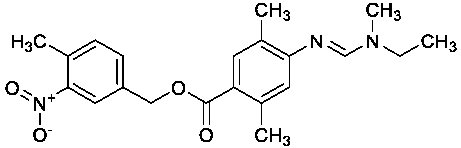 |
| 150 | 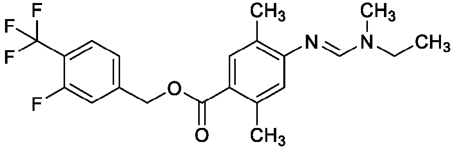 |
| 151 | 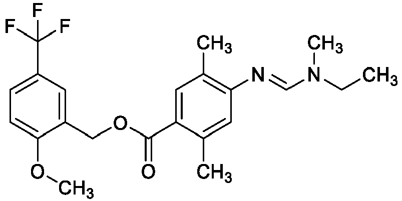 |
| 152 | 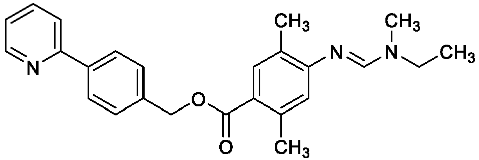 |
| 153 | 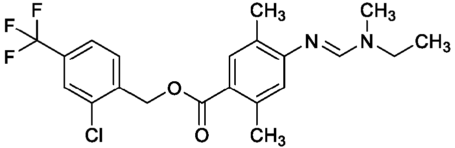 |
| 154 | 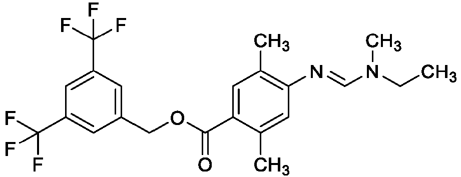 |
| 155 | 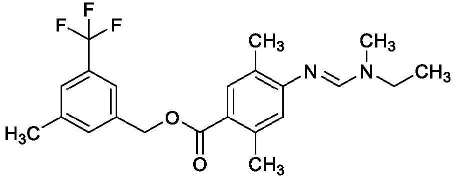 |
| 156 | 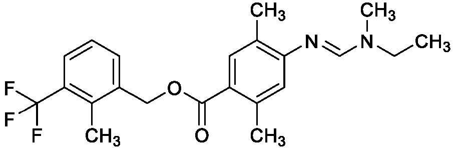 |
| 157 | 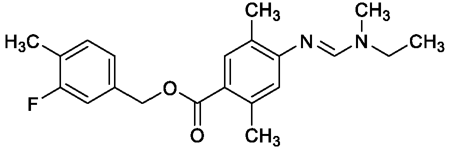 |
| 158 | 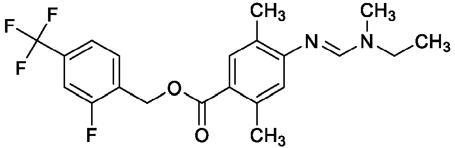 |
| 159 | 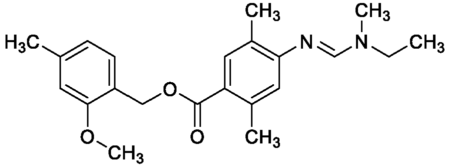 |
| 160 | 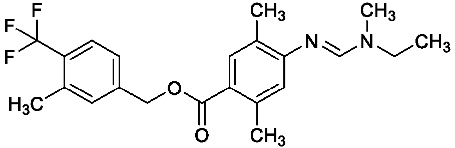 |
| 161 | 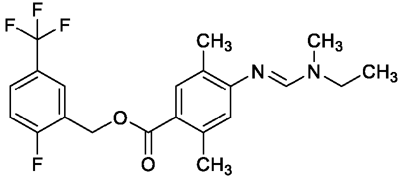 |
| 162 | 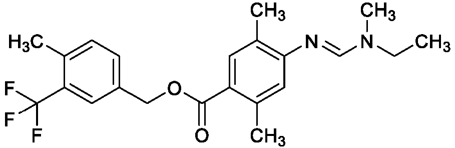 |
| 163 | 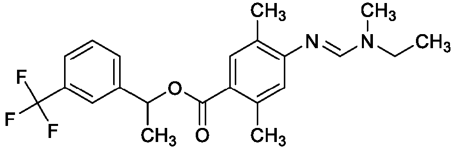 |
| 164 | 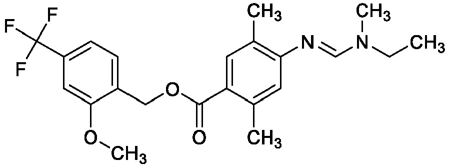 |
| 165 | 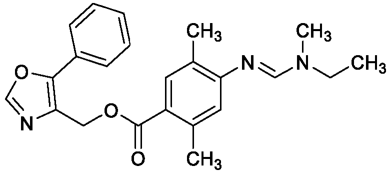 |
| 166 | 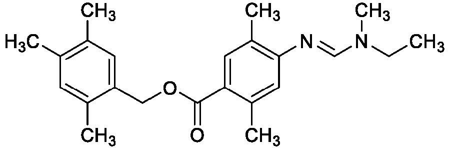 |
| 167 | 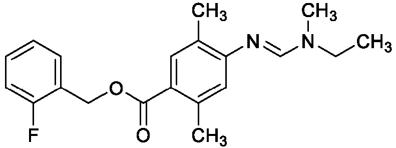 |
| 168 | 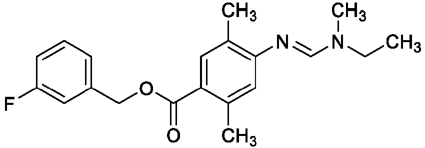 |
| 169 | 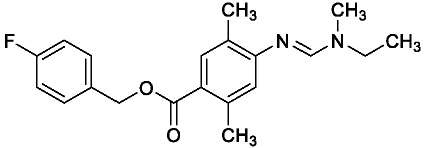 |
| 170 | 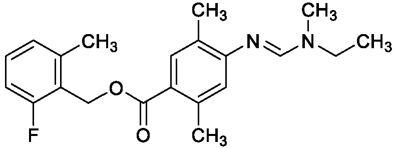 |
| 171 | 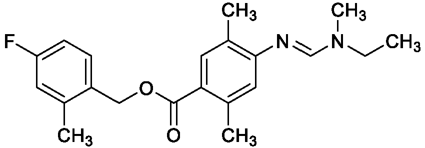 |
| 172 | 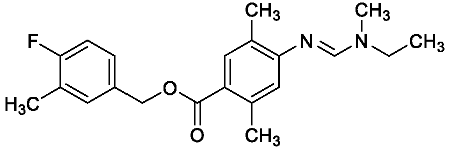 |
| 173 | 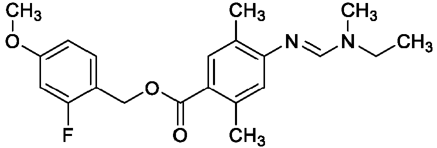 |
| 174 | 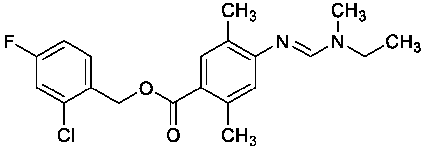 |
| 175 | 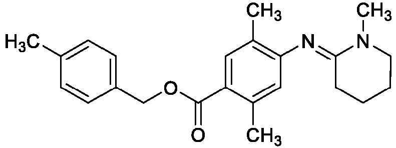 |
| 176 | 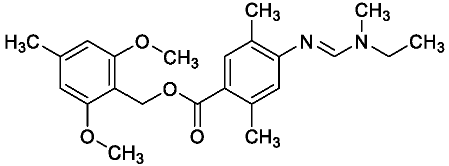 |
| 177 | 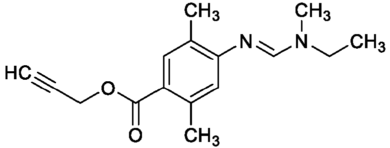 |
| 178 | 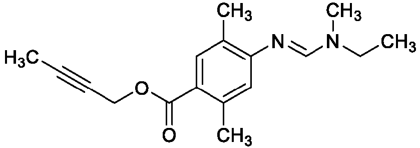 |
| 179 | 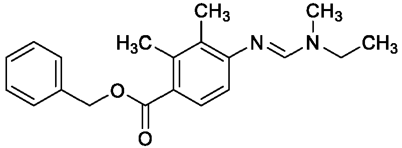 |
| 180 | 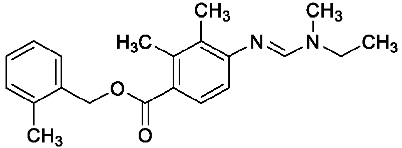 |
| 181 | 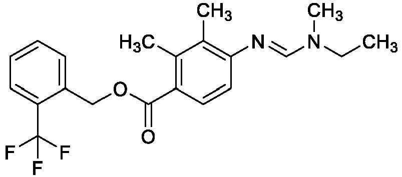 |
| 182 | 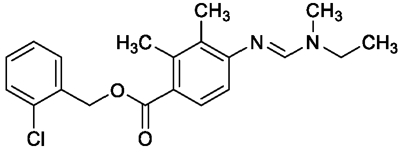 |
| 183 | 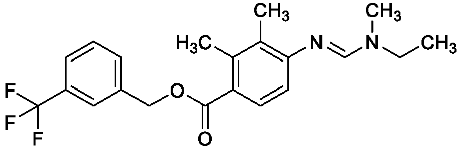 |
| 184 | 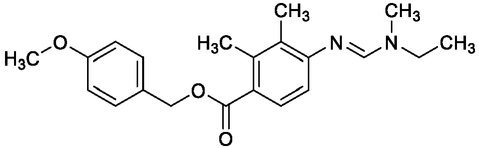 |
| 185 | 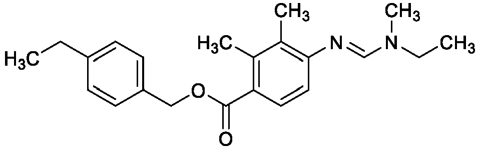 |
| 186 | 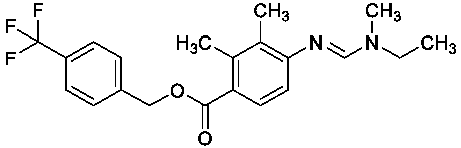 |
| 187 | 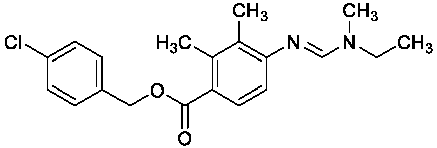 |
| 188 | 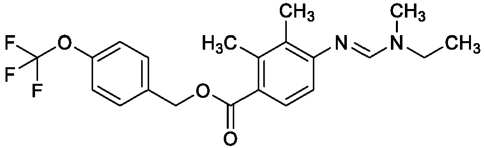 |
| 189 | 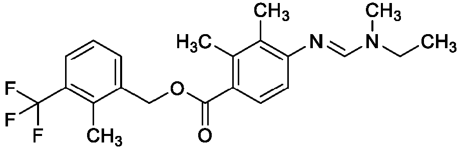 |
| 190 | 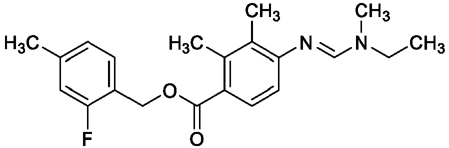 |
| 191 | 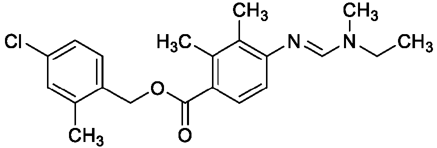 |
| 192 | 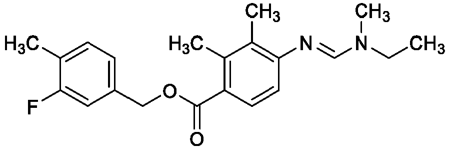 |
| 193 | 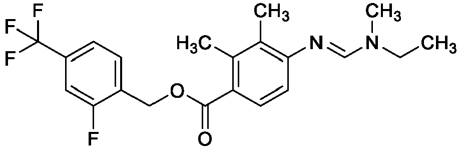 |
| 194 | 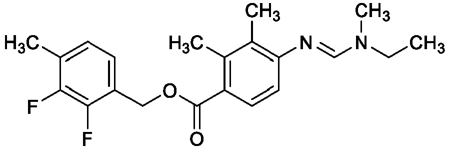 |
| 195 | 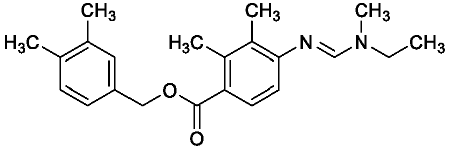 |
| 196 | 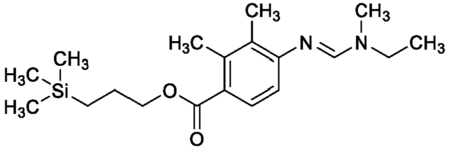 |
| 197 | 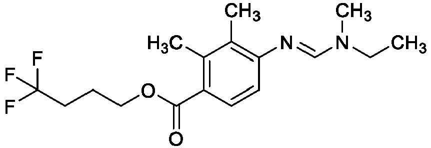 |
| 198 | 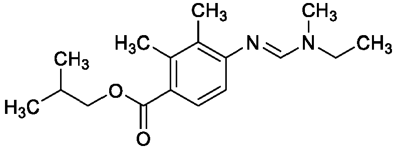 |
| 199 | 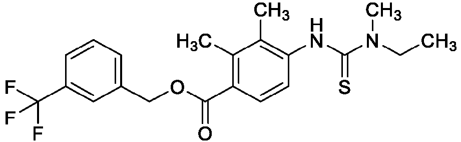 |
| 200 | 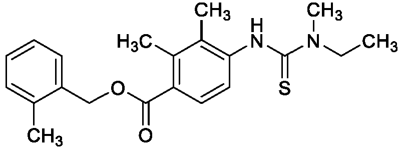 |
| 201 | 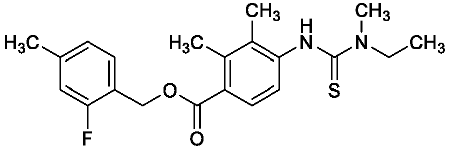 |
| 202 | 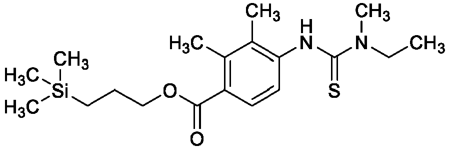 |
| 203 | 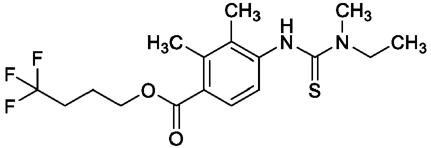 |
| 204 | 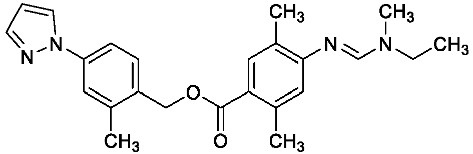 |
| 205 | 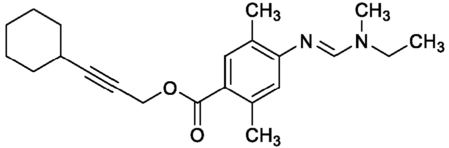 |
| 206 | 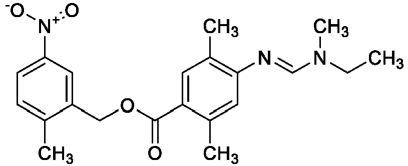 |
| 207 | 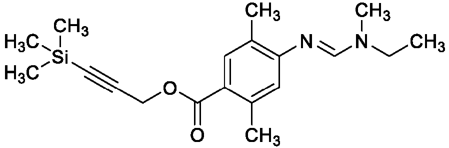 |
| 208 | 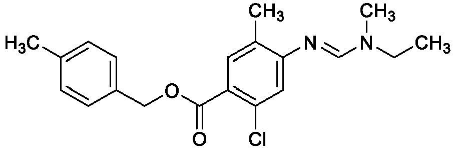 |
| 209 | 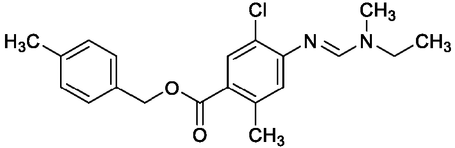 |
| 210 | 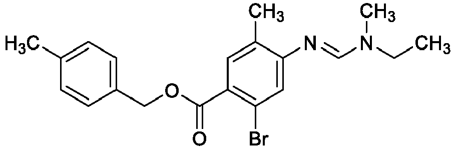 |
| | |
| 212 | 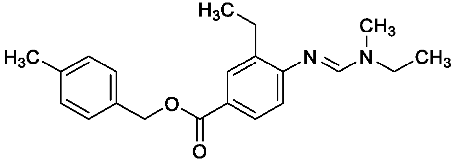 |
| 213 | 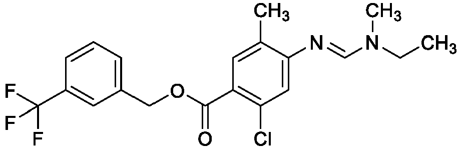 |
| 214 | 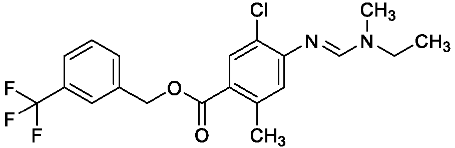 |
| 215 | 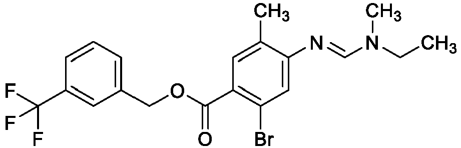 |
| | |
| 217 | 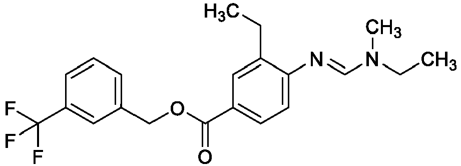 |
| 218 | 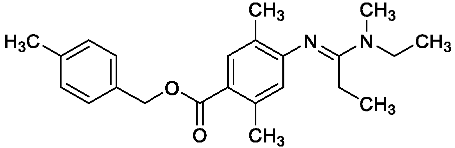 |
| 219 | 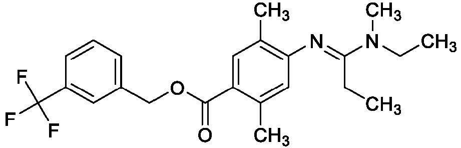 |
| 220 | 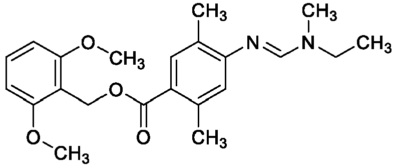 |
| 221 | 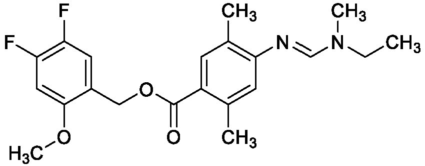 |
| 222 | 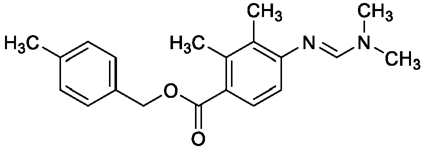 |
| 223 | 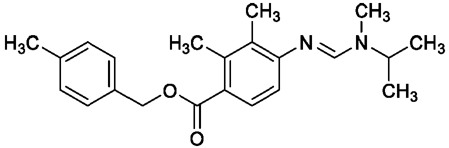 |
| 224 | 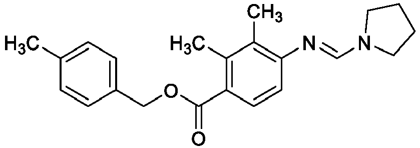 |
| 225 | 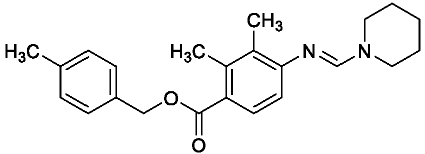 |
| 226 | 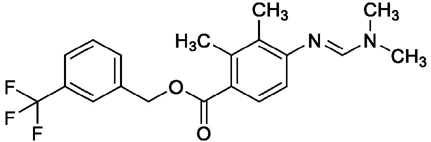 |
| 227 | 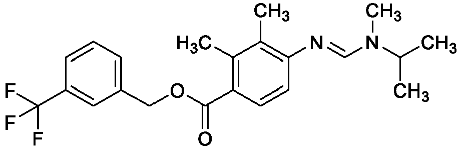 |
| 228 | 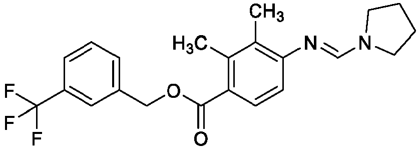 |
| 229 | 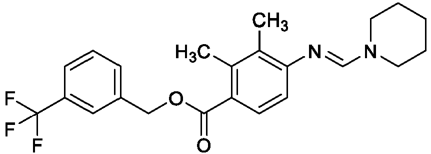 |
| 230 | 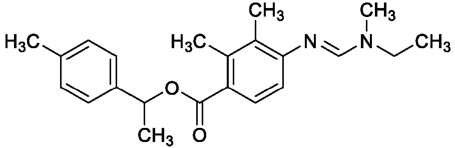 |
| 231 | 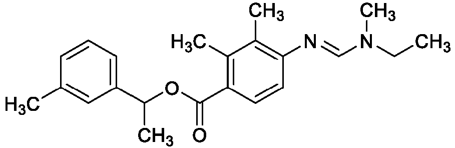 |
| 232 | 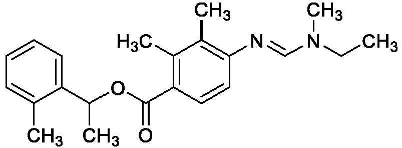 |
| 233 | 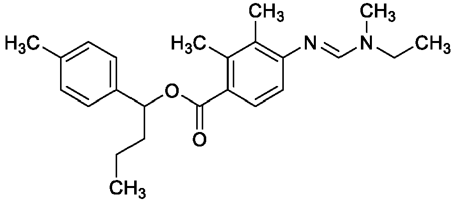 |
| 234 | 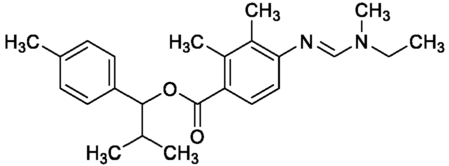 |
| 235 | 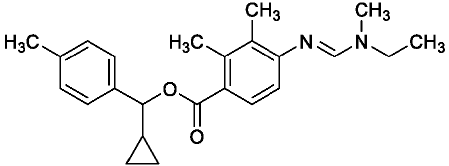 |
| 236 | 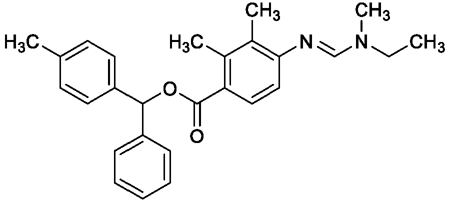 |
| 237 | 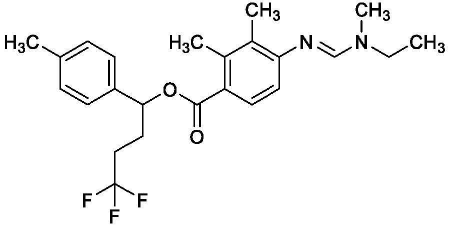 |
| 238 | 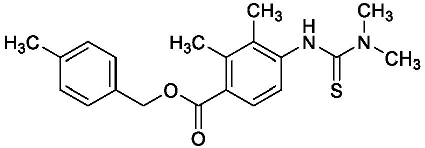 |
| 239 | 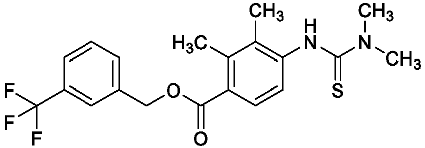 |
| 240 | 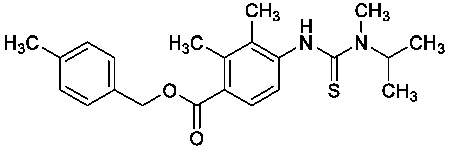 |
| 241 | 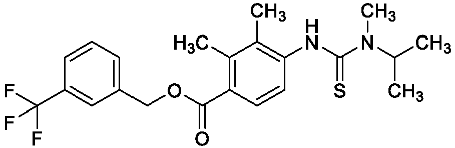 |
| 242 | 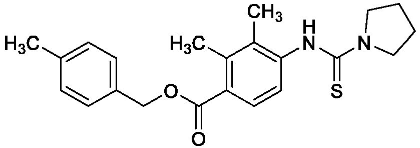 |
| 243 | 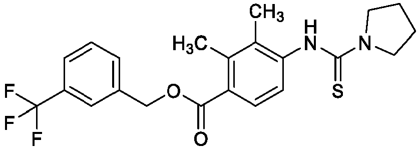 |
| 244 | 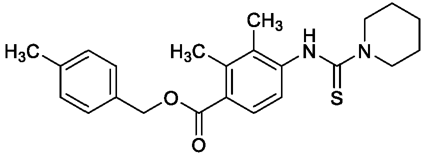 |
| 245 | 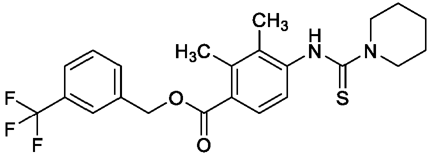 |
| 246 | 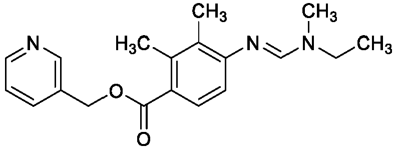 |
| 247 | 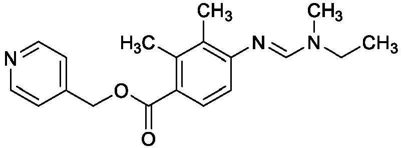 |
| 248 | 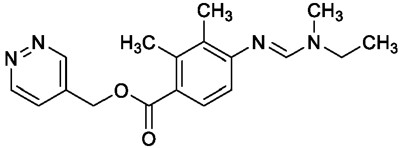 |
| 249 | 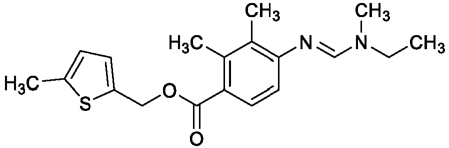 |
| 250 | 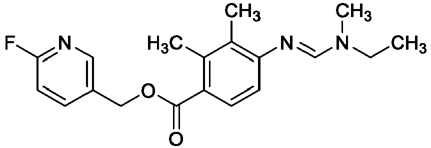 |
| 251 | 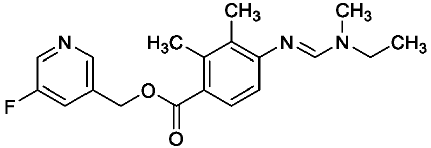 |
| 252 | 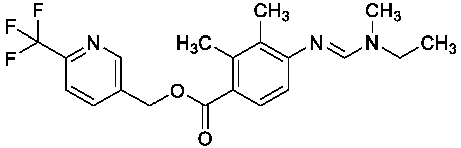 |
| 253 | 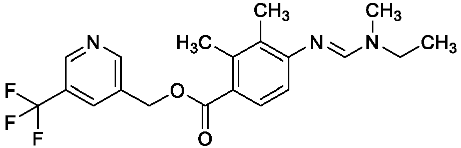 |
| 254 | 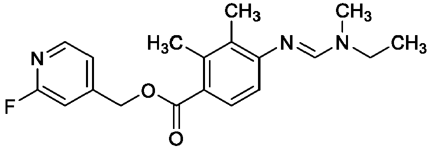 |
| 255 | 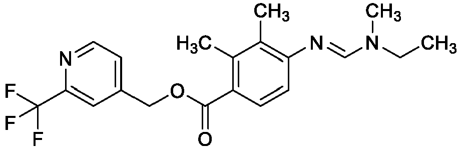 |
| 256 | 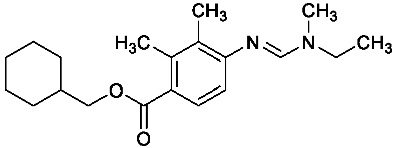 |
| 257 | 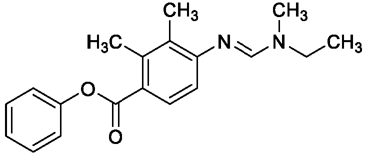 |
| 258 | 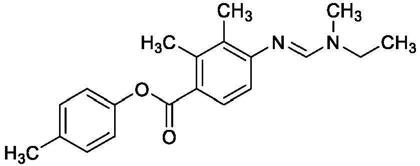 |
| 259 | 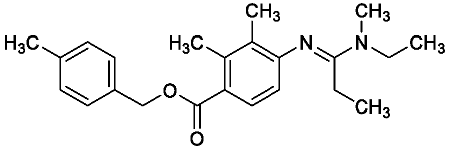 |
| 260 | 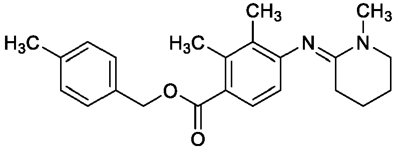 |
| 261 | 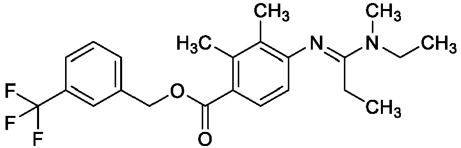 |
| 262 | 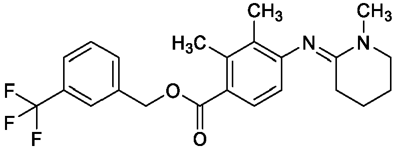 |
| 263 | 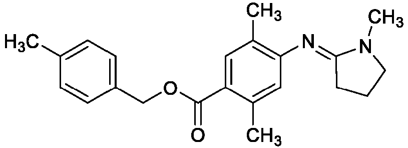 |
| 264 | 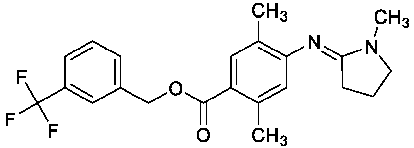 |
| 265 | 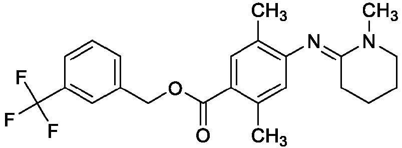 |
| 266 | 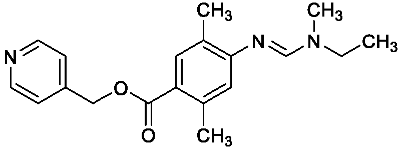 |
| 267 | 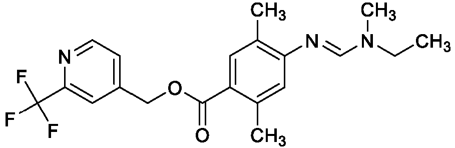 |
| 268 | 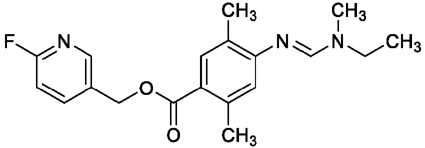 |
| 269 | 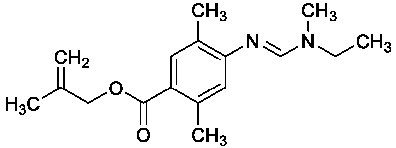 |
| 270 | 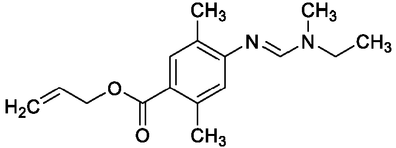 |
| 271 | 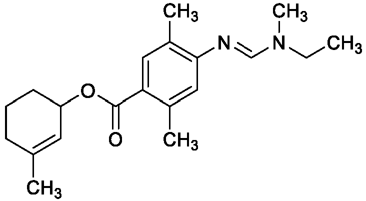 |
| 272 | 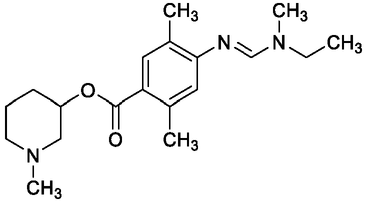 |
| 273 | 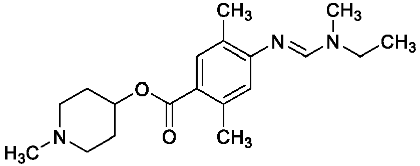 |
| 274 | 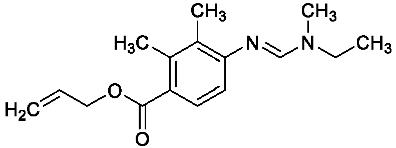 |
| 275 | 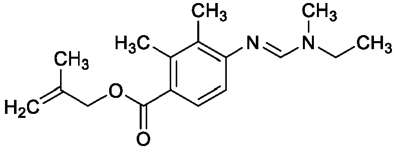 |
| 276 | 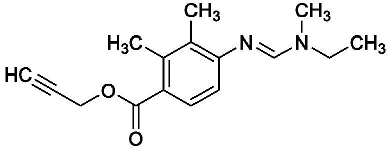 |
| 277 | 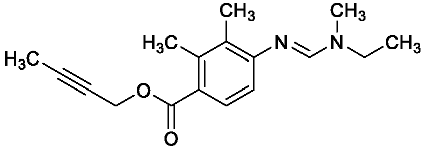 |
| 278 | 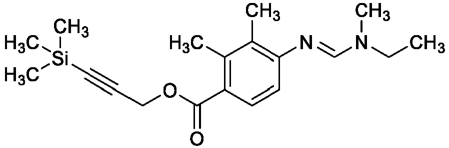 |
| 279 | 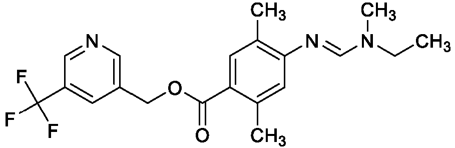 |
| 280 | 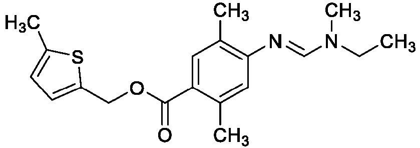 |
| 285 | 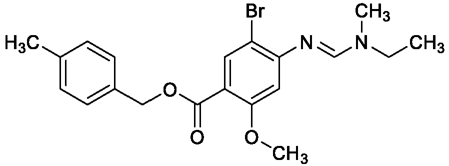 |
| 286 | 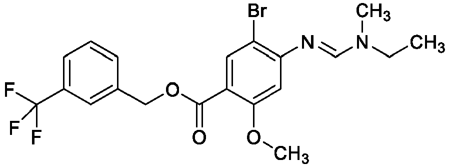 |
| 287 | 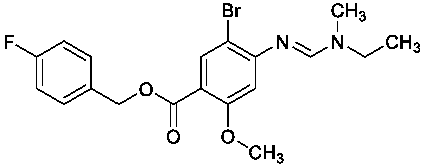 |
| 288 | 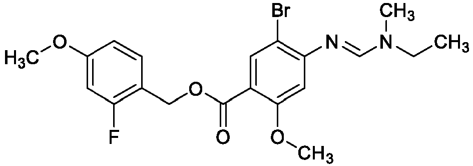 |
| 289 | 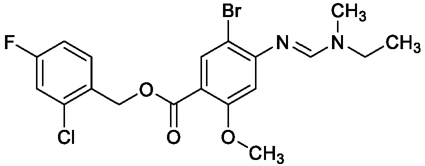 |
| 290 | 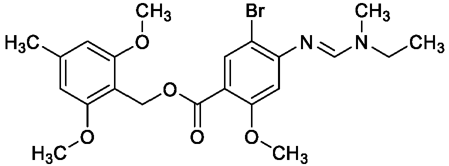 |
| 291 | 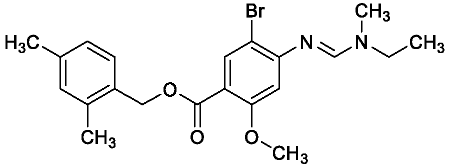 |
| 292 | 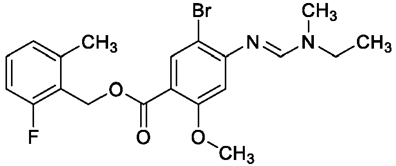 |
| 293 | 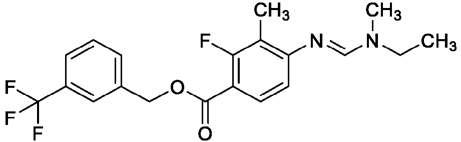 |
| 294 | 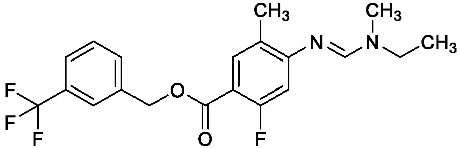 |
| 295 | 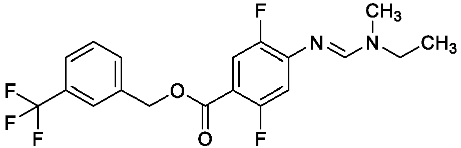 |
| 296 | 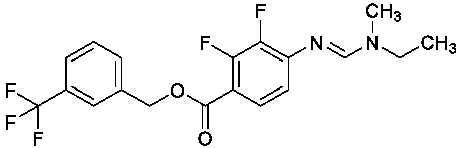 |
| 297 | 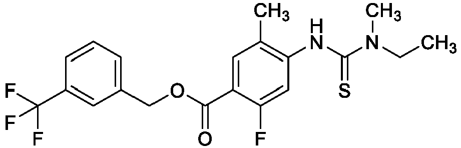 |
| 298 | 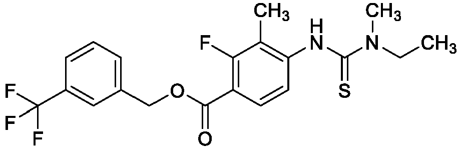 |
| 299 | 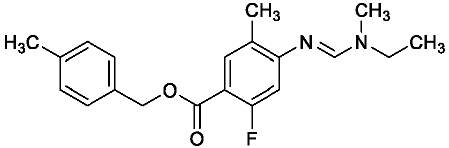 |
| 300 | 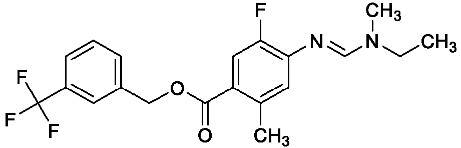 |
| 301 | 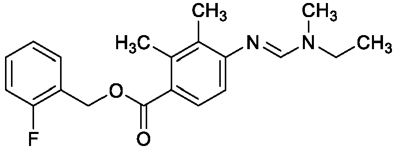 |
| 302 | 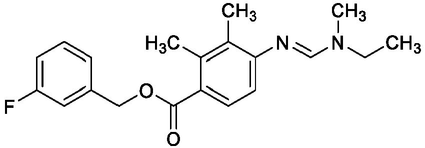 |
| 303 | 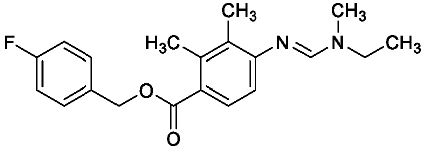 |
| 304 | 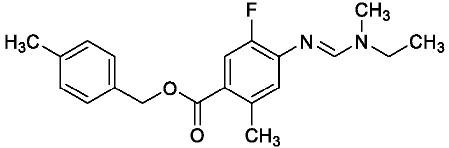 |
| 305 | 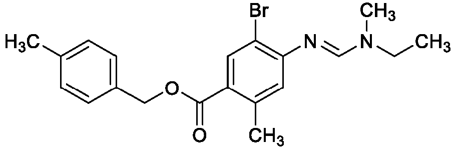 |
| 306 | 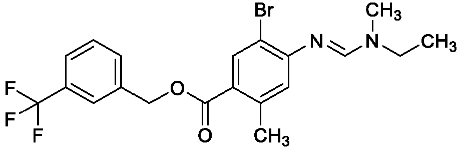 |
| 307 | 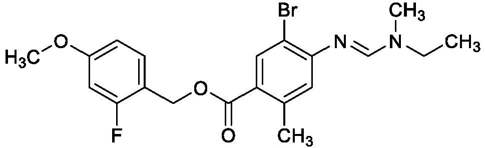 |
| 308 | 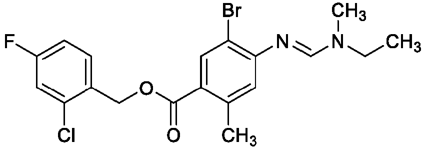 |
| 309 | 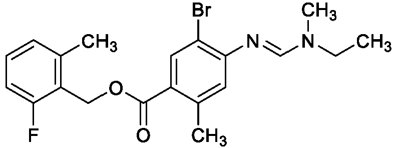 |
| 310 | 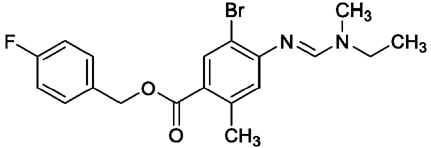 |
| 311 | 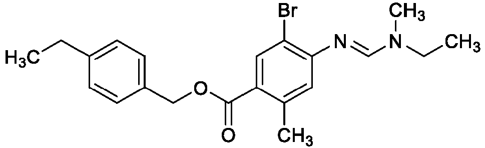 |
| 312 | 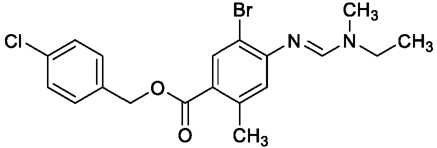 |
| 313 | 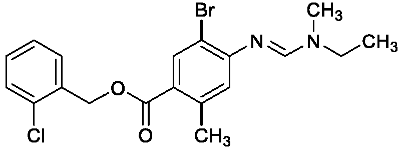 |
| 314 | 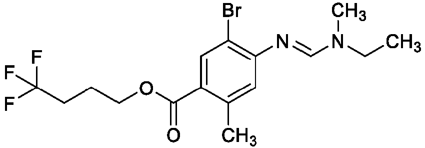 |
| 315 | 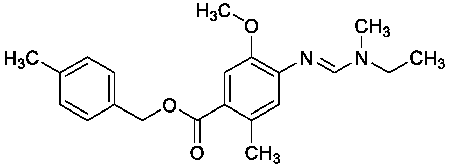 |
| 316 | 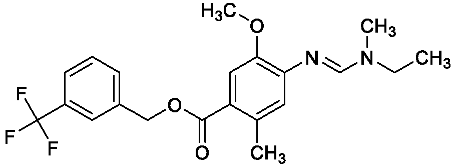 |
| 317 | 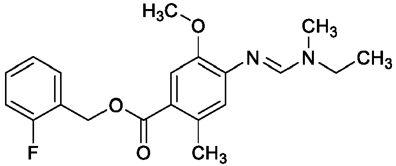 |
| 318 | 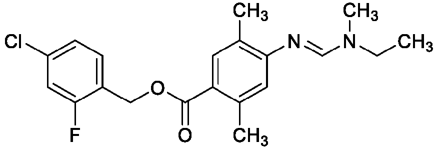 |
| 319 | 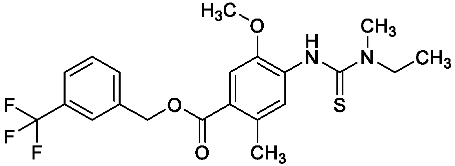 |
| 320 | 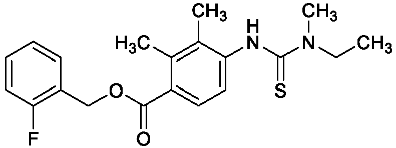 |
| 321 | 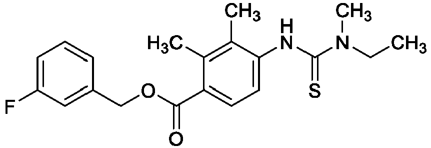 |
| 322 | 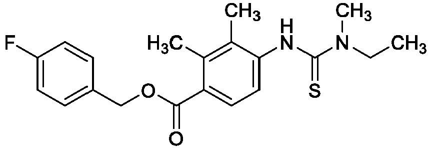 |
| 323 | 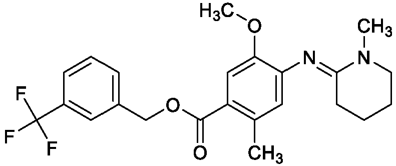 |
| 324 | 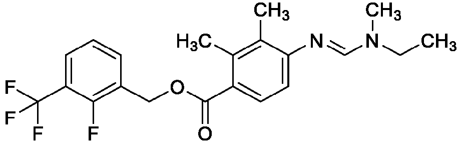 |
| 325 | 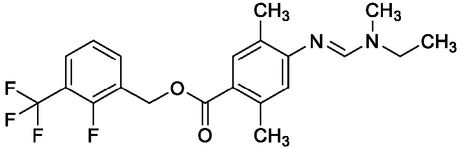 |
| 326 | 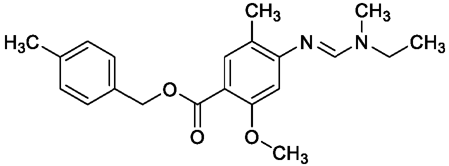 |
| 327 | 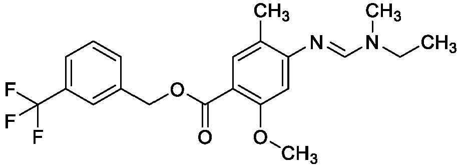 |
| 328 | 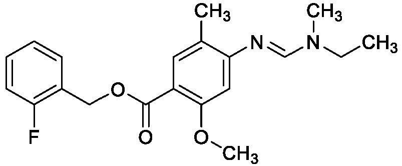 |
| 329 | 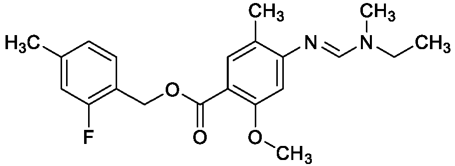 |
| 330 | 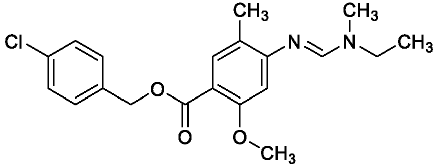 |
| 331 | 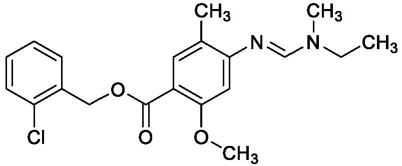 |
| 332 | 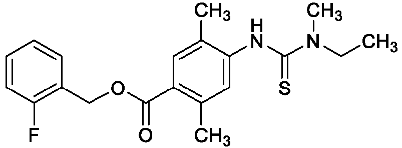 |
| 333 | 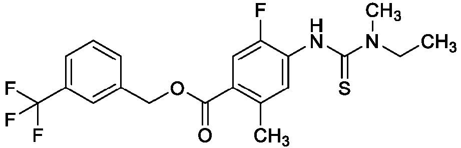 |
| 334 | 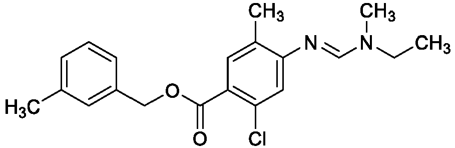 |
| 335 | 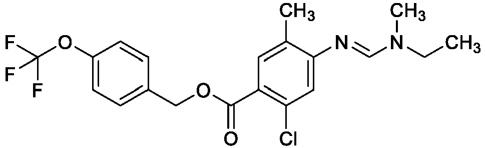 |
| 336 | 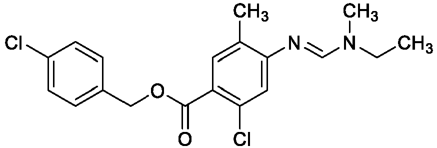 |
| 337 | 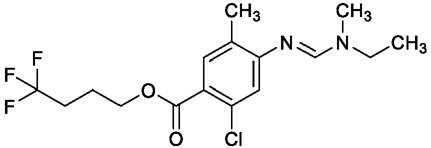 |
| 338 | 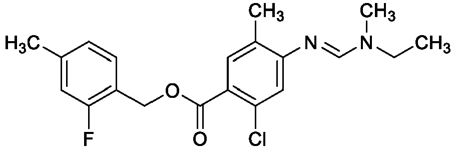 |
| 339 | 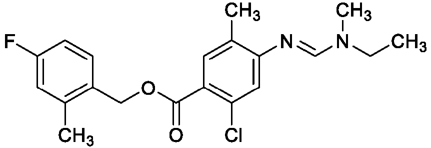 |
| 340 | 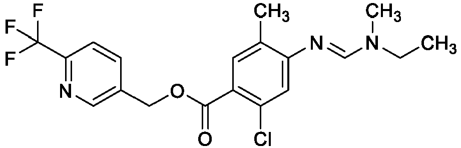 |
| 341 | 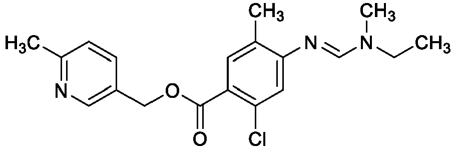 |
| 342 | 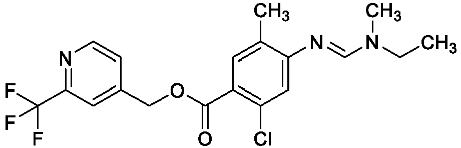 |
| 343 | 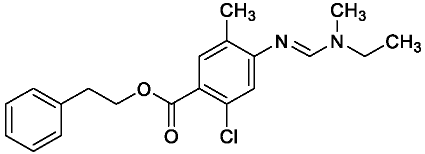 |
| 344 | 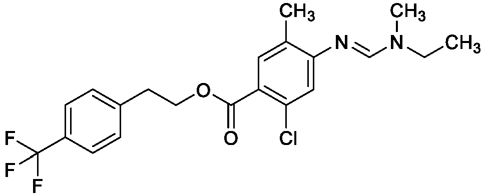 |
| 345 | 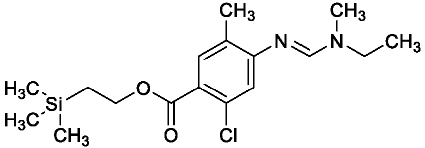 |
| 346 | 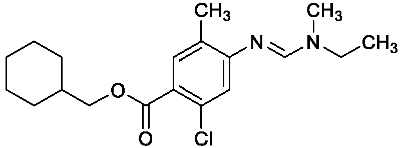 |
| 347 | 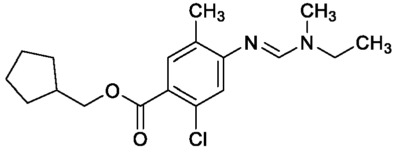 |
| 348 | 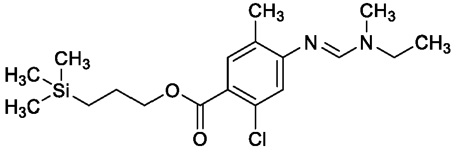 |
| 349 | 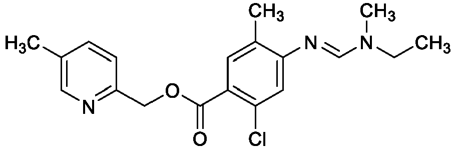 |
| 350 | 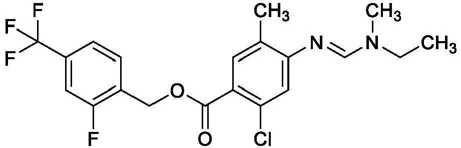 |
| 351 | 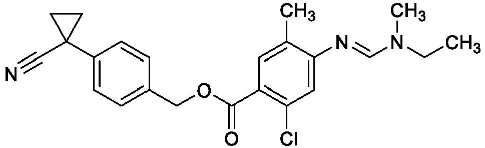 |
| 352 | 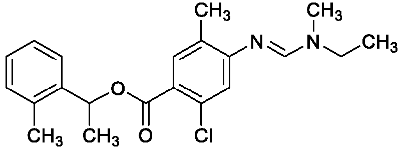 |
| 353 | 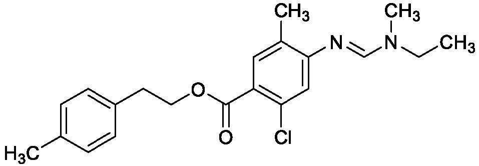 |
| 354 | 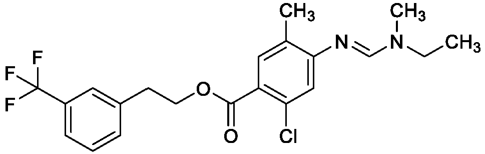 |
| 355 | 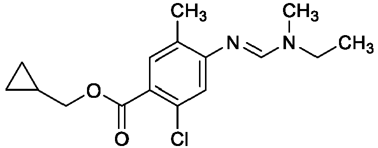 |
| 356 | 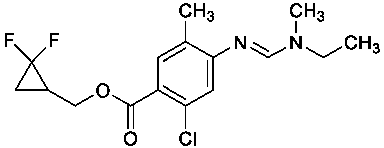 |
| 357 | 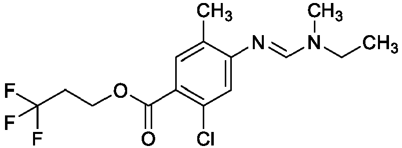 |
| 358 | 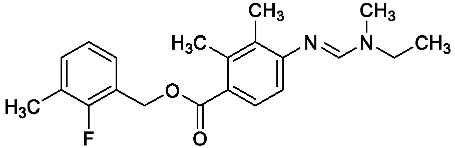 |
| 359 | 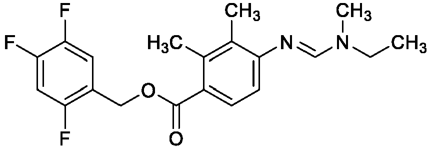 |
| 360 | 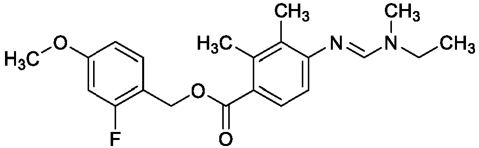 |
| 361 | 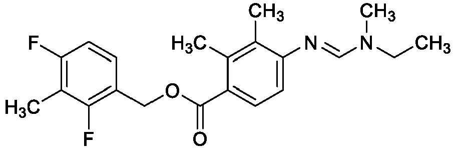 |
| 362 | 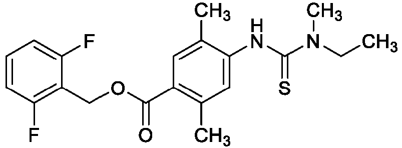 |
| 363 | 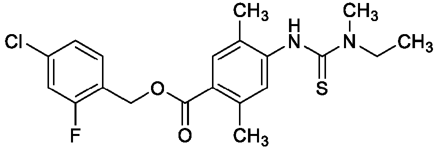 |
| 364 | 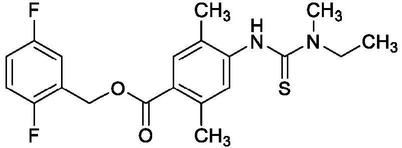 |
| 365 | 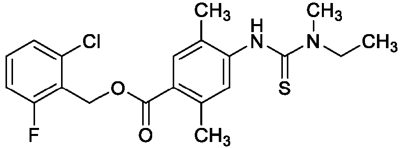 |
| 366 | 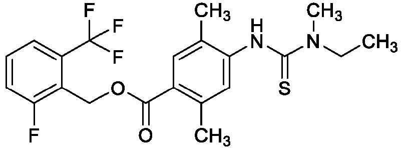 |
| 367 | 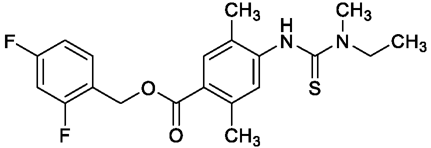 |
| 368 | 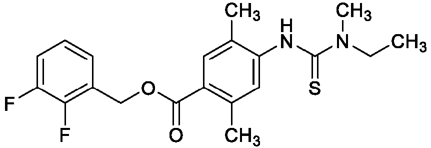 |
| 369 | 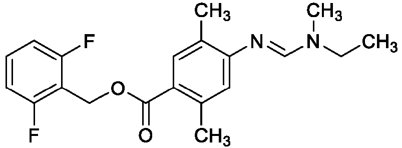 |
| 370 | 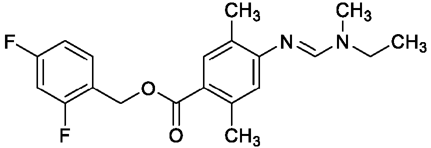 |
| 371 | 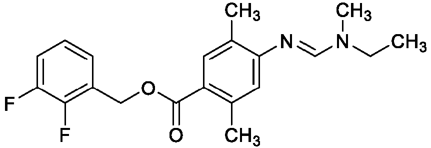 |
| 372 | 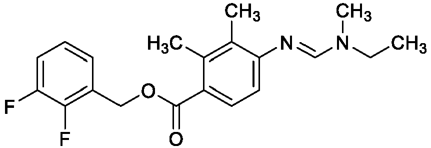 |
| 373 | 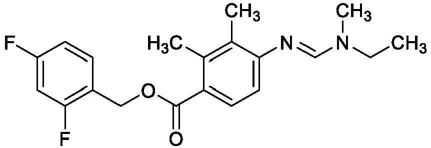 |
| 374 | 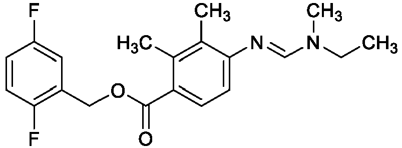 |
| 375 | 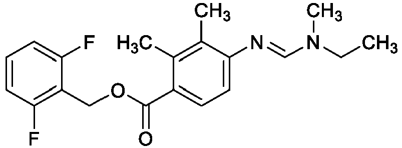 |
| 376 | 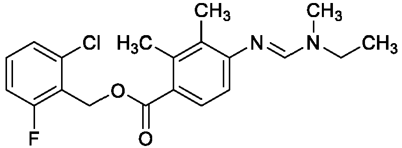 |
| 377 | 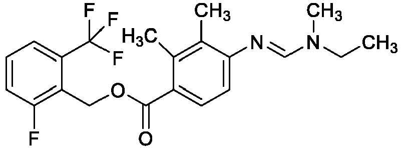 |
| 378 | 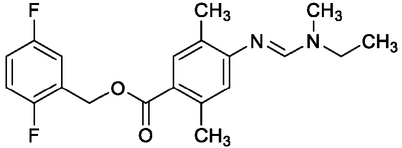 |
| 379 | 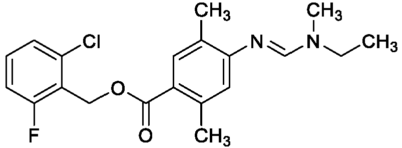 |
| 380 | 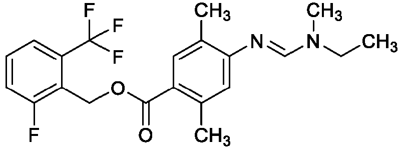 |
| 381 | 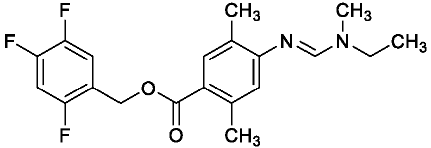 |
| 382 | 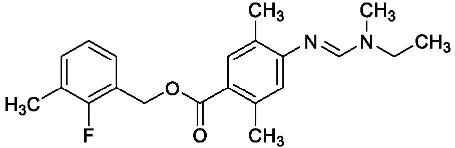 |
| 383 | 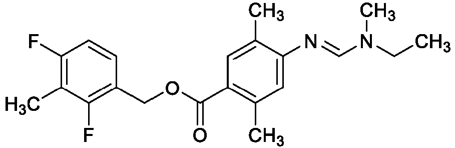 |
| 384 | 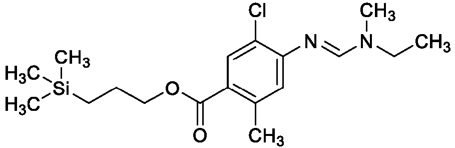 |
| 385 | 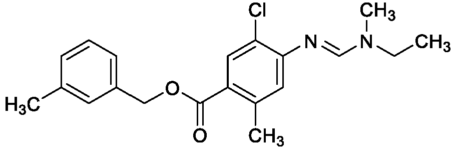 |
| 386 | 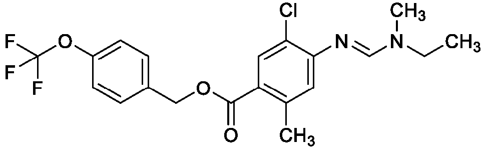 |
| 387 | 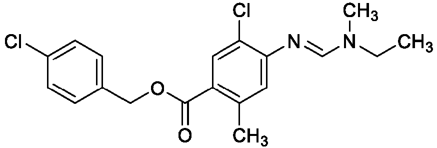 |
| 388 | 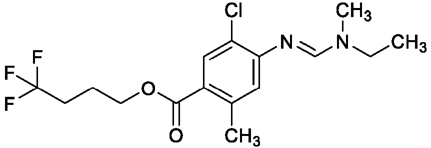 |
| 389 | 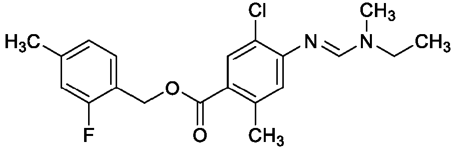 |
| 390 | 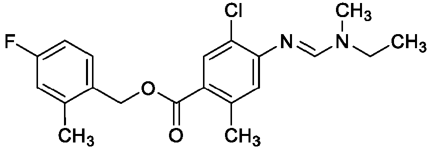 |
| 391 | 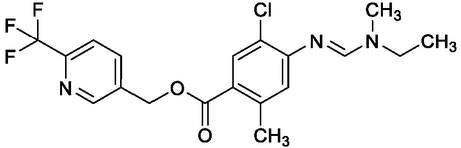 |
| 392 | 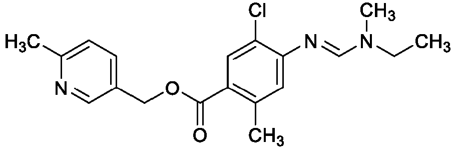 |
| 393 | 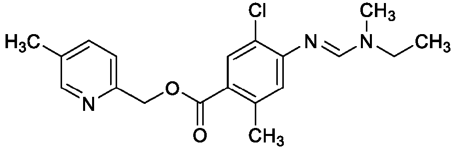 |
| 394 | 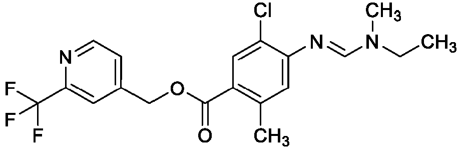 |
| 395 | 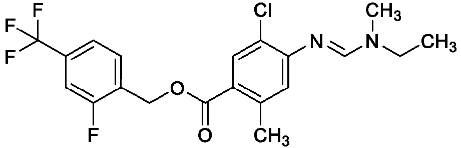 |
| 396 | 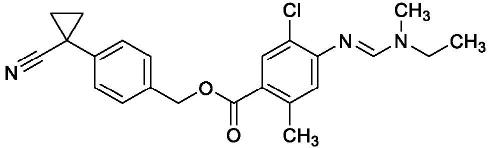 |
| 397 | 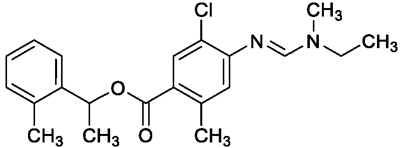 |
| 398 | 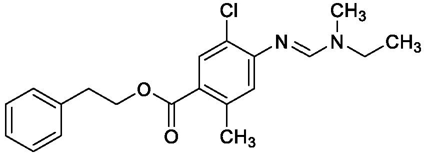 |
| 399 | 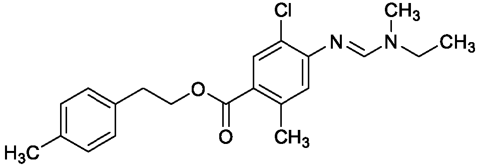 |
| 400 | 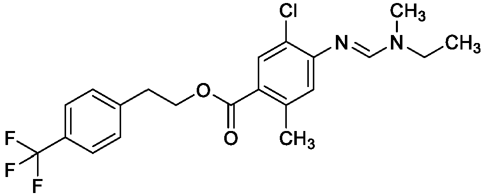 |
| 401 | 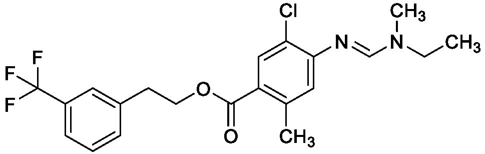 |
| 402 | 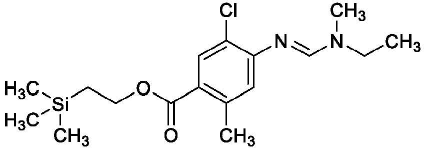 |
| 403 | 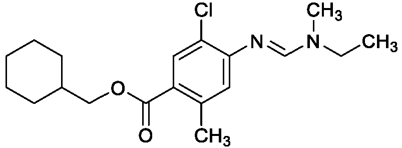 |
| 404 | 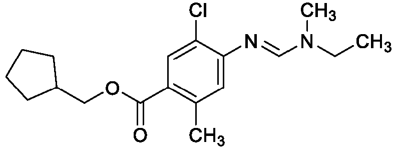 |
| 405 | 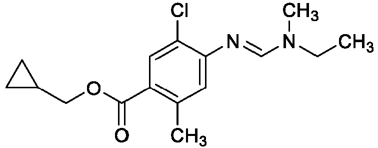 |
| 406 | 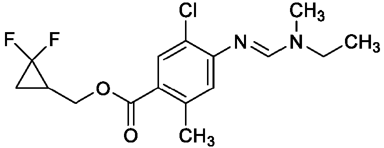 |
| 407 | 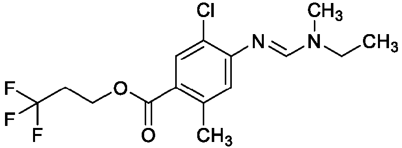 |
| 408 | 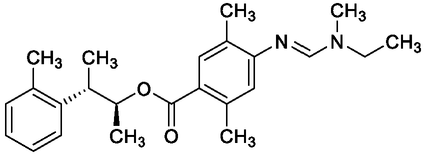 |
| 409 | 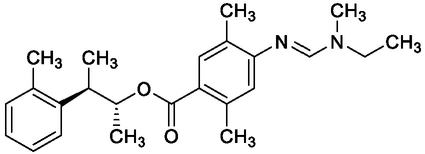 |
| 410 | 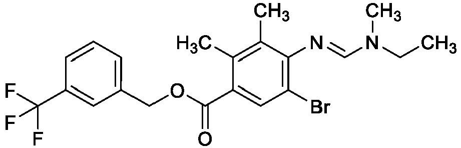 |
| 411 | 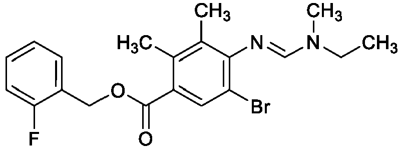 |
| 412 | 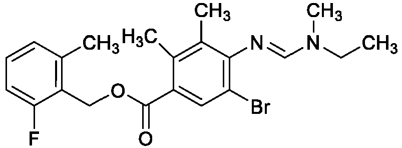 |
| 413 | 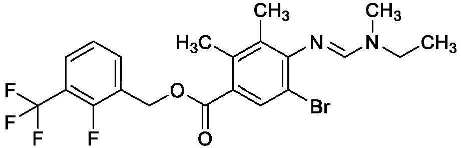 |
| 414 | 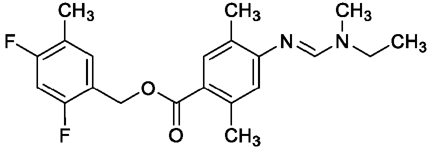 |
| 415 | 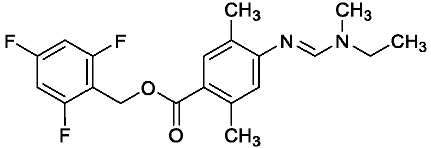 |
| 416 | 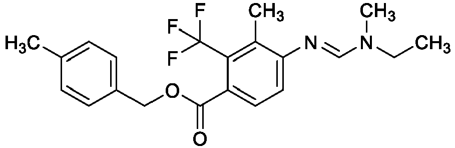 |
| 417 | 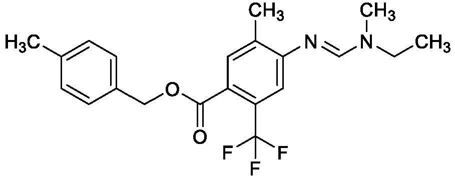 |
| 418 | 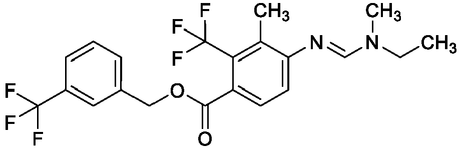 |
| 419 | 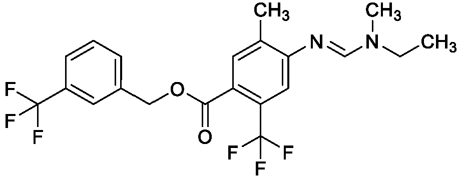 |
| 420 | 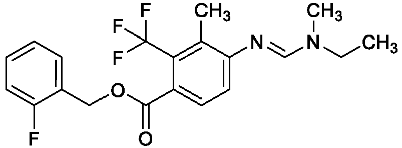 |
| 421 | 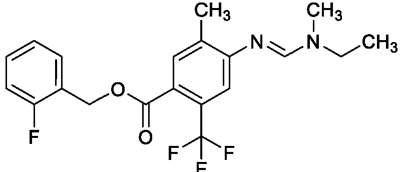 |
| 422 | 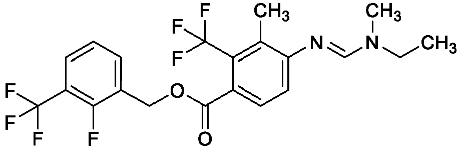 |
| 423 | 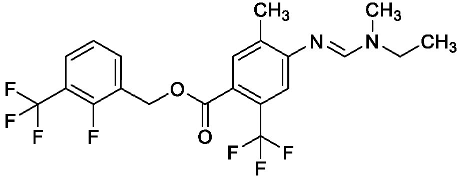 |
| 424 | 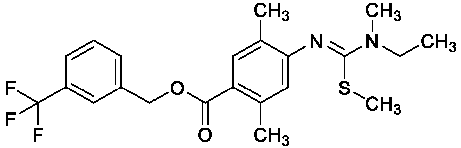 |
| 425 | 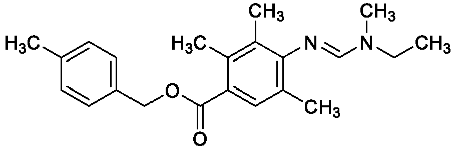 |
| 426 | 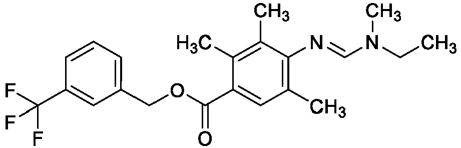 |
| 427 | 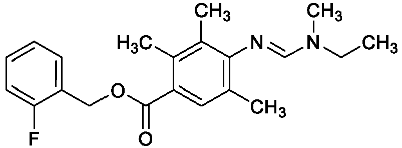 |
| 428 | 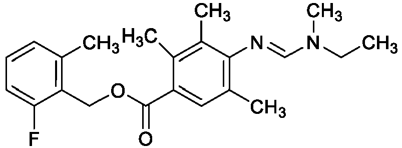 |
| 429 | 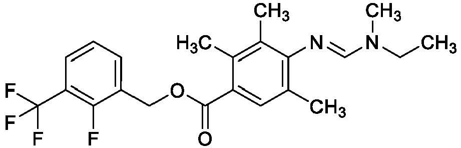 |
| 430 | 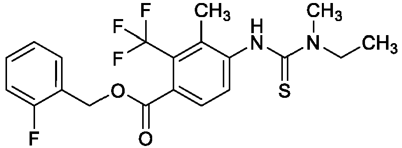 |
| 431 | 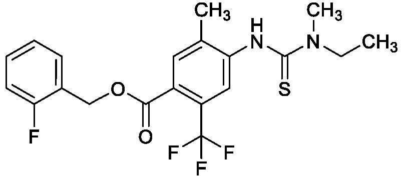 |
| 432 | 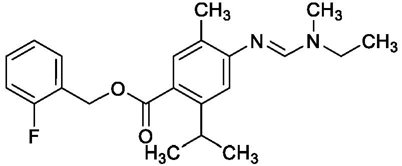 |
| 433 | 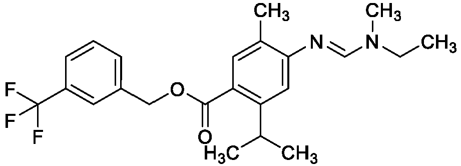 |
| 434 | 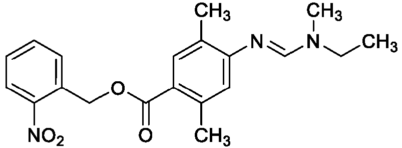 |
| 435 | 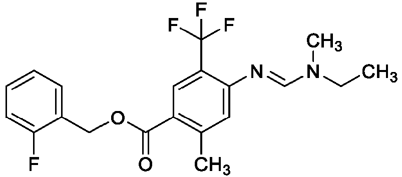 |
| 436 | 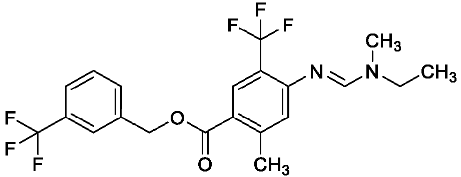 |
| 437 | 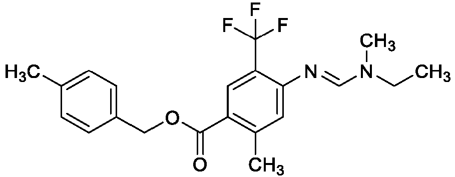 |
| 438 | 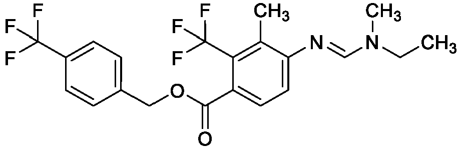 |
| 439 | 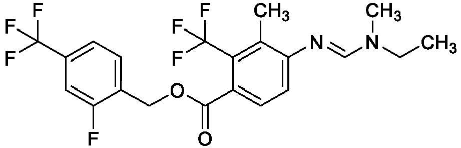 |
| 440 | 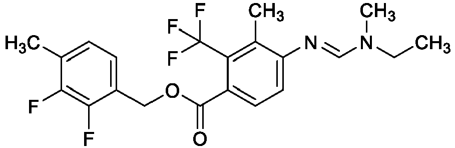 |
| 441 | 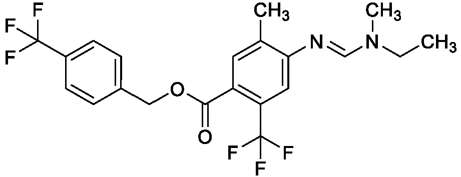 |
| 442 | 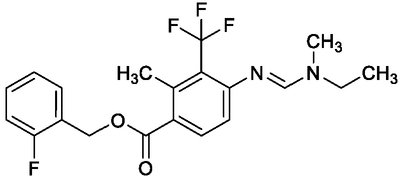 |
| 443 | 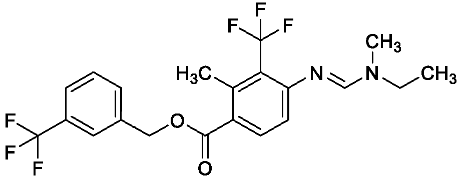 |
| 444 | 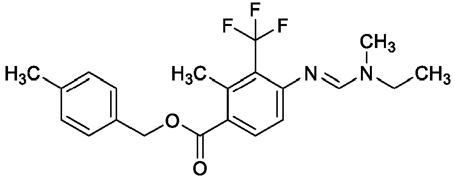 |
| 445 | 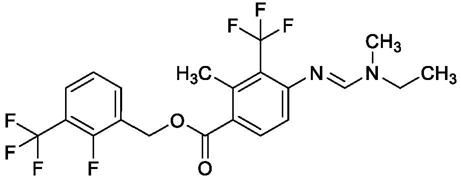 |
| 446 | 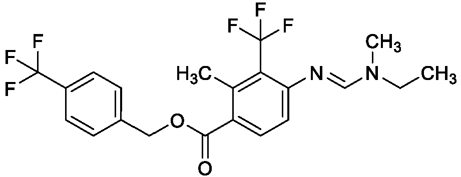 |
| 447 | 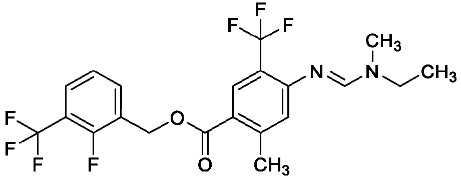 |
| 448 | 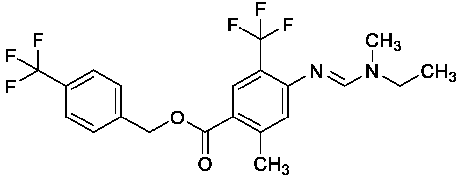 |
| 449 | 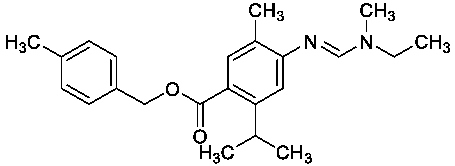 |
| 450 | 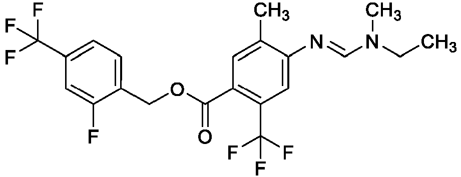 |
| 451 | 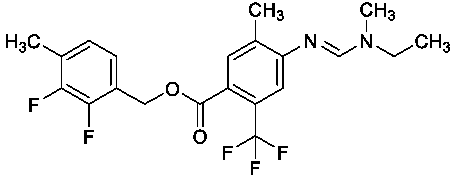 |
| 452 | 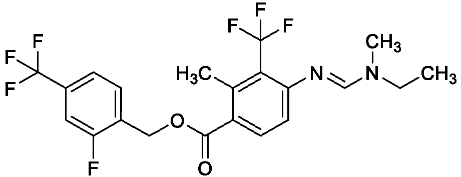 |
| 453 | 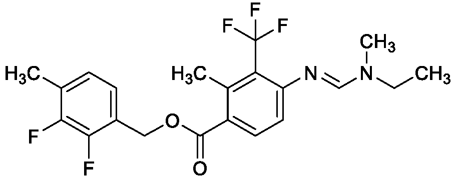 |
| 454 | 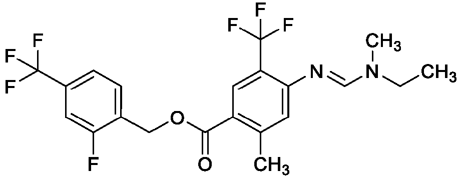 |
| 455 | 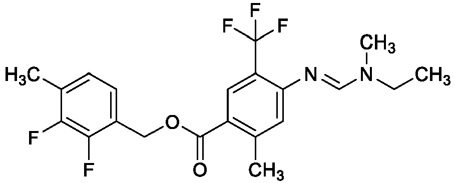 |
| 456 | 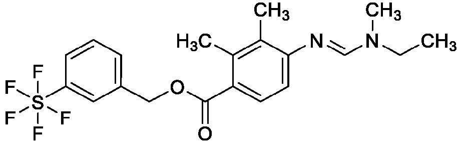 |
| 457 | 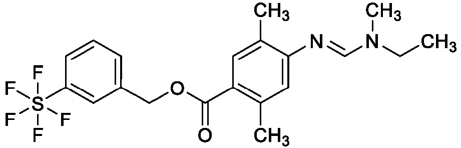 |
| 458 | 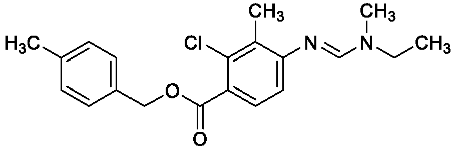 |
| 459 | 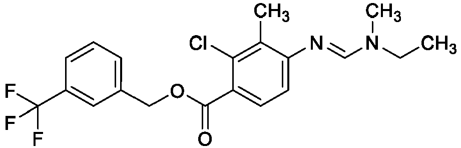 |
| 460 | 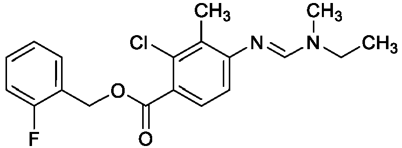 |
| 461 | 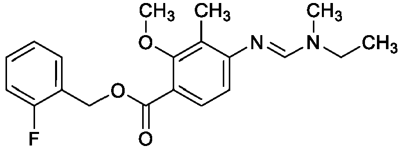 |
| 462 | 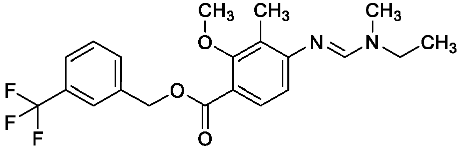 |
| 463 | 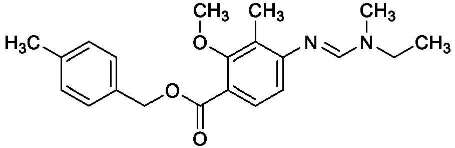 |
| 464 | 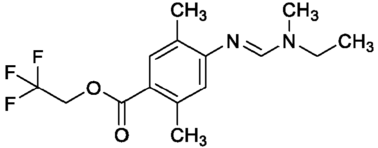 |
| 465 | 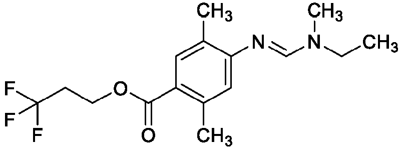 |
| 466 | 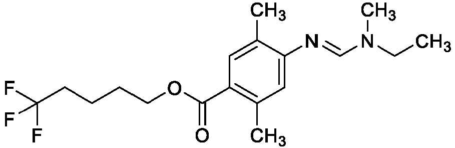 |
| 467 | 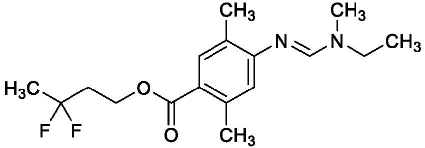 |
| 468 | 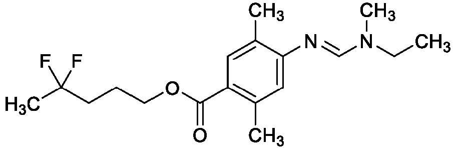 |
| 469 | 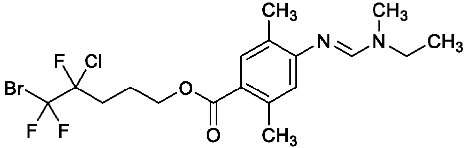 |
| 470 | 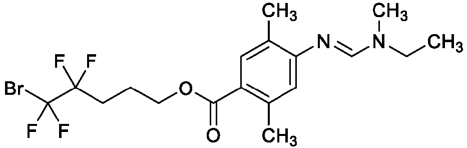 |
| 471 | 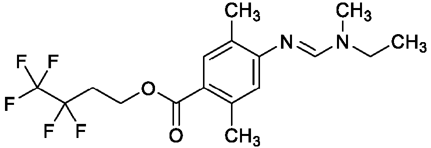 |
| 472 | 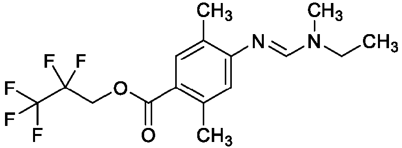 |
| 473 | 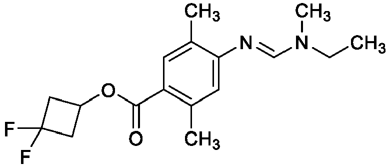 |
| 474 | 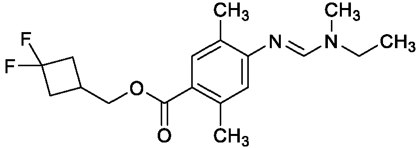 |
| 475 | 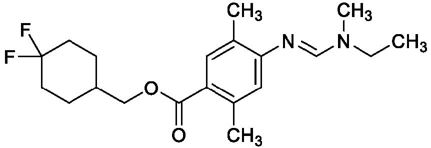 |
| 476 | 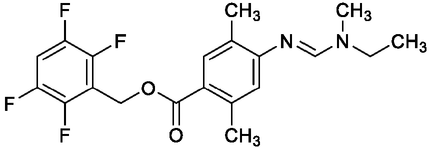 |
| 477 | 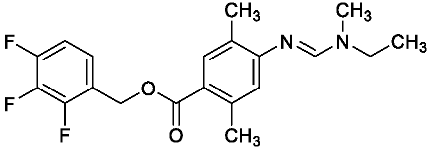 |
| 478 | 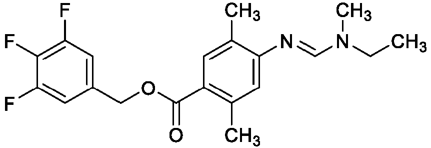 |
| 479 | 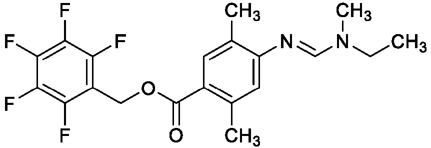 |
| 480 | 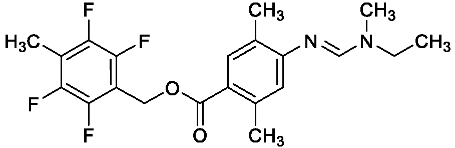 |
| 481 | 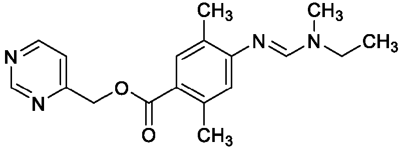 |
| 482 | 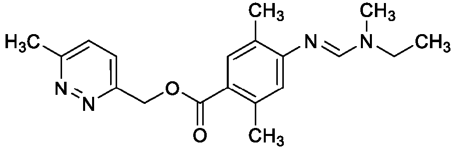 |
| 483 | 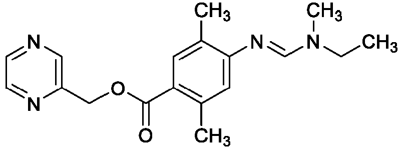 |
| 484 | 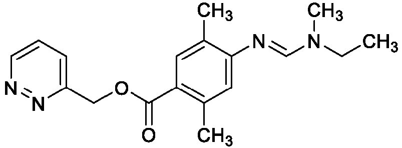 |
| 485 | 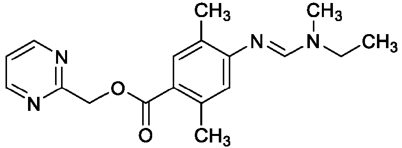 |
| 486 | 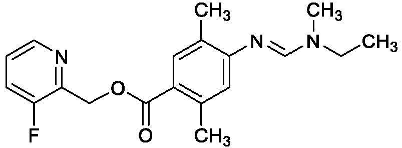 |
| 487 | 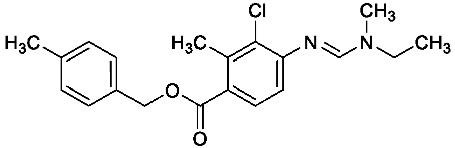 |
| 488 | 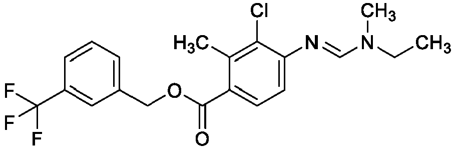 |
| 489 | 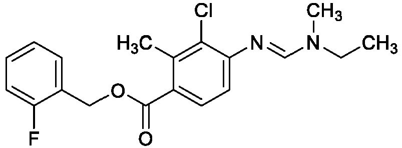 |
| 490 | 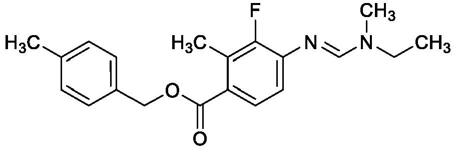 |
| 491 | 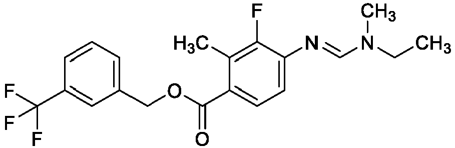 |
| 492 | 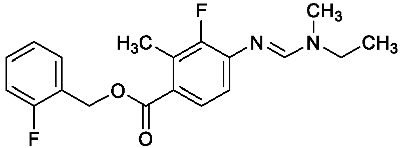 |
| 493 | 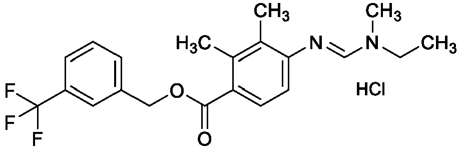 |
| 494 | 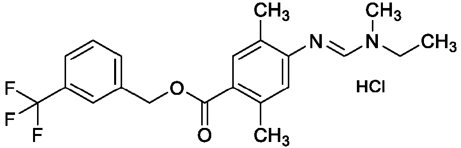 |
| 495 | 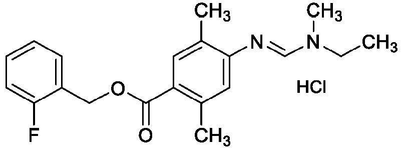 |
| 496 | 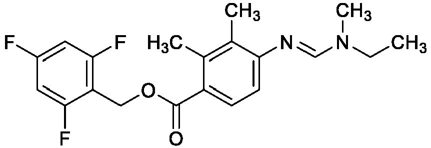 |
| 497 | 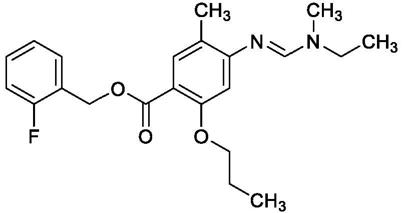 |
| 498 | 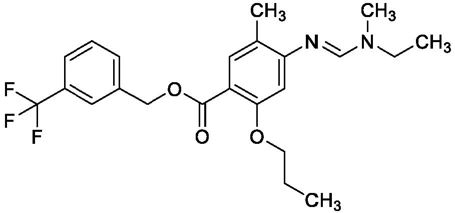 |
| 499 | 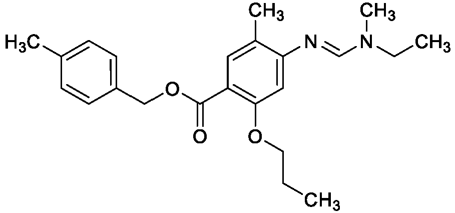 |
| 500 | 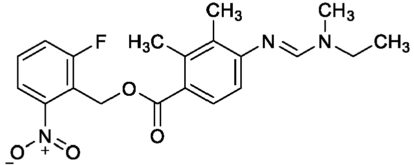 |
| 501 | 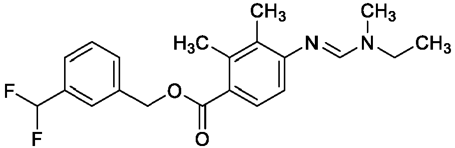 |
| 502 | 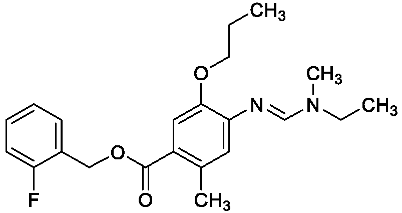 |
| 503 | 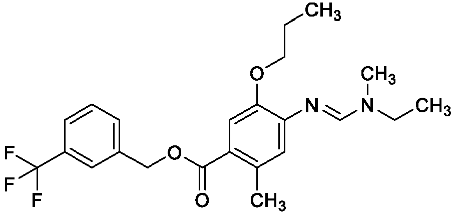 |
| 504 | 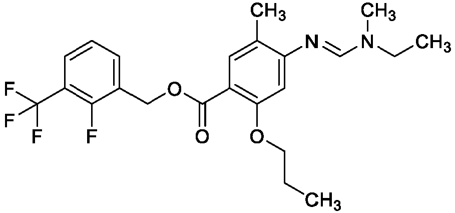 |
| 505 | 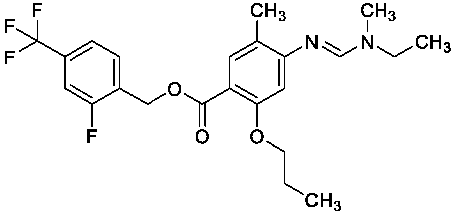 |
| 506 | 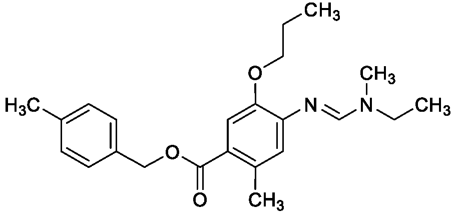 |
| 507 | 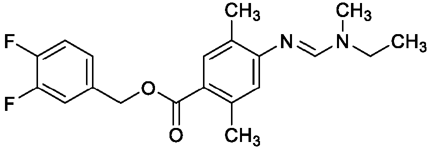 |
| 508 | 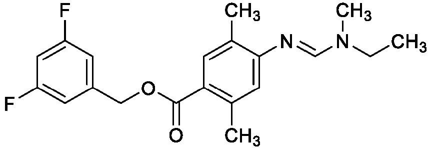 |
| 509 | 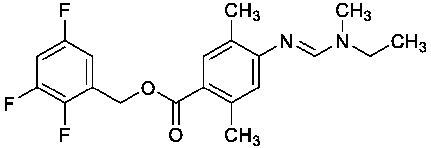 |
| 510 | 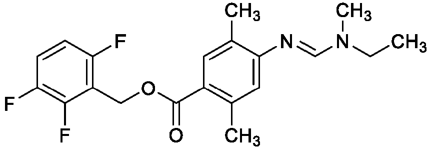 |
| 511 | 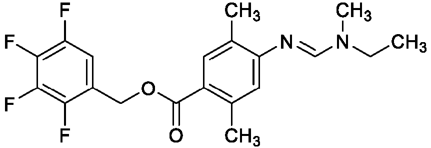 |
| 512 | 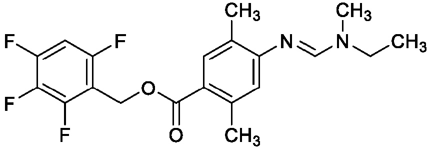 |
| 513 | 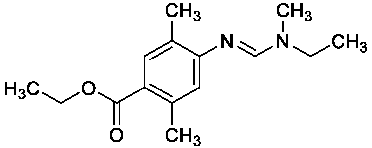 |
| 514 | 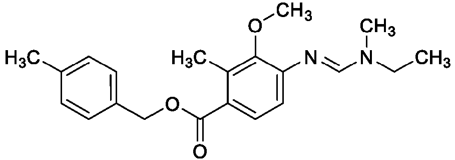 |
| 515 | 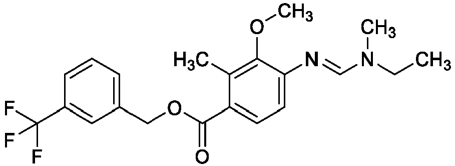 |
| 516 | 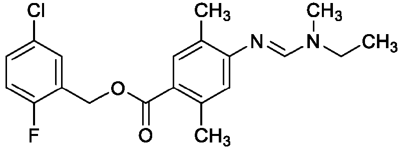 |
| 517 | 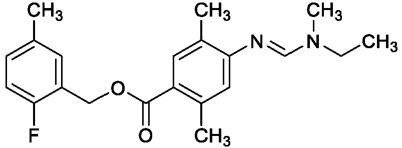 |
| 518 | 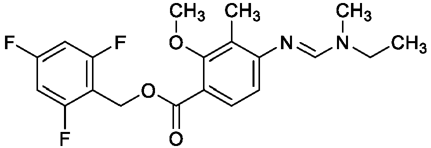 |
| 519 | 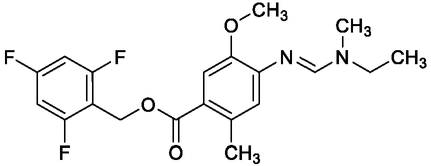 |
| 520 | 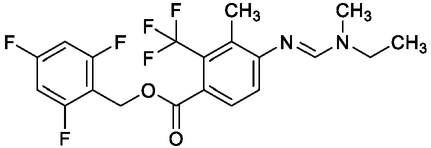 |
| 521 | 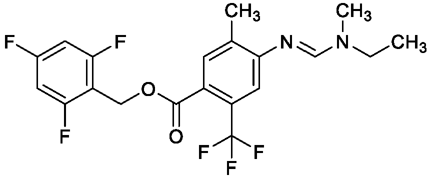 |
| 522 | 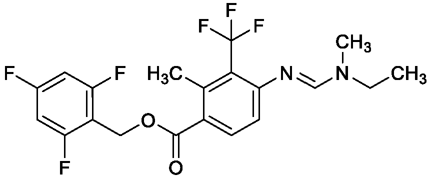 |
| 523 | 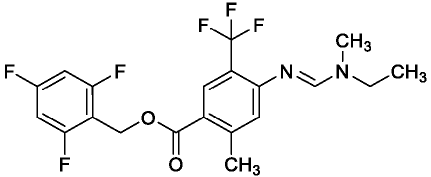 |
| 524 | 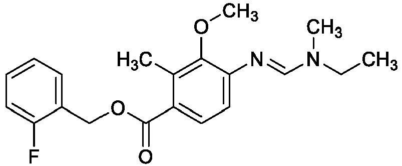 |
| 525 | 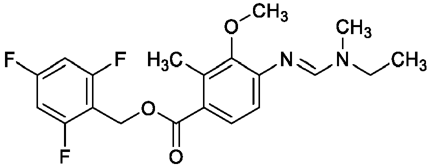 |
| 526 | 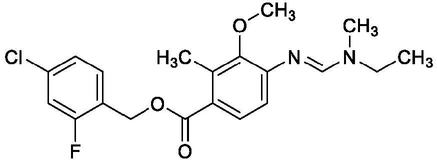 |
| 527 | 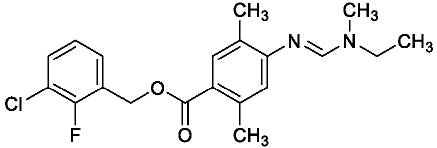 |
| 528 | 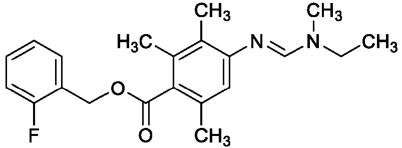 |
| | |
| 530 | 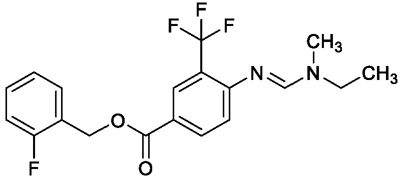 |
| 531 | 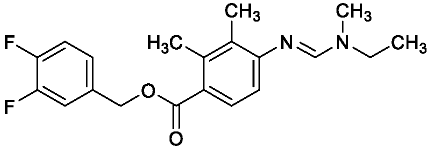 |
| 532 | 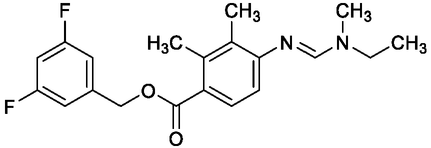 |
| 533 | 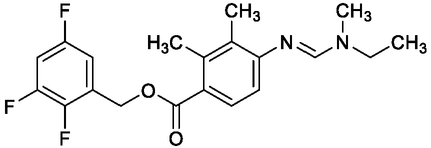 |
| 534 | 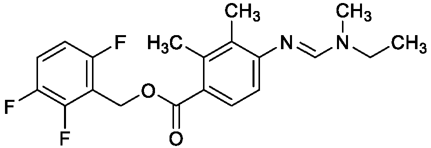 |
| 535 | 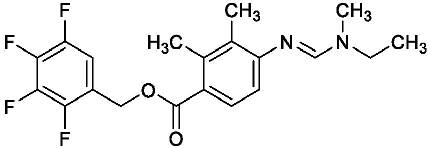 |
| 536 | 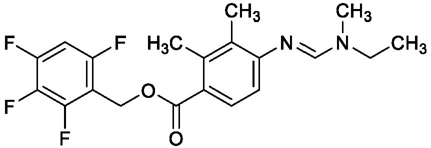 |
| 537 | 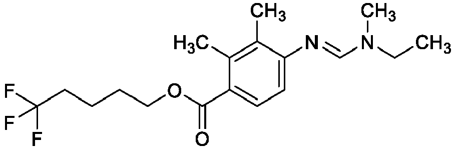 |
| 538 | 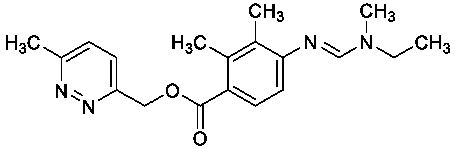 |
| 539 | 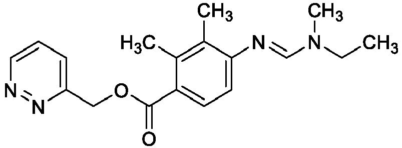 |
| 540 | 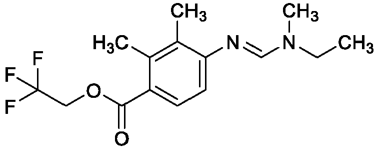 |
| 541 | 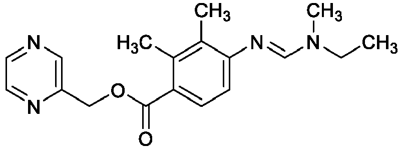 |
| 542 | 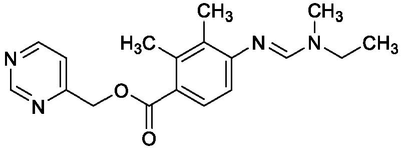 |
| 543 | 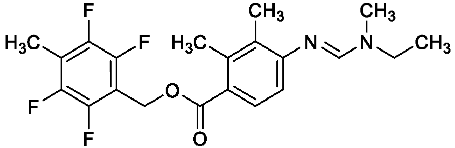 |
| 544 | 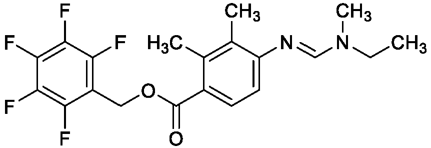 |
| 545 | 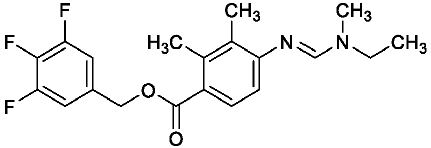 |
| 546 | 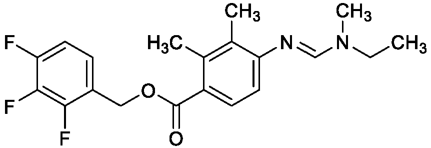 |
| 547 | 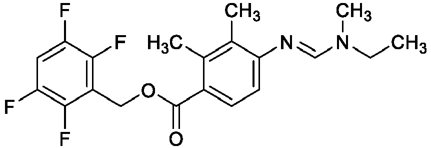 |
| 548 | 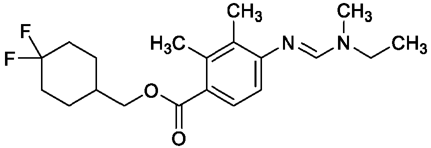 |
| 549 | 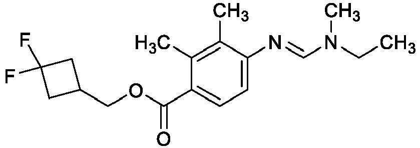 |
| 550 | 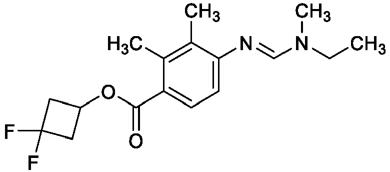 |
| 551 | 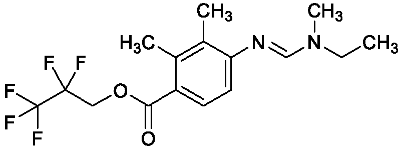 |
| 552 | 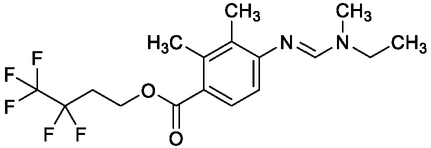 |
| 553 | 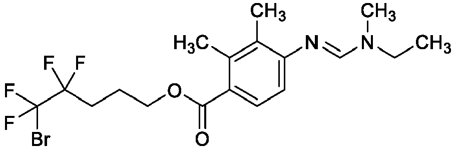 |
| 554 | 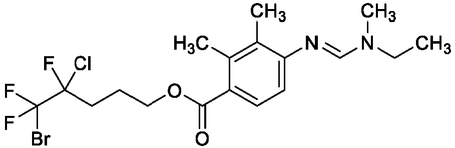 |
| 555 | 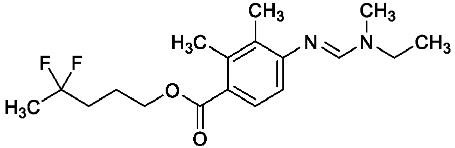 |
| 556 | 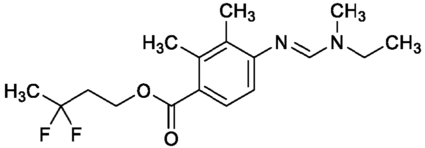 |
| 557 | 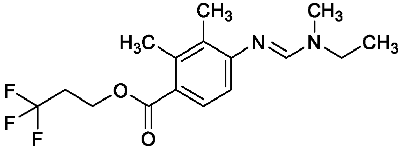 |
| 558 | 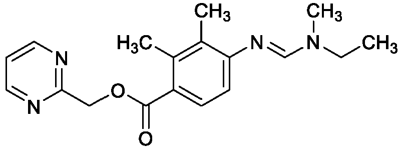 |
| 559 | 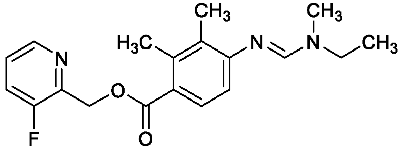 |
| 560 | 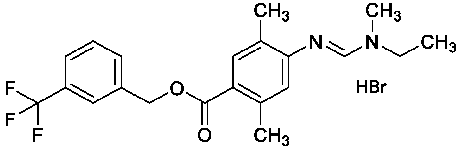 |
| 561 | 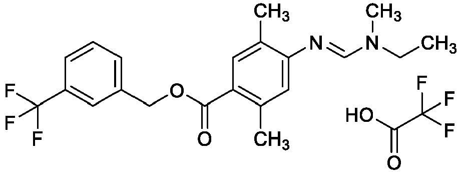 |
| 562 | 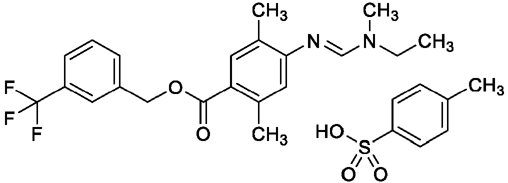 |
| 563 | 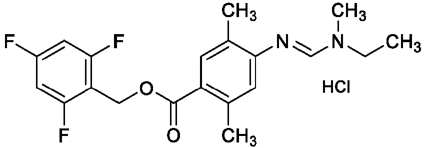 |

9. A fungicide composition comprising a phytologically acceptable amount of a compound of Formula I of any one of claims 1-8 and a carrier, preferably wherein the carrier is one or more of a thickener, emulsifier, rheology agent, dispersant and polymer.

10. A method of controlling fungal attack on a plant, the method comprising contacting an area adjacent to the plant, soil adapted to support growth of the plant, a root of the plant, and foliage of the plant, with a phytologically acceptable amount of a compound of Formula I of any one of claims 1-8 or a composition of claim 9.

11. A compound of Formula I of any one of claims 1-8 for use in the control of a fungal pathogen.

12. The compound for use of claim 11, wherein the fungal pathogen is one of Zymoseptoria tritici, Cochliobolus sativus, Puccinia triticina, Puccinia striiformis, Venturia inaequalis, Ustilago maydis, Uncinula necator, Rhynchosporium commune, Magnaporthe grisea, Phakopsora pachyrhizi, Parastagonospora nodorum, Glomerella lagenarium, Cercospora beticola, Alternaria solani, Pyrenophora teres, Blumeria graminis f. sp. tritici, Blumeria graminis f. sp. hordei, Erysiphe cichoracearum, Fusarium virguliforme, Rhizoctonia solani, Pythium ultimum, and Botrytis cinerea; or
wherein the compound treats one of the following diseases from the fungal pathogen: Septoria Leaf Blotch of Wheat (*Zymoseptoria tritici*), Spot Blotch of Barley (*Cochliobolus sativus*), Wheat Brown Rust (*Puccinia triticina*), Stripe Rust (*Puccinia striiformis*), Scab of Apple (*Venturia inaequalis*), Blister Smut of Maize (*Ustilago maydis*), Powdery Mildew of Grapevine (*Uncinula necator*), Leaf Blotch of Barley (*Rhynchosporium commune*), Blast of Rice (*Magnaporthe grisea*), Asian Soybean Rust (*Phakopsora pachyrhizi*), Glume Blotch of Wheat (*Parastagonospora nodorum*), Anthracnose of Cucurbits (*Glomerella lagenarium*), Leaf Spot of Beet (*Cercospora beticola*), Early Blight of Tomato (*Alternaria solani*), Net Blotch of Barley (*Pyrenophora teres*), Powdery Mildew of Wheat (*Blumeria graminis f. sp. tritici*), Powdery Mildew of Barley (*Blumeria graminis f. sp. hordei*), Powdery Mildew of Cucurbits (*Erysiphe cichoracearum*), Sudden Death Syndrome of Soybean (*Fusarium virguliforme*), Collar Rot or Damping-Off of Seedlings (*Rhizoctonia solani*), Root Rot (*Pythium ultimum*), Grey Mold (*Botrytis cinerea*).

13. A composition for use in the control of a fungal pathogen, the composition comprising a phytologically acceptable amount of a compound of Formula I of any one of claims 1-8, 11 or 12 and a carrier.

14. The composition for use of claim 13 wherein the fungal pathogen is one of Zymoseptoria tritici, Cochliobolus sativus, Puccinia triticina, Puccinia striiformis, Venturia inaequalis, Ustilago maydis, Uncinula necator, Rhynchosporium commune, Magnaporthe grisea, Phakopsora pachyrhizi, Parastagonospora nodorum, Glomerella lagenarium, Cercospora beticola, Alternaria solani, Pyrenophora teres, Blumeria graminis f. sp. tritici, Blumeria graminis f. sp. hordei, Erysiphe cichoracearum, Fusarium virguliforme, Rhizoctonia solani, Pythium ultimum, and Botrytis cinerea; or
wherein the composition treats one of the following diseases from the fungal pathogen: Septoria Leaf Blotch of Wheat (*Zymoseptoria tritici*), Spot Blotch of Barley (*Cochliobolus sativus*), Wheat Brown Rust (*Puccinia triticina*), Stripe Rust (*Puccinia striiformis*), Scab of Apple (*Venturia inaequalis*), Blister Smut of Maize (*Ustilago maydis*), Powdery Mildew of Grapevine (*Uncinula necator*), Leaf Blotch of Barley (*Rhynchosporium commune*), Blast of Rice (*Magnaporthe grisea*), Asian Soybean Rust (*Phakopsora pachyrhizi*), Glume Blotch of Wheat (*Parastagonospora nodorum*), Anthracnose of Cucurbits (*Glomerella lagenarium*), Leaf Spot of Beet (*Cercospora beticola*), Early Blight of Tomato (*Alternaria solani*), Net Blotch of Barley (*Pyrenophora teres*), Powdery Mildew of Wheat (*Blumeria graminis f. sp. tritici*), Powdery Mildew of Barley (*Blumeria graminis f. sp. hordei*), Powdery Mildew of Cucurbits (*Erysiphe cichoracearum*), Sudden Death Syndrome of Soybean (*Fusarium virguliforme*), Collar Rot or Damping-Off of Seedlings (*Rhizoctonia solani*), Root Rot (*Pythium ultimum*), Grey Mold (*Botrytis cinerea*), or
wherein the disease is one of Septoria Leaf Blotch of Wheat, Spot Blotch of Barley, Wheat Brown Rust, and Asian Soybean Rust.

## Patentansprüche

1. Verbindung der Formel I: 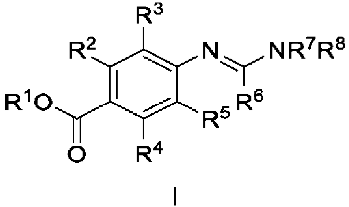 wobei
R¹ ausgewählt ist aus der Gruppe bestehend aus C₁-C₈-Alkyl, C₁-C₈-substituiertem Alkyl, C₂-C₈-Alkenyl, C₂-C₈-substituiertem Alkenyl, C₂-C₈-Alkinyl, C₂-C₈-substituiertem Alkinyl, C₃-C₈-Cycloalkyl, C₃-C₈-substituiertem Cycloalkyl, C₃-C₈-Heterocycloalkyl, C₃-C₈-substituiertem Heterocycloalkyl, C₅-C₇-Heteroaryl, C₅-C₇-substituiertem Heteroaryl, Phenyl, substituiertem Phenyl, Benzyl und substituiertem Benzyl;
jedes R², R⁴ und R⁵ unabhängig ausgewählt ist aus der Gruppe bestehend aus Wasserstoff, Halogen, Cyano, Nitro, C₁-C₈-Alkyl, C₁-C₈-substituiertem Alkyl, C₂-C₈-Alkenyl, C₂-C₈-substituiertem Alkenyl, C₂-C₈-Alkinyl, C₂-C₈-substituiertem Alkinyl, C₁-C₈-Alkoxy und C₁-C₈-substituiertem Alkoxy;
R³ ausgewählt ist aus der Gruppe bestehend aus bestehend aus Halogen, Cyano, C₁-C₈-Alkyl, C₁-C₈-substitutem Alkyl, C₂-C₈-Alkenyl, C₂-C₈-substitutem Alkenyl, C₂-C₈-Alkinyl, C₂-C₈ substitutem Alkinyl, C₁-C₈-Alkoxy und C₁-C₈-substitutem Alkoxy;
R⁶ ausgewählt ist aus der Gruppe bestehend aus Wasserstoff, C₁-C₈-Alkyl, C₁-C₈-substituiertem Alkyl, C₂-C₈-Alkenyl, C₂₋-C₈-substituiertem Alkenyl, C₂-C₈-Alkinyl, C₂-C₈-substituiertem Alkinyl, C₁-C₈-Alkoxy, C₁-C₈-substituiertem Alkoxy, Thiol, Alkylthio und substituiertem Alkylthio;
oder R⁶ und R⁷ kovalent aneinander gebunden sein können, um eine gesättigte oder ungesättigte C₃-C₈-Heterocycloalkyl- oder C₃-C₈-substituierte Heterocycloalkylgruppe zu bilden;
jedes R⁷ und R⁸ unabhängig ausgewählt ist aus der Gruppe bestehend aus C₁-C₈-Alkyl, C₁-C₈-substituiertem Alkyl, C₂-C₈-Alkenyl, C₂-C₈-substituiertem Alkenyl, C₂-C₈-Alkinyl, C₂-C₈-substituiertem Alkinyl, C₃-C₈-Cycloalkyl, C₃-C₈-substituiertem Cycloalkyl, Benzyl und substituiertem Benzyl;
oder R⁷ und R⁸ kovalent aneinander gebunden sein können, um eine gesättigte oder ungesättigte C₃-C₈-Heterocycloalkyl- oder C₃-C₈-substituierte Heterocycloalkylgruppe zu bilden;
wobei beliebige und alle heterocyclischen Ringe bis zu drei Heteroatome ausgewählt aus der Gruppe bestehend aus OO, N und S enthalten können;
oder ein Tautomer oder Salz davon,
mit der Maßgabe, dass die Verbindung nicht Ethyl-4-(((dimethylamino)methylen)amino)-3,5-dimethylbenzoat-Hydrochlorid oder Methyl-3-brom-4-[(E)-dimethylaminomethylidenamino]benzoat ist.

2. Verbindung nach Anspruch 1, wobei R¹ ausgewählt ist aus der Gruppe bestehend aus C₁-C₈-Alkyl, C₁-C₈-substituiertem Alkyl, C₂-C₈-Alkinyl, C₂-C₈-substituiertem Alkinyl, C₃-C₈-Cycloalkyl, C₃-C₈-substituiertem Cycloalkyl, Phenyl, substituiertem Phenyl, Benzyl und substituiertem Benzyl, vorzugsweise wobei R¹ ausgewählt ist aus der Gruppe bestehend aus Benzyl und substituiertem Benzyl.

3. Verbindung nach Anspruch 1 oder 2, wobei R² und R⁵ beide Wasserstoff sind.

4. Verbindung nach Anspruch 3, wobei R³ und R⁴ unabhängig ausgewählt sind aus der Gruppe bestehend aus Halogen, C₁-C₈-Alkyl, C₁-C₈-substituiertem Alkyl und C₁-C₈-Alkoxy, wobei R³ und R⁴ beide CH₃ sind.

5. Verbindung nach Anspruch 1 oder 2, wobei R⁴ und R⁵ beide Wasserstoff sind.

6. Verbindung nach Anspruch 5, wobei R² und R³ unabhängig ausgewählt sind aus der Gruppe bestehend aus Halogen, C₁-C₈-Alkyl, C₁-C₈-substituiertem Alkyl und C₁-C₈-Alkoxy, wobei R² und R³ beide CH₃ sind.

7. Verbindung nach einem der Ansprüche 1-6, wobei R⁷ und R⁸ jeweils unabhängig ausgewählt sind aus der Gruppe bestehend aus C₁-C₈-Alkyl, C₁-C₈-substituiertem Alkyl, C₂-C₈-Alkenyl, C₃-C₈-Cycloalkyl, Benzyl und substituiertem Benzyl; und/oder
wobei R⁶ ausgewählt ist aus der Gruppe bestehend aus Wasserstoff, C₁-C₈-Alkyl, C₁-C₈-substituiertem Alkyl, Thiol, Alkylthio und substituiertem Alkylthio.

8. Verbindung nach einem der vorstehenden Ansprüche, die ausgewählt ist aus der folgenden Tabelle:
| Verb. Nr. | Struktur |
|---|---|
| 1 | 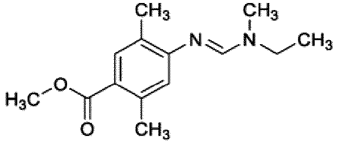 |
| 3 | 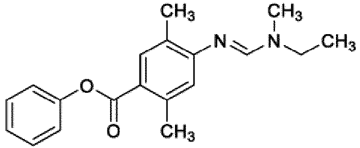 |
| 4 | 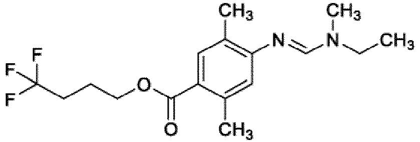 |
| 5 | 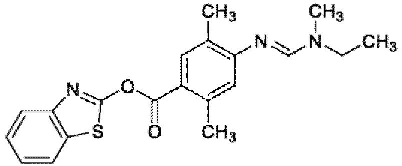 |
| 6 | 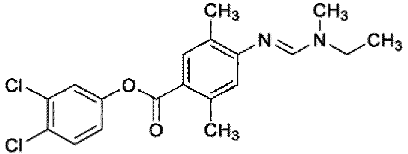 |
| 7 | 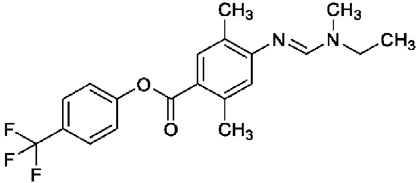 |
| 8 | 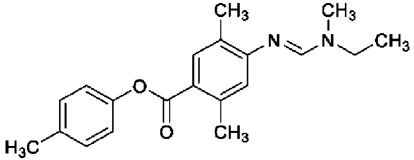 |
| 9 | 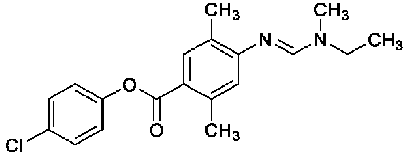 |
| 10 | 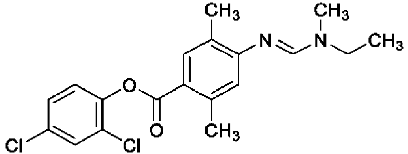 |
| 11 | 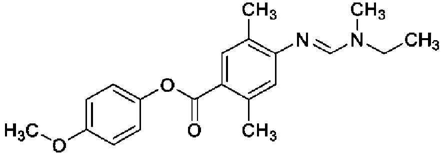 |
| 12 | 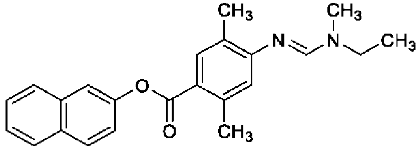 |
| 13 | 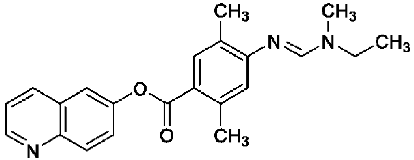 |
| 14 | 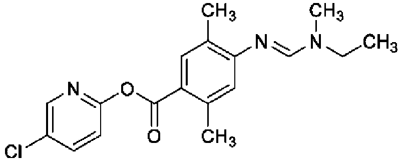 |
| 15 | 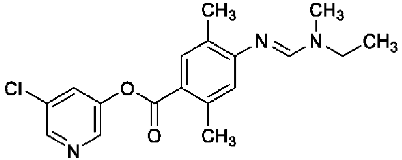 |
| 16 | 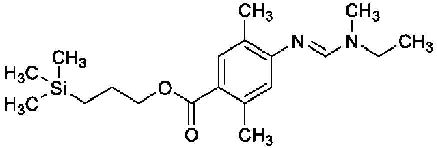 |
| 17 | 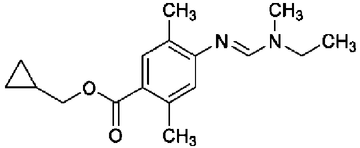 |
| 18 | 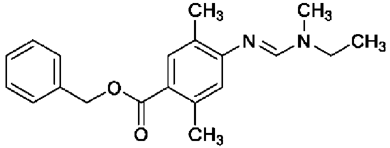 |
| 19 | 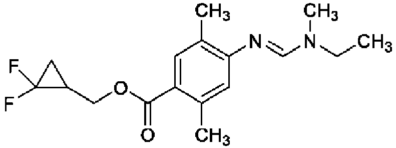 |
| 20 | 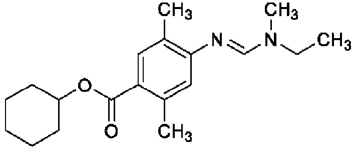 |
| 21 | 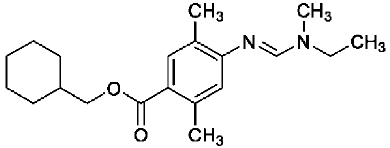 |
| 22 | 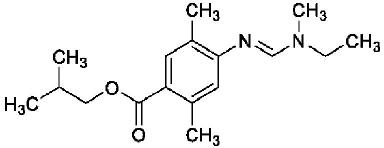 |
| 23 | 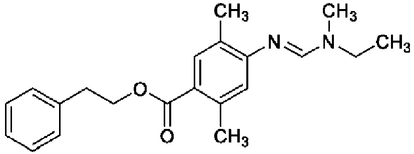 |
| 24 | 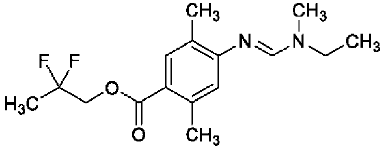 |
| 25 | 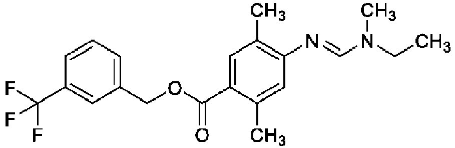 |
| 26 | 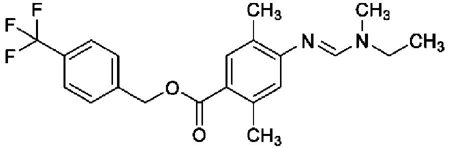 |
| 27 | 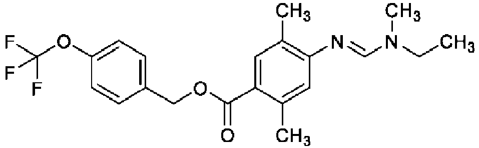 |
| 28 | 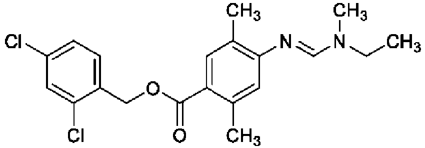 |
| 29 | 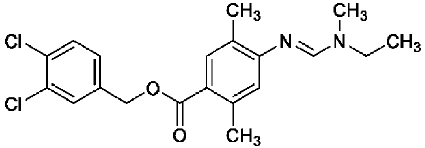 |
| 30 | 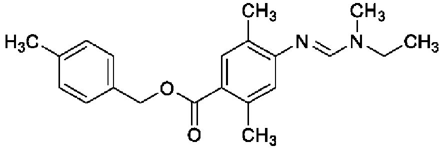 |
| 31 | 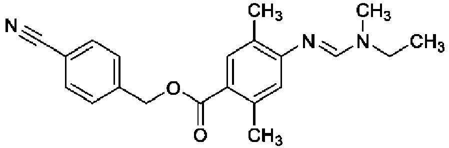 |
| 32 | 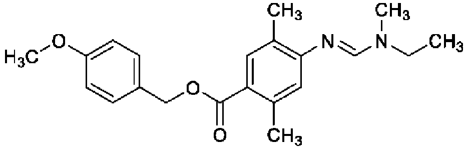 |
| 33 | 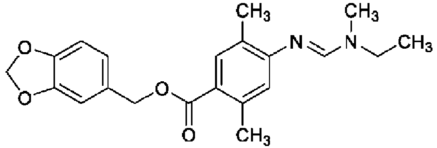 |
| 34 | 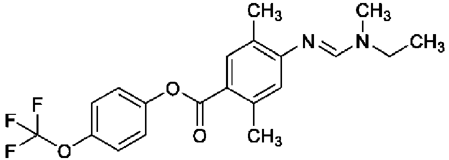 |
| 35 | 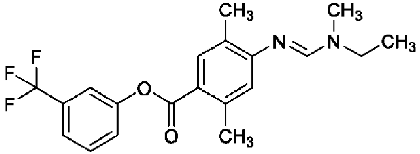 |
| 36 | 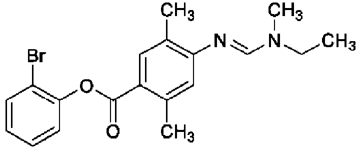 |
| 37 | 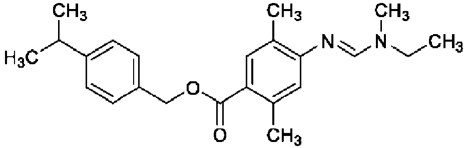 |
| 38 | 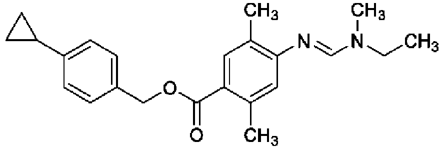 |
| 39 | 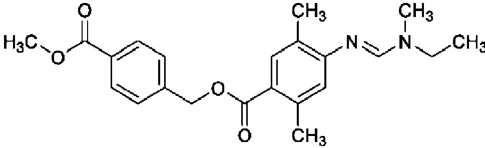 |
| 40 | 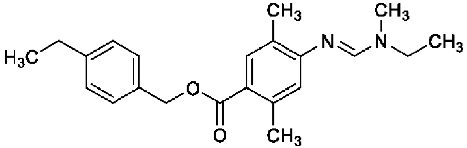 |
| 41 | 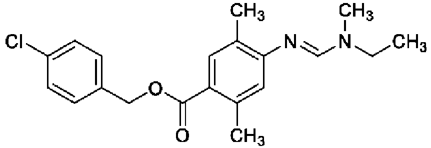 |
| 42 | 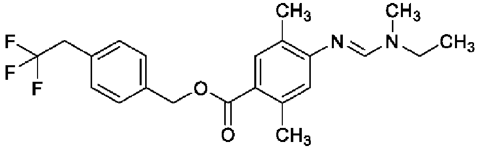 |
| 43 | 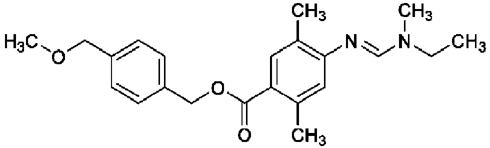 |
| 44 | 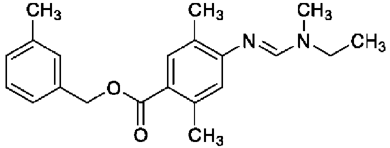 |
| 45 | 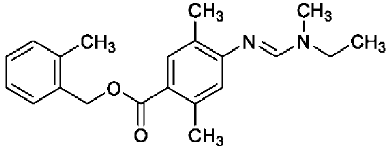 |
| 46 | 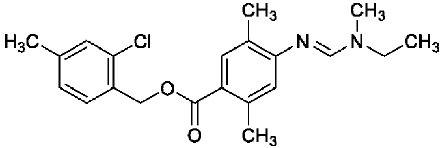 |
| 47 | 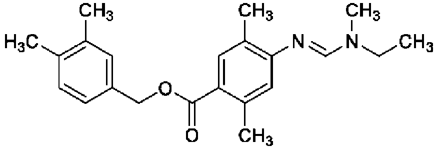 |
| 48 | 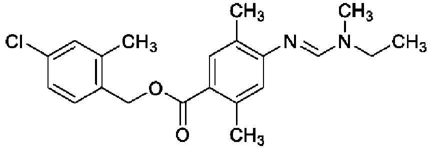 |
| 49 | 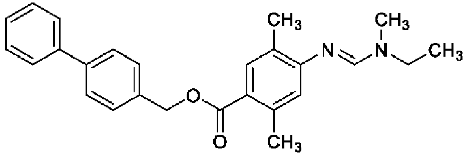 |
| 50 | 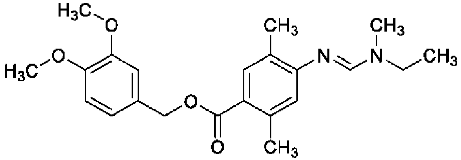 |
| 51 | 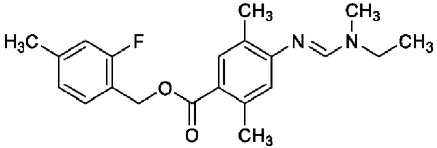 |
| 52 | 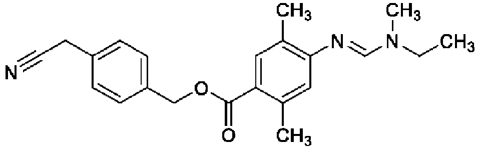 |
| 53 | 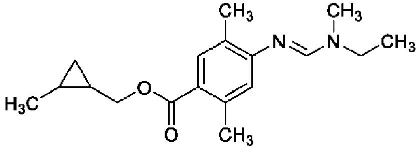 |
| 54 | 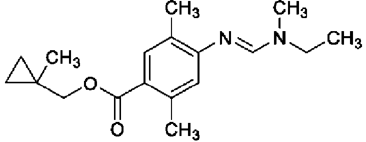 |
| 55 | 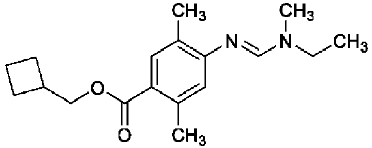 |
| 56 | 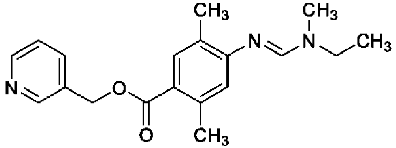 |
| 57 | 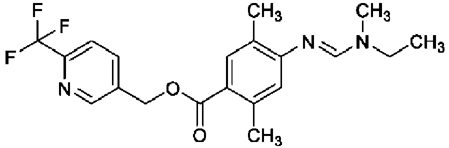 |
| 58 | 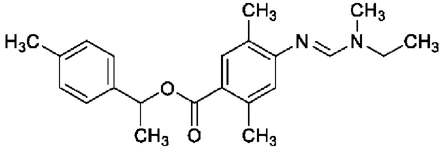 |
| 59 | 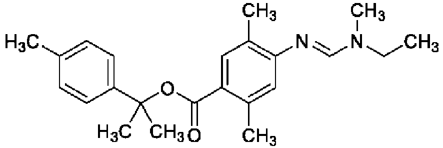 |
| 60 | 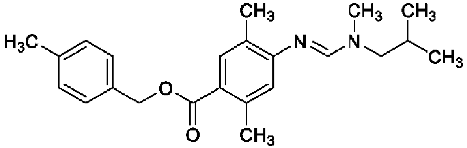 |
| 61 | 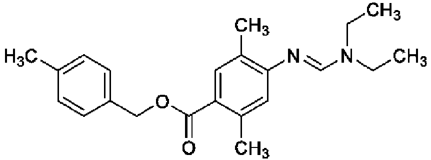 |
| 62 | 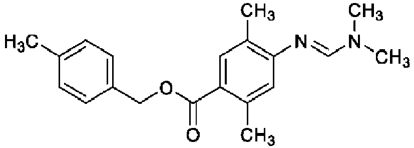 |
| 63 | 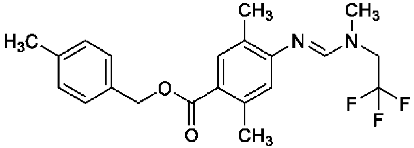 |
| 64 | 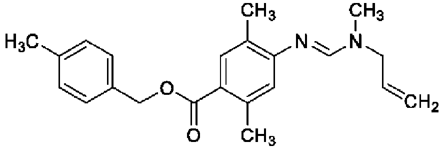 |
| 65 | 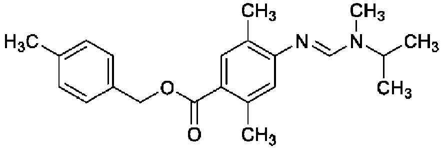 |
| 66 | 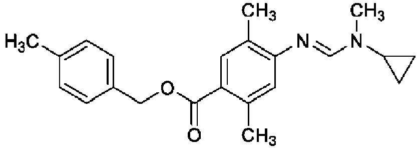 |
| 67 | 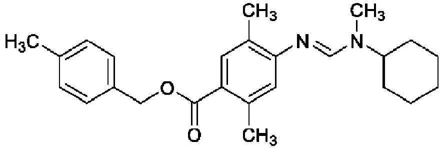 |
| 68 | 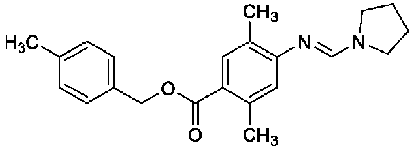 |
| 69 | 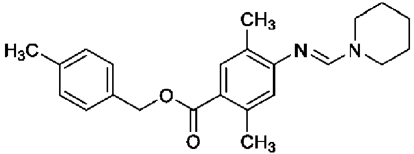 |
| 70 | 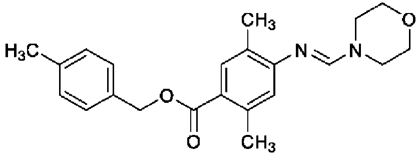 |
| 72 | 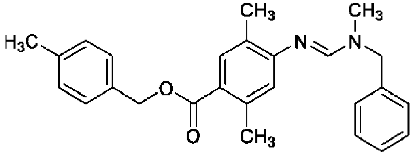 |
| 73 | 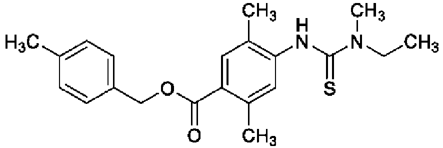 |
| 74 | 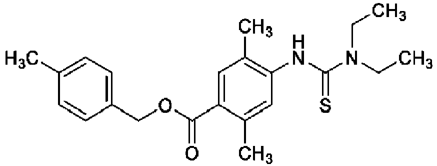 |
| 75 | 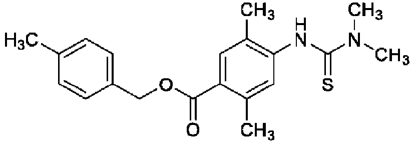 |
| 76 | 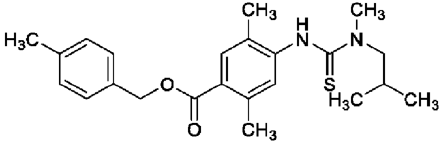 |
| 77 | 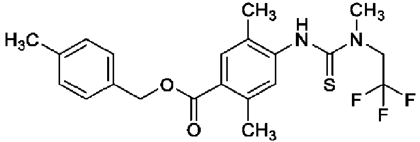 |
| 78 | 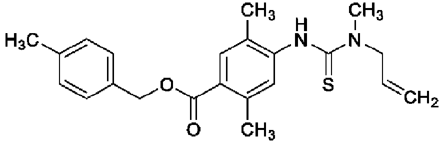 |
| 79 | 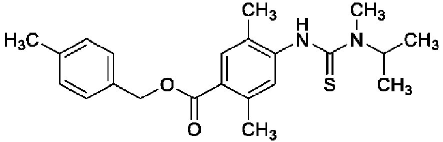 |
| 80 | 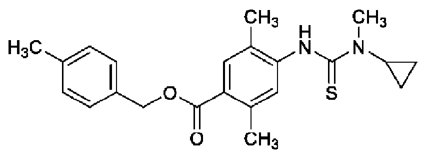 |
| 81 | 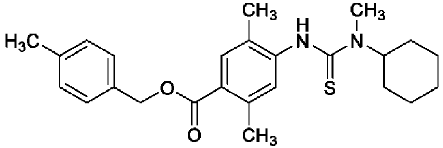 |
| 82 | 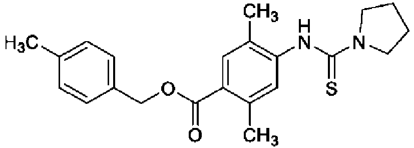 |
| 83 | 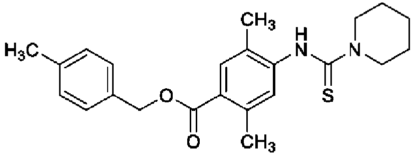 |
| 84 | 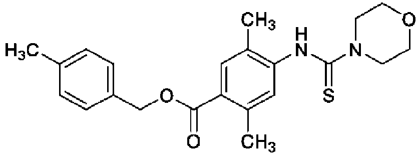 |
| 86 | 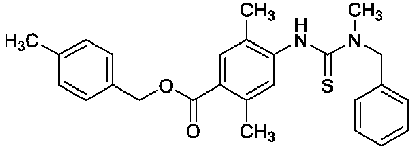 |
| | |
| | |
| 89 | 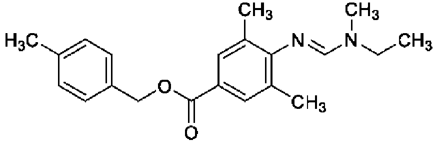 |
| 90 | 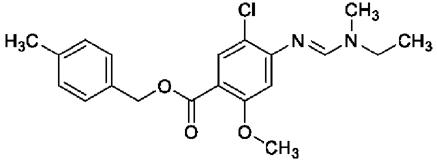 |
| 91 | 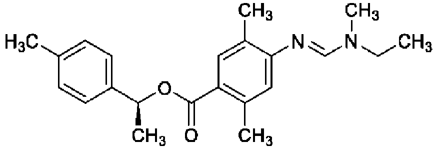 |
| 92 | 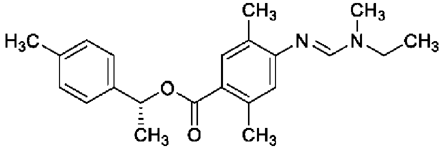 |
| | |
| 94 | 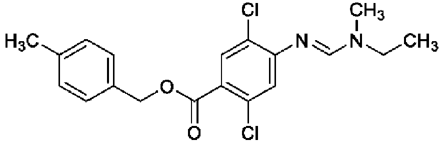 |
| 95 | 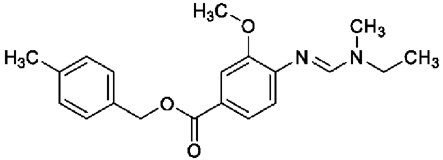 |
| 96 | 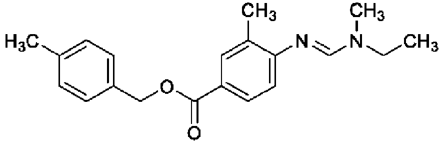 |
| 97 | 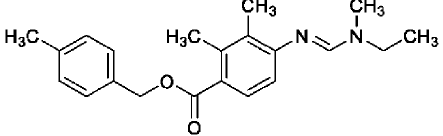 |
| 98 | 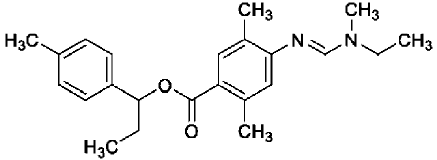 |
| 99 | 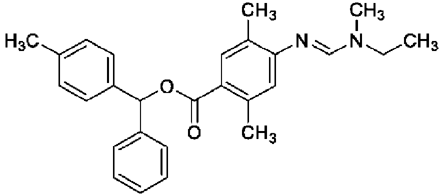 |
| 100 | 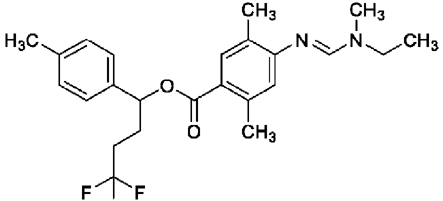 |
| 101 | 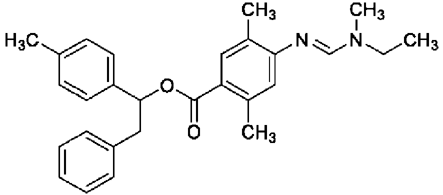 |
| | |
| 103 | 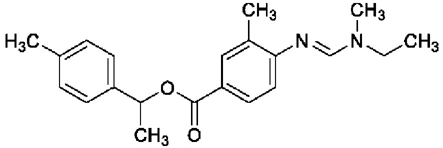 |
| 105 | 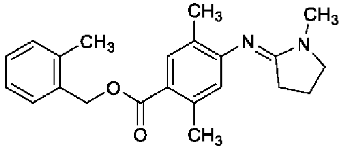 |
| 106 | 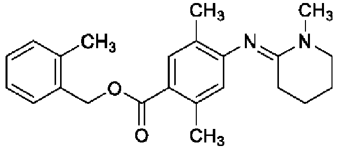 |
| 107 | 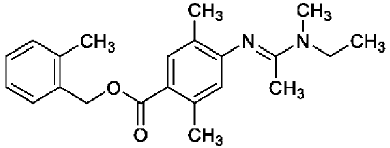 |
| 109 | 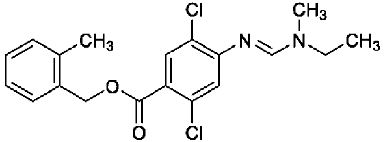 |
| 110 | 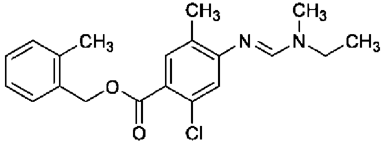 |
| 111 | 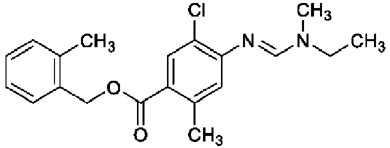 |
| 112 | 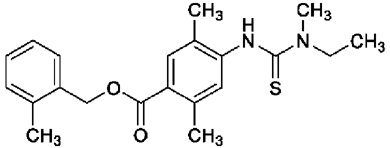 |
| 113 | 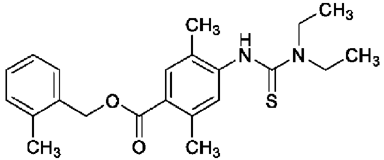 |
| 114 | 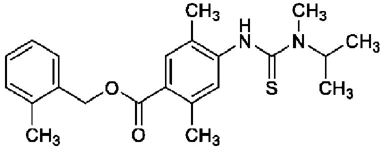 |
| 115 | 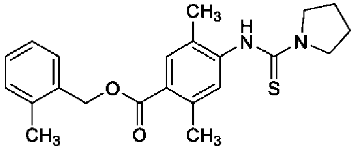 |
| 116 | 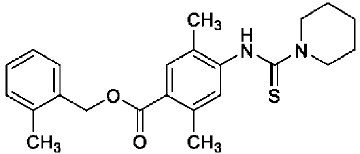 |
| 117 | 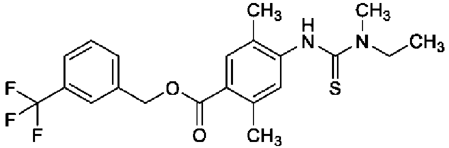 |
| 118 | 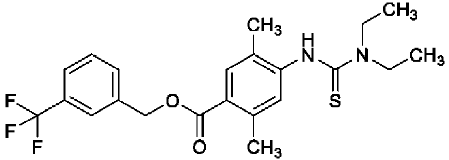 |
| 119 | 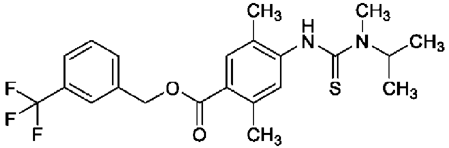 |
| 120 | 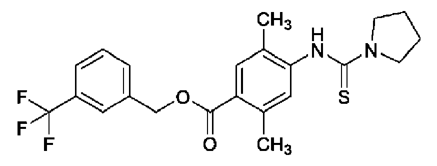 |
| 121 | 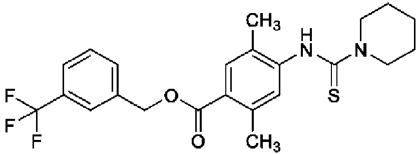 |
| 122 | 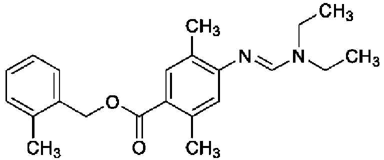 |
| 123 | 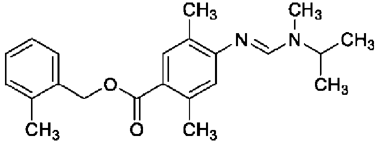 |
| 124 | 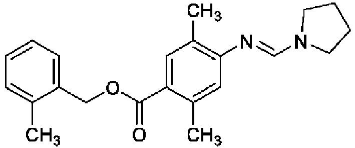 |
| 125 | 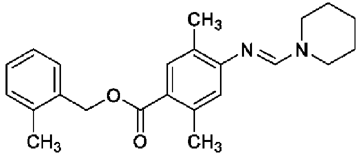 |
| 126 | 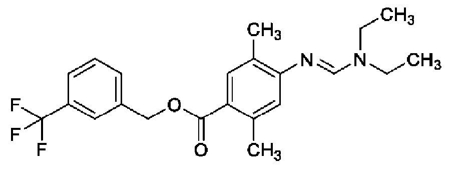 |
| 127 | 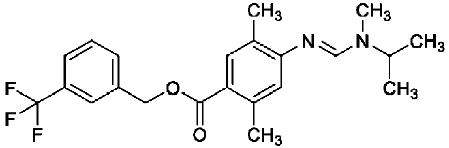 |
| 128 | 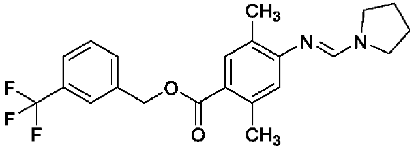 |
| 129 | 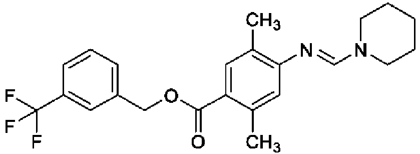 |
| 130 | 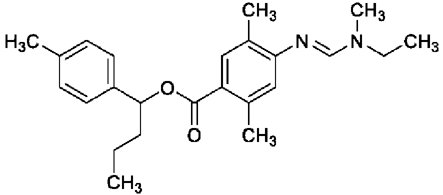 |
| 131 | 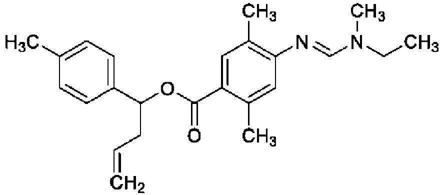 |
| 132 | 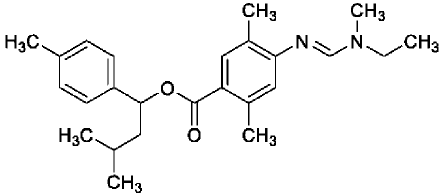 |
| 133 | 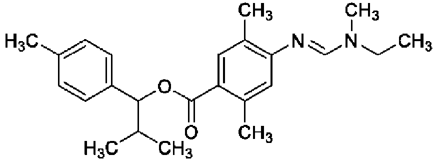 |
| 134 | 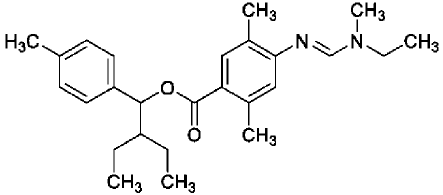 |
| 135 | 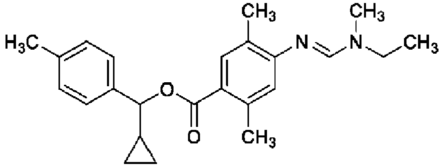 |
| 136 | 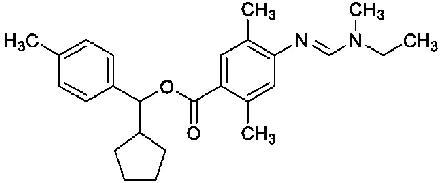 |
| 137 | 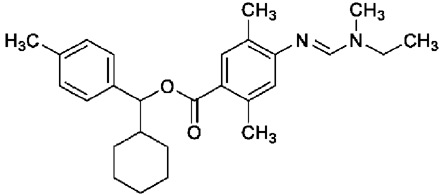 |
| 138 | 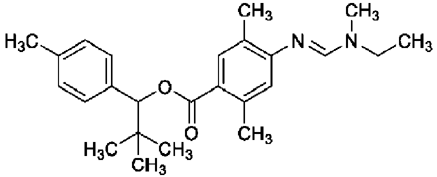 |
| 139 | 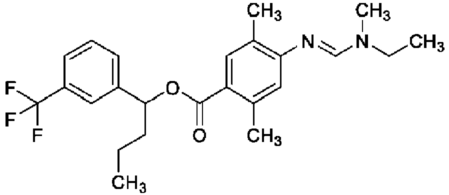 |
| 140 | 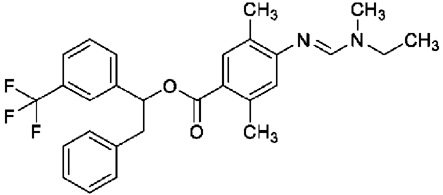 |
| 141 | 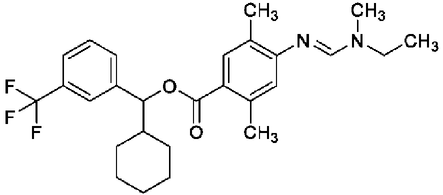 |
| 142 | 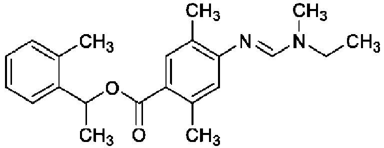 |
| 143 | 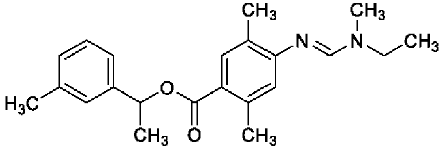 |
| 144 | 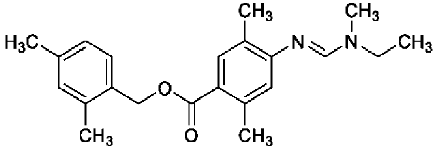 |
| 145 | 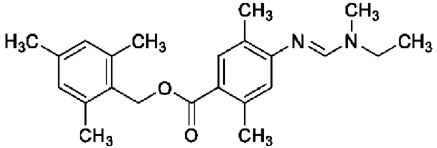 |
| 146 | 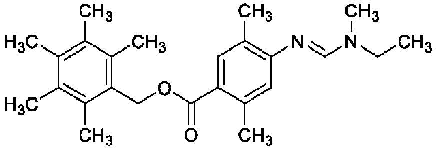 |
| 147 | 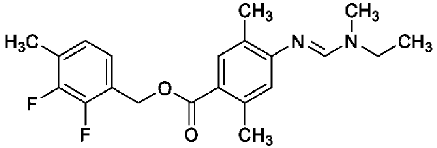 |
| 148 | 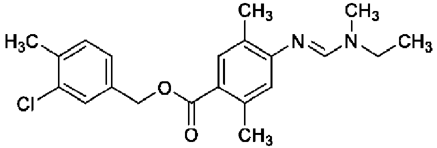 |
| 149 | 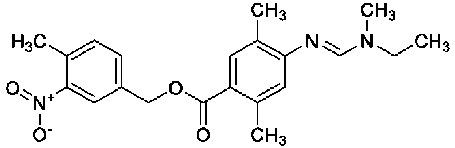 |
| 150 | 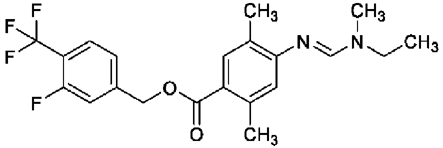 |
| 151 | 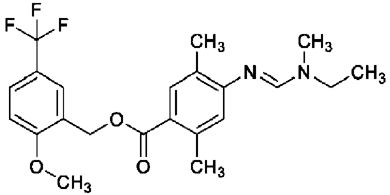 |
| 152 | 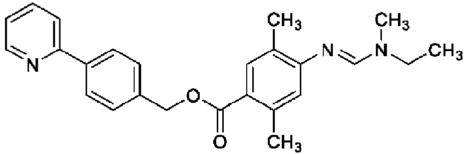 |
| 153 | 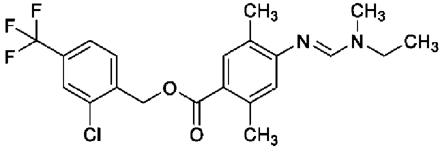 |
| 154 | 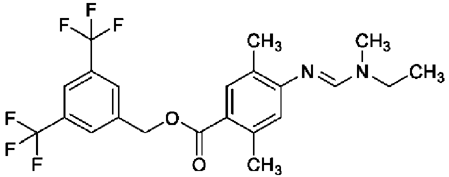 |
| 155 | 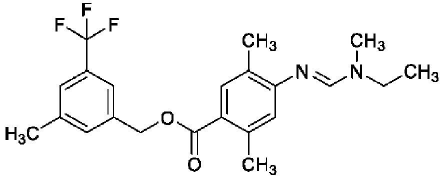 |
| 156 | 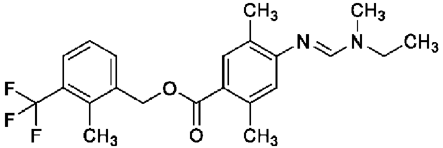 |
| 157 | 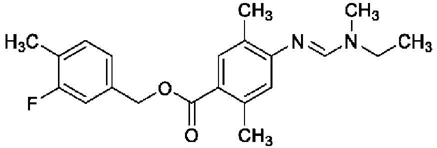 |
| 158 | 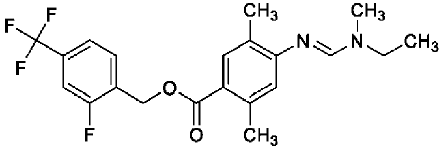 |
| 159 | 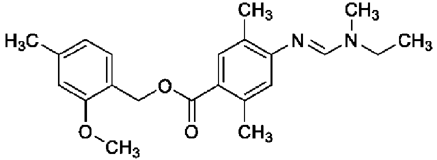 |
| 160 | 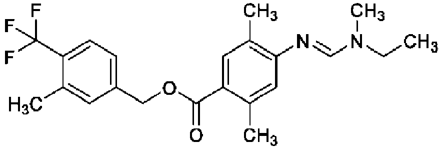 |
| 161 | 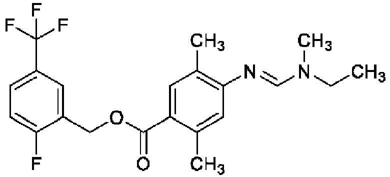 |
| 162 | 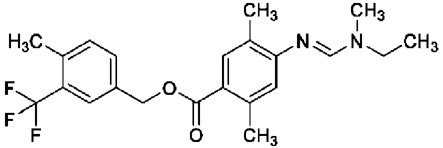 |
| 163 | 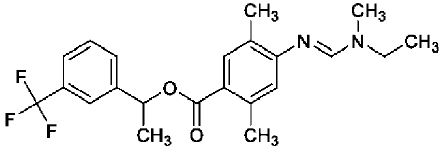 |
| 164 | 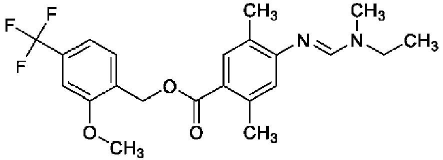 |
| 165 | 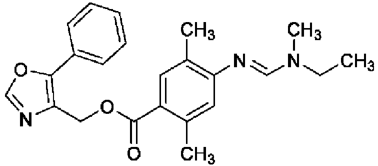 |
| 166 | 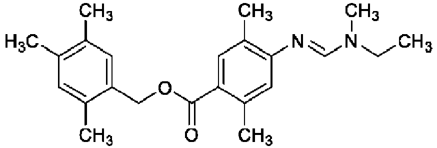 |
| 167 | 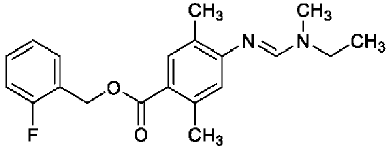 |
| 168 | 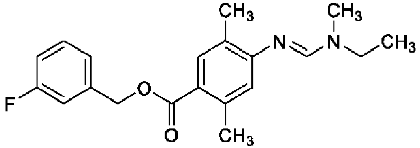 |
| 169 | 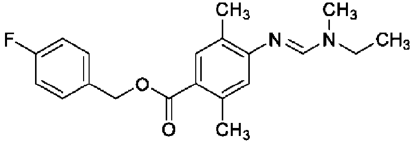 |
| 170 | 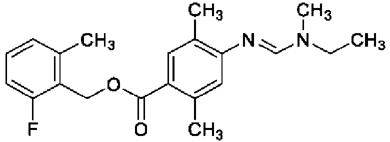 |
| 171 | 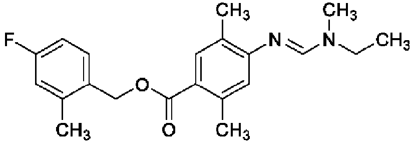 |
| 172 | 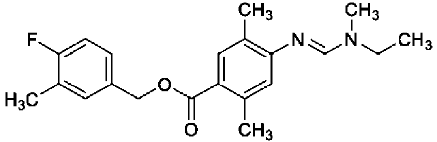 |
| 173 | 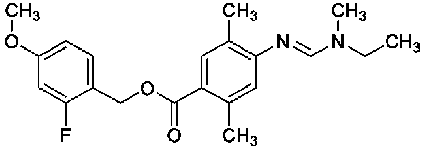 |
| 174 | 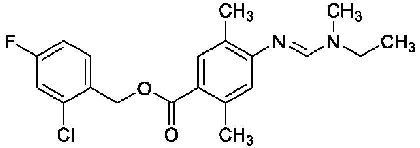 |
| 175 | 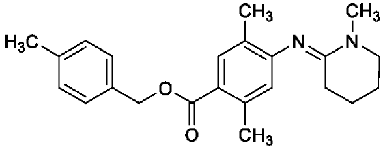 |
| 176 | 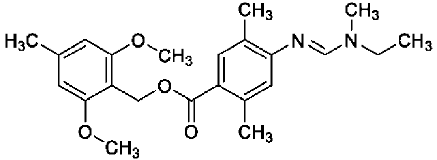 |
| 177 | 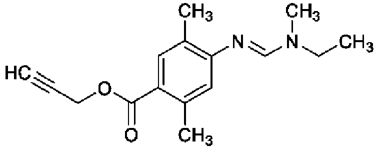 |
| 178 | 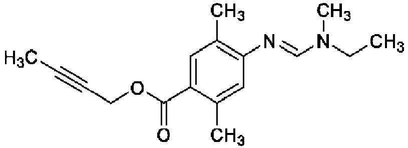 |
| 179 | 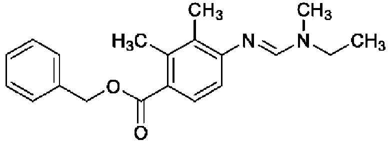 |
| 180 | 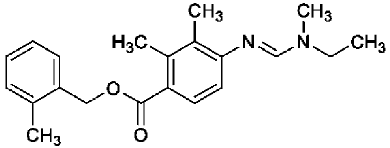 |
| 181 | 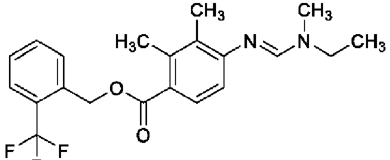 |
| 172 | 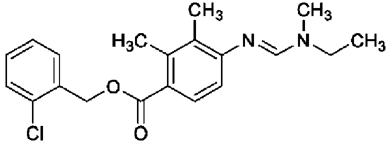 |
| 183 | 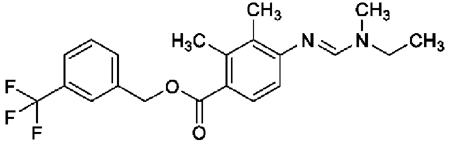 |
| 184 | 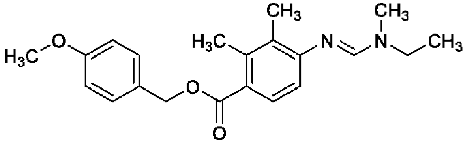 |
| 185 | 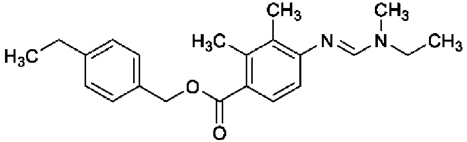 |
| 186 | 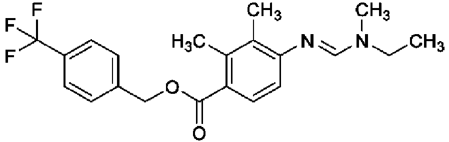 |
| 187 | 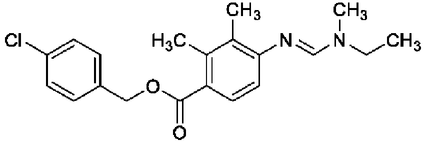 |
| 188 | 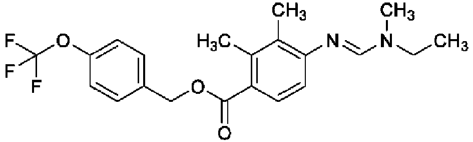 |
| 189 | 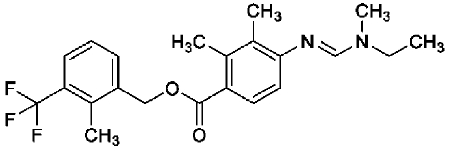 |
| 190 | 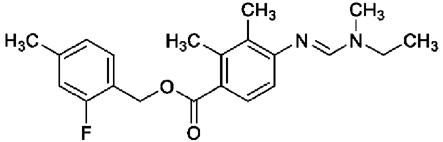 |
| 191 | 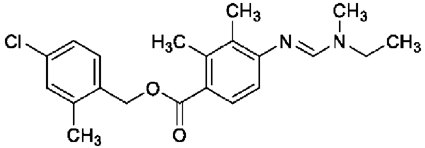 |
| 192 | 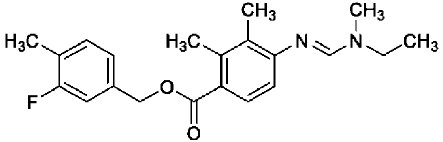 |
| 193 | 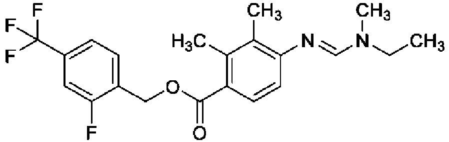 |
| 194 | 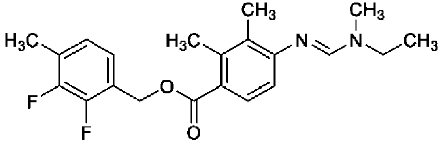 |
| 195 | 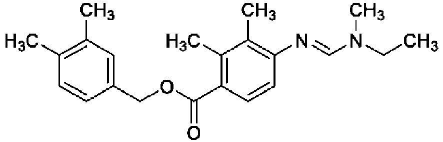 |
| 196 | 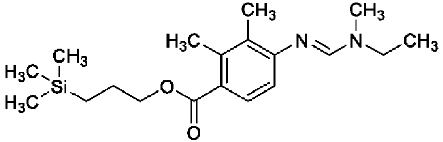 |
| 197 | 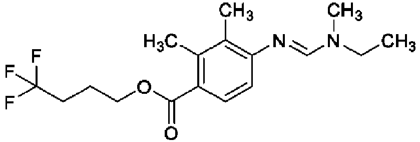 |
| 198 | 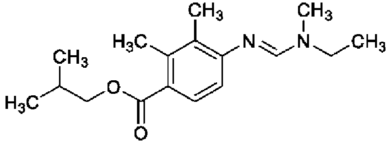 |
| 199 | 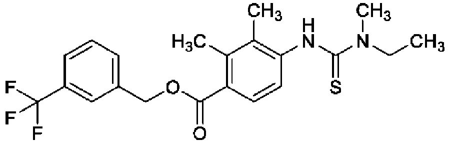 |
| 200 | 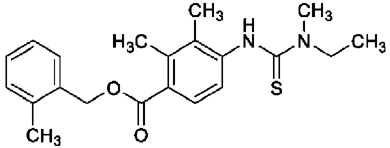 |
| 201 | 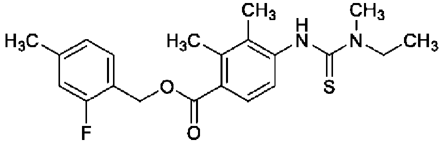 |
| 202 | 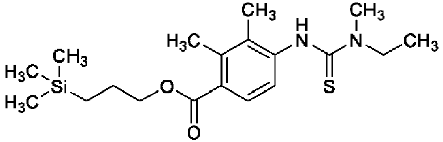 |
| 203 | 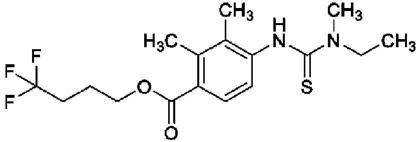 |
| 204 | 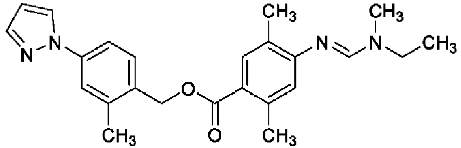 |
| 205 | 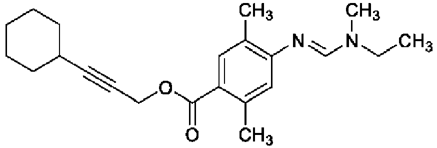 |
| 206 | 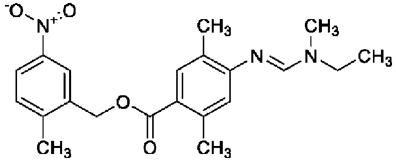 |
| 207 | 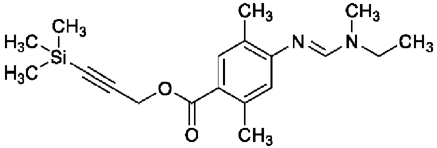 |
| 208 | 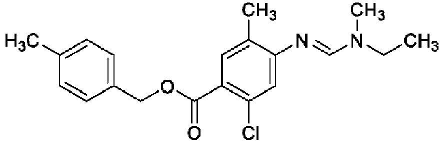 |
| 209 | 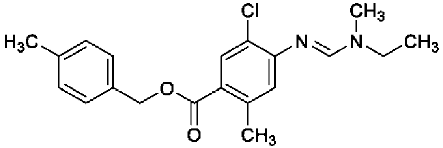 |
| 210 | 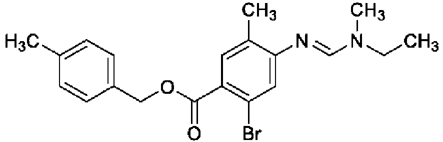 |
| | |
| 212 | 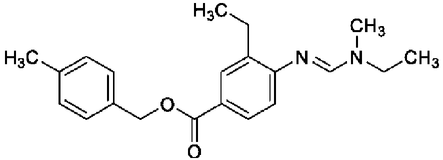 |
| 213 | 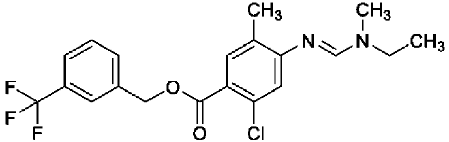 |
| 214 | 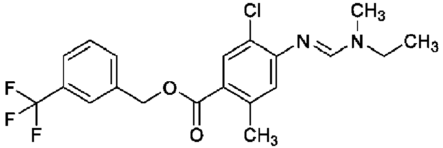 |
| 215 | 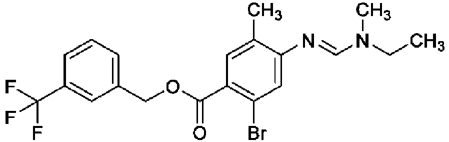 |
| | |
| 217 | 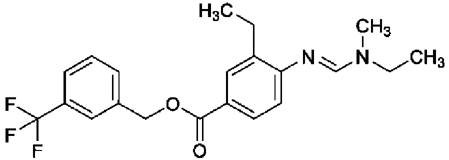 |
| 218 | 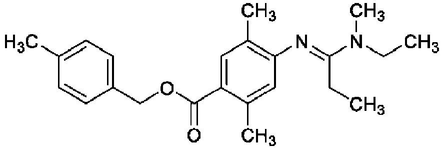 |
| 219 | 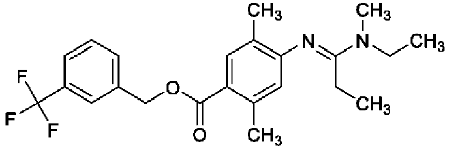 |
| 220 | 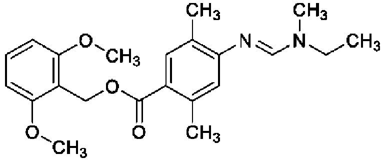 |
| 221 | 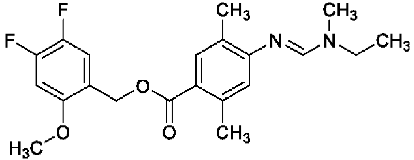 |
| 222 | 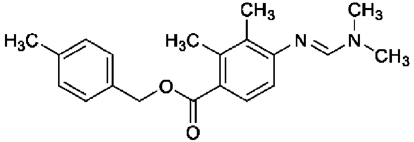 |
| 223 | 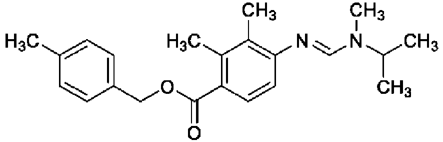 |
| 224 | 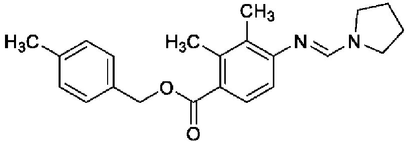 |
| 225 | 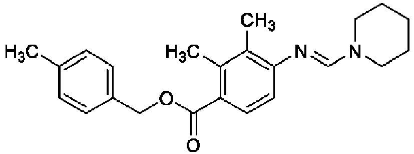 |
| 226 | 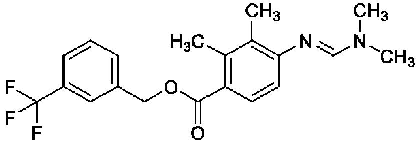 |
| 227 | 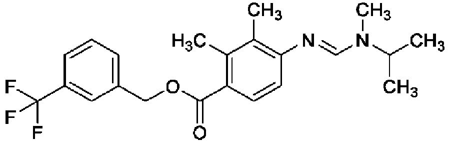 |
| 228 | 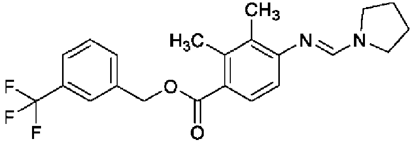 |
| 229 | 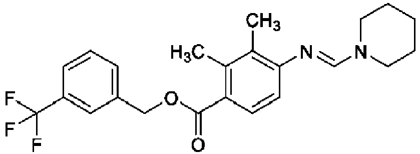 |
| 230 | 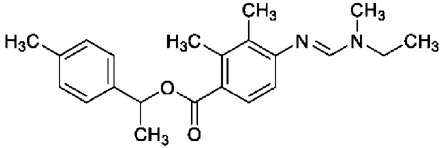 |
| 231 | 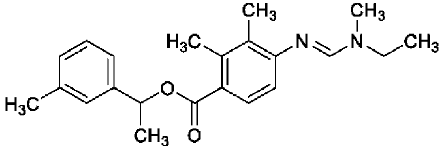 |
| 232 | 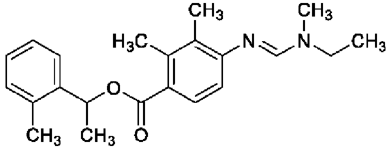 |
| 233 | 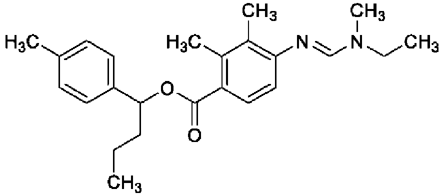 |
| 234 | 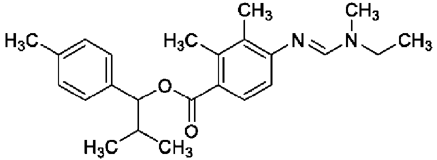 |
| 235 | 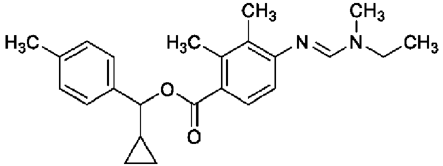 |
| 236 | 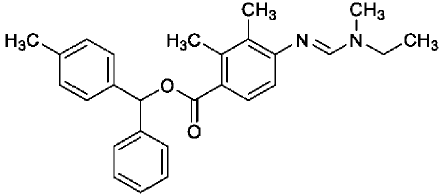 |
| 237 | 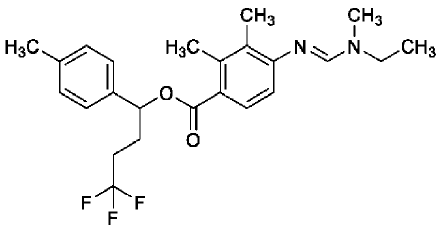 |
| 238 | 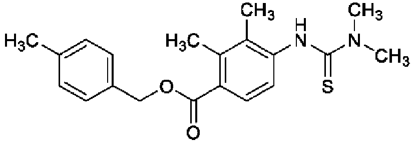 |
| 239 | 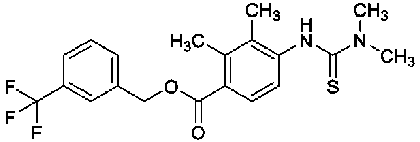 |
| 240 | 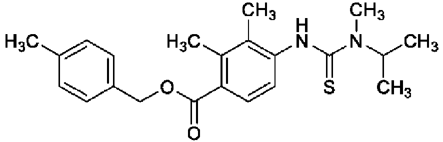 |
| 241 | 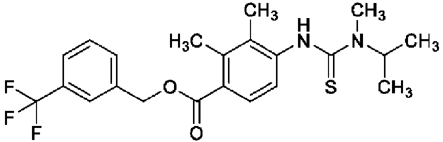 |
| 242 | 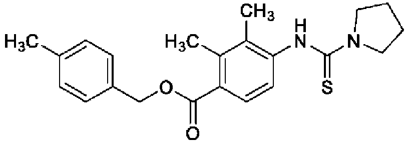 |
| 243 | 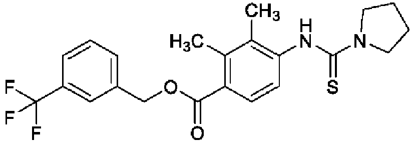 |
| 244 | 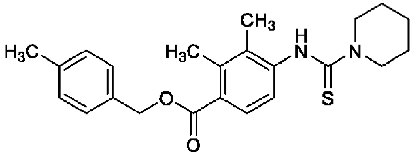 |
| 245 | 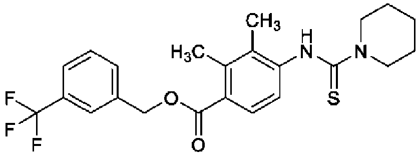 |
| 246 | 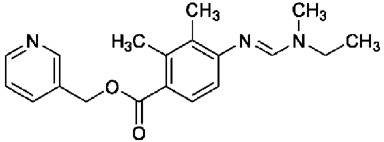 |
| 247 | 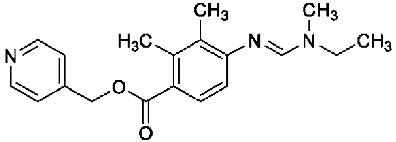 |
| 248 | 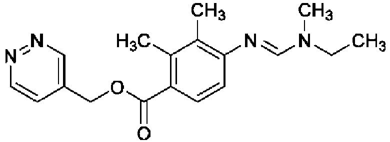 |
| 249 | 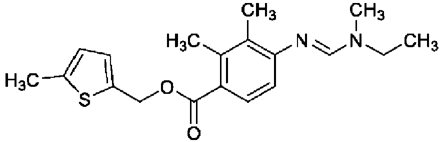 |
| 250 | 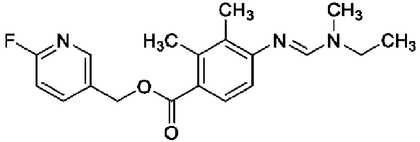 |
| 251 | 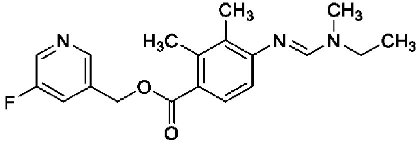 |
| 252 | 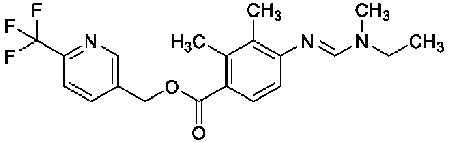 |
| 253 | 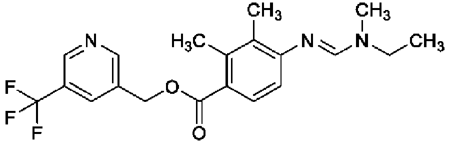 |
| 254 | 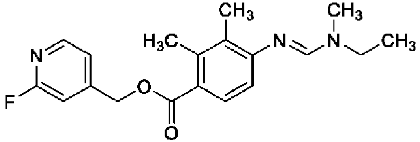 |
| 255 | 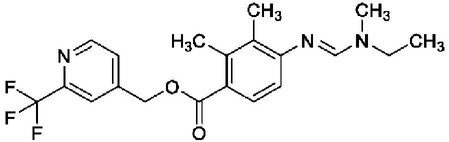 |
| 256 | 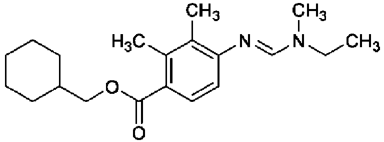 |
| 257 | 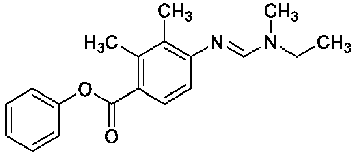 |
| 258 | 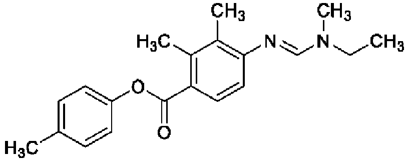 |
| 259 | 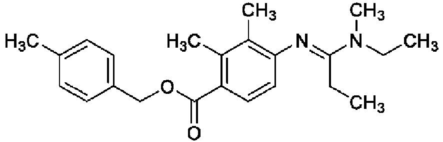 |
| 260 | 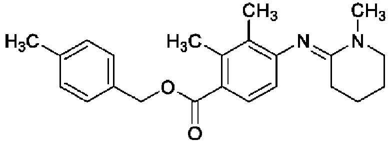 |
| 261 | 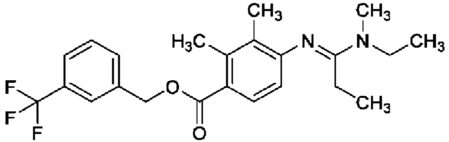 |
| 262 | 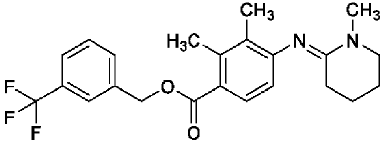 |
| 263 | 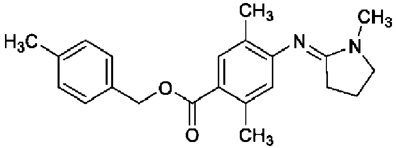 |
| 264 | 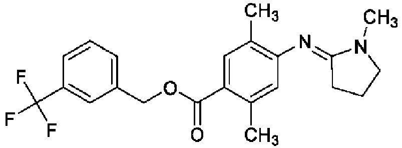 |
| 265 | 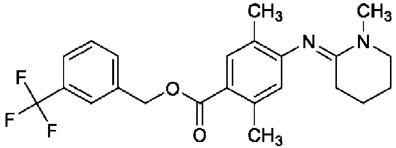 |
| 266 | 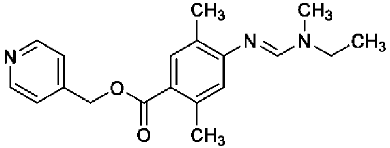 |
| 267 | 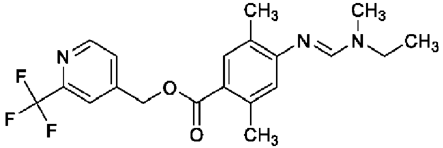 |
| 268 | 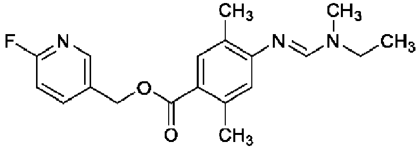 |
| 269 | 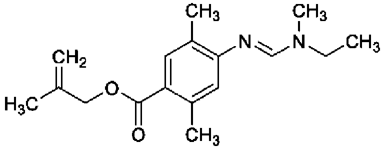 |
| 270 | 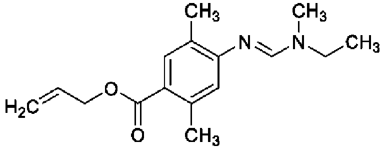 |
| 271 | 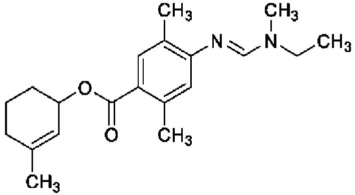 |
| 272 | 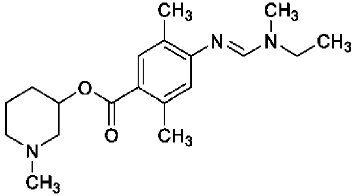 |
| 273 | 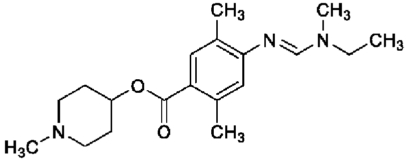 |
| 274 | 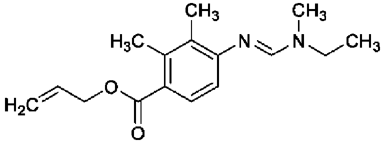 |
| 275 | 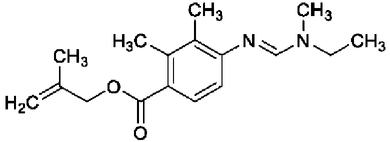 |
| 276 | 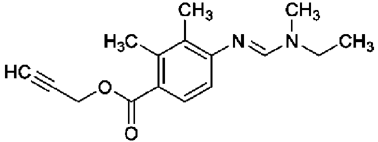 |
| 277 | 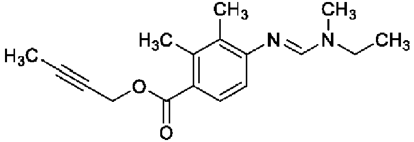 |
| 278 | 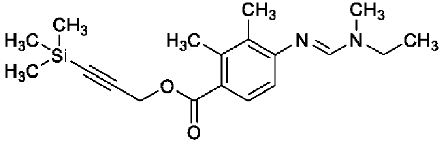 |
| 279 | 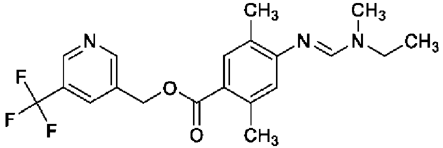 |
| 280 | 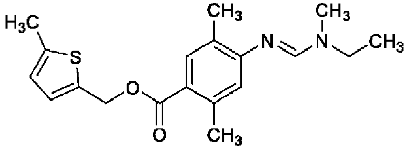 |
| 285 | 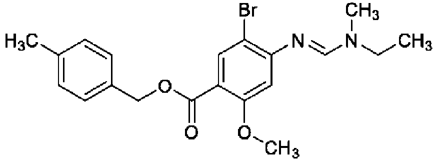 |
| 286 | 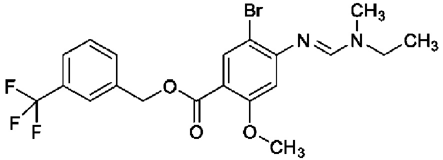 |
| 287 | 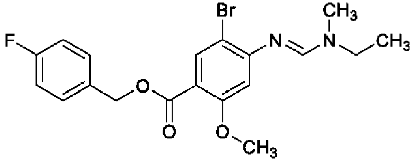 |
| 288 | 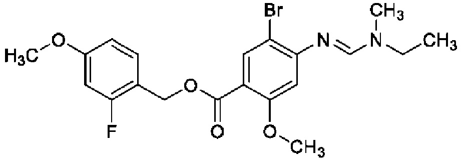 |
| 289 | 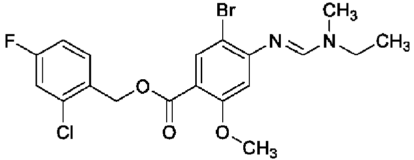 |
| 290 | 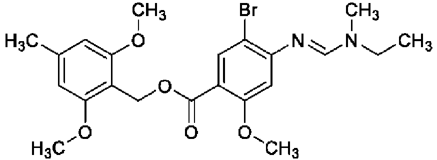 |
| 291 | 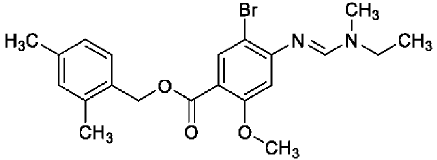 |
| 292 | 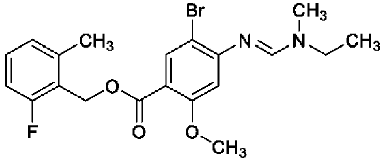 |
| 293 | 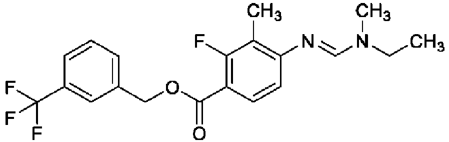 |
| 294 | 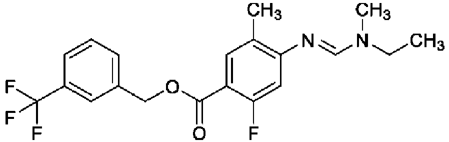 |
| 295 | 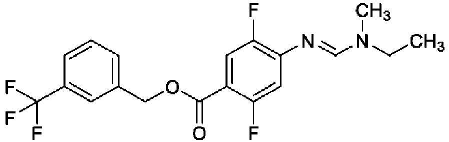 |
| 296 | 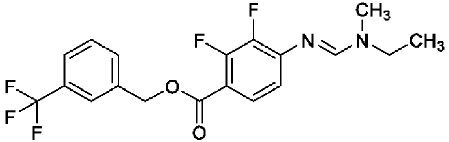 |
| 297 | 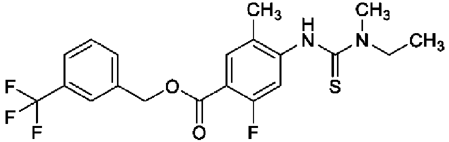 |
| 298 | 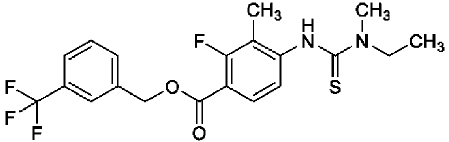 |
| 299 | 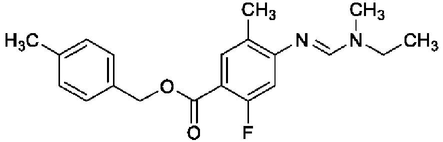 |
| 300 | 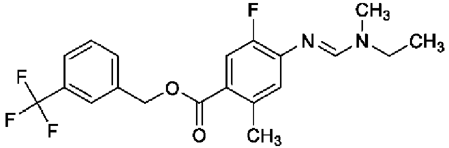 |
| 301 | 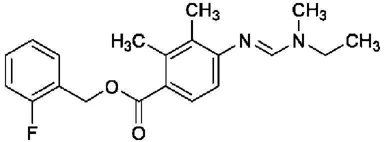 |
| 302 | 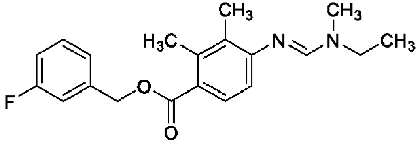 |
| 303 | 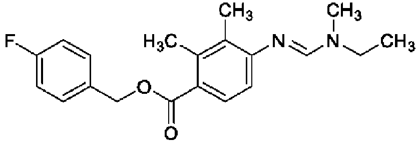 |
| 304 | 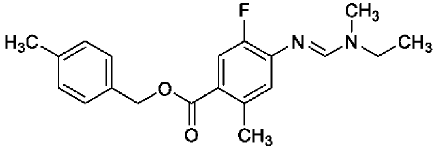 |
| 305 | 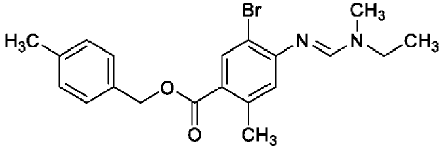 |
| 306 | 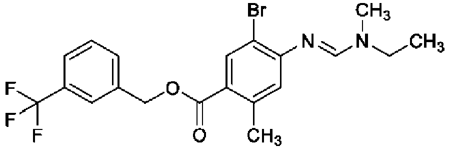 |
| 307 | 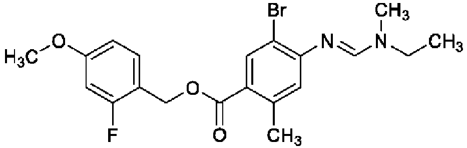 |
| 308 | 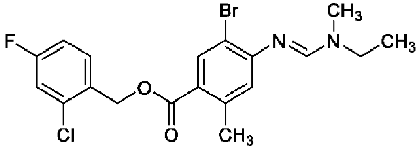 |
| 309 | 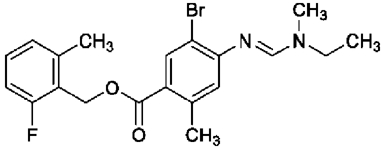 |
| 310 | 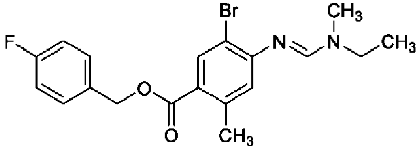 |
| 311 | 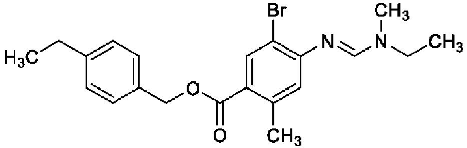 |
| 312 | 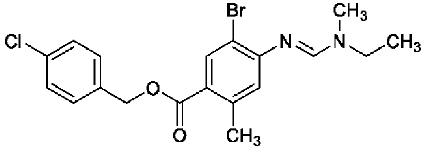 |
| 313 | 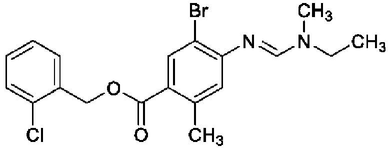 |
| 314 | 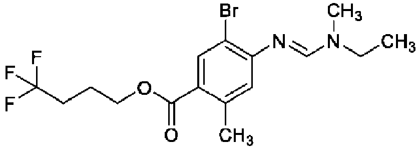 |
| 315 | 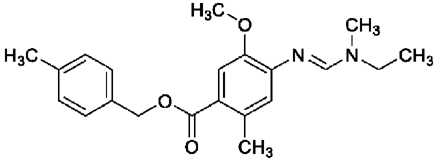 |
| 316 | 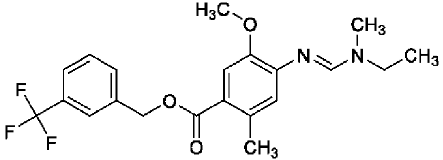 |
| 317 | 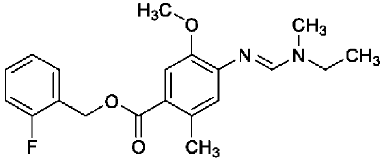 |
| 318 | 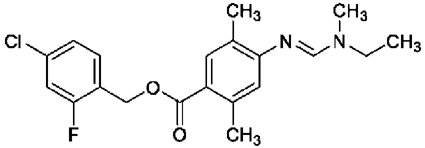 |
| 319 | 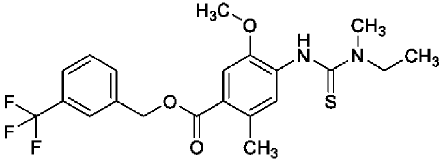 |
| 320 | 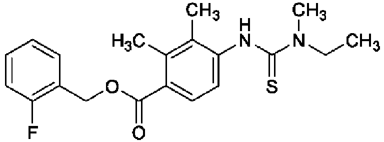 |
| 321 | 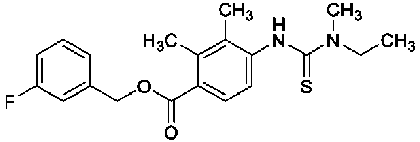 |
| 322 | 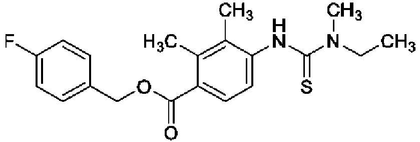 |
| 323 | 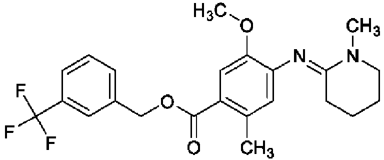 |
| 324 | 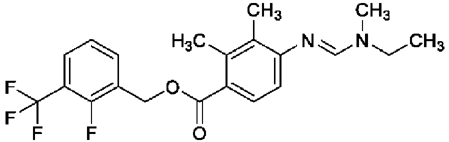 |
| 325 | 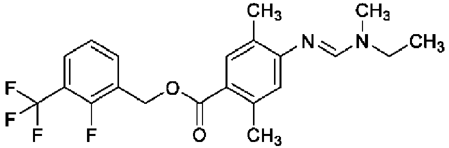 |
| 326 | 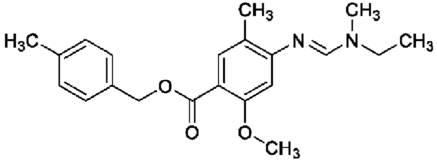 |
| 327 | 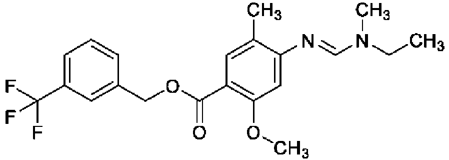 |
| 328 | 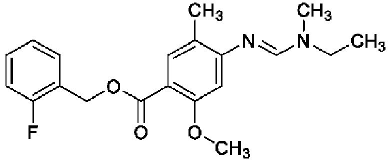 |
| 329 | 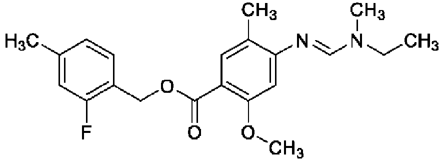 |
| 330 | 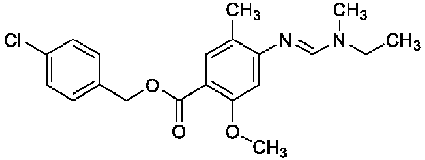 |
| 331 | 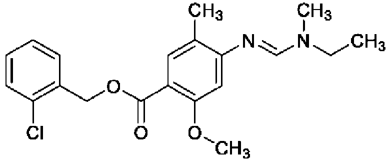 |
| 332 | 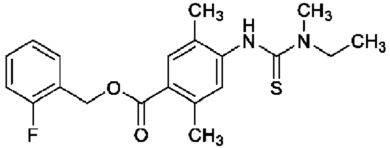 |
| 333 | 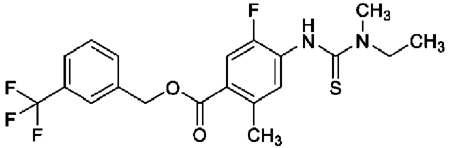 |
| 334 | 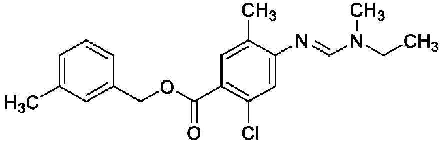 |
| 335 | 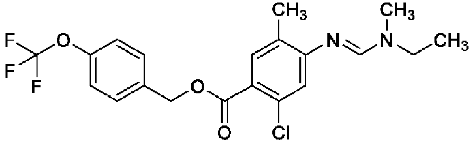 |
| 336 | 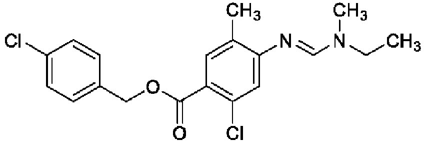 |
| 337 | 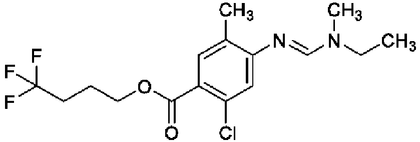 |
| 338 | 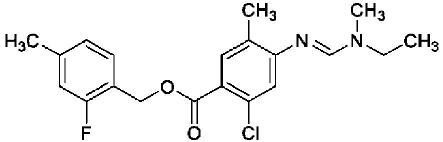 |
| 339 | 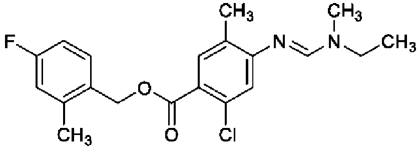 |
| 340 | 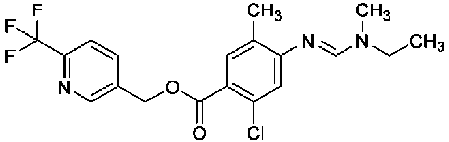 |
| 341 | 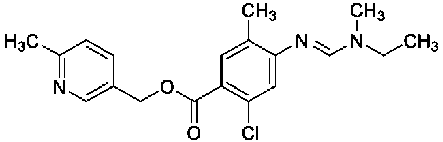 |
| 342 | 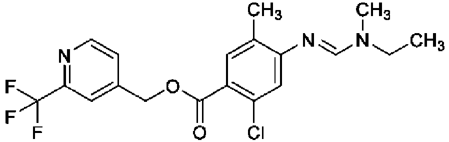 |
| 343 | 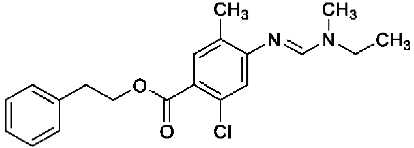 |
| 344 | 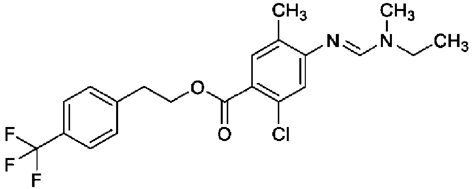 |
| 345 | 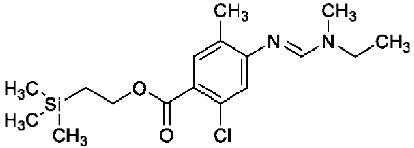 |
| 346 | 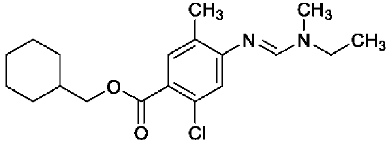 |
| 347 | 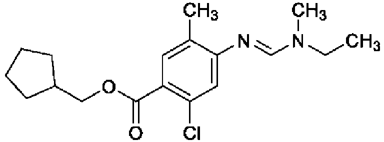 |
| 348 | 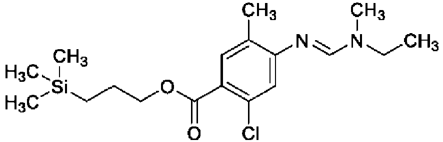 |
| 349 | 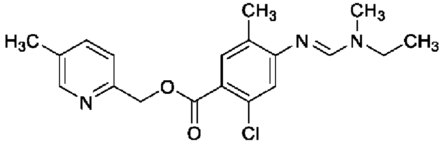 |
| 350 | 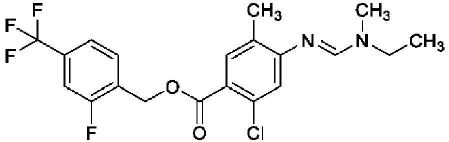 |
| 351 | 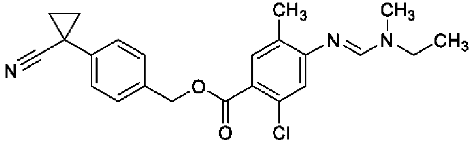 |
| 352 | 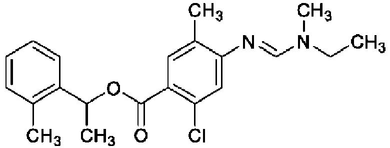 |
| 353 | 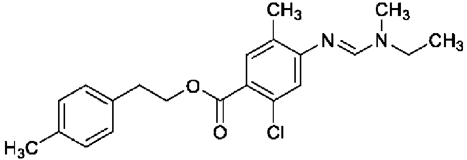 |
| 354 | 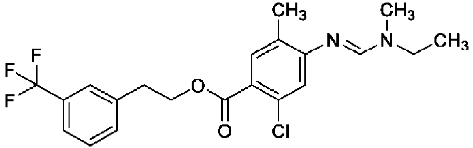 |
| 355 | 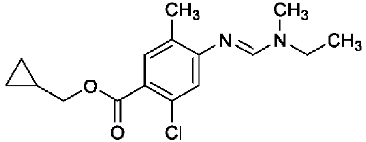 |
| 356 | 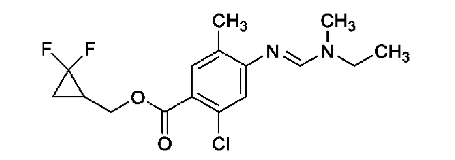 |
| 357 | 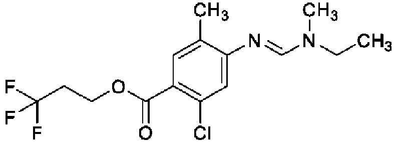 |
| 358 | 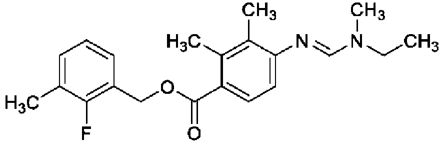 |
| 359 | 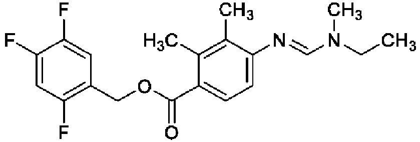 |
| 360 | 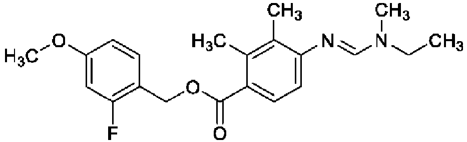 |
| 361 | 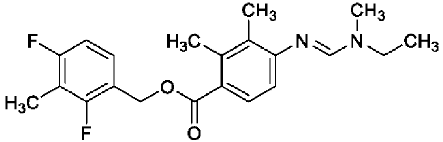 |
| 362 | 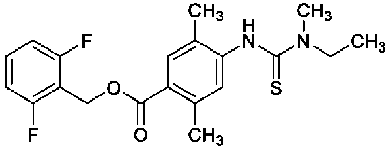 |
| 363 | 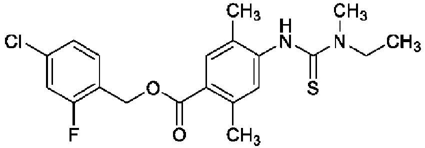 |
| 364 | 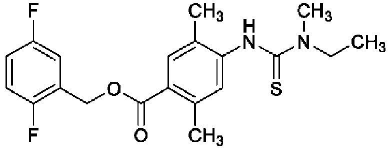 |
| 365 | 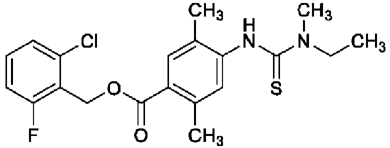 |
| 366 | 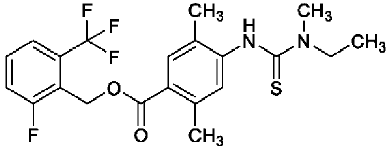 |
| 367 | 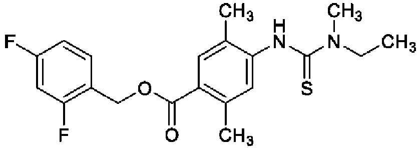 |
| 368 | 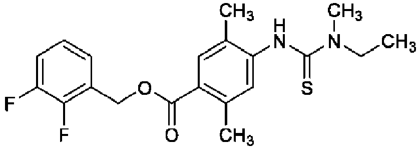 |
| 369 | 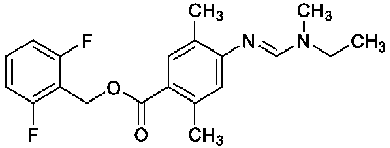 |
| 370 | 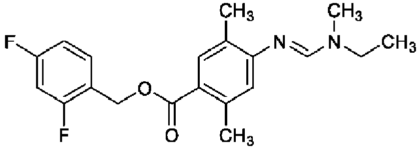 |
| 371 | 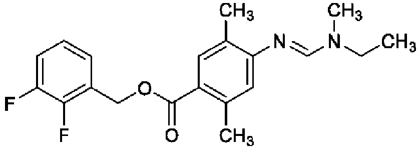 |
| 372 | 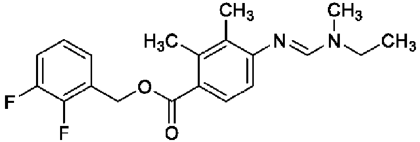 |
| 373 | 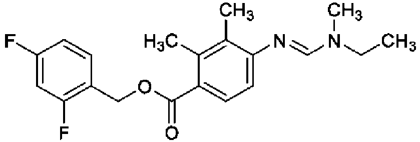 |
| 374 | 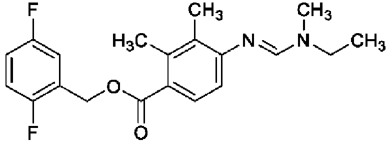 |
| 375 | 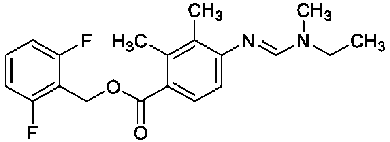 |
| 376 | 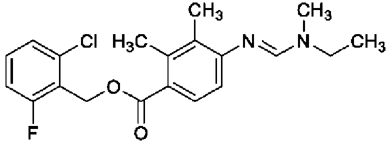 |
| 377 | 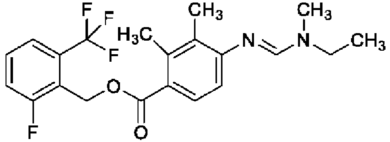 |
| 378 | 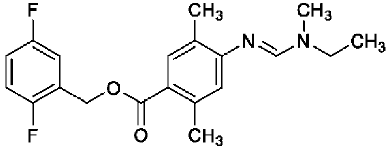 |
| 379 | 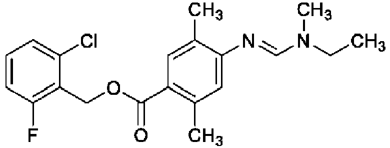 |
| 380 | 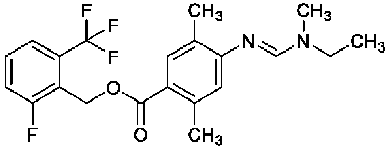 |
| 381 | 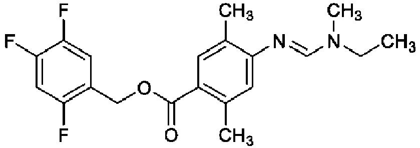 |
| 382 | 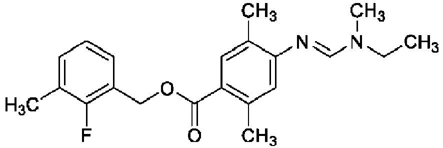 |
| 383 | 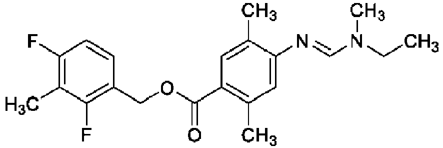 |
| 384 | 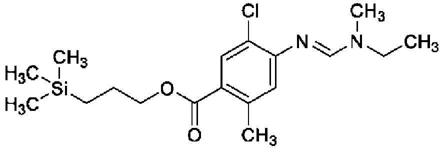 |
| 385 | 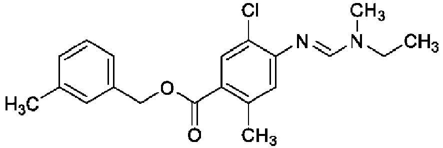 |
| 386 | 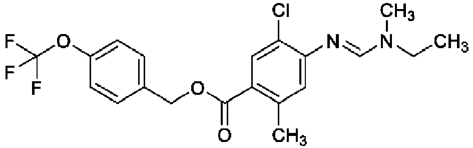 |
| 387 | 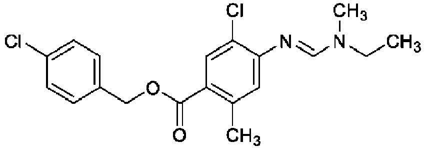 |
| 388 | 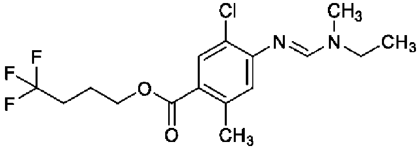 |
| 389 | 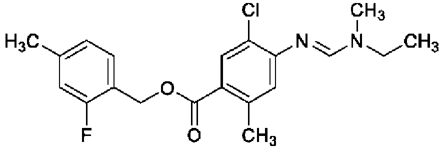 |
| 390 | 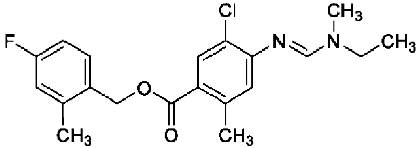 |
| 391 | 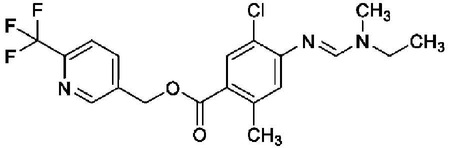 |
| 392 | 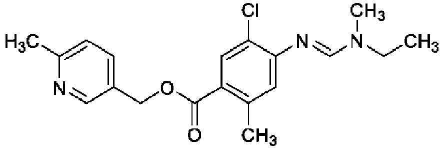 |
| 393 | 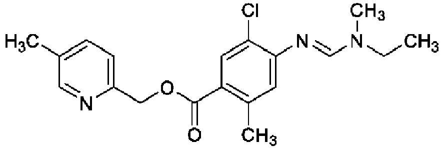 |
| 394 | 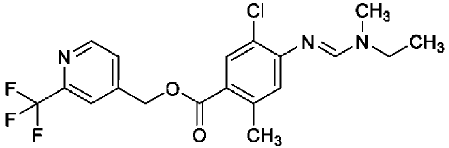 |
| 395 | 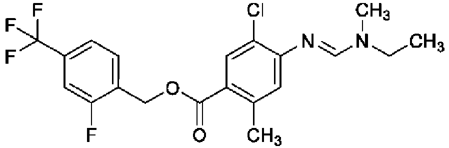 |
| 396 | 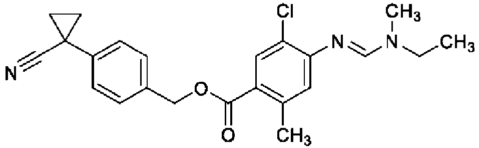 |
| 397 | 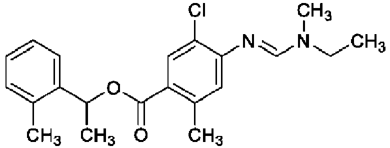 |
| 398 | 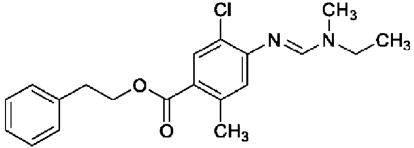 |
| 399 | 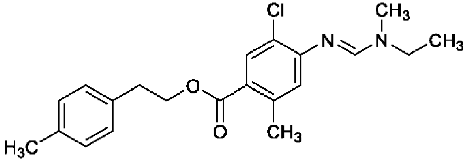 |
| 400 | 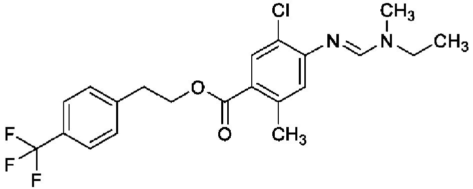 |
| 401 | 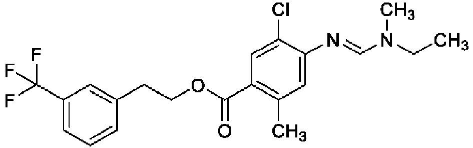 |
| 402 | 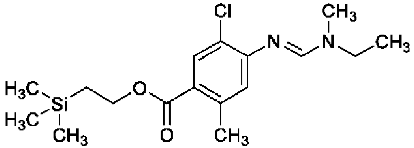 |
| 403 | 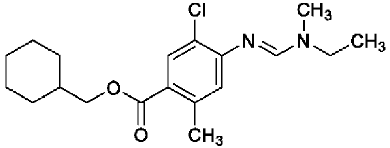 |
| 404 | 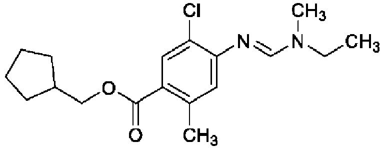 |
| 405 | 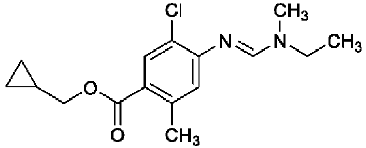 |
| 406 | 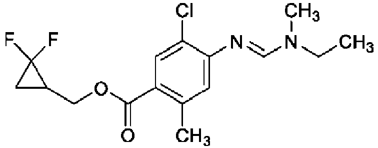 |
| 407 | 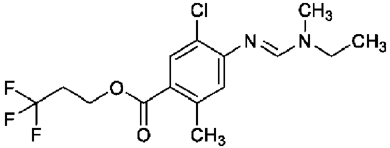 |
| 408 | 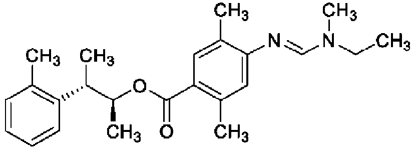 |
| 409 | 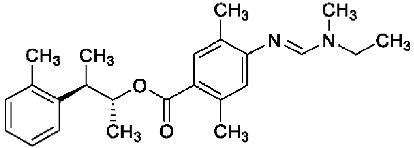 |
| 410 | 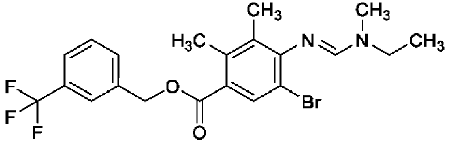 |
| 411 | 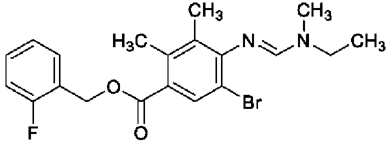 |
| 412 | 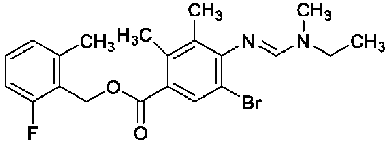 |
| 413 | 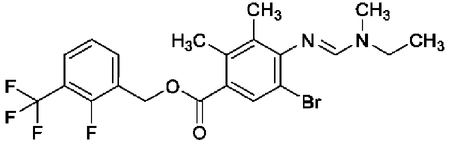 |
| 414 | 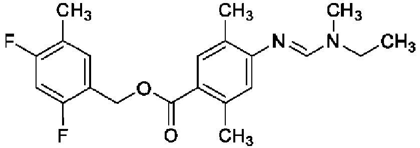 |
| 415 | 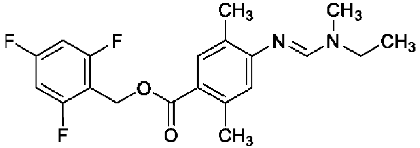 |
| 416 | 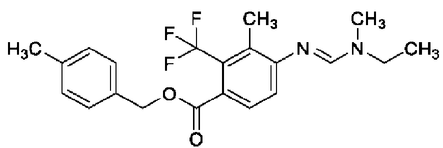 |
| 417 | 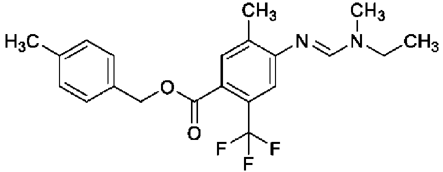 |
| 418 | 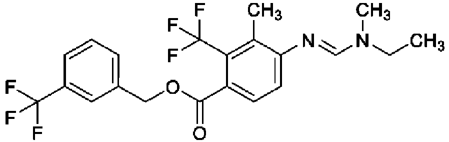 |
| 419 | 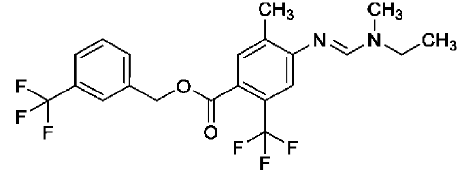 |
| 420 | 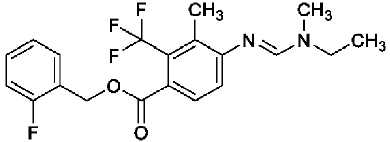 |
| 421 | 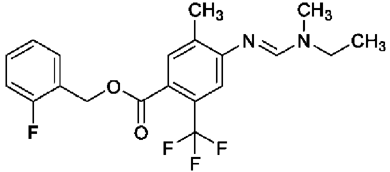 |
| 422 | 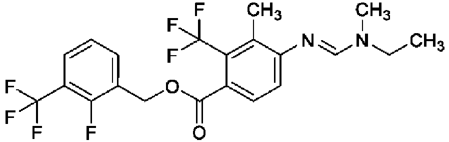 |
| 423 | 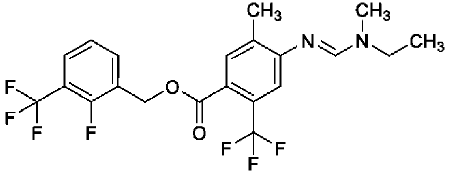 |
| 424 | 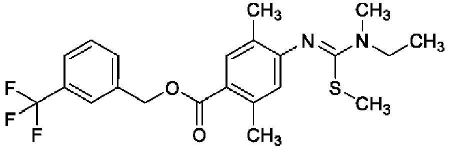 |
| 425 | 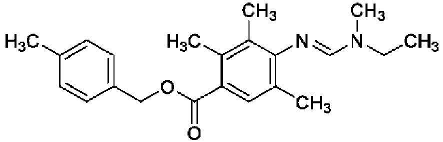 |
| 426 | 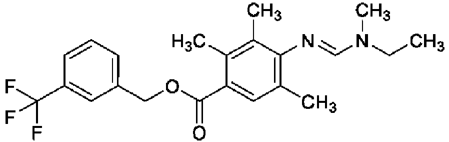 |
| 427 | 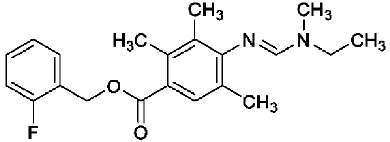 |
| 428 | 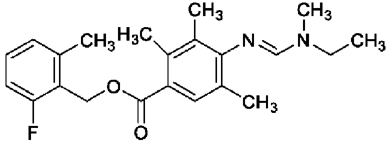 |
| 429 | 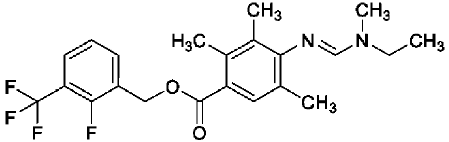 |
| 430 | 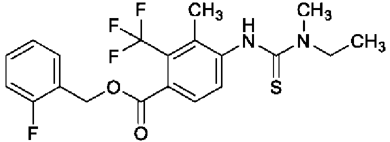 |
| 431 | 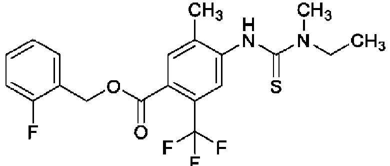 |
| 432 | 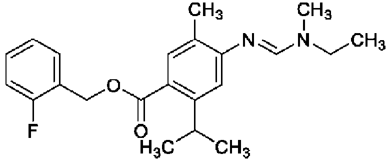 |
| 433 | 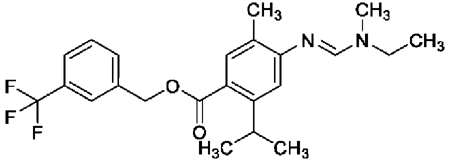 |
| 434 | 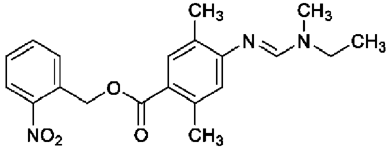 |
| 435 | 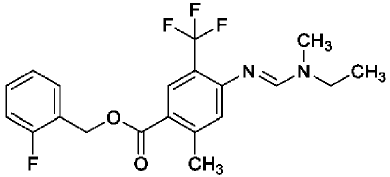 |
| 436 | 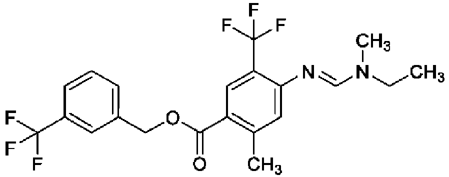 |
| 437 | 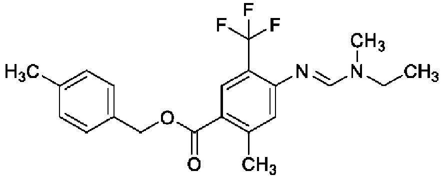 |
| 438 | 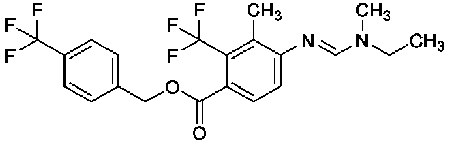 |
| 439 | 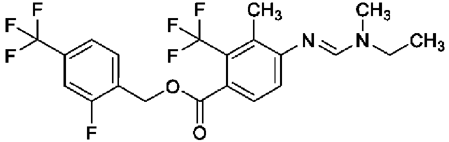 |
| 440 | 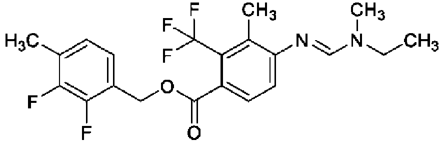 |
| 441 | 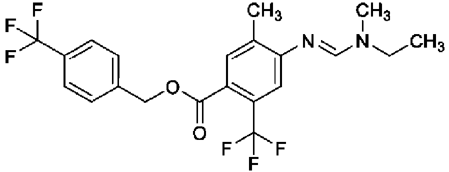 |
| 442 | 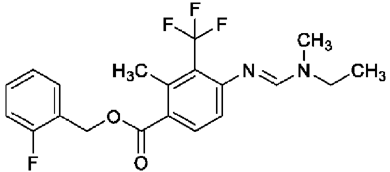 |
| 443 | 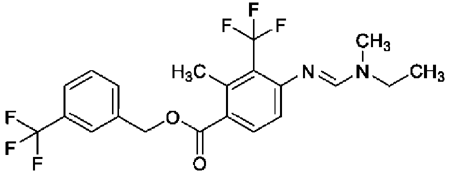 |
| 444 | 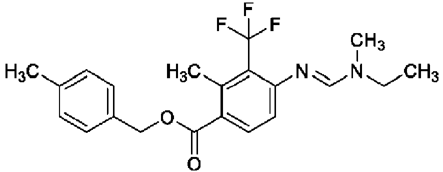 |
| 445 | 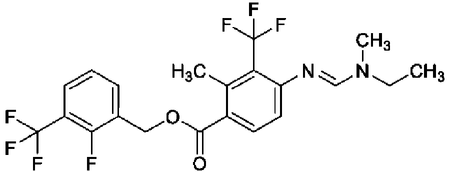 |
| 446 | 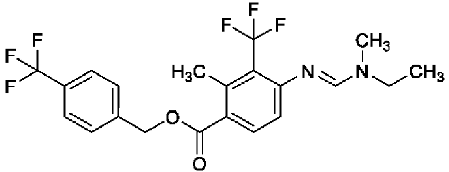 |
| 447 | 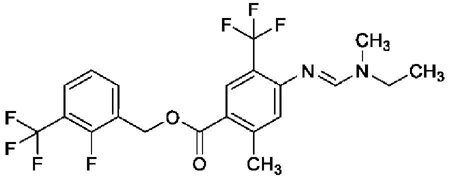 |
| 448 | 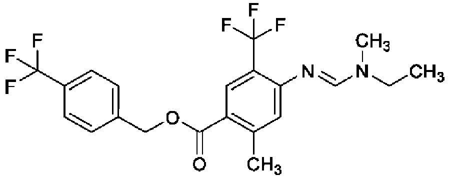 |
| 449 | 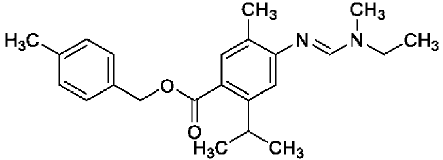 |
| 450 | 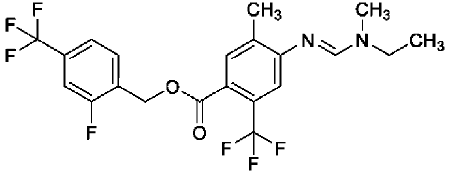 |
| 451 | 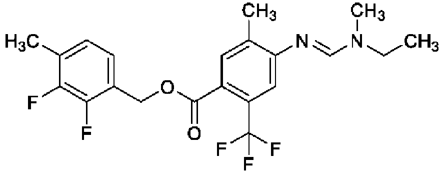 |
| 452 | 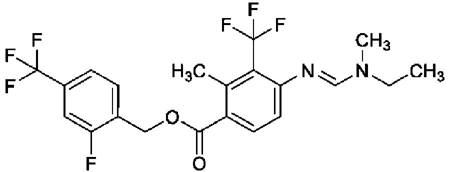 |
| 453 | 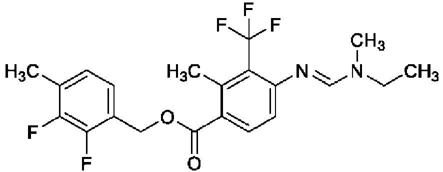 |
| 454 | 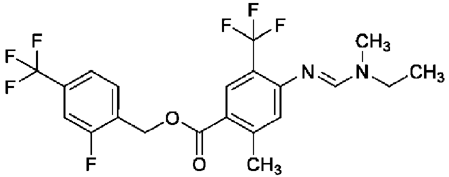 |
| 455 | 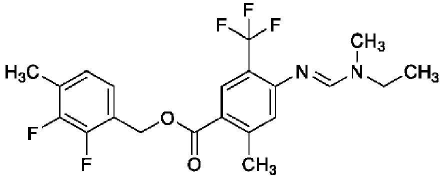 |
| 456 | 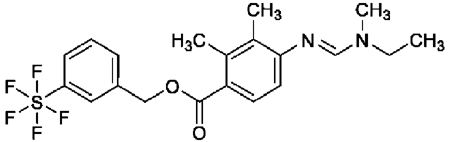 |
| 457 | 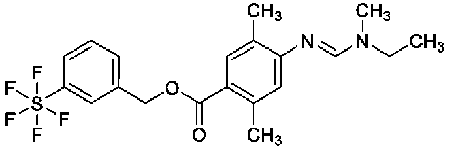 |
| 458 | 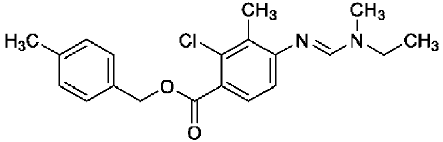 |
| 459 | 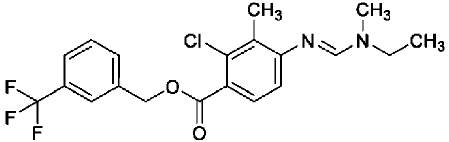 |
| 460 | 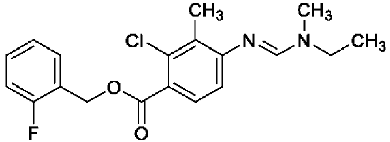 |
| 461 | 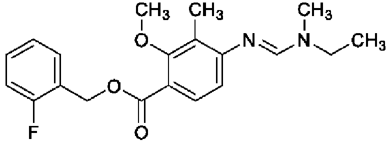 |
| 462 | 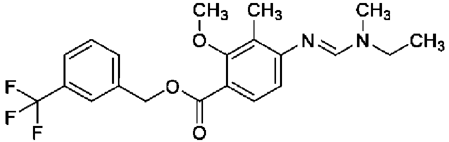 |
| 463 | 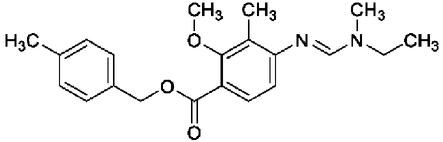 |
| 464 | 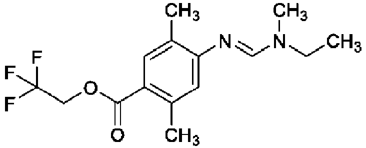 |
| 465 | 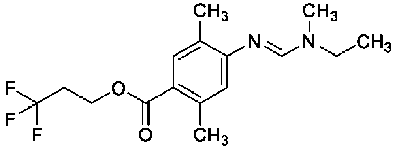 |
| 466 | 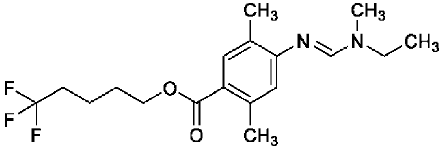 |
| 467 | 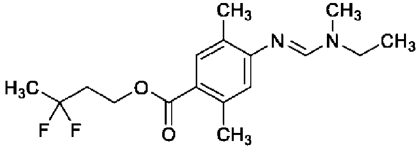 |
| 468 | 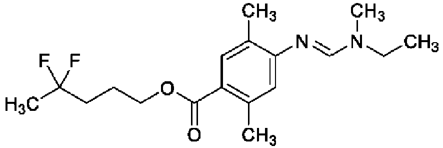 |
| 469 | 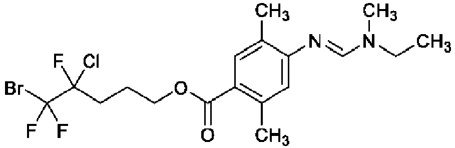 |
| 470 | 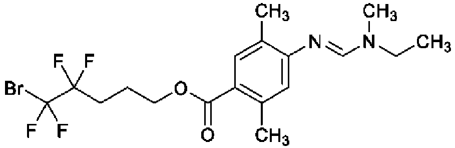 |
| 471 | 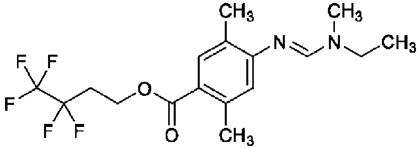 |
| 472 | 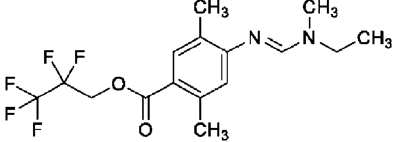 |
| 473 | 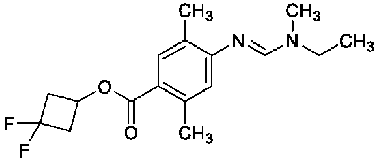 |
| 474 | 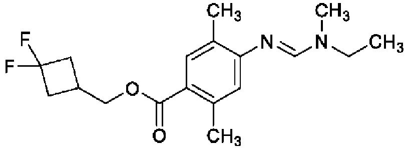 |
| 475 | 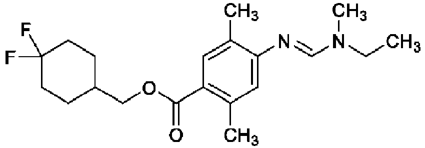 |
| 476 | 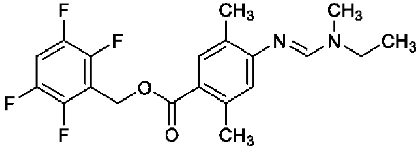 |
| 477 | 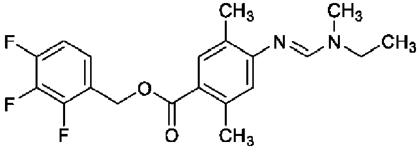 |
| 478 | 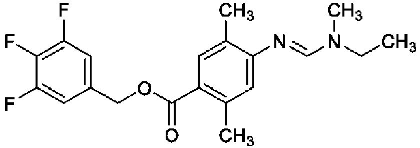 |
| 479 | 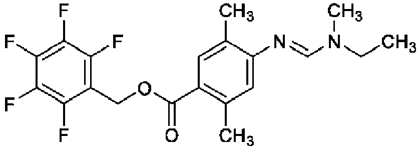 |
| 480 | 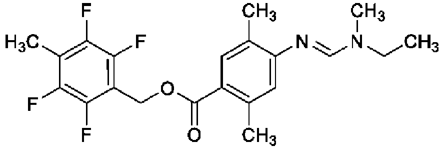 |
| 481 | 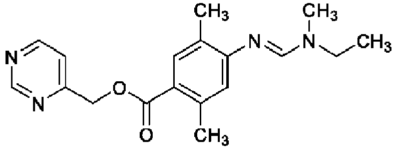 |
| 482 | 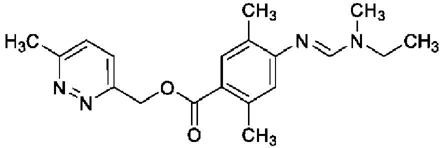 |
| 493 | 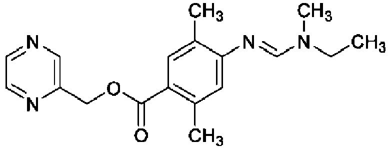 |
| 484 | 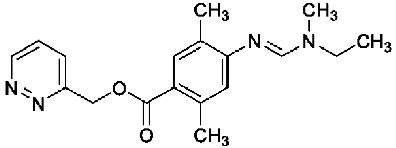 |
| 485 | 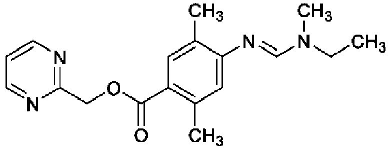 |
| 486 | 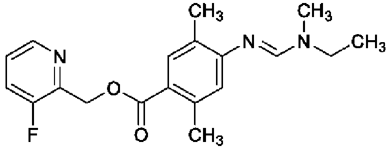 |
| 487 | 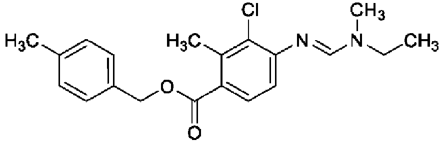 |
| 488 | 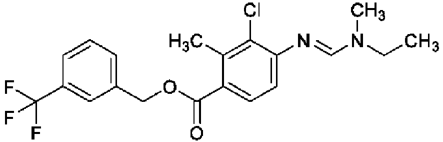 |
| 489 | 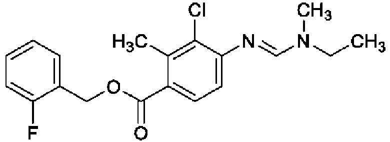 |
| 490 | 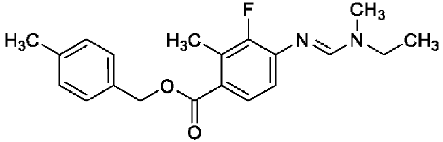 |
| 491 | 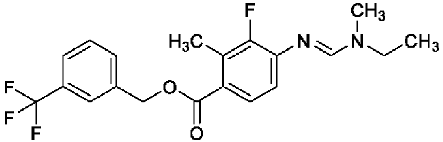 |
| 492 | 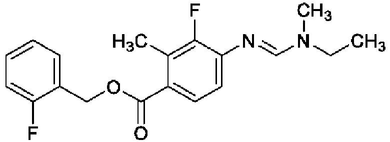 |
| 493 | 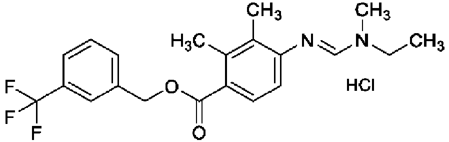 |
| 494 | 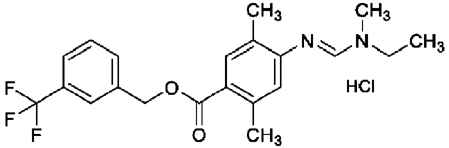 |
| 495 | 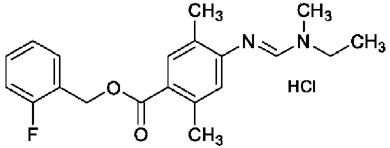 |
| 496 | 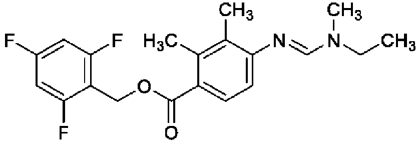 |
| 497 | 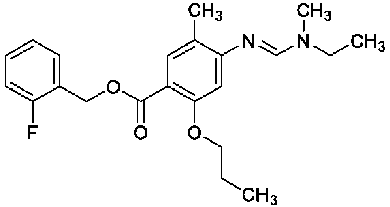 |
| 498 | 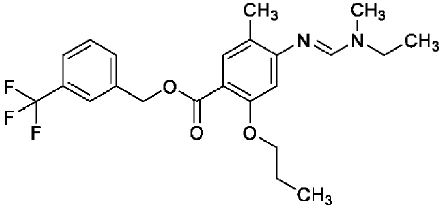 |
| 499 | 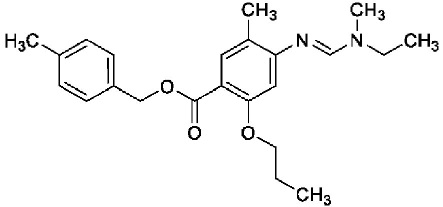 |
| 500 | 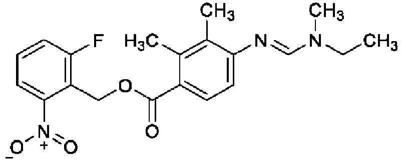 |
| 501 | 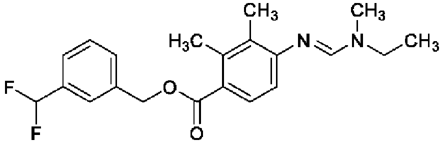 |
| 502 | 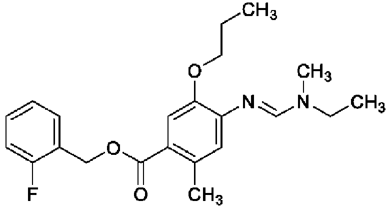 |
| 503 | 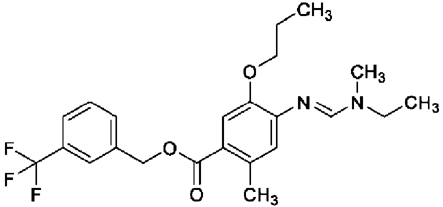 |
| 504 | 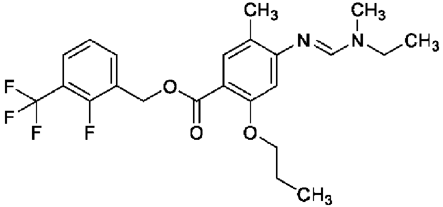 |
| 505 | 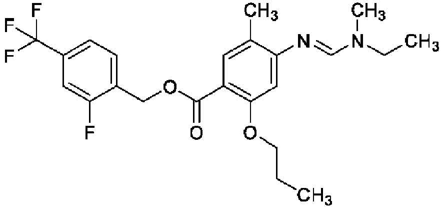 |
| 506 | 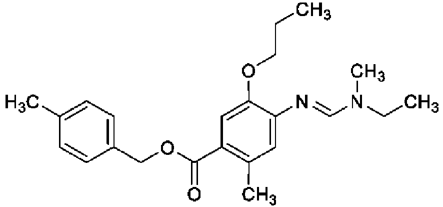 |
| 507 | 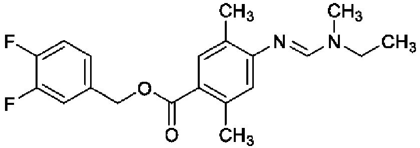 |
| 508 | 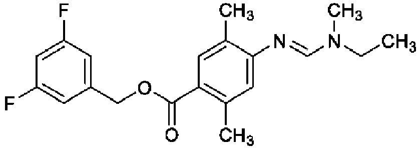 |
| 509 | 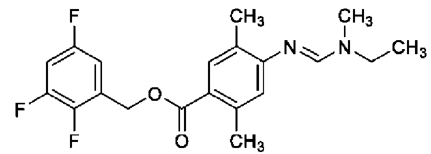 |
| 510 | 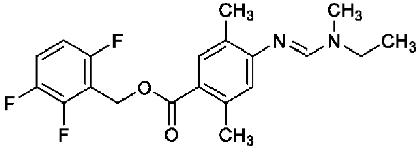 |
| 511 | 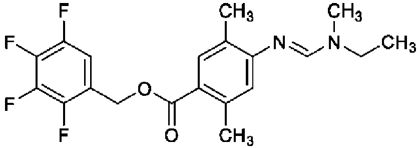 |
| 512 | 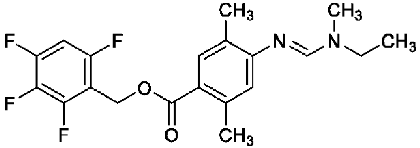 |
| 513 | 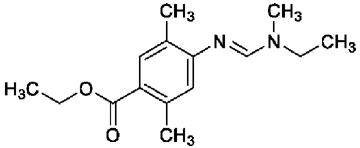 |
| 514 | 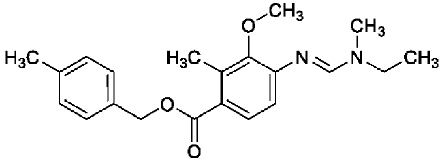 |
| 515 | 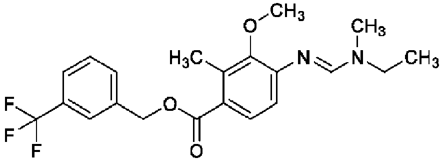 |
| 516 | 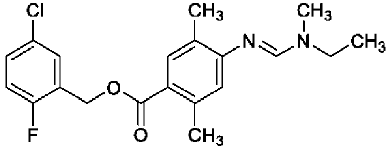 |
| 517 | 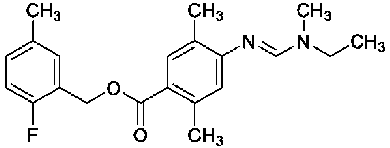 |
| 518 | 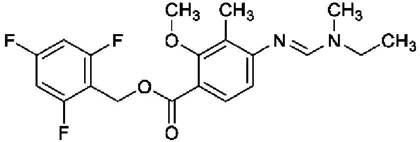 |
| 519 | 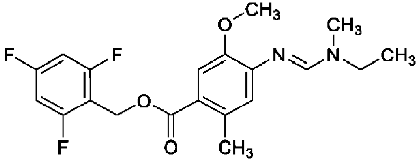 |
| 520 | 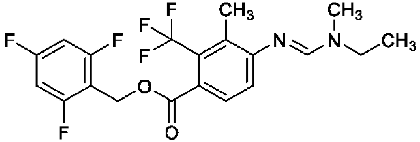 |
| 521 | 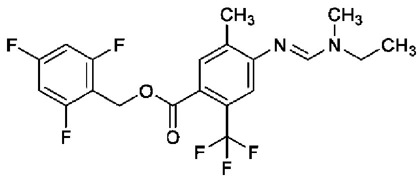 |
| 522 | 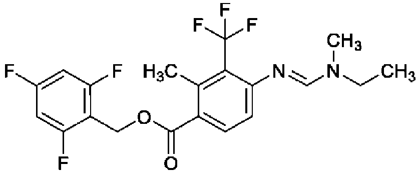 |
| 523 | 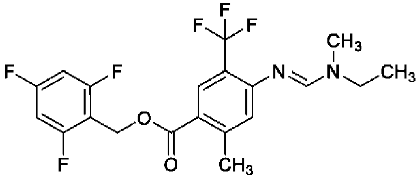 |
| 524 | 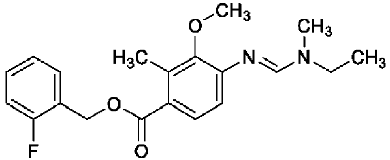 |
| 525 | 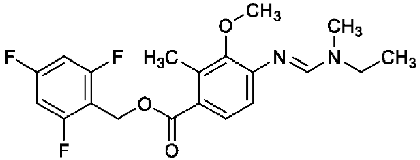 |
| 526 | 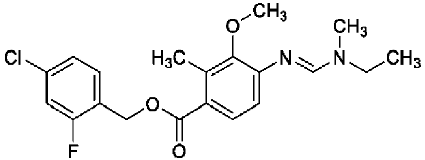 |
| 527 | 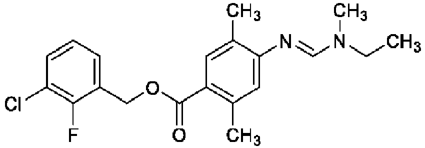 |
| 528 | 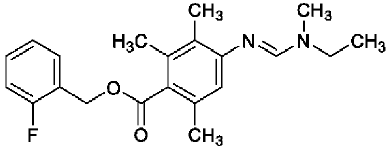 |
| | |
| 530 | 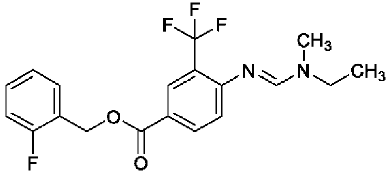 |
| 531 | 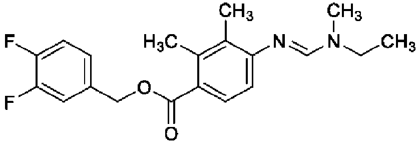 |
| 532 | 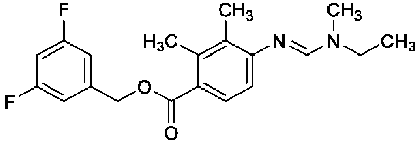 |
| 533 | 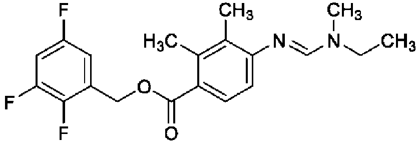 |
| 534 | 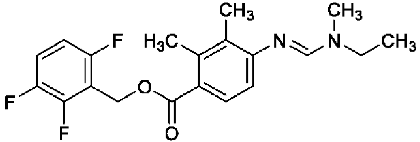 |
| 535 | 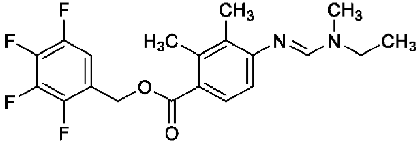 |
| 536 | 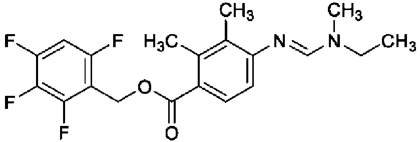 |
| 537 | 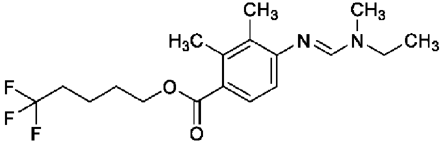 |
| 538 | 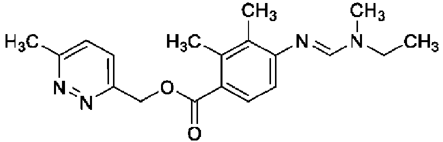 |
| 539 | 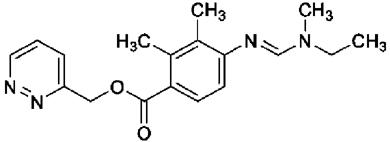 |
| 540 | 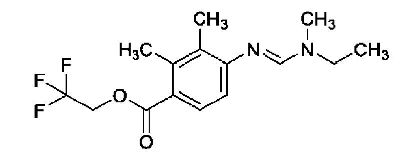 |
| 541 | 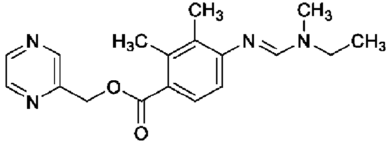 |
| 542 | 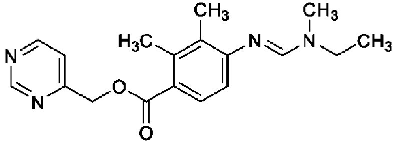 |
| 543 | 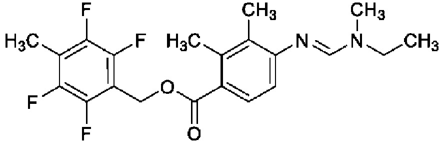 |
| 544 | 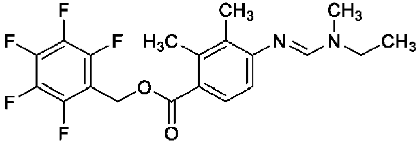 |
| 545 | 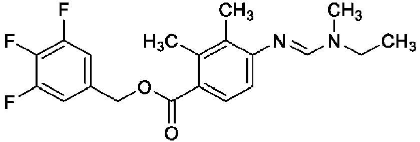 |
| 546 | 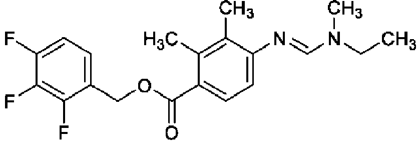 |
| 547 | 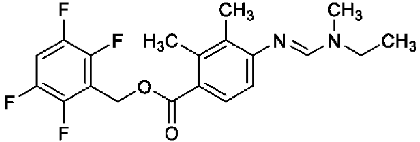 |
| 548 | 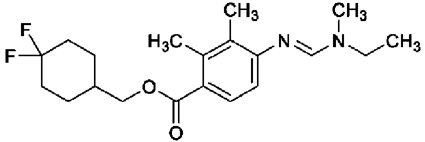 |
| 549 | 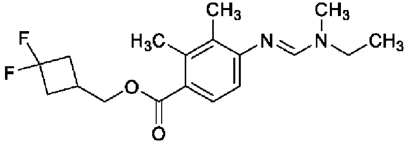 |
| 550 | 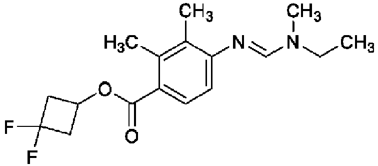 |
| 551 | 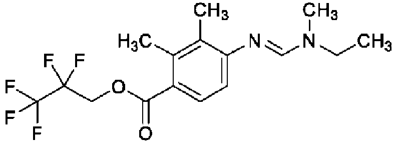 |
| 552 | 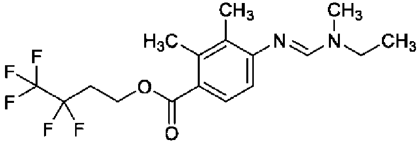 |
| 553 | 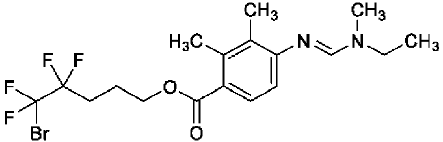 |
| 554 | 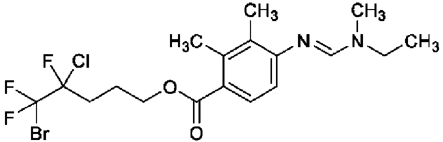 |
| 555 | 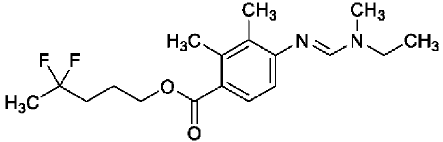 |
| 556 | 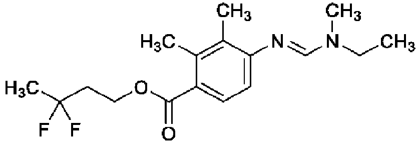 |
| 557 | 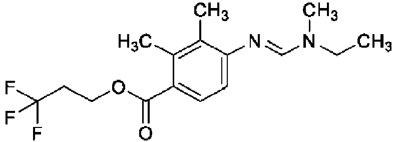 |
| 558 | 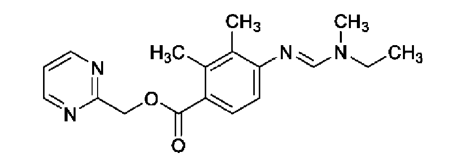 |
| 559 | 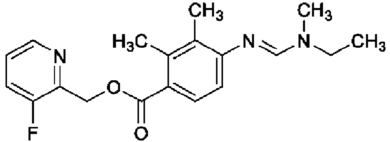 |
| 560 | 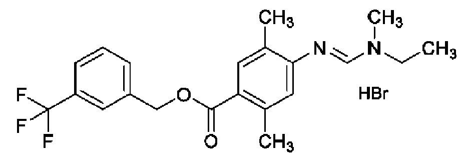 |
| 561 | 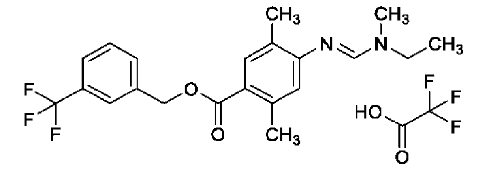 |
| 562 | 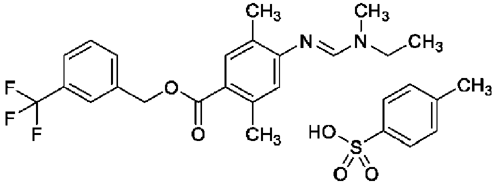 |
| 563 | 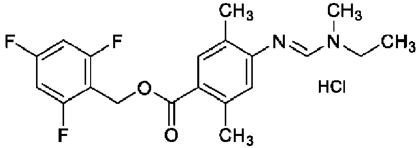 |

9. Fungizidzusammensetzung umfassend eine phytologisch annehmbare Menge einer Verbindung der Formel I nach einem der Ansprüche 1-8 und einen Träger, wobei der Träger vorzugsweise eines oder mehrere von einem Verdickungsmittel, Emulgator, Rheologiemittel, Dispergiermittel und Polymer ist.

10. Verfahren zur Bekämpfung von Pilzbefall auf einer Pflanze, wobei das Verfahren Inkontaktbringen eines Bereichs benachbart zu der Pflanze, eines Bodens, der zum Unterstützen des Wachstums der Pflanze angepasst ist, einer Wurzel der Pflanze und eines Blattwerks der Pflanze mit einer phytologisch annehmbaren Menge einer Verbindung der Formel I nach einem der Ansprüche 1-8 oder einer Zusammensetzung nach Anspruch 9 umfasst.

11. Verbindung der Formel I nach einem der Ansprüche 1-8 zur Verwendung bei der Bekämpfung eines Pilzpathogens.

12. Verbindung zur Verwendung nach Anspruch 11, wobei das Pilzpathogen eines von Zymoseptoria tritici, Cochliobolus sativus, Puccinia triticina, Puccinia striiformis, Venturia inaequalis, Ustilago maydis, Uncinula necator, Rhynchosporium commune, Magnaporthe grisea, Phakopsora pachyrhizi, Parastagonospora nodorum, Glomerella lagenarium, Cercospora beticola, Alternaria solani, Pyrenophora teres, Blumeria graminis f. sp. Tritici, Blumeria graminis f. sp. Hordei, Erysiphe cichoracearum, Fusarium virguliforme, Rhizoctonia solani, Pythium ultimum und Botrytis cinerea ist; oder wobei die Verbindung eine der folgenden Krankheiten durch das Pilzpathogen behandelt: Septoria-Blattdürre von Weizen (Zymoseptoria tritici), Fleckenkrankheit von Gerste (Cochliobolus sativus), Weizen-Braunrost (Puccinia triticina), Gelbrost (Puccinia striiformis), Apfelschorf (Venturia inaequalis), Blasenbrand von Mais (Ustilago maydis), Mehltau von Weinreben (Uncinula necator), Blattdürre von Gerste (Rhynchosporium commune), Reisbrand (Magnaporthe grisea), Asiatischer Sojarost (Phakopsora pachyrhizi), Spelzenbräune von Weizen (Parastagonospora nodorum), Anthracnose von Kürbisgewächsen (Glomerella lagenarium), Blattflecken von Rüben (Cercospora beticola), Dürrflecken von Tomaten (Alternaria solani), Netzflecken von Gerste (Pyrenophora teres), Mehltau von Weizen (Blumeria graminis f. sp. tritici), Mehltau von Gerste (Blumeria graminis f. sp. hordei), Mehltau von Kürbisgewächsen (Erysiphe cichoracearum), Plötzlicher-Tod-Syndrom von Sojabohnen (Fusarium virguliforme), Wurzelhalsfäule oder Keimlingskrankheit von Sämlingen (Rhizoctonia solani), Wurzelfäule (Pythium ultimum), Grauschimmel (Botrytis cinerea).

13. Zusammensetzung zur Verwendung bei der Bekämpfung eines Pilzpathogens, wobei die Zusammensetzung eine phytologisch annehmbare Menge einer Verbindung der Formel I nach einem der Ansprüche 1-8, 11 oder 12 und einen Träger umfasst.

14. Zusammensetzung zur Verwendung nach Anspruch 13, wobei das Pilzpathogen eines von Zymoseptoria tritici, Cochliobolus sativus, Puccinia triticina, Puccinia striiformis, Venturia inaequalis, Ustilago maydis, Uncinula necator, Rhynchosporium commune, Magnaporthe grisea, Phakopsora pachyrhizi, Parastagonospora nodorum, Glomerella lagenarium, Cercospora beticola, Alternaria solani, Pyrenophora teres, Blumeria graminis f. sp. Tritici, Blumeria graminis f. sp. Hordei, Erysiphe cichoracearum, Fusarium virguliforme, Rhizoctonia solani, Pythium ultimum und Botrytis cinerea ist; oder
wobei die Zusammensetzung eine der folgenden Krankheiten durch das Pilzpathogen behandelt: Septoria-Blattdürre von Weizen (Zymoseptoria tritici), Fleckenkrankheit von Gerste (Cochliobolus sativus), Weizen-Braunrost (Puccinia triticina), Gelbrost (Puccinia striiformis), Apfelschorf (Venturia inaequalis), Blasenbrand von Mais (Ustilago maydis), Mehltau von Weinreben (Uncinula necator), Blattdürre von Gerste (Rhynchosporium commune), Reisbrand (Magnaporthe grisea), Asiatischer Sojarost (Phakopsora pachyrhizi), Spelzenbräune von Weizen (Parastagonospora nodorum), Anthracnose von Kürbisgewächsen (Glomerella lagenarium), Blattflecken von Rüben (Cercospora beticola), Dürrflecken von Tomaten (Alternaria solani), Netzflecken von Gerste (Pyrenophora teres), Mehltau von Weizen (Blumeria graminis f. sp. tritici), Mehltau von Gerste (Blumeria graminis f. sp. hordei), Mehltau von Kürbisgewächsen (Erysiphe cichoracearum), Plötzlicher-Tod-Syndrom von Sojabohnen (Fusarium virguliforme), Wurzelhalsfäule oder Keimlingskrankheit von Sämlingen (Rhizoctonia solani), Wurzelfäule (Pythium ultimum), Grauschimmel (Botrytis cinerea); oder
wobei die Krankheit eine von Septoria-Blattdürre von Weizen, Fleckenkrankheit von Gerste, Weizen-Braunrost und Asiatischer Sojarost ist.

## Revendications

1. Composé de formule I : 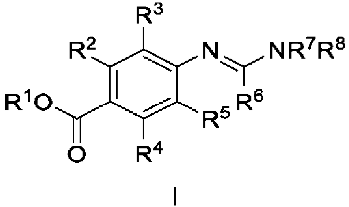
R¹ étant choisi dans le groupe constitué par C₁-C₈ alkyle, C₁-C₈ alkyle substitué, C₂-C₈ alcényle, C₂-C₈ alcényle substitué, C₂-C₈ alcynyle, C₂-C₈ alcynyle substitué, C₃-C₈ cycloalkyle, C₃-C₈ cycloalkyle substitué, C₃-C₈ hétérocycloalkyle, C₃-C₈ hétérocycloalkyle substitué, C₅-C₇ hétéroaryle, C₅-C₇ hétéroaryle substitué, phényle, phényle substitué, benzyle, et benzyle substitué ; chaque R², R⁴, et R⁵ étant indépendamment choisi dans le groupe constitué par hydrogène, halogène, cyano, nitro, C₁-C₈ alkyle, C₁-C₈ alkyle substitué, C₂-C₈ alcényle, C₂-C₈ alcényle substitué, C₂-C₈ alcynyle, C₂-C₈ alcynyle substitué, C₁-C₈ alcoxy, et C₁-C₈ alcoxy substitué ;
R³ étant choisi dans le groupe constitué par halogène, cyano, C₁-C₈ alkyle, C₁-C₈ alkyle substitué, C₂-C₈ alcényle, C₂-C₈ alcényle substitué, C₂-C₈ alcynyle, C₂-C₈ alcynyle substitué, C₁-C₈ alcoxy, et C₁-C₈ alcoxy substitué ;
R⁶ étant choisi dans le groupe constitué par hydrogène, C₁-C₈ alkyle, C₁-C₈ alkyle substitué, C₂-C₈ alcényle, C₂₋-C₈ alcényle substitué, C₂-C₈ alcynyle, C₂-C₈ alcynyle substitué, C₁-C₈ alcoxy, C₁-C₈ alcoxy substitué, thiol, alkylthio, et alkylthio substitué ;
ou R⁶ et R⁷ pouvant être liés ensemble de manière covalente pour former un groupe saturé ou insaturé C₃-C₈ hétérocycloalkyle ou C₃-C₈ hétérocycloalkyle substitué ; chaque R⁷ et R⁸ étant indépendamment choisi dans le groupe constitué par C₁-C₈ alkyle, C₁-C₈ alkyle substitué, C₂-C₈ alcényle, C₂-C₈ alcényle substitué, C₂-C₈ alcynyle, C₂-C₈ alcynyle substitué, C₃-C₈ cycloalkyle, C₃-C₈ cycloalkyle substitué, benzyle, et benzyle substitué ;
ou R⁷ et R⁸ pouvant être liés ensemble de manière covalente pour former un groupe saturé ou insaturé C₃-C₈ hétérocycloalkyle ou C₃-C₈ hétérocycloalkyle substitué ; l'un quelconque et la totalité des cycles hétérocycliques pouvant contenir jusqu'à trois hétéroatomes choisis dans le groupe constitué par O, N, et S ;
ou forme tautomère ou sel correspondant,
à la condition que le composé ne soit pas le chlorhydrate de 4-(((diméthylamino)méthylène)amino)-3,5-diméthylbenzoate d'éthyle, ou le 3-bromo-4-[(E)-diméthylaminométhylidèneamino]benzoate de méthyle.

2. Composé selon la revendication 1, R¹ étant choisi dans le groupe constitué par C₁-C₈ alkyle, C₁-C₈ alkyle substitué, C₂-C₈ alcynyle, C₂-C₈ alcynyle substitué, C₃-C₈ cycloalkyle, C₃-C₈ cycloalkyle substitué, phényle, phényle substitué, benzyle, et benzyle substitué, de préférence R¹ étant choisi dans le groupe constitué par benzyle et benzyle substitué.

3. Composé selon les revendications 1 ou 2, R² et R⁵ étant tous deux hydrogène.

4. Composé selon la revendication 3, R³ et R⁴ étant indépendamment choisis dans le groupe constitué par halogène, C₁-C₈ alkyle, C₁-C₈ alkyle substitué, et C₁-C₈ alcoxy, de préférence R³ et R⁴ étant tous deux CH₃.

5. Composé selon les revendications 1 ou 2, R⁴ et R⁵ étant tous deux hydrogène.

6. Composé selon la revendication 5, R² et R³ étant indépendamment choisis dans le groupe constitué par halogène, C₁-C₈ alkyle, C₁-C₈ alkyle substitué, et C₁-C₈ alcoxy, de préférence R² et R³ étant tous deux CH₃.

7. Composé selon l'une quelconque des revendications 1-6, chaque R⁷ et R⁸ étant indépendamment choisi dans le groupe constitué par C₁-C₈ alkyle, C₁-C₈ alkyle substitué, C₂-C₈ alcényle, C₃-C₈ cycloalkyle, benzyle, et benzyle substitué ; et/ou
R⁶ étant choisi dans le groupe constitué par hydrogène, C₁-C₈ alkyle, C₁-C₈ alkyle substitué, thiol, alkylthio, et alkylthio substitué.

8. Composé selon l'une quelconque des revendications précédentes qui est choisi dans le tableau suivant :
| Comp. n° | Structure |
|---|---|
| 1 | 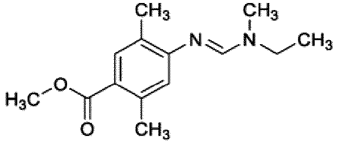 |
| 3 | 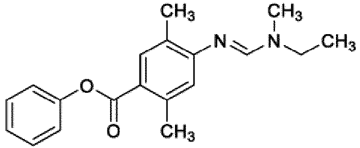 |
| 4 | 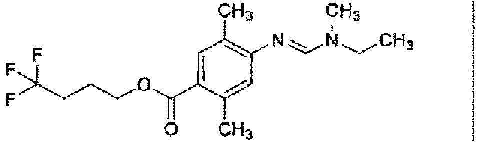 |
| 5 | 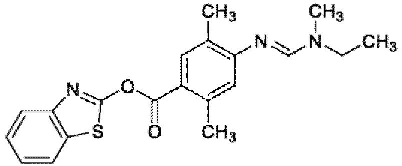 |
| 6 | 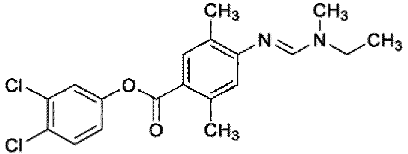 |
| 7 | 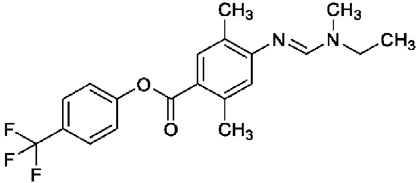 |
| 8 | 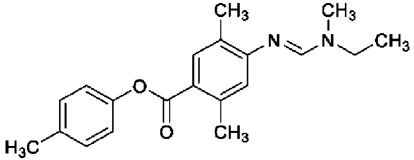 |
| 9 | 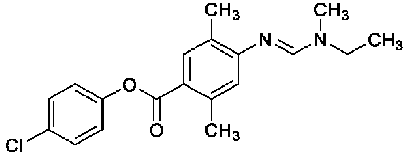 |
| 10 | 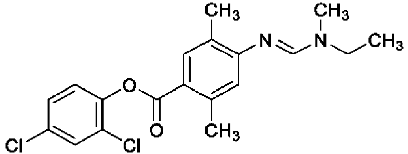 |
| 11 | 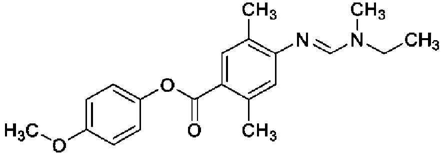 |
| 12 | 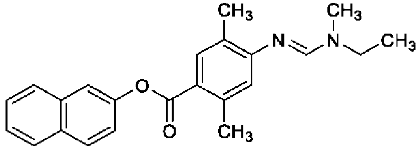 |
| 13 | 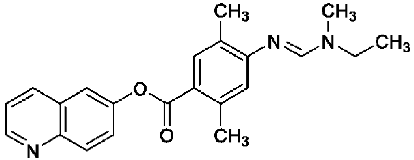 |
| 14 | 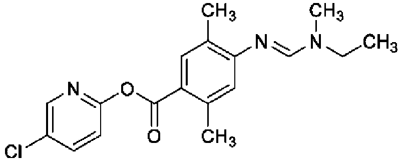 |
| 15 | 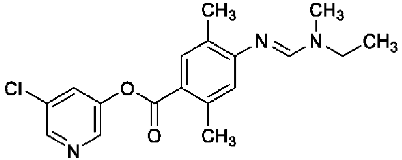 |
| 16 | 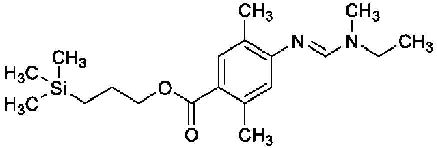 |
| 17 | 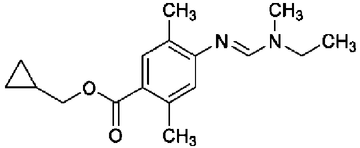 |
| 18 | 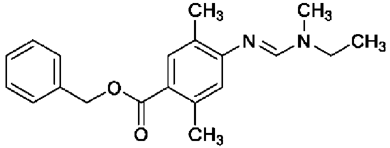 |
| 19 | 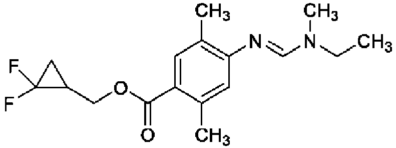 |
| 20 | 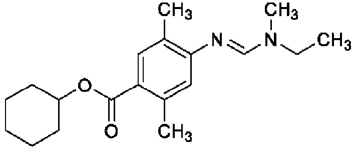 |
| 21 | 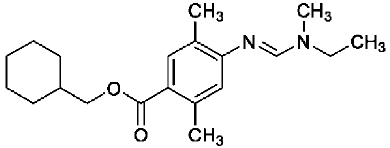 |
| 22 | 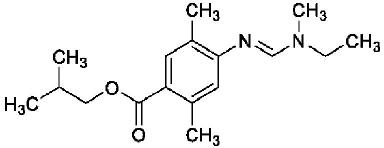 |
| 23 | 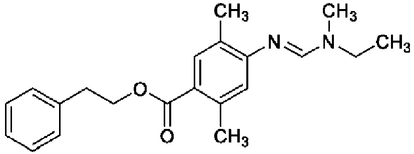 |
| 24 | 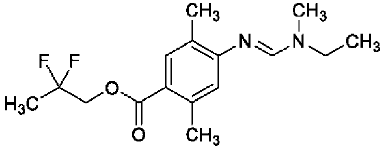 |
| 25 | 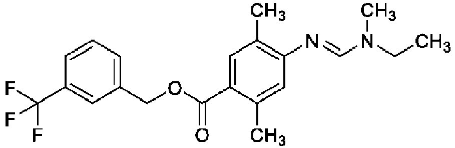 |
| 26 | 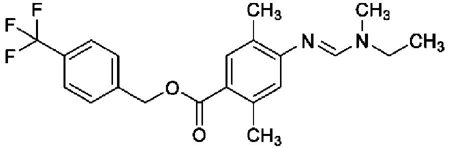 |
| 27 | 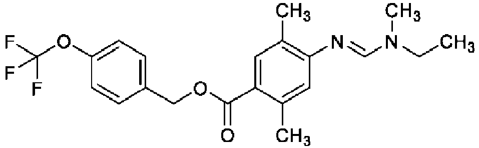 |
| 28 | 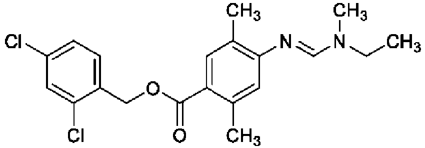 |
| 29 | 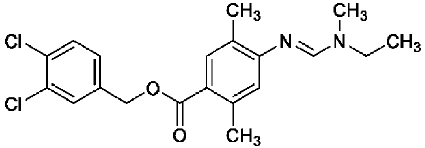 |
| 30 | 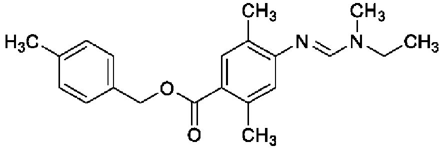 |
| 31 | 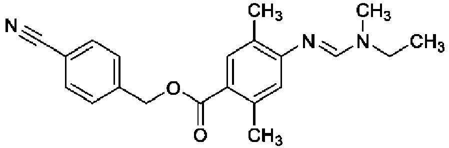 |
| 32 | 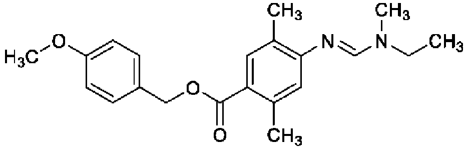 |
| 33 | 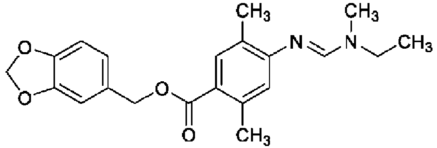 |
| 34 | 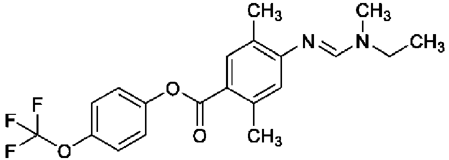 |
| 35 | 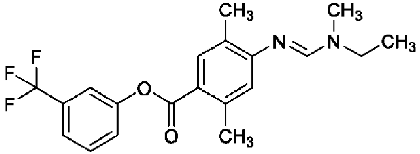 |
| 36 | 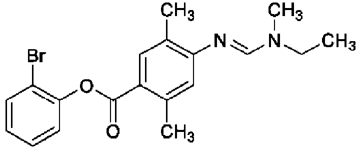 |
| 37 | 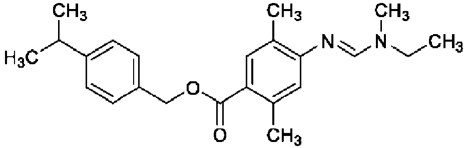 |
| 38 | 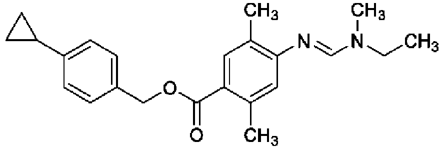 |
| 39 | 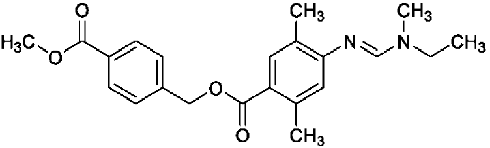 |
| 40 | 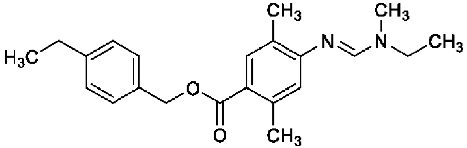 |
| 41 | 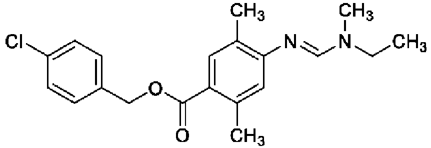 |
| 42 | 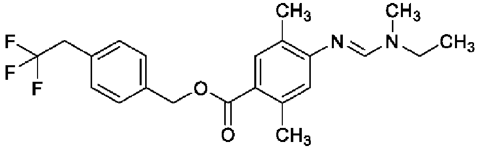 |
| 43 | 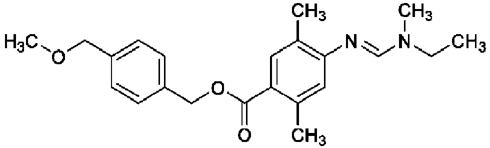 |
| 44 | 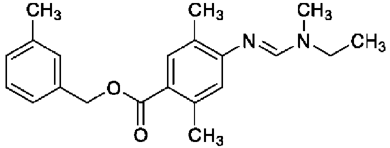 |
| 45 | 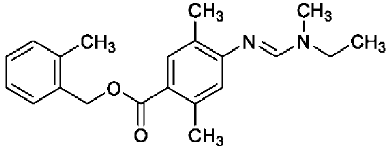 |
| 46 | 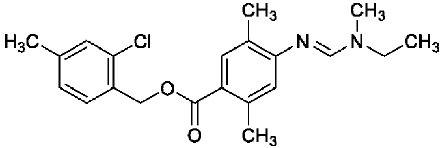 |
| 47 | 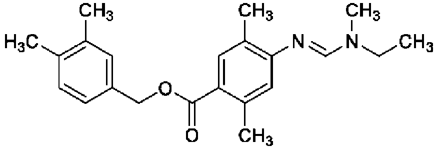 |
| 48 | 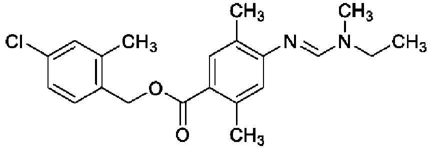 |
| 49 | 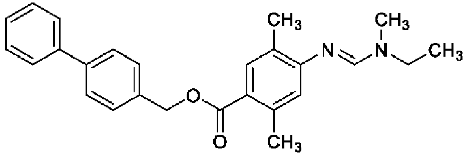 |
| 50 | 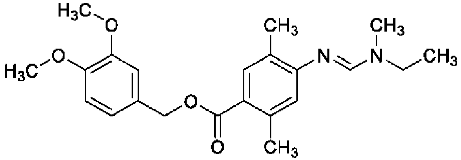 |
| 51 | 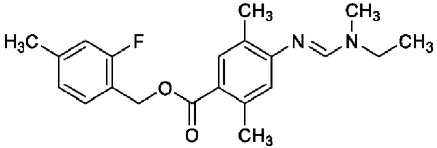 |
| 52 | 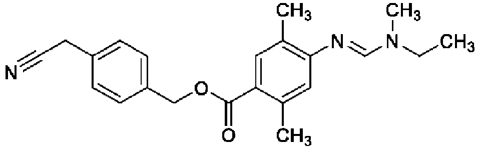 |
| 53 | 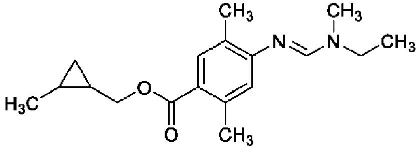 |
| 54 | 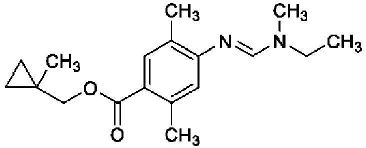 |
| 55 | 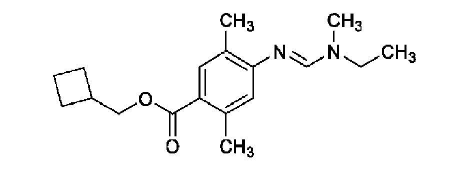 |
| 56 | 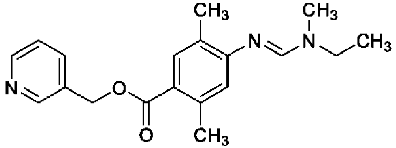 |
| 57 | 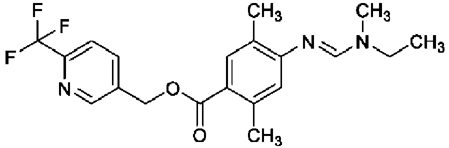 |
| 58 | 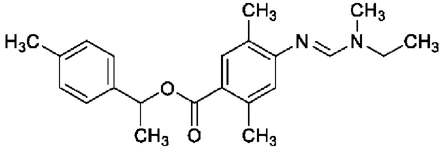 |
| 59 | 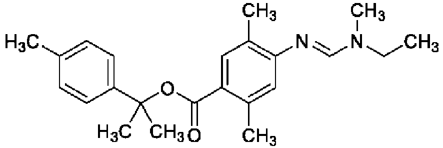 |
| 60 | 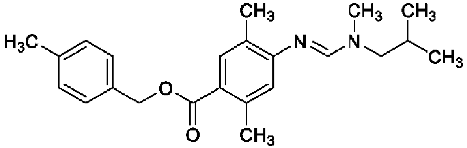 |
| 61 | 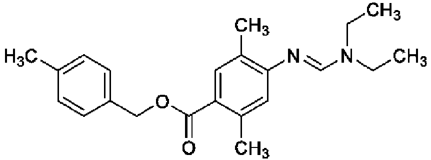 |
| 62 | 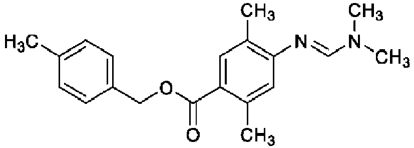 |
| 63 | 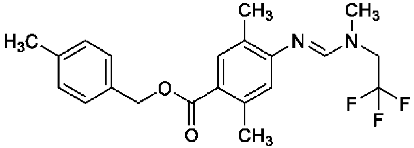 |
| 64 | 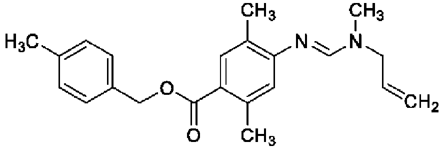 |
| 65 | 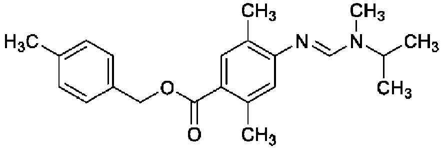 |
| 66 | 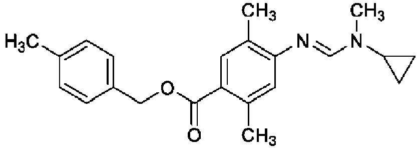 |
| 67 | 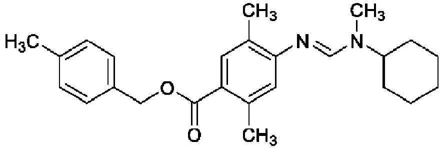 |
| 68 | 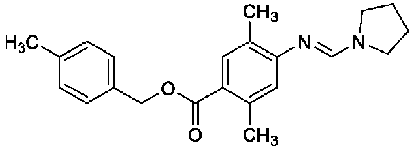 |
| 69 | 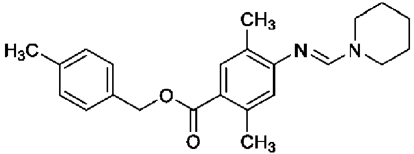 |
| 70 | 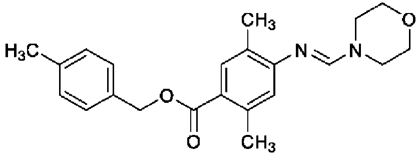 |
| 71 | 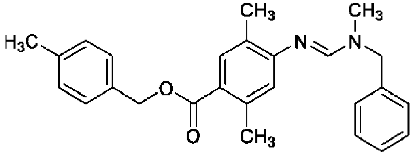 |
| 72 | 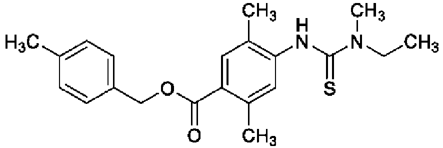 |
| 74 | 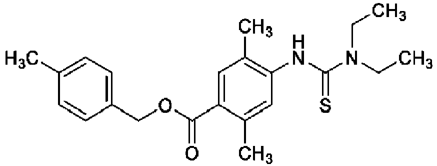 |
| 75 | 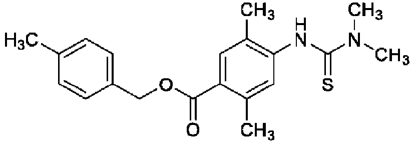 |
| 76 | 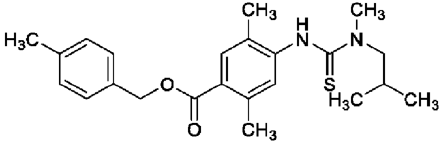 |
| 77 | 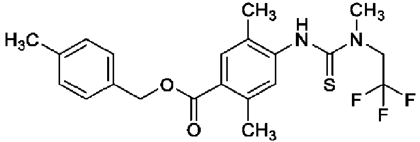 |
| 78 | 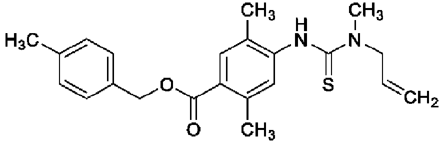 |
| 79 | 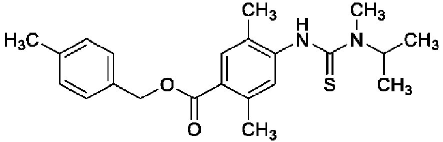 |
| 80 | 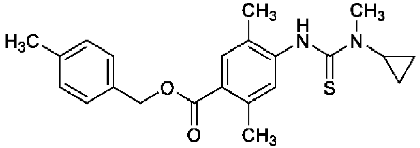 |
| 81 | 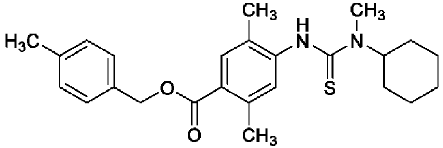 |
| 82 | 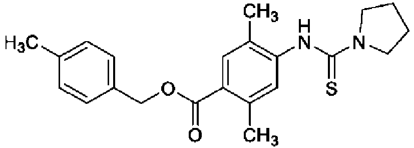 |
| 83 | 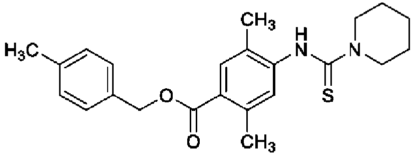 |
| 84 | 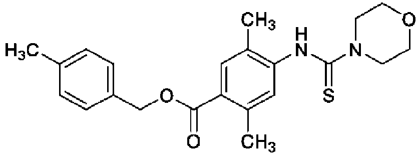 |
| 86 | 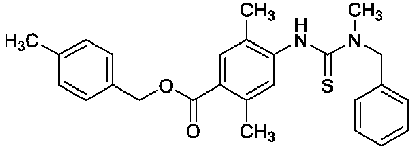 |
| | |
| | |
| 89 | 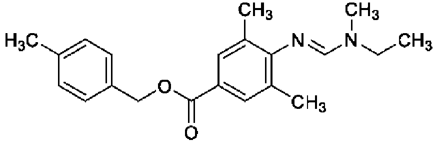 |
| 90 | 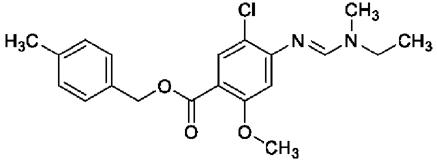 |
| 91 | 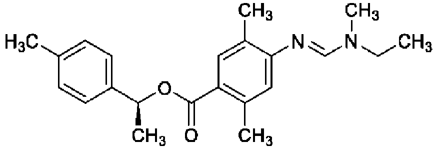 |
| 92 | 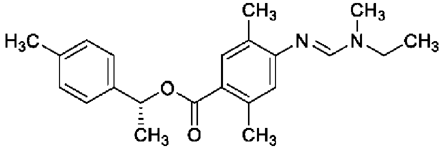 |
| | |
| 94 | 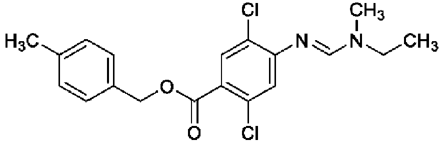 |
| 95 | 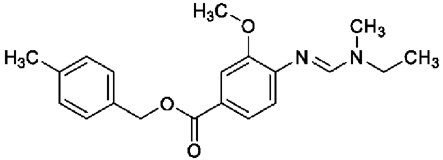 |
| 96 | 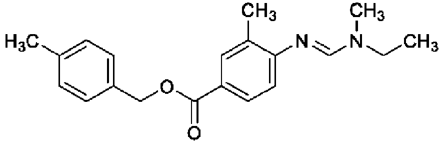 |
| 97 | 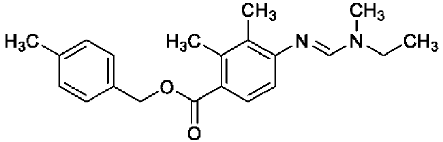 |
| 98 | 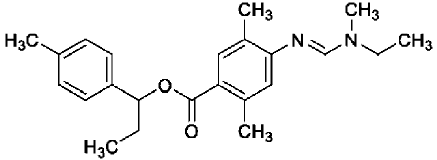 |
| 99 | 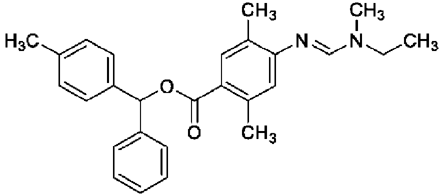 |
| 100 | 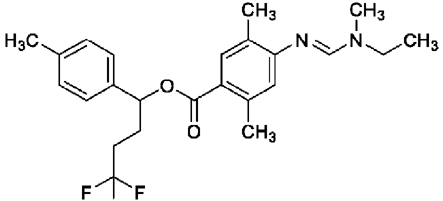 |
| 101 | 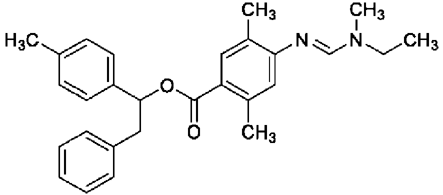 |
| | |
| 103 | 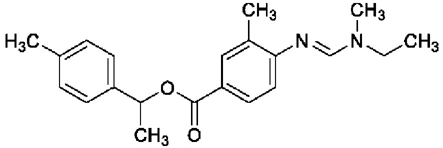 |
| 105 | 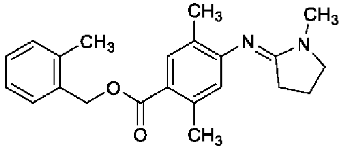 |
| 106 | 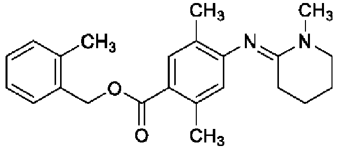 |
| 107 | 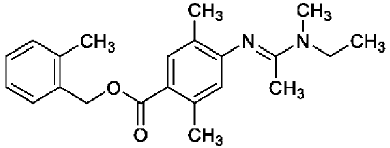 |
| 109 | 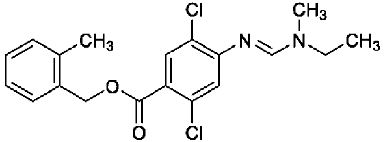 |
| 110 | 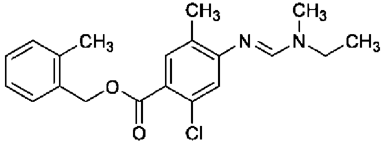 |
| 111 | 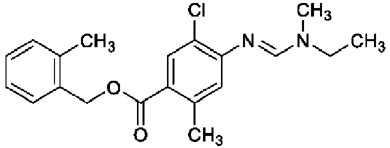 |
| 112 | 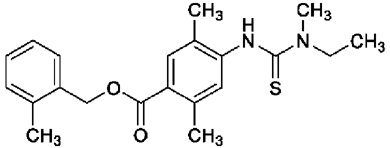 |
| 113 | 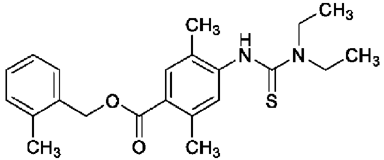 |
| 114 | 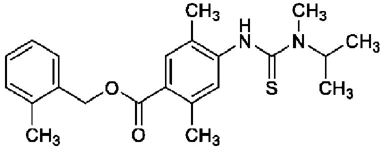 |
| 115 | 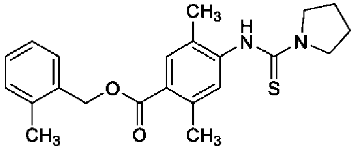 |
| 116 | 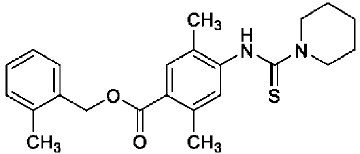 |
| 117 | 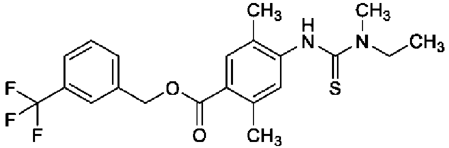 |
| 118 | 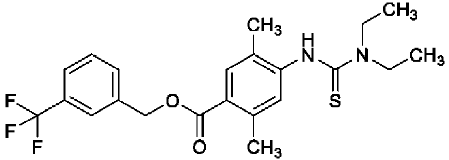 |
| 119 | 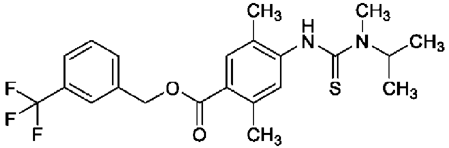 |
| 120 | 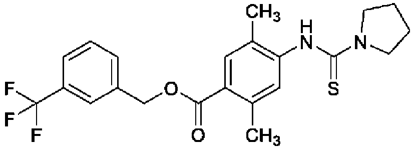 |
| 121 | 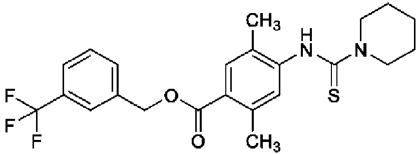 |
| 122 | 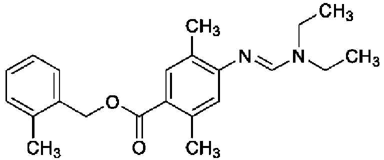 |
| 123 | 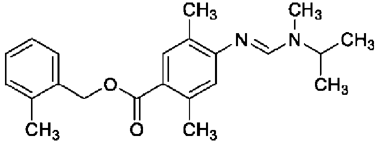 |
| 124 | 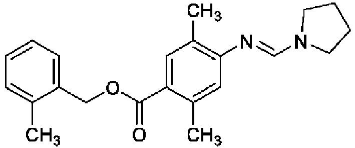 |
| 125 | 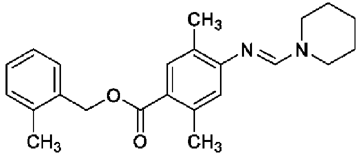 |
| 126 | 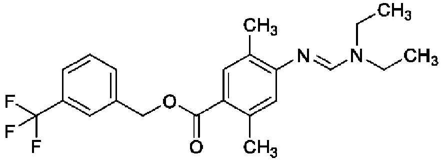 |
| 127 | 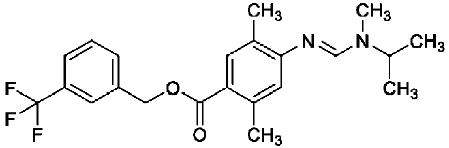 |
| 128 | 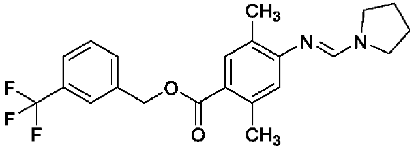 |
| 129 | 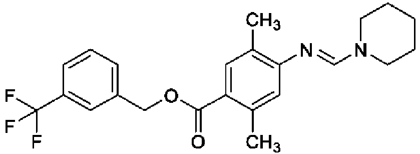 |
| 130 | 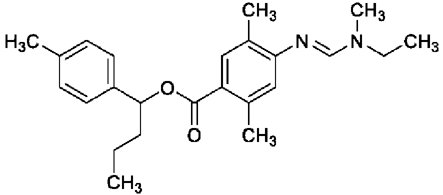 |
| 131 | 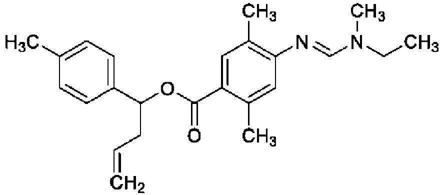 |
| 132 | 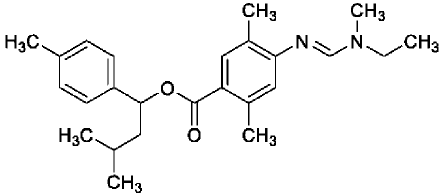 |
| 133 | 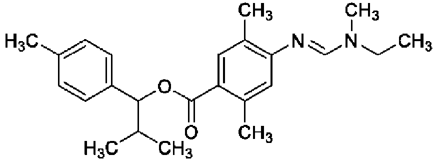 |
| 134 | 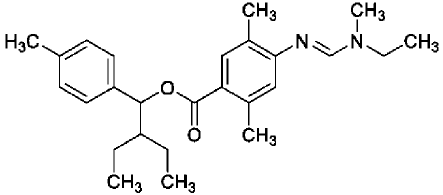 |
| 135 | 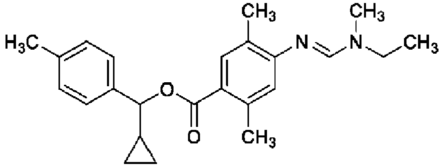 |
| 136 | 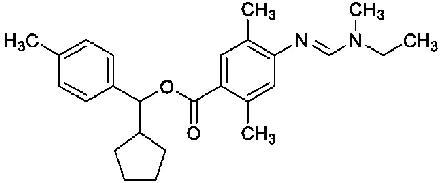 |
| 137 | 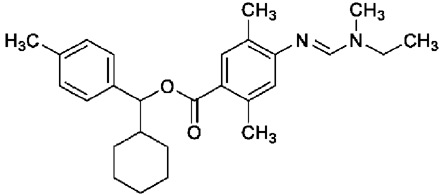 |
| 138 | 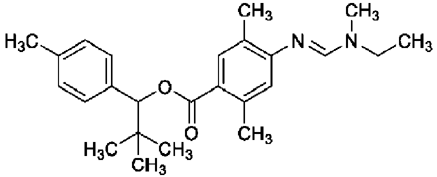 |
| 139 | 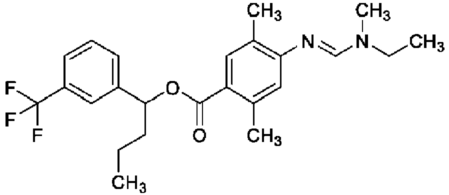 |
| 140 | 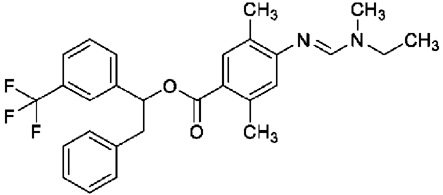 |
| 141 | 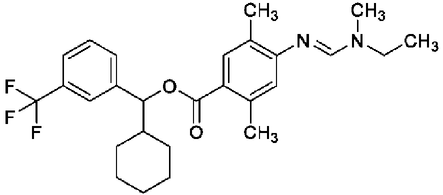 |
| 142 | 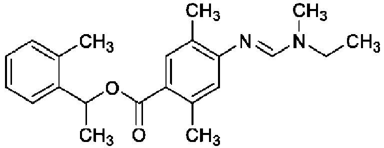 |
| 143 | 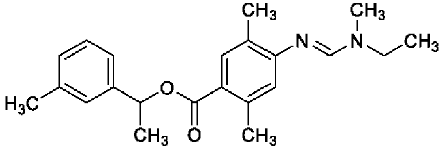 |
| 144 | 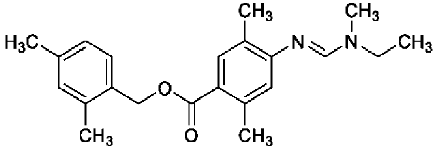 |
| 145 | 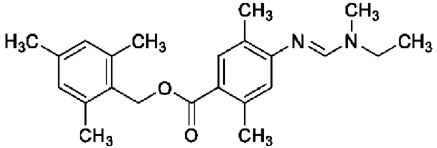 |
| 146 | 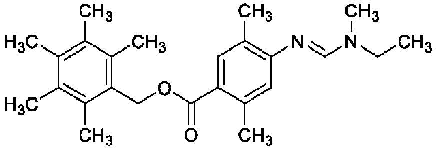 |
| 147 | 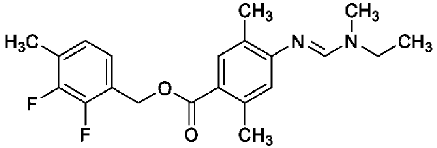 |
| 148 | 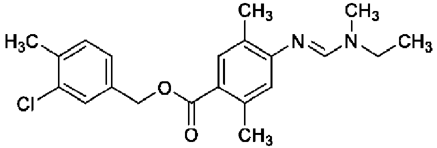 |
| 149 | 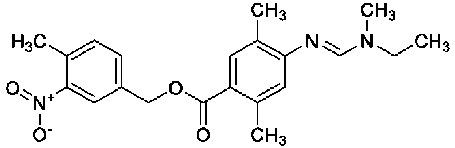 |
| 150 | 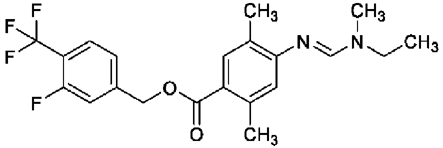 |
| 151 | 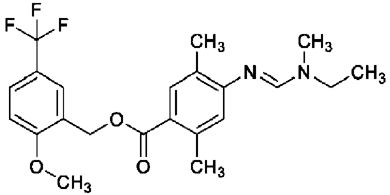 |
| 152 | 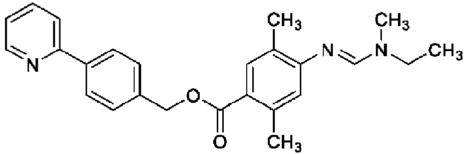 |
| 153 | 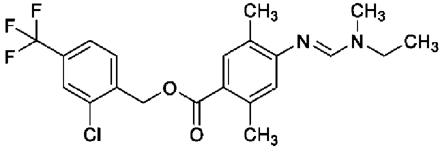 |
| 154 | 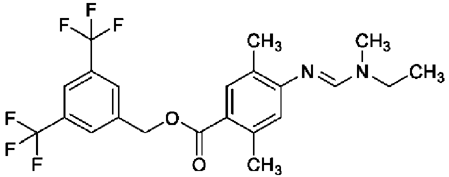 |
| 155 | 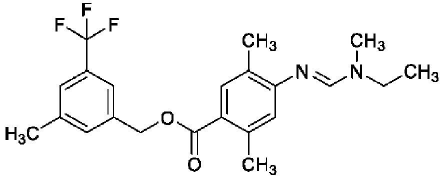 |
| 156 | 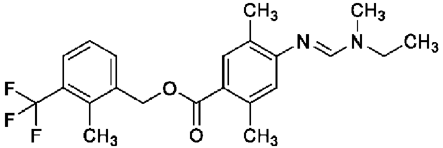 |
| 157 | 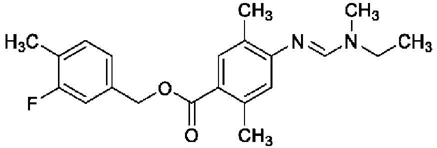 |
| 158 | 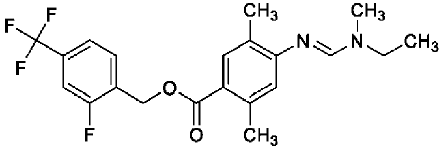 |
| 159 | 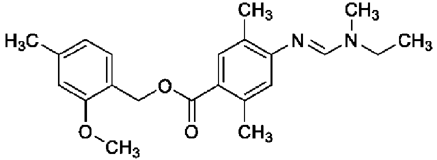 |
| 160 | 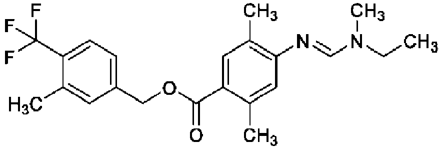 |
| 161 | 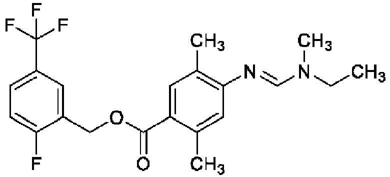 |
| 162 | 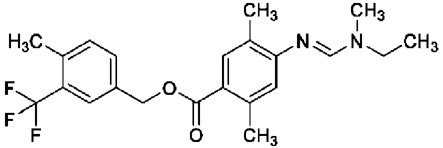 |
| 163 | 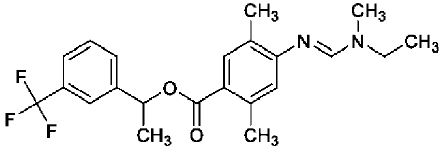 |
| 164 | 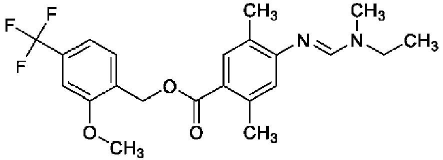 |
| 165 | 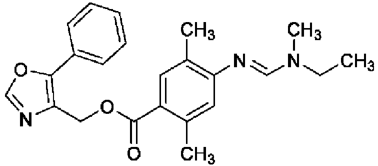 |
| 166 | 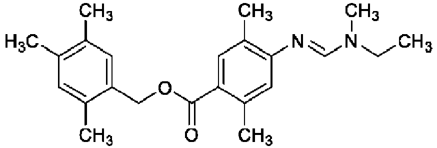 |
| 167 | 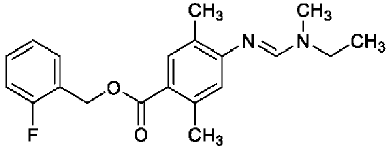 |
| 168 | 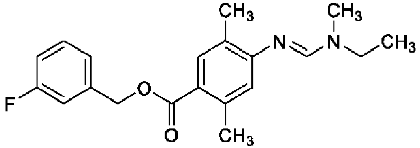 |
| 169 | 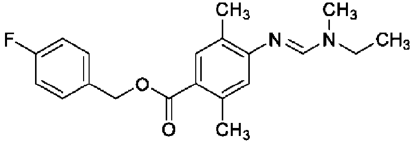 |
| 170 | 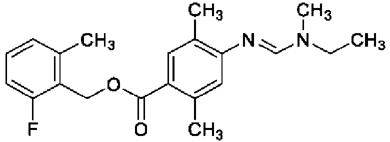 |
| 171 | 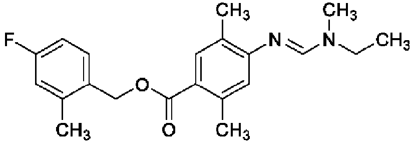 |
| 172 | 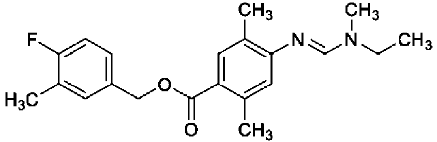 |
| 173 | 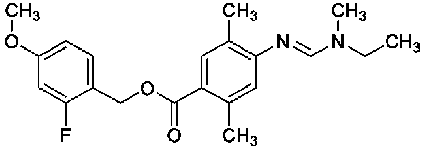 |
| 174 | 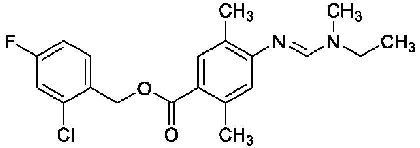 |
| 175 | 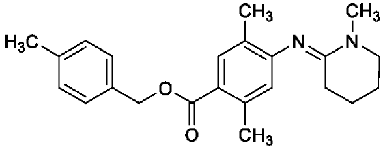 |
| 176 | 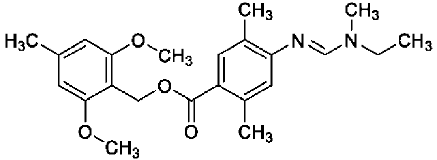 |
| 177 | 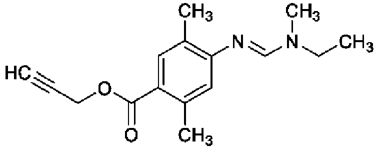 |
| 178 | 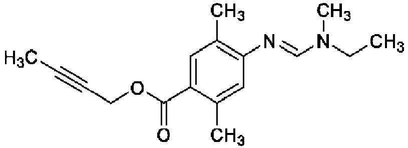 |
| 179 | 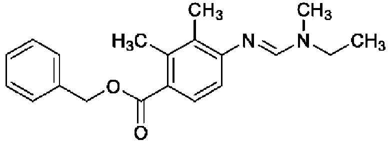 |
| 180 | 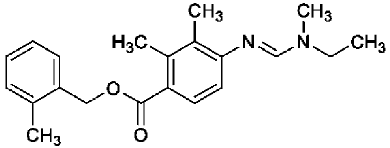 |
| 181 | 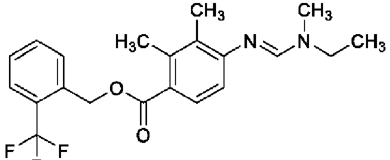 |
| 182 | 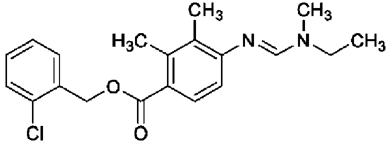 |
| 183 | 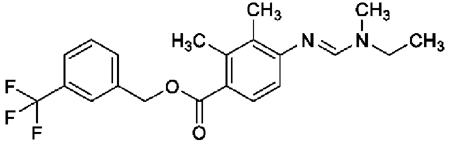 |
| 184 | 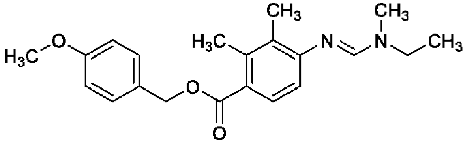 |
| 185 | 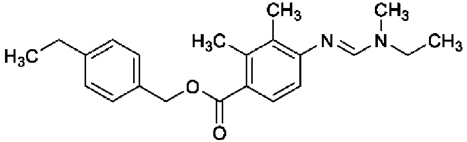 |
| 186 | 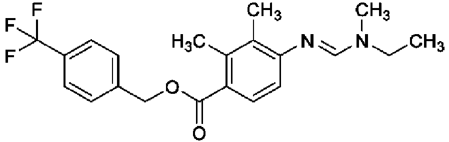 |
| 187 | 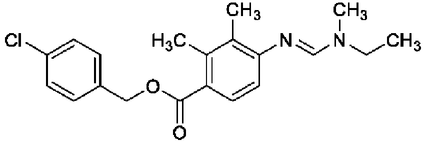 |
| 188 | 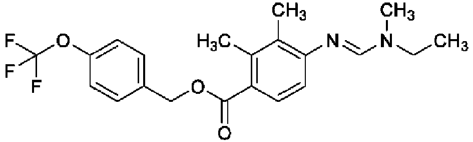 |
| 189 | 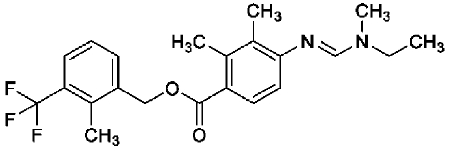 |
| 190 | 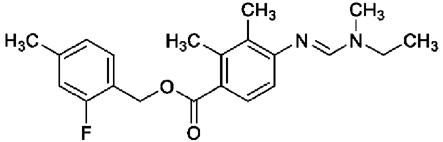 |
| 191 | 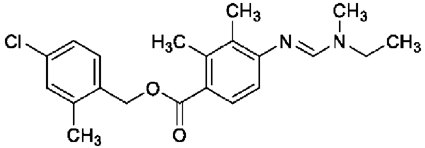 |
| 192 | 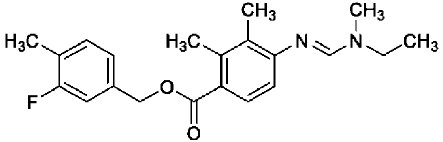 |
| 193 | 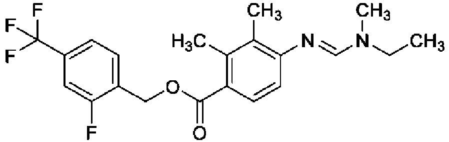 |
| 194 | 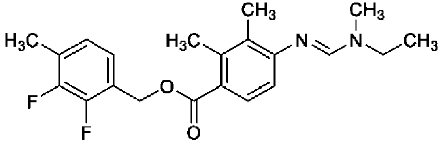 |
| 195 | 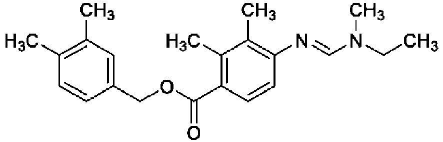 |
| 196 | 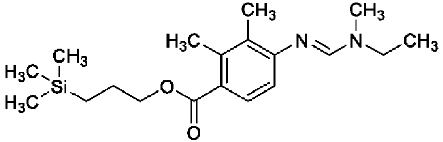 |
| 197 | 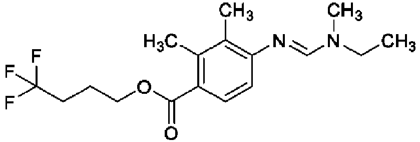 |
| 198 | 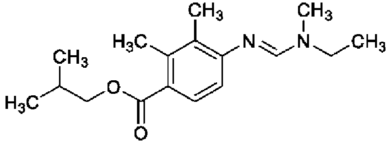 |
| 199 | 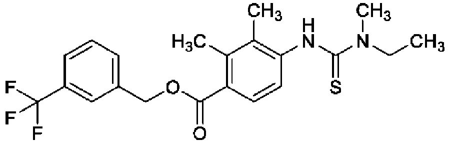 |
| 200 | 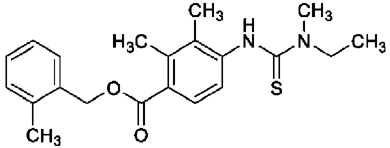 |
| 201 | 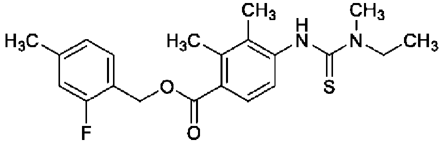 |
| 202 | 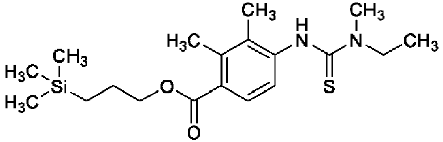 |
| 203 | 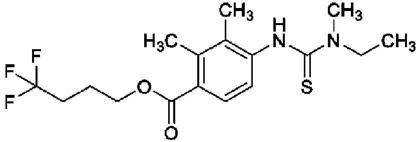 |
| 204 | 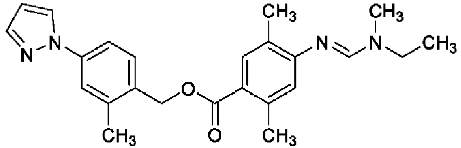 |
| 205 | 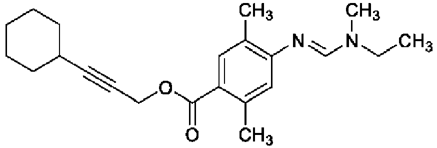 |
| 206 | 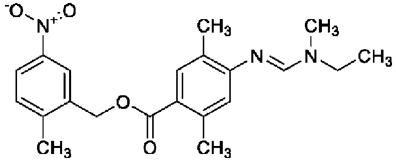 |
| 207 | 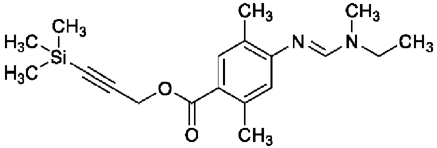 |
| 206 | 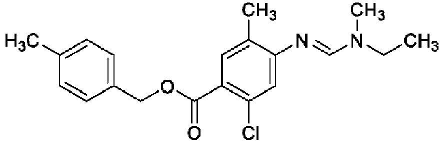 |
| 209 | 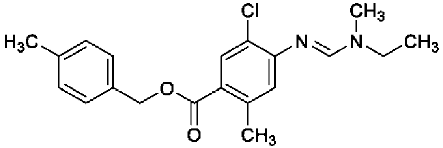 |
| 210 | 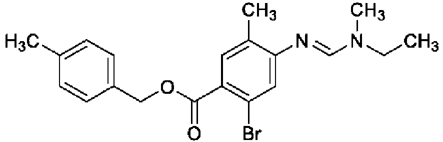 |
| | |
| 212 | 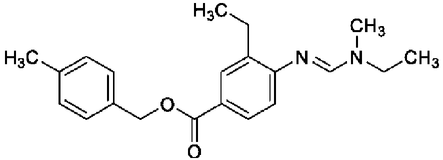 |
| 213 | 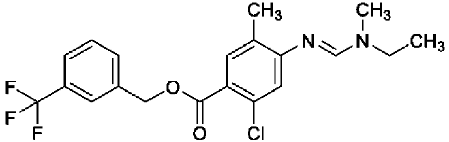 |
| 214 | 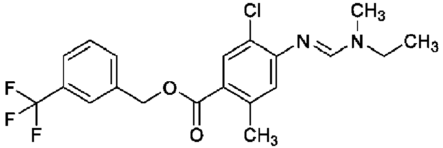 |
| 215 | 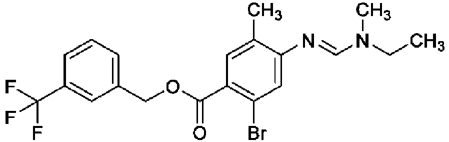 |
| | |
| 217 | 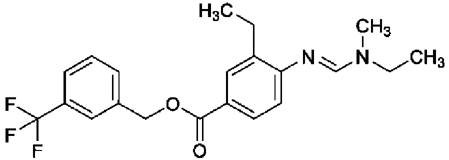 |
| 218 | 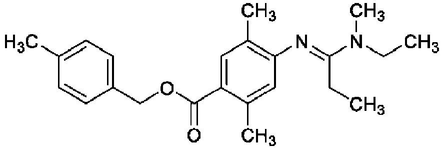 |
| 219 | 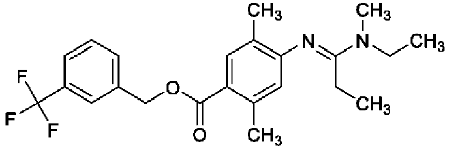 |
| 220 | 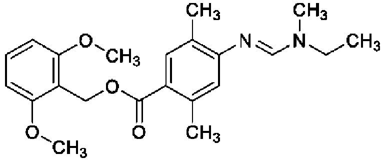 |
| 221 | 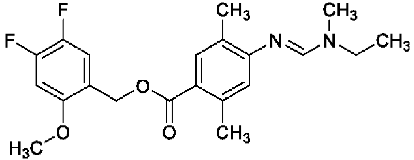 |
| 222 | 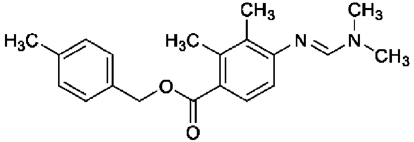 |
| 223 | 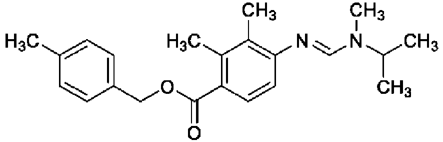 |
| 224 | 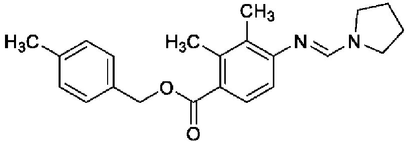 |
| 225 | 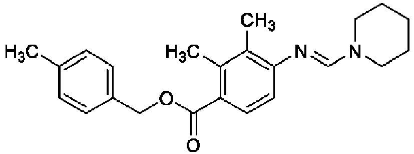 |
| 226 | 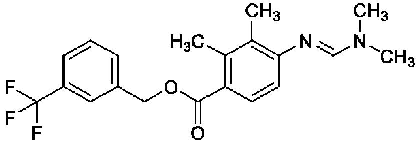 |
| 227 | 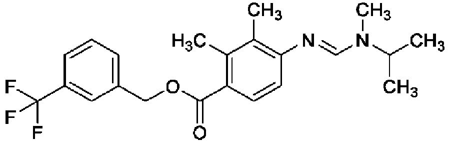 |
| 228 | 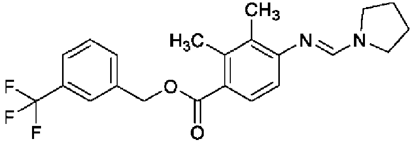 |
| 229 | 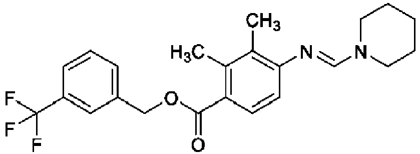 |
| 230 | 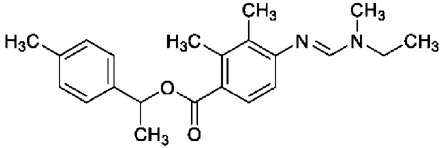 |
| 231 | 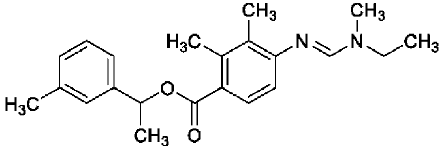 |
| 232 | 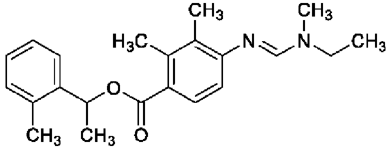 |
| 233 | 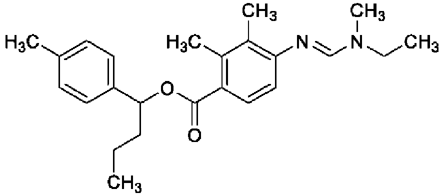 |
| 234 | 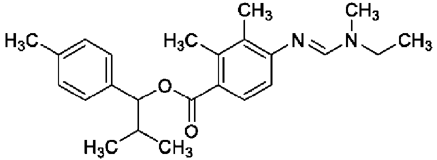 |
| 235 | 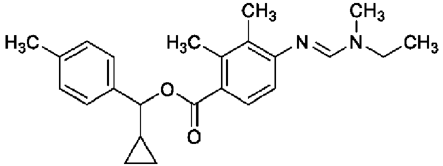 |
| 236 | 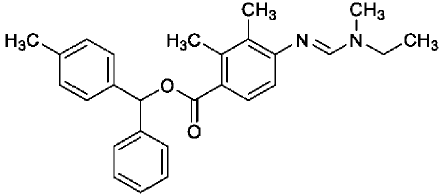 |
| 237 | 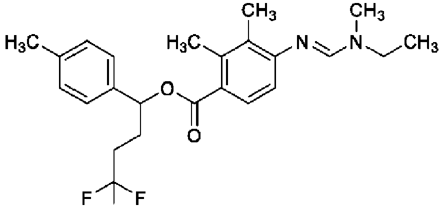 |
| 238 | 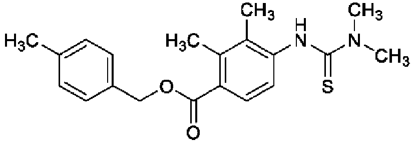 |
| 239 | 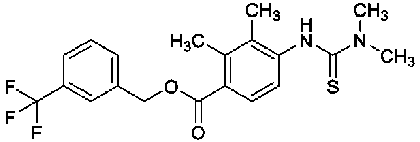 |
| 240 | 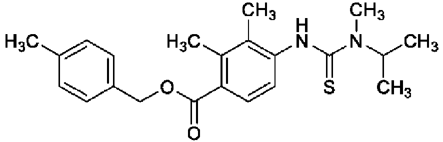 |
| 241 | 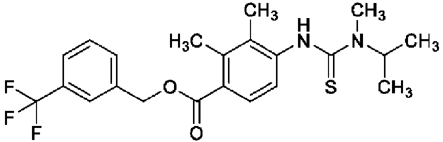 |
| 242 | 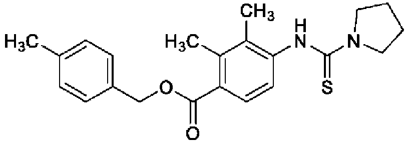 |
| 243 | 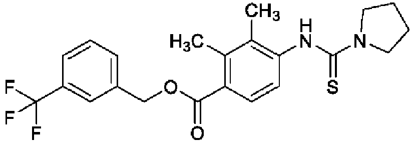 |
| 244 | 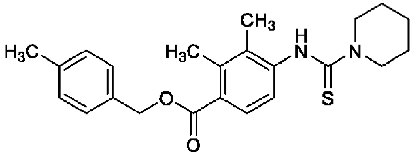 |
| 245 | 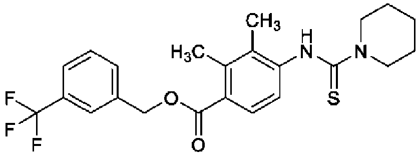 |
| 246 | 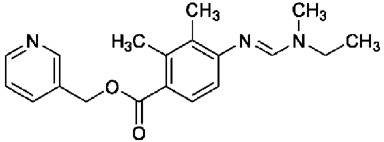 |
| 247 | 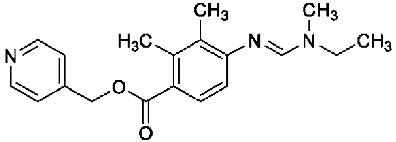 |
| 248 | 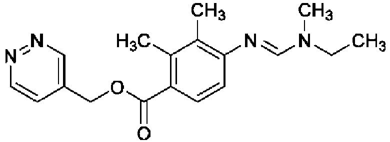 |
| 249 | 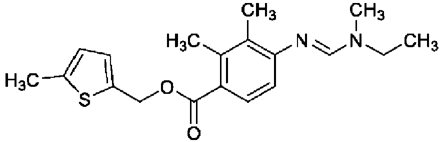 |
| 250 | 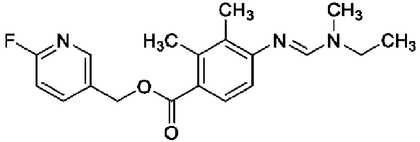 |
| 251 | 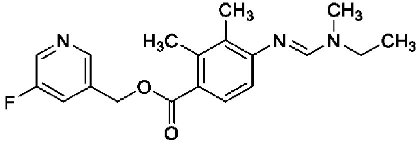 |
| 252 | 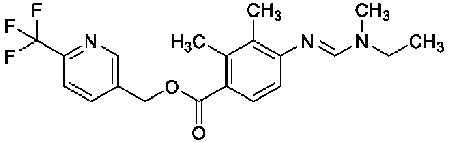 |
| 253 | 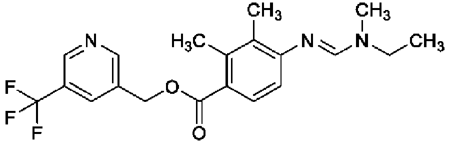 |
| 254 | 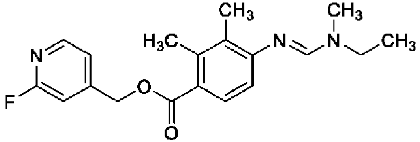 |
| 255 | 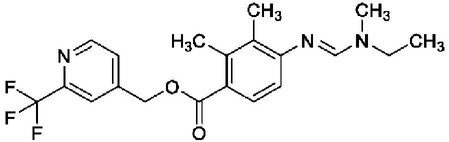 |
| 256 | 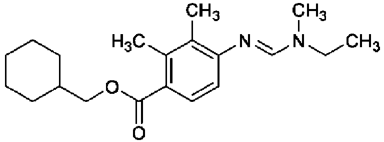 |
| 257 | 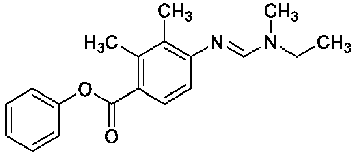 |
| 258 | 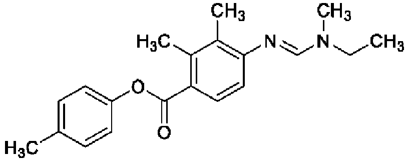 |
| 259 | 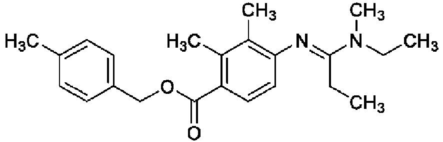 |
| 260 | 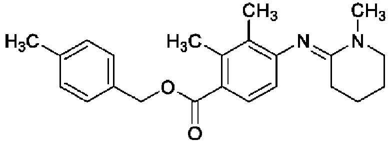 |
| 261 | 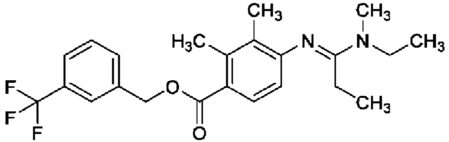 |
| 262 | 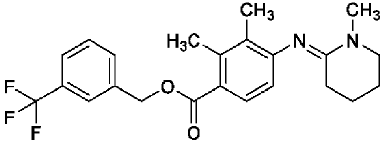 |
| 263 | 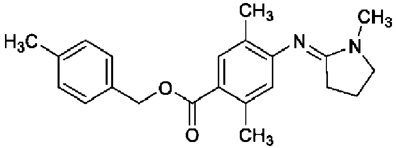 |
| 264 | 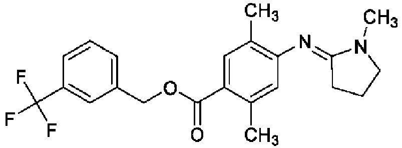 |
| 265 | 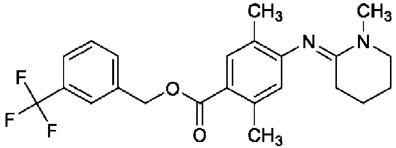 |
| 266 | 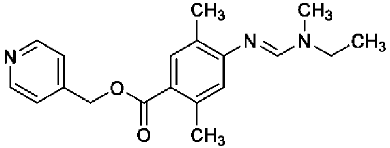 |
| 267 | 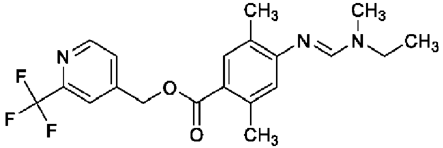 |
| 268 | 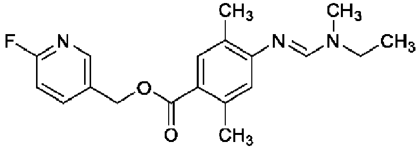 |
| 269 | 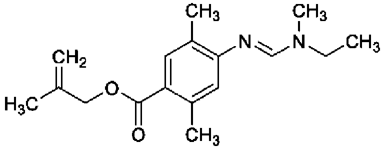 |
| 270 | 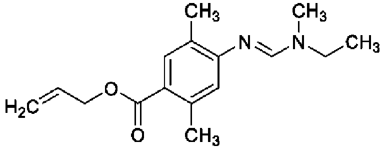 |
| 271 | 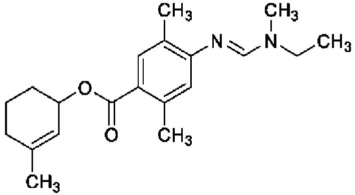 |
| 272 | 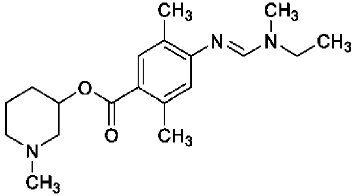 |
| 273 | 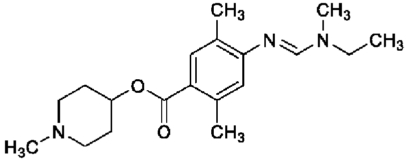 |
| 274 | 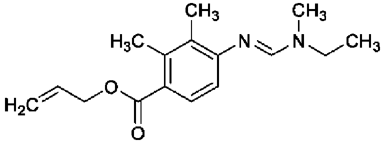 |
| 275 | 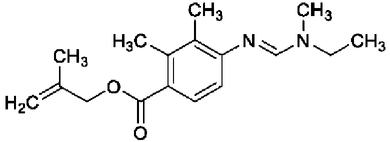 |
| 276 | 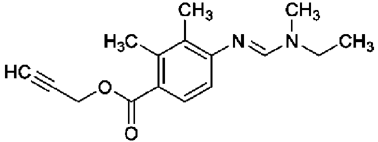 |
| 277 | 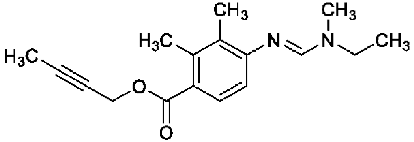 |
| 278 | 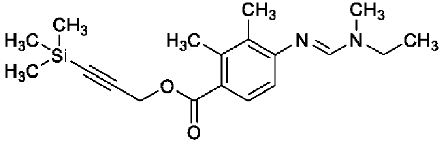 |
| 279 | 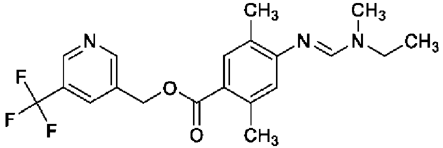 |
| 280 | 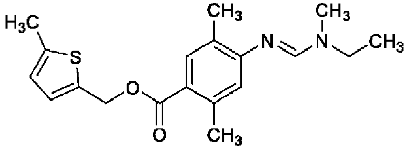 |
| 285 | 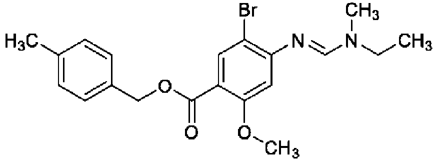 |
| 286 | 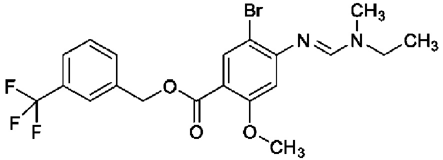 |
| 287 | 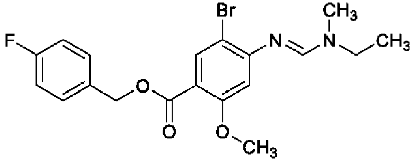 |
| 288 | 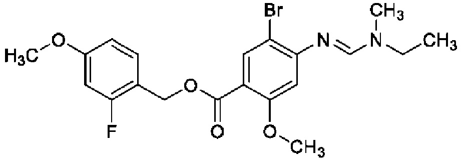 |
| 289 | 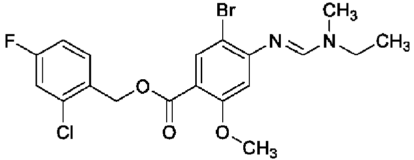 |
| 290 | 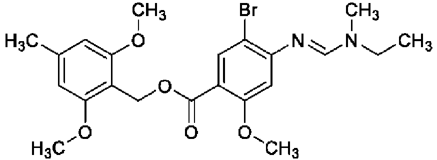 |
| 291 | 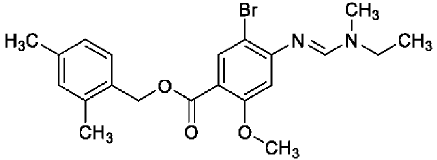 |
| 292 | 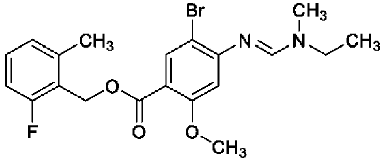 |
| 293 | 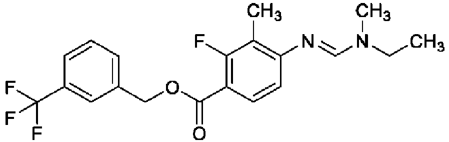 |
| 2944 | 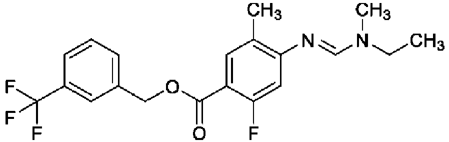 |
| 295 | 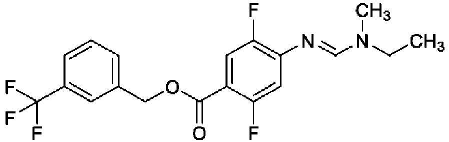 |
| 296 | 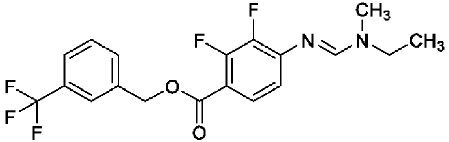 |
| 297 | 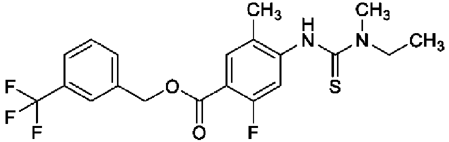 |
| 298 | 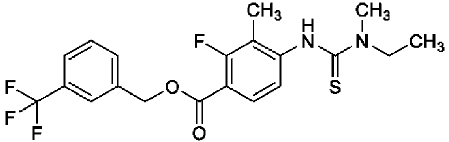 |
| 299 | 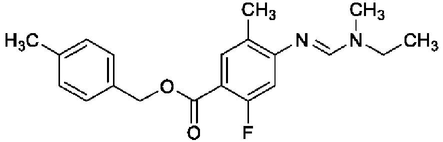 |
| 300 | 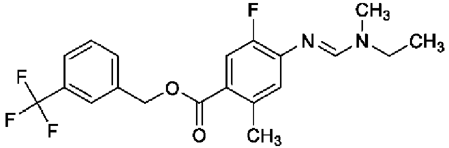 |
| 301 | 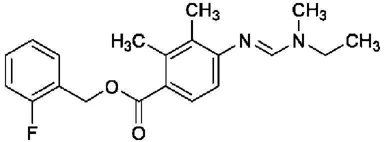 |
| 302 | 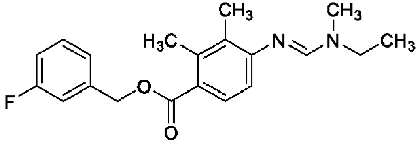 |
| 303 | 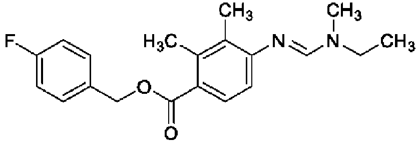 |
| 304 | 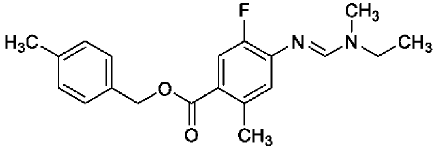 |
| 305 | 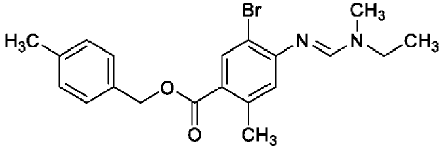 |
| 306 | 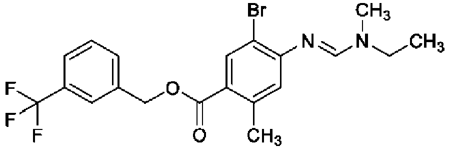 |
| 307 | 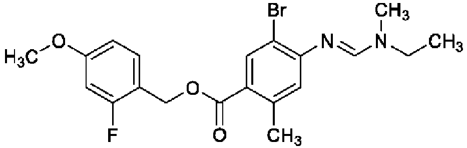 |
| 308 | 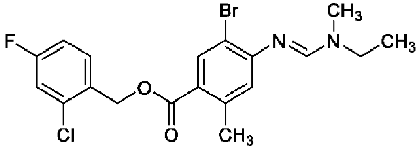 |
| 309 | 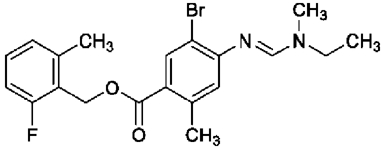 |
| 310 | 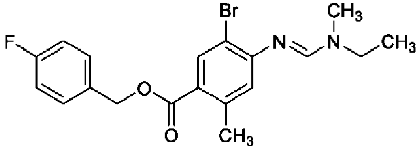 |
| 311 | 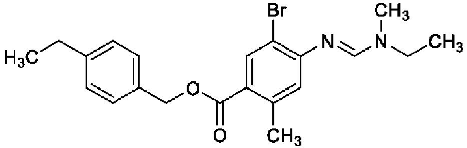 |
| 312 | 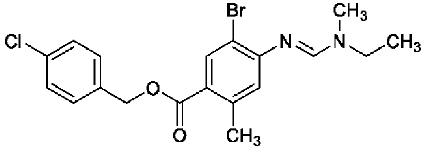 |
| 313 | 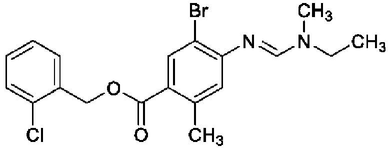 |
| 314 | 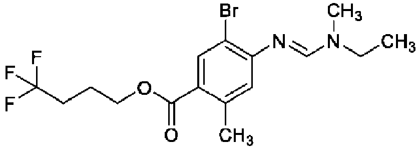 |
| 315 | 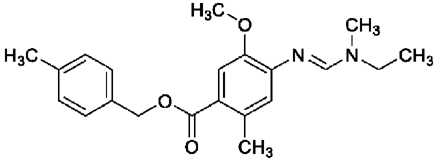 |
| 316 | 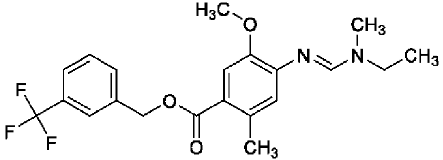 |
| 317 | 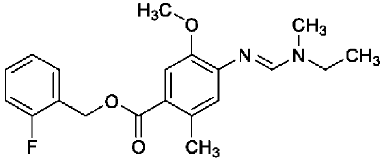 |
| 318 | 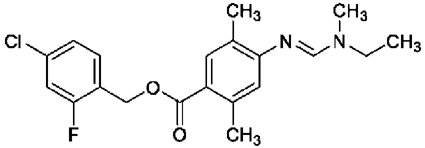 |
| 319 | 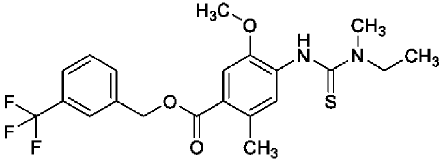 |
| 320 | 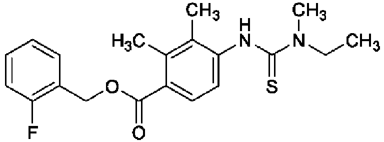 |
| 321 | 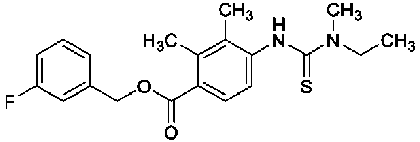 |
| 322 | 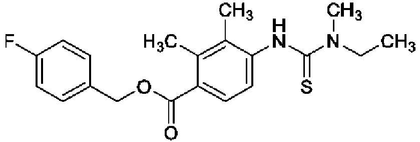 |
| 323 | 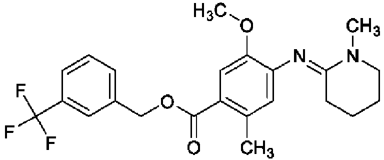 |
| 324 | 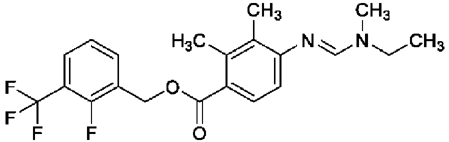 |
| 325 | 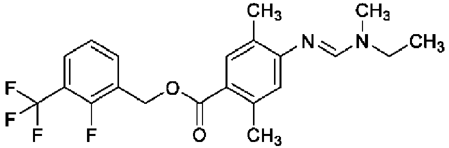 |
| 326 | 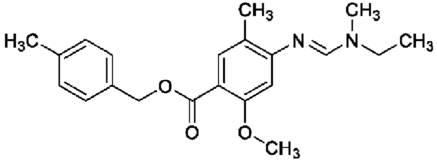 |
| 327 | 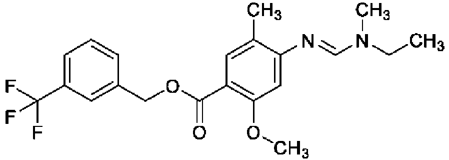 |
| 328 | 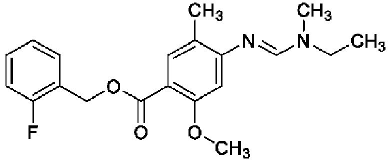 |
| 329 | 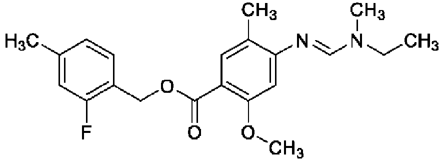 |
| 330 | 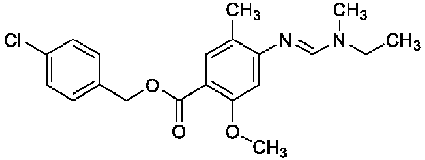 |
| 331 | 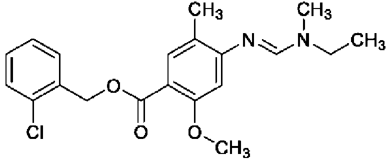 |
| 332 | 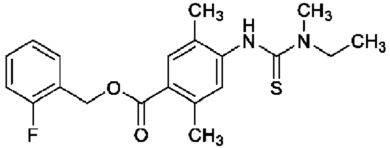 |
| 333 | 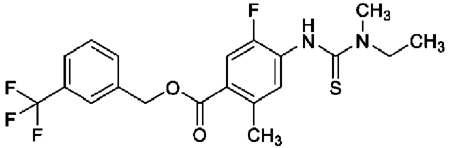 |
| 334 | 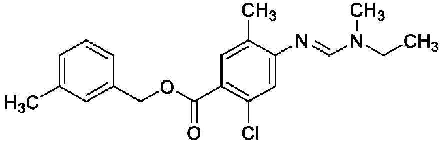 |
| 335 | 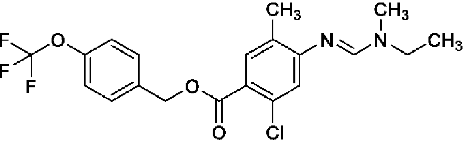 |
| 336 | 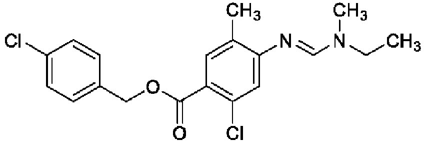 |
| 337 | 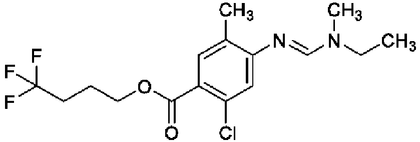 |
| 338 | 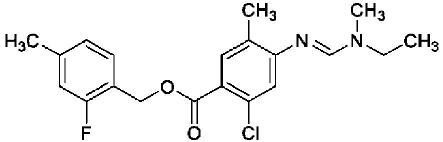 |
| 339 | 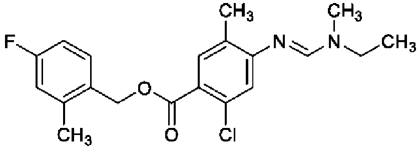 |
| 340 | 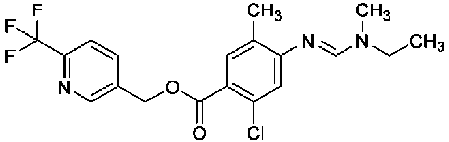 |
| 341 | 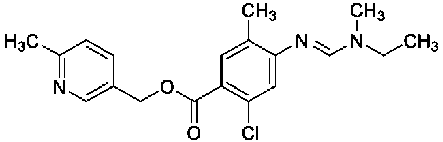 |
| 342 | 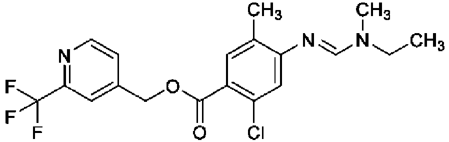 |
| 343 | 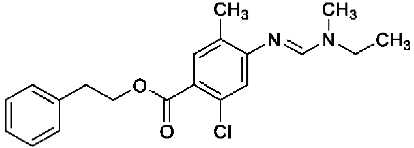 |
| 344 | 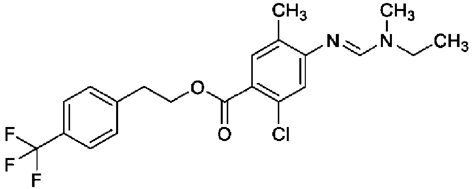 |
| 345 | 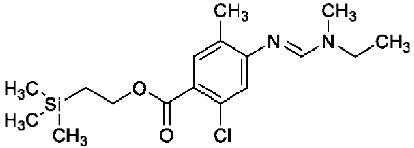 |
| 346 | 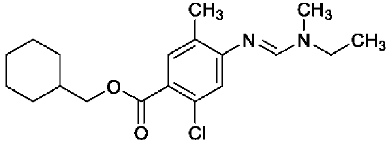 |
| 347 | 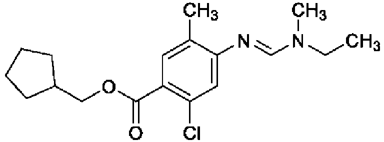 |
| 348 | 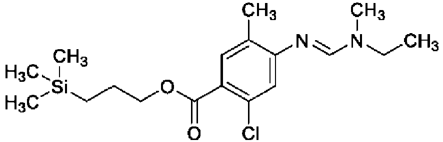 |
| 349 | 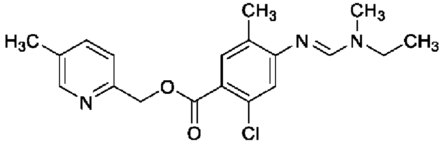 |
| 350 | 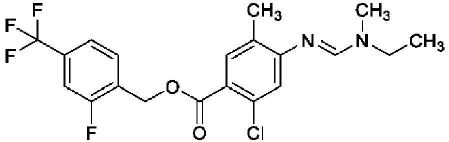 |
| 351 | 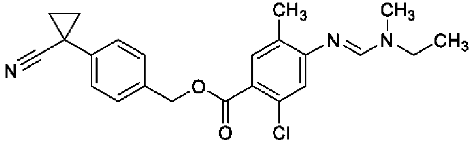 |
| 352 | 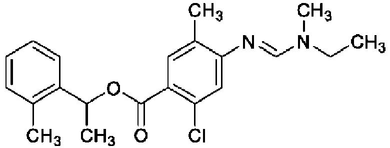 |
| 353 | 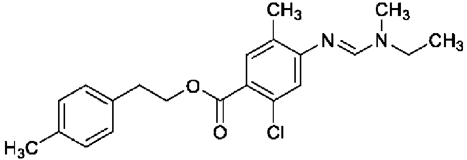 |
| 354 | 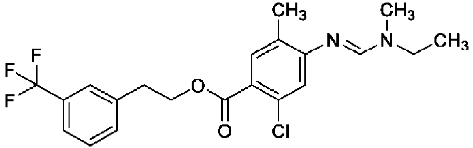 |
| 355 | 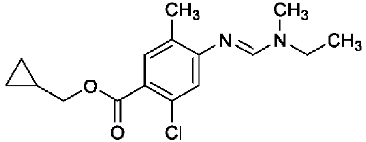 |
| 356 | 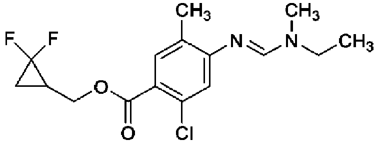 |
| 357 | 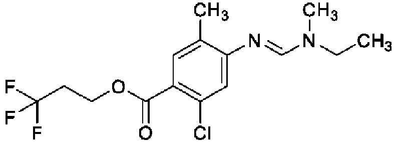 |
| 358 | 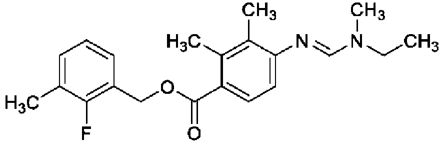 |
| 359 | 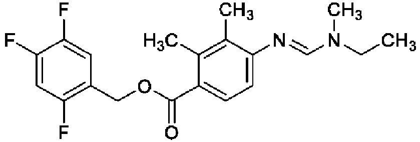 |
| 360 | 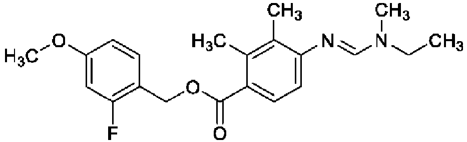 |
| 361 | 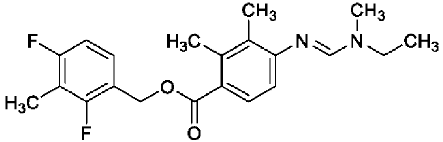 |
| 362 | 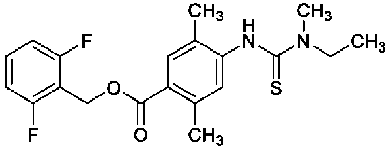 |
| 363 | 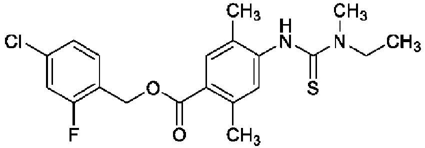 |
| 364 | 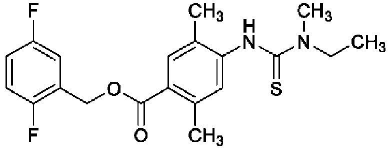 |
| 365 | 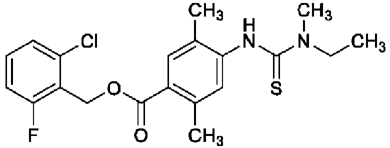 |
| 366 | 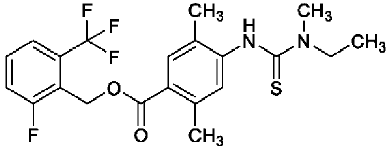 |
| 367 | 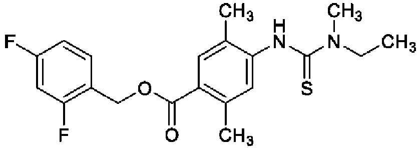 |
| 368 | 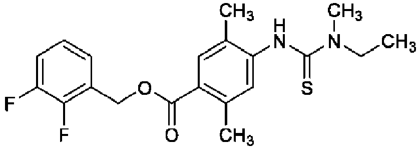 |
| 369 | 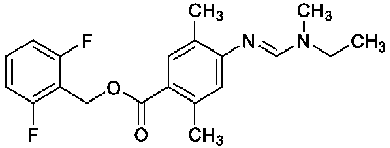 |
| 370 | 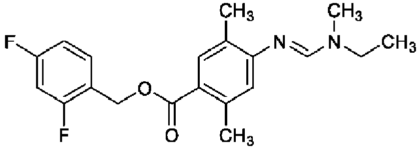 |
| 371 | 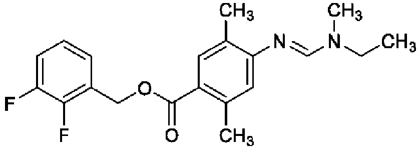 |
| 372 | 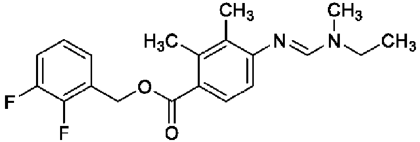 |
| 373 | 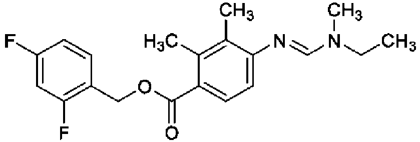 |
| 374 | 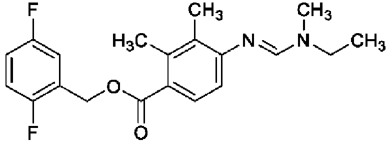 |
| 375 | 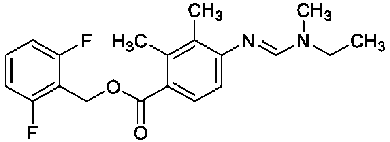 |
| 376 | 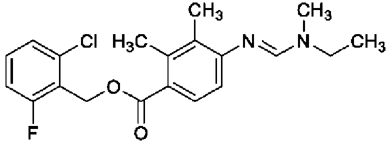 |
| 377 | 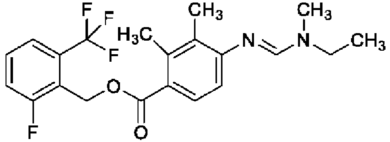 |
| 378 | 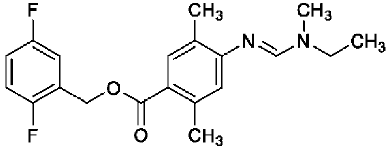 |
| 379 | 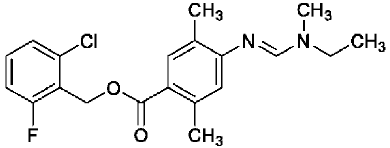 |
| 380 | 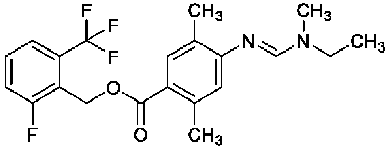 |
| 381 | 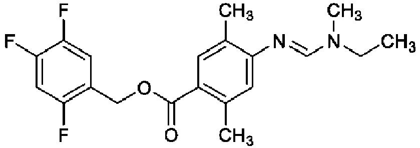 |
| 382 | 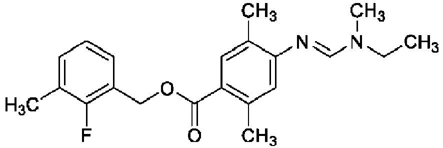 |
| 383 | 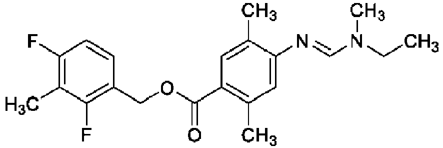 |
| 384 | 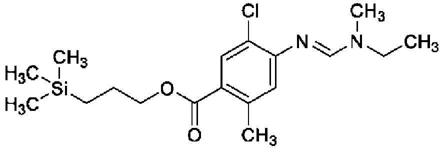 |
| 385 | 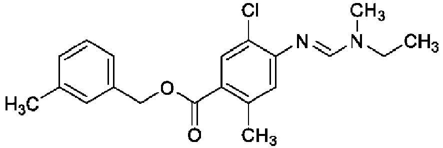 |
| 386 | 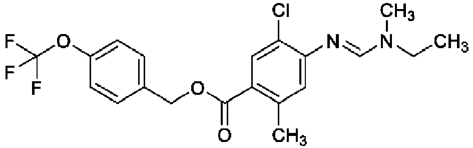 |
| 387 | 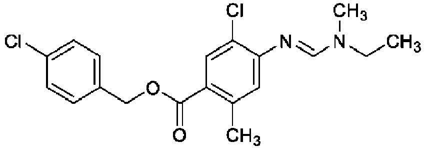 |
| 388 | 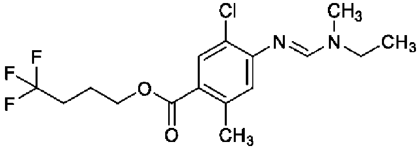 |
| 389 | 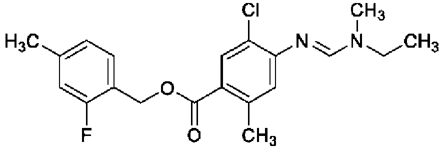 |
| 390 | 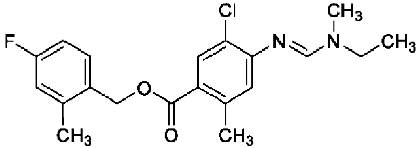 |
| 391 | 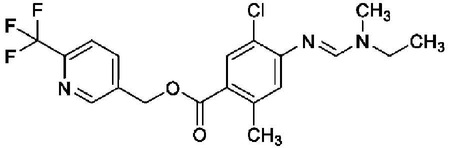 |
| 392 | 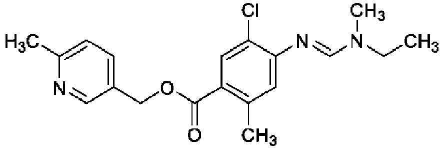 |
| 393 | 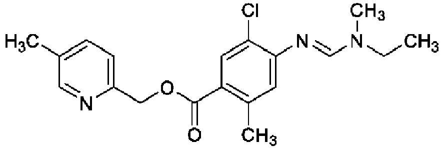 |
| 394 | 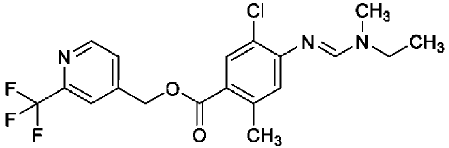 |
| 395 | 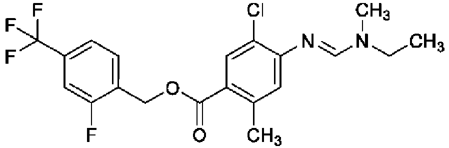 |
| 396 | 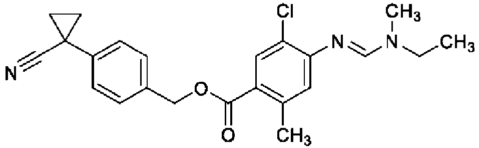 |
| 397 | 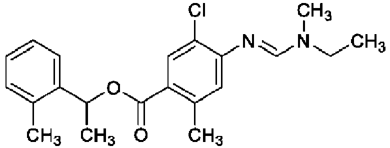 |
| 398 | 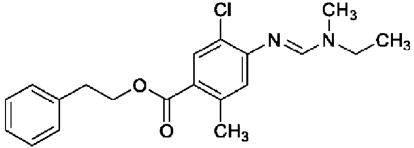 |
| 399 | 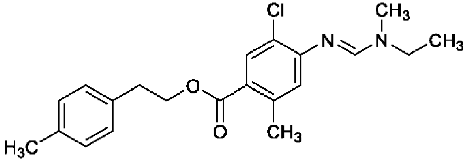 |
| 400 | 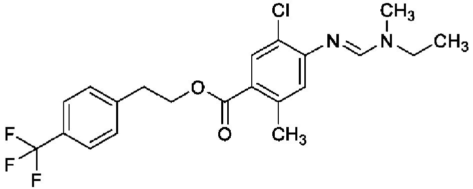 |
| 401 | 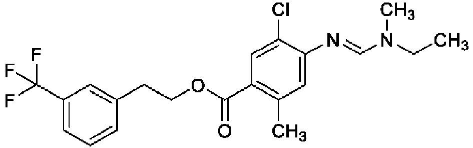 |
| 402 | 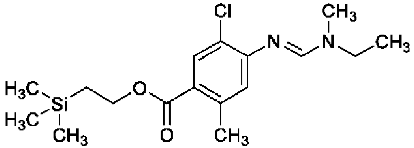 |
| 403 | 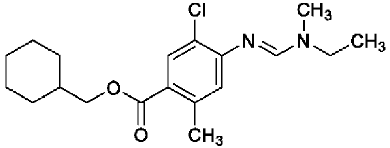 |
| 404 | 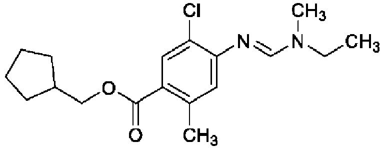 |
| 405 | 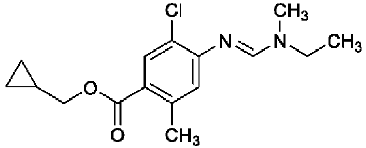 |
| 406 | 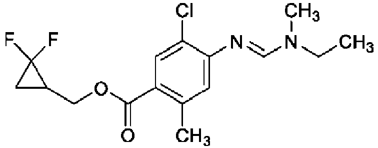 |
| 407 | 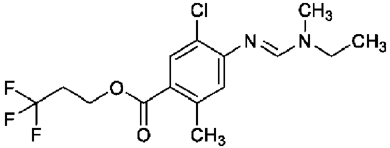 |
| 408 | 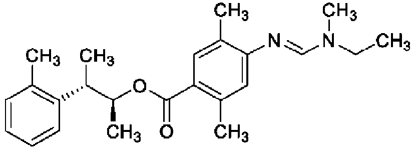 |
| 409 | 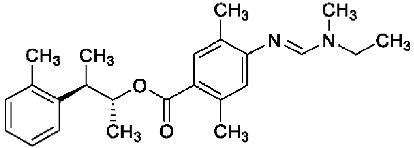 |
| 410 | 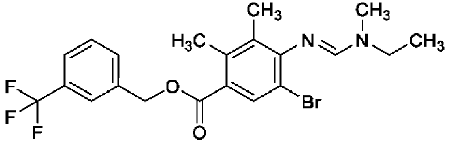 |
| 411 | 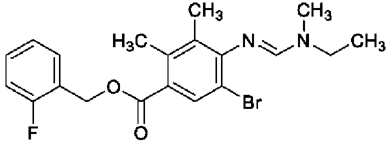 |
| 412 | 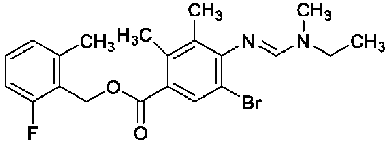 |
| 413 | 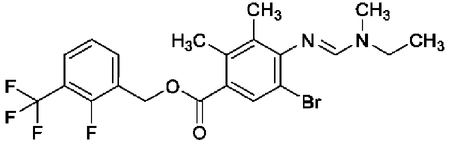 |
| 414 | 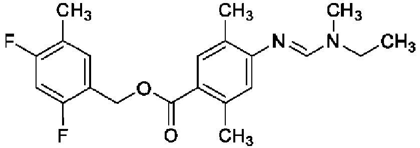 |
| 415 | 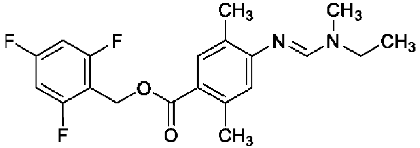 |
| 416 | 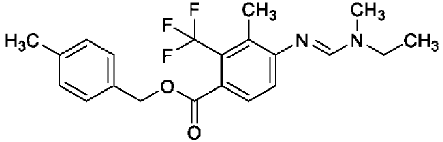 |
| 417 | 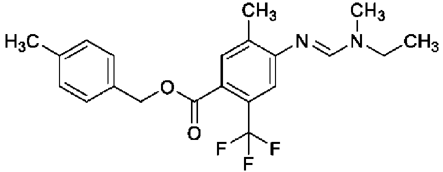 |
| 418 | 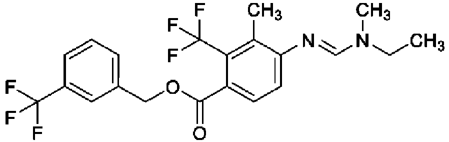 |
| 419 | 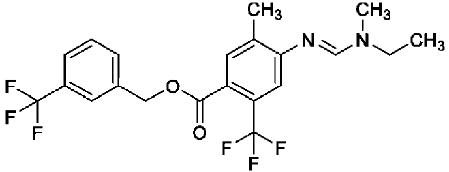 |
| 420 | 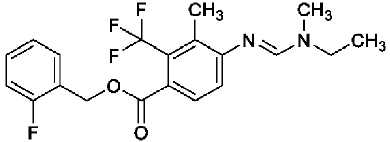 |
| 421 | 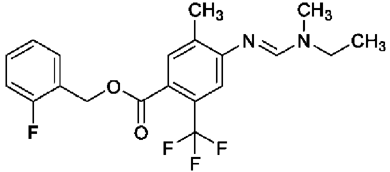 |
| 422 | 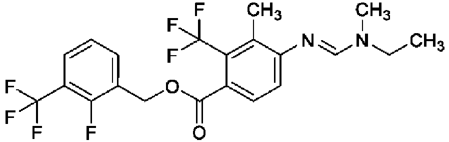 |
| 423 | 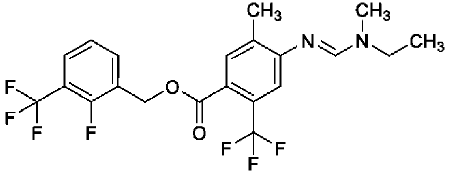 |
| 424 | 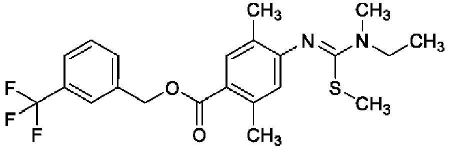 |
| 425 | 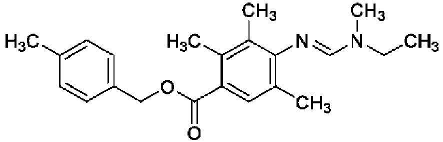 |
| 426 | 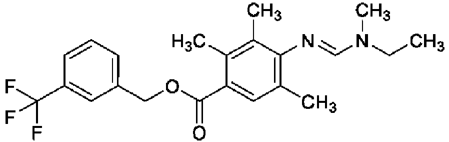 |
| 427 | 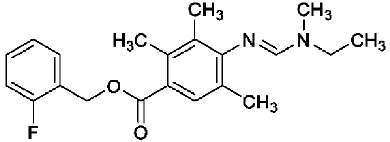 |
| 428 | 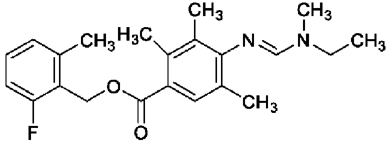 |
| 429 | 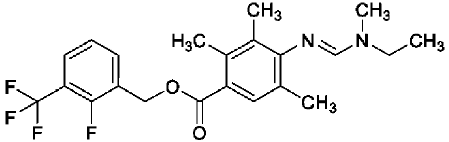 |
| 430 | 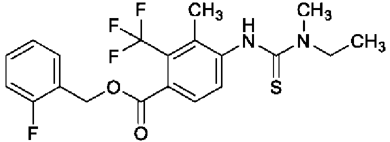 |
| 431 | 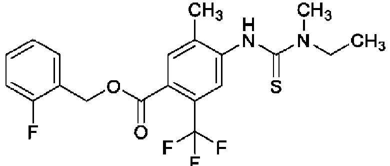 |
| 432 | 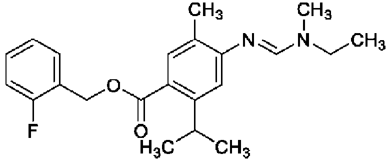 |
| 433 | 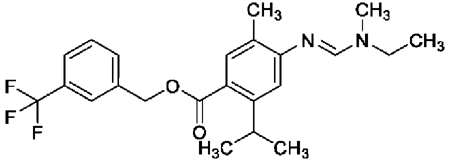 |
| 434 | 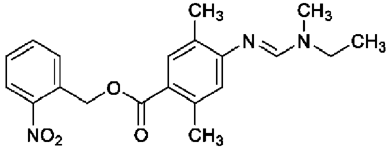 |
| 435 | 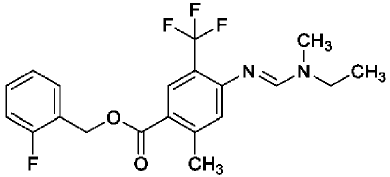 |
| 436 | 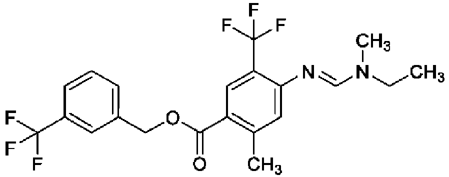 |
| 437 | 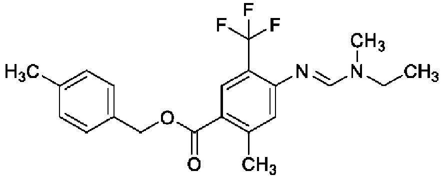 |
| 438 | 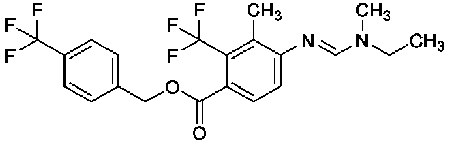 |
| 439 | 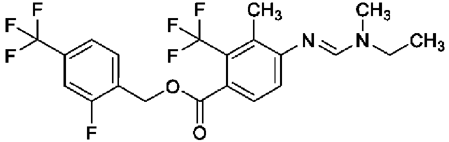 |
| 440 | 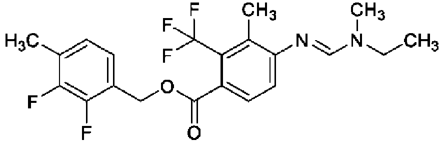 |
| 441 | 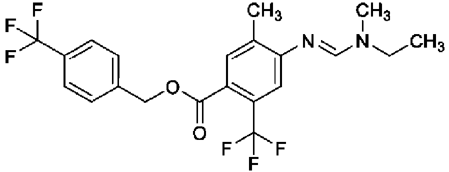 |
| 442 | 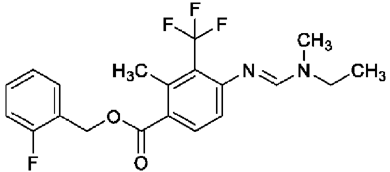 |
| 443 | 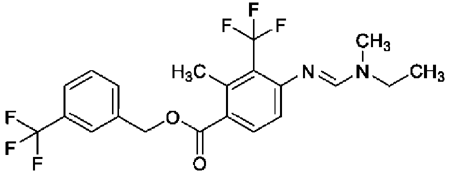 |
| 444 | 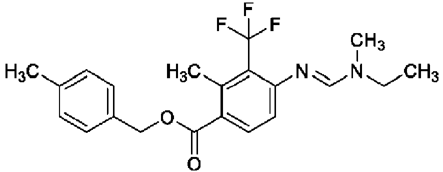 |
| 445 | 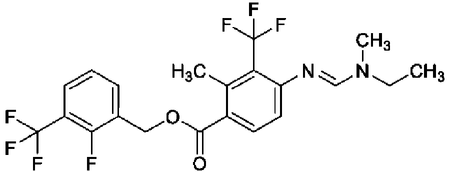 |
| 446 | 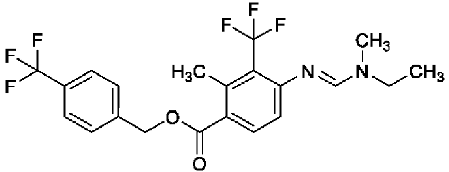 |
| 447 | 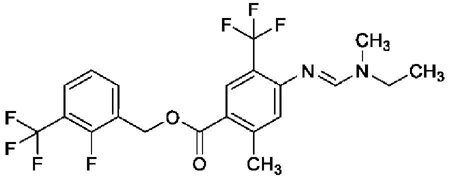 |
| 448 | 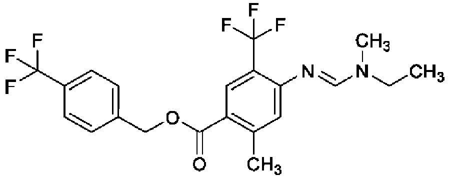 |
| 449 | 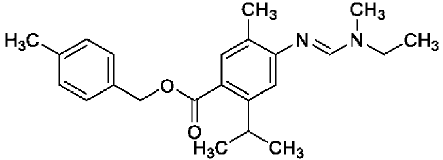 |
| 450 | 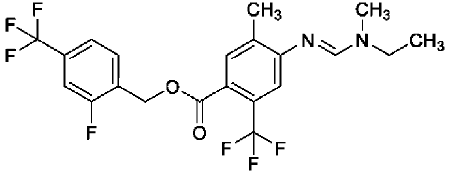 |
| 451 | 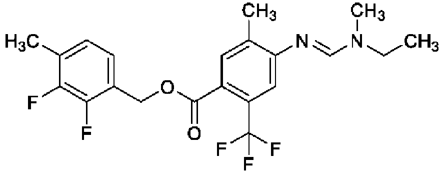 |
| 452 | 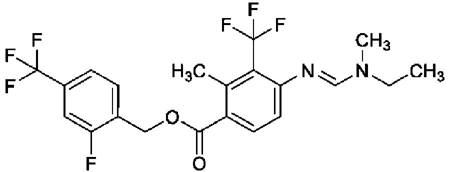 |
| 453 | 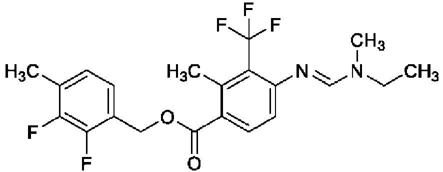 |
| 454 | 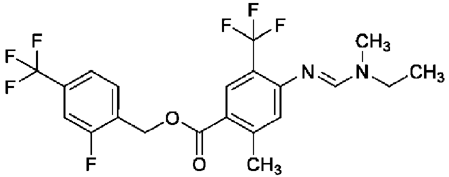 |
| 455 | 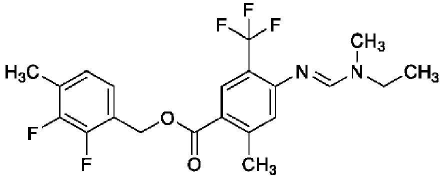 |
| 456 | 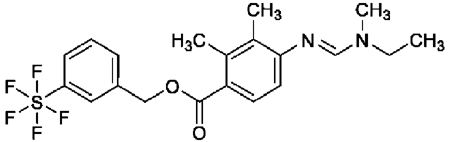 |
| 457 | 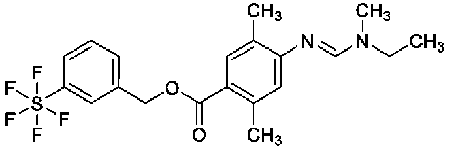 |
| 458 | 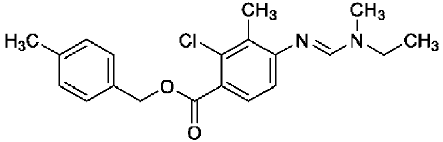 |
| 459 | 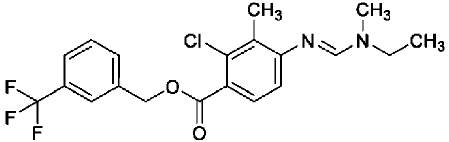 |
| 460 | 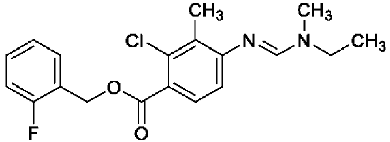 |
| 461 | 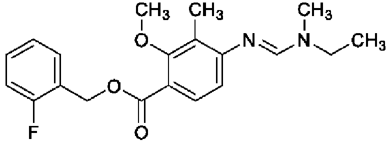 |
| 462 | 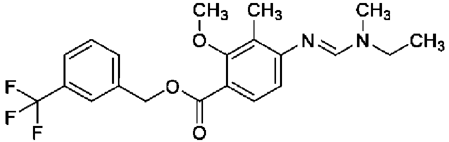 |
| 463 | 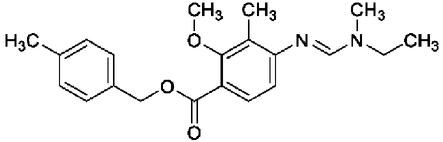 |
| 464 | 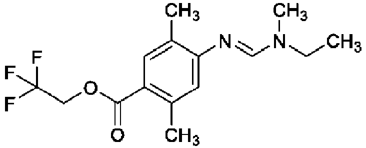 |
| 465 | 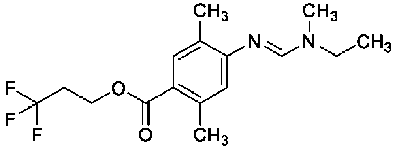 |
| 466 | 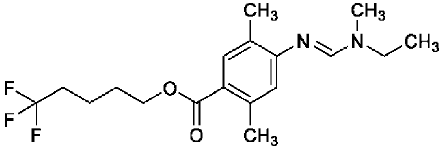 |
| 467 | 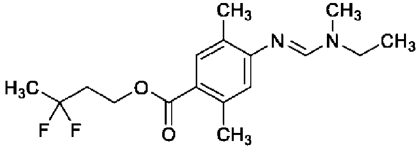 |
| 468 | 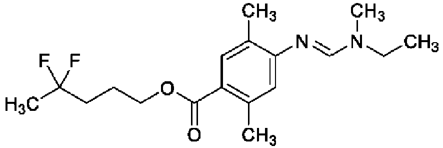 |
| 469 | 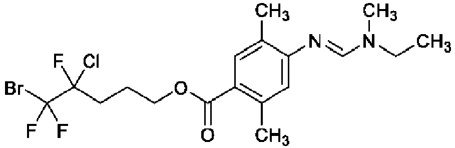 |
| 470 | 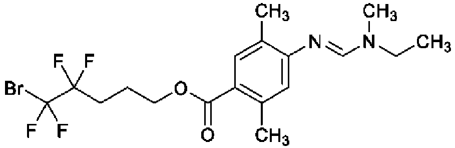 |
| 471 | 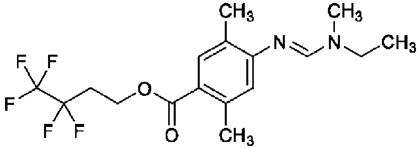 |
| 472 | 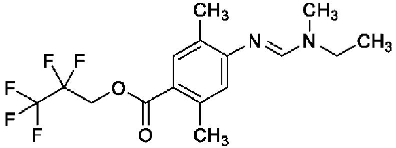 |
| 473 | 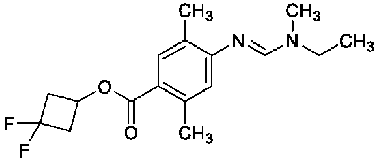 |
| 474 | 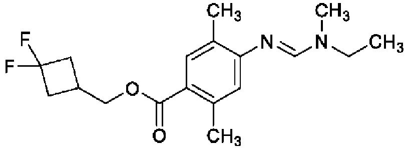 |
| 475 | 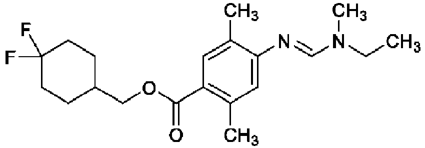 |
| 476 | 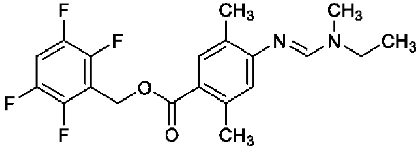 |
| 477 | 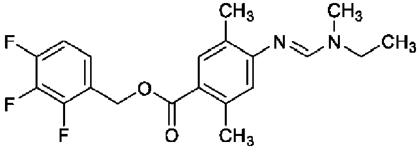 |
| 478 | 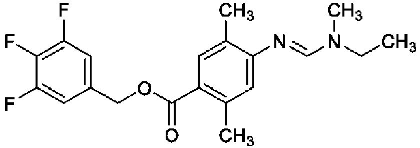 |
| 479 | 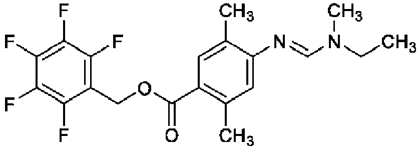 |
| 480 | 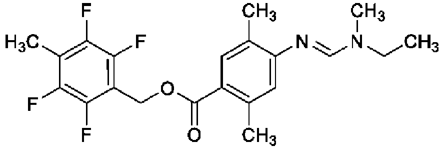 |
| 481 | 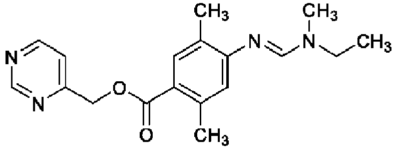 |
| 482 | 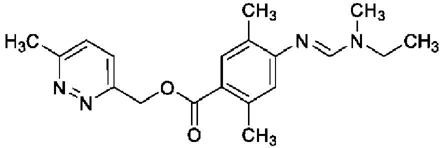 |
| 483 | 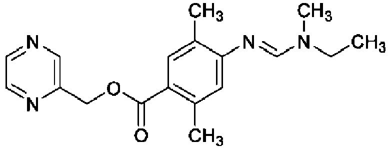 |
| 484 | 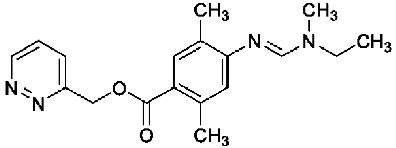 |
| 485 | 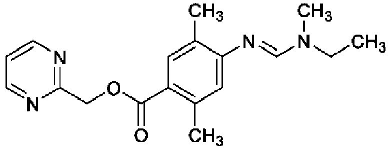 |
| 486 | 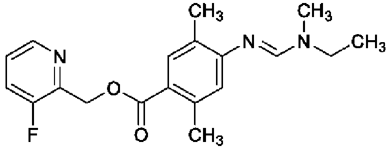 |
| 487 | 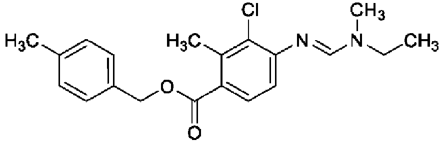 |
| 488 | 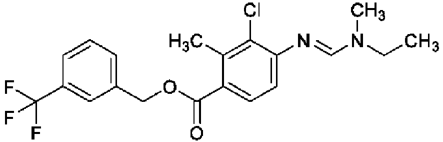 |
| 489 | 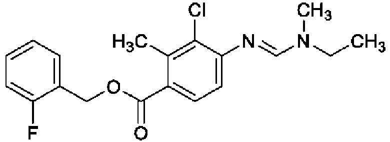 |
| 490 | 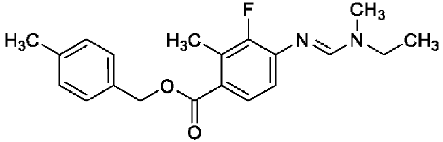 |
| 491 | 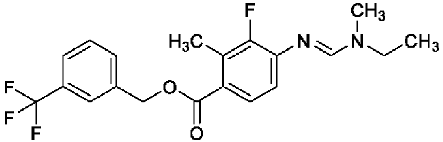 |
| 492 | 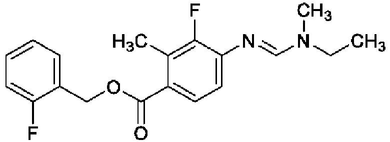 |
| 493 | 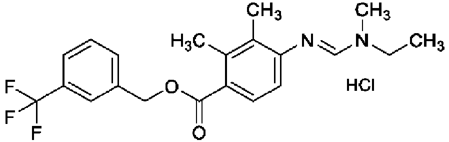 |
| 494 | 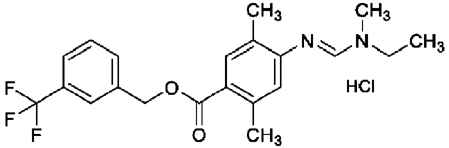 |
| 495 | 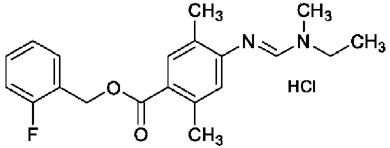 |
| 496 | 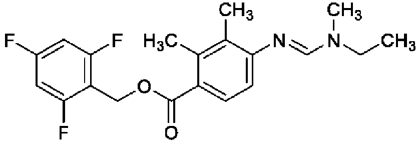 |
| 497 | 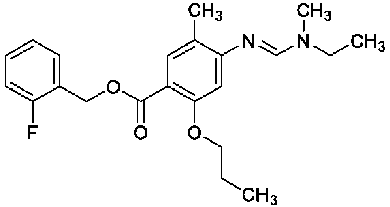 |
| 498 | 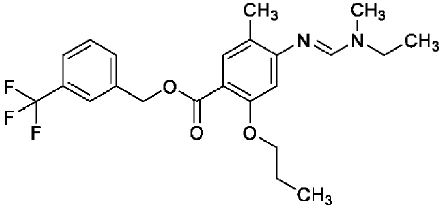 |
| 499 | 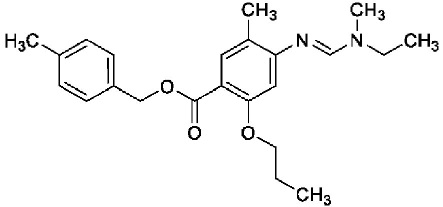 |
| 500 | 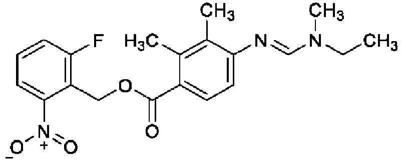 |
| 501 | 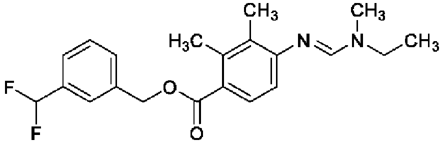 |
| 502 | 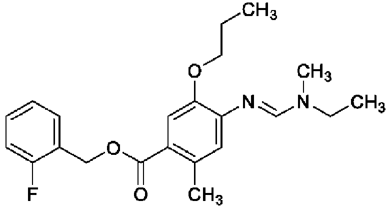 |
| 503 | 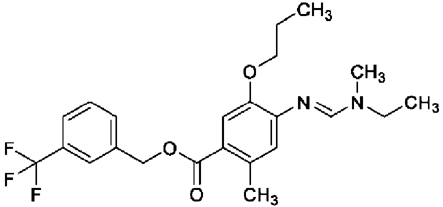 |
| 504 | 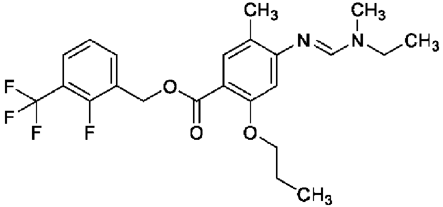 |
| 505 | 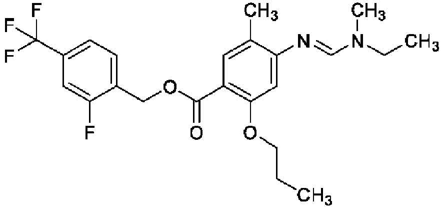 |
| 506 | 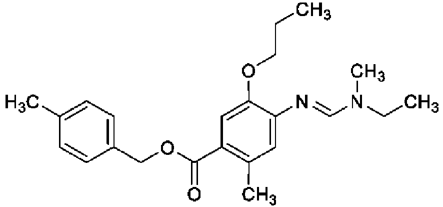 |
| 507 | 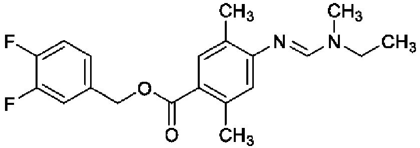 |
| 508 | 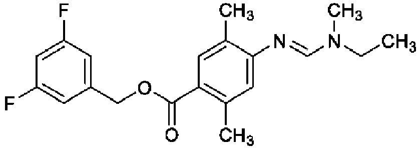 |
| 509 | 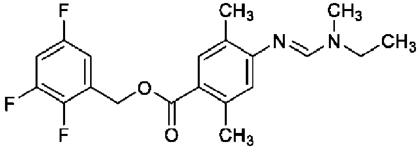 |
| 510 | 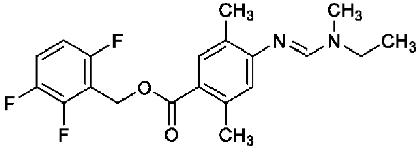 |
| 511 | 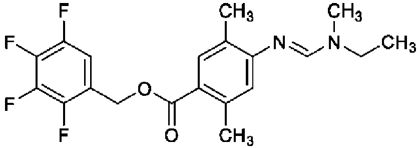 |
| 512 | 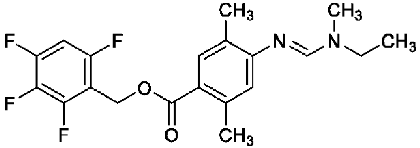 |
| 513 | 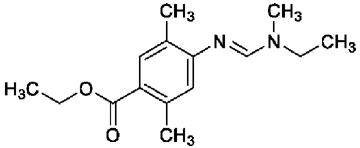 |
| 514 | 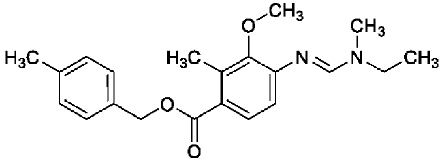 |
| 515 | 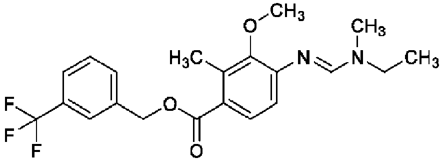 |
| 516 | 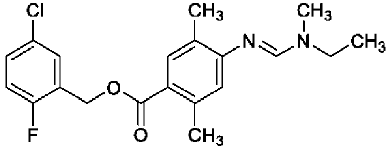 |
| 517 | 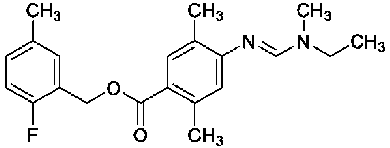 |
| 518 | 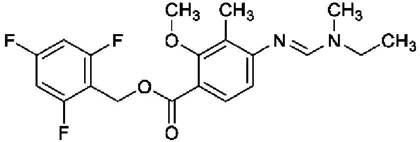 |
| 519 | 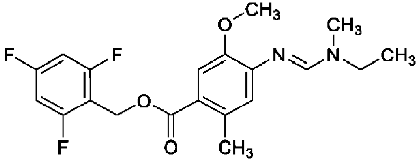 |
| 520 | 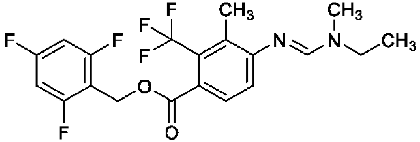 |
| 521 | 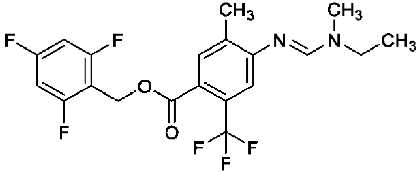 |
| 522 | 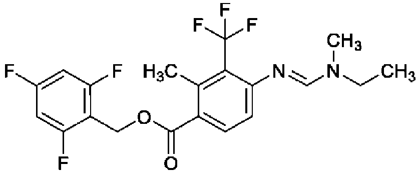 |
| 523 | 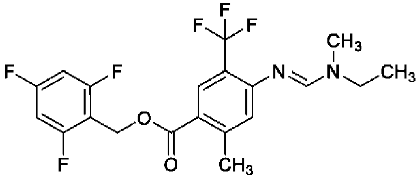 |
| 524 | 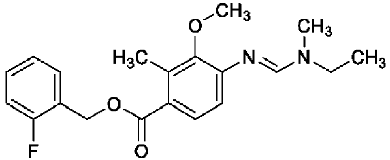 |
| 525 | 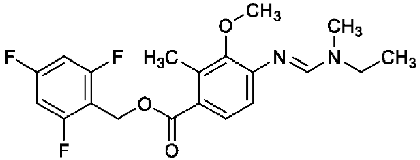 |
| 526 | 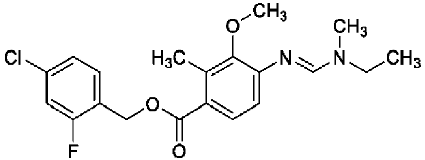 |
| 527 | 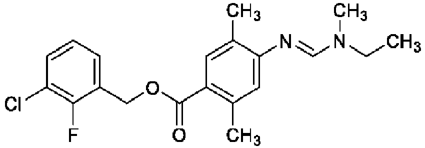 |
| 528 | 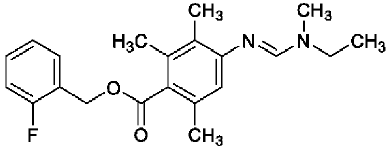 |
| | |
| 530 | 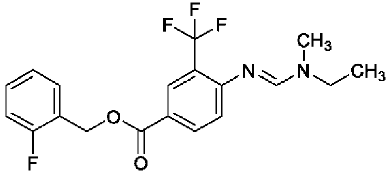 |
| 531 | 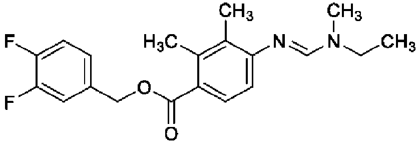 |
| 532 | 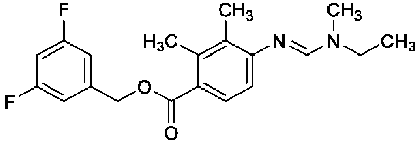 |
| 533 | 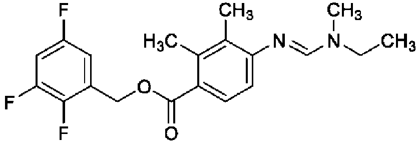 |
| 534 | 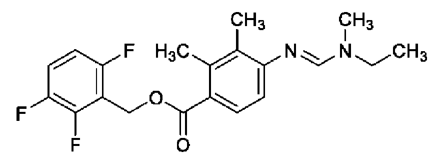 |
| 535 | 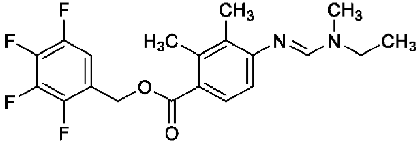 |
| 536 | 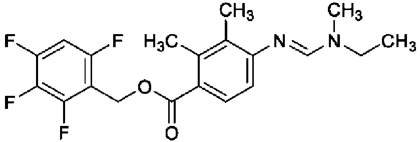 |
| 537 | 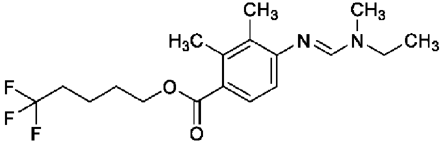 |
| 538 | 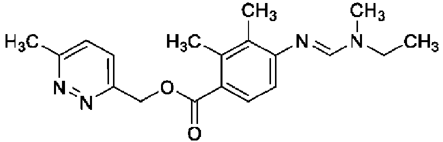 |
| 539 | 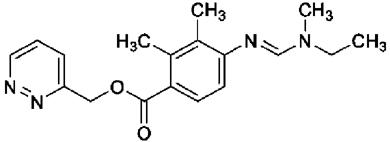 |
| 540 | 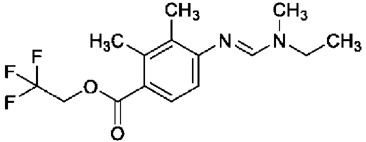 |
| 541 | 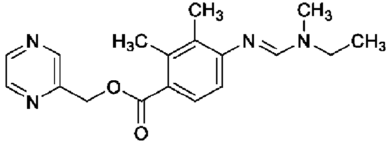 |
| 542 | 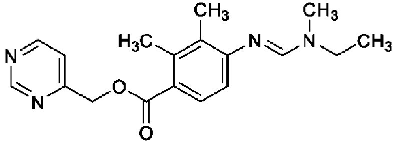 |
| 543 | 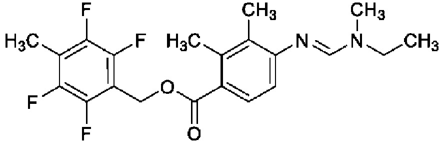 |
| 544 | 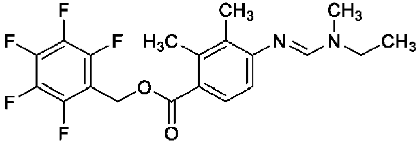 |
| 545 | 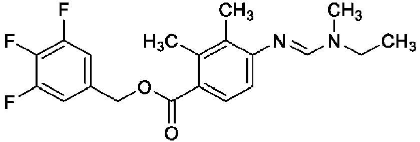 |
| 546 | 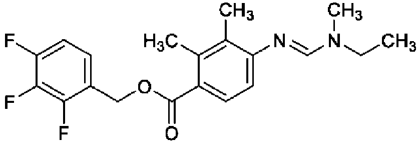 |
| 547 | 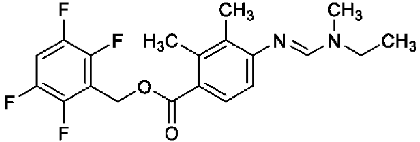 |
| 548 | 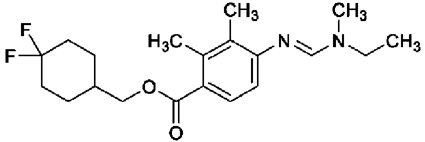 |
| 549 | 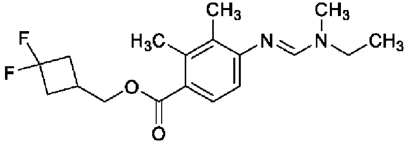 |
| 550 | 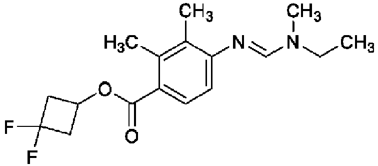 |
| 551 | 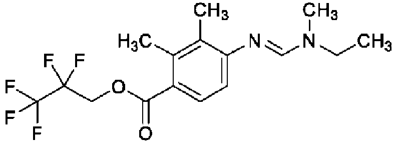 |
| 552 | 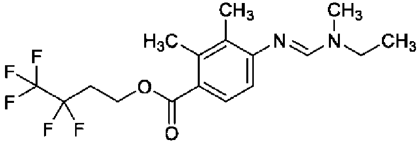 |
| 553 | 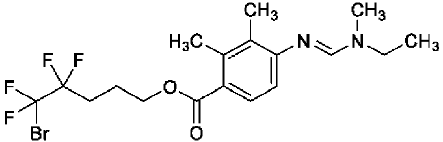 |
| 554 | 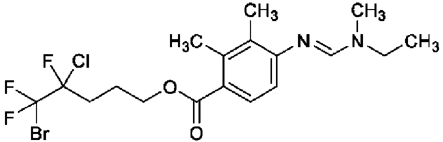 |
| 555 | 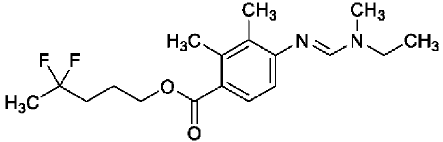 |
| 556 | 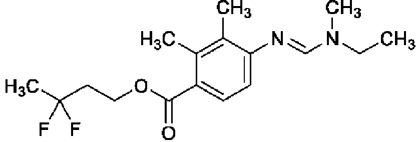 |
| 557 | 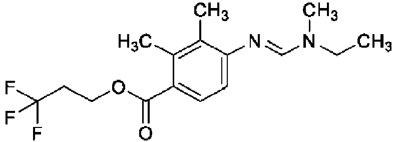 |
| 558 | 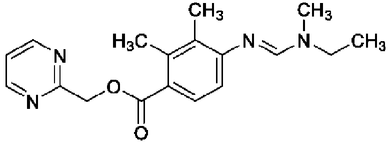 |
| 559 | 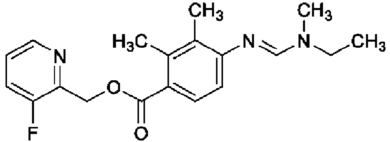 |
| 560 | 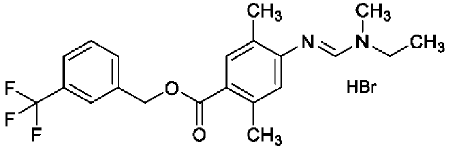 |
| 561 | 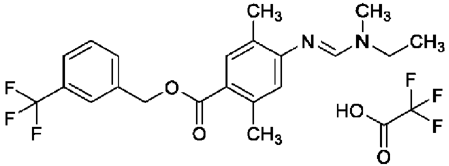 |
| 562 | 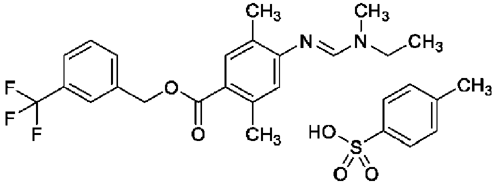 |
| 563 | 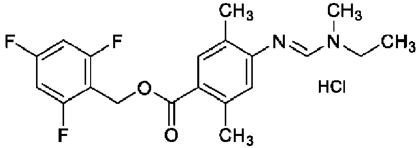 |
| | |

9. Composition fongicide comprenant une quantité acceptable du point de vue phytologique d'un composé de formule I de l'une quelconque des revendications 1-8 et un support, de préférence dans laquelle le support est l'un ou plusieurs parmi un épaississant, un émulsifiant, un agent de rhéologie, un dispersant et un polymère.

10. Procédé de lutte contre une attaque fongique sur une plante, le procédé comprenant la mise en contact d'une zone adjacente à la plante, d'un sol adapté pour soutenir la croissance de la plante, d'une racine de la plante et du feuillage de la plante, avec une quantité acceptable du point de vue phytologique d'un composé de formule I selon l'une quelconque des revendications 1-8 ou d'une composition selon la revendication 9.

11. Composé de formule I selon l'une quelconque des revendications 1-8 pour une utilisation dans la lutte contre un pathogène fongique.

12. Composé pour utilisation selon la revendication 11, dans lequel l'agent pathogène fongique est l'un des suivants : Zymoseptoria tritici, Cochliobolus sativus, Puccinia triticina, Puccinia striiformis, Venturia inaequalis, Ustilago maydis, Uncinula necator, Rhynchosporium commune, Magnaporthe grisea, Phakopsora pachyrhizi, Parastagonospora nodorum, Glomerella lagenarium, Cercospora beticola, Alternaria solani, Pyrenophora teres, Blumeria graminis f. sp. tritici, Blumeria graminis f. sp. hordei, Erysiphe cichoracearum, Fusarium virguliforme, Rhizoctonia solani, Pythium ultimum, et Botrytis cinerea ; ou
le composé traitant l'une des maladies suivantes de l'agent pathogène fongique : septoriose du blé (Zymoseptoria tritici), tache helminthosporienne de l'orge (Cochliobolus sativus), rouille brune du blé (Puccinia triticina), rouille striée (Puccinia striiformis), tavelure du pommier (Venturia inaequalis), charbon vésiculaire du maïs (Ustilago maydis), oïdium de la vigne (Uncinula necator), tache foliaire de l'orge (Rhynchosporium commune), pyriculariose du riz (Magnaporthe grisea), rouille asiatique du soja (Phakopsora pachyrhizi), tache des glumes du blé (Parastagonospora nodorum), anthracnose des cucurbitacées (Glomerella lagenarium), tache foliaire de la betterave (Cercospora beticola), mildiou précoce de la tomate (Alternaria solani), tache réticulée de l'orge (Pyrenophora teres), oïdium du blé (Blumeria graminis f. sp. tritici), oïdium de l'orge (Blumeria graminis f. sp. hordei), oïdium des cucurbitacées (Erysiphe cichoracearum), syndrome de mort subite du soja (Fusarium virguliforme), pourriture du collet ou fonte des semis (Rhizoctonia solani), pourriture des racines (Pythium ultimum), pourriture grise (Botrytis cinerea).

13. Composition pour utilisation dans la lutte contre un pathogène fongique, la composition comprenant une quantité acceptable du point de vue phytologique d'un composé de formule I selon l'une quelconque des revendications 1 à 8, 11 ou 12 et un support.

14. Composition pour utilisation selon la revendication 13, dans laquelle l'agent pathogène fongique est parmi Zymoseptoria tritici, Cochliobolus sativus, Puccinia triticina, Puccinia striiformis, Venturia inaequalis, Ustilago maydis, Uncinula necator, Rhynchosporium commune, Magnaporthe grisea, Phakopsora pachyrhizi, Parastagonospora nodorum, Glomerella lagenarium, Cercospora beticola, Alternaria solani, Pyrenophora teres, Blumeria graminis f. sp. tritici, Blumeria graminis f. sp. hordei, Erysiphe cichoracearum, Fusarium virguliforme, Rhizoctonia solani, Pythium ultimum, et Botrytis cinerea; ou
dans laquelle la composition traite l'une des maladies suivantes du pathogène fongique : septoriose du blé (Zymoseptoria tritici), tache helminthosporienne de l'orge (Cochliobolus sativus), rouille brune du blé (Puccinia triticina), rouille striée (Puccinia striiformis), tavelure du pommier (Venturia inaequalis), charbon vésiculaire du maïs (Ustilago maydis), oïdium de la vigne (Uncinula necator), tache foliaire de l'orge (Rhynchosporium commune), pyriculariose du riz (Magnaporthe grisea), rouille asiatique du soja (Phakopsora pachyrhizi), tache des glumes du blé (Parastagonospora nodorum), anthracnose des cucurbitacées (Glomerella lagenarium), tache foliaire de la betterave (Cercospora beticola), mildiou précoce de la tomate (Alternaria solani), tache réticulée de l'orge (Pyrenophora teres), oïdium du blé (Blumeria graminis f. sp. tritici), oïdium de l'orge (Blumeria graminis f. sp. hordei), oïdium des cucurbitacées (Erysiphe cichoracearum), syndrome de mort subite du soja (Fusarium virguliforme), pourriture du collet ou fonte des semis (Rhizoctonia solani), pourriture des racines (Pythium ultimum), pourriture grise (Botrytis cinerea) ; ou
dans laquelle la maladie est l'une parmi la septoriose du blé, la tache helminthosporienne de l'orge, la rouille brune du blé et la rouille asiatique du soja.
